# EUROPEAN PATENT SPECIFICATION

(11) **EP 2 901 154 B1**
(45) Date of publication and mention of the grant of the patent: **21.11.2018**
(21) Application number: 13842727.3
(22) Date of filing: 18.09.2013
(51) Int. Cl.: G01N 33/483, C07K 1/32, C07K 14/62, C07K 14/72, G01N 23/20, G01N 23/207, G01N 33/74, G06F 19/12

(54) **STRUCTURE OF INSULIN IN COMPLEX WITH N- AND C-TERMINAL REGIONS OF THE INSULIN RECEPTOR ALPHA-CHAIN**
STRUKTUR VON INSULIN IN EINEM KOMPLEX MIT N- UND C-TERMINALEN REGIONEN DER INSULINREZEPTOR-ALPHA-KETTE
STRUCTURE D'INSULINE DANS UN COMPLEXE AVEC DES RÉGIONS TERMINALES EN N ET EN C DE LA CHAÎNE ALPHA DU RÉCEPTEUR DE L'INSULINE

(30) Priority: 25.09.2012 US 201261705199 P
(43) Date of publication of application: 05.08.2015
(73) Proprietor: The Walter and Eliza Hall Institute of Medical Research, Melbourne, VIC 3052 (AU)
(72) Inventor: LAWRENCE, Michael Colin, Melbourne, Victoria 3052 (AU); MENTING, John Gerbrandt Tasman, Melbourne, Victoria 3052 (AU); SMITH, Brian John, Melbourne, Victoria 3052 (AU)
(74) Representative: Sweetinburgh, Mark Roger
(86) International application number: PCT/AU2013/001068
(87) International publication number: WO 2014/047673

(56) References cited:
- WO-A1-2007/147213
- WO-A1-2010/121288
- WO-A2-2012/123519
- US-A1- 2011 294 729
- JOHN G. MENTING ET AL: "How insulin engages its primary binding site on the insulin receptor", NATURE, vol. 493, no. 7431, 9 January 2013 (2013-01-09), pages 241-245, XP055262772, United Kingdom ISSN: 0028-0836, DOI: 10.1038/nature11781
- JOHN G. MENTING ET AL: "How insulin engages its primary binding site on the insulin receptor - Supplementary Information", NATURE, vol. 493, no. 7431, 9 January 2013 (2013-01-09), pages 241-245, XP055151811, ISSN: 0028-0836, DOI: 10.1038/nature11781
- J. WHITTAKER ET AL: "-Helical element at the hormone-binding surface of the insulin receptor functions as a signaling element to activate its tyrosine kinase", PROCEEDINGS OF THE NATIONAL ACADEMY OF SCIENCES, vol. 109, no. 28, 26 June 2012 (2012-06-26), pages 11166-11171, XP055262734, US ISSN: 0027-8424, DOI: 10.1073/pnas.1205681109
- MCKERN, N.M. ET AL.: 'Structure of the insulin receptor ectodomain reveals a folded-over conformation' NATURE vol. 443, no. 7108, 2006, pages 218 - 221, XP008102956
- SMITH, B.J. ET AL.: 'Structural resolution of a tandem hormone-binding element in the insulin receptor and its implications for design of peptide agonists' PROCEEDINGS OF THE NATIONAL ACADEMY OF SCIENCES USA vol. 107, no. 15, 2010, pages 6771 - 6776, XP055043610
- DE MEYTS, P.: 'Structural Biology of the Insulin Receptor Interaction and Implications for Drug Design' AMERICAN PHARMACEUTICAL REVIEW vol. 10, no. 7, 2007, pages 63 - 66, XP002541649
- WARD, C.W. ET AL.: 'Landmarks in insulin research' FRONTIERS IN ENDOCRINOLOGY vol. 2, no. 76, 2011, XP055197193

### SEQUENCE LISTING

<110> The Walter and Eliza Hall Institute of Medical Research
<120> STRUCTURE OF INSULIN IN COMPLEX WITH N- AND C-TERMINAL REGIONS OF THE INSULIN RECEPTOR ALPHA-CHAIN
<130> A/16/270
<160> 15
<170> PatentIn version 3.5
<210> 1
   <211> 1343
   <212> PRT
   <213> Homo sapiens
<220>
   <223> Human insulin receptor isoform A
<400> 1
   His Leu Tyr Pro Gly Glu Val Cys Pro Gly Met Asp Ile Arg Asn Asn
   1 5 10 15
   Leu Thr Arg Leu His Glu Leu Glu Asn Cys Ser Val Ile Glu Gly His
   20 25 30
   Leu Gln Ile Leu Leu Met Phe Lys Thr Arg Pro Glu Asp Phe Arg Asp
   35 40 45
   Leu Ser Phe Pro Lys Leu Ile Met Ile Thr Asp Tyr Leu Leu Leu Phe
   50 55 60
   Arg Val Tyr Gly Leu Glu Ser Leu Lys Asp Leu Phe Pro Asn Leu Thr
   65 70 75 80
   Val Ile Arg Gly Ser Arg Leu Phe Phe Asn Tyr Ala Leu Val Ile Phe
   85 90 95
   Glu Met Val His Leu Lys Glu Leu Gly Leu Tyr Asn Leu Met Asn Ile
   100 105 110
   Thr Arg Gly Ser Val Arg Ile Glu Lys Asn Asn Glu Leu Cys Tyr Leu
   115 120 125
   Ala Thr Ile Asp Trp Ser Arg Ile Leu Asp Ser Val Glu Asp Asn Tyr
   130 135 140
   Ile Val Leu Asn Lys Asp Asp Asn Glu Glu Cys Gly Asp Ile Cys Pro
   145 150 155 160
   Gly Thr Ala Lys Gly Lys Thr Asn Cys Pro Ala Thr Val Ile Asn Gly
   165 170 175
   Gln Phe Val Glu Arg Cys Trp Thr His Ser His Cys Gln Lys Val Cys
   180 185 190
   Pro Thr Ile Cys Lys Ser His Gly Cys Thr Ala Glu Gly Leu Cys Cys
   195 200 205
   His Ser Glu Cys Leu Gly Asn Cys Ser Gln Pro Asp Asp Pro Thr Lys
   210 215 220
   Cys Val Ala Cys Arg Asn Phe Tyr Leu Asp Gly Arg Cys Val Glu Thr
   225 230 235 240
   Cys Pro Pro Pro Tyr Tyr His Phe Gln Asp Trp Arg Cys Val Asn Phe
                   245                 250                 255
   Ser Phe Cys Gln Asp Leu His His Lys Cys Lys Asn Ser Arg Arg Gln
               260                 265                 270
   Gly Cys His Gln Tyr Val Ile His Asn Asn Lys Cys Ile Pro Glu Cys
           275                 280                 285
   Pro Ser Gly Tyr Thr Met Asn Ser Ser Asn Leu Leu Cys Thr Pro Cys
       290                 295                 300
   Leu Gly Pro Cys Pro Lys Val Cys His Leu Leu Glu Gly Glu Lys Thr
   305                 310                 315                 320
   Ile Asp Ser Val Thr Ser Ala Gln Glu Leu Arg Gly Cys Thr Val Ile
                   325                 330                 335
   Asn Gly Ser Leu Ile Ile Asn Ile Arg Gly Gly Asn Asn Leu Ala Ala
               340                 345                 350
   Glu Leu Glu Ala Asn Leu Gly Leu Ile Glu Glu Ile Ser Gly Tyr Leu
           355                 360                 365
   Lys Ile Arg Arg Ser Tyr Ala Leu Val Ser Leu Ser Phe Phe Arg Lys
       370                 375                 380
   Leu Arg Leu Ile Arg Gly Glu Thr Leu Glu Ile Gly Asn Tyr Ser Phe
   385                 390                 395                 400
   Tyr Ala Leu Asp Asn Gln Asn Leu Arg Gln Leu Trp Asp Trp Ser Lys
                   405                 410                 415
   His Asn Leu Thr Ile Thr Gln Gly Lys Leu Phe Phe His Tyr Asn Pro
               420                 425                 430
   Lys Leu Cys Leu Ser Glu Ile His Lys Met Glu Glu Val Ser Gly Thr
           435                 440                 445
   Lys Gly Arg Gln Glu Arg Asn Asp Ile Ala Leu Lys Thr Asn Gly Asp
       450                 455                 460
   Gln Ala Ser Cys Glu Asn Glu Leu Leu Lys Phe Ser Tyr Ile Arg Thr
   465                 470                 475                 480
   Ser Phe Asp Lys Ile Leu Leu Arg Trp Glu Pro Tyr Trp Pro Pro Asp
                   485                 490                 495
   Phe Arg Asp Leu Leu Gly Phe Met Leu Phe Tyr Lys Glu Ala Pro Tyr
               500                 505                 510
   Gln Asn Val Thr Glu Phe Asp Gly Gln Asp Ala Cys Gly Ser Asn Ser
           515                 520                 525
   Trp Thr Val Val Asp Ile Asp Pro Pro Leu Arg Ser Asn Asp Pro Lys
       530                 535                 540
   Ser Gln Asn His Pro Gly Trp Leu Met Arg Gly Leu Lys Pro Trp Thr
   545                 550                 555                 560
   Gln Tyr Ala Ile Phe Val Lys Thr Leu Val Thr Phe Ser Asp Glu Arg
                   565                 570                 575
   Arg Thr Tyr Gly Ala Lys Ser Asp Ile Ile Tyr Val Gln Thr Asp Ala
               580                 585                 590
   Thr Asn Pro Ser Val Pro Leu Asp Pro Ile Ser Val Ser Asn Ser Ser
           595                 600                 605
   Ser Gln Ile Ile Leu Lys Trp Lys Pro Pro Ser Asp Pro Asn Gly Asn
       610                 615                 620
   Ile Thr His Tyr Leu Val Phe Trp Glu Arg Gln Ala Glu Asp Ser Glu
   625                 630                 635                 640
   Leu Phe Glu Leu Asp Tyr Cys Leu Lys Gly Leu Lys Leu Pro Ser Arg
                   645                 650                 655
   Thr Trp Ser Pro Pro Phe Glu Ser Glu Asp Ser Gln Lys His Asn Gln
               660                 665                 670
   Ser Glu Tyr Glu Asp Ser Ala Gly Glu Cys Cys Ser Cys Pro Lys Thr
           675                 680                 685
   Asp Ser Gln Ile Leu Lys Glu Leu Glu Glu Ser Ser Phe Arg Lys Thr
       690                 695                 700
   Phe Glu Asp Tyr Leu His Asn Val Val Phe Val Pro Arg Pro Ser Arg
   705                 710                 715                 720
   Lys Arg Arg Ser Leu Gly Asp Val Gly Asn Val Thr Val Ala Val Pro
                   725                 730                 735
   Thr Val Ala Ala Phe Pro Asn Thr Ser Ser Thr Ser Val Pro Thr Ser
               740                 745                 750
   Pro Glu Glu His Arg Pro Phe Glu Lys Val Val Asn Lys Glu Ser Leu
           755                 760                 765
   Val Ile Ser Gly Leu Arg His Phe Thr Gly Tyr Arg Ile Glu Leu Gln
       770                 775                 780
   Ala Cys Asn Gln Asp Thr Pro Glu Glu Arg Cys Ser Val Ala Ala Tyr
   785                 790                 795                 800
   Val Ser Ala Arg Thr Met Pro Glu Ala Lys Ala Asp Asp Ile Val Gly
                   805                 810                 815
   Pro Val Thr His Glu Ile Phe Glu Asn Asn Val Val His Leu Met Trp
               820                 825                 830
   Gln Glu Pro Lys Glu Pro Asn Gly Leu Ile Val Leu Tyr Glu Val Ser
           835                 840                 845
   Tyr Arg Arg Tyr Gly Asp Glu Glu Leu His Leu Cys Val Ser Arg Lys
       850                 855                 860
   His Phe Ala Leu Glu Arg Gly Cys Arg Leu Arg Gly Leu Ser Pro Gly
   865                 870                 875                 880
   Asn Tyr Ser Val Arg Ile Arg Ala Thr Ser Leu Ala Gly Asn Gly Ser
                   885                 890                 895
   Trp Thr Glu Pro Thr Tyr Phe Tyr Val Thr Asp Tyr Leu Asp Val Pro
               900                 905                 910
   Ser Asn Ile Ala Lys Ile Ile Ile Gly Pro Leu Ile Phe Val Phe Leu
           915                 920                 925
   Phe Ser Val Val Ile Gly Ser Ile Tyr Leu Phe Leu Arg Lys Arg Gln
       930                 935                 940
   Pro Asp Gly Pro Leu Gly Pro Leu Tyr Ala Ser Ser Asn Pro Glu Tyr
   945                 950                 955                 960
   Leu Ser Ala Ser Asp Val Phe Pro Cys Ser Val Tyr Val Pro Asp Glu
                   965                 970                 975
   Trp Glu Val Ser Arg Glu Lys Ile Thr Leu Leu Arg Glu Leu Gly Gln
               980                 985                 990
   Gly Ser Phe Gly Met Val Tyr Glu Gly Asn Ala Arg Asp Ile Ile Lys
           995                 1000                 1005
   Gly Glu Ala Glu Thr Arg Val Ala Val Lys Thr Val Asn Glu Ser
       1010                 1015                 1020
   Ala Ser Leu Arg Glu Arg Ile Glu Phe Leu Asn Glu Ala Ser Val
       1025                 1030                 1035
   Met Lys Gly Phe Thr Cys His His Val Val Arg Leu Leu Gly Val
       1040                 1045                 1050
   Val Ser Lys Gly Gln Pro Thr Leu Val Val Met Glu Leu Met Ala
       1055                 1060                 1065
   His Gly Asp Leu Lys Ser Tyr Leu Arg Ser Leu Arg Pro Glu Ala
       1070                 1075                 1080
   Glu Asn Asn Pro Gly Arg Pro Pro Pro Thr Leu Gln Glu Met Ile
       1085                 1090                 1095
   Gln Met Ala Ala Glu Ile Ala Asp Gly Met Ala Tyr Leu Asn Ala
       1100                 1105                 1110
   Lys Lys Phe Val His Arg Asp Leu Ala Ala Arg Asn Cys Met Val
           1115                    1120                    1125
   Ala His  Asp Phe Thr Val Lys  Ile Gly Asp Phe Gly  Met Thr Arg
       1130                    1135                    1140
   Asp Ile  Tyr Glu Thr Asp Tyr  Tyr Arg Lys Gly Gly  Lys Gly Leu
       1145                    1150                    1155
   Leu Pro  Val Arg Trp Met Ala  Pro Glu Ser Leu Lys  Asp Gly Val
       1160                    1165                    1170
   Phe Thr  Thr Ser Ser Asp Met  Trp Ser Phe Gly Val  Val Leu Trp
       1175                    1180                    1185
   Glu Ile  Thr Ser Leu Ala Glu  Gln Pro Tyr Gln Gly  Leu Ser Asn
       1190                    1195                    1200
   Glu Gln  Val Leu Lys Phe Val  Met Asp Gly Gly Tyr  Leu Asp Gln
       1205                    1210                    1215
   Pro Asp  Asn Cys Pro Glu Arg  Val Thr Asp Leu Met  Arg Met Cys
       1220                    1225                    1230
   Trp Gln  Phe Asn Pro Lys Met  Arg Pro Thr Phe Leu  Glu Ile Val
       1235                    1240                    1245
   Asn Leu  Leu Lys Asp Asp Leu  His Pro Ser Phe Pro  Glu Val Ser
       1250                    1255                    1260
   Phe Phe  His Ser Glu Glu Asn  Lys Ala Pro Glu Ser  Glu Glu Leu
       1265                    1270                    1275
   Glu Met  Glu Phe Glu Asp Met  Glu Asn Val Pro Leu  Asp Arg Ser
       1280                    1285                    1290
   Ser His  Cys Gln Arg Glu Glu  Ala Gly Gly Arg Asp  Gly Gly Ser
       1295                    1300                    1305
   Ser Leu  Gly Phe Lys Arg Ser  Tyr Glu Glu His Ile  Pro Tyr Thr
       1310                    1315                    1320
   His Met  Asn Gly Gly Lys Lys  Asn Gly Arg Ile Leu  Thr Leu Pro
       1325                    1330                    1335
   Arg Ser  Asn Pro Ser
       1340
<210> 2
   <211> 1355
   <212> PRT
   <213> Homo sapiens
<220>
   <223> Human insulin receptor isoform B
<400> 2
   His Leu Tyr Pro Gly Glu Val Cys Pro Gly Met Asp Ile Arg Asn Asn
   1               5                   10                  15
   Leu Thr Arg Leu His Glu Leu Glu Asn Cys Ser Val Ile Glu Gly His
               20                  25                  30
   Leu Gln Ile Leu Leu Met Phe Lys Thr Arg Pro Glu Asp Phe Arg Asp
           35                  40                  45
   Leu Ser Phe Pro Lys Leu Ile Met Ile Thr Asp Tyr Leu Leu Leu Phe
       50                  55                  60
   Arg Val Tyr Gly Leu Glu Ser Leu Lys Asp Leu Phe Pro Asn Leu Thr
   65                  70                  75                  80
   Val Ile Arg Gly Ser Arg Leu Phe Phe Asn Tyr Ala Leu Val Ile Phe
                   85                  90                  95
   Glu Met Val His Leu Lys Glu Leu Gly Leu Tyr Asn Leu Met Asn Ile
   100 105 110
   Thr Arg Gly Ser Val Arg Ile Glu Lys Asn Asn Glu Leu Cys Tyr Leu
   115 120 125
   Ala Thr Ile Asp Trp Ser Arg Ile Leu Asp Ser Val Glu Asp Asn Tyr
   130 135 140
   Ile Val Leu Asn Lys Asp Asp Asn Glu Glu Cys Gly Asp Ile Cys Pro
   145 150 155 160
   Gly Thr Ala Lys Gly Lys Thr Asn Cys Pro Ala Thr Val Ile Asn Gly
   165 170 175
   Gln Phe Val Glu Arg Cys Trp Thr His Ser His Cys Gln Lys Val Cys
   180 185 190
   Pro Thr Ile Cys Lys Ser His Gly Cys Thr Ala Glu Gly Leu Cys Cys
   195 200 205
   His Ser Glu Cys Leu Gly Asn Cys Ser Gln Pro Asp Asp Pro Thr Lys
   210 215 220
   Cys Val Ala Cys Arg Asn Phe Tyr Leu Asp Gly Arg Cys Val Glu Thr
   225 230 235 240
   Cys Pro Pro Pro Tyr Tyr His Phe Gln Asp Trp Arg Cys Val Asn Phe
   245 250 255
   Ser Phe Cys Gln Asp Leu His His Lys Cys Lys Asn Ser Arg Arg Gln
   260 265 270
   Gly Cys His Gln Tyr Val Ile His Asn Asn Lys Cys Ile Pro Glu Cys
   275 280 285
   Pro Ser Gly Tyr Thr Met Asn Ser Ser Asn Leu Leu Cys Thr Pro Cys
   290 295 300
   Leu Gly Pro Cys Pro Lys Val Cys His Leu Leu Glu Gly Glu Lys Thr
   305 310 315 320
   Ile Asp Ser Val Thr Ser Ala Gln Glu Leu Arg Gly Cys Thr Val Ile
   325 330 335
   Asn Gly Ser Leu Ile Ile Asn Ile Arg Gly Gly Asn Asn Leu Ala Ala
   340 345 350
   Glu Leu Glu Ala Asn Leu Gly Leu Ile Glu Glu Ile Ser Gly Tyr Leu
   355 360 365
   Lys Ile Arg Arg Ser Tyr Ala Leu Val Ser Leu Ser Phe Phe Arg Lys
   370 375 380
   Leu Arg Leu Ile Arg Gly Glu Thr Leu Glu Ile Gly Asn Tyr Ser Phe
   385 390 395 400
   Tyr Ala Leu Asp Asn Gln Asn Leu Arg Gln Leu Trp Asp Trp Ser Lys
   405 410 415
   His Asn Leu Thr Ile Thr Gln Gly Lys Leu Phe Phe His Tyr Asn Pro
   420 425 430
   Lys Leu Cys Leu Ser Glu Ile His Lys Met Glu Glu Val Ser Gly Thr
   435 440 445
   Lys Gly Arg Gln Glu Arg Asn Asp Ile Ala Leu Lys Thr Asn Gly Asp
   450 455 460
   Gln Ala Ser Cys Glu Asn Glu Leu Leu Lys Phe Ser Tyr Ile Arg Thr
   465 470 475 480
   Ser Phe Asp Lys Ile Leu Leu Arg Trp Glu Pro Tyr Trp Pro Pro Asp
   485 490 495
   Phe Arg Asp Leu Leu Gly Phe Met Leu Phe Tyr Lys Glu Ala Pro Tyr
   500 505 510
   Gln Asn Val Thr Glu Phe Asp Gly Gln Asp Ala Cys Gly Ser Asn Ser
   515 520 525
   Trp Thr Val Val Asp Ile Asp Pro Pro Leu Arg Ser Asn Asp Pro Lys
   530 535 540
   Ser Gln Asn His Pro Gly Trp Leu Met Arg Gly Leu Lys Pro Trp Thr
   545 550 555 560
   Gln Tyr Ala Ile Phe Val Lys Thr Leu Val Thr Phe Ser Asp Glu Arg
   565 570 575
   Arg Thr Tyr Gly Ala Lys Ser Asp Ile Ile Tyr Val Gln Thr Asp Ala
   580 585 590
   Thr Asn Pro Ser Val Pro Leu Asp Pro Ile Ser Val Ser Asn Ser Ser
   595 600 605
   Ser Gln Ile Ile Leu Lys Trp Lys Pro Pro Ser Asp Pro Asn Gly Asn
   610 615 620
   Ile Thr His Tyr Leu Val Phe Trp Glu Arg Gln Ala Glu Asp Ser Glu
   625 630 635 640
   Leu Phe Glu Leu Asp Tyr Cys Leu Lys Gly Leu Lys Leu Pro Ser Arg
   645 650 655
   Thr Trp Ser Pro Pro Phe Glu Ser Glu Asp Ser Gln Lys His Asn Gln
   660 665 670
   Ser Glu Tyr Glu Asp Ser Ala Gly Glu Cys Cys Ser Cys Pro Lys Thr
   675 680 685
   Asp Ser Gln Ile Leu Lys Glu Leu Glu Glu Ser Ser Phe Arg Lys Thr
   690 695 700
   Phe Glu Asp Tyr Leu His Asn Val Val Phe Val Pro Arg Lys Thr Ser
   705 710 715 720
   Ser Gly Thr Gly Ala Glu Asp Pro Arg Pro Ser Arg Lys Arg Arg Ser
   725 730 735
   Leu Gly Asp Val Gly Asn Val Thr Val Ala Val Pro Thr Val Ala Ala
   740 745 750
   Phe Pro Asn Thr Ser Ser Thr Ser Val Pro Thr Ser Pro Glu Glu His
   755 760 765
   Arg Pro Phe Glu Lys Val Val Asn Lys Glu Ser Leu Val Ile Ser Gly
   770 775 780
   Leu Arg His Phe Thr Gly Tyr Arg Ile Glu Leu Gln Ala Cys Asn Gln
   785 790 795 800
   Asp Thr Pro Glu Glu Arg Cys Ser Val Ala Ala Tyr Val Ser Ala Arg
   805 810 815
   Thr Met Pro Glu Ala Lys Ala Asp Asp Ile Val Gly Pro Val Thr His
   820 825 830
   Glu Ile Phe Glu Asn Asn Val Val His Leu Met Trp Gln Glu Pro Lys
   835 840 845
   Glu Pro Asn Gly Leu Ile Val Leu Tyr Glu Val Ser Tyr Arg Arg Tyr
   850 855 860
   Gly Asp Glu Glu Leu His Leu Cys Val Ser Arg Lys His Phe Ala Leu
   865 870 875 880
   Glu Arg Gly Cys Arg Leu Arg Gly Leu Ser Pro Gly Asn Tyr Ser Val
   885 890 895
   Arg Ile Arg Ala Thr Ser Leu Ala Gly Asn Gly Ser Trp Thr Glu Pro
   900 905 910
   Thr Tyr Phe Tyr Val Thr Asp Tyr Leu Asp Val Pro Ser Asn Ile Ala
   915 920 925
   Lys Ile Ile Ile Gly Pro Leu Ile Phe Val Phe Leu Phe Ser Val Val
   930 935 940
   Ile Gly Ser Ile Tyr Leu Phe Leu Arg Lys Arg Gln Pro Asp Gly Pro
   945 950 955 960
   Leu Gly Pro Leu Tyr Ala Ser Ser Asn Pro Glu Tyr Leu Ser Ala Ser
   965 970 975
   Asp Val Phe Pro Cys Ser Val Tyr Val Pro Asp Glu Trp Glu Val Ser
               980                 985                 990
   Arg Glu Lys Ile Thr Leu Leu Arg  Glu Leu Gly Gln Gly  Ser Phe Gly
           995                 1000                 1005
   Met Val  Tyr Glu Gly Asn Ala  Arg Asp Ile Ile Lys  Gly Glu Ala
       1010                 1015                 1020
   Glu Thr  Arg Val Ala Val Lys  Thr Val Asn Glu Ser  Ala Ser Leu
       1025                 1030                 1035
   Arg Glu  Arg Ile Glu Phe Leu  Asn Glu Ala Ser Val  Met Lys Gly
       1040                 1045                 1050
   Phe Thr  Cys His His Val Val  Arg Leu Leu Gly Val  Val Ser Lys
       1055                 1060                 1065
   Gly Gln  Pro Thr Leu Val Val  Met Glu Leu Met Ala  His Gly Asp
       1070                 1075                 1080
   Leu Lys  Ser Tyr Leu Arg Ser  Leu Arg Pro Glu Ala  Glu Asn Asn
       1085                 1090                 1095
   Pro Gly  Arg Pro Pro Pro Thr  Leu Gln Glu Met Ile  Gln Met Ala
       1100                 1105                 1110
   Ala Glu  Ile Ala Asp Gly Met  Ala Tyr Leu Asn Ala  Lys Lys Phe
       1115                 1120                 1125
   Val His  Arg Asp Leu Ala Ala  Arg Asn Cys Met Val  Ala His Asp
       1130                 1135                 1140
   Phe Thr  Val Lys Ile Gly Asp  Phe Gly Met Thr Arg  Asp Ile Tyr
       1145                 1150                 1155
   Glu Thr  Asp Tyr Tyr Arg Lys  Gly Gly Lys Gly Leu  Leu Pro Val
       1160                 1165                 1170
   Arg Trp  Met Ala Pro Glu Ser  Leu Lys Asp Gly Val  Phe Thr Thr
       1175                 1180                 1185
   Ser Ser  Asp Met Trp Ser Phe  Gly Val Val Leu Trp  Glu Ile Thr
       1190                 1195                 1200
   Ser Leu  Ala Glu Gln Pro Tyr  Gln Gly Leu Ser Asn  Glu Gln Val
       1205                 1210                 1215
   Leu Lys  Phe Val Met Asp Gly  Gly Tyr Leu Asp Gln  Pro Asp Asn
       1220                 1225                 1230
   Cys Pro  Glu Arg Val Thr Asp  Leu Met Arg Met Cys  Trp Gln Phe
       1235                 1240                 1245
   Asn Pro  Lys Met Arg Pro Thr  Phe Leu Glu Ile Val  Asn Leu Leu
       1250                 1255                 1260
   Lys Asp  Asp Leu His Pro Ser  Phe Pro Glu Val Ser  Phe Phe His
       1265                 1270                 1275
   Ser Glu  Glu Asn Lys Ala Pro  Glu Ser Glu Glu Leu  Glu Met Glu
       1280                 1285                 1290
   Phe Glu  Asp Met Glu Asn Val  Pro Leu Asp Arg Ser  Ser His Cys
       1295                 1300                 1305
   Gln Arg  Glu Glu Ala Gly Gly  Arg Asp Gly Gly Ser  Ser Leu Gly
       1310                 1315                 1320
   Phe Lys  Arg Ser Tyr Glu Glu  His Ile Pro Tyr Thr  His Met Asn
       1325                 1330                 1335
   Gly Gly  Lys Lys Asn Gly Arg  Ile Leu Thr Leu Pro  Arg Ser Asn
       1340                 1345                 1350
   Pro Ser
       1355
<210> 3
   <211> 1337
   <212> PRT
   <213> homo sapiens
<220>
   <223> Human Type 1 insulin-like growth factor receptor
<400> 3
   Glu Ile Cys Gly Pro Gly Ile Asp Ile Arg Asn Asp Tyr Gln Gln Leu
   1               5                   10                  15
   Lys Arg Leu Glu Asn Cys Thr Val Ile Glu Gly Tyr Leu His Ile Leu
               20                  25                  30
   Leu Ile Ser Lys Ala Glu Asp Tyr Arg Ser Tyr Arg Phe Pro Lys Leu
           35                  40                  45
   Thr Val Ile Thr Glu Tyr Leu Leu Leu Phe Arg Val Ala Gly Leu Glu
       50                  55                  60
   Ser Leu Gly Asp Leu Phe Pro Asn Leu Thr Val Ile Arg Gly Trp Lys
   65                  70                  75                  80
   Leu Phe Tyr Asn Tyr Ala Leu Val Ile Phe Glu Met Thr Asn Leu Lys
                   85                  90                  95
   Asp Ile Gly Leu Tyr Asn Leu Arg Asn Ile Thr Arg Gly Ala Ile Arg
               100                 105                 110
   Ile Glu Lys Asn Ala Asp Leu Cys Tyr Leu Ser Thr Val Asp Trp Ser
           115                 120                 125
   Leu Ile Leu Asp Ala Val Ser Asn Asn Tyr Ile Val Gly Asn Lys Pro
       130                 135                 140
   Pro Lys Glu Cys Gly Asp Leu Cys Pro Gly Thr Met Glu Glu Lys Pro
   145                 150                 155                 160
   Met Cys Glu Lys Thr Thr Ile Asn Asn Glu Tyr Asn Tyr Arg Cys Trp
                   165                 170                 175
   Thr Thr Asn Arg Cys Gln Lys Met Cys Pro Ser Thr Cys Gly Lys Arg
               180                 185                 190
   Ala Cys Thr Glu Asn Asn Glu Cys Cys His Pro Glu Cys Leu Gly Ser
           195                 200                 205
   Cys Ser Ala Pro Asp Asn Asp Thr Ala Cys Val Ala Cys Arg His Tyr
       210                 215                 220
   Tyr Tyr Ala Gly Val Cys Val Pro Ala Cys Pro Pro Asn Thr Tyr Arg
   225                 230                 235                 240
   Phe Glu Gly Trp Arg Cys Val Asp Arg Asp Phe Cys Ala Asn Ile Leu
                   245                 250                 255
   Ser Ala Glu Ser Ser Asp Ser Glu Gly Phe Val Ile His Asp Gly Glu
               260                 265                 270
   Cys Met Gln Glu Cys Pro Ser Gly Phe Ile Arg Asn Gly Ser Gln Ser
           275                 280                 285
   Met Tyr Cys Ile Pro Cys Glu Gly Pro Cys Pro Lys Val Cys Glu Glu
       290                 295                 300
   Glu Lys Lys Thr Lys Thr Ile Asp Ser Val Thr Ser Ala Gln Met Leu
   305                 310                 315                 320
   Gln Gly Cys Thr Ile Phe Lys Gly Asn Leu Leu Ile Asn Ile Arg Arg
                   325                 330                 335
   Gly Asn Asn Ile Ala Ser Glu Leu Glu Asn Phe Met Gly Leu Ile Glu
               340                 345                 350
   Val Val Thr Gly Tyr Val Lys Ile Arg His Ser His Ala Leu Val Ser
           355                 360                 365
   Leu Ser Phe Leu Lys Asn Leu Arg Leu Ile Leu Gly Glu Glu Gln Leu
       370                 375                 380
   Glu Gly Asn Tyr Ser Phe Tyr Val Leu Asp Asn Gln Asn Leu Gln Gln
   385                 390                 395                 400
   Leu Trp Asp Trp Asp His Arg Asn Leu Thr Ile Lys Ala Gly Lys Met
                   405                 410                 415
   Tyr Phe Ala Phe Asn Pro Lys Leu Cys Val Ser Glu Ile Tyr Arg Met
               420                 425                 430
   Glu Glu Val Thr Gly Thr Lys Gly Arg Gln Ser Lys Gly Asp Ile Asn
           435                 440                 445
   Thr Arg Asn Asn Gly Glu Arg Ala Ser Cys Glu Ser Asp Val Leu His
       450                 455                 460
   Phe Thr Ser Thr Thr Thr Ser Lys Asn Arg Ile Ile Ile Thr Trp His
   465                 470                 475                 480
   Arg Tyr Arg Pro Pro Asp Tyr Arg Asp Leu Ile Ser Phe Thr Val Tyr
                   485                 490                 495
   Tyr Lys Glu Ala Pro Phe Lys Asn Val Thr Glu Tyr Asp Gly Gln Asp
               500                 505                 510
   Ala Cys Gly Ser Asn Ser Trp Asn Met Val Asp Val Asp Leu Pro Pro
           515                 520                 525
   Asn Lys Asp Val Glu Pro Gly Ile Leu Leu His Gly Leu Lys Pro Trp
       530                 535                 540
   Thr Gln Tyr Ala Val Tyr Val Lys Ala Val Thr Leu Thr Met Val Glu
   545                 550                 555                 560
   Asn Asp His Ile Arg Gly Ala Lys Ser Glu Ile Leu Tyr Ile Arg Thr
                   565                 570                 575
   Asn Ala Ser Val Pro Ser Ile Pro Leu Asp Val Leu Ser Ala Ser Asn
               580                 585                 590
   Ser Ser Ser Gln Leu Ile Val Lys Trp Asn Pro Pro Ser Leu Pro Asn
           595                 600                 605
   Gly Asn Leu Ser Tyr Tyr Ile Val Arg Trp Gln Arg Gln Pro Gln Asp
       610                 615                 620
   Gly Tyr Leu Tyr Arg His Asn Tyr Cys Ser Lys Asp Lys Ile Pro Ile
   625                 630                 635                 640
   Arg Lys Tyr Ala Asp Gly Thr Ile Asp Ile Glu Glu Val Thr Glu Asn
                   645                 650                 655
   Pro Lys Thr Glu Val Cys Gly Gly Glu Lys Gly Pro Cys Cys Ala Cys
               660                 665                 670
   Pro Lys Thr Glu Ala Glu Lys Gln Ala Glu Lys Glu Glu Ala Glu Tyr
           675                 680                 685
   Arg Lys Val Phe Glu Asn Phe Leu His Asn Ser Ile Phe Val Pro Arg
       690                 695                 700
   Pro Glu Arg Lys Arg Arg Asp Val Met Gln Val Ala Asn Thr Thr Met
   705                 710                 715                 720
   Ser Ser Arg Ser Arg Asn Thr Thr Ala Ala Asp Thr Tyr Asn Ile Thr
                   725                 730                 735
   Asp Pro Glu Glu Leu Glu Thr Glu Tyr Pro Phe Phe Glu Ser Arg Val
               740                 745                 750
   Asp Asn Lys Glu Arg Thr Val Ile Ser Asn Leu Arg Pro Phe Thr Leu
           755                 760                 765
   Tyr Arg Ile Asp Ile His Ser Cys Asn His Glu Ala Glu Lys Leu Gly
       770                 775                 780
   Cys Ser Ala Ser Asn Phe Val Phe Ala Arg Thr Met Pro Ala Glu Gly
   785                 790                 795                 800
   Ala Asp Asp Ile Pro Gly Pro Val Thr Trp Glu Pro Arg Pro Glu Asn
                   805                 810                 815
   Ser Ile Phe Leu Lys Trp Pro Glu Pro Glu Asn Pro Asn Gly Leu Ile
               820                 825                 830
   Leu Met Tyr Glu Ile Lys Tyr Gly Ser Gln Val Glu Asp Gln Arg Glu
           835                 840                 845
   Cys Val Ser Arg Gln Glu Tyr Arg Lys Tyr Gly Gly Ala Lys Leu Asn
       850                 855                 860
   Arg Leu Asn Pro Gly Asn Tyr Thr Ala Arg Ile Gln Ala Thr Ser Leu
   865                 870                 875                 880
   Ser Gly Asn Gly Ser Trp Thr Asp Pro Val Phe Phe Tyr Val Gln Ala
                   885                 890                 895
   Lys Thr Gly Tyr Glu Asn Phe Ile His Leu Ile Ile Ala Leu Pro Val
               900                 905                 910
   Ala Val Leu Leu Ile Val Gly Gly Leu Val Ile Met Leu Tyr Val Phe
           915                 920                 925
   His Arg Lys Arg Asn Asn Ser Arg Leu Gly Asn Gly Val Leu Tyr Ala
       930                 935                 940
   Ser Val Asn Pro Glu Tyr Phe Ser Ala Ala Asp Val Tyr Val Pro Asp
   945                 950                 955                 960
   Glu Trp Glu Val Ala Arg Glu Lys Ile Thr Met Ser Arg Glu Leu Gly
                   965                 970                 975
   Gln Gly Ser Phe Gly Met Val Tyr Glu Gly Val Ala Lys Gly Val Val
               980                 985                 990
   Lys Asp Glu Pro Glu Thr Arg Val Ala Ile Lys Thr Val Asn Glu Ala
           995                 1000                1005
   Ala Ser Met Arg Glu Arg Ile Glu Phe Leu Asn Glu Ala Ser Val
       1010                1015                1020
   Met Lys Glu Phe Asn Cys His His Val Val Arg Leu Leu Gly Val
       1025                1030                1035
   Val Ser Gln Gly Gln Pro Thr Leu Val Ile Met Glu Leu Met Thr
       1040                1045                1050
   Arg Gly Asp Leu Lys Ser Tyr Leu Arg Ser Leu Arg Pro Glu Met
       1055                1060                1065
   Glu Asn Asn Pro Val Leu Ala Pro Pro Ser Leu Ser Lys Met Ile
       1070                1075                1080
   Gln Met Ala Gly Glu Ile Ala Asp Gly Met Ala Tyr Leu Asn Ala
       1085                1090                1095
   Asn Lys Phe Val His Arg Asp Leu Ala Ala Arg Asn Cys Met Val
       1100                1105                1110
   Ala Glu Asp Phe Thr Val Lys Ile Gly Asp Phe Gly Met Thr Arg
       1115                1120                1125
   Asp Ile Tyr Glu Thr Asp Tyr Tyr Arg Lys Gly Gly Lys Gly Leu
       1130                1135                1140
   Leu Pro Val Arg Trp Met Ser Pro Glu Ser Leu Lys Asp Gly Val
       1145                1150                1155
   Phe Thr Thr Tyr Ser Asp Val Trp Ser Phe Gly Val Val Leu Trp
       1160                1165                1170
   Glu Ile Ala Thr Leu Ala Glu Gln Pro Tyr Gln Gly Leu Ser Asn
       1175                1180                1185
   Glu Gln Val Leu Arg Phe Val Met Glu Gly Gly Leu Leu Asp Lys
       1190                1195                1200
   Pro Asp Asn Cys Pro Asp Met Leu Phe Glu Leu Met Arg Met Cys
       1205                1210                1215
   Trp Gln Tyr Asn Pro Lys Met Arg Pro Ser Phe Leu Glu Ile Ile
       1220                1225                1230
   Ser Ser Ile Lys Glu Glu Met Glu Pro Gly Phe Arg Glu Val Ser
   1235 1240 1245
   Phe Tyr Tyr Ser Glu Glu Asn Lys Leu Pro Glu Pro Glu Glu Leu
   1250 1255 1260
   Asp Leu Glu Pro Glu Asn Met Glu Ser Val Pro Leu Asp Pro Ser
   1265 1270 1275
   Ala Ser Ser Ser Ser Leu Pro Leu Pro Asp Arg His Ser Gly His
   1280 1285 1290
   Lys Ala Glu Asn Gly Pro Gly Pro Gly Val Leu Val Leu Arg Ala
   1295 1300 1305
   Ser Phe Asp Glu Arg Gln Pro Tyr Ala His Met Asn Gly Gly Arg
   1310 1315 1320
   Lys Asn Glu Arg Ala Leu Pro Leu Pro Gln Ser Ser Thr Cys
   1325 1330 1335
<210> 4
   <211> 21
   <212> PRT
   <213> homo sapiens
<220>
   <223> Human insulin A-chain
<400> 4
   Gly Ile Val Glu Gln Cys Cys Thr Ser Ile Cys Ser Leu Tyr Gln Leu
   1 5 10 15
   Glu Asn Tyr Cys Asn
   20
<210> 5
   <211> 30
   <212> PRT
   <213> Homo sapiens
<220>
   <223> Human insulin B-chain
<400> 5
   Phe Val Asn Gln His Leu Cys Gly Ser His Leu Val Glu Ala Leu Tyr
   1 5 10 15
   Leu Val Cys Gly Glu Arg Gly Phe Phe Tyr Thr Pro Lys Thr
   20 25 30
<210> 6
   <211> 70
   <212> PRT
   <213> Homo sapiens
<220>
   <223> Human insulin-like growth factor I
<400> 6
   Gly Pro Glu Thr Leu Cys Gly Ala Glu Leu Val Asp Ala Leu Gln Phe
   1               5                   10                  15
   Val Cys Gly Asp Arg Gly Phe Tyr Phe Asn Lys Pro Thr Gly Tyr Gly
               20                  25                  30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Lys Pro Ala Lys Ser Ala
   65                  70
<210> 7
   <211> 67
   <212> PRT
   <213> Homo sapiens
<220>
   <223> Human insulin-like growth factor II
<400> 7
   Ala Tyr Arg Pro Ser Glu Thr Leu Cys Gly Gly Glu Leu Val Asp Thr
   1               5                   10                  15
   Leu Gln Phe Val Cys Gly Asp Arg Gly Phe Tyr Phe Ser Arg Pro Ala
               20                  25                  30
   Ser Arg Val Ser Arg Arg Ser Arg Gly Ile Val Glu Glu Cys Cys Phe
           35                  40                  45
   Arg Ser Cys Asp Leu Ala Leu Leu Glu Thr Tyr Cys Ala Thr Pro Ala
       50                  55                  60
   Lys Ser Glu
   65
<210> 8
   <211> 317
   <212> PRT
   <213> Artificial Sequence
<220>
   <223> IR310.T
<400> 8
   His Leu Tyr Pro Gly Glu Val Cys Pro Gly Met Asp Ile Arg Asn Asn
   1               5                   10                  15
   Leu Thr Arg Leu His Glu Leu Glu Asn Cys Ser Val Ile Glu Gly His
               20                  25                  30
   Leu Gln Ile Leu Leu Met Phe Lys Thr Arg Pro Glu Asp Phe Arg Asp
           35                  40                  45
   Leu Ser Phe Pro Lys Leu Ile Met Ile Thr Asp Tyr Leu Leu Leu Phe
       50                  55                  60
   Arg Val Tyr Gly Leu Glu Ser Leu Lys Asp Leu Phe Pro Asn Leu Thr
   65                  70                  75                  80
   Val Ile Arg Gly Ser Arg Leu Phe Phe Asn Tyr Ala Leu Val Ile Phe
                   85                  90                  95
   Glu Met Val His Leu Lys Glu Leu Gly Leu Tyr Asn Leu Met Asn Ile
               100                 105                 110
   Thr Arg Gly Ser Val Arg Ile Glu Lys Asn Asn Glu Leu Cys Tyr Leu
           115                 120                 125
   Ala Thr Ile Asp Trp Ser Arg Ile Leu Asp Ser Val Glu Asp Asn Tyr
       130                 135                 140
   Ile Val Leu Asn Lys Asp Asp Asn Glu Glu Cys Gly Asp Ile Cys Pro
   145                 150                 155                 160
   Gly Thr Ala Lys Gly Lys Thr Asn Cys Pro Ala Thr Val Ile Asn Gly
                   165                 170                 175
   Gln Phe Val Glu Arg Cys Trp Thr His Ser His Cys Gln Lys Val Cys
               180                 185                 190
   Pro Thr Ile Cys Lys Ser His Gly Cys Thr Ala Glu Gly Leu Cys Cys
           195                 200                 205
   His Ser Glu Cys Leu Gly Asn Cys Ser Gln Pro Asp Asp Pro Thr Lys
       210                 215                 220
   Cys Val Ala Cys Arg Asn Phe Tyr Leu Asp Gly Arg Cys Val Glu Thr
   225                 230                 235                 240
   Cys Pro Pro Pro Tyr Tyr His Phe Gln Asp Trp Arg Cys Val Asn Phe
                   245                 250                 255
   Ser Phe Cys Gln Asp Leu His His Lys Cys Lys Asn Ser Arg Arg Gln
               260                 265                 270
   Gly Cys His Gln Tyr Val Ile His Asn Asn Lys Cys Ile Pro Glu Cys
           275                 280                 285
   Pro Ser Gly Tyr Thr Met Asn Ser Ser Asn Leu Leu Cys Thr Pro Cys
       290                 295                 300
   Leu Gly Pro Cys Pro Lys Ser Ser Ser Leu Val Pro Arg
   305                 310                 315
<210> 9
   <211> 16
   <212> PRT
   <213> Artificial Sequence
<220>
   <223> Amino acid sequence of the alpha-chain C-terminal peptide of
   IR-A
<400> 9
   Thr Phe Glu Asp Tyr Leu His Asn Val Val Phe Val Pro Arg Pro Ser
   1 5 10 15
<210> 10
   <211> 26
   <212> PRT
   <213> Artificial Sequence
<220>
   <223> Amino acid sequence of the alpha-chain C-terminal peptide of
   IR-B
<400> 10
   Thr Phe Glu Asp Tyr Leu His Asn Val Val Phe Val Pro Arg Lys Thr
   1 5 10 15
   Ser Ser Gly Thr Gly Ala Glu Asp Pro Arg
   20 25
<210> 1-1
   <211> 239
   <212> PRT
   <213> Artificial Sequence
<220>
   <223> MAb 83-7 IgG1 heavy chain
<400> 11
<210> 12
   <211> 239
   <212> PRT
   <213> Artificial Sequence
<220>
   <223> MAb 83-3 kappa light chain
<400> 12
   Met Asp Ser Gln Ala Gln Val Leu Met Leu Leu Leu Leu Trp Ile Ser
   1               5                   10                  15
   Gly Thr Cys Gly Asp Ile Val Met Ser Gln Ser Pro Ser Ser Leu Val
               20                  25                  30
   Val Ser Val Gly Glu Lys Val Thr Met Ser Cys Lys Ser Ser Gln Ser
           35                  40                  45
   Leu Leu Tyr Ser Ser Asn Gln Lys Asn Phe Leu Ala Trp Tyr Gln Gln
       50                  55                  60
   Lys Pro Gly Gln Ser Pro Lys Leu Leu Ile Tyr Trp Ala Ser Thr Arg
   65                  70                  75                  80
   Glu Ser Gly Val Pro Asp Arg Phe Thr Gly Ser Gly Ser Gly Thr Asp
                   85                  90                  95
   Phe Thr Leu Thr Ile Ser Ser Val Lys Ala Glu Asp Leu Ala Val Tyr
               100                 105                 110
   Tyr Cys Gln Gln Tyr Phe Arg Tyr Arg Thr Phe Gly Gly Gly Thr Lys
           115                 120                 125
   Leu Glu Ile Lys Arg Ala Asp Ala Ala Pro Thr Val Ser Ile Phe Pro
       130                 135                 140
   Pro Ser Ser Glu Gln Leu Thr Ser Gly Gly Ala Ser Val Val Cys Phe
   145                 150                 155                 160
   Leu Asn Asn Phe Tyr Pro Lys Asp Ile Asn Val Lys Trp Lys Ile Asp
                   165                 170                 175
   Gly Ser Glu Arg Gln Asn Gly Val Leu Asn Ser Trp Thr Asp Gln Asp
               180                 185                 190
   Ser Lys Asp Ser Thr Tyr Ser Met Ser Ser Thr Leu Thr Leu Thr Lys
           195                 200                 205
   Asp Glu Tyr Glu Arg His Asn Ser Tyr Thr Cys Glu Ala Thr His Lys
       210                 215                 220
   Thr Ser Thr Ser Pro Ile Val Lys Ser Phe Asn Arg Gly Glu Cys
   225                 230                 235
<210> 13
   <211> 219
   <212> PRT
   <213> Artificial Sequence
<220>
   <223> FAb 83-7 kappa light chain
<400> 13
   Asp Ile Val Met Ser Gln Ser Pro Ser Ser Leu Val Val Ser Val Gly
   1               5                   10                  15
   Glu Lys Val Thr Met Ser Cys Lys Ser Ser Gln Ser Leu Leu Tyr Ser
               20                  25                  30
   Ser Asn Gln Lys Asn Phe Leu Ala Trp Tyr Gln Gln Lys Pro Gly Gln
                   35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Ser Ser Val Lys Ala Glu Asp Leu Ala Val Tyr Tyr Cys Gln Gln
                   85                  90                  95
   Tyr Phe Arg Tyr Arg Thr Phe Gly Gly Gly Thr Lys Leu Glu Ile Lys
               100                 105                 110
   Arg Ala Asp Ala Ala Pro Thr Val Ser Ile Phe Pro Pro Ser Ser Glu
           115                 120                 125
   Gln Leu Thr Ser Gly Gly Ala Ser Val Val Cys Phe Leu Asn Asn Phe
       130                 135                 140
   Tyr Pro Lys Asp Ile Asn Val Lys Trp Lys Ile Asp Gly Ser Glu Arg
   145                 150                 155                 160
   Gln Asn Gly Val Leu Asn Ser Trp Thr Asp Gln Asp Ser Lys Ser Ser
                   165                 170                 175
   Thr Tyr Ser Met Ser Ser Thr Leu Thr Leu Thr Lys Asp Glu Tyr Glu
               180                 185                 190
   Arg His Asn Ser Tyr Thr Cys Glu Ala Thr His Lys Thr Ser Thr Ser
           195                 200                 205
   Pro Ile Val Lys Ser Phe Asn Arg Gly Glu Cys
       210                 215
<210> 14
   <211> 220
   <212> PRT
   <213> Artificial Sequence
<220>
   <223> FAb 83-7 IgG1 heavy chain
<400> 14
   Gln Val Gln Leu Lys Glu Ser Gly Pro Gly Leu Val Ala Pro Ser Gln
   1               5                   10                  15
   Ser Leu Ser Ile Thr Cys Thr Val Ser Gly Phe Pro Leu Thr Ala Tyr
               20                  25                  30
   Gly Val Asn Trp Val Arg Gln Pro Pro Gly Lys Gly Leu Glu Trp Leu
           35                  40                  45
   Gly Met Ile Trp Gly Asp Gly Asn Thr Asp Tyr Asn Ser Ala Leu Lys
       50                  55                  60
   Ser Arg Leu Ser Ile Ser Lys Asp Asn Ser Lys Ser Gln Val Phe Leu
   65                  70                  75                  80
   Lys Met Asn Ser Leu Gln Thr Asp Asp Thr Ala Arg Tyr Tyr Cys Ala
                   85                  90                  95
   Arg Asp Pro Tyr Gly Ser Lys Pro Met Asp Tyr Trp Gly Gln Gly Thr
               100                 105                 110
   Ser Val Thr Val Ser Ser Ala Lys Thr Thr Pro Pro Ser Val Tyr Pro
           115                 120                 125
   Leu Ala Pro Gly Ser Ala Ala Gln Thr Asn Ser Met Val Thr Leu Gly
       130                 135                 140
   Cys Leu Val Lys Gly Tyr Phe Pro Glu Pro Val Thr Val Thr Trp Asn
   145                 150                 155                 160
   Ser Gly Ser Leu Ser Ser Gly Val His Thr His Pro Ala Val Leu Gln
                   165                 170                 175
   Ser Asp Leu Tyr Thr Leu Ser Ser Ser Val Thr Val Pro Ser Ser Thr
               180                 185                 190
   Trp Pro Ser Glu Thr Val Thr Cys Asn Val Ala His Pro Ala Ser Ser
           195                 200                 205
   Thr Lys Val Asp Lys Lys Ile Val Pro Arg Asp Cys
       210                 215                 220
<210> 15
   <211> 540
   <212> PRT
   <213> Artificial Sequence
<220>
   <223> cIR485
<400> 15
   Met Gly Thr Gly Gly Arg Arg Gly Ala Ala Ala Ala Pro Leu Leu Val
   1 5 10 15
   Ala Val Ala Ala Leu Leu Leu Gly Ala Ala Gly His Leu Tyr Pro Gly
   20 25 30
   Glu Val Cys Pro Gly Met Asp Ile Arg Asn Asn Leu Thr Arg Leu His
   35 40 45
   Glu Leu Glu Asn Cys Ser Val Ile Glu Gly His Leu Gln Ile Leu Leu
   50 55 60
   Met Phe Lys Thr Arg Pro Glu Asp Phe Arg Asp Leu Ser Phe Pro Lys
   65 70 75 80
   Leu Ile Met Ile Thr Asp Tyr Leu Leu Leu Phe Arg Val Tyr Gly Leu
   85 90 95
   Glu Ser Leu Lys Asp Leu Phe Pro Asn Leu Thr Val Ile Arg Gly Ser
   100 105 110
   Arg Leu Phe Phe Asn Tyr Ala Leu Val Ile Phe Glu Met Val His Leu
   115 120 125
   Lys Glu Leu Gly Leu Tyr Asn Leu Met Asn Ile Thr Arg Gly Ser Val
   130 135 140
   Arg Ile Glu Lys Asn Asn Glu Leu Cys Tyr Leu Ala Thr Ile Asp Trp
   145 150 155 160
   Ser Arg Ile Leu Asp Ser Val Glu Asp Asn His Ile Val Leu Asn Lys
   165 170 175
   Asp Asp Asn Glu Glu Cys Gly Asp Ile Cys Pro Gly Thr Ala Lys Gly
   180 185 190
   Lys Thr Asn Cys Pro Ala Thr Val Ile Asn Gly Gln Phe Val Glu Arg
   195 200 205
   Cys Trp Thr His Ser His Cys Gln Lys Val Cys Pro Thr Ile Cys Lys
   210 215 220
   Ser His Gly Cys Thr Ala Glu Gly Leu Cys Cys His Ser Glu Cys Leu
   225 230 235 240
   Gly Asn Cys Ser Gln Pro Asp Asp Pro Thr Lys Cys Val Ala Cys Arg
   245 250 255
   Asn Phe Tyr Leu Asp Gly Arg Cys Val Glu Thr Cys Pro Pro Pro Tyr
   260 265 270
   Tyr His Phe Gln Asp Trp Arg Cys Val Asn Phe Ser Phe Cys Gln Asp
           275                 280                 285
   Leu His His Lys Cys Lys Asn Ser Arg Arg Gln Gly Cys His Gln Tyr
       290                 295                 300
   Val Ile His Asn Asn Lys Cys Ile Pro Glu Cys Pro Ser Gly Tyr Thr
   305                 310                 315                 320
   Met Asn Ser Ser Asn Leu Leu Cys Thr Pro Cys Leu Gly Pro Cys Pro
                   325                 330                 335
   Lys Ser Ser Ser Leu Val Pro Arg Gly Ser Ser Ser Val Cys His Leu
               340                 345                 350
   Leu Glu Gly Glu Lys Thr Ile Asp Ser Val Thr Ser Ala Gln Glu Leu
           355                 360                 365
   Arg Gly Cys Thr Val Ile Asn Gly Ser Leu Ile Ile Asn Ile Arg Gly
       370                 375                 380
   Gly Asn Asn Leu Ala Ala Glu Leu Glu Ala Asn Leu Gly Leu Ile Glu
   385                 390                 395                 400
   Glu Ile Ser Gly Tyr Leu Lys Ile Arg Arg Ser Tyr Ala Leu Val Ser
                   405                 410                 415
   Leu Ser Phe Phe Arg Lys Leu Arg Leu Ile Arg Gly Glu Thr Leu Glu
               420                 425                 430
   Ile Gly Asn Tyr Ser Phe Tyr Ala Leu Asp Asn Gln Asn Leu Arg Gln
           435                 440                 445
   Leu Trp Asp Trp Ser Lys His Asn Leu Thr Ile Thr Gln Gly Lys Leu
       450                 455                 460
   Phe Phe His Tyr Asn Pro Lys Leu Cys Leu Ser Glu Ile His Lys Met
   465                 470                 475                 480
   Glu Glu Val Ser Gly Thr Lys Gly Arg Gln Glu Arg Asn Asp Ile Ala
                   485                 490                 495
   Leu Lys Thr Asn Gly Asp Gln Ala Ser Cys Glu Asn Glu Leu Leu Lys
               500                 505                 510
   Phe Ser Tyr Ile Arg Thr Ser Phe Asp Lys Ile Ser Asp Asp Asp Asp
           515                 520                 525
   Lys Glu Gln Lys Leu Ile Ser Glu Glu Asp Leu Asn
       530                 535                 540

## Description

### TECHNICAL FIELD

The present invention relates generally to structural studies of insulin binding to the insulin receptor ("IR"). In particular, the present invention relates to the crystal structure of insulin in complex with N- and C-terminal regions of the IR α-chain, and to methods of using the crystal structure and related structural information to identify, design and/or screen for agonists and antagonists that interact with and/or modulate the function of the IR.

### BACKGROUND ART

The insulin receptor ("IR"), and its homologue the type 1 insulin-like growth factor receptor ("IGF-1R"), are closely related members of the tyrosine kinase receptor family, and are large, transmembrane, glycoprotein dimers consisting of several structural domains.

Insulin, a hormone produced by the pancreas, interacts with the IR, and via the IR signals into a complex network that ultimately controls the fundamental cellular processes of cell growth and differentiation, protein synthesis, as well as glucose uptake, synthesis and homeostasis (Cohen, 2006; Taniguchi et al., 2006). Insulin signalling also plays a role in neuronal development (Chiu and Cline, 2010). Insulin's role in the treatment of diabetes has underpinned decades of research into the biochemical and biophysical properties of the hormone (Ward and Lawrence, 2011). More recently, aberrant insulin receptor signalling has become implicated in cancer, in part due to its overlap with the closely-related insulin-like growth factor ("IGF") signalling system and to an increased number of insulin receptors on cancer cells providing a mechanism for increased glucose uptake (Pollak, 2012). Brain insulin resistance is increasingly being seen as a potential causal factor in Alzheimer's disease (Talbot et al., 2012).

Despite these fundamental biological roles of insulin and the role of insulin signalling system in major diseases, there is to date no three-dimensional description of the way in which insulin interacts with the IR.

IR exists as two splice variant isoforms, IR-A and IR-B, which respectively lack or contain the 12 amino acids coded by exon 11. The longer variant, IR-B, is the isoform responsible for signalling metabolic responses. In contrast, IR-A signals predominantly mitogenic responses and is the preferentially expressed isoform in several cancers (Denley et al., 2003). Also, IR-A is capable of binding insulin-like growth factor 2 ("IGF-II") with high affinity (Frasca *et al.,* 1999; Denley et al., 2004).

The sequence of IR is highly homologous to the sequence of IGF-1R, indicating that their three-dimensional structures are highly likely to be similar. The mature human IR and IGF-1R molecules are each homodimers comprising two α-chains and two β-chains, the α- and β-chains arising from the post-translational cleavage at the furin cleavage site at residues 720-723 (IR-A numbering with the mature N-terminal residue numbered 1) or 707-710 (IGF-1R).

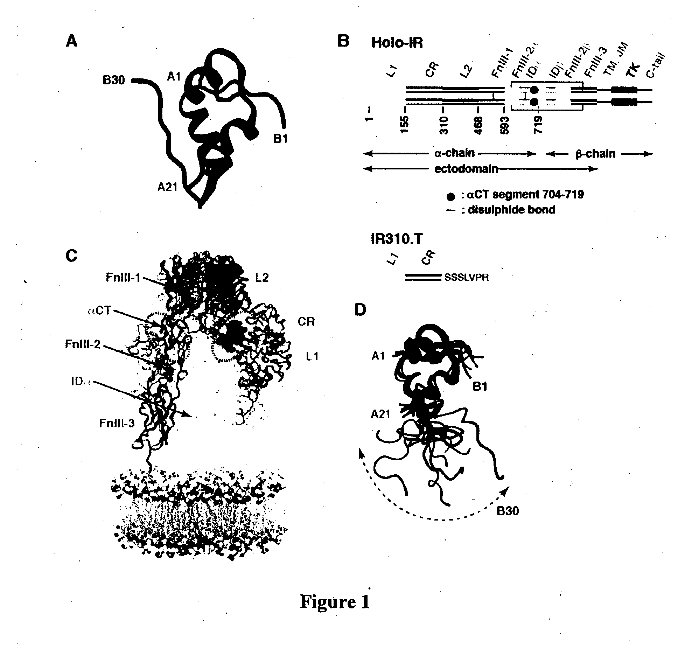

IR is a heavily-glycosylated, disulphide-linked (αβ)₂ homodimer of ∼380 kDa molecular weight. Each IR or IGF-1R monomer comprises, from its N-terminus, a leucine-rich repeat domain ("L1"), a cysteine-rich region ("CR"), a leucine-rich repeat domain ("L2"), three fibronectin Type III domains ("FnIII-1"; "FnIII-2"; and "FnIII-3"), trans- and juxtamembrane segments ("TM" and "JM"), a tyrosine kinase domain ("TK") and a C-terminal tail (McKern et al., 2006). The FnIII-2 domain contains, within its CC' loop, an insert domain ("ID") of ∼120 mostly-disordered residues within which lies the α/β cleavage site. Near the C-terminal region of the IR α-chain lies an amphipathic helical segment ("αCT") that in *apo*-IR spans residues 693-710 and packs against the central β-sheet (L1-β₂) of the L1 domain of the alternate IR monomer (Smith et al., 2010). The domain organisation of IR and the structure of *apo*-IR are presented in Figures 1B and 1C.

Intracellularly, the TK domain is flanked by two regulatory regions that contain the phosphotyrosine binding sites for signalling molecules. Each α-chain is linked to its β-chain via a disulphide bond between residues Cys647 and Cys860 (Sparrow *et al.,* 1997) in the case of IR and/or Cys633-Cys849 in the case of IGF-1R. The α-chains of both IR and IGF-1R are cross-linked by disulphide bonds in two places. The first is at Cys524 (IR) or Cys514 (IGF-1R) in the FnIII-1 domain, cross-linked to its counterpart in the opposite monomer, and the second involves one or more of the residues Cys682, Cys683 and Cys685 (IR) or Cys669, Cys670 and Cys672 (IGF-1R) in the insert region of each FnIII-2 domain, cross-linked to their counterparts in the opposite monomer (Sparrow *et al.,* 1997).

The domains of IR and IGF-1R exhibit high (47-67%) amino acid sequence identity indicative of high conservation of three-dimensional structure. The crystal structure of the first three domains of IGF-1R (L1-CR-L2) has been determined (Garrett *et al.,* 1998) and revealed that the L domains consist of a single-stranded right-handed β-helix (a helical arrangement of β-strands), while the cysteine-rich region is composed of eight related disulphide-bonded modules. The crystal structure of the first three domains of IR (L1-CR-L2) has also been determined (WO 07/147213, Lou et al., 2006) and as anticipated is closely similar to that of its IGF-1R counterpart. Other evidence for the close structural similarity of IR and IGF-1R arises from : (i) electron microscopic analyses (Tulloch *et al.,* 1999), (ii) the fact that hybrid receptors (heterodimers of one IR monomer disulphide-bonded to one of IGF-1R monomer) exist naturally and are commonly found in tissues expressing both receptors (Bailyes *et al.,* 1997), and (iii) the fact that receptor chimeras can be constructed which have whole domains or smaller segments of polypeptide from one receptor replaced by the corresponding domain or sequence from the other (reviewed in Adams *et al.,* 2000). Further to (ii) and (iii) above, physiologically relevant IR/IGF-1R receptor chimeras have been shown to form on cell surfaces (Belfiore *et al.,* 2009).

The current model for insulin binding proposes that, in the basal state, the IR homodimer contains two identical pairs of binding sites (referred to as "Site 1" and "Site 2") on each monomer (De Meyts and Whittaker, 2002; Schäffer, 1994; De Meyts, 1994; De Meyts, 2004; Kiselyov *et al.,* 2009). Binding of insulin to a low affinity site ("Site 1") on one α-subunit is followed by a second binding event between the bound insulin and a different region of the second IR α-subunit ("Site 2"). This ligand-mediated bridging between the two α-subunits generates the high affinity state that results in signal transduction. In contrast, soluble IR ectodomain, which is not tethered at its C-terminus, cannot generate the high affinity receptor-ligand complex. The soluble IR ectodomain can bind two molecules of insulin simultaneously at its two Site Is, but only with nanomolar affinity (Adams *et al.,* 2000).

The model for IGF-I or IGF-II binding to IGF-1R is the same as that just described for insulin binding to IR and involves IGF-I (or IGF-II) binding to an initial low affinity site ("Site 1") and subsequent cross-linking to a second site ("Site 2") on the opposite monomer to form the high affinity state, as described for the IR. However, the values of the kinetic parameters describing these events are somewhat different in the two systems (Surinya *et al.,* 2008; Kiselyov *et al.,* 2009).

In addition to the above models of ligand binding, IGF (IGF-I and IGF-II) and insulin have been shown to bind to IR and the IGF-1R, respectively, albeit at a lower affinity than their cognate receptors (Kristensen *et al.,* 1999; Nakae *et al.,* 2001; Kjeldsen *et al.,* 1991). However, as previously mentioned, the IR-A isoform variant of the IR is a high-affinity receptor for IGF-II (Frasca *et al.,* 1999).

While similar in structure, IGF-1R and IR serve different physiological functions. IGF-1R is expressed in almost all normal adult tissue except for liver, which is itself the major site of IGF-I production (Buttel *et al.,* 1999). A variety of signalling pathways are activated following binding of IGF-I or IGF-II to IGF-1R, including Src and Ras, as well as downstream pathways, such as the MAP kinase cascade and the P13K/AKT axis (Chow *et al.,* 1998). IR is primarily involved in metabolic functions whereas IGF-1R mediates growth and differentiation. Consistent with this, ablation of IGF-I (i.e., in IGF-I knock-out mice) results in embryonic growth deficiency, impaired postnatal growth, and infertility. In addition, IGF-1R knock-out mice were only 45% of normal size and died of respiratory failure at birth (Liu *et al.,* 1993). However, both insulin and IGF-I can induce both mitogenic and metabolic effects.

The insulin monomer comprises a 30-residue B-chain and 21-residue A-chain, together containing three α-helices, A1-A8, A12-A18 and B9-B19, connected by three disulphide bonds (Adams et al., 1969). In its pancreatic (storage) form insulin forms a hexamer (a trimer of dimers). The structure of the insulin monomer is presented in Figure 1A.

Two distinct surfaces of insulin are understood to interact with IR to initiate signalling (Schäffer, 1994). The first ("classical") surface consists predominantly of residues involved in hormone dimerization, and the second mostly of residues involved in hexamerization (De Meyts,2004).

The tandem L1-β₂/αCT element from the IR is understood to interact with the classical binding surface of insulin and forms part of Site 1 (Ward and Lawrence, 2009). The second binding surface of insulin is understood to interact with residues at the junction of the FnIII-1 and FnIII-2 domains of the opposite α-chain to that contributing the L1 domain to Site 1 (McKern et al., 2006; Whittaker et al., 2008), referred to as Site 2 (Ward and Lawrence, 2009). IR site 1 is the primary binding hormone site; its dissociation constant for insulin is estimated to be ∼6.4 nM vs ∼400 nM for Site 2 (Kiselyov et al., 2009).

Insulin and the IR ectodomain are proposed to undergo a conformational change upon hormone binding. A key proposed change in insulin is the detachment of the C-terminal B-chain residues B20-B30 (Hua et al., 1991) from the helical core. In the absence of structural characterization of the hormone-receptor complex, however, details of this conformational change have remained speculative. The proposed change in insulin is presented in Figure ID.

Insulin remains the key therapeutic for the treatment of both Type 1 and Type 2 diabetes. Its use as a therapeutic has, however, two major complications. The first concerns the method of delivery and the second concerns its profile of action.

In terms of the method of delivery, insulin is not orally bioavailable, and its delivery has to thus be via other means (injection, pumps, pens, nasal sprays, etc.). Formulation of insulin appropriate to each of these means of delivery remains an area of active research.

In terms of profile of action, none of the current modes of delivery can currently deliver insulin in such a way that the respective prandial, post-prandial and sleep concentrations of insulin can be accurately mimicked through a 24-hour cycle.

The above concerns have led to a new class of therapeutics, namely insulin analogues (or "designer" insulins). Examples of designer insulins include:
1. Humalog™ (or lispro insulin), wherein a two-residue segment of insulin ProB28-LysB29 is replaced by LysB28-ProB29. This modification does not alter receptor binding, but blocks the formation of insulin dimers and hexamers, allowing larger amounts of active monomeric insulin to be immediately available upon postprandial injection;
2. Lantus™ (or glargine), designed to be a long-acting insulin analogue. Glargine has a substitution AsnA21Gly and two arginine residues added to the B-chain C-terminus. Glargine is more soluble at acidic pH, thus allowing injection of a clear solution. However, in the neutral subcutaneous space, higher-order aggregates form, resulting in a slow, peakless dissolution and absorption of insulin from the site of injection. It can achieve a peakless level for at least 24 hours; and
3. Novolog™ (or aspart insulin), which is another fast-acting insulin with a substitution ProB28Asp. This analogue has increased charge repulsion, which prevents the formation of hexamers, to create faster-acting insulin.

However, the above designer insulins have been developed without the benefit of knowledge of the three-dimensional atomic details of the insulin/IR interaction. The absence of structural data on the insulin/IR complex has hindered a detailed understanding of ligand/IR interactions.

As mentioned above, structural information to date has been available only for insulin and parts of the IR ectodomain. Thus, there has been no structural information detailing the interactions between insulin and the IR, particularly the conformational changes posited to occur on insulin binding to the IR.

Accordingly, there is a need to determine the structure of insulin in complex with IR in order to better understand the nature of the insulin/IR interaction and its role in IR signalling in order to rationally design or redesign agonists and antagonists, particularly alternative insulin analogues, useful *inter alia* in the treatment of aberrant IR signalling diseases and/or disorders.

### SUMMARY OF INVENTION

The present invention is predicated in part by the determination of the crystal structure of insulin in complex with the N- and C-terminal regions of the IR α-chain, which allows visualisation, for the first time, of the long posited conformational change in insulin upon IR binding. The structure also reveals, for the first time, insulin bound in a stabilised form on the L1-β₂ surface of the IR by the αCT recognition helix and shows the accompanied remodelling of the Site 1 L1-β₂/αCT tandem element of the IR upon insulin binding (Smith et al., 2010). The atomic coordinates for the structure are presented in Appendix I.

The crystal structure reveals much needed structural information about insulin residues PheB24 and PheB25 during receptor binding. The critical importance of these insulin residues B24 and B25 for receptor binding is well known (Mayer et al., 2007). Clinical mutations at these sites include PheB24Ser (insulin Los Angeles) and PheB25Leu (insulin Chicago), these having 16% and 1-5% of the activity of normal insulin respectively (Shoelson et al., 1983). Studies have shown that PheB24Gly leads to reduced receptor affinity, while D-Ala and D-Phe substitutions at B24 results in a 150% and 140-180% increase in affinity relative to native insulin. Modifications to the phenyl ring of B24 mostly results in deleterious effects, while B25 accommodates sizable changes in side-chain structure, but is highly demanding in that the phenyl ring must be positioned at the β-carbon (Quan et al., 2006). A TyrB25 analogue demonstrates full *in vitro* activity. The significance of the size and orientation of the aromatic side-chain is revealed in the binding affinity for the 1- and 2-naphthylalanine analogues (24% and 50%, respectively). In contrast, non-aromatic substitution at B25 invariably produces low potency. SerB25 and AlaB25 analogues exhibit respective binding potencies of ∼1% and 7% of native insulin. Distance of the phenyl ring from the backbone appears critical to activity, as the homo-phenylalanine and phenylglycine derivatives are as impotent as the nonaromatic analogues.

Thus, derivatization at insulin residues B24 and B25 appears to modulate the potency of insulin. Accordingly, the present inventors consider that the determination of the crystal structure of human insulin in complex with Site 1 of the human IR α-chain, including detailed structural information regarding the interaction between insulin residues B24 and B25 and IR, will assist in the rational design of insulin B24 and B25 analogues. Similarly, it is considered that the structural information provided concerning the nature of the interactions between insulin B24 and B25 residues and IR, will assist in the use of molecular modelling and related techniques to design insulin analogues that have altered binding affinity and or pharmacological profiles due to their alterations at insulin B24 and/or insulin B25.

With the foregoing in view, one aspect of the present invention provides an insulin/IR α-chain crystalline complex comprising the N- and C-terminal regions of the IR α-chain having the atomic coordinates as set forth in Appendix I. Generally, the crystalline complex comprising insulin and the N- and C-terminal regions of the IR α-chain or their derivatives or components thereof are in essentially pure native form. Derivatives and homologues, higher order complexes and soluble forms of the crystalline complex or its components are also described herein.

Also described herein is a data set of atomic coordinates defining the Site 1 interaction between IR including the N- and C-terminal regions of the IR α-chain and insulin.

Further described are conformational mimetics of the Site 1 binding surface of insulin useful as antagonists or agonists of insulin signalling via the IR. The mimetics may interfere or interact directly with Site 1 or may interact elsewhere causing conformational changes to Site 1 or may influence the form or level of the insulin/IR complex.

In particular, a Site 1 antagonist is proposed to, for example, reduce aberrant IR signalling, which as previously mentioned has become implicated in cancer, to reduce the increased glucose uptake attributable to the increased number of IRs on cancer cells.

In particular, a Site 1 antagonist is proposed, for example, in the treatment of cancer by reducing aberrant IR signalling attributable to increased glucose uptake in cancer cells due to an increased number of IRs located on cancer cells.

Likewise, a Site 1 agonist is proposed, for example, in the treatment of both Type 1 and Type 2 diabetes.

More particularly, a Site 1 mimetic molecule is proposed that binds to the low affinity insulin binding site of IR with a high degree of specificity and imparts a selective agonistic or antagonistic activity.

With the foregoing in view, one aspect of the present invention provides an insulin/IR α-chain crystalline complex comprising the N- and C-terminal regions of the IR α-chain having the atomic coordinates as set forth in Appendix I. Generally, the crystalline complex comprising insulin and the N- and C-terminal regions of the IR α-chain or their derivatives or components thereof are in essentially pure native form.

Accordingly, the present invention in one form resides in an insulin/IR complex in crystalline form wherein the complex comprises the components of the structure defined by the atomic coordinates shown in Appendix I or a subset thereof.

In one embodiment the complex comprises the N- and C-terminal regions of the IR α-chain in complex with insulin.

In a preferred embodiment, the complex comprises a leucine-rich repeat domain ("L1") and an adjacent cysteine-rich region ("CR") from the N-terminus of the IR α-chain and a portion of an amphipathic helical segment ("αCT") from the C-terminus of the IR α-chain in complex with insulin.

In a more preferred embodiment, the complex comprises portions of the central β-sheet of the L1 domain and the αCT of the IR α-chain in complex with a portion of insulin.

As noted above, the complex of the present invention comprises the components of the structure defined by the atomic coordinates shown in Appendix I or a subset thereof.

The present invention in another form provides a method of identifying, designing or screening for a compound that can interact with IR, including performing structure-based identification, design, or screening of a compound based on the compound's interactions with an IR structure defined by the atomic coordinates of Appendix I or a subset thereof.

In another form, the present invention provides a method of identifying, designing or screening for a compound that can mimic insulin interacting with IR, including performing structure-based identification, design, or screening of a compound based on (i) the compound's interaction with an IR structure defined by the atomic coordinates of Appendix I or a subset thereof and/or (ii) the compound's similarity with an insulin structure in complex with an IR structure defined by the atomic coordinates of Appendix 1 or a subset thereof.

In one embodiment, the method includes identifying, designing or screening for a compound which interacts with the three-dimensional structure of the low affinity insulin binding site of IR, the structure being defined by the atomic coordinates shown in Appendix 1, wherein interaction of the compound with the structure is favoured energetically.

In another embodiment, the method includes identifying, designing or screening for a compound based upon the three-dimensional structure of insulin in complex with components of the low affinity insulin binding site of IR, the structure being defined by the atomic coordinates shown in Appendix 1, wherein interaction of the compound with the structure is favoured energetically.

In another embodiment, the method further includes synthesising or obtaining an identified or designed candidate compound and determining the ability of the candidate compound to interact with IR and/or mimic insulin in complex with IR.

The present invention in another form provides a method for identifying an agonist or an antagonist compound comprising an entity selected from the group consisting of an antibody, a peptide, a non-peptide molecule and a chemical compound, wherein said compound is capable of enhancing, eliciting or blocking biological activity resulting from an interaction with insulin and/or the IR, wherein said process includes:
introducing into a suitable computer program parameters defining an interacting surface based on the conformation of insulin and/or IR corresponding to the atomic coordinates of Appendix I or a subset thereof, wherein said program displays a three-dimensional model of the interacting surface;
creating a three-dimensional structure of a test compound in said computer program;
displaying a superimposing model of said test compound on the three-dimensional model of the interacting surface;
assessing whether said test compound model fits spatially and optionally energetically into a binding site;
optionally incorporating said test compound in a biological activity assay; and
optionally determining whether said test compound inhibits or enhances the biological activity of insulin or IR signalling or signalling by a derivative of insulin or IR.

In one embodiment, the method includes identifying an agonist or an antagonist compound capable of interacting with the nanomolar affinity insulin binding site ("Site 1") of the IR as defined by the atomic coordinates shown in Appendix I.

In a further embodiment, the atomic coordinates as shown in Appendix I or a subset thereof define one or more regions of insulin in complex with N- and C-terminal regions of the IR α-chain.

In a preferred embodiment, the atomic coordinates as shown in Appendix I or a subset thereof define insulin in complex with a L1 domain and an adjacent CR domain from the N-terminus of the IR α-chain and a portion of the αCT from the C-terminus of the IR α-chain.

In another preferred embodiment, the atomic coordinates as shown in Appendix I or a subset thereof define portions of the molecular surface of the central β-sheet of the L1 domain and the αCT of the IR α-chain, which interact with a portion of the molecular surface of insulin.

In a particularly preferred embodiment, the atomic coordinates as shown in Appendix I or a subset thereof define the molecular surface of a B-helix of insulin interacting with the molecular surface of the C-terminal edge of L1-β₂ of the IR α-chain, the atomic coordinates or a subset thereof defining the B-helix of insulin as lying parallel with and adjacent to a helical portion of the αCT of the IR α-chain.

In another particularly preferred embodiment, the atomic coordinates as shown in Appendix I or a subset thereof define the αCT of the IR α-chain occupying a volume that would otherwise be occupied by the insulin B-chain if insulin was not bound to components of Site 1 of IR. In a more particularly preferred embodiment, a volume that would otherwise be occupied by the C-terminal portion of the B-chain of unbound insulin. In a most particularly preferred embodiment, a volume that would otherwise be occupied by amino acids 26 to 30 from the B-chain of unbound insulin.

In another embodiment, the atomic coordinates define one or more amino acids selected from 1 to 310 and 704 to 719 of the IR α-chain, 1 to 30 from the insulin B-chain and 1 to 21 from the insulin A-chain.

In another preferred embodiment, the one or more amino acids selected from 1 to 310 and 704 to 719 of the IR α-chain include one or more amino acids selected from the group consisting of Asp12, Arg14, Leu36, Leu37, Phe39, Lys40, Leu62, Phe64, Arg65, Phe88, Phe89, Tyr91, Val94, Phe96, Arg118, Glu120, His144, Phe705, Tyr708, Leu709, His710, Asn711, Val712, Val713, Phe714 and Val715.

In another preferred embodiment, the one or more amino acids selected from 1 to 30 from the insulin B-chain include one or more amino acids selected from the group consisting of Gly8, Ser9, Leu11, Val12, Leul5, Tyr16, Phe24, Phe25 and Tyr26.

In another preferred embodiment, the one or more amino acids selected from 1 to 21 from the insulin A-chain include one or more amino acids selected from the group consisting of Glyl, Ile2, Val3, Glu4 and Tyr19.

In another embodiment, IGF-1 or IGF-II may be chemically modified to modify the binding affinity of IGF-I or IGF-II to bind to IR as a result of structure-based evaluation using the atomic coordinates as defined in Appendix I or a subset thereof.

In another form, the present invention includes use of the atomic coordinates or a subset thereof as shown in Appendix I at least representing:
(i) insulin; and/or
(ii) one or more regions of insulin in complex with the N- and C-terminal regions of the IR α-chain,
in identifying, designing or screening for a compound that can mimic insulin interacting with IR, including performing structure-based identification, design, or screening of a compound based on (a) the compound's interactions with an IR structure and/or (b) the compound's similarity with an insulin structure in complex with an IR defined by the atomic coordinates or a subset thereof.

In another form, the present invention includes use of the atomic coordinates or a subset thereof as shown in Appendix I at least representing:
(i) the N-terminal region of the IR α-chain;
(ii) the C-terminal region of the IR α-chain; and/or
(iii) one or more regions of the N- and C-terminal regions of the IR α-chain in complex with insulin,
in identifying, designing or screening for a compound that can interact with IR, including performing structure-based identification, design, or screening of a compound based on the compound's interactions with an IR structure defined by the atomic coordinates or a subset thereof.

Also described herein is a set of atomic coordinates as shown in Appendix I, or a subset of thereof, at least representing:
(i) the N-terminal region of the IR α-chain;
(ii) the C-terminal region of the IR α-chain;
(iii) insulin; and/or
(iv) one or more regions of insulin in complex with the N- and C-terminal regions of the IR α-chain.

Further described is an agonist or antagonist of a site comprising one or more amino acids selected from 1 to 310 and 704 to 719 of the IR α-chain including one or more amino acids selected from the group consisting of Asp12, Arg14, Leu36, Leu37, Phe39, Lys40, Leu62, Phe64, Arg65, Phe88, Phe89, Tyr91, Val94, Phe96, Arg118, Glu120, His144, Phe705, Tyr708, Leu709, His710, Asn711, Val712, Val713, Phe714 and Val715.

Also described is an agonist or antagonist that can potentially mimic insulin interacting with IR, said agonist or antagonist comprising one or more amino acids selected from 1 to 30 from the insulin B-chain including one or more amino acids selected from the group consisting of Gly8, Ser9, Leu11, Va112, Leu15, Tyr16, Phe24, Phe25 and Tyr26.

Additionally described is an agonist or antagonist that can potentially mimic insulin interacting with IR, said agonist or antagonist comprising one or more amino acids selected from the insulin A-chain including one or more amino acids selected from the group consisting of Gly1, Ile2, Val3, Glu4 and Tyr19.

The present invention has enabled the identification of molecular surface interactions between Site 1 on the IR α-chain and the Site 1 binding surface on insulin. Additionally, the present invention has enabled, for the first time, determination of conformational changes in insulin upon binding to the IR α-chain. In particular, the present invention has enabled the determination of key amino acid residues involved in the binding of insulin to the Site 1 binding surface on the IR α-chain. It will be evident to the skilled person that these findings can be transposed onto related receptors such as the IGF-1R, to which IGF-1 or IGF-II are modelled to bind in a similar fashion as insulin to IR.

The present invention is therefore also useful in the identification, screening and/or design of candidate compounds that bind to Site 1 of IGF-1R.

In one embodiment, candidate compounds for interacting with IR and/or IGF-1R may be chemically modified as a result of structure-based evaluation using the atomic coordinates as defined in Appendix I or a subset thereof.

In another embodiment, the chemical modification is designed to either:
(i) reduce the potential for the candidate compound to bind to IR whilst maintaining binding to IGF-1R; or
(ii) reduce the potential for the candidate compound to bind to IGF-1R, whilst maintaining binding to IR.

In another embodiment, insulin may be chemically modified to modify the binding affinity of insulin to bind to IGF-1R as a result of structure-based evaluation using the atomic coordinates as defined in Appendix I or a subset thereof.

The present invention is also useful in the identification, screening and/or design of candidate compounds that bind to IR/IGF-1R receptor chimeras. In particular, the present invention is useful in the identification, screening and/or design of candidate compounds that bind to the Site 1 binding site of an IR/IGF-1R receptor chimera. The Site 1 binding site of the IR/IGF-1R receptor chimera may include the L1 domain from one monomer of one receptor type and the aCT peptide from another monomer of the other receptor type.

In one embodiment, candidate compounds for interacting with an IR/IGF-1R receptor or part thereof may be chemically modified as a result of structure-based evaluation using the atomic coordinates as defined in Appendix I or a subset thereof.

In a particular embodiment, candidate compounds for interacting with a Site 1 binding site of an IR/IGF-1R receptor or part thereof may be chemically modified as a result of structure-based evaluation using the atomic coordinates as defined in Appendix I or a subset thereof.

In another embodiment, a ligand selected from the group consisting of insulin, IGF-I and IGF-II may be chemically modified to modify the binding affinity for the ligand to bind to an IR/IGF-1R receptor or part thereof as a result of structure-based evaluation using the atomic coordinates as defined in Appendix I or a subset thereof.

Candidate compounds and compounds identified or designed using a method or process of the present invention may be any suitable compound, including naturally occurring compounds, *de novo* designed compounds, library generated compounds (chemically or recombinantly generated), mimetics etc., and may include organic compounds, new chemical entities, antibodies, binding proteins other than antibody-based molecules (nonimmunoglobulin proteins) including, for example, protein scaffolds such as lipocalins, designed ankyrin repeat proteins (DARPins, Stumpp *et al.,* 2007) and protein A domains (reviewed in Binz *et al,* 2005), avimers (Silverman *et al.,* 2005), and other new biological entities such as nucleic acid aptamers (reviewed in Ulrich, 2006).

The present invention is also useful for improving the properties of known ligands for the Site 1 binding site of IR and/or IGF-1R. For example, existing IR or IGF-1R ligands may be screened against the 3D structure of the insulin binding site of IR or a region of the insulin binding site of IR as defined by the atomic coordinates of Appendix I or a subset thereof, and an assessment made of the potential to energetically interact with the insulin binding site of IR.

Similarly, existing IR or IGF-1R Site 1 ligands can be screened against the 3D structure of the binding surface of insulin bound to IR as defined by the atomic coordinates of Appendix I or a subset thereof, and an assessment made of the potential to energetically interact with the insulin binding site of IR.

Thus, the present invention also provides a method of redesigning a compound which is known to bind to IR and/or IGF-1R comprising performing structure-based evaluation of the compound based on the compound's interactions with an IR structure defined by the atomic coordinates of Appendix I or a subset thereof and redesigning or chemically modifying the compound as a result of the evaluation.

In another form, the present invention provides a method of redesigning a compound which is known to bind to IR and/or IGF-1R comprising performing structure-based evaluation of the compound's similarity with an insulin structure in complex with an IR defined by the atomic coordinates of Appendix I or a subset thereof and redesigning or chemically modifying the compound as a result of the evaluation.

In one embodiment, the compound which is known to bind to IR and/or IGF-1R is redesigned or chemically modified to (i) improve affinity for binding to IR, and/or (ii) lower affinity for binding to IGF-1R.

In another embodiment, the compound which is known to bind to IR and/or IGF-1R is redesigned or chemically modified to (i) improve affinity for binding to IGF-1R, and/or (ii) lower affinity for binding to IR.

In a further embodiment the compound is redesigned or modified so as to lower the affinity to IR or IGF-1R by virtue of the structural information provided by the structure of insulin in complex with the IR α-chain, as defined by the atomic coordinates shown in Appendix I, or a subset thereof.

Also described herein is a computer system for identifying one or more compounds that can potentially interact with IR and/or IGF-1R, the system containing data representing the structure of: (i) the Site 1 binding site of IR, the structure being defined by a subset of the atomic coordinates shown in Appendix I; (ii) the Site 1 binding site on insulin, the structure being defined by a subset of the atomic coordinates shown in Appendix I; and/or (iii) a combination thereof, the structure being defined by the atomic coordinates shown in Appendix I.

Further described is a computer-readable medium having recorded data thereon representing a model and/or the atomic coordinates as shown in Appendix I, or a subset of atomic coordinates thereof, the model and/or the atomic coordinates at least representing:
(i) the N-terminal region of the IR α-chain;
(ii) the C-terminal region of the IR α-chain;
(iii) insulin; and/or
(iv) one or more regions of insulin in complex with the N- and C-terminal regions of the IR α-chain.

Additionally described are a set of atomic coordinates as shown in Appendix I, or a subset of thereof, at least representing:
(i) the N-terminal region of the IR α-chain;
(ii) the C-terminal region of the IR α-chain;
(iii) insulin; and/or
(iv) one or more regions of insulin in complex with the N- and C-terminal regions of the IR α-chain.

The three-dimensional structure of the N- and/or C-terminal regions of the IR α-chain and/or insulin and/or the one or more regions of insulin in complex with the N- and C-terminal regions of the IR α-chain may be used to develop models useful for drug design and/or *in silico* screening of candidate compounds that interact with and/or modulate IR. Other physicochemical characteristics may also be used in developing the model, e.g. bonding, electrostatics, etc.

Generally, the term *"in silico"* refers to the creation in a computer memory, i.e., on a silicon or other like chip. Stated otherwise *"in silico"* means "virtual". When used herein the term *"in silico"* is intended to refer to screening methods based on the use of computer models rather than *in vitro* or *in vivo* experiments.

Accordingly, the present invention also provides a computer-assisted method of identifying a compound that interacts with IR and/or IGF-1R, which method comprises fitting the structure of: (i) the Site 1 binding site of IR, the structure being defined by a subset of the atomic coordinates shown in Appendix I; and/or (ii) portions of the N- and C-terminal regions of the IR α-chain, which are in complex with insulin, the structure being defined by a subset of the atomic coordinates shown in Appendix I, to the structure of a candidate compound.

Also provided by the present invention is a computer-assisted method for identifying a molecule able to interact with IR and/or IGF-1R using a programmed computer comprising a processor, which method comprises the steps of: (a) generating, using computer methods, a set of atomic coordinates of a structure that possesses energetically favourable interactions with the atomic coordinates of: (i) the Site 1 binding site of IR, the structure being defined by a subset of the atomic coordinates shown in Appendix I; and/or (ii) portions of the N- and C-terminal regions of the IR α-chain, which are in complex with insulin, the structure being defined by a subset of the atomic coordinates shown in Appendix I, which coordinates are entered into the computer thereby generating a criteria data set; (b) comparing, using the processor, the criteria data set to a computer database of chemical structures; (c) selecting from the database, using computer methods, chemical structures which are complementary or similar to a region of the criteria data set; and optionally, (d) outputting, to an output device, the selected chemical structures which are complementary to or similar to a region of the criteria data set.

The present invention further provides a computer-assisted method for identifying mimetics of IR, insulin and/or IGF-1R using a programmed computer comprising a processor, the method comprising the steps of: (a) generating a criteria data set from a set of atomic coordinates of: (i) the Site 1 binding site of IR, the structure being defined by a subset of the atomic coordinates shown in Appendix I; (ii) portions of the N- and C-terminal regions of the IR α-chain, which are in complex with insulin, the structure being defined by a subset of the atomic coordinates shown in Appendix I; (iii) insulin, the structure being defined by a subset of the atomic coordinates shown in Appendix I; and/or (iv) the Site 1 binding site of insulin or portions thereof, the structure being defined by a subset of the atomic coordinates shown in Appendix I, which coordinates are entered into the computer; (b) (i) comparing, using the processor, the criteria data set to a computer database of chemical structures stored in a computer data storage system and selecting from the database, using computer methods, chemical structures having a region that is structurally similar to the criteria data set; or (ii) constructing, using computer methods, a model of a chemical structure having a region that is structurally similar to the criteria data set; and, optionally, (c) outputting to an output device: (i) the selected chemical structures from step (b)(i) having a region similar to the criteria data set; or (ii) the constructed model from step (b)(ii).

The present invention further provides a method for evaluating the ability of a compound to interact with IR and/or IGF-1R, the method comprising the steps of: (a) employing computational means to perform: (i) a fitting operation between the compound and the binding surface of a computer model of the Site 1 binding site for insulin on IR; and/or (ii) a superimposing operation between the compound and insulin, the Site 1 binding site of insulin, or a portion thereof, using atomic coordinates wherein the root mean square deviation between the atomic coordinates and a subset of atomic coordinates of Appendix I or a subset of atomic coordinates of one or more thereof at least representing the N-terminal region of the IR α-chain, the C-terminal region of IR α-chain, insulin, the Site 1 binding site of insulin, or a portion of the Site 1 binding site of insulin, is not more than 1.5 Å; and (b) analysing the results of the fitting operation and/or superimposing operation to quantify the association between the compound and the binding surface model.

The present invention also provides a method of using molecular replacement to obtain structural information about a molecule or a molecular complex of an unknown structure, comprising the steps of: (i) generating an X-ray diffraction pattern of the crystallized molecule or molecular complex; and (ii) applying the atomic coordinates of Appendix I, or a subset of atomic coordinates thereof at least representing the N-terminal region of the IR α-chain, the C-terminal region of IR α-chain, insulin, mimetics thereof, derivatives thereof, or portions thereof, to the X-ray diffraction pattern to generate a three-dimensional electron density map of at least a region of the molecule or molecular complex whose structure is unknown.

Also described herein are compounds that bind to IR and/or IGF-1R designed, redesigned or modified using the methods or processes of the present invention. Preferably, such compounds have an affinity (K_{d}) for IR and/or IGF-1R of less than 10⁻⁵ M. In a particularly preferred embodiment, the compounds binds to the Site 1 binding site of IR and/or to the Site 1 binding site of IGF-1R.

In view of the high sequence homology between the ligands insulin, IGF-I and IGF-II (Adams *et al.* 2000), also described are compounds that bind to insulin, IGF-I or IGF-II, said compounds being designed, redesigned or modified using the methods or processes of the present invention.

Further described is a composition comprising a compound as described herein, a peptide or mimetic as described herein, and optionally an acceptable carrier or diluent, more preferably a pharmaceutically acceptable carrier or diluent.

Further described is a method for preventing or treating a disease associated with aberrant IR and/or IGF-1R functioning and/or signalling, the method comprising administering to a subject in need thereof a composition, compound, peptide or mimetic as described herein.

Also described is use of a composition, a compound, a peptide or mimetic as described herein in the manufacture of a medicament for treating a disease or disorder in a subject associated with aberrant IR and/or IGF-1R functioning and/or signalling.

Examples of diseases associated with aberrant IR and/or IGF-1R functioning and/or signalling include, but are not limited to, obesity, type I and type II diabetes, cardiovascular disease, osteoporosis, dementia and cancer.

Also described are manufacturing steps such as incorporating the compound, such as a peptide, into a pharmaceutical composition in the manufacture of a medicament.

Throughout this specification, preferred aspects and embodiments apply, as appropriate, separately, or in combination, to other aspects and embodiments, mutatis mutandis, whether or not explicitly stated as such.

The present invention will now be described further with reference to the following examples, which are illustrative only and non-limiting.

### BRIEF DESCRIPTION OF THE DRAWINGS

Various embodiments of the invention will be described with reference to the following drawings. Some of the figures contain colour representations or entities. Coloured versions of the figures are available from the applicant upon request or from an appropriate patent office.
**Figure 1****:** Shows: (A) a model structure of the insulin monomer in which the A-chain is shown in brown, the B-chain is shown in grey and the three disulphide bonds joining the A-chain to the B-chain are shown in green; (B) a graphical representation of the domain profile of the IR, which, from the N-terminus to the C-terminus shows: a leucine-rich repeat domain ("L1") shown in cyan; a cysteine-rich region ("CR") shown in brown; another leucine-rich repeat domain ("L2") shown in red; three fibronectin Type III domains ("FnIII-1; FnIII-2; and FnIII-3") shown in green, gold and blue, respectively; trans- and juxtamembrane segments ("TM" and "JM") shown in grey; a tyrosine kinase domain ("TK") shown in black and a C-terminal tail shown in grey. The FnIII-2 domain contains an insert domain ("ID") shown in grey, which contains the α-chain/β-chain cleavage site. An amphipathic helical segment ("αCT") is positioned at the C-terminal region of the IR α-chain, shown as a magenta circle. Disulphide bonds are shown as green braces above and below the domain profile; (C) a model structure of the IR dimer showing how the IR dimer would sit on the membrane surface. Each monomer of the dimer includes the ectodomain of IR with one monomer shown in ribbon form and the other shown as a space-filled model. The domains of the monomers are coloured as in (B); and (D) a model structure of the insulin monomer modelling the proposed conformational changes to the C-terminal B-chain residues 20 to 30 upon binding to the IR (Hua et al., 1991).

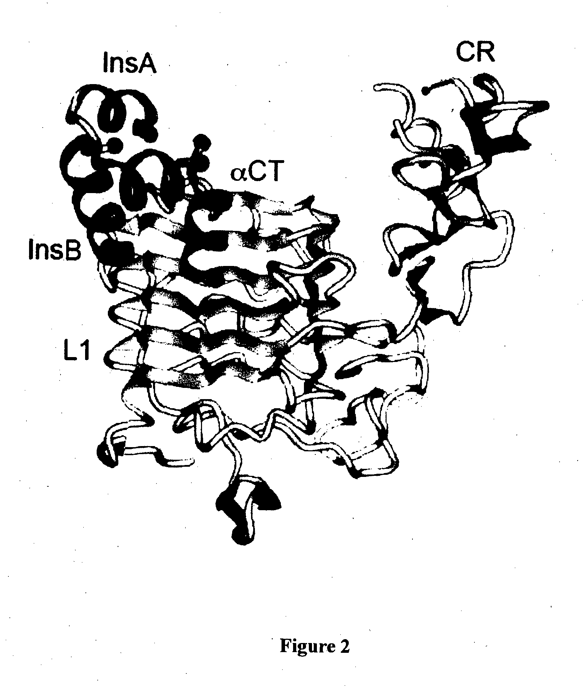

**Figure 2****:** Shows the crystal structure of insulin in complex with the N- and C-terminal regions of the IR α-chain. The structure is shown in ribbon form with the N-terminal region of the IR α-chain shown with a grey backbone, cyan coloured β-sheets in the L1 domain and gold coloured β-sheets in the CR region. The C-terminal region of the IR α-chain, including the αCT segment, is shown as a magenta coloured α-helix. The insulin monomer is shown with a grey backbone. The α-helices of the A-chain of the insulin monomer are coloured gold and the α-helix of the B-chain is coloured black. The N- and C-termini of the A- and B-chain of the insulin monomer are coloured blue and red, respectively.

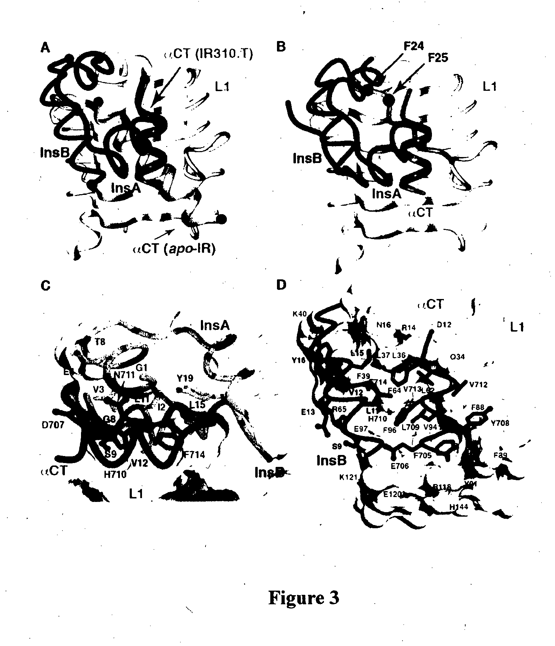

**Figure 3****:** Shows: (A) a model of the crystal structure of insulin in complex with the N- and C-terminal regions of the IR α-chain. The model shows the interaction between insulin and the L1 domain from the N-terminal region of the IR α-chain and the αCT segment from the C-terminal region of the IR α-chain. Secondary structural elements from the L1 domain are coloured cyan with loops shown in white. The A-chain from insulin monomer is coloured brown and the B-chain is coloured black. The αCT segment from the crystal structure is coloured magenta. An αCT segment from a crystal structure of the apo form of the IR, shown in yellow, has been superimposed over the model structure. The N- and C-termini of the A- and B-chain of the insulin monomer and the αCT segments are coloured blue and red, respectively; (B) Insulin B-chain residues Phe24 (F24) and Phe25 (F25) are shown in the model structure from (A), without the apo αCT segment; (C) a model of the crystal structure of insulin in complex with the N- and C-terminal regions of the IR α-chain showing the interface between insulin (A-chain shown in brown and B-chain shown in black), the αCT segment (shown in magenta) and the L1 domain (shown in cyan). Selected residues are shown in stick form. The residues from the insulin B-chain are shown with green carbon atoms. Oxygen atoms are coloured red and nitrogen atoms are coloured blue; (D) a model of the crystal structure of insulin in complex with the N- and C-terminal regions of the IR α-chain showing the interface between insulin (A-chain not shown and B-chain shown in black), the αCT segment (shown in magenta) and the L1 domain (shown in cyan). Selected residues from the L1 domain, the αCT segment and the insulin B-chain are shown with carbon atoms coloured cyan, magenta, green. Oxygen atoms are coloured red and nitrogen atoms are coloured blue.

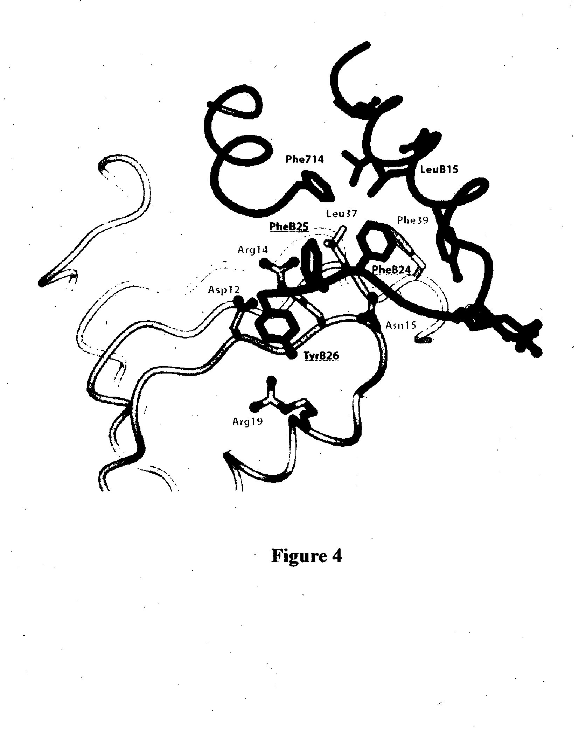

**Figure 4****:** Shows a model of the L1 domain (coloured in cyan), the insulin B-chain (coloured in black) and the αCT segment (coloured in magenta) from the crystal structure of insulin in complex with the N- and C-terminal regions of the IR α-chain. The L1 domain, the αCT segment and the insulin B-chain are shown with carbon atoms coloured cyan, magenta, green respectively. Oxygen atoms are coloured red and nitrogen atoms are coloured blue.

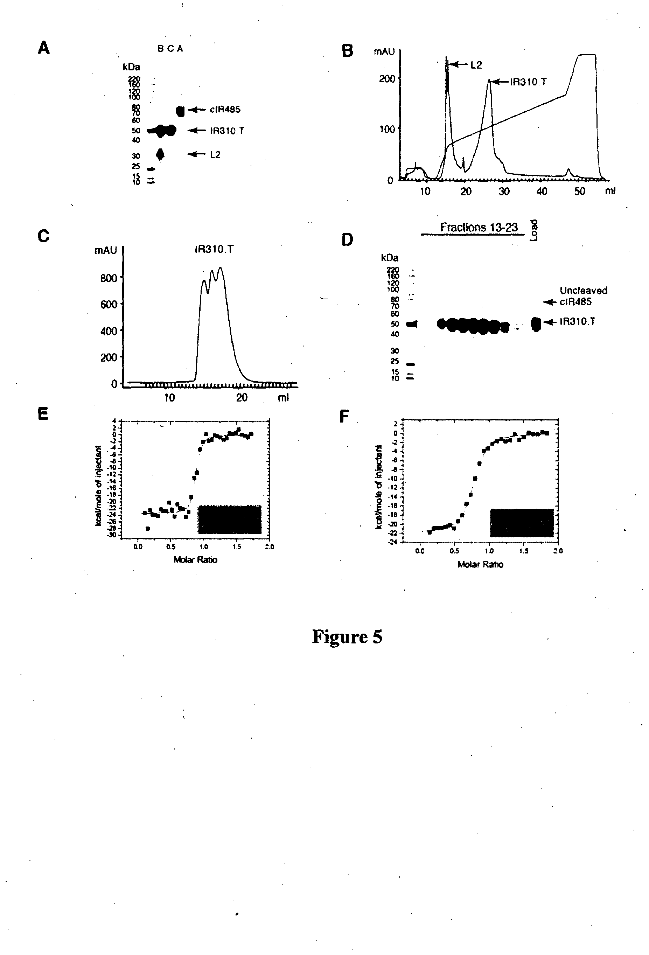

**Figure 5****:** Shows: (A) an SDS-PAGE gel showing bands that correspond to purified cIR485 (lane A), IR310.T (lane B) and L2 domain (lane C); (B) an ion exchange chromatography elution profile showing the elution of fractions corresponding to the L2 domain and IR310.T (in red) against a volume buffer gradient (in blue); (C) a size-exclusion chromatography elution profile showing separation of the IR310.T fraction from (B); (D) an SDS-PAGE gel showing bands that correspond to fractions of IR310.T collected from (C); (E) ITC curves for the titration of zinc-free human insulin against IR310.T precomplexed with a 10 fold molar ratio of αCT⁷⁰⁴⁻⁷¹⁹; and (F) ITC curves for the titration of zinc-free insulin against Fab 83-7 bound IR310.T precomplexed with a 10 fold molar ratio of αCT⁷⁰⁴⁻⁷¹⁹.

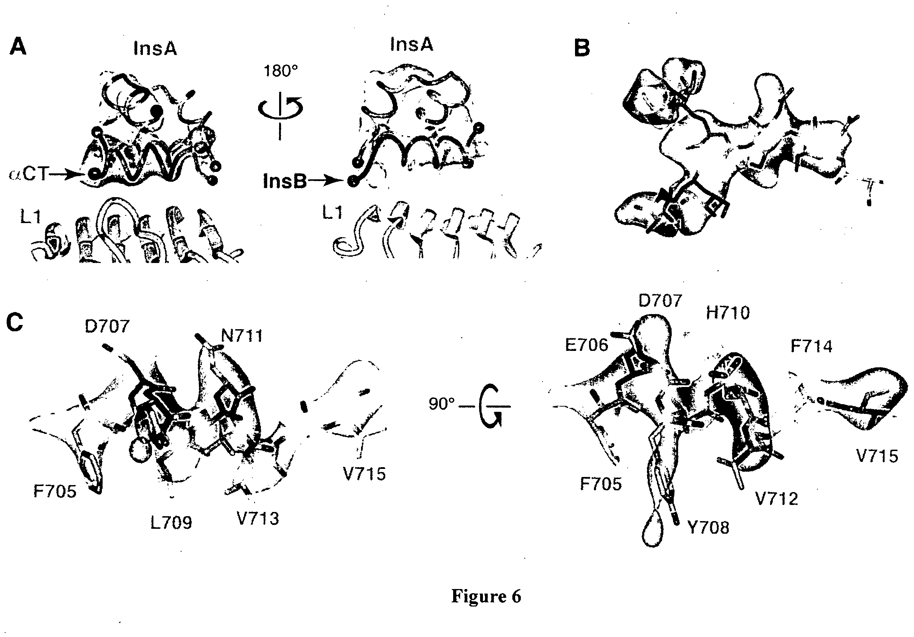

**Figure 6****:** Shows: (A) a model of the L1 domain, the aCT segment, insulin chain-A and insulin chain-B from the crystal structure of insulin in complex with the N- and C-terminal regions of the IR α-chain. The electron difference density maps corresponding to the location of the αCT segment (in magenta), insulin chain-A and insulin chain-B (in yellow) are also shown; (B) a model of Asn11 (coloured in cyan) with an attached glycan (coloured in green) from the crystal structure of the uncomplexed IR485 (Lou et al., 2006) fitted to the difference electron density map as described herein. Oxygen atoms are coloured red and nitrogen atoms are coloured blue; (C) a model of a portion of αCT segment (coloured magenta) fitted to a difference electron density map shown in magenta. Oxygen atoms are coloured red and nitrogen atoms are coloured blue.

### KEY TO SEQUENCE LISTING

SEQ ID NO: 1-Amino acid sequence of mature human insulin receptor (isoform A).
SEQ ID NO: 2-Amino acid sequence of mature human insulin receptor (isoform B).
SEQ ID NO: 3-Amino acid sequence of mature Type 1 insulin-like growth factor receptor.
SEQ ID NO: 4-Amino acid sequence of insulin A-chain.
SEQ ID NO: 5-Amino acid sequence of insulin B-chain.
SEQ ID NO: 6-Amino acid sequence of mature insulin-like growth factor I ("IGF-I").
SEQ ID NO: 7-Amino acid sequence of mature insulin-like growth factor II ("IGF-II").
SEQ ID NO: 8-Amino acid sequence of IR310.T.
SEQ ID NO: 9-Amino acid sequence of the IR α-chain C-terminal peptide from the IR-A isoform ("αCT").
SEQ ID NO: 10-Amino acid sequence of the IR α-chain C-terminal peptide from the IR-B isoform.
SEQ ID NO: 11-Amino acid sequence of MAb 83-7 IgG1 heavy chain.
SEQ ID NO: 12-Amino acid sequence of MAb 83-7 kappa light chain.
SEQ ID NO: 13-Amino acid sequence of Fab 83-7 kappa light chain.
SEQ ID NO: 14-Amino acid sequence of Fab 83-7 IgG1 heavy chain.
SEQ ID NO: 15-Amino acid sequence of cIR485.

### DETAILED DESCRIPTION

Unless defined otherwise, all technical and scientific terms used herein have the same meaning as commonly understood by one of ordinary skill in the art (e.g. in molecular biology, biochemistry, structural biology, and/or computational biology). Standard techniques are used for molecular and biochemical methods (see generally, Sambrook *et al.,* 2001, and Ausubel *et al.,* 1999, and chemical methods.

In the present specification and claims, the word 'comprising' and its derivatives including 'comprises' and 'comprise' include each of the stated integers but does not exclude the inclusion of one or more further integers.

Reference throughout this specification to 'one embodiment' or 'an embodiment' means that a particular feature, structure, or characteristic described in connection with the embodiment is included in at least one embodiment of the present invention. Thus, the appearance of the phrases 'in one embodiment' or 'in an embodiment' in various places throughout this specification are not necessarily all referring to the same embodiment. Furthermore, the particular features, structures, or characteristics may be combined in any suitable manner in one or more combinations.

As used herein the term "homologue" means a protein having at least 30% amino acid sequence identity with IR, insulin, IGF-1R, and/or portions thereof. Preferably, the percentage identity is 40 or 50%, more preferably 60 or 70% and most preferably 80 or 90%. A 95% or above identity is most particularly preferred such as 95%, 96%, 97%, 98%, 99% or 100%.

As used herein the term "higher order complex" is used to describe a multimer of the ternary complex of insulin in complex with the IR and the αCT peptide. A higher order complex also includes a co-complex of one or more components of the insulin/IR/αCT complex and another molecule. Various higher order complexes may have different signalling properties.

As used herein the term "derivatives" means IR/insulin/αCT peptide polypeptide complexes that display the biological activity of the wild-type IR/insulin/αCT peptide interactions, characterised by the replacement of at least one amino acid from the wild-type sequence or the modification of one or more of the naturally-occurring amino acids.

### Crystals and the crystal structure of insulin in complex with the N- and C-terminal regions of the IR α-chain

The present invention provides a crystal comprising an N-terminal region of the IR α-chain based on cIR485 (a "cleavable" construct consisting of the first 3 N-terminal domains of the IR α-chain; SEQ ID NO: 15) in complex with Fab 83-7 (SEQ ID NOs 13 and 14), a C-terminal region of the IR α-chain based on the IR classical αCT peptide ("αCT⁷⁰⁴⁻⁷¹⁹"; residues 704-719; Genscript; SEQ ID NO: 9) and human insulin (Sigma-Aldrich; SEQ ID NOs 4 and 5; see Examples).

The crystal structure of human insulin complexed IR310.T/83-7/αCT⁷⁰⁴⁻⁷¹⁹ is presented in Figure 2. Insulin is seen to interact with both the central β-sheet of the L1 domain and with the αCT peptide. The details of these interactions are presented in Figure 3. The B-helix of insulin lies along the C-terminal edge of L1-β₂, parallel and adjacent to the helical portion of αCT (Figure 3A). Although, the insulin A-chain has no interactions with the L1 domain, extensive interactions are observed with the αCT peptide. Critically, the helical component (residues 705-714 in all structures) of the αCT peptide occupies volume that would otherwise be occupied by the insulin B-chain C-terminal segment B26-B30 if the hormone remained in its free conformation (Figure 3B). No electron density was discerned for insulin B1-B6.

In the human insulin-complexed IR310.T structure, weak electron density extended beyond insulin GluB21 in a direction anti-parallel to the first strand of L1-β2, suggestive of partial ordering of a folded-out B-chain C-terminal strand.

The interface between insulin, the αCT segment and the L1-β₂ surface involves well-defined elements of secondary structure, allowing ready discernment in the electron density maps of the overall direction of the constituent residue side chains. The refined model thus allows key residue-specific interactions at the interface to be discerned with confidence, even though a determination of detailed side-chain conformation is precluded. The major interaction between αCT and insulin is seen to be mediated by the side chains of (a) His710, which is directed into a pocket formed by insulin residues ValA3, GlyB8, SerB9 and ValB12, and (b) Phe714, which is directed into the now exposed hydrophobic crevice of insulin lined by residues GlyA1, IleA2, TyrA19, LeuB11, ValB12 and LeuB15 (Figure 3C). Asn711 of the αCT peptide is positioned to interact with GlyA1, ValA3 and GluA4. The interaction between the L1 domain and the insulin B-chain helix is not extensive with the side chain of ValB12 being positioned between Phe39, Phe64 and Arg65, while the side chain of TyrB16 is positioned to interact with Phe39 and Lys40 (Figure 3D). The non-polar face of the amphipathic αCT helix (residues Phe705, Tyr708, Leu709, Val712 and Val 713) engages the non-polar surface of L1-β₂ (Leu36, Leu37, Leu62, Phe64, Phe88, Phe89, Val94 and Phe96). The αCT helix appears to be stabilized further by "clamps" formed by Arg118 and Arg14 (Figure 3D), with the conformation of Arg118 stabilized by interactions with Tyr91, Glu120, His144 and Phe705 and the conformation of Arg14 stabilized by interactions with Asp12 and the insulin A-chain C-terminus.

Initially, no interpretable density was discerned for insulin residues B20-B26. Final resolution of residues B20-B26 required careful reprocessing of the X-ray data and re-refinement of the model. Ultimately, B20-B24 could be built in a near native-like conformation, with the side chain of residue B24 directed into a hydrophobic pocket formed by the side chains of residues Phe714, Phe39, Leu37 and LeuB15 (Figure 4). PheB25 and TyrB26 extended in a non-native-like fashion beyond PheB24 with the side chain of residue TyrB25 directed towards the aCT peptide in the vicinity of residue Val715 and the side chain of residue TyrB26 directed towards the L1 domain residues Arg19 and Asp12.

As used herein, the term "crystal" means a structure (such as a three-dimensional (3D) solid aggregate) in which the plane faces intersect at definite angles and in which there is a regular structure (such as an internal structure) of the constituent chemical species. The term "crystal" refers in particular to a solid physical crystal form such as an experimentally prepared crystal.

Crystals according to the invention may be prepared using any IR ectodomain, i.e., the IR polypeptide containing the extracellular domain, including the N- and C-terminal regions, and lacking the transmembrane domain and the intracellular tyrosine kinase domain. Typically, the extracellular domain comprises residues 1 to 917 (mature receptor numbering) of human IR, or the equivalent thereof together with any post-translational modifications of these residues such as N- or O-linked glycosylation.

In a preferred embodiment the IR polypeptide is human IR (SEQ ID NOs: 1 or 2). However, the IR polypeptide may also be obtained from other species, such as other mammalian, vertebrate or invertebrate species.

Crystals may be constructed with wild-type IR polypeptide ectodomain sequences or variants thereof, including allelic variants and naturally occurring mutations as well as genetically engineered variants. Typically, variants have at least 95 or 98% sequence identity with a corresponding wild-type IR ectodomain polypeptide.

Crystals according to the invention may be prepared using any insulin, i.e., insulin polypeptides containing the insulin A-chain and insulin B-chain. Typically, the insulin will be in a monomeric form.

In a preferred embodiment the insulin polypeptides encode human insulin (SEQ ID NOs: 4 and 5).

Crystals may be constructed with wild-type insulin polypeptide sequences or variants thereof, including allelic variants and naturally occurring mutations as well as genetically engineered variants. Typically, variants have at least 95 or 98% sequence identity with a corresponding wild-type insulin polypeptide.

Optionally, the crystal of the IR ectodomain in complex with insulin may comprise one or more compounds which bind to the ectodomain and/or insulin, or otherwise are soaked into the crystal or co-crystallised with the IR ectodomain and/or insulin. Such compounds include ligands or small molecules, which may be candidate pharmaceutical agents intended to modulate the interaction between IR and insulin or IR and biological targets. The crystal of the IR ectodomain in complex with insulin may also be a molecular complex with other receptors of the IGF receptor family such as IGF-1R (SEQ ID NO: 3). The complex may also comprise additional molecules such as the ligands to these receptors, e.g., IGF-I (SEQ ID NO: 6), IGF-II (SEQ ID NO: 7), etc.

The production of IR ectodomain crystals is described below.

In a preferred embodiment, a crystal of the IR ectodomain in complex with insulin of the invention comprises the N- and C-terminal regions of the IR α-chain in complex with insulin having the atomic coordinates set forth in Appendix I. As used herein, the term "atomic coordinates" or "set of coordinates" refers to a set of values which define the position of one or more atoms with reference to a system of axes. It will be understood by those skilled in the art that the atomic coordinates may be varied, without affecting significantly the accuracy of models derived therefrom. Thus, although the invention provides a very precise definition of a preferred atomic structure, it will be understood that minor variations are envisaged and the claims are intended to encompass such variations.

It will be understood that any reference herein to the atomic coordinates or subset of the atomic coordinates shown in Appendix I shall include, unless specified otherwise, atomic coordinates having a root mean square deviation of backbone atoms of not more than 1.5 Å, preferably not more than 1 Å, when superimposed on the corresponding backbone atoms described by the atomic coordinates shown in Appendix I.

The following defines what is intended by the term "root mean square deviation ('RMSD')" between two data sets. For each element in the first data set, its deviation from the corresponding item in the second data set is computed. The squared deviation is the square of that deviation, and the mean squared deviation is the mean of all these squared deviations. The root mean square deviation is the square root of the mean squared deviation.

Preferred variants are those in which the RMSD of the x, y and z coordinates for all backbone atoms other than hydrogen is less than 1.5 Å (preferably less than 1 Å, 0.7 Å or less than 0.3 Å) compared with the coordinates given in Appendix I. It will be readily appreciated by those skilled in the art that a 3D rigid body rotation and/or translation of the atomic coordinates does not alter the structure of the molecule concerned.

In a highly preferred embodiment, the crystal has the atomic coordinates as shown in Appendix I.

The present invention also provides a crystal structure of the Site 1 binding site of IR polypeptide comprising the N- and C-terminal regions of the IR α-chain, or regions thereof.

The atomic coordinates obtained experimentally for: amino acids 5 to 310 (the "N-terminal region of the IR α-chain") and amino acids 705 to 714 (the "C-terminal region of the IR α-chain") of human IR-A (mature receptor numbering; SEQ ID NO: 1); amino acids 1 to 20 of the human insulin A-chain; and amino acids 7 to 26 of the human insulin B-chain (SEQ ID NOs: 4 and 5) are shown in Appendix I. However, a person skilled in the art will appreciate that a set of atomic coordinates determined by X-ray crystallography is not without standard error. Accordingly, any set of structure coordinates for an IR polypeptide comprising the N- and C-terminal regions of the IR α-chain in complex with human insulin that has a root mean square deviation of protein backbone atoms of less than 0.75 Å when superimposed (using backbone atoms) on the atomic coordinates listed in Appendix I shall be considered identical.

The present invention also comprises the atomic coordinates of the N- and C-terminal regions of the IR α-chain in complex with insulin that substantially conforms to the atomic coordinates listed in Appendix I.

A structure that "substantially conforms" to a given set of atomic coordinates is a structure wherein at least about 50% of such structure has an RMSD of less than about 1.5 Å for the backbone atoms in secondary structure elements in each domain, and more preferably, less than about 1.3 Å for the backbone atoms in secondary structure elements in each domain, and, in increasing preference, less than about 1.0 Å, less than about 0.7 Å, less than about 0.5 Å, and most preferably, less than about 0.3 Å for the backbone atoms in secondary structure elements in each domain.

In a more preferred embodiment, a structure that substantially conforms to a given set of atomic coordinates is a structure wherein at least about 75% of such structure has the recited RMSD value, and more preferably, at least about 90% of such structure has the recited RMSD value, and most preferably, about 100% of such structure has the recited RMSD value.

In an even more preferred embodiment, the above definition of "substantially conforms" can be extended to include atoms of amino acid side chains. As used herein, the phrase "common amino acid side chains" refers to amino acid side chains that are common to both the structure which substantially conforms to a given set of atomic coordinates and the structure that is actually represented by such atomic coordinates.

As used herein, the term "IR ectodomain" refers to the extracellular domain of IR lacking the transmembrane domain and the intracellular tyrosine kinase domain of IR, typically comprising residues 1 to 917 (mature IR-A receptor numbering) of human IR, or the equivalent thereof, together with any post-translational modifications of these residues such as N- or O-linked glycosylation.

As used herein, the term "low affinity binding site" for IR means the regions of IR involved in forming the nanomolar affinity binding site (also known as "Site 1") of IR for insulin, comprising the N- and C-terminal regions of the IR α-chain including one or both of the L1 domain of IR and the CR domain of IR. Insulin binding to the low affinity binding site of IR induces formation of the high affinity insulin binding site of IR and subsequent signal transduction.

As used herein, the term "classical α-chain C-terminal peptide", or "aCT", refers in IR to a region of the C-terminal α-chain of IR previously described in the literature as being important for insulin binding (Kurose et al., 1994; Kristensen et al, 2002), and comprising amino acids 704-719 (mature IR-A receptor numbering) as given in SEQ ID NO: 9.

As used herein, the term "leucine-rich repeat domain 1" or "L1 domain" refers in IR to a leucine-rich domain comprising amino acids 1-156 of mature human IR (SEQ ID NO: 1). The L1 domain of IR comprises a central β-sheet, which comprises amino acids selected from 10-15, 32-37, 60-65, 88-97, 116-121 and 142-147 of mature human IR (SEQ ID NO: 1).

As used herein, the term "leucine-rich repeat domain 2" or "L2" domain refers in IR to a leucine-rich domain comprising amino acids 310-469 of mature human IR (SEQ ID NO: 1).

As used herein, the term "loop in the fourth leucine-rich repeat (LRR) rung of the L1 domain", or variations thereof, refers in IR to a leucine-rich domain comprising amino acids 85-91 of mature human IR (SEQ ID NO: 1).

As used herein, the term "cysteine-rich domain" or "CR" domain refers in IR to a cysteine-rich domain comprising amino acids 157-309 of mature human IR (SEQ ID NO: 1). The CR domain contains many different modules. As used herein, the term "module 6 of the CR domain" refers in IR to amino acids 256-286 of mature human IR (SEQ ID NO: 1).

### Manipulation of the atomic coordinates

It will be appreciated that a set of atomic coordinates for one or more polypeptides is a relative set of points that define a shape in three dimensions. Thus, it is possible that an entirely different set of coordinates could define a similar or identical shape. Moreover, slight variations in the individual coordinates will have little effect on overall shape.

The variations in coordinates may be generated due to mathematical manipulations of the atomic coordinates. For example, the atomic coordinates set forth in Appendix I could be manipulated by crystallographic permutations of the atomic coordinates, fractionalisation of the atomic coordinates, integer additions or subtractions to sets of the structure coordinates, inversion of the atomic coordinates, or any combination thereof.

Alternatively, modification in the crystal structure due to mutations, additions, substitutions, and/or deletions of amino acids, or other changes in any of the components that make up the crystal could also account for variations in atomic coordinates.

Various computational analyses are used to determine whether a molecular complex or a portion thereof is sufficiently similar to all or parts of the structure of the extracellular domain of IR in complex with insulin described above. Such analyses may be carried out in current software applications, such as the Sequoia program (Bruns et al., 1999).

The Molecular Similarity program permits comparisons between different structures, different conformations of the same structure, and different parts of the same structure.

Comparisons typically involve calculation of the optimum translations and rotations required such that the root mean square deviation of the fit over the specified pairs of equivalent atoms is an absolute minimum. This number is given in Angstroms ("Å").

Accordingly, atomic coordinates of an IR ectodomain comprising the Site 1 binding site in complex with insulin of the present invention include atomic coordinates related to the atomic coordinates listed in Appendix I by whole body translations and/or rotations. Accordingly, RMSD values listed above assume that at least the backbone atoms of the structures are optimally superimposed which may require translation and/or rotation to achieve the required optimal fit from which to calculate the RMSD value.

A three dimensional structure of an IR ectodomain polypeptide or a region thereof and/or a three dimensional structure of an insulin polypeptide or a region thereof which substantially conforms to a specified set of atomic coordinates can be modelled by a suitable modelling computer program such as MODELLER (Sali & Blundell, 1993), using information, for example, derived from the following data: (1) the amino acid sequence of the human IR ectodomain polypeptide and/or the amino acid sequence of the human insulin polypeptide; (2) the amino acid sequence of the related portion(s) of the protein represented by the specified set of atomic coordinates having a three dimensional configuration; and (3) the atomic coordinates of the specified three dimensional configuration. A three dimensional structure of an IR ectodomain polypeptide and/or a three dimensional structure of an insulin polypeptide which substantially conforms to a specified set of atomic coordinates can also be calculated by a method such as molecular replacement, which is described in detail below.

Atomic coordinates are typically loaded onto a machine-readable medium for subsequent computational manipulation. Thus models and/or atomic coordinates are advantageously stored on machine-readable media, such as magnetic or optical media and random-access or read-only memory, including tapes, diskettes, hard disks, CD-ROMs and DVDs, flash memory cards or chips, servers and the internet. The machine is typically a computer.

The atomic coordinates may be used in a computer to generate a representation, e.g. an image of the three-dimensional structure of the IR ectodomain in complex with insulin which can be displayed by the computer and/or represented in an electronic file.

The atomic coordinates and models derived therefrom may also be used for a variety of purposes such as drug discovery, biological reagent (binding protein) selection and X-ray crystallographic analysis of other protein crystals.

### Molecular replacement

The structure coordinates of IR comprising the N- and C-terminal regions of the α-chain in complex with insulin, such as those set forth in Appendix I, or a subset thereof, can also be used for determining the three-dimensional structure of a molecular complex which contains at least the N- and/or C-terminal regions of the α-chain of IR. In particular, structural information about another crystallised molecular complex may be obtained. This may be achieved by any of a number of well-known techniques, including molecular replacement.

Methods of molecular replacement are generally known by those of skill in the art (generally described in Brunger, 1997; Navaza & Saludjian, 1997; Tong & Rossmann, 1997; Bentley, 1997; Lattman, 1985; Rossmann, 1972; McCoy, 2007).

Generally, molecular replacement involves the following steps. X-ray diffraction data are collected from the crystal of a crystallised target structure. The X-ray diffraction data are transformed to calculate a Patterson function. The Patterson function of the crystallised target structure is compared with a Patterson function calculated from a known structure (referred to herein as a search structure). The Patterson function of the search structure is rotated on the target structure Patterson function to determine the correct orientation of the search structure in the crystal. A translation function is then calculated to determine the location of the search structure with respect to the crystal axes. Once the search structure has been correctly positioned in the unit cell, initial phases for the experimental data can be calculated. These phases are necessary for calculation of an electron density map from which structural differences can be observed and for refinement of the structure. Preferably, the structural features (e.g., amino acid sequence, conserved di-sulphide bonds, and beta-strands or beta-sheets) of the search molecule are related to the crystallised target structure.

The electron density map can, in turn, be subjected to any well-known model building and structure refinement techniques to provide a final, accurate structure of the unknown (i.e., target) crystallised molecular complex (e.g. see Jones *et al.,* 1991; Brünger *et al.,* 1998).

Obtaining accurate values for the phases, by methods other than molecular replacement, is a time-consuming process that involves iterative cycles of approximations and refinements and greatly hinders the solution of crystal structures. However, when the crystal structure of a protein containing at least a homologous portion has been solved, the phases from the known structure provide a satisfactory starting estimate of the phases for the unknown structure.

By using molecular replacement, all or part of the structure coordinates of IR comprising the N- and C-terminal regions of the IR α-chain in complex with insulin provided herein (and set forth in Appendix I) can be used to determine the structure of a crystallised molecular complex whose structure is unknown more rapidly and more efficiently than attempting to determine such information *ab initio.* This method is especially useful in determining the structure of IR.

The structure of any portion of any crystallised molecular complex that is sufficiently homologous to any portion of the extracellular domain of IR and/or IGF-1R can be solved by this method.

Such structure coordinates are also particularly useful to solve the structure of crystals of IR co-complexed with a variety of molecules, such as chemical entities. For example, this approach enables the determination of the optimal sites for the interaction between chemical entities, and the interaction of candidate IR and/or IGF-1R agonists or antagonists.

All of the complexes referred to above may be studied using well-known X-ray diffraction techniques and may be refined against 1.5-3.5 Å resolution X-ray data to an R value of about 0.25 or less using computer software, such as X-PLOR (Yale University, distributed by Molecular Simulations, Inc.; see Brünger, 1996). This information may thus be used to optimize known IR agonist/antagonists, such as anti-IR antibodies, and more importantly, to design new or improved IR agonists/antagonists.

### Target sites for compound identification, design or screening

The three-dimensional structure of the Site 1 binding site of IR in complex with insulin provided by the present invention (Appendix I) can be used to identify potential target binding sites in the Site 1 binding site of IR and/or IGF-1R (i.e., to identify those regions of the Site 1 binding site of IR and/or IGF-1R involved in and important to the binding of insulin, IGF-I and/or IGF-II and subsequent signal transduction) as well as in methods for identifying or designing compounds which interact with the low affinity binding site of IR and/or IGF-1R, e.g., potential modulators of IR and/or IGF-1R.

The three-dimensional structure of IR in complex with insulin provided by the present invention (Appendix I) can be used to identify potential target binding sites in the L1 domain of IR and/or IGF-1R important for binding to insulin and in the C-terminal region of the IR and/or IGF-1R α-chain important for binding to insulin as well as in methods for identifying or designing compounds which interact with the L1 domain of IR and/or IGF-1R and/or with the C-terminal region of the IR and/or IGF-1R α-chain in a manner similar to insulin in complex with the L1 domain and the C-terminal region of the IR α-chain, e.g., potential modulators of IR and/or IGF-1R.

The low affinity binding site of IR is a region of IR ectodomain involved in insulin docking to the receptor. The preferred low affinity target binding site may comprise the C-terminal region of the IR α-chain and one or more regions from the L1 domain of the IR ectodomain. With regards to the L1 domain, the target binding site preferably comprises portions of the molecular surface of the central β-sheet of L1, preferably containing Asp12, Arg14, Leu 36, Leu 37, Leu62, Phe64, Val94, Phe96, Arg118, Glu120 and His144 and portions of the molecular surface of the second leucine-rich repeat (LRR), preferably containing Phe88, Phe89 and Tyr 91. Most preferably, the low affinity binding site contains both portions of the molecular surface of the central β-sheet of L1 and portions of the molecular surface of the second LRR as defined above.

Alternatively, the low affinity target binding site in IR may comprise one or more amino acids from amino acids 704 to 719 (encompassing the C-terminal region of the IR α-chain) plus one or more of the following amino acid sequences: (i) amino acids 1-156 and (ii) amino acids 157-310.

With regards to amino acids 1-156, the target binding site preferably comprises one or more amino acids selected from Asp12, Arg14, Asn15, Gln34, Leu36, Leu37, Leu62, Phe39, Pro43, Phe46, Leu62, Phe64, Leu87, Phe88, Phe89, Asn90, Val94, Phe96, Glu97, Arg118, Gle120 and His144.

With regards to amino acids 157-310, the target binding site preferably comprises one or more amino acids from the amino acid sequence 192-310, more preferably one or more amino acids from the sequence 227-303, yet more preferably one or more amino acids selected from the sequence 259-284.

In a preferred embodiment, van der Waals and/or hydrophobic interactions account for the major portion of the binding energy between a compound and a low affinity insulin binding site of IR.

The three-dimensional structure of the N- and C-terminal regions of the IR α-chain provided by the present invention can also be used to identify or more clearly elucidate potential target binding sites on IGF-1R ectodomain (i.e., to identify those regions, or at least more accurately elucidate those regions of IGF-1R ectodomain involved in and important to the binding of IGF and signal transduction) as well as in methods used for identifying or designing compounds which interact with potential target binding sites of IGF-1R ectodomain, e.g. potential modulators of IGF-1R.

Preferred target binding sites are those governing specificity, i.e., those regions of IGF-1R ectodomain involved in the initial nanomolar affinity binding of IGF (i.e., the initial binding of IGF to IGF-1R).

The low affinity binding site of IGF-1R is a region of IGF-1R ectodomain involved in IGF-I binding to the receptor. Preferred low affinity target binding sites comprise the C-terminal region of IGF-1R α-chain and one or more regions from the L1 domain and/or the CR domain of IGF-1R ectodomain. With regards to the L1 domain, the target binding site preferably comprises the central β-sheet of the L1 domain, and/or that part of the second LRR containing Ser35, and/or the loop in the fourth LRR rung of the L1 domain, or preferably all of these, as defined above. With regards the CR domain, the target binding site preferably comprises module 6 of the CR domain, as defined above.

In a preferred embodiment, van der Waals and/or hydrophobic interactions account for the major portion of the binding energy between a compound and a low affinity binding site of IGF-1R.

Additional preferred binding sites in the case of both IR and IGF-1R, particularly for biological macromolecules such as proteins or aptamers, are those that are devoid of glycosylation or devoid of steric hindrance from glycan covalently attached to the polypeptide at sites in the spatial vicinity.

### Design, selection, fitting and assessment of chemical entities that bind IR and/or IGF-1R

Using a variety of known modelling techniques, the crystal structure of the present invention can be used to produce a model for the low affinity binding site of IR and/or IGF-1R.

As used herein, the term "modelling" includes the quantitative and qualitative analysis of molecular structure and/or function based on atomic structural information and interaction models. The term "modelling" includes conventional numeric-based molecular dynamic and energy minimisation models, interactive computer graphic models, modified molecular mechanics models, distance geometry and other structure-based constraint models.

Molecular modelling techniques can be applied to the atomic coordinates of the low affinity binding site of IR in complex with insulin, or at least part of the C-terminal region of the α-chain of IR or insulin, or regions thereof to derive a range of 3D models and to investigate the structure of binding sites, such as the binding sites of monoclonal antibodies, nonimmunoglobulin binding proteins and inhibitory peptides.

These techniques may also be used to screen for or design small and large chemical entities which are capable of binding IR and modulating the ability of IR to interact with extracellular biological targets, such as insulin or members of the IGF receptor family e.g. which modulate the ability of IR to heterodimerise. The screen may employ a solid 3D screening system or a computational screening system.

Such modelling methods are to design or select chemical entities that possess stereochemical complementary to the low affininty binding site of IR and/or IGF-1R, to the regions of the L1 domain of IR and/or IGF-1R with which the C-terminal region of the α-chain of IR and/or IGF-1R interact, or to the regions of the L1 domain of IR and/or IGF-1R with which insulin or IGF interact. By "stereochemical complementarity" we mean that the compound or a portion thereof makes a sufficient number of energetically favourable contacts with the receptor as to have a net reduction of free energy on binding to the receptor.

Modelling method may also be used to design or select chemical entities that possess stereochemical similarity to the low affinity binding site surface of insulin, to the regions of the A-chain and B-chain of insulin that interact with the C-terminal region of the α-chain of IR or to the regions of the A-chain and B-chain of insulin that interact with the L1 domain of IR. By "stereochemical similarity" we mean that the compound or portion thereof makes about the same number of energetically favourable contacts with the receptor as insulin makes as determined by the crystal structure of insulin in complex with the IR α-chain set out by the coordinates shown in Appendix I.

Stereochemical complementarity is characteristic of a molecule that matches intra-site surface residues lining the groove of the receptor site as enumerated by the coordinates set out in Appendix I or a subset thereof. By "match" we mean that the identified portions interact with the surface residues, for example, via hydrogen bonding or by non-covalent Van der Waals and Coulomb interactions (with surface or residue) which promote desolvation of the molecule within the site, in such a way that retention of the molecule at the binding site is favoured energetically.

It is preferred that the stereochemical complementarity is such that the compound has a K_{d} for the receptor site of less than 10⁻⁴M, more preferably less than 10⁻⁵M and more preferably 10⁻⁶M. In a most preferred embodiment, the K_{d} value is less than 10⁻⁸M and more preferably less than 10⁻⁹M.

Chemical entities which are complementary to the shape and electrostatics or chemistry of the receptor site characterised by amino acids positioned at atomic coordinates set out in Appendix I will be able to bind to the receptor, and when the binding is sufficiently strong, substantially prohibit the interaction of the IR and/or IGF-1R ectodomain with biological target molecules such as insulin or IGF.

It will be appreciated that it is not necessary that the complementarity between chemical entities and the receptor site or similarity between the chemical entities and biological receptor target molecules such as insulin or IGF need extend over all residues of the receptor site or target molecule in order to inhibit or mimic binding of a molecule or complex that naturally interacts with IR and/or IGF-1R ectodomain.

A number of methods may be used to identify chemical entities possessing stereochemical complementarity to the low affinitybinding site of IR and/or IGF-1R, to the regions of the L1 domain of IR and/or IGF-1R with which the C-terminal region of the α-chain of IR and/or IGF-1R interact, or to the regions of the L1 domain of IR and/or IGF-1R with which insulin or IGF interact. For instance, the process may begin by visual inspection of the entire low affinity insulin binding site comprising the N- and C-terminal regions of the α-chain of IR, or the equivalent region in IGF-1R, on the computer screen based on the coordinates in Appendix I generated from the machine-readable storage medium. Alternatively, selected fragments or chemical entities may then be positioned in a variety of orientations, or docked, within the low affinity binding site of IR and/or IGF-1R, or within the L1 domain of IR and/or IGF-1R in a manner similar to insulin and/or the C-terminal region of the α-chain of IR, as defined above. Similar methods could be used to identify chemical entities or compounds that may interact with the L1 domain of IR and/or IGF-1R in a manner similar to that of insulin and/or the C-terminal region of the α-chain of IR.

Modelling software that is well known and available in the art may be used (Guida, 1994). These include Discovery Studio (Accelrys Software Inc., San Diego), SYBYL (Tripos Associates, Inc., St. Louis, Mo., 1992), Maestro (Schrödinger LLC, Portland), MOE (Chemical Computing Group Inc., Montreal, Canada). This modelling step may be followed by energy minimization with standard molecular mechanics force fields such as AMBER (Weiner *et al.,* 1984), OPLS (Jorgensen and Tirado-Rives, 1988) and CHARMM (Brooks *et al.,* 1983). In addition, there are a number of more specialized computer programs to assist in the process of selecting the binding moieties.

Specialised computer programs may also assist in the process of selecting fragments or chemical entities. These include, inter alia:
1. GRID (Goodford, 1985). GRID is available from Molecular Discovery Ltd., Italy;
2. AUTODOCK (Goodsell & Olsen, 1990). AUTODOCK is available from Scripps Research Institute, La Jolla, CA;
3. DOCK (Kuntz *et al.,* 1982). DOCK is available from University of California, San Francisco, CA;
4. GLIDE (Friesner *et al.,* 2004). GLIDE is available from Schrödinger LLC, Portland; and
5. GOLD (Cole *et al.,* 2005). GOLD is available from The Cambridge Crystallographic Data Centre, Cambridge, UK.

Once suitable chemical entities or fragments have been selected, they can be assembled into a single compound. In one embodiment, assembly may proceed by visual inspection of the relationship of the fragments to each other on the three-dimensional image displayed on a computer screen in relation to the structure coordinates of the low affinity site of IR, or the L1 domain to which insulin and/or the C-terminal region of the α-chain of IR binds. This is followed by manual model building using software such as Discovery Studio, Maestro, MOE or Sybyl. Alternatively, fragments may be joined to additional atoms using standard chemical geometry.

The above-described evaluation process for chemical entities may be performed in a similar fashion for chemical compounds.

Useful programs to aid one of skilled in the art in connecting the individual chemical entities or fragments include:
1. CAVEAT (Bartlett *et al.,* 1989). CAVEAT is available from the University of California, Berkeley, CA; and
2. GANDI (Day and Caflisch, 2008). GANDI is available from the University of Zurich.

Other molecular modelling techniques may also be employed in accordance with this invention, see, e.g., Cohen *et al.* (1990) and Navia & Murcko (1992).

There are two preferred approaches to designing a molecule that complement the stereochemistry of the low affinity binding site of IR and/or IGF-1R, or the L1 domain to which insulin and/or the C-terminal region of the α-chain of IR binds. The first approach is to *in silico* directly dock molecules from a three-dimensional structural database, to the target binding site, using mostly, but not exclusively, geometric criteria to assess the goodness-of-fit of a particular molecule to the site. In this approach, the number of internal degrees of freedom (and the corresponding local minima in the molecular conformation space) is reduced by considering only the geometric (hard-sphere) interactions of two rigid bodies, where one body (the active site) contains "pockets" or "grooves" that form binding sites for the second body (the complementing molecule).

Flexibility of the receptor, IR or IGF-1R, can be incorporated into the *in silico* screening by the application of multiple conformations of the receptor (Totrov and Abagyan, 2008). The multiple conformations of the IR receptor can be generated from the coordinates listed in Appendix I computationally by use of molecular dynamics simulation or similar approaches.

This approach is illustrated by Kuntz *et al.* (1982) and Ewing *et al.* (2001), whose algorithm for ligand design is implemented in a commercial software package, DOCK version 4.0, distributed by the Regents of the University of California and further described in a document, provided by the distributor, which is entitled "Overview of the DOCK program suite". Pursuant to the Kuntz algorithm, the shape of the cavity in which the C-terminal region of the α-chain of IR fits and/or insulin fits is defined as a series of overlapping spheres of different radii. One or more extant databases of crystallographic data, such as the Cambridge Structural Database System (The Cambridge Crystallographic Data Centre, Cambridge, U.K.), the Protein Data Bank maintained by the Research Collaboratory for Structural Bioinformatics (Rutgers University, N.J., U.S.A.), LeadQuest (Tripos Associates, Inc., St. Louis, MO), Available Chemicals Directory (Symyx Technologies Inc.), and the NCI database (National Cancer Institute, U.S.A) is then searched for molecules which approximate the shape thus defined.

Molecules identified on the basis of geometric parameters, can then be modified to satisfy criteria associated with chemical complementarity, such as hydrogen bonding, ionic interactions and van der Waals interactions. Different scoring functions can be employed to rank and select the best molecule from a database. See for example Bohm & Stahl (1999). The software package FlexX, marketed by Tripos Associates, Inc. (St. Louis, MO) is another program that can be used in this direct docking approach (see Rarey *et al.,* 1996).

The second preferred approach entails an assessment of the interaction of respective chemical groups ("probes") with the active site at sample positions within and around the site, resulting in an array of energy values from which three-dimensional contour surfaces at selected energy levels can be generated. The chemical-probe approach to ligand design is described, for example, by Goodford, (1985), and is implemented in several commercial software packages, such as GRID (product of Molecular Discovery Ltd., Italy).

Pursuant to this approach, the chemical prerequisites for a site-complementing molecule are identified at the outset, by probing the active site with different chemical probes, e.g., water, a methyl group, an amine nitrogen, a carboxyl oxygen, or a hydroxyl. Favoured sites for interaction between the active site and each probe are thus determined, and from the resulting three-dimensional pattern of such sites a putative complementary molecule can be generated. This may be done either by programs that can search three-dimensional databases to identify molecules incorporating desired pharmacophore patterns or by programs which use the favoured sites and probes as input to perform de novo design. Suitable programs for determining and designing pharmacophores include CATALYST (Accelrys Software, Inc), and CERIUS2, DISCO (Abbott Laboratories, Abbott Park, IL; distributed by Tripos Associates Inc.).

The pharmacophore can be used to screen in silico compound libraries/ three-dimensional databases, using a program such as CATALYST (Accelrys Software, Inc) and Sybyl/3DB Unity (Tripos Associates, Inc., St. Louis, MO).

Databases of chemical structures are available from a number of sources including Cambridge Crystallographic Data Centre (Cambridge, U.K.), Molecular Design, Ltd., (San Leandro, CA), Tripos Associates, Inc. (St. Louis, MO), Chemical Abstracts Service (Columbus, OH), the Available Chemical Directory (Symyx Technologies, Inc.), the Derwent World Drug Index (WDI), BioByteMasterFile, the National Cancer Institute database (NCI), Medchem Database (BioByte Corp.), and the Maybridge catalogue.

De novo design programs include LUDI (Accelrys Software Inc., San Diego, CA), Leapfrog (Tripos Associates, Inc.), and LigBuilder (Peking University, China).

Once an entity or compound has been designed or selected by the above methods, the efficiency with which that entity or compound may bind to IR and/or IGF-1R can be tested and optimised by computational evaluation. For example, a compound that has been designed or selected to function as an IR binding compound must also preferably traverse a volume not overlapping that occupied by the binding site when it is bound to the native IR. An effective IR binding compound must preferably demonstrate a relatively small difference in energy between its bound and free states (i.e., a small deformation energy of binding). Thus, the most efficient IR binding compound should preferably be designed with a deformation energy of binding of not greater than about 10 kcal/mole, preferably, not greater than 7 kcal/mole. IR and/or IGF-1R binding compounds may interact with IR and/or IGF-1R in more than one conformation that are similar in overall binding energy. In those cases, the deformation energy of binding is taken to be the difference between the energy of the free compound and the average energy of the conformations observed when the compound binds to the protein.

A compound designed or selected as binding to IR and/or IGF-1R may be further computationally optimised so that in its bound state it would preferably lack repulsive electrostatic interaction with the target protein.

Such non-complementary (e.g., electrostatic) interactions include repulsive charge-charge, dipole-dipole and charge-dipole interactions. Specifically, the sum of all electrostatic interactions between the compound and the protein when the compound is bound to IR and/or IGF-1R, preferably make a neutral or favourable contribution to the enthalpy of binding.

Once an IR-binding compound and/or IGF-1R-binding compound has been optimally selected or designed, as described above, substitutions may then be made in some of its atoms or side groups to improve or modify its binding properties. Generally, initial substitutions are conservative, i.e., the replacement group will have approximately the same size, shape, hydrophobicity and charge as the original group. It should, of course, be understood that components known in the art to alter conformation should be avoided. Such substituted chemical compounds may then be analysed for efficiency of fit to IR and/or IGF-1R by the same computer methods described in detail above.

Specific computer software is available in the art to evaluate compound deformation energy and electrostatic interaction. Examples of programs designed for such uses include: Gaussian 03, (Frisch, Gaussian, Inc., Pittsburgh, PA); GAMESS (Gordon et al., Iowa State University); Jaguar (Schrödinger LLC, Portland);AMBER, version 9.0 (Case et al, University of California at San Francisco); CHARMM (Accelrys Software, Inc., San Diego, CA); and GROMACS version 4.0 (van der Spoel et al.).

The screening/design methods may be implemented in hardware or software, or a combination of both. However, preferably, the methods are implemented in computer programs executing on programmable computers each comprising a processor, a data storage system (including volatile and non-volatile memory and/or storage elements), at least one input device, and at least one output device. Program code is applied to input data to perform the functions described above and generate output information. The output information is applied to one or more output devices, in known fashion. The computer may be, for example, a personal computer, microcomputer, or workstation of conventional design.

Each program is preferably implemented in a high level procedural or object-oriented programming language to communicate with a computer system. However, the programs can be implemented in assembly or machine language, if desired. In any case, the language may be compiled or interpreted language.

Each such computer program is preferably stored on a storage medium or device (e.g., ROM or magnetic diskette) readable by a general or special purpose programmable computer, for configuring and operating the computer when the storage media or device is read by the computer to perform the procedures described herein. The system may also be considered to be implemented as a computer-readable storage medium, configured with a computer program, where the storage medium so configured causes a computer to operate in a specific and predefined manner to perform the functions described herein.

### Compounds

Compounds described herein include both those designed or identified using a screening method of the invention and those which are capable of recognising and binding to the low affinity binding site of IR and/or IGF-1R as defined above. Also described are compounds that bind to the L1 domain of IR in a manner similar to that of insulin and/or the C-terminal region of the α-chain of IR, i.e., compounds which mimic insulin and/or the C-terminal region of the α-chain of IR.

Compounds capable of recognising and binding to the low affinity binding site of IR and/or IGF-1R may be produced using (i) a screening method based on use of the atomic coordinates corresponding to the 3D structure of the low affinity binding site of IR in complex with insulin; or (ii) a screening method based on the use of the atomic coordinates corresponding to the 3D structure of insulin in complex with IR. Alternatively, compounds may be identified by screening against a specific target molecule which is indicative of the capacity to bind to the low affinity binding site of IR.

Compounds capable of recognising and binding to the L1 domain of IR and/or IGF-1R in a manner similar to that of insulin and/or the C-terminal region of the α-chain of IR to the L1 domain of IR (i.e. compounds which mimic insulin and/or the C-terminal region of the α-chain of IR) may be produced using a screening method based on use of the atomic coordinates corresponding to the 3D structure of the insulin and/or the C-terminal region of the α-chain of IR in isolation or as it associates with IR, or alternatively may be identified by screening against a specific target molecule which is indicative of the capacity to bind to the low affinity binding site of IR.

The candidate compounds and/or compounds identified or designed using a method of the present invention may be any suitable compound, synthetic or naturally occurring, preferably synthetic. In one embodiment, a synthetic compound selected or designed by the methods of the invention preferably has a molecular weight equal to or less than about 5000, 4000, 3000, 2000, 1000 or 500 daltons. A compound as described herein is preferably soluble under physiological conditions.

The compounds may encompass numerous chemical classes, though typically they are organic molecules, preferably small organic compounds having a molecular weight of more than 50 and less than about 2,500 daltons, preferably less than 1,500, more preferably less than 1,000 and yet more preferably less than 500. Such compounds can comprise functional groups necessary for structural interaction with proteins, particularly hydrogen bonding, and typically include at least an amine, carbonyl, hydroxyl or carboxyl group, preferably at least two of the functional chemical groups. The compound may comprise cyclical carbon or heterocyclic structures and/or aromatic or polyaromatic structures substituted with one or more of the above functional groups. Compounds can also comprise biomolecules including peptides, saccharides, fatty acids, steroids, purines, pyrimidines, derivatives, structural analogues, or combinations thereof.

Compounds may include, for example: (1) peptides such as soluble peptides, including Ig-tailed fusion peptides and members of random peptide libraries (see, e.g., Lam *et al.,* 1991; Houghten *et al.,* 1991) and combinatorial chemistry-derived molecular libraries made of D-and/or L-configuration amino acids; (2) phosphopeptides (e.g., members of random and partially degenerate, directed phosphopeptide libraries, see, e.g., Songyang *et al.,* 1993); (3) antibodies (e.g., polyclonal, monoclonal, humanized, anti-idiotypic, chimeric, and single chain antibodies as well as Fab, (Fab)_{2'}, Fab expression library and epitope-binding fragments of antibodies); (4) nonimmunoglobulin binding proteins such as but not restricted to avimers, DARPins and lipocalins; (5) nucleic acid-based aptamers; and (6) small organic and inorganic molecules.

Ligands can be obtained from a wide variety of sources including libraries of synthetic or natural compounds. Synthetic compound libraries are commercially available from, for example, Maybridge Chemical Co. (Tintagel, Cornwall, UK), AMRI (Budapest, Hungary) and ChemDiv (San Diego, CA), Specs (Delft, The Netherlands).

Natural compound libraries comprising bacterial, fungal, plant or animal extracts are available from, for example, Pan Laboratories (Bothell, WA), TimTec (Newark, DE). In addition, numerous means are available for random and directed synthesis of a wide variety of organic compounds and biomolecules, including expression of randomized oligonucleotides.

Alternatively, libraries of natural compounds in the form of bacterial, fungal, plant and animal extracts can be readily produced. Methods for the synthesis of molecular libraries are readily available (see, e.g., DeWitt *et al.,* 1993; Erb *et al.,* 1994; Zuckermann *et al.,* 1994; Cho *et al.,* 1993; Carell *et al.,* 1994a; Carell *et al.,* 1994b; and Gallop *et al.,* 1994). In addition, natural or synthetic compound libraries and compounds can be readily modified through conventional chemical, physical and biochemical means (see, e.g., Blondelle and Houghton, 1996), and may be used to produce combinatorial libraries. In another approach, previously identified pharmacological agents can be subjected to directed or random chemical modifications, such as acylation, alkylation, esterification, amidification, and the analogues can be screened for IR and/or IGF-1R-modulating activity.

Numerous methods for producing combinatorial libraries are known in the art, including those involving biological libraries; spatially addressable parallel solid phase or solution phase libraries; synthetic library methods requiring deconvolution; the "one-bead one-compound" library method; and synthetic library methods using affinity chromatography selection. The biological library approach is limited to polypeptide or peptide libraries, while the other four approaches are applicable to polypeptide, peptide, nonpeptide oligomer, or small molecule libraries of compounds (Lam, 1997).

Compounds also include those that may be synthesized from leads generated by fragment-based drug design, wherein the binding of such chemical fragments is assessed by soaking or co-crystallizing such screen fragments into crystals provided by the invention and then subjecting these to an X-ray beam and obtaining diffraction data. Difference Fourier techniques are readily applied by those skilled in the art to determine the location within the IR ectodomain structure at which these fragments bind, and such fragments can then be assembled by synthetic chemistry into larger compounds with increased affinity for the receptor.

### Isolated peptides or mimetics thereof

Compounds identified or designed using the methods of the invention can be a peptide or a mimetic thereof.

The isolated peptides or mimetics may be conformationally constrained molecules or alternatively molecules which are not conformationally constrained such as, for example, non-constrained peptide sequences. The term "conformationally constrained molecules" means conformationally constrained peptides and conformationally constrained peptide analogues and derivatives.

The term "analogues" refers to molecules having a chemically analogous structure to naturally occurring α-amino acids. Examples include molecules containing gem-diaminoalkyl groups or alklylmalonyl groups.

The term "derivatives" includes α-amino acids wherein one or more side groups found in the naturally occurring α-amino acids have been modified. Thus, for example the amino acids may be replaced with a variety of uncoded or modified amino acids such as the corresponding D-amino acid or N-methyl amino acid. Other modifications include substitution of hydroxyl, thiol, amino and carboxyl functional groups with chemically similar groups.

With regard to peptides and mimetics thereof, other examples of other unnatural amino acids or chemical amino acid analogues/derivatives which can be introduced as a substitution or addition include, but are not limited to, 2,4-diaminobutyric acid, α-amino isobutyric acid, 4-aminobutyric acid, 2-aminobutyric acid, 6-amino hexanoic acid, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, β-alanine, fluoro-amino acids, designer amino acids such as β-methyl amino acids, Ca-methyl amino acids, Na-methyl amino acids, and amino acid analogues in general.

The mimetic may be a peptidomimetic. A "peptidomimetic" is a molecule that mimics the biological activity of a peptide but is no longer peptidic in chemical nature. By strict definition, a peptidomimetic is a molecule that no longer contains any peptide bonds (that is, amide bonds between amino acids). However, the term peptide mimetic is sometimes used to describe molecules that are no longer completely peptidic in nature, such as pseudopeptides, semi-peptides and peptoids. Whether completely or partially non-peptide, peptidomimetics provide a spatial arrangement of reactive chemical moieties that closely resembles the three-dimensional arrangement of active groups in the peptide on which the peptidomimetic is based. As a result of this similar active-site geometry, the peptidomimetic has effects on biological systems which are similar to the biological activity of the peptide.

There are sometimes advantages for using a mimetic of a given peptide rather than the peptide itself, because peptides commonly exhibit two undesirable properties: (1) poor bioavailability; and (2) short duration of action. Peptide mimetics offer an obvious route around these two major obstacles, since the molecules concerned are small enough to be both orally active and have a long duration of action. There are also considerable cost savings and improved patient compliance associated with peptide mimetics, since they can be administered orally compared with parenteral administration for peptides. Furthermore, peptide mimetics are generally cheaper to produce than peptides.

Suitable peptidomimetics based on insulin, a fragment thereof, and/or the C-terminal region of the α-chain of IR and/or IGR-1R can be developed using readily available techniques. Thus, for example, peptide bonds can be replaced by non-peptide bonds that allow the peptidomimetic to adopt a similar structure, and therefore biological activity, to the original peptide. Further modifications can also be made by replacing chemical groups of the amino acids with other chemical groups of similar structure. The development of peptidomimetics derived from peptides of insulin, a fragment thereof, and/or the C-terminal region of the IR and/or IGF-1R α-chain can be aided by reference to the three dimensional structure of these residues as provided in Appendix I. This structural information can be used to search three-dimensional databases to identify molecules having a similar structure, using programs such as Sybyl/3DB Unity (Tripos Associates, St. Louis, MO).

Those skilled in the art will recognize that the design of a peptidomimetic may require slight structural alteration or adjustment of a chemical structure designed or identified using the methods of the invention. In general, chemical compounds identified or designed using the methods of the invention can be synthesized chemically and then tested for ability to modulate IR and/or IGF-1R activity using any of the methods described herein. The methods of the invention are particularly useful because they can be used to greatly decrease the number potential mimetics which must be screened for their ability to modulate IR and/or IGF-1R activity.

The peptides or peptidomimetics can be used in assays for screening for candidate compounds which bind to regions of IR and/or IGF-1R and potentially interfere with the binding of insulin to IR and/or signal transduction and/or the binding of IGF to IGF-1R and/or signal transduction. Peptides or peptidomimetics which mimic target binding sites are particularly useful as specific target molecules for identifying potentially useful ligands for IR and/or IGF-1R.

Standard solid-phase ELISA assay formats are particularly useful for identifying compounds that bind to the receptor. In accordance with this embodiment, the peptide or peptidomimetic immobilized on a solid matrix, such as, for example an array of polymeric pins or a glass support. Conveniently, the immobilized peptide or peptidomimetic is a fusion polypeptide comprising Glutathione-S-transferase (GST; e.g. a CAP-ERK fusion), wherein the GST moiety facilitates immobilization of the protein to the solid phase support. This assay format can then be used to screen for candidate compounds that bind to the immobilised peptide or peptidomimetic and/or interfere with binding of a natural binding partner of IR and/or IGF-1R to the immobilised peptide or peptidomimetic.

As used herein a "fragment" is a portion of a peptide which maintains a defined activity of the "full-length" peptide, namely the ability to bind to the low affinity binding site of IR and/or IGF-1R, or to bind to the L1 domain of IR and/or IGF-1R. Fragments can be any size as long as they maintain the defined activity. Preferably, the fragment maintains at least 50%, more preferably at least 75%, of the activity of the full length polypeptide.

The % identity of a peptide is determined by GAP (Needleman and Wunsch, 1970) analysis (GCG program) with a gap creation penalty=5, and a gap extension penalty=0.3. The query sequence is at least 10 amino acids in length, and the GAP analysis aligns the two sequences over a region of at least 10 amino acids. More preferably, the GAP analysis aligns two sequences over their entire length.

With regard to a defined peptide, it will be appreciated that % identity figures higher than those provided above will encompass preferred embodiments. Thus, where applicable, in light of the minimum % identity figures, it is preferred that the peptide comprises an amino acid sequence which is at least 50%, more preferably at least 55%, more preferably at least 60%, more preferably at least 65%, more preferably at least 70%, more preferably at least 75%, more preferably at least 80%, more preferably at least 85%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, and even more preferably at least 99.9% identical to the relevant nominated SEQ ID NO.

Amino acid sequence mutants of the peptides identified or designed using the methods of the invention, can be prepared by introducing appropriate nucleotide changes into a nucleic acid as described herein, or by *in vitro* synthesis of the desired peptide. Such mutants include, for example, deletions, insertions or substitutions of residues within the amino acid sequence. A combination of deletion, insertion and substitution can be made to arrive at the final construct, provided that the final peptide product possesses the desired characteristics.

In designing amino acid sequence mutants, the location of the mutation site and the nature of the mutation will depend on characteristic(s) to be modified. The sites for mutation can be modified individually or in series, e.g., by (1) substituting first with conservative amino acid choices and then with more radical selections depending upon the results achieved, (2) deleting the target residue, or (3) inserting other residues adjacent to the located site.

Substitution mutants have at least one amino acid residue in the peptide removed and a different residue inserted in its place. Sites of interest are those in which particular residues obtained from various strains or species are identical. These sites, especially those falling within a sequence of at least three other identically conserved sites, are preferably substituted in a relatively conservative manner. Such conservative substitutions are shown in Table 1 under the heading of "exemplary substitutions".

**Table 1 - Exemplary substitutions.**

| **Original** | **Exemplary** |
|---|---|
| **Residue** | **Substitutions** |
| Ala (A) | val; leu; ile; gly |
| Arg (R) | lys |
| Asn (N) | gln; his |
| Asp (D) | glu |
| Cys (C) | ser |
| Gln (Q) | asn; his |
| Glu (E) | asp |
| Gly (G) | pro, ala |
| His (H) | asn; gln |
| Ile (I) | leu; val; ala |
| Leu (L) | ile; val; met; ala; phe |
| Lys (K) | arg |
| Met (M) | leu; phe |
| Phe (F) | leu; val; ala |
| Pro (P) | gly |
| Ser (S) | thr |
| Thr (T) | ser |
| Trp (W) | tyr |
| Tyr (Y) | trp; phe |
| Val (V) | ile; leu; met; phe, ala |

In a preferred embodiment a mutant/variant peptide has one or two or three or four conservative amino acid changes when compared to a peptide defined herein. Details of conservative amino acid changes are provided in Table 1.

Also described herein are peptides which are differentially modified during or after synthesis, e.g., by biotinylation, benzylation, glycosylation, acetylation, phosphorylation, amidation, derivatization by known protecting/blocking groups, proteolytic cleavage, linkage to an antibody molecule or other cellular ligand, etc. These modifications may serve to increase the stability and/or bioactivity of the peptide.

The residues that form the αCT segment of the IR α-chain, namely residues 704 to 719, can be grouped into four classes: Class A - those whose side chains are completely buried in the interface with insulin (His710, Asn711 and Phe714); Class B - those whose sides chains are completely buried in the interface with L1 (Phe703, Phe705 Tyr708, Leu709, Val712, Val713); Class C - those whose side chains lie at the periphery of the interface with insulin and/or the L1 domain; and Class D - those whose side chains appear to have no interaction with insulin or L1. In terms of using the αCT segment itself as a scaffold for mimetics that might compete with the naturally-occurring aCT segment in its binding to insulin and/or the L1 domain, design might focus in the first instance on substitution of those residues belonging to Class C, given that the residues lying in Classes A and B are relatively optimally packed within the interfaces and already have few degrees of freedom. Higher affinity binding might be achieved by substitution of one or more of the Class C residues with either naturally-occurring amino acids or non-natural amino acids. For example, the substitution of one or more of the charged residues with residues that have reduced rotameric degrees of freedom (i.e., reduced entropy) may lead to higher affinity binding or altered physicochemical properties of the compound. Such modification for example may include substitution by the naturally-occurring amino acids Phe, Tyr or Trp. As a further example, it may be possible to substitute residues with more bulky non-natural amino acids that retain the terminal cationic character, for example substitution by the basic phenyl propanoic acid derivatives App (L-2-amino-3-(4-aminophenyl)propanoic acid) and/or Gpp, (L-2-amino-3-(4-guanidinophenyl)propanoic acid) (Svenson *et al.,* 2009). A further strategy for design might involve substitutions to improve the overall stability of the helical structure (for example helix stapling - see Danial *et al.,* 2008). Such substitutions would likely be made within Class D residues. A yet further strategy to improve affinity or physicochemical properties might involve truncation of the helical segment and/or attaching an N- or C-terminal group also designed to improve affinity. Similar principles of design may be applied to generate modified peptides based on the corresponding native IGF-1R peptide as outlined above for the IR peptide.

Likewise, the residues that form the Site 1 binding surface of insulin can be grouped into five classes: Class A - those whose side chains are complete buried in the interface with the L1 domain (TyrB16, PheB24); Class B - those whose sides chains are completely buried in the interface with the aCT segment (ValA3, GlyB8, SerB9, GlyA1, IleA2, TyrA19, LeuB11, LeuB15, GluA4 and PheB25); Class C - those whose side chains mediate the interface with both the L1 domain and the aCT segment (ValB12 and TyrB26); Class D -those whose side chains lie at the periphery of the interface with the L1 domain and/or the aCT segment; and Class E - those whose side chains appear to have no interaction with the L1 domain or the aCT segment. In terms of using the insulin monomer as defined in complex with IR itself as a scaffold for mimetics that might compete with the naturally-occurring insulin in its binding to the L1 domain and/or the αCT segment, design might focus in the first instance on substitution of those residues belonging to Class D, given that the residues lying in Classes A and B are relatively optimally packed within the interfaces and already have few degrees of freedom. Higher affinity binding might be achieved by substitution of one or more of the Class D residues with either naturally-occurring amino acids or non-natural amino acids. For example, the substitution of one or more of the charged residues with residues that have reduced rotameric degrees of freedom (i.e., reduced entropy) may lead to higher affinity binding or altered physicochemical properties of the compound. Such modification for example may include substitution by the naturally-occurring amino acids Phe, Tyr or Trp. As a further example, it may be possible to substitute residues with more bulky non-natural amino acids that retain the terminal cationic character, for example substitution by the basic phenyl propanoic acid derivatives App (L-2-amino-3-(4-aminophenyl)propanoic acid) and/or Gpp, (L-2-amino-3-(4-guanidinophenyl)propanoic acid) (Svenson *et al.,* 2009). A further strategy for design might involve substitutions to improve the overall stability of the conformational change in insulin upon binding to the IR. Such substitutions would likely be made within Class E residues. A yet further strategy to improve affinity or physicochemical properties might involve truncation of the C-terminus residues B26 to B30 and/or attaching an N- or C-terminal group also designed to improve affinity. Similar principles of design may be applied to generate modified peptides based on the corresponding native IGF-1R peptide as outlined above for the IR peptide.

The design of synthetic non-peptide mimetics of α-helices is an established art (see for example Davis *et al.,* 2006). In particular, methods of mimicry of i, i+4, i+7 motifs (such as those identified within the aCT segment of the α-chain of IR and IGF-1R and which interact the respective L1 domains are known. For example, these motifs may be mimicked by terphenyl, oligophenyl, chalcone or 1,4-benzodiazepine-2,5-dione scaffolds (Davis *et al.,* 2006) or by benzoylurea scaffolds (US 2008153802). Non-peptide mimetics of α-helices have been investigated as therapeutics in a number of disease contexts, for example HIV1 infection (disruption of the assembly of the hexameric helical bundle (Ernst *et al.,* 2001)) and cancer (disruption of the assembly of the HDM2-p53 complex (Yin *et al.,* 2005); inhibitors of Bcl-2 family heterodimerisation (Degterev *et al.,* 2001).

With regard to redesigning compounds using a method of the invention, in an embodiment the compound is redesigned to be more structurally similar to the native aCT segment of the α-chain of IR and/or the aCT segment of the α-chain of IGF-1R. Examples of peptides which could be redesigned in this manner include, but are not limited to, those described by Schäffer et al. (2003) and/or US 7,173,005.

In an alternative embodiment, the compound is redesigned to be more structurally similar to native insulin in complex with the IR. Preferably, the compound is redesigned to mimic the conformational structural changes in insulin upon binding to the IR. More preferably, the compound is redesigned to mimic the conformational structural changes in the insulin B-chain upon binding to the IR.

### Interaction of compounds with IR and/or IGF-1R

A compound may interact with the low affinity binding site of IR and/or IGF-1R by binding either directly or indirectly to that region. A compound which binds directly, binds to the specified region. A compound which binds indirectly, binds to a region in close proximity to or adjacent to the low affinity binding site of IR and/or IGF-1R with the result that it interferes with the ability of IR to bind to insulin, or IGF-1R to bind IGF, either antagonistically or agonistically. Such interference may be steric, electrostatic, or allosteric. Preferably, a compound interacts with the low affinity binding site of IR and/or IGF-1R by binding directly to the specified region. In the case of compounds that bind to specific target molecules, such compounds bind directly to the specific target molecule.

A compound may alternatively interact with the L1 domain of IR and/or IGF-1R in a manner similar to that of the αCT segment of the α-chain of IR by binding either directly or indirectly to that region. A compound which binds directly, binds to the specified region. A compound which binds indirectly, binds to a region in close proximity to or adjacent to the L1 domain of IR and/or IGF-1R in a manner similar to that of the αCT segment of the α-chain of IR with the result that it interferes with the ability of IR to bind to insulin, or IGF-1R to bind IGF, either antagonistically or agonistically. Such interference may be steric, electrostatic, or allosteric. Preferably, a compound interacts with the L1 domain of IR and/or IGF-1R in a manner similar to that of the aCT segment of the α-chain of IR by binding directly to the specified region. In the case of compounds that bind to specific target molecules, such compounds bind directly to the specific target molecule.

Binding can be either by covalent or non-covalent interactions, or both. Examples of non-covalent interactions include electrostatic interactions, van der Waals interactions, hydrophobic interactions and hydrophilic interactions.

When a compound described herein interacts with IR and/or IGF-1R, it preferably "modulates" IR or IGF-1R, respectively. By "modulate" we mean that the compound changes an activity of IR or IGF-1R by at least 10%. Suitably, a compound modulates IR or IGF-1R by increasing or decreasing signal transduction via IR or IGF-1R, respectively. The phrase "decreases signal transduction" is intended to encompass partial or complete inhibition of signal transduction via IR or IGF-1R. The ability of a candidate compound to increase or decrease signal transduction via IR or IGF-1R can be assessed by any one of the IR or IGF-1R cell-based assays described herein.

Compounds may act as antagonists or agonists for insulin binding to IR or as antagonists or agonists for IGF binding to IGF-1R.

Compounds described herein preferably have an affinity for IR or IGF-1R sufficient to provide adequate binding for the intended purpose. Suitably, such compounds and compounds which bind to specific target molecules of IR or IGF-1R have an affinity (K_{d}) of from 10⁻⁵ to 10⁻¹⁵ M. For use as a therapeutic, the compound suitably has an affinity (K_{d}) of from 10⁻⁷ to 10⁻¹⁵ M, preferably from 10⁻⁸ to 10⁻¹² M and more preferably from 10⁻¹⁰ to 10⁻¹² M. Where a compound is to be used as a reagent in a competitive assay to identify other ligands, the compound suitably has an affinity (K_{d}) of from 10⁻⁵ to 10⁻¹² M.

As will be evident to the skilled person, the crystal structure presented herein has enabled, for the first time, direct visualisation of the regions binding insulin in the IR. The structure has enabled the identification of the αCT segment of the α-chain of IR, critical for the initial binding of insulin, and in the subsequent formation of the high affinity insulin-IR complex that leads to signal transduction.

In one preferred embodiment, a compound has a high specificity for IR and/or a specific target molecule of IR but not for IGF-1R, i.e., a compound selectively binds to IR or has enhanced selectivity for IR over IGF-1R. In this respect, a compound suitably has an affinity (K_{d}) for IR and/or a specific target molecule of IR of no more than 10⁻⁵ M, preferably no more than 10⁻⁷ M, and an affinity for IGF-1R of at least 10⁻⁵ M, preferably at least 10⁻³ M. Such compounds are desirable as, for example, IR agonists where the propensity to interact with IGF-1R and thus, for example, promote undesirable cell proliferation, is reduced.

In a preferred embodiment, the (IR or specific target molecule of IR)/IGF-1R binding affinity ratio for a compound is at least 10 and preferably at least 100.

In another preferred embodiment, a compound has a high specificity for IGF-1R and/or a specific target molecule of IGF-1R but not for IR, i.e., a compound selectively binds to IGF-1R or has enhanced selectivity for IGF-1R over IR. In this respect, a compound suitably has an affinity (K_{d}) for IGF-1R and/or a specific target molecule of IGF-1R of no more than 10⁻⁵ M, preferably no more than 10⁻⁷ M, and an affinity for IR of at least 10⁻⁵ M, preferably at least 10⁻³ M. Such compounds are desirable as, for example, IGF-1R agonists where there propensity to interact with IR and thus, for example, promote glucose uptake and metabolism, is reduced.

In a preferred embodiment, the (IGF-1R or specific target molecule of IGF-1R)/(IR) binding affinity ratio for a compound is at least 10 and preferably at least 100.

### Screening assays and confirmation of binding

Compounds described herein may be subjected to further confirmation of binding to IR and/or IGF-1R by co-crystallization of the compound with IR and/or IGF-1R and structural determination, as described herein.

Compounds designed or selected according to the methods of the present invention are preferably assessed by a number of *in vitro* and *in vivo* assays of IR and/or IGF-1R function to confirm their ability to interact with and modulate IR and/or IGF-1R activity. For example, compounds may be tested for their ability to bind to IR and/or IGF-1R and/or for their ability to modulate e.g. disrupt, IR and/or IGF-1R signal transduction.

Libraries may be screened in solution by methods generally known in the art for determining whether ligands competitively bind at a common binding site. Such methods may include screening libraries in solution (e.g., Houghten, 1992), or on beads (Lam, 1991), chips (Fodor, 1993), bacteria or spores (U.S. 5,223,409), plasmids (Cull *et al.,* 1992), or on phage (Scott & Smith, 1990; Devlin, 1990; Cwirla *et al.,* 1990; Felici, 1991; U.S. 5,223,409).

Where the screening assay is a binding assay, IR or IGF-1R may be joined to a label, where the label can directly or indirectly provide a detectable signal. Various labels include radioisotopes, fluorescent molecules, chemiluminescent molecules, enzymes, specific binding molecules, particles, e.g., magnetic particles, and the like. Specific binding molecules include pairs, such as biotin and streptavidin, digoxin and antidigoxin, etc. For the specific binding members, the complementary member would normally be labelled with a molecule that provides for detection, in accordance with known procedures.

A variety of other reagents may be included in the screening assay. These include reagents like salts, neutral proteins, e.g., albumin, detergents, etc., which are used to facilitate optimal protein-protein binding and/or reduce non-specific or background interactions. Reagents that improve the efficiency of the assay, such as protease inhibitors, nuclease inhibitors, antimicrobial agents, etc., may be used. The components are added in any order that produces the requisite binding. Incubations are performed at any temperature that facilitates optimal activity, typically between 4 and 40 °C.

Direct binding of compounds to IR or IGF-1R can also be done by Surface Plasmon Resonance (BIAcore) (reviewed in Morton & Myszka, 1998). Here the receptor is immobilized on a CM5 or other sensor chip by either direct chemical coupling using amine or thiol-disulphide exchange coupling (Nice & Catimel, 1999) or by capturing the receptor ectodomain as an Fc fusion protein to an appropriately derivatised sensor surface (Morten & Myszka, 1998). The potential binding molecule (called an analyte) is passed over the sensor surface at an appropriate flow rate and a range of concentrations. The classical method of analysis is to collect responses for a wide range of analyte concentrations. A range of concentrations provides sufficient information about the reaction, and by using a fitting algorithm such as CLAMP (see Morton & Myszka, 1998), rate constants can be determined (Morton & Myszka, 1998; Nice & Catimel, 1999). Normally, the ligand surface is regenerated at the end of each analyte binding cycle. Surface regeneration ensures that the same number of ligand binding sites is accessible to the analyte at the beginning of each cycle.

Incubation periods are selected for optimum activity, but may also be optimized to facilitate rapid high-throughput screening. Normally, between 0.1 and 1 hour will be sufficient. In general, a plurality of assay mixtures is run in parallel with different test agent concentrations to obtain a differential response to these concentrations. Typically, one of these concentrations serves as a negative control, i.e. at zero concentration or below the level of detection.

The basic format of an *in vitro* competitive receptor binding assay as the basis of a heterogeneous screen for small organic molecular replacements for insulin may be as follows: occupation of the low affinity binding site of IR and/or IGF-1R is quantified by time-resolved fluorometric detection (TRFD) as described by Denley *et al.,* 2004. R⁻IR-A, R⁻IR-B and P6 cells are used as sources of IR-A, IR-B and IGF-1R respectively. Cells are lysed with lysis buffer (20 mM HEPES, 150 mM NaCl, 1.5 mM MgCl₂, 10% (v/v) glycerol, 1% (v/v) Triton X-100, 1 mM EGTA pH 7.5) for 1 hour at 4°C. Lysates are centrifuged for 10 minutes at 3500 rpm and then 100 µl is added per well to a white Greiner Lumitrac 600 plate previously coated with ariti-insulin receptor antibody 83-7 or anti-IGF-1R antibody 24-31. Neither capture antibody interferes with receptor binding by insulin, IGF-I or IGF-II. Approximately 100,000 fluorescent counts of europium-labelled insulin or europium-labelled IGF-I are added to each well along with various amounts of unlabelled competitor and incubated for 16 hours at 4°C. Wells are washed with 20 mM Tris, 150 mM NaCl, 0.05% (v/v) Tween 20 (TBST) and DELFIA enhancement solution (100 µl/well) is added. Time-resolved fluorescence is measured using 340 nm excitation and 612 nm emission filters with a BMG Lab Technologies Polarstar™ Fluorimeter or a Wallac Victor II (EG & G Wallac, Inc.).

Examples of other suitable assays which may be employed to assess the binding and biological activity of compounds to and on IR are well known in the art. For example, suitable assays may be found in PCT International Publication Number WO 03/027246. Examples of suitable assays include the following:
(i) Receptor autophosphorylation (as described by Denley *et al.,* 2004). R⁻ IR-A, R⁻IR-B cells or P6 cells are plated in a Falcon 96 well flat bottom plate at 2.5 x 10⁴ cells/well and grown overnight at 37°C, 5% CO₂. Cells are washed for 4 hours in serum-free medium before treating with one of either insulin, IGF-I or IGF-II in 100µl DMEM with 1% BSA for 10 minutes at 37°C, 5% CO₂. Lysis buffer containing 2mM Na₃VO₄ and 1 mg/ml NaF is added to cells and receptors from lysates are captured on 96 well plates precoated with antibody 83-7 or 24-31 and blocked with 1x TBST/0.5% BSA. After overnight incubation at 4°C, the plates are washed with 1 x TBST. Phosphorylated receptor is detected with europium-labelled antiphosphotyrosine antibody PY20 (130 ng/well, room temperature, 2 hours). DELFIA enhancement solution (100 µl/well) is added and time resolved fluorescence detected as described above.
(ii) Glucose uptake using 2-deoxy-[U-14C] glucose (as described by Olefsky, 1978). Adipocytes between days 8-12 post-differentiation in 24-well plates are washed twice in Krebs-Ringer Bicarbonate Buffer (25mM HEPES, pH 7.4 containing 130 mM NaCl, 5 mM KCl, KH₂PO₄, 1.3 mM MgSO₄.7H₂O, 25 mM NaHCO₃ and 1.15 mM CaCl2) supplemented with 1% (w/v) RIA-grade BSA and 2 mM sodium pyruvate. Adipocytes are equilibrated for 90 min at 37°C prior to insulin addition, or for 30 min prior to agonist or antagonist addition. Insulin (Actrapid, Novogen) is added over a concentration range of 0.7 to 70 nM for 30 min at 37°C. Agonist or antagonist (0 to 500 mM) is added to adipocytes for 90 min followed by the addition of insulin in the case of antagonists. Uptake of 50 mM 2-deoxy glucose and 0.5 mCi 2-deoxy-[U-¹⁴C] glucose (NEN, PerkinElmer Life Sciences) per well is measured over the final 10 min of agonist stimulation by scintillation counting.
(iii) Glucose transporter GLUT4 translocation using plasma membrane lawns (as described by Robinson & James (1992) and Marsh *et al.* (1995)).
(iv) GLUT4 translocation using plasma membrane lawns (as described by Marsh *et al.,* 1995). 3T3-L1 fibroblasts are grown on glass coverslips in 6-well plates and differentiated into adipocytes. After 8-12 days post-differentiation, adipocytes are serum-starved for 18 hrs in DMEM containing 0.5% FBS. Cells are washed twice in Krebs-Ringer Bicarbonate Buffer, pH 7.4 and equilibrated for 90 min at 37°C prior to insulin (100nM) addition, or for 30 min prior to compound (100µM) addition. After treatments, adipocytes are washed in 0.5 mg/ml poly-L-lysine in PBS, shocked hypotonically by three washes in 1:3 (v/v) membrane buffer (30 mM HEPES, pH 7.2 containing 70 mM KCl, 5 mM MgCl₂, 3 mM EGTA and freshly added 1 mM DTT and 2 mM PMSF) on ice. The washed cells are then sonicated using a probe sonicator (Microson) at setting 0 in 1:1 (v/v) membrane buffer on ice, to generate a lawn of plasma membrane fragments that remain attached to the coverslip. The fragments are fixed in 2% (w/v) paraformaldehyde in membrane buffer for 20 min at 22°C and the fixative quenched by 100 mM glycine in PBS. The plasma membrane fragments are then blocked in 1% (w/v) Blotto in membrane buffer for 60 min at 22°C and immunolabelled with an in-house rabbit affinity purified anti-GLUT4 polyclonal antibody (clone R10, generated against a peptide encompassing the C-terminal 19 amino acids of GLUT4) and Alexa 488 goat anti-rabbit secondary antibody (Molecular Probes; 1:200). Coverslips are mounted onto slides using FluoroSave reagent (Calbiochem), and imaged using an OptiScan confocal laser scanning immunofluoroscence microscope (Optiscan, VIC., Australia). Data are analysed using ImageJ (NIH) imaging software. At least six fields are examined within each experiment for each condition, and the confocal microscope gain settings over the period of experiments are maintained to minimise between-experiment variability.

Insulin agonist activity may be determined using an adipocyte assay. Insulin increases uptake of ³H glucose into adipocytes and its conversion into lipid. Incorporation of ³H into a lipid phase is determined by partitioning of lipid phase into a scintillant mixture, which excludes water-soluble ³H products. The effect of compounds on the incorporation of ³H glucose at a sub-maximal insulin dose is determined, and the results expressed as increase relative to full insulin response. The method is adapted from Moody *et al.,* (1974). Mouse epididymal fat pads are dissected out, minced into digestion buffer (Krebs-Ringer 25 mM HEPES, 4% HSA, 1.1 mM glucose, 0.4 mg/ml Collagenase Type 1, pH 7.4), and digested for up to 1.5 hours at 36.5 C. After filtration, washing (Krebs-Ringer HEPES, 1% HSA) and resuspension in assay buffer (Krebs-Ringer HEPES, 1% HSA), free fat cells are pipetted into 96-well Picoplates containing test solution and approximately an ED₂₀ insulin.

The assay is started by addition of ³H glucose (e.g. ex. Amersham TRK 239), in a final concentration of 0.45 mM glucose. The assay is incubated for 2 hours at 36.5 °C, in a Labshaker incubation tower, 400 rpm, then terminated by the addition of Permablend/Toluene scintillant (or equivalent), and the plates sealed before standing for at least 1 hour and detection in a Packard Top Counter or equivalent. A full insulin standard curve (8 dose) is run as control on each plate.

Data are presented graphically, as the effect of the compound on an (approximate) ED₂₀ insulin response, with data normalized to a full insulin response. The assay can also be run at basal or maximal insulin concentration.

To test the *in vivo* activity of a compound, an intravenous blood glucose test may be carried out on Wistar rats as follows. Male Mol:Wistar rats, weighing about 300 g, are divided into two groups. A 10 µl sample of blood is taken from the tail vein for determination of blood glucose concentration. The rats are then anaesthetized (e.g. with Hypnorm/Dormicum) at t =30 min and blood glucose measured again at t =-20 min and at t = 0 min. After the t = 0 sample is taken, the rats are injected into the tail vein with vehicle or test substance in an isotonic aqueous buffer at a concentration corresponding to a 1ml/kg volume of injection. Blood glucose is measured at times 10, 20, 30, 40, 60, 80, 120 and 1 80 min. The anaesthetic administration is repeated at 20 min intervals.

Additional assays to determine the effect of binding molecules on IGF-1R activity are as follows:
(i) Cell Viability Assay on HT29 cells with induction of Apoptosis: The ability of compounds to inhibit IGF-mediated rescue from apoptosis is measured using the colorectal cell line HT29 cells (ATCC: HTB 38) after induction with Na Butyrate. The HT29 cells are plated out onto white Fluoronunc 96 well plates (Nunc) at 12,000cells/ml and incubated at 37°C, 5% CO₂ for 48 hours. Media is aspirated and 100µl/well of serum free DMEM/F12 is added for 2 hours to serum starve cells. IGF (100µl/ well 0.05-50 nM dilutions) in the presence and the absence of inhibitory compound is added in 0.1% BSA solution (Sigma) in DMEM/F12 (Gibco) in triplicate. A final concentration of 5mM Butyrate (Sigma) is added to each well. Plates are incubated at 37°C, 5% CO₂ for a further 48 hours. Plates are brought to room temperature and developed (as per instructions for CTG Assay (Promega)). Luminescence signal is measured on the Polarstar plate reader and data is evaluated using table curve to obtain the specific ED50.
(ii) Cell Migration Assay: The migration assays are performed in the modified 96-well Boyden chamber (Neuroprobe, Bethesda, MA). An 8 µM polycarbonate filter, which is pre- , soaked in 25 µg/ml of collagen in 10 mM acetic acid overnight at 4 °C, is placed so as to divide the chamber into an upper & lower compartment. Varying concentrations of the IGF-I analogues (25 µl of 0-100 nM) diluted in RPMI (Gibco) with 0.5% BSA (Sigma) tested for their migration inducing ability, are placed in the lower compartment in quadruplicates. The wells of the upper chamber are seeded with 50gl/well of 2x10⁵ SW480 (ATCC:CCL 228) pre-incubated for 30mins/37°C with 1.1µl of 2µM Calcein (Molecular Probes). Cells migrate for 8 hours at 37°C, 5% CO₂. Unmigrated cells are removed by wiping the filter. The filter is then analysed in the Polarstar for fluorescence at excitation wavelength of 485nm and emission wavelength of 520nm. Data is evaluated using table curve to obtain the specific ED50 value.
(iii) Mouse Xenograft studies for anti-IGF-1R antibodies: *In vivo* studies are performed in 56-week-old female athymic BALBc nude mice, homozygous for the nunu allele. Mice are maintained in autoclaved micro-isolator cages housed in a positive pressure containment rack (Thoren Caging Systems Inc., Hazelton, PA, USA. To establish xenografts, mice are injected subcutaneously into the left inguinal mammary line with 3 x 10⁶ or 5 x 10⁶ cells in 100 µl of PBS. Tumour volume (TV) is calculated by the formula (length x width²)/2 (Clarke *et al.,* 2000), where length is the longest axis and width the measurement at right angles to length.

Initial biodistribution of potential binding molecules are ascertained by injecting 40 BALBc nude mice with established xenografts with radiolabelled ¹¹¹In- or ¹²⁵I-anti-IGFR antibody (3 µg, 10 µCi) intravenously via the tail vein (total volume=0.1 ml). At designated time points after injection of the radioconjugates (*t*=4 h, days 1, 2, 3, 5 and 7), groups of mice (*n*=35) are killed by Ethrane anaesthesia. Mice are then exsanguinated by cardiac puncture, and tumours and organs (liver, spleen, kidney, muscle, skin, bone (femur), lungs, heart, stomach, brain, small bowel, tail and colon) are resected immediately. All samples are counted in a dual gamma scintillation counter (Packard Instruments). Triplicate standards prepared from the injected material are counted at each time point with tissue and tumour samples enabling calculations to be corrected for the physical decay of the isotopes. The tissue distribution data are calculated as the mean ± s.d. percent injected dose per gram tissue (%ID g⁻¹) for the candidate molecule per time point.

Pharmacokinetics for the candidate compounds are ascertained as follows: Serum obtained from mice bearing xenografts, following infusion of radiolabelled-binding molecule as described above, is aliquoted in duplicate and counted in a gamma scintillation counter (Packard Instruments, Melbourne, Australia). Triplicate standards prepared from the injected material are counted at each time point with serum samples to enable calculations to be corrected for the isotope physical decay. The results of the serum are expressed as % injected dose per litre (% ID 1⁻¹). Pharmacokinetic calculations are performed of serum data using a curve fitting program (WinNonlin, Pharsight Co., Mountain View, CA, USA). A two-compartment model is used to calculate serum pharmacokinetic parameters of AUC (area under the serum concentration curve extrapolated to infinite time), CL (total serum clearance), T_{12α} and T_{12β} (half-lives of the initial and terminal phases of disposition) for ¹²⁵I- and ¹¹¹In-labelled molecule
(iv) Therapeutic *in vivo* studies: Tumour cells (3 x 10⁶) in 100 µl of media are inoculated subcutaneously into both flanks of 46-week-old female nude mice (*n*=5 group⁻¹). Candidate molecule treatment commences day 7 post-tumour cell inoculations (mean ± s.e. tumour volume=60x15 mm³) and consists of six intraperitoneal injections over 2 weeks of appropriate amounts of the candidate molecule or vehicle control. Tumour volume in mm³ is determined as described previously. Data is expressed as mean tumour volume for each treatment group. Differences in tumour size between control and test groups are tested for statistical significance (*P*<0.05) by *t*-test.

### Uses of compounds

Compounds/chemical entities designed or selected by the methods of the invention described above may be used to modulate IR and/or IGF-1R activity in cells, i.e., activate or inhibit IR and/or IGF-1R activity. Such compounds may interact with the low affinity binding sites of IR as defined herein, mimic the αCT segment of the α-chain of IR, or mimic insulin in complex with the IR as defined herein. They may also be used to modulate homodimerisation of IR and/or IGF-1R.

Modulation of homodimerisation of IR and/or IGF-1R may be achieved by direct binding of the chemical entity to a homodimerisation surface of IR and/or IGF-1R, and/or by an allosteric interaction elsewhere in the IR and/or IGF-1R extracellular domain.

Given that aberrant IR and/or IGF-1R activity is implicated in a range of disorders, the compounds described above may also be used to treat, ameliorate or prevent disorders characterised by abnormal IR and/or IGF-1R signalling. Examples of such disorders include malignant conditions including tumours of the brain, head and neck, prostate, ovary, breast, cervix, lung, pancreas and colon; and melanoma, rhabdomyosarcoma, mesothelioma, squamous carcinomas of the skin and glioblastoma.

The compounds designed to interact or identified as interacting with the extracellular domain of IR and/or IGF-1R, and in particular to interact with the target binding sites, are useful as agonists or antagonists against the action of insulin on IR and/or IGF on IGF-1R. The compounds are useful as assay reagents for identifying other useful ligands by, for example, competition assays, as research tools for further analysis of IR and/or IGF-1R and as potential therapeutics in pharmaceutical compositions.

Compounds described herein are also useful as lead compounds for identifying other more potent or selective compounds. The mimetic compounds are also potentially useful as inhibitors of the action of insulin and in the design of assay kits directed at identifying compounds capable of binding to the low affinity binding site for insulin on IR. The mimetic compounds are also potentially useful as inhibitors of the action of IGF and in the design of assay kits directed at identifying compounds capable of binding to the low affinity binding site for IGF on IGF-1R. In particular, it is envisaged that compounds will prove particularly useful in selecting/designing ligands which are specific for IR or IGF-1R.

In one embodiment, one or more of the compounds can be provided as components in a kit for identifying other ligands (e.g., small, organic molecules) that bind to IR or IGF-1R. Such kits may also comprise IR or IGF-1R, or functional fragments thereof. The compound and receptor components of the kit may be labelled (e.g., by radioisotopes, fluorescent molecules, chemiluminescent molecules, enzymes or other labels), or may be unlabeled and labelling reagents may be provided. The kits may also contain peripheral reagents such as buffers, stabilizers, etc. Instructions for use can also be provided.

IR and IGF-1R agonists and antagonists, and in particular antagonists, are potentially useful as therapeutics. For example, compounds are potentially useful as treatments for cancers, including, but not limited to, breast, prostate, colorectal, and ovarian cancers. Human and breast cancers are responsible for over 40,000 deaths per year, as present treatments such as surgery, chemotherapy, radiation therapy, and immunotherapy show limited success. Recent reports have shown that a previously identified IGF-1R antagonist can suppress retinal neovascularization, which causes diabetic retinopathy (Smith *et al.,* 1999). IGF-1R agonist compounds (i.e. existing IGF-1R compounds which have been modified employing methods of the present invention) are useful for development as treatments for neurological disorders, including stroke and diabetic neuropathy. Reports of several different groups implicate IGF-1R in the reduction of global brain ischemia, and support the use of IGF-I for the treatment of diabetic neuropathy (reviewed in Auer *et al.,* 1998; Apfel, 1999). A number of therapeutics directed against IGF-1R are currently undergoing clinical trial as anticancer agents (Hewish *et al.,* 2009).

The IGF-1R agonist peptides described herein may be useful for enhancing the survival of cells and/or blocking apoptosis in cells.

### Administration

Compounds described herein, i.e., ligands or modulators of IR and/or IGF-1R identified or identifiable by the screening methods of the invention, may preferably be combined with various components to produce compositions. Preferably the compositions are combined with a pharmaceutically acceptable carrier or diluent to produce a pharmaceutical composition (which may be for human or animal use).

The formulation will depend upon the nature of the compound and the route of administration but typically they can be formulated for topical, parenteral, intramuscular, oral, intravenous, intra-peritoneal, intranasal inhalation, lung inhalation, intradermal or intra-articular administration. The compound may be used in an injectable form. It may therefore be mixed with any vehicle which is pharmaceutically acceptable for an injectable formulation, preferably for a direct injection at the site to be treated, although it may be administered systemically.

The pharmaceutically acceptable carrier or diluent may be, for example, sterile isotonic saline solutions, or other isotonic solutions such as phosphate-buffered saline. The compounds may be admixed with any suitable binder(s), lubricant(s), suspending agent(s), coating agent(s), solubilising agent(s). It is also preferred to formulate the compound in an orally active form.

In general, a therapeutically effective daily oral or intravenous dose of the compounds, including compounds and their salts, is likely to range from 0.01 to 50 mg/kg body weight of the subject to be treated, preferably 0.1 to 20 mg/kg. The compounds and their salts may also be administered by intravenous infusion, at a dose which is likely to range from 0.001-10 mg/kg/hr.

Tablets or capsules of the compounds may be administered singly or two or more at a time, as appropriate. It is also possible to administer the compounds in sustained release formulations.

Typically, the physician will determine the actual dosage which will be most suitable for an individual patient and it will vary with the age, weight and response of the particular patient. The above dosages are exemplary of the average case. There can, of course, be individual instances where higher or lower dosage ranges are merited.

For some applications, preferably the compositions are administered orally in the form of tablets containing excipients such as starch or lactose, or in capsules or ovules either alone or in admixture with excipients, or in the form of elixirs, solutions or suspensions containing flavouring or colouring agents.

The compositions (as well as the compounds alone) can also be injected parenterally, for example intravenously, intramuscularly or subcutaneously. In this case, the compositions will comprise a suitable carrier or diluent.

For parenteral administration, the compositions are best used in the form of a sterile aqueous solution which may contain other substances, for example enough salts or monosaccharides to make the solution isotonic with blood.

For buccal or sublingual administration the compositions may be administered in the form of tablets or lozenges which can be formulated in a conventional manner.

For oral, parenteral, buccal and sublingual administration to subjects (such as patients), the daily dosage level of the compounds and their pharmaceutically acceptable salts and solvates may typically be from 10 to 500 mg (in single or divided doses). Thus, and by way of example, tablets or capsules may contain from 5 to 100 mg of active compound for administration singly, or two or more at a time, as appropriate. As indicated above, the physician will determine the actual dosage which will be most suitable for an individual patient and it will vary with the age, weight and response of the particular patient.

The routes of administration and dosages described are intended only as a guide since a skilled practitioner will be able to determine readily the optimum route of administration and dosage for any particular patient depending on, for example, the age, weight and condition of the patient.

### EXAMPLES

### EXPERIMENTAL PROCEDURES

### Crystallisation, Structure Solution and Refinement of IR310.T in complex with Insulin

### Construction of the cIR485 expression vector

A 1741-base fragment of IR was synthesized (DNA2.0) and inserted into the HindIII/XbaI sites of the mammalian expression plasmid vector pEE14 (Bebbington & Hentschel, 1987). The fragment encoded a protein cIR485 ("cleavable" IR485; SEQ ID NO: 15) which consists, in order, of (i) the 27-residue IR native signal sequence, (ii) residues 1-310 of the IR α-chain (the L1-CR module), (iii) a thrombin cleavage site SSSLVPRGSSS, (iv) residues 311-485 of the IR α-chain (the L2 domain), (v) an enterokinase cleavage site DDDDK and (vi) a c-myc purification tag EQKLISEEDLN (Hoogenboom et al., 1991). A 106-base non-coding fragment
GTCGACGGTACCCCGGGGAATTAATTCCGGGGGCCGCCTCGGAGCATGACCCCC GCGGGCCAGCGCCGCGCGCTCTGATCCGAGGAGACCCCGCGCTCCCGCAGCC was included between the Hind III site and the start codon; bases 29-106 of this fragment corresponding to those immediately upstream of the human IR coding region.

### Cell culture and transfection

The expression plasmid pEE14/cIR485 was transfected into Lec8 mutant Chinese hamster ovary (CHO) cells (CRL-1737; ATCC) (Stanley, 1989) using LipofectAMINE 2000 (Life Technologies). The Lec8 cells were maintained in Dulbecco's Modified Eagle Medium: Nutrient Mixture F-12 (DMEM / F12; Life Technologies) containing 10% fetal bovine serum (FBS; Life Technologies). Transfected Lec8 cells were maintained in DMEM (High Glucose, Pyruvate, no Glutamine) plus 1x glutamine synthetase supplements (Sigma-Aldrich) and 10% dialyzed FBS (dFBS) containing 25 mM methionine sulphoximine (MSX, Sigma-Aldrich).

Transfectants from 96-well plates were screened for protein expression by Western blot with monoclonal antibodies 83-7 (Soos et al., 1986) and 9E10 (Evan et al., 1985). A high-yielding cell line was selected by screening a high number of transfectants.

### Protein production and purification

The Lec8 host cell line containing the cIR485 construct was sub-cultured in tissue culture flasks initially and then in roller bottles (Greiner) for two weeks to prepare inocula for the spinner flasks. DMEM / F12 media supplemented with 10% dFBS and 25 µM MSX to maintain selection pressure was used during passaging and production work.

Cells from ten fully-confluent roller bottles were transferred to four spinner flasks (New Brunswick Scientific) containing Fibra-Cel disks (New Brunswick Scientific) to provide a matrix for cell attachment. Approximately 35 × 10⁷ viable cells / spinner flask were used to initiate production. The spinners were incubated in 5% CO₂ at 37°C with 100 rpm stirring and ∼50 ml / min airflow. Spinner flasks were supplemented with 0.1% FoamAway (Life Technologies). Glucose and lactic acid levels were monitored daily, with spent media replaced to maintain residual glucose levels of ∼1.6 g/L.

The production phase lasted three weeks and ∼50 L of harvest collected, with the media being supplemented with 0.8 mM butyric acid (Sigma-Aldrich) during the last week.

Daily harvests from the spinners were kept at 4° C after addition of 0.02% NaN₃ and 0.1 mM phenylmethanesulfonylfluoride (PMSF; Sigma-Aldrich). Pooled harvest batches were filtered with a 1.2 / 0.2µ filter to remover cell debris and concentrated approximately 10× using a 30 kDa cut-off membrane. All of the large-scale mammalian cell culture was performed under contract by CSIRO (Parkville, Australia).

Initial purification of cIR485 was by 9E10 antibody affinity chromatography and size-exclusion chromatography (SEC) using procedures effectively identical to those described for purification of IR485 (Lou et al., 2006).

Purified cIR485 in Tris-buffered saline (TBS; 24.8 mM Tris-HCl pH 8.0, 137 mM NaCl, 2.7 mM KCl) plus 0.02% sodium azide (TBSA) was then incubated overnight at 37 °C with 0.25 units human thrombin (Roche) per mg of cIR485 in the presence of 10 mM CaCl₂.

Completion of proteolysis was assessed by SDS-PAGE, which revealed bands corresponding to the estimated molecular weight of the IR310.T fragment and the IR L2 domain (51 kDa and 30 kDa, respectively; Figure 5A).

Western blotting with Mab 83-7 (Soos et al., 1986) confirmed that the CR domain was contained in the upper band alone. The sample was diluted 8-fold in buffer A (10 mM ethanolamine-HCl, pH 9.6 + 0.02% sodium azide), centrifuged for 5 min at 17,000 g to remove particulates and then loaded onto MonoQ 5/50 GL column (GE Healthcare). The sample was eluted with a 60 column-volume gradient of buffer A to buffer B (10 mM ethanolamine-HCl, 400 mM NaCl, pH 9.6 + 0.02% sodium azide; Figure 5B) and the fractions assessed by SDS-PAGE (Figure 5A).

Fractions containing IR310.T (SEQ ID NO: 8) were pooled, concentrated and rerun in TBSA on a Superdex 200 10/300 column (GE Healthcare). The chromatogram exhibited three overlapping peaks, likely arising from multimerization (Figure 5C).

SDS-PAGE of fractions revealed the presence of three closely-spaced bands, which we attributed to varying glycosylation (Figure 5D). Fractions containing IR310.T were pooled, concentrated and buffer exchanged into either TBSA or 10 mM HEPES-NaOH (pH 7.5) + 0.02% NaN₃.

### Mab 83-7 production and purification

A hybridoma cell line expressing Mab 83-7 (a gift from Prof. Ken Siddle, University of Cambridge, UK; SEQ ID NOs: 11 and 12) was then grown in large scale using Gibco Hybridoma Serum-free medium (H-SFM). Cells lines originally required 7-10% serum for optimal growth and 30-60 mg/l final yield.

A brief adaption experiment to lower serum levels was unsuccessful, at lower than 5% serum levels both viability and yield dropped.

The cells were then grown in 950 cm² roller bottles at 37°C, 5% CO₂ balanced with air and 10 rpm using approximately 300 ml H-SFM media supplemented with 5% fetal calf serum. Typically, 2-3 litres of cell culture at the viable cell density in the range of 2-3*10⁶ cells/ml was achieved. The cell culture was then pelleted (5 mins, 350 g) and resuspended in serum-free medium. When cell viability dropped below 30% (after 5-7 days), the culture was harvested and the Mab 83-7 captured using a Protein-A column (Millipore). Under these conditions final yield was between 5-12 mg/l of cell culture medium.

### Fab 83-7production and purification

Production and purification of Fab 83-7 (SEQ ID NOs: 13 and 14) from Mab 83-7 was based on protocols described previously (McKern et al., 2006). Briefly, Mab 83-7 was digested with dithiothreitol-activated papain (Sigma-Aldrich) at 37 °C. The digestion was stopped by adding iodoacetamide (IAA; Sigma-Aldrich), and the reaction mixture passed down a Superdex 200 26/60 column (GE Healthcare). Fractions containing 83-7 F(ab')₂ were isolated and reduced with mercaptoethylamine (Sigma-Aldrich) and then alkylated with IAA, followed by further SEC and anion-exchange chromatography on Mono S (GE Healthcare).

### IR310.T/Fab 83-7purification

IR310.T was mixed with a slight molar excess of Fab 83-7 and the complex purified by SEC using a S200 10 / 300 column (GE Healthcare) in TBSA buffer. Fractions containing the complex of IR310.T / Fab 83-7 were then pooled, concentrated and exchanged into 10 mM HEPES-NaOH (pH 7.5) + 0.02% NaN₃.

### Crystallization

Samples of the IR310.T/Fab 83-7 complex were prepared for crystallization by combining with a 1.5x molar ratio of αCT⁷⁰⁴⁻⁷¹⁹ (Genscript; SEQ ID NO: 9) and 3x molar ratio of human insulin (Sigma-Aldrich) to a final concentration of 3.5 mg/ml in 10 mM HEPES, pH 7.0. [*D*-Pro^{B26}]-DTI-NH₂ was prepared as described previously (Zakova et al., 2008).

A single crystallization condition (1.0 M trisodium citrate, 0.1 M imidazole HCl, pH 8.0 + 0.02% NaN₃) was detected for the IR310.T/Fab83-7/[*D*-Pro^{B26}]-DTI-NH₂/αCT⁷⁰⁴⁻⁷¹⁹ complex in a large (792-condition) sparse matrix sitting-drop screen set up in SBS-format sitting-drop plates, performed under contract by the CSIRO Collaborative Crystallization Centre (Parkville, Australia). This condition was refined manually for both [*D*-ProB²⁶]-DTI-NH₂ and human insulin complexes within a range of 0.9 -1.1 M trisodium citrate, 0.1 M imidazole HCl, pH 8.0 + 0.02% NaN₃ in hanging-drop plates. Crystals grew to a maximum size of 200-250 µm.

### Diffraction data collection

Single crystals of the human insulin IR310.T quaternary complex were mounted in cryo-loops in paraffin oil HR403 (Hampton Research) and flash frozen in liquid nitrogen. Data Set 1 for IR310.T human insulin quaternary complex was collected (McPhillips et al., 2002) at the MX2 beamline at the Australian Synchrotron (Melbourne, Australia) using a Quantum 315 detector (ADSC). Data Set 2 was collected in similar fashion from a further crystal at beamline 124 at the Diamond Light Source (Oxford, UK) using a Pilatus 6M detector (Dectris).

Crystals were transferred to reservoir solutions supplemented with 20% glycerol, flash frozen in liquid nitrogen and mounted directly on the MX2 beamline at the Australian Synchrotron. A number of crystals of 50-200 µm size were obtained, but only one diffracted to 4.4 Å, with the remainder diffracting to ∼6.0 Å Diffraction data were collected (McPhillips et al., 2002) from multiple volumes within the crystal in an endeavour to reduce the effects of radiation damage; however, only 88% completeness was achieved.

All diffraction data were processed using the XDS suite (Kabsch, 2010) and TRUNCATE within the CCP4 suite. Precision-indicating merging *R* factors (*R*ₚᵢₘ) were computed using SCALA within the CCP4 suite. Statistics for all data sets are provided in Table 2.

**Table 2: X-ray data processing and refinement**

| | Data set 1 | Data set 2 | Data set 3 |
|---|---|---|---|
| X-ray source | Australian Synchrotron | Diamond Light Source | (combined Data sets 1+2) |
| No. of frames | 141 | 180 | |
| Oscillation range (°) | 1.0 | 0.2 | |
| Exposure / frame (s) | 3-15 | 0.2 | |
| Unit cell dimensions | *α* = 168.15 Å | *α* = 168.91 Å | |
| Space group | P23 | P23 | |
| Solvent content (%) | 78 | 78 | |
| Data completeness (%) | 99.8 (98.9)¹ | 98.4 (99.6) | 99.8 (99.7) |
| Resolution (A) | 59.5 - 4.0 (4.1 - 4.0) | 46.8 - 3.9 (4.0 - 3.9) | 59.5 - 3.5 (3.6 - 3.5) |
| *R*_{merge} | 0.156 (2.33)² | 0.089 (1.32) | 0.216 (8.71) |
| *CC*^{(1/2)} | 0.998 (0.580)³ | 0.998 (0.342) | 0.998 (0.177) |
| *<I*/*s(I)>* | 11.46 (1.6) | 8.95 (1.0) | 9.32 (0.4) |
| Redundancy | 17.3 (17.4) | 4.1 (4.2) | 23.8 (20.7) |
| | | | |
| Protein atoms | | 4466 | 4466 |
| Carbohydrate atoms | | 131 | 131 |
| *R*_{work} | | 0.265⁴ | 0.258 |
| *R*_{free} (5% of refls) | | 0.293⁴ | 0.279 |
| *s*_{bond} (Å) | | 0.011 | 0.010 |
| *s*_{angle} (°) | | 1.4 | 1.4 |
| Ramachandran plot: | | | |
| favoured region (%) | | 90.9 | 90.8 |
| acceptable region (%) | | 5.1 | 5.8 |
| outliers (%) | | 4.0 | 3.8 |

| | | | |
|---|---|---|---|
| ¹ Numbers in parentheses refer to the statistic in the highest resolution shell. ² *R*_{merge}=\|*hxi i*-*Iᵢ*(*hkl*)-*<I*(*hkl*)*>*\|/*hkl i Iᵢ*(*hkl*) ³ *CC*^{(1/2)} is the Pearson correlation coefficient between independently merged halves of the data set. Values for the highest resolution shell are significant at p=0.001 and justify the use of data to the stated resolution (Karplus, 2012). ⁴ *R*_{work} and *R*_{free} are computed using *R* = <\|*F*ₕ^{xpct} - *F*ₕ*^{obs}*\|> / <\|*F*ₕ^{obs}\|> (where *F*ₕ^{xpct} is the expectation value of the model structure amplitude; Blanc et al., 2004). | | | |

### Structure solution and refinement

Molecular replacement employed Data Set 1 (Table 2). A single copy of an L1-CR search fragment (residues 4-310, from PDB entry 3LOH) was located within the asymmetric unit using PHASER (TFZ=26.7) (McCoy, 2007). The variable module of Fab 83-7 (Fv, from PDB entry 3LOH) was then located in the presence of the L1-CR module (TFZ=37.6). Attempts to locate the Fab 83-7 constant module failed and it proved disordered. TLS parameters and individual isotropic B-factors for the L1-CR/Fv complex were then refined using autoBUSTER (Bricogne et al., 2011), followed by a subsequent atomic coordinate-only refinement, yielding *R*^{xpct}_{work}/*R*^{xpct}_{free} = 0.368/0.363, where "xpct" denotes the statistic's expectation value (Blanc et al., 2004). The resultant difference density maps revealed tubular helix-like features (the 1^{st}, 3^{rd}, 4^{th,} 5^{th} and 7^{th} highest peaks within the map) in the immediate vicinity of the L1-β₂ surface, into which PHASER unambiguously positioned a rigid model comprising the three insulin helices (TFZ=11.4). The correctness of this solution was confirmed by an exhaustive 6-dimensional real-space search using ESSENS (Kleywegt and Jones, 1997) to locate the core fragment within the *F*ₒ-*F*_{c} map. The ESSENS search, refined on a (1°, 0.33 Å) grid, yielded a single solution, with a Z-score of 12.6 and an rmsd of 0.7 Å from that obtained by PHASER. The 2^{nd} highest difference electron density feature corresponded to glycan attached to Asn111 (Figure 6A), configured effectively identically to its counterpart in the structure of uncomplexed IR485 (Lou et al., 2006). The L1-CR/Fv/insulin core model was then refined further, yielding *R*^{xpct}_{work}/*R*^{xpct}_{free} = 0.320/0.339.

The remaining helix-like feature in the above difference map (encompassing the 1^{st} and 5^{th} highest peaks) revealed clear side chain protrusions upon B-factor map sharpening, spaced consistent with an underlying α-helix (Figure 6B). We concluded that the feature arose from the αCT peptide. A 10-residue polyalanine α-helix was docked into this feature using an ESSENS search with all constituent atoms contributing to the target function to allow discernment of helix direction. The best fit had Z-score of 7.3 and was adequately discriminated from lower-scoring fits. Visual inspection confirmed the overall correctness of the fit to the density and, in particular, the direction of the helix as judged by protruding side-chain density.

Register assignment of the αCT⁷⁰⁴⁻⁷¹⁹ sequence to the decameric polyalanine helix was achieved using a procedure that assessed both compatibility of individual residue side chains with difference electron density and compatibility of individual residue side chains with their surrounding protein environment. (a) Fit to difference density employed the following method that was designed not only to assess the fit of atoms within the density, but also to penalize the existence of volumes of positive difference density into which no atoms had been placed. Briefly, the map feature assigned to the αCT segment was excised from the B-factor sharpened map using CHIMERA (Pettersen et al., 2004) (cut-off level = 0.16 e⁻/A³) and placed within a rectangular grid volume large enough to allow a >8 Å buffer zone around the αCT feature. All grid points outside of the feature were set to 0 e/Å³, with the resultant map being termed *M*_{obs}. Coordinates for residues 1, 2 and 10 of the fitted polyalanine helix were then deleted from the model, as the density associated with them displayed somewhat poorer α-helical geometry. Ten comparisons corresponding to all possible alignments of heptameric sequences from αCT⁷⁰⁴⁻⁷¹⁹ with the heptameric polyalanine structure were considered. Rotamers for each residue within each of these ten models were then selected manually using COOT (Emsley and Cowtan, 2004) based on visual inspection of the fit of the trial rotamer to the density at the corresponding site in *M*_{obs}. The ten individual models were then "real-space-refined" within COOT to achieve optimal fit to *M*_{obs}, maintaining tight helical restraints. An electron density map *M*_{calc}, on the same grid as *M*_{obs} was then generated (using SFALL within CCP4) for each heptamer model in isolation, with the B-factors of all main-chain atoms being set to 10 Å² and of all side-chain atoms set to 20 Å² to allow for subsequent comparison with a sharpened map. All density within *M*_{calc} lower than 0.45 e⁻/A³ was set to 0 e⁻/A³. Correlation coefficients CC=(<xy> - <x><y>)/√ [(<x²> - <x>²) (<y²>-<y>²)] between *M*_{obs} and each *M*_{calc} were then calculated using MAPMAN (Jones and Thirup, 1986). Each CC was then "normalized" by dividing its value by the CC value calculated for the underlying polyalanine heptamer, and the quotient termed the trial sequence's "density score". Trial register 705-711 was seen to have the highest density score (Table 3). (b) Compatibility of residue side-chain environment within the putative L1/αCT/insulin interface was assessed as follows. For each of the ten trial heptamer (real-space refined) models described above, an environment score was generated using VERIFY3D (Luthy et al., 1992) in order to assess compatibility with the surrounding L1-β₂ and insulin surfaces. Trial register 705-711 again scored highest (Table 3). A "combined score" was then computed as the product (density score) x (environment score) in order to assist with assessing lower ranked trial registers (Table 3). The next highest combined score was for trial register 709-715, which is related trial register 705-711 by a one-turn translation along the helix, effectively maintaining hydrophobic-to-hydrophobic docking with the L1 surface. However, register 709-715 was judged to be most unlikely, as it would bring Pro716 and Pro718 into the remaining C-terminal part of the overall helix. We thus concluded that register assignment 705-711 was correct, given that it was the highest ranking on all criteria. The assignment was subsequently confirmed by the structure determination of the insulin-complexed IR593.αCT construct, which has the segment directly and covalently attached to the C-terminus of FnIII-1.

**Table 3: Density and environmental fit scores for various trial αCT registers, indicating the consistently high score assigned to register 705-711.**

| IR αCT register | Density fit score¹ | Environment fit score² | Combined score³ |
|---|---|---|---|
| 704-710 | 1.13 | 11.3 | 12.8 |
| 705-711 | 1.15⁴ | 18.5 | 21.3 |
| 706-712 | 1.11 | 13.4 | 14.9 |
| 707-713 | 1.13 | 13.8 | 15.6 |
| 708-714 | 1.03 | 15.4 | 15.9 |
| 709-715 | 1.13 | 16.9 | 19.0 |
| 710-716 | 1.06 | 14.4 | 15.2 |
| 711-717 | 1.06 | 14.4 | 15.2 |
| 712-718 | 1.04 | 12.0 | 12.5 |
| 713-719 | 1.04 | 8.19 | 8.5 |

| | | | |
|---|---|---|---|
| ¹ Density score = (Correlation of the computed electron density for the trial IR αCT heptamer with the difference electron density feature shown in Figure 6B) / (Correlation of the computed electron density for the initial polyalanine heptamer with the difference electron density feature shown in Figure 6B). ² Environment fit score computed using VERIFY3D (Luthy et al., 1992). ³ Combined score = (density fit score) x (environment fit score). ⁴ Highest scores within each column are underlined. | | | |

The 705-711 peptide model was then included in the insulin/IR310.T/Fab83-7 atomic model and autoBUSTER refinement continued against Data Set 2, which became available in the interim (Table 2, assigned identical free-R set to Data Set 1). Residues 712-715 were built as a further α-helical turn beyond residue 711 and a total of nine sugars added at sites 16, 25, 111, 225 and 255 (Lou et al., 2006; Sparrow et al., 2008), followed by iterative rounds of refinement and model rebuilding (COOT). LSSR restraints to higher-resolution structures (Smart et al., 2012) were included for IR310.T (restrained to PDB entry 2HR7-A) and the Fab83-7 light chain (to PDB entry 1IL1-B) and heavy chain (to PDB entry 1FNS-C). Final refinement statistics are detailed in Table 2. Inclusion of data to 3.9 Å was justified *α posteriori* - the average figure of merit for free set reflections remained above 0.70 at this limit.

### Extension of resolution to 3.5 Å and model building

Data sets 1 and 2 for the human insulin complexed IR310.T/83-7/αCT⁷⁰⁴⁻⁷¹⁹ crystal were then re-processed to extended resolution of 3.5Å, based on a recent report that additional information could be obtained by inclusion of weak data at the very limit of diffraction. The integrated reflection data from the two sets were then scaled and merged together, the combined data set having *CC*^{(1/2)} = 0.998, significant at the *p*=0.001 level of confidence.

The prior model of human insulin complexed IR310.T/83-7/αCT⁷⁰⁴⁻⁷¹⁹ refined at 3.9Å resolution against Data set 2 was then re-refined against this extended resolution data set using autoBUSTER.

Examination of the resultant difference density maps showed that insulin residue A21 was misplaced in the earlier refinement. This residue was then removed from model and the model re-refined. The resultant difference maps revealed that residues B21-B24 could be built in a near native-like conformation, with the side chain of residue B24 directed into a hydrophobic pocket formed by the side chains of residues Phe714, Phe39, Leu37 and LeuB15. We then observed that residues PheB25 and TyrB26 could be built into difference electron density, in a non-native like fashion with the side chain of residue TyrB25 directed towards the αCT peptide in the vicinity of residue Val715 and the side chain of residue TyrB26 directed towards L1 domain residues Asn12 and Arg19.

Va1715 was then removed from the model to avoid steric clash with TyrB25. We noted that Val715 is replaced by glutamate in some insulin receptors, incompatible with it being directed towards the L1 surface in the fashion prescribed by the lower resolution model and hence it like A21 had been built incorrectly in the lower resolution model.

A number of rounds of model building then followed using autoBUSTER, with the loop B20-B23 restrained to a native-like conformation. The resultant model showed a good fit to the electron density and a reduced *R*/*R*_{free} with respect to the starting structure.

### Example 1: A thermodynamic study of insulin binding to IR310.T

### Introduction

In order to assess insulin binding to IR310.T in the presence of Fab 83.7 isothermal titration calorimetry ("ITC") was used. ITC allowed a direct assay of the interactions between the construct IR310.T (described above) complexed with Fab 83.7 and zinc free human insulin and between the construct IR310.T (not complexed with Fab 83.7) and zinc free human insulin. All interactions were assayed in presence of a 10x molar ratio of αCT⁷⁰⁴⁻⁷¹⁹.

### Reagents

Protocols and instrumentation for ITC determination of the thermodynamic parameters of binding of (a) zinc-free porcine insulin to a pre-formed complex of IR310.T and αCT⁷⁰⁴⁻⁷¹⁹, (b) zinc-free porcine insulin to a pre-formed complex of IR310.T/Fab83-7 and αCT⁷¹⁴⁻⁷¹⁹, (c) alanine-substituted aCT peptide to the L1-CR-L2 construct IR485 and (d) zinc-free porcine insulin to a pre-formed complex of alanine-substituted αCT⁷⁰⁴⁻⁷¹⁹ and IR485 were identical to those described previously (Menting et al., 2009).

Briefly, all experiments were conducted using a VP-ITC isothermal titration calorimeter (MicroCal) with the calorimeter cell held at 25°C. All samples were degassed prior to injection or placement into the cell, and the instrument was temperature-equilibrated prior to the start of the injections. In all experiments, the volume of the sample placed in the cell was 1.4 ml and the titrant was injected in 7 µl volumes over 14 s at 3 min intervals, with the total number of injections being 40. The sample contents were stirred at a speed of 310 rpm over the duration of the titration. For peptide titrations, the titrant was prepared at 80 µM in TBSA, with the sample cell holding IR485 at 10 µM concentration. For insulin and insulin analogue titrations, the titrant was prepared at 32 to 48 µM concentration in TBSA with the sample cell containing IR485 at 4 to 6 µM concentration in TBSA and pre-incubated with a 10x molar ratio of the respective Ala-substituted aCT peptide. Data were analysed using the instrument's software and in all cases fitted as a single-site interaction. All measurements were performed in triplicate.

### Results

ITC analysis showed that zinc-free human insulin bound IR310.T with *K*_{d} = 30 nM in the presence of a 10x molar ratio of αCT⁷⁰⁴⁻⁷¹⁹ (Figure 5E), and that zinc-free human insulin bound Fab 83-7 complexed IR310.T with *K*_{d} = 48 nM in the presence of a 10x molar ratio of αCT⁷⁰⁴⁻⁷¹⁹ (Figure 5F).

### CITATION LIST

Adams, M.J., Blundell, T.L., Dodson, E.J., Dodson, G.G., Vijayan, M., Baker, E.N., Harding, M.M., Hodgkin, D.C., Rimmer, B., and Sheat, S. (1969). Structure of Rhombohedral 2 Zinc Insulin Crystals. Nature 224, 491-495.
Adams et al. (2000) Cell. Mol. Life Sci., 57, 1050-1093.
Apfel (1999) Am. J. Med., 107, 34S-42S.
Auer (1998) Neurology, 51, S39-S43.
Ausubel et al. (1999) Short Protocols in Molecular Biology, 4th Ed, John Wiley & Sons, Inc.; and the full version entitled Current Protocols in Molecular Biology.
Bailyes et al. (1997) Biochem. J., 327, 209-215.
Bartlett et al. (1989) Royal Chem. Soc., 78, 182-196.
Bebbington, C.R. & Hentschel, C.C.G. (1987) The use of vector based on gene amplification for the expression of cloned genes in mammalian cells, in DNA Cloning, ed. Glover, D.M. Academic Press, San Diego. Vol 3, p163.
Belfiore, A. et al. (2009) Endocr Rev., 30, 586-623.
Bentley (1997) Methods Enzymol., 276, 611-619.
Binz et al. (2005) Nature Biotech., 23, 1257-1268.
Blanc, E., Roversi, P., Vonrhein, C., Flensburg, C., Lea, S.M., and Bricogne, G. (2004). Refinement of severely incomplete structures with maximum likelihood in BUSTER-TNT. Acta Crystallogr D Biol Crystallogr 60, 2210-2221.
Blondelle and Houghten (1996) Trends Biotechnol., 14, 60-65.
Bohm and Stahl (1999) M. Med. Chem. Res., 9, 445.
Bricogne, G., Blanc, E., Brandl, M., Flensburg, C., Keller, P., Paciorek, W., Roversi, P., Sharff, A., Smart, O.S., Vonrhein, C., et al. (2011). BUSTER version 2.10, Cambridge, United Kingdom: Global Phasing Ltd.
Brooks et al. (1983) Comp. Chem., 4, 187-217.
[00362] Brünger, A.T. (1996) X-PLOR reference manual 3.851 (Yale Univ., New Haven, CT).
Brünger (1997) Methods Enzymol., 276, 558-580.
Brünger et al. (1998) Acta Cryst. D54, 905-921.
Bruns et al. (1999) J Mol Biol., 288, 427-439.
Buttel et al. (1999) Immunol. Cell Biol., 77, 256-262.
Carell et al. (1994a) Angew.Chem. Int. Ed. Engl., 33, 2059.
Carell et al. (1994b) Angew. Chem. Int. Ed. Engl., 33, 2061.
Chiu, S.L., and Cline, H.T. (2010). Insulin receptor signaling in the development of neuronal structure and function. Neural Dev 5, 7.
Cho et al. (1993) Science, 261, 1303.
Chow et al. (1998) Biol. Chem., 273, 4672-4680.
Clarke et al. (2000) Cancer Res., 60,4804-4811.
Cohen et al. (1990) J. Med. Chem., 33, 883-894.
Cohen, P. (2006). The twentieth century struggle to decipher insulin signalling. Nat Rev Mol Cell Biol 7, 867-873.
Cole et al. (2005) in "Virtual Screening in Drug Discovery (Eds. B. Shoichet, J. Alvarez)", Taylor & Francis CRC Press, Florida, USA.
Cull et al. (1992) Proc. Natl. Acad. Sci. USA, 89, 1865-1869.
Cwirla et al. (1990) Proc. Natl. Acad. Sci. USA, 97, 6378-6382.
Danial et al. (2008) Nat. Med. 14, 144-153.
Davis et al. (2006) Chem. Soc. Rev. 36, 326-334.
Day and Caflisch (2008) J. Chem. Inform. Model. 48, 679-90.
Degterev et al. (2001) Nat. Cell. Biol. 3, 173-182.
De Meyts (1994) Diabetologia, 37, S135-S148.
De Meyts and Whittaker (2002) Nat. Rev. Drug Discov., 1, 769-783.
De Meyts, P. (2004). Insulin and its receptor: structure, function and evolution. Bioessays 26, 1351-1362.
Denley et al. (2003) Horm. Metab. Res., 35, 778-785.
Denley et al. (2004) Mol. Endocrinol., 18, 2502-2512.
Devlin (1990) Science, 249, 404-406.
DeWitt et al. (1993) Proc. Natl. Acad. Sci. USA, 90, 6909.
Emsley, P., and Cowtan, K. (2004). Coot: model-building tools for molecular graphics. Acta Crystallogr D Biol Crystallogr 60, 2126-2132.
Erb et al. (1994) Proc. Natl. Acad. Sci. USA, 91, 11422.
Ernst et al. (2000) J. Magn. Reson. Imaging, 12, 859-865.
Evan, G.I., Lewis, G.K., Ramsay, G., and Bishop, J.M. (1985). Isolation of monoclonal antibodies specific for human c-myc proto-oncogene product. Mol Cell Biol 5, 3610-3616.
Ewing et al. (2001) J. Comput-Aid. Mol. Design, 15,411.
Frasca et al. (1999) Mol Cell Biol, 19, 3278-3288.
Felici (1991) J. Mol. Biol., 222, 301-310.
Fodor (1993) Nature, 364, 555-556.
Friesner et al. (2004) J. Med. Chem. 47, 1739-1749.
Gallop et al. (1994). J. Med. Chem., 37, 1233.
Garrett et al. (1998) Nature, 394, 395-399.
Goodford (1985) J. Med. Chem., 28, 849-857 (1985).
Goodsell and Olsen (1990) Proteins: Struct. Funct. Genet., 8, 195-202.
Guida (1994) Curr. Opin. Struct. Biol., 4, 777-781.
Hewish et al. (2009) Recent Patents Anticancer Drug Discov, 4, 54-72.
Hoogenboom, H.R., Griffiths, A.D., Johnson, K.S., Chiswell, D.J., Hudson, P., and Winter, G. (1991). Multi-subunit proteins on the surface of filamentous phage: methodologies for displaying antibody (Fab) heavy and light chains. Nucleic Acids Res 19, 4133-4137.
Houghten et al. (1991) Nature, 354, 84-86.
Houghten (1992) Biotechniques, 13,412-421.
Hua, Q.X., Shoelson, S.E., Kochoyan, M., and Weiss, M.A. (1991). Receptor binding redefined by a structural switch in a mutant human insulin. Nature 354, 238-241.
Jones, T.A., and Thirup, S. (1986). Using known substructures in protein model building and crystallography. EMBO J 5, 819-822.
Jones et al. (1991) Acta Cryst. A47, 110-119.
Jorgensen and Tirado-Rives (1988) Am. Chem. Soc., 110, 1657-1666.
Kabsch, W. (2010). Integration, scaling, space-group assignment and post-refinement. Acta Crystallogr D Biol Crystallogr 66, 133-144.
Karplus, P.A., and Diederichs, K. (2012). Linking crystallographic model and data quality. Science 336, 1030-1033.
Kiselyov, V.V., Versteyhe, S., Gauguin, L., and De Meyts, P. (2009). Harmonic oscillator model of the insulin and IGF1 receptors' allosteric binding and activation. Molec Sys Biol 5,243.
Kjeldsen, T. et al. (1991) Proc Natl Acad Sci USA 88, 4404-4408.
Kleywegt, G.J., and Jones, T.A. (1997). Template convolution to enhance or detect structural features in macromolecular electron-density maps. Acta Crystallogr D Biol Crystallogr 53, 179-185.
Kristensen, C. et al. (1999). J Biol Chem 274, 37351-37356.
Kristensen, C., Andersen, A.S., Østergaard, S., Hansen, P.H., and Brandt, J. (2002). Functional reconstitution of insulin receptor binding site from non-binding receptor fragments. J Biol Chem 277, 18340-18345.
Kuntz et al. (1982) J. Mol. Biol., 161, 269-288.
Kurose et al. (1994) J. Biol. Chem., 269, 29190-29197.
Lam et al. (1991) Nature, 354, 82-84.
Lam (1997) Anticancer Drug Des., 12, 145.
Lattman (1985) Use of the Rotation and Translation Functions, Meth. Enzymol., 115, 55-77.
Liu et al. (1993) Cell, 75, 59-72.
Lou, M., Garrett, T.P., McKern, N.M., Hoyne, P.A., Epa, V.C., Bentley, J.D., Lovrecz, G.O., Cosgrove, L.J., Frenkel, M.J., and Ward, C.W. (2006). The first three domains of the insulin receptor differ structurally from the insulin-like growth factor 1 receptor in the regions governing ligand specificity. Proc Natl Acad Sci USA 103, 12429-12434.
Luthy, R., Bowie, J.U., and Eisenberg, D. (1992). Assessment of protein models with three-dimensional profiles. Nature 356, 83-85.
Marsh et al. (1995) J. Cell Biol., 130, 1081-1091.
Mayer, J.P., Zhang, F., and DiMarchi, R.D. (2007). Insulin structure and function. Biopolymers 88, 687-713.
McCoy, A.J. (2007). Solving structures of protein complexes by molecular replacement with Phaser. Acta Crystallogr D Biol Crystallogr 63, 32-41.
McKern, N.M., Lawrence, M.C., Streltsov, V.A., Lou, M.Z., Adams, T.E., Lovrecz, G.O., Elleman, T.C., Richards, K.M., Bentley, J.D., Pilling, P.A., et al. (2006). Structure of the insulin receptor ectodomain reveals a folded-over conformation. Nature 443, 218-221.
McPhillips, T.M., McPhillips, S.E., Chiu, H.J., Cohen, A.E., Deacon, A.M., Ellis, P.J., Garman, E., Gonzalez, A., Sauter, N.K., Phizackerley, R.P., et al. (2002). Blu-Ice and the Distributed Control System: software for data acquisition and instrument control at macromolecular crystallography beamlines. J Synchrotron Radiat 9, 401-406.
Moody et al. (1974) Horm. Metab. Res., 6, 12-16.
Morton and Myszka (1998) Methods Enzymol., 295, 268-294.
Nakae, J. et al. (2001) Endocr Rev., 22, 818-835.
Navaza and Saludjian (1997) Methods Enzymol., 276, 581-594.
Navia and Murcko (1992) Curr. Opin. Struct. Biol., 2, 202-210.
Nice and Catimel (1999) Bioessays, 21, 339-352.
Olefsky (1978) Biochem. J., 172, 137-145.
Pettersen, E.F., Goddard, T.D., Huang, C.C., Couch, G.S., Greenblatt, D.M., Meng, E.C., and Ferrin, T.E. (2004). UCSF Chimera--a visualization system for exploratory research and analysis. J Comp Chem 25, 1605-1612.
Pollak, M. (2012). The insulin and insulin-like growth factor receptor family in neoplasia: an update. Nat Rev Cancer 12, 159-169.
Quan, B., Smiley, D.L., Gelfanov, V.M., and DiMarchi, R.D. (2006). Site Specific Introduction of Unnatural Amino Acids at Sites Critical to Insulin Receptor Recognition and Biological Activity. In Understanding Biology Using Peptides Proceedings of the 19th American Peptide Symposium,, S.E. Blondelle, ed. (New York, N.Y.: Springer), pp. 311-312.
Rarey et al. (1996) J. Mol. Biol., 261, 470.
Robinson and James (1992) Am. J. Physiol., 263, E383-E393.
Rossmann, ed. (1972) The Molecular Replacement Method, Int. Sci. Rev. Ser., No. 13, Gordon & Breach, New York.
Sali and Blundell (1993) J. Mol. Biol., 234, 779-815.
Sambrook et al. (2001) Molecular Cloning: A Laboratory Manual, 3rd ed. Cold Spring Harbor Laboratory Press, Cold Spring Harbor, N.Y.
Schäffer, L. (1994). A model for insulin binding to the insulin receptor. Eur J Biochem 221, 1127-1132.
Schäffer et al. (2003) Proc. Natl. Acad. Sci. USA, 100, 4435-4439.
Scott and Smith (1990) Science, 249, 386-390.
Seino, S., and Bell, G.I. (1989). Alternative splicing of human insulin receptor messenger RNA. Biochem Biophys Res Commun 159, 312-316.
Shoelson, S., Fickova, M., Haneda, M., Nahum, A., Musso, G., Kaiser, E.T., Rubenstein, A.H., and Tager, H. (1983). Identification of a mutant human insulin predicted to contain a serine-for-phenylalanine substitution. Proc Natl Acad Sci USA 80, 7390-7394.
Silverman et al. (2005) Nature Biotech., 23, 1556-1561.
Smart, O.S., Womack, T.O., Flensburg, C., Keller, P., Paciorek, W., Sharff, A., Vonrhein, C., and Bricogne, G. (2012). Exploiting structure similarity in refinement: automated NCS and target-structure restraints in BUSTER. Acta Crystallogr D Biol Crystallogr 68, 368-380.
Smith et al. (1999) Nat. Med., 5, 1390-1395.
Smith, B.J., Huang, K., Kong, G., Chan, S.J., Nakagawa, S., Menting, J.G., Hu, S.-Q., Whittaker, J., Steiner, D.F., Katsoyannis, P.G., et al. (2010). Structural resolution of a tandem hormone-binding element in the insulin receptor and its implications for design of peptide agonists. Proc Natl Acad Sci USA 107,6771-6776.
Songyang et al. (1993) Cell, 72, 767-778.
Soos, M.A., Siddle, K., Baron, M.D., Heward, J.M., Luzio, J.P., Bellatin, J., and Lennox, E.S. (1986). Monoclonal antibodies reacting with multiple epitopes on the human insulin receptor. Biochem J 235, 199-208.
Sparrow et al. (1997) J. Biol. Chem., 272, 29460-29467.
Sparrow, L.G., Lawrence, M.C., Gorman, J.J., Strike, P.M., Robinson, C.P., McKern, N.M., and Ward, C.W. (2008). N-linked glycans of the human insulin receptor and their distribution over the crystal structure. Proteins: Struct Funct Bioinform 71, 426-439.
Stanley, P. (1989). Chinese hamster ovary cell mutants with multiple glycosylation defects for production of glycoproteins with minimal carbohydrate heterogeneity. Mol Cell Biol 9, 377-383.
Stumpp et al. (2007) Curr. Opin. Drug Discov. Develop., 10, 153-159.
Surinya et al. (2008) J. Biol. Chem. 283, 5355-5363.
Svenson et al. (2009) Mol. Pharm., published online 2 April 2009 (DOI: 10.1021/mp900057k).
Talbot, K., Wang, H.Y., Kazi, H., Han, L.Y., Bakshi, K.P., Stucky, A., Fuino, R.L., Kawaguchi, K.R., Samoyedny, A.J., Wilson, R.S., et al. (2012). Demonstrated brain insulin resistance in Alzheimer's disease patients is associated with IGF-1 resistance, IRS-1 dysregulation, and cognitive decline. J Clin Invest 122, 1316-1338.
Taniguchi, C.M., Emanuelli, B., and Kahn, C.R. (2006). Critical nodes in signalling pathways: insights into insulin action. Nat Rev Mol Cell Biol 7, 85-96.
Tong and Rossmann, (1997) Methods Enzymol., 276, 594-611.
Tulloch et al. (1999) J. Struct. Biol., 125, 11-18.
Ulrich (2006) Handb Exp Pharmacol., 173, 305-326.
van der Spoel et al. (2005) J. Comp. Chem., 26, 1701-1718.
Ward, C.W., and Lawrence, M.C. (2009). Ligand-induced activation of the insulin receptor: a multi-step process involving structural changes in both the ligand and the receptor. Bioessays 31, 422-434.
Ward, C.W., and Lawrence, M.C. (2011). Landmarks in insulin research. Front Endocrin 2, Article 76, 71-11, doi: 10.3389/fendo.2011.00076.
Weiner et al. (1984) J. Am. Chem. Soc., 106, 765-784.
Whittaker, L., Hao, C., Fu, W., and Whittaker, J. (2008). High-Affinity Insulin Binding: Insulin Interacts with Two Receptor Ligand Binding Sites. Biochemistry 47, 12900-12909.
Yin et al. (2005) Angew. Chem. Int. Ed. Engl., 44, 2704-2707.
Zakova, L., Kazdova, L., Hanclova, I., Protivinska, E., Sanda, M., Budesinsky, M., and Jiracek, J. (2008). Insulin analogues with modifications at position B26. Divergence of binding affinity and biological activity. Biochemistry 47, 5858-5868.
Zuckermann et al. (1994) J. Med. Chem., 37,2678.

### APPENDIX I: ATOMIC COORDINATES FOR IR310.T MONOMER (CHAIN E) WITH ATTACHED αCT PEPTIDE (CHAIN F), HUMAN INSULIN MONOMER (CHAINS A AND B) AND Fab 83-7 (CHAINS C AND D)

| | | | | | | | | | |
|---|---|---|---|---|---|---|---|---|---|
| ATOM | 1 | N | GLY | A | 1 | 31.737 | -47.052 | -15.556 | 1.00186.71 |
| ATOM | 2 | CA | GLY | A | 1 | 32.951 | -46.963 | -16.365 | 1.00186.36 |
| ATOM | 3 | C | GLY | A | 1 | 32.828 | -45.909 | -17.446 | 1.00189.75 |
| ATOM | 4 | O | GLY | A | 1 | 32.585 | -44.748 | -17.129 | 1.00188.96 |
| ATOM | 5 | N | ILE | A | 2 | 32.937 | -46.314 | -18.736 | 1.00186.15 |
| ATOM | 6 | CA | ILE | A | 2 | 32.787 | -45.454 | -19.928 | 1.00185.79 |
| ATOM | 7 | C | ILE | A | 2 | 31.534 | -44.590 | -19.816 | 1.00192.27 |
| ATOM | 8 | O | ILE | A | 2 | 31.519 | -43.475 | -20.317 | 1.00191.57 |
| ATOM | 9 | CB | ILE | A | 2 | 32.769 | -46.301 | -21.241 | 1.00187.88 |
| ATOM | 10 | CG1 | ILE | A | 2 | 32.843 | -45.406 | -22.494 | 1.00187.01 |
| ATOM | 11 | CG2 | ILE | A | 2 | 31.584 | -47.311 | -21.287 | 1.00188.88 |
| ATOM | 12 | CD1 | ILE | A | 2 | 32.317 | -45.992 | -23.743 | 1.00186.23 |
| ATOM | 13 | N | VAL | A | 3 | 30.499 | -45.132 | -19.149 | 1.00192.14 |
| ATOM | 14 | CA | VAL | A | 3 | 29.171 | -44.566 | -18.879 | 1.00194.24 |
| ATOM | 15 | C | VAL | A | 3 | 29.234 | -43.566 | -17.742 | 1.00202.11 |
| ATOM | 16 | O | VAL | A | 3 | 28.583 | -42.518 | -17.789 | 1.00201.42 |
| ATOM | 17 | CB | VAL | A | 3 | 28.188 | -45.721 | -18.532 | 1.00198.80 |
| ATOM | 18 | CG1 | VAL | A | 3 | 26.795 | -45.215 | -18.126 | 1.00198.93 |
| ATOM | 19 | CG2 | VAL | A | 3 | 28.100 | -46.716 | -19.681 | 1.00198.25 |
| ATOM | 20 | N | GLU | A | 4 | 29.974 | -43.922 | -16.696 | 1.00202.33 |
| ATOM | 21 | CA | GLU | A | 4 | 30.086 | -43.072 | -15.525 | 1.00204.40 |
| ATOM | 22 | C | GLU | A | 4 | 31.245 | -42.072 | -15.608 | 1.00208.87 |
| ATOM | 23 | O | GLU | A | 4 | 31.227 | -41.080 | -14.882 | 1.00208.80 |
| ATOM | 24 | CB | GLU | A | 4 | 30.025 | -43.906 | -14.225 | 1.00206.53 |
| ATOM | 25 | CG | GLU | A | 4 | 28.728 | -44.707 | -14.074 | 1.00221.10 |
| ATOM | 26 | CD | GLU | A | 4 | 27.428 | -43.914 | -14.123 | 1.00253.39 |
| ATOM | 27 | OE1 | GLU | A | 4 | 26.930 | -43.519 | -13.044 | 1.00256.12 |
| ATOM | 28 | OE2 | GLU | A | 4 | 26.901 | -43.693 | -15.238 | 1.00250.04 |
| ATOM | 29 | N | GLN | A | 5 | 32.181 | -42.292 | -16.568 | 1.00205.56 |
| ATOM | 30 | CA | GLN | A | 5 | 33.376 | -41.488 | -16.876 | 1.00205.86 |
| ATOM | 31 | C | GLN | A | 5 | 33.108 | -40.479 | -18.011 | 1.00211.44 |
| ATOM | 32 | O | GLN | A | 5 | 33.743 | -39.421 | -18.034 | 1.00211.70 |
| ATOM | 33 | CB | GLN | A | 5 | 34.543 | -42.437 | -17.233 | 1.00206.26 |
| ATOM | 34 | CG | GLN | A | 5 | 35.846 | -41.828 | -17.753 | 1.00209.80 |
| ATOM | 35 | CD | GLN | A | 5 | 36.640 | -42.888 | -18.483 | 1.00223.23 |
| ATOM | 36 | OE1 | GLN | A | 5 | 36.089 | -43.691 | -19.238 | 1.00217.40 |
| ATOM | 37 | NE2 | GLN | A | 5 | 37.951 | -42.935 | -18.272 | 1.00214.35 |
| ATOM | 38 | N | CYS | A | 6 | 32.180 | -40.809 | -18.945 | 1.00208.45 |
| ATOM | 39 | CA | CYS | A | 6 | 31.843 | -39.957 | -20.089 | 1.00208.46 |
| ATOM | 40 | C | CYS | A | 6 | 30.379 | -39.501 | -20.183 | 1.00211.68 |
| ATOM | 41 | O | CYS | A | 6 | 30.099 | -38.573 | -20.944 | 1.00209.81 |
| ATOM | 42 | CB | CYS | A | 6 | 32.367 | -40.536 | -21.400 | 1.00208.91 |
| ATOM | 43 | SG | CYS | A | 6 | 34.063 | -41.190 | -21.302 | 1.00213.56 |
| ATOM | 44 | N | CYS | A | 7 | 29.466 | -40.075 | -19.357 | 1.00210.76 |
| ATOM | 45 | CA | CYS | A | 7 | 28.081 | -39.600 | -19.303 | 1.00212.79 |
| ATOM | 46 | C | CYS | A | 7 | 27.757 | -38.804 | -18.059 | 1.00219.11 |
| ATOM | 47 | O | CYS | A | 7 | 27.671 | -37.575 | -18.168 | 1.00219.63 |
| ATOM | 48 | CB | CYS | A | 7 | 27.037 | -40.668 | -19.605 | 1.00213.87 |
| ATOM | 49 | SG | CYS | A | 7 | 25.876 | -40.195 | -20.921 | 1.00218.12 |
| ATOM | 50 | N | THR | A | 8 | 27.621 | -39.457 | -16.876 | 1.00217.02 |
| ATOM | 51 | CA | THR | A | 8 | 27.404 | -38.715 | -15.619 | 1.00218.63 |
| ATOM | 52 | C | THR | A | 8 | 28.618 | -37.768 | -15.488 | 1.00223.50 |
| ATOM | 53 | O | THR | A | 8 | 28.441 | -36.547 | -15.595 | 1.00223.51 |
| ATOM | 54 | CB | THR | A | 8 | 27.128 | -39.639 | -14.401 | 1.00225.58 |
| ATOM | 55 | OG1 | THR | A | 8 | 28.312 | -40.357 | -14.045 | 1.00226.59 |
| ATOM | 56 | CG2 | THR | A | 8 | 25.932 | -40.589 | -14.613 | 1.00219.68 |
| ATOM | 57 | N | SER | A | 9 | 29.853 | -38.344 | -15.435 | 1.00220.28 |
| ATOM | 58 | CA | SER | A | 9 | 31.123 | -37.593 | -15.481 | 1.00220.67 |
| ATOM | 59 | C | SER | A | 9 | 31.389 | -37.222 | -16.970 | 1.00223.32 |
| ATOM | 60 | O | SER | A | 9 | 30.848 | -37.895 | -17.850 | 1.00222.92 |
| ATOM | 61 | CB | SER | A | 9 | 32.274 | -38.407 | -14.880 | 1.00224.27 |
| ATOM | 62 | OG | SER | A | 9 | 33.525 | -38.269 | -15.542 | 1.00233.29 |
| ATOM | 63 | N | ILE | A | 10 | 32.173 | -36.141 | -17.252 | 1.00217.80 |
| ATOM | 64 | CA | ILE | A | 10 | 32.424 | -35.684 | -18.632 | 1.00215.17 |
| ATOM | 65 | C | ILE | A | 10 | 33.838 | -36.044 | -19.173 | 1.00215.54 |
| ATOM | 66 | O | ILE | A | 10 | 34.839 | -35.982 | -18.453 | 1.00214.66 |
| ATOM | 67 | CB | ILE | A | 10 | 31.873 | -34.241 | -18.922 | 1.00218.28 |
| ATOM | 68 | CG1 | ILE | A | 10 | 30.383 | -34.014 | -18.378 | 1.00218.40 |
| ATOM | 69 | CG2 | ILE | A | 10 | 32.037 | -33.829 | -20.394 | 1.00218.12 |
| ATOM | 70 | CD1 | ILE | A | 10 | 29.056 | -34.647 | -19.169 | 1.00214.17 |
| ATOM | 71 | N | CYS | A | 11 | 33.866 | -36.501 | -20.431 | 1.00210.74 |
| ATOM | 72 | CA | CYS | A | 11 | 35.005 | -37.064 | -21.155 | 1.00210.30 |
| ATOM | 73 | C | CYS | A | 11 | 35.909 | -36.089 | -21.888 | 1.00212.63 |
| ATOM | 74 | O | CYS | A | 11 | 35.448 | -35.239 | -22.657 | 1.00210.85 |
| ATOM | 75 | CB | CYS | A | 11 | 34.518 | -38.170 | -22.095 | 1.00210.31 |
| ATOM | 76 | SG | CYS | A | 11 | 35.393 | -39.762 | -21.944 | 1.00214.15 |
| ATOM | 77 | N | SER | A | 12 | 37.218 | -36.288 | -21.690 | 1.00209.84 |
| ATOM | 78 | CA | SER | A | 12 | 38.304 | -35.593 | -22.382 | 1.00209.99 |
| ATOM | 79 | C | SER | A | 12 | 38.610 | -36.502 | -23.563 | 1.00211.83 |
| ATOM | 80 | O | SER | A | 12 | 38.563 | -37.725 | -23.400 | 1.00211.06 |
| ATOM | 81 | CB | SER | A | 12 | 39.528 | -35.467 | -21.471 | 1.00214.68 |
| ATOM | 82 | OG | SER | A | 12 | 40.761 | -35.834 | -22.075 | 1.00223.54 |
| ATOM | 83 | N | LEU | A | 13 | 38.933 | -35.932 | -24.735 | 1.00207.24 |
| ATOM | 84 | CA | LEU | A | 13 | 39.213 | -36.730 | -25.931 | 1.00206.83 |
| ATOM | 85 | C | LEU | A | 13 | 40.276 | -37.829 | -25.792 | 1.00209.14 |
| ATOM | 86 | O | LEU | A | 13 | 40.432 | -38.666 | -26.695 | 1.00207.43 |
| ATOM | 87 | CB | LEU | A | 13 | 39.425 | -35.859 | -27.166 | 1.00207.56 |
| ATOM | 88 | CG | LEU | A | 13 | 38.707 | -36.307 | -28.442 | 1.00212.48 |
| ATOM | 89 | CD1 | LEU | A | 13 | 37.217 | -35.965 | -28.391 | 1.00211.59 |
| ATOM | 90 | CD2 | LEU | A | 13 | 39.370 | -35.704 | -29.684 | 1.00216.13 |
| ATOM | 91 | N | TYR | A | 14 | 40.965 | -37.863 | -24.637 | 1.00206.66 |
| ATOM | 92 | CA | TYR | A | 14 | 41.901 | -38.941 | -24.375 | 1.00207.15 |
| ATOM | 93 | C | TYR | A | 14 | 41.134 | -40.151 | -23.869 | 1.00209.90 |
| ATOM | 94 | O | TYR | A | 14 | 41.283 | -41.234 | -24.434 | 1.00209.79 |
| ATOM | 95 | CB | TYR | A | 14 | 43.016 | -38.603 | -23.369 | 1.00209.13 |
| ATOM | 96 | CG | TYR | A | 14 | 43.706 | -39.890 | -22.968 | 1.00211.84 |
| ATOM | 97 | CD1 | TYR | A | 14 | 44.316 | -40.702 | -23.927 | 1.00214.41 |
| ATOM | 98 | CD2 | TYR | A | 14 | 43.596 | -40.389 | -21.671 | 1.00212.97 |
| ATOM | 99 | CE1 | TYR | A | 14 | 44.839 | -41.953 | -23.598 | 1.00216.03 |
| ATOM | 100 | CE2 | TYR | A | 14 | 44.149 | -41.627 | -21.321 | 1.00214.39 |
| ATOM | 101 | CZ | TYR | A | 14 | 44.774 | -42.406 | -22.291 | 1.00222.21 |
| ATOM | 102 | OH | TYR | A | 14 | 45.342 | -43.630 | -21.992 | 1.00220.55 |
| ATOM | 103 | N | GLN | A | 15 | 40.378 | -39.991 | -22.759 | 1.00204.72 |
| ATOM | 104 | CA | GLN | A | 15 | 39.613 | -41.076 | -22.142 | 1.00203.03 |
| ATOM | 105 | C | GLN | A | 15 | 38.930 | -41.983 | -23.154 | 1.00203.72 |
| ATOM | 106 | O | GLN | A | 15 | 38.768 | -43.159 | -22.873 | 1.00202.89 |
| ATOM | 107 | CB | GLN | A | 15 | 38.636 | -40.559 | -21.087 | 1.00204.31 |
| ATOM | 108 | CG | GLN | A | 15 | 39.304 | -40.122 | -19.787 | 1.00216.06 |
| ATOM | 109 | CD | GLN | A | 15 | 39.279 | -38.628 | -19.606 | 1.00225.02 |
| ATOM | 110 | OE1 | GLN | A | 15 | 38.208 | -37.987 | -19.579 | 1.00213.61 |
| ATOM | 111 | NE2 | GLN | A | 15 | 40.466 | -38.055 | -19.419 | 1.00217.98 |
| ATOM | 112 | N | LEU | A | 16 | 38.607 | -41.451 | -24.354 | 1.00198.95 |
| ATOM | 113 | CA | LEU | A | 16 | 38.038 | -42.196 | -25.477 | 1.00197.96 |
| ATOM | 114 | C | LEU | A | 16 | 39.107 | -42.912 | -26.366 | 1.00204.30 |
| ATOM | 115 | O | LEU | A | 16 | 38.754 | -43.904 | -27.017 | 1.00203.61 |
| ATOM | 116 | CB | LEU | A | 16 | 37.060 | -41.355 | -26.319 | 1.00196.58 |
| ATOM | 117 | CG | LEU | A | 16 | 35.753 | -40.931 | -25.677 | 1.00198.33 |
| ATOM | 118 | CD1 | LEU | A | 16 | 35.860 | -39.537 | -25.192 | 1.00198.12 |
| ATOM | 119 | CD2 | LEU | A | 16 | 34.644 | -40.937 | -26.691 | 1.00197.87 |
| ATOM | 120 | N | GLU | A | 17 | 40.393 | -42.436 | -26.396 | 1.00203.04 |
| ATOM | 121 | CA | GLU | A | 17 | 41.464 | -43.149 | -27.119 | 1.00204.91 |
| ATOM | 122 | C | GLU | A | 17 | 41.527 | -44.494 | -26.429 | 1.00213.93 |
| ATOM | 123 | O | GLU | A | 17 | 41.495 | -45.522 | -27.106 | 1.00215.72 |
| ATOM | 124 | CB | GLU | A | 17 | 42.862 | -42.487 | -26.970 | 1.00206.66 |
| ATOM | 125 | CG | GLU | A | 17 | 44.060 | -43.342 | -27.439 | 1.00214.83 |
| ATOM | 126 | CD | GLU | A | 17 | 44.520 | -44.611 | -26.709 | 1.00224.64 |
| ATOM | 127 | OE1 | GLU | A | 17 | 44.942 | -44.529 | -25.530 | 1.00203.51 |
| ATOM | 128 | OE2 | GLU | A | 17 | 44.513 | -45.688 | -27.352 | 1.00212.80 |
| ATOM | 129 | N | ASN | A | 18 | 41.650 | -44.471 | -25.069 | 1.00211.20 |
| ATOM | 130 | CA | ASN | A | 18 | 41.753 | -45.620 | -24.171 | 1.00211.23 |
| ATOM | 131 | C | ASN | A | 18 | 41.284 | -46.928 | -24.816 | 1.00216.76 |
| ATOM | 132 | O | ASN | A | 18 | 42.113 | -47.819 | -25.070 | 1.00217.67 |
| ATOM | 133 | CB | ASN | A | 18 | 40.932 | -45.362 | -22.897 | 1.00210.56 |
| ATOM | 134 | CG | ASN | A | 18 | 41.619 | -44.591 | -21.804 | 1.00230.65 |
| ATOM | 135 | OD1 | ASN | A | 18 | 42.829 | -44.709 | -21.603 | 1.00228.38 |
| ATOM | 136 | ND2 | ASN | A | 18 | 40.838 | -43.885 | -20.988 | 1.00218.23 |
| ATOM | 137 | N | TYR | A | 19 | 39.954 | -46.963 | -25.170 | 1.00212.26 |
| ATOM | 138 | CA | TYR | A | 19 | 39.116 | -48.065 | -25.681 | 1.00211.48 |
| ATOM | 139 | C | TYR | A | 19 | 39.274 | -48.525 | -27.145 | 1.00214.08 |
| ATOM | 140 | O | TYR | A | 19 | 38.342 | -49.153 | -27.669 | 1.00213.48 |
| ATOM | 141 | CB | TYR | A | 19 | 37.636 | -47.728 | -25.434 | 1.00211.91 |
| ATOM | 142 | CG | TYR | A | 19 | 37.307 | -47.248 | -24.041 | 1.00214.04 |
| ATOM | 143 | CD1 | TYR | A | 19 | 37.361 | -48.116 | -22.951 | 1.00216.92 |
| ATOM | 144 | CD2 | TYR | A | 19 | 36.841 | -45.956 | -23.822 | 1.00214.35 |
| ATOM | 145 | CE1 | TYR | A | 19 | 37.009 | -47.692 | -21.669 | 1.00218.74 |
| ATOM | 146 | CE2 | TYR | A | 19 | 36.477 | -45.523 | -22.548 | 1.00215.16 |
| ATOM | 147 | CZ | TYR | A | 19 | 36.566 | -46.392 | -21.473 | 1.00224.76 |
| ATOM | 148 | OH | TYR | A | 19 | 36.208 | -45.965 | -20.219 | 1.00226.93 |
| ATOM | 149 | N | CYS | A | 20 | 40.433 | -48.271 | -27.794 | 1.00209.85 |
| ATOM | 150 | CA | CYS | A | 20 | 40.578 | -48.616 | -29.210 | 1.00214.12 |
| ATOM | 151 | C | CYS | A | 20 | 41.666 | -49.637 | -29.665 | 1.00210.58 |
| ATOM | 152 | O | CYS | A | 20 | 42.766 | -49.736 | -29.116 | 1.00156.27 |
| ATOM | 153 | CB | CYS | A | 20 | 40.556 | -47.359 | -30.079 | 1.00214.21 |
| ATOM | 154 | SG | CYS | A | 20 | 39.473 | -46.029 | -29.464 | 1.00216.29 |
| ATOM | 156 | N | CYS | B | 7 | 23.960 | -38.221 | -23.082 | 1.00232.97 |
| ATOM | 157 | CA | CYS | B | 7 | 25.056 | -38.561 | -24.003 | 1.00229.98 |
| ATOM | 158 | C | CYS | B | 7 | 24.970 | -40.015 | -24.562 | 1.00225.15 |
| ATOM | 159 | O | CYS | B | 7 | 25.917 | -40.482 | -25.206 | 1.00222.28 |
| ATOM | 160 | CB | CYS | B | 7 | 26.417 | -38.269 | -23.364 | 1.00231.56 |
| ATOM | 161 | SG | CYS | B | 7 | 27.143 | -39.672 | -22.458 | 1.00235.21 |
| ATOM | 162 | N | GLY | B | 8 | 23.830 | -40.676 | -24.313 | 1.00217.38 |
| ATOM | 163 | CA | GLY | B | 8 | 23.520 | -42.044 | -24.709 | 1.00213.28 |
| ATOM | 164 | C | GLY | B | 8 | 24.120 | -42.490 | -26.023 | 1.00210.24 |
| ATOM | 165 | O | GLY | B | 8 | 25.270 | -42.944 | -26.052 | 1.00208.44 |
| ATOM | 166 | N | SER | B | 9 | 23.348 | -42.341 | -27.124 | 1.00202.95 |
| ATOM | 167 | CA | SER | B | 9 | 23.744 | -42.737 | -28.486 | 1.00199.17 |
| ATOM | 168 | C | SER | B | 9 | 25.045 | -42.082 | -28.921 | 1.00198.52 |
| ATOM | 169 | O | SER | B | 9 | 25.894 | -42.739 | -29.522 | 1.00195.47 |
| ATOM | 170 | CB | SER | B | 9 | 22.629 | -42.431 | -29.489 | 1.00202.12 |
| ATOM | 171 | OG | SER | B | 9 | 22.321 | -41.050 | -29.598 | 1.00207.98 |
| ATOM | 172 | N | HIS | B | 10 | 25.189 | -40.781 | -28.565 | 1.00194.66 |
| ATOM | 173 | CA | HIS | B | 10 | 26.294 | -39.852 | -28.847 | 1.00192.96 |
| ATOM | 174 | C | HIS | B | 10 | 27.662 | -40.379 | -28.406 | 1.00192.77 |
| ATOM | 175 | O | HIS | B | 10 | 28.654 | -40.128 | -29.088 | 1.00190.71 |
| ATOM | 176 | CB | HIS | B | 10 | 25.995 | -38.448 | -28.265 | 1.00195.43 |
| ATOM | 177 | CG | HIS | B | 10 | 24.532 | -38.075 | -28.241 | 1.00200.51 |
| ATOM | 178 | ND1 | HIS | B | 10 | 23.642 | -38.518 | -29.220 | 1.00201.87 |
| ATOM | 179 | CD2 | HIS | B | 10 | 23.849 | -37.316 | -27.351 | 1.00204.64 |
| ATOM | 180 | CE1 | HIS | B | 10 | 22.458 | -38.029 | -28.882 | 1.00203.13 |
| ATOM | 181 | NE2 | HIS | B | 10 | 22.530 | -37.301 | -27.766 | 1.00205.30 |
| ATOM | 182 | N | LEU | B | 11 | 27.706 | -41.147 | -27.298 | 1.00187.77 |
| ATOM | 183 | CA | LEU | B | 11 | 28.940 | -41.761 | -26.811 | 1.00185.44 |
| ATOM | 184 | C | LEU | B | 11 | 29.329 | -42.880 | -27.747 | 1.00185.19 |
| ATOM | 185 | O | LEU | B | 11 | 30.527 | -43.088 | -27.958 | 1.00182.23 |
| ATOM | 186 | CB | LEU | B | 11 | 28.805 | -42.254 | -25.354 | 1.00185.86 |
| ATOM | 187 | CG | LEU | B | 11 | 29.952 | -43.090 | -24.734 | 1.00189.01 |
| ATOM | 188 | CD1 | LEU | B | 11 | 31.332 | -42.458 | -24.932 | 1.00188.16 |
| ATOM | 189 | CD2 | LEU | B | 11 | 29.704 | -43.316 | -23.272 | 1.00193.32 |
| ATOM | 190 | N | VAL | B | 12 | 28.325 | -43.585 | -28.338 | 1.00182.38 |
| ATOM | 191 | CA | VAL | B | 12 | 28.645 | -44.640 | -29.308 | 1.00181.99 |
| ATOM | 192 | C | VAL | B | 12 | 29.279 | -43.915 | -30.501 | 1.00187.02 |
| ATOM | 193 | O | VAL | B | 12 | 30.314 | -44.368 | -30.998 | 1.00188.18 |
| ATOM | 194 | CB | VAL | B | 12 | 27.514 | -45.642 | -29.754 | 1.00185.60 |
| ATOM | 195 | CG1 | VAL | B | 12 | 28.107 | -46.995 | -30.137 | 1.00183.94 |
| ATOM | 196 | CG2 | VAL | B | 12 | 26.427 | -45.827 | -28.699 | 1.00186.32 |
| ATOM | 197 | N | GLU | B | 13 | 28.723 | -42.736 | -30.878 | 1.00181.73 |
| ATOM | 198 | CA | GLU | B | 13 | 29.219 | -41.943 | -31.998 | 1.00180.32 |
| ATOM | 199 | C | GLU | B | 13 | 30.543 | -41.230 | -31.732 | 1.00182.27 |
| ATOM | 200 | O | GLU | B | 13 | 31.435 | -41.334 | -32.578 | 1.00181.29 |
| ATOM | 201 | CB | GLU | B | 13 | 28.122 | -41.050 | -32.570 | 1.00182.32 |
| ATOM | 202 | CG | GLU | B | 13 | 27.083 | -41.867 | -33.323 | 1.00194.34 |
| ATOM | 203 | CD | GLU | B | 13 | 25.640 | -41.400 | -33.250 | 1.00230.28 |
| ATOM | 204 | OE1 | GLU | B | 13 | 25.396 | -40.169 | -33.238 | 1.00226.43 |
| ATOM | 205 | OE2 | GLU | B | 13 | 24.747 | -42.280 | -33.248 | 1.00233.16 |
| ATOM | 206 | N | ALA | B | 14 | 30.702 | -40.584 | -30.535 | 1.00178.12 |
| ATOM | 207 | CA | ALA | B | 14 | 31.936 | -39.909 | -30.086 | 1.00177.54 |
| ATOM | 208 | C | ALA | B | 14 | 33.097 | -40.906 | -30.140 | 1.00182.19 |
| ATOM | 209 | O | ALA | B | 14 | 34.128 | -40.608 | -30.735 | 1.00179.92 |
| ATOM | 210 | CB | ALA | B | 14 | 31.768 | -39.363 | -28.669 | 1.00178.72 |
| ATOM | 211 | N | LEU | B | 15 | 32.875 | -42.141 | -29.615 | 1.00182.04 |
| ATOM | 212 | CA | LEU | B | 15 | 33.858 | -43.231 | -29.649 | 1.00182.35 |
| ATOM | 213 | C | LEU | B | 15 | 34.176 | -43.695 | -31.065 | 1.00189.26 |
| ATOM | 214 | O | LEU | B | 15 | 35.210 | -44.338 | -31.262 | 1.00190.00 |
| ATOM | 215 | CB | LEU | B | 15 | 33.525 | -44.417 | -28.722 | 1.00181.61 |
| ATOM | 216 | CG | LEU | B | 15 | 34.648 | -44.729 | -27.731 | 1.00185.56 |
| ATOM | 217 | CD1 | LEU | B | 15 | 34.214 | -44.482 | -26.333 | 1.00186.41 |
| ATOM | 218 | CD2 | LEU | B | 15 | 35.158 | -46.129 | -27.864 | 1.00185.02 |
| ATOM | 219 | N | TYR | B | 16 | 33.327 | -43.354 | -32.063 | 1.00186.36 |
| ATOM | 220 | CA | TYR | B | 16 | 33.721 | -43.723 | -33.404 | 1.00186.42 |
| ATOM | 221 | C | TYR | B | 16 | 34.750 | -42.737 | -33.915 | 1.00198.07 |
| ATOM | 222 | O | TYR | B | 16 | 35.846 | -43.176 | -34.299 | 1.00199.72 |
| ATOM | 223 | CB | TYR | B | 16 | 32.591 | -43.907 | -34.397 | 1.00184.87 |
| ATOM | 224 | CG | TYR | B | 16 | 33.123 | -44.612 | -35.621 | 1.00184.81 |
| ATOM | 225 | CD1 | TYR | B | 16 | 34.233 | -45.449 | -35.541 | 1.00186.73 |
| ATOM | 226 | CD2 | TYR | B | 16 | 32.516 | -44.454 | -36.856 | 1.00186.19 |
| ATOM | 227 | CE1 | TYR | B | 16 | 34.729 | -46.105 | -36.664 | 1.00189.25 |
| ATOM | 228 | CE2 | TYR | B | 16 | 32.994 | -45.116 | -37.988 | 1.00188.21 |
| ATOM | 229 | CZ | TYR | B | 16 | 34.092 | -45.955 | -37.884 | 1.00196.82 |
| ATOM | 230 | OH | TYR | B | 16 | 34.561 | -46.634 | -38.987 | 1.00199.80 |
| ATOM | 231 | N | LEU | B | 17 | 34.443 | -41.409 | -33.875 | 1.00197.09 |
| ATOM | 232 | CA | LEU | B | 17 | 35.423 | -40.420 | -34.328 | 1.00197.79 |
| ATOM | 233 | C | LEU | B | 17 | 36.798 | -40.640 | -33.667 | 1.00203.69 |
| ATOM | 234 | O | LEU | B | 17 | 37.797 | -40.670 | -34.385 | 1.00204.11 |
| ATOM | 235 | CB | LEU | B | 17 | 34.941 | -38.928 | -34.362 | 1.00197.83 |
| ATOM | 236 | CG | LEU | B | 17 | 34.071 | -38.317 | -33.224 | 1.00201.49 |
| ATOM | 237 | CD1 | LEU | B | 17 | 34.365 | -36.818 | -33.034 | 1.00201.24 |
| ATOM | 238 | CD2 | LEU | B | 17 | 32.601 | -38.461 | -33.524 | 1.00203.23 |
| ATOM | 239 | N | VAL | B | 18 | 36.816 | -40.985 | -32.355 | 1.00201.18 |
| ATOM | 240 | CA | VAL | B | 18 | 38.042 | -41.235 | -31.593 | 1.00202.21 |
| ATOM | 241 | C | VAL | B | 18 | 38.853 | -42.503 | -31.985 | 1.00211.88 |
| ATOM | 242 | O | VAL | B | 18 | 40.058 | -42.516 | -31.740 | 1.00211.89 |
| ATOM | 243 | CB | VAL | B | 18 | 37.891 | -41.009 | -30.060 | 1.00205.16 |
| ATOM | 244 | CG1 | VAL | B | 18 | 39.248 | -40.846 | -29.375 | 1.00205.05 |
| ATOM | 245 | CG2 | VAL | B | 18 | 37.033 | -39.787 | -29.773 | 1.00205.11 |
| ATOM | 246 | N | CYS | B | 19 | 38.246 | -43.536 | -32.625 | 1.00212.92 |
| ATOM | 247 | CA | CYS | B | 19 | 39.047 | -44.719 | -33.007 | 1.00215.52 |
| ATOM | 248 | C | CYS | B | 19 | 39.237 | -44.835 | -34.509 | 1.00222.37 |
| ATOM | 249 | O | CYS | B | 19 | 40.242 | -45.384 | -34.944 | 1.00222.72 |
| ATOM | 250 | CB | CYS | B | 19 | 38.510 | -46.026 | -32.412 | 1.00216.15 |
| ATOM | 251 | SG | CYS | B | 19 | 37.878 | -45.918 | -30.719 | 1.00219.65 |
| ATOM | 252 | N | GLY | B | 20 | 38.235 | -44.436 | -35.291 | 1.00220.86 |
| ATOM | 253 | CA | GLY | B | 20 | 38.269 | -44.572 | -36.740 | 1.00222.90 |
| ATOM | 254 | C | GLY | B | 20 | 38.616 | -45.976 | -37.198 | 1.00231.14 |
| ATOM | 255 | O | GLY | B | 20 | 38.179 | -46.977 | -36.608 | 1.00229.96 |
| ATOM | 256 | N | GLU | B | 21 | 39.565 | -46.036 | -38.113 | 1.00232.64 |
| ATOM | 257 | CA | GLU | B | 21 | 40.038 | -47.273 | -38.722 | 1.00235.94 |
| ATOM | 258 | C | GLU | B | 21 | 40.657 | -48.280 | -37.743 | 1.00241.56 |
| ATOM | 259 | O | GLU | B | 21 | 40.635 | -49.490 | -38.011 | 1.00243.26 |
| ATOM | 260 | CB | GLU | B | 21 | 40.980 | -46.950 | -39.900 | 1.00240.01 |
| ATOM | 261 | CG | GLU | B | 21 | 40.285 | -46.762 | -41.255 | 1.00255.11 |
| ATOM | 262 | CD | GLU | B | 21 | 40.965 | -45.875 | -42.288 | 1.00284.51 |
| ATOM | 263 | OE1 | GLU | B | 21 | 42.182 | -46.053 | -42.522 | 1.00289.71 |
| ATOM | 264 | OE2 | GLU | B | 21 | 40.263 | -45.044 | -42.912 | 1.00276.74 |
| ATOM | 265 | N | ARG | B | 22 | 41.158 | -47.804 | -36.597 | 1.00236.16 |
| ATOM | 266 | CA | ARG | B | 22 | 41.777 | -48.670 | -35.594 | 1.00235.07 |
| ATOM | 267 | C | ARG | B | 22 | 40.776 | -49.650 | -34.986 | 1.00236.02 |
| ATOM | 268 | O | ARG | B | 22 | 41.130 | -50.761 | -34.568 | 1.00235.36 |
| ATOM | 269 | CB | ARG | B | 22 | 42.355 | -47.790 | -34.492 | 1.00233.08 |
| ATOM | 270 | CG | ARG | B | 22 | 43.670 | -47.117 | -34.861 | 1.00241.66 |
| ATOM | 271 | CD | ARG | B | 22 | 43.815 | -46.031 | -35.953 | 1.00255.79 |
| ATOM | 272 | NE | ARG | B | 22 | 44.366 | -46.527 | -37.236 | 1.00267.56 |
| ATOM | 273 | CZ | ARG | B | 22 | 45.529 | -46.160 | -37.788 | 1.00278.48 |
| ATOM | 274 | NH1 | ARG | B | 22 | 46.345 | -45.329 | -37.151 | 1.00266.51 |
| ATOM | 275 | NH2 | ARG | B | 22 | 45.895 | -46.650 | -38.967 | 1.00258.49 |
| ATOM | 276 | N | GLY | B | 23 | 39.541 | -49.195 | -34.883 | 1.00230.04 |
| ATOM | 277 | CA | GLY | B | 23 | 38.541 | -50.026 | -34.237 | 1.00227.91 |
| ATOM | 278 | C | GLY | B | 23 | 38.646 | -50.044 | -32.710 | 1.00228.27 |
| ATOM | 279 | O | GLY | B | 23 | 39.457 | -49.333 | -32.113 | 1.00226.72 |
| ATOM | 280 | N | PHE | B | 24 | 37.778 | -50.847 | -32.037 | 1.00223.26 |
| ATOM | 281 | CA | PHE | B | 24 | 37.610 | -50.941 | -30.560 | 1.00221.71 |
| ATOM | 282 | C | PHE | B | 24 | 38.446 | -52.073 | -29.833 | 1.00227.25 |
| ATOM | 283 | O | PHE | B | 24 | 39.307 | -52.660 | -30.494 | 1.00228.96 |
| ATOM | 284 | CB | PHE | B | 24 | 36.095 | -50.988 | -30.223 | 1.00221.97 |
| ATOM | 285 | CG | PHE | B | 24 | 35.264 | -49.809 | -30.714 | 1.00222.42 |
| ATOM | 286 | CD1 | PHE | B | 24 | 34.611 | -48.976 | -29.818 | 1.00224.25 |
| ATOM | 287 | CD2 | PHE | B | 24 | 35.105 | -49.560 | -32.073 | 1.00224.10 |
| ATOM | 288 | CE1 | PHE | B | 24 | 33.841 | -47.898 | -30.273 | 1.00224.62 |
| ATOM | 289 | CE2 | PHE | B | 24 | 34.366 | -48.466 | -32.520 | 1.00226.18 |
| ATOM | 290 | CZ | PHE | B | 24 | 33.736 | -47.644 | -31.617 | 1.00223.52 |
| ATOM | 291 | O | PHE | B | 25 | 37.255 | -53.905 | -26.185 | 1.00218.98 |
| ATOM | 292 | N | PHE | B | 25 | 38.246 | -52.347 | -28.482 | 1.00222.49 |
| ATOM | 293 | CA | PHE | B | 25 | 39.032 | -53.388 | -27.745 | 1.00222.12 |
| ATOM | 294 | C | PHE | B | 25 | 38.325 | -54.262 | -26.694 | 1.00221.10 |
| ATOM | 295 | CB | PHE | B | 25 | 40.442 | -52.890 | -27.287 | 1.00225.15 |
| ATOM | 296 | CG | PHE | B | 25 | 40.864 | -52.760 | -25.823 | 1.00226.85 |
| ATOM | 297 | CD1 | PHE | B | 25 | 41.962 | -53.466 | -25.333 | 1.00230.25 |
| ATOM | 298 | CD2 | PHE | B | 25 | 40.275 | -51.802 | -24.988 | 1.00228.79 |
| ATOM | 299 | CE1 | PHE | B | 25 | 42.425 | -53.262 | -24.020 | 1.00231.09 |
| ATOM | 300 | CE2 | PHE | B | 25 | 40.729 | -51.611 | -23.666 | 1.00231.35 |
| ATOM | 301 | CZ | PHE | B | 25 | 41.809 | -52.331 | -23.199 | 1.00229.78 |
| ATOM | 302 | O | TYR | B | 26 | 39.488 | -58.312 | -24.237 | 1.00198.04 |
| ATOM | 303 | N | TYR | B | 26 | 38.953 | -55.436 | -26.407 | 1.00216.09 |
| ATOM | 304 | CA | TYR | B | 26 | 38.483 | -56.481 | -25.493 | 1.00236.98 |
| ATOM | 305 | C | TYR | B | 26 | 39.559 | -57.556 | -25.218 | 1.00245.61 |
| ATOM | 306 | CB | TYR | B | 26 | 37.176 | -57.124 | -26.024 | 1.00238.00 |
| ATOM | 307 | CG | TYR | B | 26 | 37.184 | -57.640 | -27.456 | 1.00240.71 |
| ATOM | 308 | CD1 | TYR | B | 26 | 36.631 | -58.877 | -27.774 | 1.00242.87 |
| ATOM | 309 | CD2 | TYR | B | 26 | 37.674 | -56.858 | -28.505 | 1.00242.05 |
| ATOM | 310 | CE1 | TYR | B | 26 | 36.596 | -59.341 | -29.090 | 1.00244.60 |
| ATOM | 311 | CE2 | TYR | B | 26 | 37.663 | -57.320 | -29.822 | 1.00243.99 |
| ATOM | 312 | CZ | TYR | B | 26 | 37.099 | -58.554 | -30.115 | 1.00251.05 |
| ATOM | 313 | OH | TYR | B | 26 | 37.068 | -59.015 | -31.415 | 1.00250.94 |
| ATOM | 315 | N | GLN | C | 1 | 11.311 | -99.334 | -34.481 | 1.00197.85 |
| ATOM | 316 | CA | GLN | C | 1 | 11.076 | -100.772 | -34.608 | 1.00197.44 |
| ATOM | 317 | C | GLN | C | 1 | 12.144 | -101.453 | -33.778 | 1.00199.98 |
| ATOM | 318 | O | GLN | C | 1 | 12.893 | -102.222 | -34.362 | 1.00201.18 |
| ATOM | 319 | CB | GLN | C | 1 | 11.244 | -101.198 | -36.098 | 1.00201.73 |
| ATOM | 320 | CG | GLN | C | 1 | 10.262 | -100.602 | -37.118 | 1.00209.58 |
| ATOM | 321 | CD | GLN | C | 1 | 10.377 | -99.106 | -37.366 | 1.00224.21 |
| ATOM | 322 | OE1 | GLN | C | 1 | 11.470 | -98.526 | -37.428 | 1.00219.38 |
| ATOM | 323 | NE2 | GLN | C | 1 | 9.237 | -98.444 | -37.528 | 1.00214.07 |
| ATOM | 324 | N | VAL | C | 2 | 12.312 | -101.099 | -32.477 | 1.00194.14 |
| ATOM | 325 | CA | VAL | C | 2 | 13.420 | -101.605 | -31.645 | 1.00191.93 |
| ATOM | 326 | C | VAL | C | 2 | 13.431 | -103.118 | -31.472 | 1.00201.23 |
| ATOM | 327 | O | VAL | C | 2 | 12.476 | -103.682 | -30.948 | 1.00200.75 |
| ATOM | 328 | CB | VAL | C | 2 | 13.586 | -100.870 | -30.292 | 1.00192.02 |
| ATOM | 329 | CG1 | VAL | C | 2 | 14.753 | -101.437 | -29.495 | 1.00189.08 |
| ATOM | 330 | CG2 | VAL | C | 2 | 13.794 | -99.380 | -30.507 | 1.00192.95 |
| ATOM | 331 | N | GLN | C | 3 | 14.526 | -103.766 | -31.907 | 1.00202.34 |
| ATOM | 332 | CA | GLN | C | 3 | 14.717 | -105.214 | -31.806 | 1.00203.12 |
| ATOM | 333 | C | GLN | C | 3 | 16.131 | -105.584 | -31.436 | 1.00208.12 |
| ATOM | 334 | O | GLN | C | 3 | 17.096 | -104.926 | -31.837 | 1.00207.84 |
| ATOM | 335 | CB | GLN | C | 3 | 14.356 | -105.911 | -33.113 | 1.00207.35 |
| ATOM | 336 | CG | GLN | C | 3 | 12.872 | -105.887 | -33.415 | 1.00218.60 |
| ATOM | 337 | CD | GLN | C | 3 | 12.507 | -107.080 | -34.229 | 1.00242.36 |
| ATOM | 338 | OE1 | GLN | C | 3 | 12.817 | -108.213 | -33.851 | 1.00238.61 |
| ATOM | 339 | NE2 | GLN | C | 3 | 11.849 | -106.861 | -35.360 | 1.00237.02 |
| ATOM | 340 | N | LEU | C | 4 | 16.237 | -106.648 | -30.665 | 1.00205.59 |
| ATOM | 341 | CA | LEU | C | 4 | 17.501 | -107.205 | -30.244 | 1.00206.04 |
| ATOM | 342 | C | LEU | C | 4 | 17.300 | -108.701 | -30.296 | 1.00216.94 |
| ATOM | 343 | O | LEU | C | 4 | 16.221 | -109.194 | -29.931 | 1.00217.18 |
| ATOM | 344 | CB | LEU | C | 4 | 17.879 | -106.781 | -28.814 | 1.00203.08 |
| ATOM | 345 | CG | LEU | C | 4 | 18.075 | -105.295 | -28.547 | 1.00206.08 |
| ATOM | 346 | CD1 | LEU | C | 4 | 16.789 | -104.684 | -28.046 | 1.00205.23 |
| ATOM | 347 | CD2 | LEU | C | 4 | 19.173 | -105.066 | -27.506 | 1.00206.30 |
| ATOM | 348 | N | LYS | C | 5 | 18.304 | -109.425 | -30.825 | 1.00218.00 |
| ATOM | 349 | CA | LYS | C | 5 | 18.255 | -110.881 | -30.949 | 1.00219.97 |
| ATOM | 350 | C | LYS | C | 5 | 19.645 | -111.452 | -30.731 | 1.00226.85 |
| ATOM | 351 | O | LYS | C | 5 | 20.617 | -110.984 | -31.316 | 1.00227.49 |
| ATOM | 352 | CB | LYS | C | 5 | 17.705 | -111.336 | -32.322 | 1.00225.47 |
| ATOM | 353 | CG | LYS | C | 5 | 16.634 | -110.443 | -32.965 | 1.00243.07 |
| ATOM | 354 | CD | LYS | C | 5 | 16.061 | -111.036 | -34.239 | 1.00255.86 |
| ATOM | 355 | CE | LYS | C | 5 | 15.154 | -110.063 | -34.958 | 1.00261.22 |
| ATOM | 356 | NZ | LYS | C | 5 | 14.231 | -110.755 | -35.898 | 1.00271.57 |
| ATOM | 357 | N | GLU | C | 6 | 19.730 | -112.478 | -29.909 | 1.00225.50 |
| ATOM | 358 | CA | GLU | C | 6 | 20.977 | -113.162 | -29.590 | 1.00226.93 |
| ATOM | 359 | C | GLU | C | 6 | 21.178 | -114.369 | -30.533 | 1.00238.32 |
| ATOM | 360 | O | GLU | C | 6 | 20.208 | -115.076 | -30.857 | 1.00240.02 |
| ATOM | 361 | CB | GLU | C | 6 | 20.945 | -113.659 | -28.134 | 1.00226.31 |
| ATOM | 362 | CG | GLU | C | 6 | 20.497 | -112.620 | -27.114 | 1.00231.37 |
| ATOM | 363 | CD | GLU | C | 6 | 18.994 | -112.414 | -26.960 | 1.00243.21 |
| ATOM | 364 | OE1 | GLU | C | 6 | 18.238 | -112.715 | -27.913 | 1.00223.73 |
| ATOM | 365 | OE2 | GLU | C | 6 | 18.578 | -111.882 | -25.908 | 1.00237.51 |
| ATOM | 366 | N | SER | C | 7 | 22.429 | -114.587 | -30.981 | 1.00238.35 |
| ATOM | 367 | CA | SER | C | 7 | 22.827 | -115.745 | -31.792 | 1.00242.29 |
| ATOM | 368 | C | SER | C | 7 | 23.885 | -116.420 | -30.966 | 1.00247.19 |
| ATOM | 369 | O | SER | C | 7 | 25.010 | -115.916 | -30.881 | 1.00246.61 |
| ATOM | 370 | CB | SER | C | 7 | 23.382 | -115.349 | -33.164 | 1.00248.70 |
| ATOM | 371 | OG | SER | C | 7 | 23.934 | -114.042 | -33.211 | 1.00254.86 |
| ATOM | 372 | N | GLY | C | 8 | 23.489 | -117.496 | -30.296 | 1.00244.80 |
| ATOM | 373 | CA | GLY | C | 8 | 24.366 | -118.239 | -29.410 | 1.00244.75 |
| ATOM | 374 | C | GLY | C | 8 | 24.718 | -119.591 | -29.977 | 1.00251.22 |
| ATOM | 375 | O | GLY | C | 8 | 24.148 | -120.006 | -30.995 | 1.00252.95 |
| ATOM | 376 | N | PRO | C | 9 | 25.646 | -120.311 | -29.319 | 1.00248.44 |
| ATOM | 377 | CA | PRO | C | 9 | 26.038 | -121.625 | -29.832 | 1.00252.42 |
| ATOM | 378 | C | PRO | C | 9 | 25.159 | -122.772 | -29.330 | 1.00256.51 |
| ATOM | 379 | O | PRO | C | 9 | 25.321 | -123.906 | -29.796 | 1.00260.58 |
| ATOM | 380 | CB | PRO | C | 9 | 27.470 | -121.756 | -29.323 | 1.00255.02 |
| ATOM | 381 | CG | PRO | C | 9 | 27.409 | -121.113 | -27.973 | 1.00255.59 |
| ATOM | 382 | CD | PRO | C | 9 | 26.417 | -119.969 | -28.105 | 1.00247.91 |
| ATOM | 383 | N | GLY | C | 10 | 24.278 | -122.481 | -28.367 | 1.00248.27 |
| ATOM | 384 | CA | GLY | C | 10 | 23.399 | -123.471 | -27.761 | 1.00248.79 |
| ATOM | 385 | C | GLY | C | 10 | 24.093 | -124.338 | -26.728 | 1.00252.08 |
| ATOM | 386 | O | GLY | C | 10 | 23.618 | -124.449 | -25.593 | 1.00250.75 |
| ATOM | 387 | N | LEU | C | 11 | 25.216 | -124.971 | -27.116 | 1.00249.31 |
| ATOM | 388 | CA | LEU | C | 11 | 25.979 | -125.865 | -26.245 | 1.00249.44 |
| ATOM | 389 | C | LEU | C | 11 | 27.421 | -125.436 | -26.109 | 1.00252.08 |
| ATOM | 390 | O | LEU | C | 11 | 28.060 | -125.034 | -27.085 | 1.00251.88 |
| ATOM | 391 | CB | LEU | C | 11 | 25.925 | -127.316 | -26.758 | 1.00253.71 |
| ATOM | 392 | CG | LEU | C | 11 | 24.542 | -127.948 | -26.957 | 1.00257.43 |
| ATOM | 393 | CD1 | LEU | C | 11 | 24.648 | -129.329 | -27.523 | 1.00258.15 |
| ATOM | 394 | CD2 | LEU | C | 11 | 23.758 | -127.978 | -25.671 | 1.00259.04 |
| ATOM | 395 | N | VAL | C | 12 | 27.930 | -125.523 | -24.892 | 1.00248.11 |
| ATOM | 396 | CA | VAL | C | 12 | 29.316 | -125.189 | -24.589 | 1.00248.35 |
| ATOM | 397 | C | VAL | C | 12 | 29.84.5 | -126.333 | -23.744 | 1.00255.02 |
| ATOM | 398 | O | VAL | C | 12 | 29.216 | -126.680 | -22.746 | 1.00254.64 |
| ATOM | 399 | CB | VAL | C | 12 | 29.476 | -123.828 | -23.845 | 1.00248.71 |
| ATOM | 400 | CG1 | VAL | C | 12 | 30.923 | -123.600 | -23.391 | 1.00249.58 |
| ATOM | 401 | CG2 | VAL | C | 12 | 29.011 | -122.670 | -24.713 | 1.00246.11 |
| ATOM | 402 | N | ALA | C | 13 | 30.994 | -126.915 | -24.139 | 1.00254.27 |
| ATOM | 403 | CA | ALA | C | 13 | 31.649 | -127.980 | -23.388 | 1.00256.24 |
| ATOM | 404 | C | ALA | C | 13 | 32.343 | -127.331 | -22.195 | 1.00258.29 |
| ATOM | 405 | O | ALA | C | 13 | 32.885 | -126.225 | -22.322 | 1.00256.04 |
| ATOM | 406 | CB | ALA | C | 13 | 32.669 | -128.691 | -24.258 | 1.00257.95 |
| ATOM | 407 | N | PRO | C | 14 | 32.333 | -127.984 | -21.020 | 1.00255.63 |
| ATOM | 408 | CA | PRO | C | 14 | 32.994 | -127.378 | -19.855 | 1.00254.38 |
| ATOM | 409 | C | PRO | C | 14 | 34.430 | -127.007 | -20.214 | 1.00257.45 |
| ATOM | 410 | O | PRO | C | 14 | 35.072 | -127.703 | -21.003 | 1.00257.51 |
| ATOM | 411 | CB | PRO | C | 14 | 32.896 | -128.465 | -18.787 | 1.00256.74 |
| ATOM | 412 | CG | PRO | C | 14 | 31.773 | -129.350 | -19.240 | 1.00260.04 |
| ATOM | 413 | CD | PRO | C | 14 | 31.809 | -129.329 | -20.716 | 1.00256.97 |
| ATOM | 414 | N | SER | C | 15 | 34.886 | -125.852 | -19.713 | 1.00253.09 |
| ATOM | 415 | CA | SER | C | 15 | 36.211 | -125.274 | -19.963 | 1.00254.74 |
| ATOM | 416 | C | SER | C | 15 | 36.369 | -124.537 | -21.281 | 1.00259.01 |
| ATOM | 417 | O | SER | C | 15 | 37.408 | -123.912 | -21.514 | 1.00259.43 |
| ATOM | 418 | CB | SER | C | 15 | 37.326 | -126.287 | -19.753 | 1.00258.90 |
| ATOM | 419 | OG | SER | C | 15 | 37.423 | -126.568 | -18.370 | 1.00264.44 |
| ATOM | 420 | N | GLN | C | 16 | 35.340 | -124.589 | -22.138 | 1.00255.08 |
| ATOM | 421 | CA | GLN | C | 16 | 35.339 | -123.849 | -23.397 | 1.00254.27 |
| ATOM | 422 | C | GLN | C | 16 | 34.713 | -122.446 | -23.198 | 1.00253.72 |
| ATOM | 423 | O | GLN | C | 16 | 34.105 | -122.147 | -22.161 | 1.00249.79 |
| ATOM | 424 | CB | GLN | C | 16 | 34.598 | -124.617 | -24.506 | 1.00256.25 |
| ATOM | 425 | CG | GLN | C | 16 | 35.484 | -125.392 | -25.469 | 1.00258.15 |
| ATOM | 426 | CD | GLN | C | 16 | 36.796 | -124.735 | -25.838 | 1.00266.06 |
| ATOM | 427 | OE1 | GLN | C | 16 | 37.874 | -125.295 | -25.615 | 1.00263.70 |
| ATOM | 428 | NE2 | GLN | C | 16 | 36.736 | -123.556 | -26.447 | 1.00251.39 |
| ATOM | 429 | N | SER | C | 17 | 34.847 | -121.609 | -24.220 | 1.00250.17 |
| ATOM | 430 | CA | SER | C | 17 | 34.322 | -120.262 | -24.195 | 1.00246.15 |
| ATOM | 431 | C | SER | C | 17 | 32.925 | -120.187 | -24.798 | 1.00247.04 |
| ATOM | 432 | O | SER | C | 17 | 32.552 | -121.019 | -25.637 | 1.00247.81 |
| ATOM | 433 | CB | SER | C | 17 | 35.248 | -119.333 | -24.968 | 1.00251.44 |
| ATOM | 434 | OG | SER | C | 17 | 35.279 | -119.725 | -26.331 | 1.00262.40 |
| ATOM | 435 | N | LEU | C | 18 | 32.179 | -119.142 | -24.390 | 1.00239.71 |
| ATOM | 436 | CA | LEU | C | 18 | 30.845 | -118.808 | -24.877 | 1.00236.03 |
| ATOM | 437 | C | LEU | C | 18 | 30.951 | -117.535 | -25.690 | 1.00236.59 |
| ATOM | 438 | O | LEU | C | 18 | 31.449 | -116.520 | -25.201 | 1.00234.42 |
| ATOM | 439 | CB | LEU | C | 18 | 29.866 | -118.603 | -23.711 | 1.00232.94 |
| ATOM | 440 | CG | LEU | C | 18 | 28.521 | -117.971 | -24.018 | 1.00234.99 |
| ATOM | 441 | CD1 | LEU | C | 18 | 27.694 | -118.856 | -24.950 | 1.00236.30 |
| ATOM | 442 | CD2 | LEU | C | 18 | 27.746 | -117.705 | -22.724 | 1.00235.75 |
| ATOM | 443 | N | SER | C | 19 | 30.497 | -117.597 | -26.927 | 1.00233.64 |
| ATOM | 444 | CA | SER | C | 19 | 30.486 | -116.438 | -27.799 | 1.00233.48 |
| ATOM | 445 | C | SER | C | 19 | 29.043 | -116.213 | -28.232 | 1.00236.48 |
| ATOM | 446 | O | SER | C | 19 | 28.398 | -117.163 | -28.712 | 1.00238.69 |
| ATOM | 447 | CB | SER | C | 19 | 31.385 | -116.655 | -29.015 | 1.00240.18 |
| ATOM | 448 | OG | SER | C | 19 | 32.746 | -116.388 | -28.722 | 1.00247.52 |
| ATOM | 449 | N | ILE | C | 20 | 28.515 | -114.983 | -28.020 | 1.00227.96 |
| ATOM | 450 | CA | ILE | C | 20 | 27.159 | -114.655 | -28.446 | 1.00224.65 |
| ATOM | 451 | C | ILE | C | 20 | 27.181 | -113.355 | -29.198 | 1.00227.83 |
| ATOM | 452 | O | ILE | C | 20 | 27.792 | -112.394 | -28.741 | 1.00226.77 |
| ATOM | 453 | CB | ILE | C | 20 | 26.121 | -114.581 | -27.298 | 1.00223.52 |
| ATOM | 454 | CG1 | ILE | C | 20 | 26.103 | -115.842 | -26.457 | 1.00222.52 |
| ATOM | 455 | CG2 | ILE | C | 20 | 24.722 | -114.298 | -27.873 | 1.00224.78 |
| ATOM | 456 | CD1 | ILE | C | 20 | 25.387 | -115.688 | -25.173 | 1.00212.68 |
| ATOM | 457 | N | THR | C | 21 | 26.484 | -113.301 | -30.325 | 1.00225.00 |
| ATOM | 458 | CA | THR | C | 21 | 26.351 | -112.052 | -31.039 | 1.00224.68 |
| ATOM | 459 | C | THR | C | 21 | 24.926 | -111.537 | -30.829 | 1.00225.10 |
| ATOM | 460 | O | THR | C | 21 | 23.956 | -112.268 | -31.035 | 1.00224.93 |
| ATOM | 461 | CB | THR | C | 21 | 26.677 | -112.208 | -32.522 | 1.00232.85 |
| ATOM | 462 | OG1 | THR | C | 21 | 28.048 | -112.566 | -32.667 | 1.00231.60 |
| ATOM | 463 | CG2 | THR | C | 21 | 26.395 | -110.934 | -33.306 | 1.00230.66 |
| ATOM | 464 | N | CYS | C | 22 | 24.801 | -110.280 | -30.435 | 1.00218.33 |
| ATOM | 465 | CA | CYS | C | 22 | 23.497 | -109.651 | -30.303 | 1.00215.04 |
| ATOM | 466 | C | CYS | C | 22 | 23.364 | -108.761 | -31.526 | 1.00220.75 |
| ATOM | 467 | O | CYS | C | 22 | 24.202 | -107.870 | -31.733 | 1.00221.72 |
| ATOM | 468 | CB | CYS | C | 22 | 23.424 | -108.838 | -29.020 | 1.00211.51 |
| ATOM | 469 | SG | CYS | C | 22 | 21.899 | -107.871 | -28.812 | 1.00212.59 |
| ATOM | 470 | N | THR | C | 23 | 22.353 | -109.025 | -32.361 | 1.00216.64 |
| ATOM | 471 | CA | THR | C | 23 | 22.121 | -108.249 | -33.574 | 1.00217.30 |
| ATOM | 472 | C | THR | C | 23 | 20.965 | -107.308 | -33.226 | 1.00216.14 |
| ATOM | 473 | O | THR | C | 23 | 19.918 | -107.777 | -32.766 | 1.00212.99 |
| ATOM | 474 | CB | THR | C | 23 | 21.846 | -109.183 | -34.789 | 1.00220.47 |
| ATOM | 475 | OG1 | THR | C | 23 | 21.795 | -110.570 | -34.404 | 1.00211.66 |
| ATOM | 476 | CG2 | THR | C | 23 | 22.862 | -109.019 | -35.905 | 1.00222.87 |
| ATOM | 477 | N | VAL | C | 24 | 21.171 | -105.988 | -33.359 | 1.00211.41 |
| ATOM | 478 | CA | VAL | C | 24 | 20.105 | -105.051 | -33.006 | 1.00208.14 |
| ATOM | 479 | C | VAL | C | 24 | 19.610 | -104.298 | -34.237 | 1.00214.75 |
| ATOM | 480 | O | VAL | C | 24 | 20.346 | -104.150 | -35.218 | 1.00217.17 |
| ATOM | 481 | CB | VAL | C | 24 | 20.502 | -104.069 | -31.874 | 1.00208.29 |
| ATOM | 482 | CG1 | VAL | C | 24 | 21.138 | -104.781 | -30.695 | 1.00205.19 |
| ATOM | 483 | CG2 | VAL | C | 24 | 21.425 | -102.978 | -32.394 | 1.00210.37 |
| ATOM | 484 | N | SER | C | 25 | 18.377 | -103.799 | -34.176 | 1.00211.21 |
| ATOM | 485 | CA | SER | C | 25 | 17.833 | -102.978 | -35.255 | 1.00214.41 |
| ATOM | 486 | C | SER | C | 25 | 16.849 | -101.965 | -34.702 | 1.00216.37 |
| ATOM | 487 | O | SER | C | 25 | 16.310 | -102.161 | -33.613 | 1.00213.91 |
| ATOM | 488 | CB | SER | C | 25 | 17.206 | -103.829 | -36.362 | 1.00221.50 |
| ATOM | 489 | OG | SER | C | 25 | 16.068 | -104.545 | -35.914 | 1.00228.46 |
| ATOM | 490 | N | GLY | C | 26 | 16.640 | -100.883 | -35.441 | 1.00213.67 |
| ATOM | 491 | CA | GLY | C | 26 | 15.697 | -99.850 | -35.046 | 1.00211.94 |
| ATOM | 492 | C | GLY | C | 26 | 16.282 | -98.735 | -34.213 | 1.00212.67 |
| ATOM | 493 | O | GLY | C | 26 | 15.547 | -97.844 | -33.789 | 1.00212.53 |
| ATOM | 494 | N | PHE | C | 27 | 17.591 | -98.769 | -33.971 | 1.00207.09 |
| ATOM | 495 | CA | PHE | C | 27 | 18.288 | -97.732 | -33.221 | 1.00205.58 |
| ATOM | 496 | C | PHE | C | 27 | 19.756 | -97.843 | -33.493 | 1.00209.71 |
| ATOM | 497 | O | PHE | C | 27 | 20.206 | -98.942 | -33.853 | 1.00209.50 |
| ATOM | 498 | CB | PHE | C | 27 | 18.043 | -97.875 | -31.709 | 1.00203.51 |
| ATOM | 499 | CG | PHE | C | 27 | 18.668 | -99.083 | -31.052 | 1.00202.69 |
| ATOM | 500 | CD2 | PHE | C | 27 | 19.874 | -98.974 | -30.357 | 1.00203.91 |
| ATOM | 501 | CD1 | PHE | C | 27 | 18.023 | -100.322 | -31.079 | 1.00204.50 |
| ATOM | 502 | CE2 | PHE | C | 27 | 20.441 | -100.088 | -29.726 | 1.00204.86 |
| ATOM | 503 | CE1 | PHE | C | 27 | 18.578 | -101.438 | -30.434 | 1.00203.53 |
| ATOM | 504 | CZ | PHE | C | 27 | 19.782 | -101.312 | -29.755 | 1.00201.77 |
| ATOM | 505 | N | PRO | C | 28 | 20.523 | -96.747 | -33.243 | 1.00206.63 |
| ATOM | 506 | CA | PRO | C | 28 | 21.977 | -96.799 | -33.460 | 1.00208.79 |
| ATOM | 507 | C | PRO | C | 28 | 22.748 | -97.154 | -32.183 | 1.00210.19 |
| ATOM | 508 | O | PRO | C | 28 | 22.468 | -96.590 | -31.128 | 1.00208.24 |
| ATOM | 509 | CB | PRO | C | 28 | 22.310 | -95.377 | -33.942 | 1.00214.37 |
| ATOM | 510 | CG | PRO | C | 28 | 20.991 | -94.554 | -33.784 | 1.00217.56 |
| ATOM | 511 | CD | PRO | C | 28 | 20.103 | -95.373 | -32.904 | 1.00208.35 |
| ATOM | 512 | N | LEU | C | 29 | 23.718 | -98.091 | -32.272 | 1.00206.64 |
| ATOM | 513 | CA | LEU | C | 29 | 24.543 | -98.499 | -31.123 | 1.00203.72 |
| ATOM | 514 | C | LEU | C | 29 | 25.421 | -97.342 | -30.648 | 1.00207.57 |
| ATOM | 515 | O | LEU | C | 29 | 25.982 | -97.406 | -29.552 | 1.00206.13 |
| ATOM | 516 | CB | LEU | C | 29 | 25.417 | -99.729 | -31.460 | 1.00204.57 |
| ATOM | 517 | CG | LEU | C | 29 | 24.711 | -101.088 | -31.565 | 1.00206.92 |
| ATOM | 518 | CD1 | LEU | C | 29 | 25.657 | -102.149 | -32.081 | 1.00209.72 |
| ATOM | 519 | CD2 | LEU | C | 29 | 24.138 | -101.519 | -30.229 | 1.00204.33 |
| ATOM | 520 | N | THR | C | 30 | 25.547 | -96.288 | -31.469 | 1.00205.88 |
| ATOM | 521 | CA | THR | C | 30 | 26.348 | -95.132 | -31.087 | 1.00207.97 |
| ATOM | 522 | C | THR | C | 30 | 25.623 | -94.319 | -29.982 | 1.00210.17 |
| ATOM | 523 | O | THR | C | 30 | 26.282 | -93.666 | -29.177 | 1.00210.78 |
| ATOM | 524 | CB | THR | C | 30 | 26.718 | -94.262 | -32.324 | 1.00216.82 |
| ATOM | 525 | OG1 | THR | C | 30 | 25.548 | -93.677 | -32.878 | 1.00213.22 |
| ATOM | 526 | CG2 | THR | C | 30 | 27.421 | -95.039 | -33.418 | 1.00218.01 |
| ATOM | 527 | N | ALA | C | 31 | 24.272 | -94.367 | -29.945 | 1.00203.75 |
| ATOM | 528 | CA | ALA | C | 31 | 23.427 | -93.580 | -29.026 | 1.00200.55 |
| ATOM | 529 | C | ALA | C | 31 | 22.799 | -94.337 | -27.872 | 1.00196.90 |
| ATOM | 530 | O | ALA | C | 31 | 22.058 | -93.747 | -27.101 | 1.00193.76 |
| ATOM | 531 | CB | ALA | C | 31 | 22.329 | -92.882 | -29.823 | 1.00202.43 |
| ATOM | 532 | N | TYR | C | 32 | 23.008 | -95.637 | -27.795 | 1.00191.80 |
| ATOM | 533 | CA | TYR | C | 32 | 22.377 | -96.430 | -26.752 | 1.00188.57 |
| ATOM | 534 | C | TYR | C | 32 | 23.357 | -97.434 | -26.205 | 1.00193.25 |
| ATOM | 535 | O | TYR | C | 32 | 24.368 | -97.757 | -26.841 | 1.00195.66 |
| ATOM | 536 | CB | TYR | C | 32 | 21.118 | -97.154 | -27.290 | 1.00188.15 |
| ATOM | 537 | CG | TYR | C | 32 | 19.949 | -96.239 | -27.595 | 1.00190.21 |
| ATOM | 538 | CD1 | TYR | C | 32 | 19.816 | -95.632 | -28.841 | 1.00194.62 |
| ATOM | 539 | CD2 | TYR | C | 32 | 18.979 | -95.978 | -26.642 | 1.00189.28 |
| ATOM | 540 | CE1 | TYR | C | 32 | 18.762 | -94.763 | -29.117 | 1.00195.12 |
| ATOM | 541 | CE2 | TYR | C | 32 | 17.915 | -95.117 | -26.910 | 1.00190.93 |
| ATOM | 542 | CZ | TYR | C | 32 | 17.802 | -94.522 | -28.155 | 1.00197.11 |
| ATOM | 543 | OH | TYR | C | 32 | 16.735 | -93.700 | -28.434 | 1.00194.23 |
| ATOM | 544 | N | GLY | C | 33 | 23.081 | -97.913 | -25.016 | 1.00187.51 |
| ATOM | 545 | CA | GLY | C | 33 | 23.960 | -98.920 | -24.454 | 1.00186.96 |
| ATOM | 546 | C | GLY | C | 33 | 23.301 | -100.254 | -24.667 | 1.00188.61 |
| ATOM | 547 | O | GLY | C | 33 | 22.103 | -100.298 | -24.990 | 1.00187.43 |
| ATOM | 548 | N | VAL | C | 34 | 24.074 | -101.339 | -24.518 | 1.00184.25 |
| ATOM | 549 | CA | VAL | C | 34 | 23.529 | -102.694 | -24.602 | 1.00182.32 |
| ATOM | 550 | C | VAL | C | 34 | 24.083 | -103.493 | -23.444 | 1.00184.30 |
| ATOM | 551 | O | VAL | C | 34 | 25.299 | -103.567 | -23.252 | 1.00183.93 |
| ATOM | 552 | CB | VAL | C | 34 | 23.753 | -103.416 | -25.960 | 1.00187.86 |
| ATOM | 553 | CG1 | VAL | C | 34 | 23.394 | -104.906 | -25.865 | 1.00186.65 |
| ATOM | 554 | CG2 | VAL | C | 34 | 22.948 | -102.748 | -27.071 | 1.00188.75 |
| ATOM | 555 | N | ASN | C | 35 | 23.189 | -104.086 | -22.678 | 1.00180.82 |
| ATOM | 556 | CA | ASN | C | 35 | 23.557 | -104.903 | -21.534 | 1.00182.01 |
| ATOM | 557 | C | ASN | C | 35 | 23.344 | -106.389 | -21.799 | 1.00188.69 |
| ATOM | 558 | O | ASN | C | 35 | 22.603 | -106.757 | -22.715 | 1.00190.26 |
| ATOM | 559 | CB | ASN | C | 35 | 22.700 | -104.531 | -20.339 | 1.00183.40 |
| ATOM | 560 | CG | ASN | C | 35 | 22.572 | -103.068 | -20.112 | 1.00215.20 |
| ATOM | 561 | OD1 | ASN | C | 35 | 22.234 | -102.303 | -21.019 | 1.00216.32 |
| ATOM | 562 | ND2 | ASN | C | 35 | 22.797 | -102.650 | -18.897 | 1.00209.15 |
| ATOM | 563 | N | TRP | C | 36 | 23.949 | -107.240 | -20.952 | 1.00183.77 |
| ATOM | 564 | CA | TRP | C | 36 | 23.749 | -108.664 | -21.002 | 1.00182.96 |
| ATOM | 565 | C | TRP | C | 36 | 23.333 | -109.071 | -19.659 | 1.00181.87 |
| ATOM | 566 | O | TRP | C | 36 | 23.905 | -108.642 | -18.658 | 1.00180.07 |
| ATOM | 567 | CB | TRP | C | 36 | 25.007 | -109.385 | -21.393 | 1.00184.61 |
| ATOM | 568 | CG | TRP | C | 36 | 25.387 | -109.152 | -22.803 | 1.00188.10 |
| ATOM | 569 | CD1 | TRP | C | 36 | 26.153 | -108.131 | -23.296 | 1.00192.31 |
| ATOM | 570 | CD2 | TRP | C | 36 | 25.012 | -109.962 | -23.924 | 1.00189.30 |
| ATOM | 571 | NE1 | TRP | C | 36 | 26.290 | -108.261 | -24.660 | 1.00193.92 |
| ATOM | 572 | CE2 | TRP | C | 36 | 25.599 | -109.380 | -25.072 | 1.00195.52 |
| ATOM | 573 | CE3 | TRP | C | 36 | 24.249 | -111.146 | -24.068 | 1.00190.76 |
| ATOM | 574 | CZ2 | TRP | C | 36 | 25.444 | -109.938 | -26.347 | 1.00196.26 |
| ATOM | 575 | CZ3 | TRP | C | 36 | 24.093 | -111.692 | -25.331 | 1.00193.58 |
| ATOM | 576 | CH2 | TRP | C | 36 | 24.682 | -111.091 | -26.453 | 1.00195.59 |
| ATOM | 577 | N | VAL | C | 37 | 22.304 | -109.875 | -19.636 | 1.00179.40 |
| ATOM | 578 | CA | VAL | C | 37 | 21.676 | -110.412 | -18.444 | 1.00181.70 |
| ATOM | 579 | C | VAL | C | 37 | 21.515 | -111.952 | -18.656 | 1.00195.83 |
| ATOM | 580 | O | VAL | C | 37 | 21.262 | -112.399 | -19.778 | 1.00197.57 |
| ATOM | 581 | CB | VAL | C | 37 | 20.305 | -109.685 | -18.247 | 1.00182.78 |
| ATOM | 582 | CG1 | VAL | C | 37 | 19.437 | -110.335 | -17.172 | 1.00182.86 |
| ATOM | 583 | CG2 | VAL | C | 37 | 20.519 | -108.215 | -17.935 | 1.00181.91 |
| ATOM | 584 | N | ARG | C | 38 | 21.672 | -112.761 | -17.602 | 1.00195.92 |
| ATOM | 585 | CA | ARG | C | 38 | 21.411 | -114.193 | -17.730 | 1.00196.88 |
| ATOM | 586 | C | ARG | C | 38 | 20.446 | -114.670 | -16.654 | 1.00204.40 |
| ATOM | 587 | O | ARG | C | 38 | 20.318 | -114.045 | -15.582 | 1.00205.00 |
| ATOM | 588 | CB | ARG | C | 38 | 22.686 | -115.025 | -17.768 | 1.00194.92 |
| ATOM | 589 | CG | ARG | C | 38 | 23.368 | -115.188 | -16.421 | 1.00201.46 |
| ATOM | 590 | CD | ARG | C | 38 | 24.634 | -115.980 | -16.563 | 1.00208.80 |
| ATOM | 591 | NE | ARG | C | 38 | 25.328 | -116.107 | -15.287 | 1.00215.75 |
| ATOM | 592 | CZ | ARG | C | 38 | 26.477 | -116.753 | -15.116 | 1.00235.95 |
| ATOM | 593 | NH1 | ARG | C | 38 | 27.082 | -117.335 | -16.146 | 1.00233.11 |
| ATOM | 594 | NH2 | ARG | C | 38 | 27.025 | -116.835 | -13.912 | 1.00222.08 |
| ATOM | 595 | N | GLN | C | 39 | 19.749 | -115.758 | -16.959 | 1.00203.58 |
| ATOM | 596 | CA | GLN | C | 39 | 18.802 | -116.343 | -16.031 | 1.00207.41 |
| ATOM | 597 | C | GLN | C | 39 | 18.976 | -117.875 | -15.962 | 1.00216.73 |
| ATOM | 598 | O | GLN | C | 39 | 18.597 | -118.578 | -16.911 | 1.00217.45 |
| ATOM | 599 | CB | GLN | C | 39 | 17.369 | -115.958 | -16.412 | 1.00208.85 |
| ATOM | 600 | CG | GLN | C | 39 | 16.281 | -116.423 | -15.442 | 1.00230.23 |
| ATOM | 601 | CD | GLN | C | 39 | 14.944 | -115.800 | -15.781 | 1.00251.88 |
| ATOM | 602 | OE1 | GLN | C | 39 | 14.645 | -115.543 | -16.955 | 1.00254.54 |
| ATOM | 603 | NE2 | GLN | C | 39 | 14.103 | -115.540 | -14.769 | 1.00232.99 |
| ATOM | 604 | N | PRO | C | 40 | 19.552 | -118.424 | -14.862 | 1.00215.34 |
| ATOM | 605 | CA | PRO | C | 40 | 19.636 | -119.880 | -14.754 | 1.00217.60 |
| ATOM | 606 | C | PRO | C | 40 | 18.204 | -120.420 | -14.689 | 1.00221.18 |
| ATOM | 607 | O | PRO | C | 40 | 17.294 | -119.737 | -14.188 | 1.00219.80 |
| ATOM | 608 | CB | PRO | C | 40 | 20.383 | -120.087 | -13.435 | 1.00221.76 |
| ATOM | 609 | CG | PRO | C | 40 | 21.125 | -118.785 | -13.218 | 1.00223.58 |
| ATOM | 610 | CD | PRO | C | 40 | 20.115 | -117.781 | -13.657 | 1.00217.27 |
| ATOM | 611 | N | PRO | C | 41 | 17.966 | -121.622 | -15.241 | 1.00219.64 |
| ATOM | 612 | CA | PRO | C | 41 | 16.595 | -122.164 | -15.232 | 1.00222.58 |
| ATOM | 613 | C | PRO | C | 41 | 15.928 | -122.098 | -13.860 | 1.00227.93 |
| ATOM | 614 | O | PRO | C | 41 | 16.542 | -122.448 | -12.856 | 1.00228.37 |
| ATOM | 615 | CB | PRO | C | 41 | 16.768 | -123.584 | -15.777 | 1.00227.48 |
| ATOM | 616 | CG | PRO | C | 41 | 18.057 | -123.518 | -16.601 | 1.00228.79 |
| ATOM | 617 | CD | PRO | C | 41 | 18.930 | -122.565 | -15.856 | 1.00221.71 |
| ATOM | 618 | N | GLY | C | 42 | 14.728 | -121.528 | -13.827 | 1.00225.44 |
| ATOM | 619 | CA | GLY | C | 42 | 13.954 | -121.351 | -12.605 | 1.00229.26 |
| ATOM | 620 | C | GLY | C | 42 | 14.552 | -120.401 | -11.588 | 1.00232.21 |
| ATOM | 621 | O | GLY | C | 42 | 14.102 | -120.363 | -10.443 | 1.00234.41 |
| ATOM | 622 | N | LYS | C | 43 | 15.565 | -119.630 | -11.977 | 1.00226.56 |
| ATOM | 623 | CA | LYS | C | 43 | 16.175 | -118.671 | -11.054 | 1.00226.60 |
| ATOM | 624 | C | LYS | C | 43 | 15.999 | -117.244 | -11.529 | 1.00230.10 |
| ATOM | 625 | O | LYS | C | 43 | 15.356 | -117.016 | -12.553 | 1.00228.89 |
| ATOM | 626 | CB | LYS | C | 43 | 17.646 | -119.011 | -10.774 | 1.00227.73 |
| ATOM | 627 | CG | LYS | C | 43 | 17.801 | -120.286 | -9.928 | 1.00235.99 |
| ATOM | 628 | CD | LYS | C | 43 | 19.257 | -120.582 | -9.548 | 1.00229.20 |
| ATOM | 629 | CE | LYS | C | 43 | 19.408 | -121.717 | -8.566 | 1.00223.07 |
| ATOM | 630 | NZ | LYS | C | 43 | 19.240 | -121.255 | -7.164 | 1.00226.42 |
| ATOM | 631 | N | GLY | C | 44 | 16.528 | -116.298 | -10.766 | 1.00227.49 |
| ATOM | 632 | CA | GLY | C | 44 | 16.403 | -114.882 | -11.077 | 1.00224.80 |
| ATOM | 633 | C | GLY | C | 44 | 17.303 | -114.358 | -12.177 | 1.00224.29 |
| ATOM | 634 | O | GLY | C | 44 | 17.899 | -115.127 | -12.948 | 1.00222.39 |
| ATOM | 635 | N | LEU | C | 45 | 17.417 | -113.015 | -12.219 | 1.00218.10 |
| ATOM | 636 | CA | LEU | C | 45 | 18.219 | -112.305 | -13.207 | 1.00212.98 |
| ATOM | 637 | C | LEU | C | 45 | 19.522 | -111.854 | -12.644 | 1.00212.75 |
| ATOM | 638 | O | LEU | C | 45 | 19.599 | -111.383 | -11.505 | 1.00213.02 |
| ATOM | 639 | CB | LEU | C | 45 | 17.451 | -111.107 | -13.773 | 1.00211.35 |
| ATOM | 640 | CG | LEU | C | 45 | 16.101 | -111.428 | -14.388 | 1.00216.60 |
| ATOM | 641 | CD1 | LEU | C | 45 | 15.349 | -110.177 | -14.707 | 1.00214.41 |
| ATOM | 642 | CD2 | LEU | C | 45 | 16.262 | -112.308 | -15.635 | 1.00220.84 |
| ATOM | 643 | N | GLU | C | 46 | 20.550 | -111.996 | -13.449 | 1.00206.80 |
| ATOM | 644 | CA | GLU | C | 46 | 21.878 | -111.598 | -13.047 | 1.00207.29 |
| ATOM | 645 | C | GLU | C | 46 | 22.413 | -110.658 | -14.091 | 1.00207.36 |
| ATOM | 646 | O | GLU | C | 46 | 22.516 | -111.028 | -15.255 | 1.00204.84 |
| ATOM | 647 | CB | GLU | C | 46 | 22.804 | -112.832 | -12.892 | 1.00210.88 |
| ATOM | 648 | CG | GLU | C | 46 | 24.185 | -112.521 | -12.308 | 1.00226.10 |
| ATOM | 649 | CD | GLU | C | 46 | 25.215 | -113.640 | -12.282 | 1.00249.52 |
| ATOM | 650 | OE1 | GLU | C | 46 | 26.176 | -113.524 | -11.488 | 1.00234.02 |
| ATOM | 651 | OE2 | GLU | C | 46 | 25.080 | -114.613 | -13.061 | 1.00250.50 |
| ATOM | 652 | N | TRP | C | 47 | 22.777 | -109.450 | -13.683 | 1.00204.03 |
| ATOM | 653 | CA | TRP | C | 47 | 23.334 | -108.509 | -14.630 | 1.00202.23 |
| ATOM | 654 | C | TRP | C | 47 | 24.787 | -108.837 | -14.858 | 1.00203.49 |
| ATOM | 655 | O | TRP | C | 47 | 25.547 | -108.937 | -13.899 | 1.00205.62 |
| ATOM | 656 | CB | TRP | C | 47 | 23.173 | -107.095 | -14.114 | 1.00202.22 |
| ATOM | 657 | CG | TRP | C | 47 | 23.687 | -106.056 | -15.067 | 1.00202.39 |
| ATOM | 658 | CD1 | TRP | C | 47 | 23.016 | -105.492 | -16.116 | 1.00203.89 |
| ATOM | 659 | CD2 | TRP | C | 47 | 24.973 | -105.422 | -15.020 | 1.00202.70 |
| ATOM | 660 | NE1 | TRP | C | 47 | 23.803 | -104.541 | -16.724 | 1.00202.88 |
| ATOM | 661 | CE2 | TRP | C | 47 | 25.000 | -104.459 | -16.054 | 1.00205.42 |
| ATOM | 662 | CE3 | TRP | C | 47 | 26.088 | -105.536 | -14.164 | 1.00205.20 |
| ATOM | 663 | CZ2 | TRP | C | 47 | 26.096 | -103.621 | -16.259 | 1.00205.20 |
| ATOM | 664 | CZ3 | TRP | C | 47 | 27.152 | -104.679 | -14.348 | 1.00207.04 |
| ATOM | 665 | CH2 | TRP | C | 47 | 27.158 | -103.748 | -15.396 | 1.00206.69 |
| ATOM | 666 | N | LEU | C | 48 | 25.177 | -108.976 | -16.117 | 1.00196.92 |
| ATOM | 667 | CA | LEU | C | 48 | 26.543 | -109.356 | -16.455 | 1.00197.76 |
| ATOM | 668 | C | LEU | C | 48 | 27.500 | -108.205 | -16.784 | 1.00202.09 |
| ATOM | 669 | O | LEU | C | 48 | 28.650 | -108.204 | -16.346 | 1.00203.51 |
| ATOM | 670 | CB | LEU | C | 48 | 26.540 | -110.406 | -17.583 | 1.00197.52 |
| ATOM | 671 | CG | LEU | C | 48 | 25.688 | -111.687 | -17.397 | 1.00201.93 |
| ATOM | 672 | CD1 | LEU | C | 48 | 25.764 | -112.574 | -18.635 | 1.00201.41 |
| ATOM | 673 | CD2 | LEU | C | 48 | 26.122 | -112.477 | -16.164 | 1.00206.21 |
| ATOM | 674 | N | GLY | C | 49 | 27.034 | -107.257 | -17.572 | 1.00197.73 |
| ATOM | 675 | CA | GLY | C | 49 | 27.842 | -106.124 | -17.996 | 1.00199.02 |
| ATOM | 676 | C | GLY | C | 49 | 27.114 | -105.325 | -19.056 | 1.00203.53 |
| ATOM | 677 | O | GLY | C | 49 | 26.000 | -105.698 | -19.443 | 1.00203.14 |
| ATOM | 678 | N | MET | C | 50 | 27.702 | -104.206 | -19.511 | 1.00199.85 |
| ATOM | 679 | CA | MET | C | 50 | 27.123 | -103.389 | -20.585 | 1.00197.85 |
| ATOM | 680 | C | MET | C | 50 | 28.200 | -102.658 | -21.372 | 1.00200.95 |
| ATOM | 681 | O | MET | C | 50 | 29.350 | -102.539 | -20.919 | 1.00200.81 |
| ATOM | 682 | CB | MET | C | 50 | 25.994 | -102.439 | -20.105 | 1.00199.09 |
| ATOM | 683 | CG | MET | C | 50 | 26.420 | -101.306 | -19.146 | 1.00204.41 |
| ATOM | 684 | SD | MET | C | 50 | 25.420 | -99.723 | -19.238 | 1.00208.15 |
| ATOM | 685 | CE | MET | C | 50 | 25.943 | -99.102 | -20.833 | 1.00205.84 |
| ATOM | 686 | N | ILE | C | 51 | 27.831 | -102.217 | -22.569 | 1.00197.98 |
| ATOM | 687 | CA | ILE | C | 51 | 28.701 | -101.428 | -23.416 | 1.00201.37 |
| ATOM | 688 | C | ILE | C | 51 | 27.969 | -100.113 | -23.758 | 1.00208.60 |
| ATOM | 689 | O | ILE | C | 51 | 26.844 | -100.119 | -24.290 | 1.00205.82 |
| ATOM | 690 | CB | ILE | C | 51 | 29.224 | -102.187 | -24.660 | 1.00205.50 |
| ATOM | 691 | CG1 | ILE | C | 51 | 30.319 | -101.341 | -25.375 | 1.00209.38 |
| ATOM | 692 | CG2 | ILE | C | 51 | 28.061 | -102.569 | -25.603 | 1.00204.42 |
| ATOM | 693 | CD1 | ILE | C | 51 | 31.298 | -102.123 | -26.288 | 1.00214.02 |
| ATOM | 694 | N | TRP | C | 52 | 28.614 | -98.993 | -23.402 | 1.00210.00 |
| ATOM | 695 | CA | TRP | C | 52 | 28.094 | -97.647 | -23.620 | 1.00211.69 |
| ATOM | 696 | C | TRP | C | 52 | 28.235 | -97.257 | -25.070 | 1.00219.19 |
| ATOM | 697 | O | TRP | C | 52 | 29.000 | -97.874 | -25.813 | 1.00219.81 |
| ATOM | 698 | CB | TRP | C | 52 | 28.856 | -96.628 | -22.757 | 1.00213.35 |
| ATOM | 699 | CG | TRP | C | 52 | 28.680 | -96.786 | -21.271 | 1.00212.95 |
| ATOM | 700 | CD1 | TRP | C | 52 | 29.347 | -97.653 | -20.466 | 1.00215.60 |
| ATOM | 701 | CD2 | TRP | C | 52 | 27.831 | -96.008 | -20.404 | 1.00212.40 |
| ATOM | 702 | NE1 | TRP | C | 52 | 28.966 | -97.475 | -19.157 | 1.00214.41 |
| ATOM | 703 | CE2 | TRP | C | 52 | 28.026 | -96.482 | -19.091 | 1.00215.77 |
| ATOM | 704 | CE3 | TRP | C | 52 | 26.943 | -94.940 | -20.605 | 1.00214.25 |
| ATOM | 705 | CZ2 | TRP | C | 52 | 27.355 | -95.937 | -17.984 | 1.00215.34 |
| ATOM | 706 | CZ3 | TRP | C | 52 | 26.259 | -94.418 | -19.510 | 1.00215.51 |
| ATOM | 707 | CH2 | TRP | C | 52 | 26.464 | -94.918 | -18.220 | 1.00215.83 |
| ATOM | 708 | N | GLY | C | 53 | 27.542 | -96.196 | -25.451 | 1.00218.79 |
| ATOM | 709 | CA | GLY | C | 53 | 27.620 | -95.683 | -26.810 | 1.00222.72 |
| ATOM | 710 | C | GLY | C | 53 | 29.044 | -95.475 | -27.285 | 1.00232.92 |
| ATOM | 711 | O | GLY | C | 53 | 29.365 | -95.826 | -28.423 | 1.00236.09 |
| ATOM | 712 | N | ASP | C | 54 | 29.923 | -94.958 | -26.399 | 1.00230.73 |
| ATOM | 713 | CA | ASP | C | 54 | 31.328 | -94.674 | -26.720 | 1.00235.34 |
| ATOM | 714 | C | ASP | C | 54 | 32.263 | -95.903 | -26.742 | 1.00235.81 |
| ATOM | 715 | O | ASP | C | 54 | 33.466 | -95.752 | -26.975 | 1.00239.34 |
| ATOM | 716 | CB | ASP | C | 54 | 31.882 | -93.583 | -25.777 | 1.00240.48 |
| ATOM | 717 | CG | ASP | C | 54 | 32.190 | -94.043 | -24.355 | 1.00253.43 |
| ATOM | 718 | OD1 | ASP | C | 54 | 31.789 | -95.167 | -23.989 | 1.00251.99 |
| ATOM | 719 | OD2 | ASP | C | 54 | 32.815 | -93.268 | -23.603 | 1.00260.96 |
| ATOM | 720 | N | GLY | C | 55 | 31.717 | -97.082 | -26.456 | 1.00225.76 |
| ATOM | 721 | CA | GLY | C | 55 | 32.500 | -98.308 | -26.440 | 1.00224.79 |
| ATOM | 722 | C | GLY | C | 55 | 33.111 | -98.656 | -25.104 | 1.00224.61 |
| ATOM | 723 | O | GLY | C | 55 | 33.688 | -99.736 | -24.966 | 1.00223.85 |
| ATOM | 724 | N | ASN | C | 56 | 33.002 | -97.762 | -24.112 | 1.00219.23 |
| ATOM | 725 | CA | ASN | C | 56 | 33.515 | -98.055 | -22.780 | 1.00218.32 |
| ATOM | 726 | C | ASN | C | 56 | 32.591 | -99.101 | -22.181 | 1.00217.60 |
| ATOM | 727 | O | ASN | C | 56 | 31.432 | -99.204 | -22.593 | 1.00213.43 |
| ATOM | 728 | CB | ASN | C | 56 | 33.725 | -96.759 | -21.939 | 1.00220.27 |
| ATOM | 729 | CG | ASN | C | 56 | 32.974 | -96.501 | -20.638 | 1.00235.55 |
| ATOM | 730 | OD1 | ASN | C | 56 | 32.899 | -97.346 | -19.743 | 1.00228.70 |
| ATOM | 731 | ND2 | ASN | C | 56 | 32.595 | -95.235 | -20.419 | 1.00224.99 |
| ATOM | 732 | N | THR | C | 57 | 33.123 | -99.944 | -21.297 | 1.00215.56 |
| ATOM | 733 | CA | THR | C | 57 | 32.343 | -101.036 | -20.711 | 1.00212.34 |
| ATOM | 734 | C | THR | C | 57 | 32.343 | -101.064 | -19.207 | 1.00217.65 |
| ATOM | 735 | O | THR | C | 57 | 33.237 | -100.515 | -18.550 | 1.00220.14 |
| ATOM | 736 | CB | THR | C | 57 | 32.892 | -102.378 | -21.151 | 1.00221.76 |
| ATOM | 737 | OG1 | THR | C | 57 | 34.263 | -102.456 | -20.765 | 1.00223.39 |
| ATOM | 738 | CG2 | THR | C | 57 | 32.766 | -102.581 | -22.624 | 1.00224.51 |
| ATOM | 739 | N | ASP | C | 58 | 31.343 | -101.764 | -18.673 | 1.00212.49 |
| ATOM | 740 | CA | ASP | C | 58 | 31.196 | -102.017 | -17.255 | 1.00212.70 |
| ATOM | 741 | C | ASP | C | 58 | 30.925 | -103.506 | -17.113 | 1.00217.17 |
| ATOM | 742 | O | ASP | C | 58 | 30.044 | -104.026 | -17.807 | 1.00215.62 |
| ATOM | 743 | CB | ASP | C | 58 | 30.042 | -101.197 | -16.655 | 1.00212.25 |
| ATOM | 744 | CG | ASP | C | 58 | 30.359 | -99.743 | -16.431 | 1.00216.30 |
| ATOM | 745 | OD1 | ASP | C | 58 | 31.446 | -99.441 | -15.868 | 1.00218.24 |
| ATOM | 746 | OD2 | ASP | C | 58 | 29.524 | -98.908 | -16.792 | 1.00220.08 |
| ATOM | 747 | N | TYR | C | 59 | 31.697 | -104.197 | -16.248 | 1.00214.67 |
| ATOM | 748 | CA | TYR | C | 59 | 31.510 | -105.620 | -16.014 | 1.00213.53 |
| ATOM | 749 | C | TYR | C | 59 | 31.189 | -105.896 | -14.578 | 1.00218.58 |
| ATOM | 750 | O | TYR | C | 59 | 31.684 | -105.193 | -13.689 | 1.00218.59 |
| ATOM | 751 | CB | TYR | C | 59 | 32.752 | -106.409 | -16.402 | 1.00217.31 |
| ATOM | 752 | CG | TYR | C | 59 | 33.173 | -106.250 | -17.844 | 1.00220.65 |
| ATOM | 753 | CD1 | TYR | C | 59 | 32.277 | -106.487 | -18.884 | 1.00220.40 |
| ATOM | 754 | CD2 | TYR | C | 59 | 34.479 | -105.900 | -18.173 | 1.00225.31 |
| ATOM | 755 | CE1 | TYR | C | 59 | 32.665 | -106.352 | -20.211 | 1.00222.17 |
| ATOM | 756 | CE2 | TYR | C | 59 | 34.879 | -105.763 | -19.497 | 1.00227.62 |
| ATOM | 757 | CZ | TYR | C | 59 | 33.969 | -105.988 | -20.511 | 1.00232.58 |
| ATOM | 758 | OH | TYR | C | 59 | 34.380 | -105.861 | -21.812 | 1.00235.10 |
| ATOM | 759 | N | ASN | C | 60 | 30.362 | -106.936 | -14.352 | 1.00217.23 |
| ATOM | 760 | CA | ASN | C | 60 | 29.962 | -107.381 | -13.021 | 1.00219.89 |
| ATOM | 761 | C | ASN | C | 60 | 31.230 | -107.895 | -12.352 | 1.00230.39 |
| ATOM | 762 | O | ASN | C | 60 | 31.969 | -108.669 | -12.973 | 1.00231.39 |
| ATOM | 763 | CB | ASN | C | 60 | 28.874 | -108.477 | -13.116 | 1.00219.44 |
| ATOM | 764 | CG | ASN | C | 60 | 28.359 | -109.021 | -11.790 | 1.00245.88 |
| ATOM | 765 | OD1 | ASN | C | 60 | 29.104 | -109.483 | -10.924 | 1.00237.16 |
| ATOM | 766 | ND2 | ASN | C | 60 | 27.045 | -109.050 | -11.625 | 1.00240.38 |
| ATOM | 767 | N | SER | C | 61 | 31.526 | -107.416 | -11.125 | 1.00230.14 |
| ATOM | 768 | CA | SER | C | 61 | 32.720 | -107.822 | -10.369 | 1.00233.35 |
| ATOM | 769 | C | SER | C | 61 | 32.950 | -109.348 | -10.296 | 1.00236.26 |
| ATOM | 770 | O | SER | C | 61 | 34.106 | -109.786 | -10.235 | 1.00237.09 |
| ATOM | 771 | CB | SER | C | 61 | 32.704 | -107.212 | -8.976 | 1.00240.05 |
| ATOM | 772 | OG | SER | C | 61 | 31.447 | -107.405 | -8.347 | 1.00247.18 |
| ATOM | 773 | N | ALA | C | 62 | 31.866 | -110.153 | -10.369 | 1.00230.99 |
| ATOM | 774 | CA | ALA | C | 62 | 32.001 | -111.607 | -10.372 | 1.00231.64 |
| ATOM | 775 | C | ALA | C | 62 | 32.853 | -112.072 | -11.589 | 1.00234.58 |
| ATOM | 776 | O | ALA | C | 62 | 33.483 | -113.130 | -11.530 | 1.00236.98 |
| ATOM | 777 | CB | ALA | C | 62 | 30.626 | -112.269 | -10.381 | 1.00230.76 |
| ATOM | 778 | N | LEU | C | 63 | 32.895 | -111.240 | -12.657 | 1.00227.35 |
| ATOM | 779 | CA | LEU | C | 63 | 33.614 | -111.470 | -13.894 | 1.00226.19 |
| ATOM | 780 | C | LEU | C | 63 | 34.951 | -110.859 | -13.923 | 1.00234.56 |
| ATOM | 781 | O | LEU | C | 63 | 35.209 | -109.744 | -14.419 | 1.00230.13 |
| ATOM | 782 | CB | LEU | C | 63 | 32.811 | -111.226 | -15.154 | 1.00222.49 |
| ATOM | 783 | CG | LEU | C | 63 | 31.887 | -112.376 | -15.491 | 1.00224.54 |
| ATOM | 784 | CD1 | LEU | C | 63 | 30.527 | -111.904 | -15.975 | 1.00221.75 |
| ATOM | 785 | CD2 | LEU | C | 63 | 32.505 | -113.267 | -16.484 | 1.00226.67 |
| ATOM | 786 | N | LYS | C | 64 | 35.764 | -111.610 | -13.210 | 1.00240.77 |
| ATOM | 787 | CA | LYS | C | 64 | 37.188 | -111.574 | -12.992 | 1.00247.57 |
| ATOM | 788 | C | LYS | C | 64 | 37.876 | -111.925 | -14.315 | 1.00258.44 |
| ATOM | 789 | O | LYS | C | 64 | 38.385 | -113.049 | -14.490 | 1.00260.48 |
| ATOM | 790 | CB | LYS | C | 64 | 37.560 | -112.651 | -11.952 | 1.00252.35 |
| ATOM | 791 | CG | LYS | C | 64 | 37.101 | -112.398 | -10.523 | 1.00256.92 |
| ATOM | 792 | CD | LYS | C | 64 | 37.818 | -113.370 | -9.577 | 1.00257.87 |
| ATOM | 793 | CE | LYS | C | 64 | 37.526 | -113.121 | -8.117 | 1.00256.02 |
| ATOM | 794 | NZ | LYS | C | 64 | 38.024 | -114.232 | -7.262 | 1.00257.37 |
| ATOM | 795 | N | SER | C | 65 | 37.849 | -110.970 | -15.261 | 1.00256.63 |
| ATOM | 796 | CA | SER | C | 65 | 38.473 | -111.100 | -16.576 | 1.00258.13 |
| ATOM | 797 | C | SER | C | 65 | 37.924 | -112.257 | -17.440 | 1.00260.27 |
| ATOM | 798 | O | SER | C | 65 | 38.493 | -112.505 | -18.504 | 1.00261.93 |
| ATOM | 799 | CB | SER | C | 65 | 39.999 | -111.158 | -16.455 | 1.00266.26 |
| ATOM | 800 | OG | SER | C | 65 | 40.525 | -110.095 | -15.674 | 1.00270.82 |
| ATOM | 801 | N | ARG | C | 66 | 36.819 | -112.944 | -17.030 | 1.00252.90 |
| ATOM | 802 | CA | ARG | C | 66 | 36.253 | -113.996 | -17.888 | 1.00250.89 |
| ATOM | 803 | C | ARG | C | 66 | 35.377 | -113.347 | -18.961 | 1.00253.03 |
| ATOM | 804 | O | ARG | C | 66 | 35.226 | -113.954 | -20.012 | 1.00253.97 |
| ATOM | 805 | CB | ARG | C | 66 | 35.370 | -115.035 | -17.165 | 1.00246.36 |
| ATOM | 806 | CG | ARG | C | 66 | 35.776 | -115.462 | -15.774 | 1.00249.47 |
| ATOM | 807 | CD | ARG | C | 66 | 34.612 | -116.134 | -15.048 | 1.00247.68 |
| ATOM | 808 | NE | ARG | C | 66 | 34.204 | -117.382 | -15.698 | 1.00241.84 |
| ATOM | 809 | CZ | ARG | C | 66 | 33.252 | -118.201 | -15.262 | 1.00246.01 |
| ATOM | 810 | NH1 | ARG | C | 66 | 32.602 | -117.937 | -14.132 | 1.00218.66 |
| ATOM | 811 | NH2 | ARG | C | 66 | 32.977 | -119.316 | -15.924 | 1.00238.07 |
| ATOM | 812 | N | LEU | C | 67 | 34.801 | -112.135 | -18.705 | 1.00246.00 |
| ATOM | 813 | CA | LEU | C | 67 | 33.894 | -111.466 | -19.640 | 1.00242.37 |
| ATOM | 814 | C | LEU | C | 67 | 34.443 | -110.317 | -20.489 | 1.00243.25 |
| ATOM | 815 | O | LEU | C | 67 | 35.098 | -109.406 | -19.983 | 1.00244.65 |
| ATOM | 816 | CB | LEU | C | 67 | 32.608 | -111.032 | -18.934 | 1.00239.67 |
| ATOM | 817 | CG | LEU | C | 67 | 31.512 | -110.331 | -19.771 | 1.00243.36 |
| ATOM | 818 | CD1 | LEU | C | 67 | 30.780 | -111.318 | -20.678 | 1.00243.13 |
| ATOM | 819 | CD2 | LEU | C | 67 | 30.483 | -109.641 | -18.881 | 1.00245.65 |
| ATOM | 820 | N | SER | C | 68 | 34.090 | -110.351 | -21.785 | 1.00235.74 |
| ATOM | 821 | CA | SER | C | 68 | 34.421 | -109.333 | -22.771 | 1.00235.00 |
| ATOM | 822 | C | SER | C | 68 | 33.167 | -108.918 | -23.554 | 1.00232.82 |
| ATOM | 823 | O | SER | C | 68 | 32.447 | -109.781 | -24.064 | 1.00231.52 |
| ATOM | 824 | CB | SER | C | 68 | 35.488 | -109.847 | -23.733 | 1.00240.32 |
| ATOM | 825 | OG | SER | C | 68 | 36.788 | -109.660 | -23.195 | 1.00247.20 |
| ATOM | 826 | N | ILE | C | 69 | 32.892 | -107.603 | -23.626 | 1.00224.77 |
| ATOM | 827 | CA | ILE | C | 69 | 31.802 | -107.063 | -24.436 | 1.00220.28 |
| ATOM | 828 | C | ILE | C | 69 | 32.430 | -106.073 | -25.423 | 1.00224.29 |
| ATOM | 829 | O | ILE | C | 69 | 33.070 | -105.111 | -25.004 | 1.00224.82 |
| ATOM | 830 | CB | ILE | C | 69 | 30.642 | -106.410 | -23.641 | 1.00218.88 |
| ATOM | 831 | CG1 | ILE | C | 69 | 30.090 | -107.332 | -22.561 | 1.00215.03 |
| ATOM | 832 | CG2 | ILE | C | 69 | 29.515 | -105.971 | -24.602 | 1.00219.69 |
| ATOM | 833 | CD1 | ILE | C | 69 | 29.211 | -106.613 | -21.602 | 1.00209.36 |
| ATOM | 834 | N | SER | C | 70 | 32.275 | -106.323 | -26.719 | 1.00220.60 |
| ATOM | 835 | CA | SER | C | 70 | 32.773 | -105.437 | -27.767 | 1.00223.29 |
| ATOM | 836 | C | SER | C | 70 | 31.634 | -105.243 | -28.746 | 1.00225.11 |
| ATOM | 837 | O | SER | C | 70 | 30.589 | -105.876 | -28.600 | 1.00220.78 |
| ATOM | 838 | CB | SER | C | 70 | 33.996 | -106.037 | -28.461 | 1.00231.14 |
| ATOM | 839 | OG | SER | C | 70 | 33.843 | -107.420 | -28.748 | 1.00237.65 |
| ATOM | 840 | N | LYS | C | 71 | 31.805 | -104.375 | -29.731 | 1.00225.14 |
| ATOM | 841 | CA | LYS | C | 71 | 30.732 | -104.160 | -30.685 | 1.00224.70 |
| ATOM | 842 | C | LYS | C | 71 | 31.254 | -103.643 | -31.996 | 1.00233.46 |
| ATOM | 843 | O | LYS | C | 71 | 32.394 | -103.167 | -32.084 | 1.00236.49 |
| ATOM | 844 | CB | LYS | C | 71 | 29.711 | -103.156 | -30.116 | 1.00224.58 |
| ATOM | 845 | CG | LYS | C | 71 | 30.291 | -101.743 | -29.877 | 1.00239.01 |
| ATOM | 846 | CD | LYS | C | 71 | 29.235 | -100.743 | -29.366 | 1.00235.71 |
| ATOM | 847 | CE | LYS | C | 71 | 29.757 | -99.331 | -29.167 | 1.00233.04 |
| ATOM | 848 | NZ | LYS | C | 71 | 28.647 | -98.347 | -29.114 | 1.00235.49 |
| ATOM | 849 | N | ASP | C | 72 | 30.387 | -103.687 | -33.000 | 1.00231.02 |
| ATOM | 850 | CA | ASP | C | 72 | 30.674 | -103.135 | -34.301 | 1.00236.86 |
| ATOM | 851 | C | ASP | C | 72 | 29.493 | -102.251 | -34.621 | 1.00240.52 |
| ATOM | 852 | O | ASP | C | 72 | 28.433 | -102.755 | -35.014 | 1.00238.21 |
| ATOM | 853 | CB | ASP | C | 72 | 30.849 | -104.234 | -35.363 | 1.00242.01 |
| ATOM | 854 | CG | ASP | C | 72 | 31.255 | -103.698 | -36.739 | 1.00256.61 |
| ATOM | 855 | OD1 | ASP | C | 72 | 30.760 | -102.610 | -37.136 | 1.00258.39 |
| ATOM | 856 | OD2 | ASP | C | 72 | 32.062 | -104.358 | -37.415 | 1.00263.41 |
| ATOM | 857 | N | ASN | C | 73 | 29.668 | -100.936 | -34.457 | 1.00239.20 |
| ATOM | 858 | CA | ASN | C | 73 | 28.585 | -100.001 | -34.712 | 1.00238.45 |
| ATOM | 859 | C | ASN | C | 73 | 27.989 | -100.111 | -36.105 | 1.00245.64 |
| ATOM | 860 | O | ASN | C | 73 | 26.765 | -100.127 | -36.238 | 1.00243.29 |
| ATOM | 861 | CB | ASN | C | 73 | 28.975 | -98.561 | -34.369 | 1.00240.93 |
| ATOM | 862 | CG | ASN | C | 73 | 29.894 | -97.803 | -35.296 | 1.00259.01 |
| ATOM | 863 | OD1 | ASN | C | 73 | 30.422 | -98.298 | -36.304 | 1.00258.99 |
| ATOM | 864 | ND2 | ASN | C | 73 | 30.081 | -96.537 | -34.969 | 1.00244.98 |
| ATOM | 865 | N | SER | C | 74 | 28.844 | -100.217 | -37.134 | 1.00246.95 |
| ATOM | 866 | CA | SER | C | 74 | 28.370 | -100.269 | -38.506 | 1.00250.11 |
| ATOM | 867 | C | SER | C | 74 | 27.572 | -101.531 | -38.832 | 1.00250.27 |
| ATOM | 868 | O | SER | C | 74 | 26.728 | -101.487 | -39.722 | 1.00251.48 |
| ATOM | 869 | CB | SER | C | 74 | 29.515 | -100.050 | -39.483 | 1.00261.26 |
| ATOM | 870 | OG | SER | C | 74 | 30.453 | -101.109 | -39.403 | 1.00272.17 |
| ATOM | 871 | N | LYS | C | 75 | 27.798 | -102.629 | -38.098 | 1.00242.24 |
| ATOM | 872 | CA | LYS | C | 75 | 27.064 | -103.861 | -38.344 | 1.00240.07 |
| ATOM | 873 | C | LYS | C | 75 | 25.964 | -104.093 | -37.336 | 1.00239.90 |
| ATOM | 874 | O | LYS | C | 75 | 25.338 | -105.151 | -37.368 | 1.00239.13 |
| ATOM | 875 | CB | LYS | C | 75 | 28.013 | -105.061 | -38.428 | 1.00243.44 |
| ATOM | 876 | CG | LYS | C | 75 | 29.052 | -104.909 | -39.534 | 1.00256.59 |
| ATOM | 877 | CD | LYS | C | 75 | 29.412 | -106.230 | -40.217 | 1.00262.20 |
| ATOM | 878 | CE | LYS | C | 75 | 28.518 | -106.519 | -41.400 | 1.00264.12 |
| ATOM | 879 | NZ | LYS | C | 75 | 29.037 | -107.644 | -42.223 | 1.00269.50 |
| ATOM | 880 | N | SER | C | 76 | 25.691 | -103.101 | -36.461 | 1.00234.10 |
| ATOM | 881 | CA | SER | C | 76 | 24.652 | -103.195 | -35.421 | 1.00228.87 |
| ATOM | 882 | C | SER | C | 76 | 24.774 | -104.497 | -34.630 | 1.00228.56 |
| ATOM | 883 | O | SER | C | 76 | 23.788 | -105.207 | -34.406 | 1.00224.53 |
| ATOM | 884 | CB | SER | C | 76 | 23.257 | -103.065 | -36.023 | 1.00233.29 |
| ATOM | 885 | OG | SER | C | 76 | 23.003 | -101.731 | -36.424 | 1.00245.47 |
| ATOM | 886 | N | GLN | C | 77 | 26.003 | -104.829 | -34.253 | 1.00226.00 |
| ATOM | 887 | CA | GLN | C | 77 | 26.237 | -106.046 | -33.523 | 1.00223.85 |
| ATOM | 888 | C | GLN | C | 77 | 27.031 | -105.784 | -32.275 | 1.00226.91 |
| ATOM | 889 | O | GLN | C | 77 | 27.928 | -104.939 | -32.262 | 1.00226.07 |
| ATOM | 890 | CB | GLN | C | 77 | 26.960 | -107.060 | -34.402 | 1.00229.27 |
| ATOM | 891 | CG | GLN | C | 77 | 26.138 | -107.607 | -35.554 | 1.00250.74 |
| ATOM | 892 | CD | GLN | C | 77 | 26.943 | -108.494 | -36.491 | 1.00275.75 |
| ATOM | 893 | OE1 | GLN | C | 77 | 26.953 | -108.294 | -37.707 | 1.00273.34 |
| ATOM | 894 | NE2 | GLN | C | 77 | 27.661 | -109.490 | -35.970 | 1.00269.24 |
| ATOM | 895 | N | VAL | C | 78 | 26.692 | -106.521 | -31.219 | 1.00224.87 |
| ATOM | 896 | CA | VAL | C | 78 | 27.355 | -106.458 | -29.915 | 1.00225.43 |
| ATOM | 897 | C | VAL | C | 78 | 27.853 | -107.862 | -29.587 | 1.00232.32 |
| ATOM | 898 | O | VAL | C | 78 | 27.072 | -108.817 | -29.631 | 1.00229.97 |
| ATOM | 899 | CB | VAL | C | 78 | 26.404 | -105.933 | -28.815 | 1.00226.53 |
| ATOM | 900 | CG1 | VAL | C | 78 | 27.051 | -106.040 | -27.434 | 1.00225.19 |
| ATOM | 901 | CG2 | VAL | C | 78 | 25.968 | -104.501 | -29.107 | 1.00226.70 |
| ATOM | 902 | N | PHE | C | 79 | 29.123 | -107.986 | -29.219 | 1.00233.57 |
| ATOM | 903 | CA | PHE | C | 79 | 29.692 | -109.291 | -28.958 | 1.00235.73 |
| ATOM | 904 | C | PHE | C | 79 | 30.024 | -109.597 | -27.522 | 1.00238.72 |
| ATOM | 905 | O | PHE | C | 79 | 30.839 | -108.907 | -26.917 | 1.00238.58 |
| ATOM | 906 | CB | PHE | C | 79 | 30.916 | -109.522 | -29.850 | 1.00243.11 |
| ATOM | 907 | CG | PHE | C | 79 | 30.755 | -109.061 | -31.283 | 1.00248.87 |
| ATOM | 908 | CD2 | PHE | C | 79 | 31.393 | -107.912 | -31.737 | 1.00255.03 |
| ATOM | 909 | CD1 | PHE | C | 79 | 30.003 | -109.805 | -32.195 | 1.00253.44 |
| ATOM | 910 | CE2 | PHE | C | 79 | 31.262 | -107.494 | -33.073 | 1.00261.66 |
| ATOM | 911 | CE1 | PHE | C | 79 | 29.881 | -109.392 | -33.531 | 1.00258.18 |
| ATOM | 912 | CZ | PHE | C | 79 | 30.507 | -108.233 | -33.956 | 1.00260.39 |
| ATOM | 913 | N | LEU | C | 80 | 29.418 | -110.664 | -26.986 | 1.00234.81 |
| ATOM | 914 | CA | LEU | C | 80 | 29.722 | -111.169 | -25.651 | 1.00233.82 |
| ATOM | 915 | C | LEU | C | 80 | 30.682 | -112.344 | -25.836 | 1.00242.87 |
| ATOM | 916 | O | LEU | C | 80 | 30.466 | -113.191 | -26.705 | 1.00243.95 |
| ATOM | 917 | CB | LEU | C | 80 | 28.457 | -111.658 | -24.899 | 1.00230.61 |
| ATOM | 918 | CG | LEU | C | 80 | 28.694 | -112.361 | -23.538 | 1.00233.90 |
| ATOM | 919 | CD1 | LEU | C | 80 | 27.610 | -112.028 | -22.559 | 1.00230.90 |
| ATOM | 920 | CD2 | LEU | C | 80 | 28.829 | -113.901 | -23.693 | 1.00238.76 |
| ATOM | 921 | N | LYS | C | 81 | 31.717 | -112.418 | -25.010 | 1.00241.69 |
| ATOM | 922 | CA | LYS | C | 81 | 32.587 | -113.585 | -25.005 | 1.00243.98 |
| ATOM | 923 | C | LYS | C | 81 | 32.888 | -113.890 | -23.569 | 1.00247.67 |
| ATOM | 924 | O | LYS | C | 81 | 33.272 | -112.979 | -22.842 | 1.00246.97 |
| ATOM | 925 | CB | LYS | C | 81 | 33.874 | -113.409 | -25.824 | 1.00249.57 |
| ATOM | 926 | CG | LYS | C | 81 | 34.760 | -114.669 | -25.809 | 1.00257.00 |
| ATOM | 927 | CD | LYS | C | 81 | 36.116 | -114.393 | -25.125 | 1.00265.30 |
| ATOM | 928 | CE | LYS | C | 81 | 37.313 | -115.029 | -25.808 | 1.00276.15 |
| ATOM | 929 | NZ | LYS | C | 81 | 38.596 | -114.437 | -25.339 | 1.00283.50 |
| ATOM | 930 | N | MET | C | 82 | 32.695 | -115.144 | -23.138 | 1.00244.94 |
| ATOM | 931 | CA | MET | C | 82 | 33.009 | -115.524 | -21.758 | 1.00245.29 |
| ATOM | 932 | C | MET | C | 82 | 33.949 | -116.755 | -21.730 | 1.00256.44 |
| ATOM | 933 | O | MET | C | 82 | 33.651 | -117.777 | -22.348 | 1.00257.84 |
| ATOM | 934 | CB | MET | C | 82 | 31.733 | -115.766 | -20.949 | 1.00244.29 |
| ATOM | 935 | CG | MET | C | 82 | 31.873 | -115.514 | -19.470 | 1.00247.03 |
| ATOM | 936 | SD | MET | C | 82 | 30.220 | -115.625 | -18.710 | 1.00248.41 |
| ATOM | 937 | CE | MET | C | 82 | 30.256 | -117.297 | -18.099 | 1.00247.48 |
| ATOM | 938 | N | ASN | C | 83 | 35.090 | -116.647 | -21.032 | 1.00256.50 |
| ATOM | 939 | CA | ASN | C | 83 | 36.045 | -117.752 | -20.963 | 1.00260.34 |
| ATOM | 940 | C | ASN | C | 83 | 35.620 | -118.843 | -19.985 | 1.00265.37 |
| ATOM | 941 | O | ASN | C | 83 | 34.852 | -118.570 | -19.054 | 1.00263.30 |
| ATOM | 942 | CB | ASN | C | 83 | 37.495 | -117.231 | -20.789 | 1.00262.96 |
| ATOM | 943 | CG | ASN | C | 83 | 38.302 | -117.623 | -19.576 | 1.00280.41 |
| ATOM | 944 | OD1 | ASN | C | 83 | 38.523 | -118.796 | -19.292 | 1.00272.90 |
| ATOM | 945 | ND2 | ASN | C | 83 | 38.946 | -116.640 | -18.968 | 1.00274.23 |
| ATOM | 946 | N | SER | C | 84 | 36.130 | -120.079 | -20.229 | 1.00264.80 |
| ATOM | 947 | CA | SER | C | 84 | 35.975 | -121.326 | -19.463 | 1.00265.99 |
| ATOM | 948 | C | SER | C | 84 | 34.686 | -121.504 | -18.671 | 1.00267.85 |
| ATOM | 949 | O | SER | C | 84 | 34.660 | -121.398 | -17.435 | 1.00266.84 |
| ATOM | 950 | CB | SER | C | 84 | 37.208 | -121.585 | -18.600 | 1.00271.28 |
| ATOM | 951 | OG | SER | C | 84 | 37.267 | -122.867 | -17.995 | 1.00276.69 |
| ATOM | 952 | N | LEU | C | 85 | 33.623 | -121.818 | -19.393 | 1.00264.45 |
| ATOM | 953 | CA | LEU | C | 85 | 32.332 | -122.057 | -18.783 | 1.00263.47 |
| ATOM | 954 | C | LEU | C | 85 | 32.335 | -123.345 | -17.981 | 1.00271.20 |
| ATOM | 955 | O | LEU | C | 85 | 33.112 | -124.269 | -18.253 | 1.00273.51 |
| ATOM | 956 | CB | LEU | C | 85 | 31.220 | -122.058 | -19.851 | 1.00262.03 |
| ATOM | 957 | CG | LEU | C | 85 | 30.673 | -120.656 | -20.147 | 1.00263.64 |
| ATOM | 958 | CD1 | LEU | C | 85 | 31.672 | -119.863 | -20.959 | 1.00264.04 |
| ATOM | 959 | CD2 | LEU | C | 85 | 29.310 | -120.704 | -20.833 | 1.00265.31 |
| ATOM | 960 | N | GLN | C | 86 | 31.508 | -123.358 | -16.943 | 1.00267.03 |
| ATOM | 961 | CA | GLN | C | 86 | 31.287 | -124.505 | -16.099 | 1.00269.78 |
| ATOM | 962 | C | GLN | C | 86 | 29.789 | -124.747 | -16.095 | 1.00273.22 |
| ATOM | 963 | O | GLN | C | 86 | 29.027 | -123.922 | -16.611 | 1.00270.26 |
| ATOM | 964 | CB | GLN | C | 86 | 31.837 | -124.252 | -14.695 | 1.00271.53 |
| ATOM | 965 | CG | GLN | C | 86 | 33.326 | -124.496 | -14.615 | 1.00273.60 |
| ATOM | 966 | CD | GLN | C | 86 | 33.602 | -125.984 | -14.614 | 1.00277.19 |
| ATOM | 967 | OE1 | GLN | C | 86 | 33.485 | -126.691 | -13.604 | 1.00274.74 |
| ATOM | 968 | NE2 | GLN | C | 86 | 33.846 | -126.506 | -15.776 | 1.00263.98 |
| ATOM | 969 | N | THR | C | 87 | 29.365 | -125.885 | -15.552 | 1.00271.67 |
| ATOM | 970 | CA | THR | C | 87 | 27.956 | -126.270 | -15.500 | 1.00271.98 |
| ATOM | 971 | C | THR | C | 87 | 27.063 | -125.152 | -14.924 | 1.00271.97 |
| ATOM | 972 | O | THR | C | 87 | 25.992 | -124.906 | -15.470 | 1.00270.41 |
| ATOM | 973 | CB | THR | C | 87 | 27.800 | -127.632 | -14.807 | 1.00279.45 |
| ATOM | 974 | OG1 | THR | C | 87 | 28.146 | -127.493 | -13.430 | 1.00281.11 |
| ATOM | 975 | CG2 | THR | C | 87 | 28.667 | -128.720 | -15.448 | 1.00276.94 |
| ATOM | 976 | N | ASP | C | 88 | 27.531 | -124.426 | -13.889 | 1.00266.30 |
| ATOM | 977 | CA | ASP | C | 88 | 26.749 | -123.336 | -13.286 | 1.00263.24 |
| ATOM | 978 | C | ASP | C | 88 | 26.565 | -122.060 | -14.159 | 1.00261.40 |
| ATOM | 979 | O | ASP | C | 88 | 25.907 | -121.102 | -13.729 | 1.00258.59 |
| ATOM | 980 | CB | ASP | C | 88 | 27.260 | -123.010 | -11.876 | 1.00266.30 |
| ATOM | 981 | CG | ASP | C | 88 | 28.689 | -122.511 | -11.799 | 1.00270.13 |
| ATOM | 982 | OD1 | ASP | C | 88 | 29.060 | -121.940 | -10.753 | 1.00270.87 |
| ATOM | 983 | OD2 | ASP | C | 88 | 29.429 | -122.651 | -12.801 | 1.00271.67 |
| ATOM | 984 | N | ASP | C | 89 | 27.134 | -122.059 | -15.378 | 1.00255.73 |
| ATOM | 985 | CA | ASP | C | 89 | 26.984 | -120.966 | -16.333 | 1.00251.01 |
| ATOM | 986 | C | ASP | C | 89 | 25.844 | -121.318 | -17.295 | 1.00251.67 |
| ATOM | 987 | O | ASP | C | 89 | 25.564 | -120.570 | -18.237 | 1.00248.94 |
| ATOM | 988 | CB | ASP | C | 89 | 28.305 | -120.694 | -17.067 | 1.00252.62 |
| ATOM | 989 | CG | ASP | C | 89 | 29.403 | -120.153 | -16.155 | 1.00266.29 |
| ATOM | 990 | OD1 | ASP | C | 89 | 29.172 | -119.102 | -15.500 | 1.00265.27 |
| ATOM | 991 | OD2 | ASP | C | 89 | 30.489 | -120.793 | -16.080 | 1.00276.06 |
| ATOM | 992 | N | THR | C | 90 | 25.174 | -122.469 | -17.042 | 1.00248.92 |
| ATOM | 993 | CA | THR | C | 90 | 24.002 | -122.911 | -17.812 | 1.00247.97 |
| ATOM | 994 | C | THR | C | 90 | 22.870 | -121.925 | -17.475 | 1.00249.05 |
| ATOM | 995 | O | THR | C | 90 | 22.477 | -121.802 | -16.301 | 1.00249.29 |
| ATOM | 996 | CB | THR | C | 90 | 23.603 | -124.360 | -17.478 | 1.00250.67 |
| ATOM | 997 | OG1 | THR | C | 90 | 24.607 | -125.263 | -17.958 | 1.00250.51 |
| ATOM | 998 | CG2 | THR | C | 90 | 22.234 | -124.736 | -18.049 | 1.00245.32 |
| ATOM | 999 | N | ALA | C | 91 | 22.377 | -121.204 | -18.497 | 1.00242.43 |
| ATOM | 1000 | CA | ALA | C | 91 | 21.361 | -120.170 | -18.301 | 1.00239.45 |
| ATOM | 1001 | C | ALA | C | 91 | 20.873 | -119.660 | -19.635 | 1.00238.56 |
| ATOM | 1002 | O | ALA | C | 91 | 21.449 | -119.988 | -20.679 | 1.00238.92 |
| ATOM | 1003 | CB | ALA | C | 91 | 21.971 | -118.994 | -17.520 | 1.00237.93 |
| ATOM | 1004 | N | ARG | C | 92 | 19.802 | -118.849 | -19.602 | 1.00230.17 |
| ATOM | 1005 | CA | ARG | C | 92 | 19.322 | -118.170 | -20.794 | 1.00226.08 |
| ATOM | 1006 | C | ARG | C | 92 | 20.026 | -116.823 | -20.723 | 1.00222.86 |
| ATOM | 1007 | O | ARG | C | 92 | 20.020 | -116.167 | -19.670 | 1.00220.46 |
| ATOM | 1008 | CB | ARG | C | 92 | 17.784 | -118.026 | -20.829 | 1.00225.00 |
| ATOM | 1009 | CG | ARG | C | 92 | 17.289 | -117.250 | -22.059 | 1.00226.42 |
| ATOM | 1010 | CD | ARG | C | 92 | 15.793 | -117.357 | -22.301 | 1.00234.80 |
| ATOM | 1011 | NE | ARG | C | 92 | 15.512 | -118.553 | -23.095 | 1.00251.51 |
| ATOM | 1012 | CZ | ARG | C | 92 | 15.197 | -118.564 | -24.391 | 1.00267.75 |
| ATOM | 1013 | NH1 | ARG | C | 92 | 15.037 | -117.428 | -25.052 | 1.00257.16 |
| ATOM | 1014 | NH2 | ARG | C | 92 | 15.020 | -119.716 | -25.028 | 1.00253.47 |
| ATOM | 1015 | N | TYR | C | 93 | 20.680 | -116.449 | -21.824 | 1.00216.93 |
| ATOM | 1016 | CA | TYR | C | 93 | 21.434 | -115.204 | -21.948 | 1.00214.66 |
| ATOM | 1017 | C | TYR | C | 93 | 20.673 | -114.201 | -22.815 | 1.00213.82 |
| ATOM | 1018 | O | TYR | C | 93 | 20.343 | -114.534 | -23.958 | 1.00216.93 |
| ATOM | 1019 | CB | TYR | C | 93 | 22.819 | -115.487 | -22.560 | 1.00217.89 |
| ATOM | 1020 | CG | TYR | C | 93 | 23.764 | -116.172 | -21.593 | 1.00224.05 |
| ATOM | 1021 | CD1 | TYR | C | 93 | 23.539 | -117.483 | -21.174 | 1.00228.47 |
| ATOM | 1022 | CD2 | TYR | C | 93 | 24.869 | -115.500 | -21.069 | 1.00225.63 |
| ATOM | 1023 | CE1 | TYR | C | 93 | 24.384 | -118.109 | -20.254 | 1.00231.23 |
| ATOM | 1024 | CE2 | TYR | C | 93 | 25.714 | -116.111 | -20.132 | 1.00228.61 |
| ATOM | 1025 | CZ | TYR | C | 93 | 25.476 | -117.422 | -19.741 | 1.00240.55 |
| ATOM | 1026 | OH | TYR | C | 93 | 26.320 | -118.047 | -18.849 | 1.00247.46 |
| ATOM | 1027 | N | TYR | C | 94 | 20.410 | -112.979 | -22.292 | 1.00201.12 |
| ATOM | 1028 | CA | TYR | C | 94 | 19.705 | -111.924 | -23.018 | 1.00196.10 |
| ATOM | 1029 | C | TYR | C | 94 | 20.589 | -110.707 | -23.249 | 1.00201.56 |
| ATOM | 1030 | O | TYR | C | 94 | 21.357 | -110.326 | -22.362 | 1.00201.70 |
| ATOM | 1031 | CB | TYR | C | 94 | 18.552 | -111.380 | -22.193 | 1.00193.18 |
| ATOM | 1032 | CG | TYR | C | 94 | 17.563 | -112.363 | -21.653 | 1.00192.67 |
| ATOM | 1033 | CD1 | TYR | C | 94 | 16.364 | -112.598 | -22.310 | 1.00196.13 |
| ATOM | 1034 | CD2 | TYR | C | 94 | 17.752 | -112.949 | -20.414 | 1.00193.31 |
| ATOM | 1035 | CE1 | TYR | C | 94 | 15.399 | -113.442 | -21.776 | 1.00199.62 |
| ATOM | 1036 | CE2 | TYR | C | 94 | 16.801 | -113.798 | -19.868 | 1.00196.58 |
| ATOM | 1037 | CZ | TYR | C | 94 | 15.617 | -114.033 | -20.547 | 1.00206.72 |
| ATOM | 1038 | OH | TYR | C | 94 | 14.663 | -114.861 | -20.014 | 1.00212.42 |
| ATOM | 1039 | N | CYS | C | 95 | 20.421 | -110.038 | -24.389 | 1.00199.59 |
| ATOM | 1040 | CA | CYS | C | 95 | 21.031 | -108.734 | -24.582 | 1.00199.61 |
| ATOM | 1041 | C | CYS | C | 95 | 19.830 | -107.782 | -24.474 | 1.00200.06 |
| ATOM | 1042 | O | CYS | C | 95 | 18.727 | -108.112 | -24.914 | 1.00201.62 |
| ATOM | 1043 | CB | CYS | C | 95 | 21.799 | -108.585 | -25.892 | 1.00202.87 |
| ATOM | 1044 | SG | CYS | C | 95 | 20.826 | -108.864 | -27.388 | 1.00209.82 |
| ATOM | 1045 | N | ALA | C | 96 | 19.998 | -106.683 | -23.756 | 1.00192.01 |
| ATOM | 1046 | CA | ALA | C | 96 | 18.921 | -105.732 | -23.499 | 1.00189.07 |
| ATOM | 1047 | C | ALA | C | 96 | 19.449 | -104.350 | -23.728 | 1.00189.32 |
| ATOM | 1048 | O | ALA | C | 96 | 20.592 | -104.057 | -23.348 | 1.00189.59 |
| ATOM | 1049 | CB | ALA | C | 96 | 18.488 | -105.856 | -22.064 | 1.00189.10 |
| ATOM | 1050 | N | ARG | C | 97 | 18.632 | -103.487 | -24.334 | 1.00182.19 |
| ATOM | 1051 | CA | ARG | C | 97 | 19.062 | -102.103 | -24.576 | 1.00180.60 |
| ATOM | 1052 | C | ARG | C | 97 | 18.861 | -101.242 | -23.336 | 1.00181.37 |
| ATOM | 1053 | O | ARG | C | 97 | 17.965 | -101.488 | -22.544 | 1.00180.82 |
| ATOM | 1054 | CB | ARG | C | 97 | 18.250 | -101.471 | -25.722 | 1.00178.25 |
| ATOM | 1055 | CG | ARG | C | 97 | 18.748 | -100.083 | -26.143 | 1.00174.95 |
| ATOM | 1056 | CD | ARG | C | 97 | 17.874 | -99.442 | -27.197 | 1.00180.01 |
| ATOM | 1057 | NE | ARG | C | 97 | 16.659 | -98.785 | -26.692 | 1.00184.42 |
| ATOM | 1058 | CZ | ARG | C | 97 | 15.915 | -97.945 | -27.425 | 1.00194.45 |
| ATOM | 1059 | NH1 | ARG | C | 97 | 16.282 | -97.620 | -28.658 | 1.00183.13 |
| ATOM | 1060 | NH2 | ARG | C | 97 | 14.803 | -97.431 | -26.932 | 1.00173.66 |
| ATOM | 1061 | N | ASP | C | 98 | 19.669 | -100.222 | -23.181 | 1.00176.02 |
| ATOM | 1062 | CA | ASP | C | 98 | 19.426 | -99.269 | -22.137 | 1.00175.16 |
| ATOM | 1063 | C | ASP | C | 98 | 19.684 | -97.885 | -22.752 | 1.00175.24 |
| ATOM | 1064 | O | ASP | C | 98 | 20.704 | -97.699 | -23.453 | 1.00175.80 |
| ATOM | 1065 | CB | ASP | C | 98 | 20.189 | -99.565 | -20.815 | 1.00177.83 |
| ATOM | 1066 | CG | ASP | C | 98 | 21.662 | -99.145 | -20.705 | 1.00200.06 |
| ATOM | 1067 | OD2 | ASP | C | 98 | 22.240 | -99.294 | -19.618 | 1.00208.72 |
| ATOM | 1068 | OD1 | ASP | C | 98 | 22.262 | -98.776 | -21.730 | 1.00203.84 |
| ATOM | 1069 | N | PRO | C | 99 | 18.642 | -97.005 | -22.654 | 1.00165.90 |
| ATOM | 1070 | CA | PRO | C | 99 | 18.760 | -95.621 | -23.124 | 1.00165.12 |
| ATOM | 1071 | C | PRO | C | 99 | 19.373 | -94.720 | -22.025 | 1.00166.32 |
| ATOM | 1072 | O | PRO | C | 99 | 18.968 | -93.577 | -21.809 | 1.00166.08 |
| ATOM | 1073 | CB | PRO | C | 99 | 17.312 | -95.253 | -23.410 | 1.00166.93 |
| ATOM | 1074 | CG | PRO | C | 99 | 16.489 | -96.305 | -22.745 | 1.00170.01 |
| ATOM | 1075 | CD | PRO | C | 99 | 17.351 | -97.194 | -21.981 | 1.00165.23 |
| ATOM | 1076 | N | TYR | C | 100 | 20.394 | -95.261 | -21.363 | 1.00161.73 |
| ATOM | 1077 | CA | TYR | C | 100 | 21.175 | -94.747 | -20.251 | 1.00162.93 |
| ATOM | 1078 | C | TYR | C | 100 | 20.868 | -93.384 | -19.584 | 1.00169.39 |
| ATOM | 1079 | O | TYR | C | 100 | 20.686 | -93.364 | -18.367 | 1.00172.49 |
| ATOM | 1080 | CB | TYR | C | 100 | 22.681 | -95.007 | -20.397 | 1.00164.59 |
| ATOM | 1081 | CG | TYR | C | 100 | 23.358 | -94.615 | -21.696 | 1.00165.93 |
| ATOM | 1082 | CD1 | TYR | C | 100 | 23.433 | -95.509 | -22.763 | 1.00166.41 |
| ATOM | 1083 | CD2 | TYR | C | 100 | 24.097 | -93.442 | -21.787 | 1.00169.01 |
| ATOM | 1084 | CE1 | TYR | C | 100 | 24.167 | -95.210 | -23.909 | 1.00167.90 |
| ATOM | 1085 | CE2 | TYR | C | 100 | 24.875 | -93.162 | -22.907 | 1.00171.74 |
| ATOM | 1086 | CZ | TYR | C | 100 | 24.873 | -94.026 | -23.983 | 1.00177.51 |
| ATOM | 1087 | OH | TYR | C | 100 | 25.562 | -93.696 | -25.118 | 1.00182.67 |
| ATOM | 1088 | N | GLY | C | 101 | 20.847 | -92.283 | -20.310 | 1.00163.55 |
| ATOM | 1089 | CA | GLY | C | 101 | 20.574 | -91.011 | -19.653 | 1.00164.92 |
| ATOM | 1090 | C | GLY | C | 101 | 19.107 | -90.784 | -19.369 | 1.00167.63 |
| ATOM | 1091 | O | GLY | C | 101 | 18.739 | -90.153 | -18.378 | 1.00166.91 |
| ATOM | 1092 | N | SER | C | 102 | 18.268 | -91.302 | -20.256 | 1.00165.55 |
| ATOM | 1093 | CA | SER | C | 102 | 16.822 | -91.143 | -20.212 | 1.00167.29 |
| ATOM | 1094 | C | SER | C | 102 | 16.096 | -92.319 | -19.550 | 1.00172.53 |
| ATOM | 1095 | O | SER | C | 102 | 15.123 | -92.077 | -18.846 | 1.00175.22 |
| ATOM | 1096 | CB | SER | C | 102 | 16.252 | -90.940 | -21.621 | 1.00171.37 |
| ATOM | 1097 | OG | SER | C | 102 | 16.746 | -89.824 | -22.345 | 1.00179.02 |
| ATOM | 1098 | N | LYS | C | 103 | 16.473 | -93.579 | -19.830 | 1.00166.61 |
| ATOM | 1099 | CA | LYS | C | 103 | 15.727 | -94.701 | -19.258 | 1.00165.71 |
| ATOM | 1100 | C | LYS | C | 103 | 16.587 | -95.920 | -18.895 | 1.00173.62 |
| ATOM | 1101 | O | LYS | C | 103 | 17.725 | -96.054 | -19.356 | 1.00172.00 |
| ATOM | 1102 | CB | LYS | C | 103 | 14.628 | -95.164 | -20.247 | 1.00165.32 |
| ATOM | 1103 | CG | LYS | C | 103 | 13.422 | -94.248 | -20.443 | 1.00159.19 |
| ATOM | 1104 | CD | LYS | C | 103 | 12.208 | -94.987 | -21.035 | 1.00161.03 |
| ATOM | 1105 | CE | LYS | C | 103 | 12.270 | -95.294 | -22.524 | 1.00169.31 |
| ATOM | 1106 | NZ | LYS | C | 103 | 11.529 | -96.550 | -22.887 | 1.00176.67 |
| ATOM | 1107 | N | PRO | C | 104 | 16.020 | -96.872 | -18.129 | 1.00175.22 |
| ATOM | 1108 | CA | PRO | C | 104 | 16.725 | -98.133 | -17.872 | 1.00175.64 |
| ATOM | 1109 | C | PRO | C | 104 | 16.303 | -99.197 | -18.915 | 1.00182.32 |
| ATOM | 1110 | O | PRO | C | 104 | 15.294 | -98.982 | -19.604 | 1.00182.87 |
| ATOM | 1111 | CB | PRO | C | 104 | 16.232 | -98.515 | -16.481 | 1.00178.75 |
| ATOM | 1112 | CG | PRO | C | 104 | 14.874 | -97.950 | -16.421 | 1.00184.39 |
| ATOM | 1113 | CD | PRO | C | 104 | 14.702 | -96.886 | -17.476 | 1.00179.12 |
| ATOM | 1114 | N | MET | C | 105 | 17.053 | -100.347 | -19.008 | 1.00179.04 |
| ATOM | 1115 | CA | MET | C | 105 | 16.848 | -101.463 | -19.949 | 1.00177.87 |
| ATOM | 1116 | C | MET | C | 105 | 15.411 | -101.597 | -20.432 | 1.00186.09 |
| ATOM | 1117 | O | MET | C | 105 | 14.582 | -102.250 | -19.805 | 1.00187.73 |
| ATOM | 1118 | CB | MET | C | 105 | 17.341 | -102.820 | -19.411 | 1.00179.30 |
| ATOM | 1119 | CG | MET | C | 105 | 18.828 | -102.970 | -19.275 | 1.00181.80 |
| ATOM | 1120 | SD | MET | C | 105 | 19.064 | -104.640 | -18.631 | 1.00185.03 |
| ATOM | 1121 | CE | MET | C | 105 | 20.457 | -104.448 | -17.762 | 1.00181.47 |
| ATOM | 1122 | N | ASP | C | 106 | 15.125 | -100.916 | -21.534 | 1.00184.24 |
| ATOM | 1123 | CA | ASP | C | 106 | 13.870 | -100.879 | -22.279 | 1.00185.24 |
| ATOM | 1124 | C | ASP | C | 106 | 14.137 | -101.801 | -23.481 | 1.00191.65 |
| ATOM | 1125 | O | ASP | C | 106 | 15.167 | -101.621 | -24.133 | 1.00192.61 |
| ATOM | 1126 | CB | ASP | C | 106 | 13.658 | -99.416 | -22.744 | 1.00186.84 |
| ATOM | 1127 | CG | ASP | C | 106 | 14.565 | -98.886 | -23.866 | 1.00181.60 |
| ATOM | 1128 | OD1 | ASP | C | 106 | 15.805 | -99.142 | -23.825 | 1.00178.19 |
| ATOM | 1129 | OD2 | ASP | C | 106 | 14.043 | -98.214 | -24.768 | 1.00181.40 |
| ATOM | 1130 | N | TYR | C | 107 | 13.314 | -102.789 | -23.783 | 1.00189.18 |
| ATOM | 1131 | CA | TYR | C | 107 | 13.700 | -103.653 | -24.910 | 1.00190.12 |
| ATOM | 1132 | C | TYR | C | 107 | 14.765 | -104.731 | -24.640 | 1.00197.98 |
| ATOM | 1133 | O | TYR | C | 107 | 15.998 | -104.528 | -24.660 | 1.00195.18 |
| ATOM | 1134 | CB | TYR | C | 107 | 13.993 | -102.915 | -26.218 | 1.00191.38 |
| ATOM | 1135 | CG | TYR | C | 107 | 12.870 | -102.025 | -26.638 | 1.00194.83 |
| ATOM | 1136 | CD2 | TYR | C | 107 | 11.736 | -102.551 | -27.237 | 1.00196.86 |
| ATOM | 1137 | CD1 | TYR | C | 107 | 12.979 | -100.643 | -26.537 | 1.00198.02 |
| ATOM | 1138 | CE2 | TYR | C | 107 | 10.724 | -101.730 | -27.714 | 1.00199.52 |
| ATOM | 1139 | CE1 | TYR | C | 107 | 11.958 | -99.807 | -26.984 | 1.00202.06 |
| ATOM | 1140 | CZ | TYR | C | 107 | 10.830 | -100.359 | -27.580 | 1.00207.92 |
| ATOM | 1141 | OH | TYR | C | 107 | 9.795 | -99.579 | -28.052 | 1.00208.83 |
| ATOM | 1142 | N | TRP | C | 108 | 14.224 | -105.927 | -24.477 | 1.00200.80 |
| ATOM | 1143 | CA | TRP | C | 108 | 14.953 | -107.163 | -24.240 | 1.00202.32 |
| ATOM | 1144 | C | TRP | C | 108 | 14.886 | -108.068 | -25.451 | 1.00211.97 |
| ATOM | 1145 | O | TRP | C | 108 | 13.849 | -108.159 | -26.126 | 1.00213.65 |
| ATOM | 1146 | CB | TRP | C | 108 | 14.303 | -107.917 | -23.083 | 1.00201.78 |
| ATOM | 1147 | CG | TRP | C | 108 | 14.445 | -107.225 | -21.780 | 1.00202.06 |
| ATOM | 1148 | CD1 | TRP | C | 108 | 13.941 | -106.006 | -21.431 | 1.00205.01 |
| ATOM | 1149 | CD2 | TRP | C | 108 | 15.128 | -107.725 | -20.637 | 1.00201.85 |
| ATOM | 1150 | NE1 | TRP | C | 108 | 14.301 | -105.703 | -20.148 | 1.00204.54 |
| ATOM | 1151 | CE2 | TRP | C | 108 | 15.023 | -106.749 | -19.628 | 1.00205.93 |
| ATOM | 1152 | CE3 | TRP | C | 108 | 15.861 | -108.892 | -20.376 | 1.00203.48 |
| ATOM | 1153 | CZ2 | TRP | C | 108 | 15.630 | -106.907 | -18.374 | 1.00205.77 |
| ATOM | 1154 | CZ3 | TRP | C | 108 | 16.432 | -109.064 | -19.124 | 1.00205.24 |
| ATOM | 1155 | CH2 | TRP | C | 108 | 16.313 | -108.082 | -18.139 | 1.00205.99 |
| ATOM | 1156 | N | GLY | C | 109 | 15.973 | -108.780 | -25.693 | 1.00210.90 |
| ATOM | 1157 | CA | GLY | C | 109 | 15.972 | -109.799 | -26.731 | 1.00213.51 |
| ATOM | 1158 | C | GLY | C | 109 | 15.179 | -110.970 | -26.175 | 1.00221.08 |
| ATOM | 1159 | O | GLY | C | 109 | 14.909 | -111.008 | -24.964 | 1.00221.60 |
| ATOM | 1160 | N | GLN | C | 110 | 14.777 | -111.918 | -27.022 | 1.00219.60 |
| ATOM | 1161 | CA | GLN | C | 110 | 14.029 | -113.082 | -26.547 | 1.00221.61 |
| ATOM | 1162 | C | GLN | C | 110 | 14.922 | -113.982 | -25.668 | 1.00226.47 |
| ATOM | 1163 | O | GLN | C | 110 | 14.406 | -114.755 | -24.866 | 1.00226.50 |
| ATOM | 1164 | CB | GLN | C | 110 | 13.495 | -113.870 | -27.739 | 1.00225.94 |
| ATOM | 1165 | CG | GLN | C | 110 | 14.631 | -114.468 | -28.572 | 1.00249.21 |
| ATOM | 1166 | CD | GLN | C | 110 | 14.251 | -114.911 | -29.963 | 1.00273.76 |
| ATOM | 1167 | OE1 | GLN | C | 110 | 13.080 | -114.885 | -30.379 | 1.00271.32 |
| ATOM | 1168 | NE2 | GLN | C | 110 | 15.253 | -115.281 | -30.750 | 1.00266.05 |
| ATOM | 1169 | N | GLY | C | 111 | 16.242 | -113.869 | -25.833 | 1.00224.51 |
| ATOM | 1170 | CA | GLY | C | 111 | 17.200 | -114.674 | -25.091 | 1.00225.89 |
| ATOM | 1171 | C | GLY | C | 111 | 17.631 | -115.917 | -25.837 | 1.00235.97 |
| ATOM | 1172 | O | GLY | C | 111 | 16.908 | -116.399 | -26.713 | 1.00237.63 |
| ATOM | 1173 | N | THR | C | 112 | 18.826 | -116.429 | -25.518 | 1.00235.92 |
| ATOM | 1174 | CA | THR | C | 112 | 19.336 | -117.681 | -26.099 | 1.00240.05 |
| ATOM | 1175 | C | THR | C | 112 | 19.712 | -118.612 | -24.960 | 1.00244.66 |
| ATOM | 1176 | O | THR | C | 112 | 20.446 | -118.205 | -24.041 | 1.00243.52 |
| ATOM | 1177 | CB | THR | C | 112 | 20.515 | -117.503 | -27.120 | 1.00258.42 |
| ATOM | 1178 | OG1 | THR | C | 112 | 20.789 | -118.785 | -27.727 | 1.00264.47 |
| ATOM | 1179 | CG2 | THR | C | 112 | 21.811 | -116.938 | -26.465 | 1.00257.47 |
| ATOM | 1180 | N | SER | C | 113 | 19.217 | -119.858 | -25.018 | 1.00241.67 |
| ATOM | 1181 | CA | SER | C | 113 | 19.515 | -120.831 | -23.984 | 1.00241.49 |
| ATOM | 1182 | C | SER | C | 113 | 20.902 | -121.445 | -24.186 | 1.00243.68 |
| ATOM | 1183 | O | SER | C | 113 | 21.243 | -121.869 | -25.291 | 1.00245.11 |
| ATOM | 1184 | CB | SER | C | 113 | 18.419 | -121.884 | -23.915 | 1.00247.36 |
| ATOM | 1185 | OG | SER | C | 113 | 18.870 | -123.025 | -23.204 | 1.00257.84 |
| ATOM | 1186 | N | VAL | C | 114 | 21.711 | -121.441 | -23.118 | 1.00237.59 |
| ATOM | 1187 | CA | VAL | C | 114 | 23.071 | -121.977 | -23.119 | 1.00237.75 |
| ATOM | 1188 | C | VAL | C | 114 | 23.148 | -123.117 | -22.117 | 1.00245.35 |
| ATOM | 1189 | O | VAL | C | 114 | 22.818 | -122.935 | -20.936 | 1.00244.72 |
| ATOM | 1190 | CB | VAL | C | 114 | 24.157 | -120.900 | -22.834 | 1.00237.73 |
| ATOM | 1191 | CG1 | VAL | C | 114 | 25.552 | -121.521 | -22.777 | 1.00239.20 |
| ATOM | 1192 | CG2 | VAL | C | 114 | 24.127 | -119.808 | -23.889 | 1.00235.25 |
| ATOM | 1193 | N | THR | C | 115 | 23.612 | -124.287 | -22.593 | 1.00244.93 |
| ATOM | 1194 | CA | THR | C | 115 | 23.787 | -125.468 | -21.759 | 1.00247.53 |
| ATOM | 1195 | C | THR | C | 115 | 25.256 | -125.809 | -21.719 | 1.00249.43 |
| ATOM | 1196 | O | THR | C | 115 | 25.887 | -125.925 | -22.768 | 1.00249.03 |
| ATOM | 1197 | CB | THR | C | 115 | 22.966 | -126.641 | -22.298 | 1.00263.16 |
| ATOM | 1198 | OG1 | THR | C | 115 | 21.616 | -126.209 | -22.474 | 1.00263.76 |
| ATOM | 1199 | CG2 | THR | C | 115 | 23.025 | -127.876 | -21.380 | 1.00266.71 |
| ATOM | 1200 | N | VAL | C | 116 | 25.801 | -125.966 | -20.522 | 1.00244.82 |
| ATOM | 1201 | CA | VAL | C | 116 | 27.200 | -126.316 | -20.410 | 1.00245.14 |
| ATOM | 1202 | C | VAL | C | 116 | 27.260 | -127.766 | -19.981 | 1.00255.05 |
| ATOM | 1203 | O | VAL | C | 116 | 26.842 | -128.094 | -18.864 | 1.00256.45 |
| ATOM | 1204 | CB | VAL | C | 116 | 27.990 | -125.376 | -19.487 | 1.00245.20 |
| ATOM | 1205 | CG1 | VAL | C | 116 | 29.472 | -125.731 | -19.514 | 1.00246.44 |
| ATOM | 1206 | CG2 | VAL | C | 116 | 27.775 | -123.923 | -19.893 | 1.00240.81 |
| ATOM | 1207 | N | SER | C | 117 | 27.743 | -128.641 | -20.890 | 1.00254.39 |
| ATOM | 1208 | CA | SER | C | 117 | 27.864 | -130.090 | -20.678 | 1.00256.49 |
| ATOM | 1209 | C | SER | C | 117 | 28.654 | -130.757 | -21.818 | 1.00258.43 |
| ATOM | 1210 | O | SER | C | 117 | 28.888 | -130.140 | -22.853 | 1.00257.63 |
| ATOM | 1211 | CB | SER | C | 117 | 26.481 | -130.736 | -20.571 | 1.00259.10 |
| ATOM | 1212 | OG | SER | C | 117 | 26.604 | -132.124 | -20.286 | 1.00263.70 |
| ATOM | 1213 | N | SER | C | 118 | 29.029 | -132.034 | -21.655 | 1.00256.05 |
| ATOM | 1214 | CA | SER | C | 118 | 29.692 | -132.806 | -22.714 | 1.00253.96 |
| ATOM | 1215 | C | SER | C | 118 | 28.846 | -134.065 | -23.045 | 1.00238.13 |
| ATOM | 1216 | O | SER | C | 118 | 28.463 | -134.337 | -24.201 | 1.00176.20 |
| ATOM | 1217 | CB | SER | C | 118 | 31.111 | -133.183 | -22.298 | 1.00256.18 |
| ATOM | 1218 | OG | SER | C | 118 | 31.113 | -133.869 | -21.057 | 1.00261.64 |
| ATOM | 1220 | N | ASP | D | 1 | 30.700 | -101.896 | -5.650 | 1.00219.75 |
| ATOM | 1221 | CA | ASP | D | 1 | 29.635 | -102.694 | -6.264 | 1.00215.21 |
| ATOM | 1222 | C | ASP | D | 1 | 28.358 | -102.601 | -5.407 | 1.00214.43 |
| ATOM | 1223 | O | ASP | D | 1 | 28.441 | -102.803 | -4.193 | 1.00215.39 |
| ATOM | 1224 | CB | ASP | D | 1 | 30.091 | -104.167 | -6.442 | 1.00218.57 |
| ATOM | 1225 | CG | ASP | D | 1 | 29.804 | -104.786 | -7.813 | 1.00226.38 |
| ATOM | 1226 | OD2 | ASP | D | 1 | 28.649 | -105.237 | -8.040 | 1.00230.15 |
| ATOM | 1227 | OD1 | ASP | D | 1 | 30.740 | -104.856 | -8.641 | 1.00228.29 |
| ATOM | 1228 | N | ILE | D | 2 | 27.195 | -102.275 | -6.017 | 1.00205.37 |
| ATOM | 1229 | CA | ILE | D | 2 | 25.953 | -102.154 | -5.255 | 1.00200.95 |
| ATOM | 1230 | C | ILE | D | 2 | 25.204 | -103.475 | -5.202 | 1.00202.02 |
| ATOM | 1231 | O | ILE | D | 2 | 24.984 | -104.107 | -6.239 | 1.00200.75 |
| ATOM | 1232 | CB | ILE | D | 2 | 25.053 | -100.996 | -5.756 | 1.00200.66 |
| ATOM | 1233 | CG1 | ILE | D | 2 | 25.840 | -99.675 | -5.865 | 1.00203.17 |
| ATOM | 1234 | CG2 | ILE | D | 2 | 23.801 | -100.850 | -4.889 | 1.00198.20 |
| ATOM | 1235 | CD1 | ILE | D | 2 | 25.003 | -98.430 | -6.360 | 1.00207.75 |
| ATOM | 1236 | N | VAL | D | 3 | 24.790 | -103.875 | -3.995 | 1.00197.39 |
| ATOM | 1237 | CA | VAL | D | 3 | 24.013 | -105.091 | -3.790 | 1.00195.24 |
| ATOM | 1238 | C | VAL | D | 3 | 22.529 | -104.776 | -3.572 | 1.00192.85 |
| ATOM | 1239 | O | VAL | D | 3 | 22.185 | -103.909 | -2.767 | 1.00191.33 |
| ATOM | 1240 | CB | VAL | D | 3 | 24.583 | -105.958 | -2.656 | 1.00202.47 |
| ATOM | 1241 | CG1 | VAL | D | 3 | 23.638 | -107.107 | -2.309 | 1,00201.27 |
| ATOM | 1242 | CG2 | VAL | D | 3 | 25.955 | -106.497 | -3.033 | 1.00205.65 |
| ATOM | 1243 | N | MET | D | 4 | 21.666 | -105.532 | -4.263 | 1.00185.60 |
| ATOM | 1244 | CA | MET | D | 4 | 20.226 | -105.403 | -4.185 | 1.00182.08 |
| ATOM | 1245 | C | MET | D | 4 | 19.599 | -106.622 | -3.561 | 1.00183.32 |
| ATOM | 1246 | O | MET | D | 4 | 19.931 | -107.740 | -3.930 | 1.00184.69 |
| ATOM | 1247 | CB | MET | D | 4 | 19.639 | -105.223 | -5.581 | 1.00183.09 |
| ATOM | 1248 | CG | MET | D | 4 | 20.292 | -104.135 | -6.41.6 | 1.00188.24 |
| ATOM | 1249 | SD | MET | D | 4 | 20.260 | -102.506 | -5.653 | 1.00194.21 |
| ATOM | 1250 | CE | MET | D | 4 | 18.473 | -102.165 | -5.632 | 1.00187.91 |
| ATOM | 1251 | N | SER | D | 5 | 18.666 | -106.421 | -2.653 | 1.00178.23 |
| ATOM | 1252 | CA | SER | D | 5 | 17.972 | -107.516 | -1.995 | 1.00179.45 |
| ATOM | 1253 | C | SER | D | 5 | 16.462 | -107.305 | -1.950 | 1.00182.40 |
| ATOM | 1254 | O | SER | D | 5 | 16.004 | -106.237 | -1.551 | 1.00181.07 |
| ATOM | 1255 | | SER | D | 5 | 18.534 | -107.736 | -0.592 | 1.00187.61 |
| ATOM | 1256 | OG | SER | D | 5 | 18.639 | -106.542 | 0.169 | 1.00198.91 |
| ATOM | 1257 | N | GLN | D | 6 | 15.689 | -108.325 | -2.340 | 1.00179.97 |
| ATOM | 1258 | CA | GLN | D | 6 | 14.234 | -108.246 | -2.365 | 1.00179.57 |
| ATOM | 1259 | C | GLN | D | 6 | 13.543 | -109.232 | -1.426 | 1.00192.26 |
| ATOM | 1260 | O | GLN | D | 6 | 14.034 | -110.341 | -1.182 | 1.00192.53 |
| ATOM | 1261 | CB | GLN | D | 6 | 13.698 | -108.472 | -3.784 | 1.00178.04 |
| ATOM | 1262 | CG | GLN | D | 6 | 14.362 | -107.659 | -4.879 | 1.00149.98 |
| ATOM | 1263 | CD | GLN | D | 6 | 13.700 | -107.937 | -6.204 | 1.00172.08 |
| ATOM | 1264 | OE1 | GLN | D | 6 | 14.377 | -108.186 | -7.194 | 1.00168.54 |
| ATOM | 1265 | NE2 | GLN | D | 6 | 12.363 | -107.888 | -6.270 | 1.00172.65 |
| ATOM | 1266 | N | SER | D | 7 | 12.363 | -108.825 | -0.948 | 1.00195.02 |
| ATOM | 1267 | CA | SER | D | 7 | 11.482 | -109.616 | -0.088 | 1.00199.12 |
| ATOM | 1268 | C | SER | D | 7 | 10.018 | -109.289 | -0.459 | 1.00204.12 |
| ATOM | 1269 | O | SER | D | 7 | 9.722 | -108.146 | -0.806 | 1.00203.72 |
| ATOM | 1270 | CB | SER | D | 7 | 11.762 | -109.345 | 1.384 | 1.00206.38 |
| ATOM | 1271 | OG | SER | D | 7 | 11.483 | -107.991 | 1.694 | 1.00218.81 |
| ATOM | 1272 | N | PRO | D | 8 | 9.101 | -110.262 | -0.440 | 1.00201.18 |
| ATOM | 1273 | CA | PRO | D | 8 | 9.320 | -111.656 | -0.101 | 1.00203.06 |
| ATOM | 1274 | C | PRO | D | 8 | 10.019 | -112.312 | -1.282 | 1.00206.04 |
| ATOM | 1275 | O | PRO | D | 8 | 10.160 | -111.701 | -2.344 | 1.00203.51 |
| ATOM | 1276 | CB | PRO | D | 8 | 7.888 | -112.168 | 0.101 | 1.00206.78 |
| ATOM | 1277 | CG | PRO | D | 8 | 7.122 | -111.418 | -0.918 | 1.00209.40 |
| ATOM | 1278 | CD | PRO | D | 8 | 7.712 | -110.036 | -0.881 | 1.00202.71 |
| ATOM | 1279 | N | SER | D | 9 | 10.451 | -113.552 | -1.092 | 1.00202.99 |
| ATOM | 1280 | CA | SER | D | 9 | 11.048 | -114.348 | -2.141 | 1.00201.21 |
| ATOM | 1281 | C | SER | D | 9 | 9.855 | -114.770 | -3.033 | 1.00205.09 |
| ATOM | 1282 | O | SER | D | 9 | 10.017 | -114.981 | -4.237 | 1.00203.20 |
| ATOM | 1283 | CB | SER | D | 9 | 11.719 | -115.558 | -1.510 | 1.00205.36 |
| ATOM | 1284 | OG | SER | D | 9 | 12.059 | -115.332 | -0.145 | 1.00210.14 |
| ATOM | 1285 | N | SER | D | 10 | 8.637 | -114.811 | -2.415 | 1.00203.61 |
| ATOM | 1286 | CA | SER | D | 10 | 7.360 | -115.151 | -3.040 | 1.00204.25 |
| ATOM | 1287 | C | SER | D | 10 | 6.149 | -114.874 | -2.123 | 1.00207.98 |
| ATOM | 1288 | O | SER | D | 10 | 6.298 | -114.788 | -0.907 | 1.00208.80 |
| ATOM | 1289 | CB | SER | D | 10 | 7.369 | -116.613 | -3.475 | 1.00210.49 |
| ATOM | 1290 | OG | SER | D | 10 | 7.631 | -117.478 | -2.382 | 1.00220.56 |
| ATOM | 1291 | N | LEU | D | 11 | 4.948 | -114.768 | -2.713 | 1.00203.36 |
| ATOM | 1292 | CA | LEU | D | 11 | 3.687 | -114.551 | -1.995 | 1.00204.47 |
| ATOM | 1293 | C | LEU | D | 11 | 2.507 | -114.921 | -2.888 | 1.00209.45 |
| ATOM | 1294 | O | LEU | D | 11 | 2.670 | -115.019 | -4.104 | 1.00206.09 |
| ATOM | 1295 | CB | LEU | D | 11 | 3.581 | -113.092 | -1.507 | 1.00202.35 |
| ATOM | 1296 | CG | LEU | D | 11 | 3.351 | -111.996 | -2.562 | 1.00203.05 |
| ATOM | 1297 | CD1 | LEU | D | 11 | 2.869 | -110.750 | -1.921 | 1.00202.20 |
| ATOM | 1298 | CD2 | LEU | D | 11 | 4.594 | -111.735 | -3.408 | 1.00199.95 |
| ATOM | 1299 | N | VAL | D | 12 | 1.328 | -115.135 | -2.291 | 1.00211.94 |
| ATOM | 1300 | CA | VAL | D | 12 | 0.114 | -115.484 | -3.042 | 1.00216.37 |
| ATOM | 1301 | C | VAL | D | 12 | -1.003 | -114.502 | -2.714 | 1.00222.00 |
| ATOM | 1302 | O | VAL | D | 12 | -1.174 | -114.145 | -1.547 | 1.00222.49 |
| ATOM | 1303 | CB | VAL | D | 12 | -0.343 | -116.964 | -2.846 | 1.00226.79 |
| ATOM | 1304 | CG1 | VAL | D | 12 | 0.840 | -117.947 | -2.&88 | 1.00226.01 |
| ATOM | 1305 | CG2 | VAL | D | 12 | -1.153 | -117.160 | -1.555 | 1.00231.27 |
| ATOM | 1306 | N | VAL | D | 13 | -1.767 | -114.081 | -3.728 | 1.00219.56 |
| ATOM | 1307 | CA | VAL | D | 13 | -2.882 | -113.144 | -3.568 | 1.00221.45 |
| ATOM | 1308 | C | VAL | D | 13 | -4.003 | -113.475 | -4.533 | 1.00227.02 |
| ATOM | 1309 | O | VAL | D | 13 | -3.734 | -113.999 | -5.617 | 1.00225.91 |
| ATOM | 1310 | CB | VAL | D | 13 | -2.395 | -111.703 | -3.798 | 1.00222.09 |
| ATOM | 1311 | CG1 | VAL | D | 13 | -2.148 | -111.424 | -5.284 | 1.00219.38 |
| ATOM | 1312 | CG2 | VAL | D | 13 | -3.388 | -110.719 | -3.228 | 1.00223.91 |
| ATOM | 1313 | N | SER | D | 14 | -5.241 | -113.122 | -4.184 | 1.00226.63 |
| ATOM | 1314 | CA | SER | D | 14 | -6.340 | -113.380 | -5.105 | 1.00230.48 |
| ATOM | 1315 | C | SER | D | 14 | -6.593 | -112.155 | -5.960 | 1.00233.23 |
| ATOM | 1316 | O | SER | D | 14 | -6.156 | -111.064 | -5.607 | 1.00228.51 |
| ATOM | 1317 | CB | SER | D | 14 | -7.610 | -113.798 | -4.359 | 1.00240.15 |
| ATOM | 1318 | OG | SER | D | 14 | -8.508 | -114.515 | -5.200 | 1.00248.33 |
| ATOM | 1319 | N | VAL | D | 15 | -7.309 | -112.337 | -7.077 | 1.00234.06 |
| ATOM | 1320 | CA | VAL | D | 15 | -7.701 | -111.267 | -8.002 | 1.00233.09 |
| ATOM | 1321 | C | VAL | D | 15 | -8.464 | -110.147 | -7.257 | 1.00237.91 |
| ATOM | 1322 | O | VAL | D | 15 | -9.333 | -110.432 | -6.433 | 1.00241.55 |
| ATOM | 1323 | CB | VAL | D | 15 | -8.544 | -111.823 | -9.178 | 1.00241.51 |
| ATOM | 1324 | CG1 | VAL | D | 15 | -9.008 | -110.707 | -10.110 | 1.00240.25 |
| ATOM | 1325 | CG2 | VAL | D | 15 | -7.767 | -112.883 | -9.950 | 1.00240.83 |
| ATOM | 1326 | N | GLY | D | 16 | -8.124 | -108.899 | -7.567 | 1.00230.71 |
| ATOM | 1327 | CA | GLY | D | 16 | -8.761 | -107.735 | -6.965 | 1.00230.65 |
| ATOM | 1328 | C | GLY | D | 16 | -8.015 | -107.129 | -5.793 | 1.00230.26 |
| ATOM | 1329 | O | GLY | D | 16 | -8.198 | -105.942 | -5.511 | 1.00228.55 |
| ATOM | 1330 | N | GLU | D | 17 | -7.179 | -107.926 | -5.093 | 1.00224.41 |
| ATOM | 1331 | CA | GLU | D | 17 | -6.402 | -107.415 | -3.963 | 1.00220.79 |
| ATOM | 1332 | C | GLU | D | 17 | -5.153 | -106.592 | -4.403 | 1.00215.13 |
| ATOM | 1333 | O | GLU | D | 17 | -4.641 | -106.765 | -5.509 | 1.00212.08 |
| ATOM | 1334 | CB | GLU | D | 17 | -5.991 | -108.567 | -3.060 | 1.00224.15 |
| ATOM | 1335 | CG | GLU | D | 17 | -5.896 | -108.204 | -1.591 | 1.00236.27 |
| ATOM | 1336 | CD | GLU | D | 17 | -4.958 | -109.093 | -0.795 | 1.00263.60 |
| ATOM | 1337 | OE1 | GLU | D | 17 | -5.096 | -110.337 | -0.864 | 1.00272.93 |
| ATOM | 1338 | OE2 | GLU | D | 17 | -4.036 | -108.547 | -0.152 | 1.00251.34 |
| ATOM | 1339 | N | LYS | D | 18 | -4.674 | -105.701 | -3.523 | 1.00206.57 |
| ATOM | 1340 | CA | LYS | D | 18 | -3.490 | -104.891 | -3.757 | 1.00199.67 |
| ATOM | 1341 | C | LYS | D | 18 | -2.291 | -105.677 | -3.263 | 1.00201.92 |
| ATOM | 1342 | O | LYS | D | 18 | -2.426 | -106.451 | -2.324 | 1.00204.60 |
| ATOM | 1343 | CB | LYS | D | 18 | -3.614 | -103.585 | -2.987 | 1.00200.65 |
| ATOM | 1344 | CG | LYS | D | 18 | -2.653 | -102.509 | -3.453 | 1.00212.59 |
| ATOM | 1345 | CD | LYS | D | 18 | -2.942 | -101.148 | -2.816 | 1.00228.84 |
| ATOM | 1346 | CE | LYS | D | 18 | -2.576 | -99.971 | -3.708 | 1.00240.61 |
| ATOM | 1347 | NZ | LYS | D | 18 | -3.105 | -98.665 | -3.214 | 1.00251.85 |
| ATOM | 1348 | N | VAL | D | 19 | -1.123 | -105.497 | -3.872 | 1.00195.38 |
| ATOM | 1349 | CA | VAL | D | 19 | 0.098 | -106.225 | -3.462 | 1.00195.06 |
| ATOM | 1350 | C | VAL | D | 19 | 1.284 | -105.320 | -3.344 | 1.00196.89 |
| ATOM | 1351 | O | VAL | D | 19 | 1.477 | -104.497 | -4.234 | 1.00196.16 |
| ATOM | 1352 | CB | VAL | D | 19 | 0.416 | -107.323 | -4.487 | 1.00199.61 |
| ATOM | 1353 | CG1 | VAL | D | 19 | 1.876 | -107.773 | -4.424 | 1.00197.04 |
| ATOM | 1354 | CG2 | VAL | D | 19 | -0.519 | -108.496 | -4.303 | 1.00204.21 |
| ATOM | 1355 | N | THR | D | 20 | 2.142 | -105.524 | -2.331 | 1.00192.45 |
| ATOM | 1356 | CA | THR | D | 20 | 3.373 | -104.732 | -2.196 | 1.00189.49 |
| ATOM | 1357 | C | THR | D | 20 | 4.629 | -105.599 | -1.956 | 1.00192.59 |
| ATOM | 1358 | O | THR | D | 20 | 4.642 | -106.452 | -1.077 | 1.00194.63 |
| ATOM | 1359 | CB | THR | D | 20 | 3.195 | -103.573 | -1.190 | 1.00194.36 |
| ATOM | 1360 | OG1 | THR | D | 20 | 2.348 | -102.602 | -1.803 | 1.00186.15 |
| ATOM | 1361 | CG2 | THR | D | 20 | 4.511 | -102.882 | -0.835 | 1.00190.19 |
| ATOM | 1362 | N | MET | D | 21 | 5.682 | -105.360 | -2.715 | 1.00185.25 |
| ATOM | 1363 | CA | MET | D | 21 | 6.928 | -106.087 | -2.554 | 1.00185.08 |
| ATOM | 1364 | C | MET | D | 21 | 8.043 | -105.084 | -2.406 | 1.00190.70 |
| ATOM | 1365 | O | MET | D | 21 | 7.937 | -104.013 | -2.991 | 1.00190.70 |
| ATOM | 1366 | CB | MET | D | 21 | 7.164 | -107.005 | -3.746 | 1.00186.69 |
| ATOM | 1367 | CG | MET | D | 21 | 7.257 | -106.295 | -5.056 | 1.00188.19 |
| ATOM | 1368 | SD | MET | D | 21 | 6.773 | -107.331 | -6.421 | 1.00193.27 |
| ATOM | 1369 | CE | MET | D | 21 | 4.963 | -106.726 | -6.632 | 1.00190.78 |
| ATOM | 1370 | N | SER | D | 22 | 9.099 | -105.394 | -1.633 | 1.00187.95 |
| ATOM | 1371 | CA | SER | D | 22 | 10.175 | -104.430 | -1.396 | 1.00186.81 |
| ATOM | 1372 | C | SER | D | 22 | 11.527 | -104.766 | -1.992 | 1.00190.81 |
| ATOM | 1373 | O | SER | D | 22 | 11.757 | -105.908 | -2.385 | 1.00189.78 |
| ATOM | 1374 | CB | SER | D | 22 | 10.305 | -104.137 | 0.087 | 1.00191.56 |
| ATOM | 1375 | OG | SER | D | 22 | 10.572 | -105.357 | 0.746 | 1.00200.62 |
| ATOM | 1376 | N | CYS | D | 23 | 12.419 | -103.750 | -2.058 | 1.00188.69 |
| ATOM | 1377 | CA | CYS | D | 23 | 13.774 | -103.814 | -2.629 | 1.00189.47 |
| ATOM | 1378 | C | CYS | D | 23 | 14.703 | -102.985 | -1.777 | 1.00191.36 |
| ATOM | 1379 | O | CYS | D | 23 | 14.318 | -101.912 | -1.326 | 1.00191.16 |
| ATOM | 1380 | CB | CYS | D | 23 | 13.757 | -103.322 | -4.083 | 1.00189.38 |
| ATOM | 1381 | SG | CYS | D | 23 | 15.388 | -103.179 | -4.884 | 1.00194.58 |
| ATOM | 1382 | N | LYS | D | 24 | 15.918 | -103.457 | -1.553 | 1.00187.81 |
| ATOM | 1383 | CA | LYS | D | 24 | 16.888 | -102.712 | -0.748 | 1.00189.71 |
| ATOM | 1384 | C | LYS | D | 24 | 18.253 | -102.685 | -1.403 | 1.00195.48 |
| ATOM | 1385 | O | LYS | D | 24 | 18.715 | -103.713 | -1.895 | 1.00197.67 |
| ATOM | 1386 | CB | LYS | D | 24 | 16.979 | -103.258 | 0.687 | 1.00195.01 |
| ATOM | 1387 | CG | LYS | D | 24 | 17.570 | -102.257 | 1.712 | 1.00205.65 |
| ATOM | 1388 | CD | LYS | D | 24 | 18.081 | -102.919 | 3.038 | 1.00215.49 |
| ATOM | 1389 | CE | LYS | D | 24 | 19.106 | -104.061 | 2.949 | 1.00214.97 |
| ATOM | 1390 | NZ | LYS | D | 24 | 19.635 | -104.476 | 4.284 | 1.00211.92 |
| ATOM | 1391 | N | SER | D | 25 | 18.921 | -101.531 | -1.361 | 1.00190.72 |
| ATOM | 1392 | CA | SER | D | 25 | 20.244 | -101.366 | -1.947 | 1.00191.20 |
| ATOM | 1393 | C | SER | D | 25 | 21.232 | -101.034 | -0.855 | 1.00198.70 |
| ATOM | 1394 | O | SER | D | 25 | 20.907 | -100.290 | 0.072 | 1.00198.77 |
| ATOM | 1395 | CB | SER | D | 25 | 20.201 | -100.251 | -2.983 | 1.00190.75 |
| ATOM | 1396 | OG | SER | D | 25 | 21.308 | -100.165 | -3.855 | 1.00195.49 |
| ATOM | 1397 | N | SER | D | 26 | 22.434 | -101.594 | -0.958 | 1.00198.42 |
| ATOM | 1398 | CA | SER | D | 26 | 23.526 | -101.381 | -0.007 | 1.00202.70 |
| ATOM | 1399 | C | SER | D | 26 | 24.050 | -99.942 | -0.009 | 1.00208.22 |
| ATOM | 1400 | O | SER | D | 26 | 24.785 | -99.561 | 0.903 | 1.00211.59 |
| ATOM | 1401 | CB | SER | D | 26 | 24.668 | -102.336 | -0.319 | 1.00209.79 |
| ATOM | 1402 | OG | SER | D | 26 | 24.999 | -102.237 | -1.694 | 1.00217.86 |
| ATOM | 1403 | N | GLN | D | 27 | 23.666 | -99.147 | -1.016 | 1.00202.53 |
| ATOM | 1404 | CA | GLN | D | 27 | 24.098 | -97.757 | -1.173 | 1.00203.52 |
| ATOM | 1405 | C | GLN | D | 27 | 22.932 | -96.910 | -1.653 | 1.00203.00 |
| ATOM | 1406 | O | GLN | D | 27 | 22.038 | -97.464 | -2.273 | 1.00199.36 |
| ATOM | 1407 | CB | GLN | D | 27 | 25.234 | -97.744 | -2.200 | 1.00207.14 |
| ATOM | 1408 | CG | GLN | D | 27 | 25.966 | -96.427. | -2.441 | 1.00224.70 |
| ATOM | 1409 | CD | GLN | D | 27 | 26.935 | -96.597 | -3.609 | 1.00237.11 |
| ATOM | 1410 | OE1 | GLN | D | 27 | 26.823 | -95.919 | -4.641 | 1.00232.36 |
| ATOM | 1411 | NE2 | GLN | D | 27 | 27.819 | -97.609 | -3.542 | 1.00219.82 |
| ATOM | 1412 | N | SER | D | 28 | 22.928 | -95.580 | -1.383 | 1.00201.25 |
| ATOM | 1413 | CA | SER | D | 28 | 21.837 | -94.679 | -1.836 | 1.00198.77 |
| ATOM | 1414 | C | SER | D | 28 | 21.831 | -94.482 | -3.328 | 1.00202.25 |
| ATOM | 1415 | O | SER | D | 28 | 22.873 | -94.224 | -3.936 | 1.00203.42 |
| ATOM | 1416 | CB | SER | D | 28 | 21.867 | -93.310 | -1.162 | 1.00204.36 |
| ATOM | 1417 | OG | SER | D | 28 | 20.909 | -92.436 | -1.751 | 1.00207.36 |
| ATOM | 1418 | N | LEU | D | 29 | 20.635 | -94.570 | -3.907 | 1.00196.93 |
| ATOM | 1419 | CA | LEU | D | 29 | 20.465 | -94.481 | -5.349 | 1.00195.59 |
| ATOM | 1420 | C | LEU | D | 29 | 19.856 | -93.148 | -5.744 | 1.00198.59 |
| ATOM | 1421 | O | LEU | D | 29 | 19.494 | -92.936 | -6.907 | 1.00197.03 |
| ATOM | 1422 | CB | LEU | D | 29 | 19.610 | -95.668 | -5.867 | 1.00193.26 |
| ATOM | 1423 | CG | LEU | D | 29 | 19.984 | -97.090 | -5.417 | 1.00198.16 |
| ATOM | 1424 | CD1 | LEU | D | 29 | 19.044 | -98.096 | -6.021 | 1.00195.29 |
| ATOM | 1425 | CD2 | LEU | D | 29 | 21.423 | -97.418 | -5.765 | 1.00202.98 |
| ATOM | 1426 | N | LEU | D | 30 | 19.770 | -92.231 | -4.784 | 1.00195.19 |
| ATOM | 1427 | CA | LEU | D | 30 | 19.185 | -90.929 | -5.059 | 1.00192.56 |
| ATOM | 1428 | C | LEU | D | 30 | 20.207 | -89.989 | -5.657 | 1.00195.61 |
| ATOM | 1429 | O | LEU | D | 30 | 21.179 | -89.623 | -4.992 | 1.00200.33 |
| ATOM | 1430 | CB | LEU | D | 30 | 18.514 | -90.377 | -3.804 | 1.00192.47 |
| ATOM | 1431 | CG | LEU | D | 30 | 17.944 | -88.975 | -3.840 | 1.00195.85 |
| ATOM | 1432 | CD1 | LEU | D | 30 | 16.984 | -88.771 | -4.996 | 1.00192.09 |
| ATOM | 1433 | CD2 | LEU | D | 30 | 17.222 | -88.704 | -2.558 | 1.00200.02 |
| ATOM | 1434 | N | TYR | D | 31 | 19.974 | -89.628 | -6.942 | 1.00185.23 |
| ATOM | 1435 | CA | TYR | D | 31 | 20.817 | -88.751 | -7.736 | 1.00183.96 |
| ATOM | 1436 | C | TYR | D | 31 | 20.714 | -87.327 | -7.229 | 1.00189.61 |
| ATOM | 1437 | O | TYR | D | 31 | 19.644 | -86.728 | -7.319 | 1.00187.01 |
| ATOM | 1438 | CB | TYR | D | 31 | 20.443 | -88.796 | -9.224 | 1.00179.36 |
| ATOM | 1439 | CG | TYR | D | 31 | 21.546 | -88.318 | -10.128 | 1.00178.22 |
| ATOM | 1440 | CD1 | TYR | D | 31 | 22.804 | -88.022 | -9.625 | 1.00182.83 |
| ATOM | 1441 | CD2 | TYR | D | 31 | 21.358 | -88.232 | -11.493 | 1.00177.09 |
| ATOM | 1442 | CE1 | TYR | D | 31 | 23.838 | -87.645 | -10.453 | 1.00184.40 |
| ATOM | 1443 | CE2 | TYR | D | 31 | 22.398 | -87.882 | -12.338 | 1.00180.80 |
| ATOM | 1444 | CZ | TYR | D | 31 | 23.640 | -87.589 | -11.812 | 1.00189.00 |
| ATOM | 1445 | OH | TYR | D | 31 | 24.689 | -87.260 | -12.627 | 1.00192.03 |
| ATOM | 1446 | N | SER | D | 32 | 21.834 | -86.784 | -6.711 | 1.00189.26 |
| ATOM | 1447 | CA | SER | D | 32 | 21.917 | -85.434 | -6.179 | 1.00190.19 |
| ATOM | 1448 | C | SER | D | 32 | 21.390 | -84.373 | -7.164 | 1.00193.89 |
| ATOM | 1449 | O | SER | D | 32 | 20.301 | -83.825 | -6.943 | 1.00191.71 |
| ATOM | 1450 | CB | SER | D | 32 | 23.340 | -85.133 | -5.729 | 1.00197.42 |
| ATOM | 1451 | OG | SER | D | 32 | 24.238 | -85.232 | -6.820 | 1.00206.93 |
| ATOM | 1452 | N | SER | D | 33 | 22.117 | -84.124 | -8.272 | 1.00191.54 |
| ATOM | 1453 | CA | SER | D | 33 | 21.699 | -83.132 | -9.257 | 1.00189.53 |
| ATOM | 1454 | C | SER | D | 33 | 20.601 | -83.676 | -10.114 | 1.00189.65 |
| ATOM | 1455 | O | SER | D | 33 | 20.809 | -83.876 | -11.310 | 1.00189.36 |
| ATOM | 1456 | CB | SER | D | 33 | 22.871 | -82.714 | -10.130 | 1.00195.21 |
| ATOM | 1457 | OG | SER | D | 33 | 23.407 | -83.839 | -10.809 | 1.00200.36 |
| ATOM | 1458 | N | ASN | D | 34 | 19.427 | -83.909 | -9.519 | 1.00184.28 |
| ATOM | 1459 | CA | ASN | D | 34 | 18.302 | -84.466 | -10.238 | 1.00182.47 |
| ATOM | 1460 | C | ASN | D | 34 | 17.129 | -84.649 | -9.314 | 1.00190.01 |
| ATOM | 1461 | O | ASN | D | 34 | 15.980 | -84.357 | -9.689 | 1.00185.92 |
| ATOM | 1462 | CB | ASN | D | 34 | 18.689 | -85.842 | -10.820 | 1.00184.45 |
| ATOM | 1463 | CG | ASN | D | 34 | 17.679 | -86.389 | -11.788 | 1.00209.29 |
| ATOM | 1464 | OD1 | ASN | D | 34 | 17.739 | -87.552 | -12.177 | 1.00212.67 |
| ATOM | 1465 | ND2 | ASN | D | 34 | 16.714 | -85.576 | -12.200 | 1.00193.35 |
| ATOM | 1466 | N | GLN | D | 35 | 17.424 | -85.216 | -8.122 | 1.00193.14 |
| ATOM | 1467 | CA | GLN | D | 35 | 16.456 | -85.569 | -7.075 | 1.00193.23 |
| ATOM | 1468 | C | GLN | D | 35 | 15.587 | -86.779 | -7.469 | 1.00193.97 |
| ATOM | 1469 | O | GLN | D | 35 | 14.464 | -86.934 | -6.982 | 1.00191.59 |
| ATOM | 1470 | CB | GLN | D | 35 | 15.629 | -84.350 | -6.627 | 1.00194.47 |
| ATOM | 1471 | CG | GLN | D | 35 | 16.472 | -83.204 | -6.051 | 1.00205.79 |
| ATOM | 1472 | CD | GLN | D | 35 | 17.046 | -83.496 | -4.683 | 1.00210.28 |
| ATOM | 1473 | OE1 | GLN | D | 35 | 17.564 | -84.582 | -4.407 | 1.00197.15 |
| ATOM | 1474 | NE2 | GLN | D | 35 | 17.101 | -82.474 | -3.839 | 1.00204.58 |
| ATOM | 1475 | N | LYS | D | 36 | 16.160 | -87.658 | -8.319 | 1.00190.17 |
| ATOM | 1476 | CA | LYS | D | 36 | 15.549 | -88.898 | -8.8,05 | 1.00188.31 |
| ATOM | 1477 | C | LYS | D | 36 | 16.351 | -90.114 | -8.317 | 1.00193.72 |
| ATOM | 1478 | O | LYS | D | 36 | 17.557 | -90.027 | -8.072 | 1.00196.83 |
| ATOM | 1479 | CB | LYS | D | 36 | 15.410 | -88.909 | -10.333 | 1.00189.11 |
| ATOM | 1480 | CG | LYS | D | 36 | 14.661 | -87.696 | -10.891 | 1.00193.77 |
| ATOM | 1481 | CD | LYS | D | 36 | 14.124 | -87.942 | -12.286 | 1.00195.32 |
| ATOM | 1482 | CE | LYS | D | 36 | 12.627 | -87.784 | -12.338 | 1.00197.19 |
| ATOM | 1483 | NZ | LYS | D | 36 | 12.114 | -88.014 | -13.701 | 1.00209.34 |
| ATOM | 1484 | N | ASN | D | 37 | 15.668 | -91.223 | -8.132 | 1.00186.47 |
| ATOM | 1485 | CA | ASN | D | 37 | 16.288 | -92.425 | -7.624 | 1.00186.10 |
| ATOM | 1486 | C | ASN | D | 37 | 16.615 | -93.289 | -8.807 | 1.00186.85 |
| ATOM | 1487 | O | ASN | D | 37 | 15.730 | -93.562 | -9.588 | 1.00183.99 |
| ATOM | 1488 | CB | ASN | D | 37 | 15.288 | -93.113 | -6.708 | 1.00187.62 |
| ATOM | 1489 | CG | ASN | D | 37 | 15.189 | -92.492 | -5.334 | 1.00210.54 |
| ATOM | 1490 | OD1 | ASN | D | 37 | 16.019 | -92.779 | -4.467 | 1.00194.98 |
| ATOM | 1491 | ND2 | ASN | D | 37 | 14.172 | -91.640 | -5.099 | 1.00204.81 |
| ATOM | 1492 | N | PHE | D | 38 | 17.856 | -93.719 | -8.973 | 1.00185.33 |
| ATOM | 1493 | CA | PHE | D | 38 | 18.239 | -94.574 | -10.103 | 1.00185.27 |
| ATOM | 1494 | C | PHE | D | 38 | 17.852 | -96.045 | -9.932 | 1.00192.74 |
| ATOM | 1495 | O | PHE | D | 38 | 18.714 | -96.917 | -9.899 | 1.00193.92 |
| ATOM | 1496 | CB | PHE | D | 38 | 19.709 | -94.345 | -10.470 | 1.00188.58 |
| ATOM | 1497 | CG | PHE | D | 38 | 19.821 | -93.123 | -11.347 | 1.00189.12 |
| ATOM | 1498 | CD2 | PHE | D | 38 | 20.663 | -93.120 | -12.440 | 1.00192.05 |
| ATOM | 1499 | CD1 | PHE | D | 38 | 18.890 | -92.083 | -11.247 | 1.00190.10 |
| ATOM | 1500 | CE2 | PHE | D | 38 | 20.726 | -92.022 | -13.295 | 1.00195.19 |
| ATOM | 1501 | CE1 | PHE | D | 38 | 18.905 | -91.014 | -12.139 | 1.00190.91 |
| ATOM | 1502 | CZ | PHE | D | 38 | 19.821 | -90.995 | -13.163 | 1.00191.75 |
| ATOM | 1503 | N | LEU | D | 39 | 16.550 | -96.316 | -9.816 | 1.00190.07 |
| ATOM | 1504 | CA | LEU | D | 39 | 16.062 | -97.674 | -9.626 | 1.00190.88 |
| ATOM | 1505 | C | LEU | D | 39 | 14.930 | -97.972 | -10.578 | 1.00195.37 |
| ATOM | 1506 | O | LEU | D | 39 | 14.160 | -97.065 | -10.923 | 1.00196.60 |
| ATOM | 1507 | CB | LEU | D | 39 | 15.656 | -97.890 | -8.158 | 1.00191.08 |
| ATOM | 1508 | CG | LEU | D | 39 | 15.019 | -99.210 | -7.696 | 1.00195.68 |
| ATOM | 1509 | CD1 | LEU | D | 39 | 15.847 | -100.453 | -7.842 | 1.00196.91 |
| ATOM | 1510 | CD2 | LEU | D | 39 | 13.537 | -99.261 | -7.865 | 1.00197.21 |
| ATOM | 1511 | N | ALA | D | 40 | 14.831 | -99.241 | -11.006 | 1.00188.96 |
| ATOM | 1512 | CA | ALA | D | 40 | 13.790 | -99.664 | -11.922 | 1.00186.21 |
| ATOM | 1513 | C | ALA | D | 40 | 13.166 | -100.919 | -11.476 | 1.00188.36 |
| ATOM | 1514 | O | ALA | D | 40 | 13.786 | -101.661 | -10.727 | 1.00189.80 |
| ATOM | 1515 | CB | ALA | D | 40 | 14.363 | -99.849 | -13.311 | 1.00187.42 |
| ATOM | 1516 | N | TRP | D | 41 | 11.944 | -101.169 | -11.946 | 1.00183.45 |
| ATOM | 1517 | CA | TRP | D | 41 | 11.190 | -102.385 | -11.691 | 1.00184.49 |
| ATOM | 1518 | C | TRP | D | 41 | 10.837 | -103.067 | -12.981 | 1.00187.31 |
| ATOM | 1519 | O | TRP | D | 41 | 10.413 | -102.427 | -13.937 | 1.00188.26 |
| ATOM | 1520 | CB | TRP | D | 41 | 9.919 | -102.093 | -10.960 | 1.00183.88 |
| ATOM | 1521 | CG | TRP | D | 41 | 10.169 | -101.797 | -9.529 | 1.00186.39 |
| ATOM | 1522 | CD1 | TRP | D | 41 | 10.266 | -100.562 | -8.969 | 1.00189.18 |
| ATOM | 1523 | CD2 | TRP | D | 41 | 10.392 | -102.749 | -8.457 | 1.00187.73 |
| ATOM | 1524 | NE1 | TRP | D | 41 | 10.465 | -100.674 | -7.607 | 1.00190.36 |
| ATOM | 1525 | CE2 | TRP | D | 41 | 10.559 | -102.006 | -7.270 | 1.00192.78 |
| ATOM | 1526 | CE3 | TRP | D | 41 | 10.418 | -104.155 | -8.375 | 1.00189.82 |
| ATOM | 1527 | CZ2 | TRP | D | 41 | 10.761 | -102.622 | -6.022 | 1.00193.13 |
| ATOM | 1528 | CZ3 | TRP | D | 41 | 10.593 | -104.760 | -7.134 | 1.00192.09 |
| ATOM | 1529 | CH2 | TRP | D | 41 | 10.757 | -104.001 | -5.979 | 1.00192.98 |
| ATOM | 1530 | N | TYR | D | 42 | 11.016 | -104.366 | -13.020 | 1.00181.80 |
| ATOM | 1531 | CA | TYR | D | 42 | 10.707 | -105.155 | -14.195 | 1.00180.50 |
| ATOM | 1532 | C | TYR | D | 42 | 9.705 | -106.215 | -13.795 | 1.00184.45 |
| ATOM | 1533 | O | TYR | D | 42 | 9.661 | -106.629 | -12.623 | 1.00186.48 |
| ATOM | 1534 | CB | TYR | D | 42 | 11.973 | -105.818 | -14.764 | 1.00180.95 |
| ATOM | 1535 | CG | TYR | D | 42 | 12.925 | -104.867 | -15.436 | 1.00180.26 |
| ATOM | 1536 | CD2 | TYR | D | 42 | 12.943 | -104.736 | -16.813 | 1.00181.92 |
| ATOM | 1537 | CD1 | TYR | D | 42 | 13.851 | -104.139 | -14.697 | 1.00181.56 |
| ATOM | 1538 | CE2 | TYR | D | 42 | 13.840 | -103.882 | -17.443 | 1.00183.61 |
| ATOM | 1539 | CE1 | TYR | D | 42 | 14.733 | -103.259 | -15.310 | 1.00184.24 |
| ATOM | 1540 | CZ | TYR | D | 42 | 14.730 | -103.137 | -16.685 | 1.00193.71 |
| ATOM | 1541 | OH | TYR | D | 42 | 15.596 | -102.248 | -17.271 | 1.00197.14 |
| ATOM | 1542 | N | GLN | D | 43 | 8.885 | -106.640 | -14.767 | 1.00177.47 |
| ATOM | 1543 | CA | GLN | D | 43 | 7.921 | -107.716 | -14.589 | 1.00176.61 |
| ATOM | 1544 | C | GLN | D | 43 | 8.240 | -108.761 | -15.628 | 1.00184.12 |
| ATOM | 1545 | O | GLN | D | 43 | 8.422 | -108.406 | -16.800 | 1.00183.39 |
| ATOM | 1546 | CB | GLN | D | 43 | 6.503 | -107.238 | -14.768 | 1.00176.04 |
| ATOM | 1547 | CG | GLN | D | 43 | 5.525 | -108.365 | -14.844 | 1.00158.91 |
| ATOM | 1548 | CD | GLN | D | 43 | 4.384 | -108.045 | -15.754 | 1.00182.31 |
| ATOM | 1549 | OE1 | GLN | D | 43 | 3.227 | -107.999 | -15.335 | 1.00182.46 |
| ATOM | 1550 | NE2 | GLN | D | 43 | 4.687 | -108.013 | -17.060 | 1.00169.98 |
| ATOM | 1551 | N | GLN | D | 44 | 8.346 | -110.046 | -15.197 | 1.00183.45 |
| ATOM | 1552 | CA | GLN | D | 44 | 8.643 | -111.156 | -16.097 | 1.00184.72 |
| ATOM | 1553 | C | GLN | D | 44 | 7.600 | -112.226 | -15.966 | 1.00190.81 |
| ATOM | 1554 | O | GLN | D | 44 | 7.619 | -112.960 | -14.982 | 1.00191.68 |
| ATOM | 1555 | CB | GLN | D | 44 | 10.058 | -111.721 | -15.893 | 1.00185.24 |
| ATOM | 1556 | CG | GLN | D | 44 | 10.364 | -112.825 | -16.882 | 1.00195.88 |
| ATOM | 1557 | CD | GLN | D | 44 | 11.762 | -113.364 | -16.797 | 1.00224.89 |
| ATOM | 1558 | OE1 | GLN | D | 44 | 12.291 | -113.660 | -15.721 | 1.00221.88 |
| ATOM | 1559 | NE2 | GLN | D | 44 | 12.370 | -113.578 | -17.954 | 1.00222.77 |
| ATOM | 1560 | N | LYS | D | 45 | 6.687 | -112.313 | -16.950 | 1.00187.58 |
| ATOM | 1561 | CA | LYS | D | 45 | 5.672 | -113.358 | -16.967 | 1.00189.76 |
| ATOM | 1562 | C | LYS | D | 45 | 6.398 | -114.637 | -17.389 | 1.00196.87 |
| ATOM | 1563 | O | LYS | D | 45 | 7.409 | -114.542 | -18.115 | 1.00197.53 |
| ATOM | 1564 | CB | LYS | D | 45 | 4.537 | -113.030 | -17.916 | 1.00192.28 |
| ATOM | 1565 | CG | LYS | D | 45 | 3.884 | -111.724 | -17.557 | 1.00187.20 |
| ATOM | 1566 | CD | LYS | D | 45 | 2.637 | -111.444 | -18.364 | 1.00190.01 |
| ATOM | 1567 | CE | LYS | D | 45 | 2.132 | -110.050 | -18.078 | 1.00188.70 |
| ATOM | 1568 | NZ | LYS | D | 45 | 0.802 | -109.799 | -18.689 | 1.00194.46 |
| ATOM | 1569 | N | PRO | D | 46 | 5.970 | -115.831 | -16.897 | 1.00192.55 |
| ATOM | 1570 | CA | PRO | D | 46 | 6.750 | -117.029 | -17.158 | 1.00192.53 |
| ATOM | 1571 | C | PRO | D | 46 | 6.814 | -117.376 | -18.624 | 1.00197.88 |
| ATOM | 1572 | O | PRO | D | 46 | 5.821 | -117.254 | -19.329 | 1.00198.74 |
| ATOM | 1573 | CB | PRO | D | 46 | 6.058 | -118.066 | -16.287 | 1.00196.19 |
| ATOM | 1574 | CG | PRO | D | 46 | 5.409 | -117.278 | -15.200 | 1.00198.93 |
| ATOM | 1575 | CD | PRO | D | 46 | 4.855 | -116.142 | -15.979 | 1.00194.21 |
| ATOM | 1576 | N | GLY | D | 47 | 8.016 | -117.694 | -19.082 | 1.00195.71 |
| ATOM | 1577 | CA | GLY | D | 47 | 8.267 | -118.078 | -20.470 | 1.00198.62 |
| ATOM | 1578 | C | GLY | D | 47 | 8.411 | -116.902 | -21.407 | 1.00201.22 |
| ATOM | 1579 | O | GLY | D | 47 | 8.686 | -117.071 | -22.603 | 1.00202.74 |
| ATOM | 1580 | N | GLN | D | 48 | 8.226 | -115.707 | -20.866 | 1.00194.79 |
| ATOM | 1581 | CA | GLN | D | 48 | 8.359 | -114.502 | -21.656 | 1.00194.24 |
| ATOM | 1582 | C | GLN | D | 48 | 9.596 | -113.738 | -21.245 | 1.00197.72 |
| ATOM | 1583 | O | GLN | D | 48 | 10.218 | -114.049 | -20.233 | 1.00196.48 |
| ATOM | 1584 | CB | GLN | D | 48 | 7.112 | -113.619 | -21.498 | 1.00194.64 |
| ATOM | 1585 | CG | GLN | D | 48 | 5.900 | -114.116 | -22.276 | 1.00202.71 |
| ATOM | 1586 | CD | GLN | D | 48 | 4.734 | -113.183 | -22.150 | 1.00220.40 |
| ATOM | 1587 | OE1 | GLN | D | 48 | 4.871 | -111.970 | -22.322 | 1.00222.06 |
| ATOM | 1588 | NE2 | GLN | D | 48 | 3.557 | -113.727 | -21.857 | 1.00206.68 |
| ATOM | 1589 | N | SER | D | 49 | 9.953 | -112.725 | -22.027 | 1.00194.86 |
| ATOM | 1590 | CA | SER | D | 49 | 11.063 | -111.870 | -21.675 | 1.00192.73 |
| ATOM | 1591 | C | SER | D | 49 | 10.567 | -110.819 | -20.674 | 1.00195.32 |
| ATOM | 1592 | O | SER | D | 49 | 9.372 | -110.502 | -20.634 | 1.00194.16 |
| ATOM | 1593 | CB | SER | D | 49 | 11.650 | -111.211 | -22.912 | 1.00196.02 |
| ATOM | 1594 | OG | SER | D | 49 | 12.543 | -112.137 | -23.498 | 1.00208.08 |
| ATOM | 1595 | N | PRO | D | 50 | 11.471 | -110.278 | -19.843 | 1.00191.78 |
| ATOM | 1596 | CA | PRO | D | 50 | 11.070 | -109.248 | -18.887 | 1.00189.02 |
| ATOM | 1597 | C | PRO | D | 50 | 10.615 | -107.962 | -19.571 | 1.00190.86 |
| ATOM | 1598 | O | PRO | D | 50 | 10.927 | -107.708 | -20.743 | 1.00192.21 |
| ATOM | 1599 | CB | PRO | D | 50 | 12.346 | -109.018 | -18.074 | 1.00190.22 |
| ATOM | 1600 | CG | PRO | D | 50 | 13.135 | -110.218 | -18.275 | 1.00197.48 |
| ATOM | 1601 | CD | PRO | D | 50 | 12.908 | -110.550 | -19.710 | 1.00194.92 |
| ATOM | 1602 | N | LYS | D | 51 | 9.865 | -107.152 | -18.827 | 1.00183.40 |
| ATOM | 1603 | CA | LYS | D | 51 | 9.313 | -105.921 | -19.348 | 1.00181.41 |
| ATOM | 1604 | C | LYS | D | 51 | 9.588 | -104.776 | -18.386 | 1.00182.97 |
| ATOM | 1605 | O | LYS | D | 51 | 9.421 | -104.962 | -17.177 | 1.00183.82 |
| ATOM | 1606 | CB | LYS | D | 51 | 7.802 | -106.112 | -19.573 | 1.00183.96 |
| ATOM | 1607 | CG | LYS | D | 51 | 7.059 | -104.848 | -19.981 | 1.00198.44 |
| ATOM | 1608 | CD | LYS | D | 51 | 5.537 | -105.025 | -19.942 | 1.00207.89 |
| ATOM | 1609 | CE | LYS | D | 51 | 4.785 | -103.726 | -20.195 | 1.00200.67 |
| ATOM | 1610 | NZ | LYS | D | 51 | 3.297 | -103.848 | -20.021 | 1.00187.98 |
| ATOM | 1611 | N | LEU | D | 52 | 10.001 | -103.595 | -18.906 | 1.00175.19 |
| ATOM | 1612 | CA | LEU | D | 52 | 10.227 | -102.444 | -18.037 | 1.00170.84 |
| ATOM | 1613 | C | LEU | D | 52 | 8.894 | -101.864 | -17.608 | 1.00173.17 |
| ATOM | 1614 | O | LEU | D | 52 | 8.038 | -101.591 | -18.439 | 1.00173.34 |
| ATOM | 1615 | CB | LEU | D | 52 | 11.079 | -101.375 | -18.719 | 1.00169.58 |
| ATOM | 1616 | CG | LEU | D | 52 | 11.354 | -100.126 | -17.876 | 1.00170.91 |
| ATOM | 1617 | CD1 | LEU | D | 52 | 12.123 | -100.472 | -16.642 | 1.00169.69 |
| ATOM | 1618 | CD2 | LEU | D | 52 | 12.105 | -99.092 | -18.673 | 1.00174.06 |
| ATOM | 1619 | N | LEU | D | 53 | 8.708 | -101.699 | -16.322 | 1.00169.53 |
| ATOM | 1620 | CA | LEU | D | 53 | 7.467 | -101.131 | -15.830 | 1.00170.08 |
| ATOM | 1621 | C | LEU | D | 53 | 7.708 | -99.702 | -15.294 | 1.00174.31 |
| ATOM | 1622 | O | LEU | D | 53 | 6.959 | -98.751 | -15.592 | 1.00174.79 |
| ATOM | 1623 | CB | LEU | D | 53 | 6.923 | -101.981 | -14.674 | 1.00170.94 |
| ATOM | 1624 | CG | LEU | D | 53 | 6.491 | -103.368 | -14.923 | 1.00178.24 |
| ATOM | 1625 | CD1 | LEU | D | 53 | 6.296 | -104.078 | -13.604 | 1.00179.59 |
| ATOM | 1626 | CD2 | LEU | D | 53 | 5.215 | -103.378 | -15.721 | 1.00183.03 |
| ATOM | 1627 | N | ILE | D | 54 | 8.719 | -99.580 | -14.432 | 1.00167.68 |
| ATOM | 1628 | CA | ILE | D | 54 | 8.971 | -98.347 | -13.752 | 1.00164.55 |
| ATOM | 1629 | C | ILE | D | 54 | 10.417 | -98.009 | -13.695 | 1.00173.06 |
| ATOM | 1630 | O | ILE | D | 54 | 11.238 | -98.869 | -13.415 | 1.00175.53 |
| ATOM | 1631 | CB | ILE | D | 54 | 8.390 | -98.470 | -12.346 | 1.00166.02 |
| ATOM | 1632 | CG1 | ILE | D | 54 | 6.881 | -98.589 | -12.395 | 1.00165.36 |
| ATOM | 1633 | CG2 | ILE | D | 54 | 8.766 | -97.266 | -11.538 | 1.00166.83 |
| ATOM | 1634 | CD1 | ILE | D | 54 | 6.275 | -98.936 | -11.109 | 1.00168.49 |
| ATOM | 1635 | N | TYR | D | 55 | 10.721 | -96.743 | -13.905 | 1.00171.69 |
| ATOM | 1636 | CA | TYR | D | 55 | 12.064 | -96.213 | -13.788 | 1.00174.22 |
| ATOM | 1637 | C | TYR | D | 55 | 12.052 | -94.961 | -12.959 | 1.00181.10 |
| ATOM | 1638 | O | TYR | D | 55 | 10.990 | -94.391 | -12.712 | 1.00179.27 |
| ATOM | 1639 | CB | TYR | D | 55 | 12.733 | -96.016 | -15.137 | 1.00177.05 |
| ATOM | 1640 | CG | TYR | D | 55 | 11.989 | -95.146 | -16.109 | 1.00180.63 |
| ATOM | 1641 | CD2 | TYR | D | 55 | 12.411 | -93.848 | -16.372 | 1.00182.07 |
| ATOM | 1642 | CD1 | TYR | D | 55 | 10.910 | -95.641 | -16.833 | 1.00183.13 |
| ATOM | 1643 | CE2 | TYR | D | 55 | 11.735 | -93.039 | -17.291 | 1.00182.80 |
| ATOM | 1644 | CE1 | TYR | D | 55 | 10.228 | -94.845 | -17.757 | 1.00184.38 |
| ATOM | 1645 | CZ | TYR | D | 55 | 10.644 | -93.543 | -17.986 | 1.00189.41 |
| ATOM | 1646 | OH | TYR | D | 55 | 9.968 | -92.764 | -18.897 | 1.00187.27 |
| ATOM | 1647 | N | TRP | D | 56 | 13.213 | -94.552 | -12.479 | 1.00182.07 |
| ATOM | 1648 | CA | TRP | D | 56 | 13.279 | -93.416 | -11.593 | 1.00183.64 |
| ATOM | 1649 | C | TRP | D | 56 | 12.486 | -93.692 | -10.341 | 1.00188.89 |
| ATOM | 1650 | O | TRP | D | 56 | 11.987 | -92.759 | -9.706 | 1.00190.14 |
| ATOM | 1651 | CB | TRP | D | 56 | 12.885 | -92.121 | -12.297 | 1.00182.29 |
| ATOM | 1652 | CG | TRP | D | 56 | 14.073 | -91.571 | -12.997 | 1.00185.37 |
| ATOM | 1653 | CD1 | TRP | D | 56 | 15.360 | -91.557 | -12.537 | 1.00190.33 |
| ATOM | 1654 | CD2 | TRP | D | 56, | 14.103 | -90.985 | -14.282 | 1.00185.74 |
| ATOM | 1655 | NE1 | TRP | D | 56 | 16.188 | -90.995 | -13.465 | 1.00191.25 |
| ATOM | 1656 | CE2 | TRP | D | 56 | 15.450 | -90.665 | -14.565 | 1.00192.12 |
| ATOM | 1657 | CE3 | TRP | D | 56 | 13.117 | -90.675 | -15.227 | 1.00186.47 |
| ATOM | 1658 | CZ2 | TRP | D | 56 | 15.825 | -89.994 | -15.728 | 1.00193.13 |
| ATOM | 1659 | CZ3 | TRP | D | 56 | 13.501 | -90.093 | -16.420 | 1.00189.44 |
| ATOM | 1660 | CH2 | TRP | D | 56 | 14.844 | -89.771 | -16.669 | 1.00192.37 |
| ATOM | 1661 | N | ALA | D | 57 | 12.356 | -95.000 | -10.004 | 1.00184.34 |
| ATOM | 1662 | CA | ALA | D | 57 | 11.611 | -95.519 | -8.870 | 1.00184.25 |
| ATOM | 1663 | C | ALA | D | 57 | 10.110 | -95.246 | -8.937 | 1.00186.13 |
| ATOM | 1664 | O | ALA | D | 57 | 9.374 | -96.057 | -8.389 | 1.00187.32 |
| ATOM | 1665 | CB | ALA | D | 57 | 12.167 | -94.981 | -7.572 | 1.00186.33 |
| ATOM | 1666 | N | SER | D | 58 | 9.635 | -94.131 | -9.562 | 1.00178.39 |
| ATOM | 1667 | CA | SER | D | 58 | 8.207 | -93.819 | -9.613 | 1.00175.61 |
| ATOM | 1668 | C | SER | D | 58 | 7.633 | -93.545 | -10.994 | 1.00176.39 |
| ATOM | 1669 | O | SER | D | 58 | 6.416 | -93.568 | -11.126 | 1.00174.82 |
| ATOM | 1670 | CB | SER | D | 58 | 7.878 | -92.684 | -8.654 | 1.00178.29 |
| ATOM | 1671 | OG | SER | D | 58 | 8.085 | -91.418 | -9.263 | 1.00187.42 |
| ATOM | 1672 | N | THR | D | 59 | 8.472 | -93.263 | -12.011 | 1.00173.68 |
| ATOM | 1673 | CA | THR | D | 59 | 7.972 | -92.995 | -13.366 | 1.00174.53 |
| ATOM | 1674 | C | THR | D | 59 | 7.487 | -94.255 | -14.062 | 1.00183.41 |
| ATOM | 1675 | O | THR | D | 59 | 8.267 | -95.168 | -14.320 | 1.00184.87 |
| ATOM | 1676 | CB | THR | D | 59 | 9.006 | -92.298 | -14.238 | 1.00183.90 |
| ATOM | 1677 | OG1 | THR | D | 59 | 9.411 | -91.079 | -13.604 | 1.00184.39 |
| ATOM | 1678 | CG2 | THR | D | 59 | 8.484 | -92.043 | -15.658 | 1.00183.39 |
| ATOM | 1679 | N | ARG | D | 60 | 6.211 | -94.277 | -14.413 | 1.00181.92 |
| ATOM | 1680 | CA | ARG | D | 60 | 5.577 | -95.397 | -15.097 | 1.00183.19 |
| ATOM | 1681 | C | ARG | D | 60 | 5.912 | -95.346 | -16.580 | 1.00187.22 |
| ATOM | 1682 | O | ARG | D | 60 | 5.802 | -94.288 | -17.205 | 1.00185.93 |
| ATOM | 1683 | CB | ARG | D | 60 | 4.065 | -95.270 | -14.924 | 1.00185.11 |
| ATOM | 1684 | CG | ARG | D | 60 | 3.299 | -96.565 | -15.017 | 1.00198.01 |
| ATOM | 1685 | CD | ARG | D | 60 | 1.979 | -96.402 | -14.289 | 1.00202.10 |
| ATOM | 1686 | NE | ARG | D | 60 | 2.180 | -95.673 | -13.026 | 1.00210.51 |
| ATOM | 1687 | CZ | ARG | D | 60 | 1.250 | -95.458 | -12.098 | 1.00221.41 |
| ATOM | 1688 | NH1 | ARG | D | 60 | 0.027 | -95.935 | -12.251 | 1.00214.54 |
| ATOM | 1689 | NH2 | ARG | D | 60 | 1.551 | -94.793 | -10.991 | 1.00196.32 |
| ATOM | 1690 | N | GLU | D | 61 | 6.301 | -96.490 | -17.146 | 1.00185.56 |
| ATOM | 1691 | CA | GLU | D | 61 | 6.623 | -96.590 | -18.564 | 1.00186.94 |
| ATOM | 1692 | C | GLU | D | 61 | 5.345 | -96.447 | -19.367 | 1.00192.95 |
| ATOM | 1693 | O | GLU | D | 61 | 4.284 | -96.841 | -18.891 | 1.00193.89 |
| ATOM | 1694 | CB | GLU | D | 61 | 7.297 | -97.945 | -18.852 | 1.00189.90 |
| ATOM | 1695 | CG | GLU | D | 61 | 7.662 | -98.218 | -20.307 | 1.00203.82 |
| ATOM | 1696 | CD | GLU | D | 61 | 8.649 | -97.249 | -20.915 | 1.00230.96 |
| ATOM | 1697 | OE1 | GLU | D | 61 | 9.687 | -96.963 | -20.275 | 1.00242.81 |
| ATOM | 1698 | OE2 | GLU | D | 61 | 8.400 | -96.815 | -22.061 | 1.00221.22 |
| ATOM | 1699 | N | SER | D | 62 | 5.431 | -95.892 | -20.573 | 1.00190.41 |
| ATOM | 1700 | CA | SER | D | 62 | 4.235 | -95.776 | -21.389 | 1.00191.83 |
| ATOM | 1701 | C | SER | D | 62 | 3.711 | -97.148 | -21.777 | 1.00194.60 |
| ATOM | 1702 | O | SER | D | 62 | 4.492 | -98.036 | -22.098 | 1.00193.38 |
| ATOM | 1703 | CB | SER | D | 62 | 4.481 | -94.925 | -22.621 | 1.00198.54 |
| ATOM | 1704 | OG | SER | D | 62 | 3.212 | -94.498 | -23.092 | 1.00212.77 |
| ATOM | 1705 | N | GLY | D | 63 | 2.400 | -97.304 | -21.682 | 1.00192.56 |
| ATOM | 1706 | CA | GLY | D | 63 | 1.708 | -98.552 | -21.958 | 1.00195.44 |
| ATOM | 1707 | C | GLY | D | 63 | 1.649 | -99.451 | -20.743 | 1.00199.81 |
| ATOM | 1708 | O | GLY | D | 63 | 1.404 | -100.657 | -20.876 | 1.00202.47 |
| ATOM | 1709 | N | VAL | D | 64 | 1.910 | -98.872 | -19.555 | 1.00192.98 |
| ATOM | 1710 | CA | VAL | D | 64 | 1.899 | -99.603 | -18.298 | 1.00192.43 |
| ATOM | 1711 | C | VAL | D | 64 | 0.658 | -99.205 | -17.526 | 1.00201.14 |
| ATOM | 1712 | O | VAL | D | 64 | 0.464 | -98.023 | -17.196 | 1.00199.33 |
| ATOM | 1713 | CB | VAL | D | 64 | 3.222 | -99.513 | -17.475 | 1.00191.77 |
| ATOM | 1714 | CG1 | VAL | D | 64 | 3.066 | -100.060 | -16.067 | 1.00190.89 |
| ATOM | 1715 | CG2 | VAL | D | 64 | 4.346 | -100.244 | -18.171 | 1.00191.38 |
| ATOM | 1716 | N | PRO D | | 65 | -0.187 | -100.216 | -17.232 | 1.00203.43 |
| ATOM | 1717 | CA | PRO | D | 65 | -1.408 | -99.961 | -16.457 | 1.00205.77 |
| ATOM | 1718 | C | PRO | D | 65 | -1.163 | -99.136 | -15.199 | 1.00208.08 |
| ATOM | 1719 | O | PRO | D | 65 | -0.107 | -99.252 | -14.565 | 1.00206.69 |
| ATOM | 1720 | CB | PRO | D | 65 | -1.881 | -101.374 | -16.100 | 1.00210.71 |
| ATOM | 1721 | CG | PRO | D | 65 | -1.401 | -102.213 | -17.233 | 1.00215.95 |
| ATOM | 1722 | CD | PRO | D | 65 | -0.049 | -101.654 | -17.552 | 1.00207.75 |
| ATOM | 1723 | N | ASP | D | 66 | -2.142 | -98.314 | -14.831 | 1.00203.72 |
| ATOM | 1724 | CA | ASP | D | 66 | -2.040 | -97.491 | -13.640 | 1.00201.67 |
| ATOM | 1725 | C | ASP | D | 66 | -2.024 | -98.341 | -12.374 | 1.00202.63 |
| ATOM | 1726 | O | ASP | D | 66 | -1.806 | -97.836 | -11.277 | 1.00200.56 |
| ATOM | 1727 | CB | ASP | D | 66 | -3.184 | -96.473 | -13.612 | 1.00205.67 |
| ATOM | 1728 | CG | ASP | D | 66 | -3.390 | -95.763 | -14.935 | 1.00221.39 |
| ATOM | 1729 | OD1 | ASP | D | 66 | -2.382 | -95.342 | -15.547 | 1.00219.88 |
| ATOM | 1730 | OD2 | ASP | D | 66 | -4.555 | -95.653 | -15.373 | 1.00232.60 |
| ATOM | 1731 | N | ARG | D | 67 | -2.218 | -99.636 | -12.542 | 1.00200.16 |
| ATOM | 1732 | CA | ARG | D | 67 | -2.249 | -100.610 | -11.465 | 1.00201.55 |
| ATOM | 1733 | C | ARG | D | 67 | -0.911 | -100.708 | -10.764 | 1.00200.30 |
| ATOM | 1734 | O | ARG | D | 67 | -0.869 | -100.986 | -9.566 | 1.00199.69 |
| ATOM | 1735 | CB | ARG | D | 67 | -2.643 | -101.992 | -12.027 | 1.00206.89 |
| ATOM | 1736 | CG | ARG | D | 67 | -3.955 | -102.002 | -12.812 | 1.00219.57 |
| ATOM | 1737 | CD | ARG | D | 67 | -4.566 | -103.387 | -12.907 | 1.00228.88 |
| ATOM | 1738 | NE | ARG | D | 67 | -3.611 | -104.405 | -13.346 | 1.00234.98 |
| ATOM | 1739 | CZ | ARG | D | 67 | -3.447 | -104.757 | -14.612 | 1.00251.02 |
| ATOM | 1740 | NH1 | ARG | D | 67 | -4.163 | -104.174 | -15.568 | 1.00247.70 |
| ATOM | 1741 | NH2 | ARG | D | 67 | -2.542 | -105.656 | -14.944 | 1.00226.98 |
| ATOM | 1742 | N | PHE | D | 68 | 0.170 | -100.453 | -11.507 | 1.00194.12 |
| ATOM | 1743 | CA | PHE | D | 68 | 1.545 | -100.551 | -11.015 | 1.00192.37 |
| ATOM | 1744 | C | PHE | D | 68 | 2.039 | -99.219 | -10.489 | 1.00192.59 |
| ATOM | 1745 | O | PHE | D | 68 | 1.997 | -98.229 | -11.210 | 1.00192.74 |
| ATOM | 1746 | CB | PHE | D | 68 | 2.445 | -101.140 | -12.109 | 1.00193.96 |
| ATOM | 1747 | CG | PHE | D | 68 | 1.983 | -102.514 | -12.582 | 1.00198.33 |
| ATOM | 1748 | CD2 | PHE | D | 68 | 2.585 | -103.672 | -12.102 | 1.00201.09 |
| ATOM | 1749 | CD1 | PHE | D | 68 | 0.913 | -102.646 | -13.472 | 1.00203.37 |
| ATOM | 1750 | CE2 | PHE | D | 68 | 2.131 | -104.935 | -12.501 | 1.00206.41 |
| ATOM | 1751 | CE1 | PHE | D | 68 | 0.449 | -103.911 | -13.855 | 1.00206.98 |
| ATOM | 1752 | CZ | PHE | D | 68 | 1.074 | -105.048 | -13.382 | 1.00206.64 |
| ATOM | 1753 | N | THR | D | 69 | 2.458 | -99.183 | -9.218 | 1.00185.83 |
| ATOM | 1754 | CA | THR | D | 69 | 2.849 | -97.959 | -8.515 | 1.00182.81 |
| ATOM | 1755 | C | THR | D | 69 | 4.142 | -98.137 | -7.718 | 1.00184.84 |
| ATOM | 1756 | O | THR | D | 69 | 4.167 | -98.814 | -6.674 | 1.00185.13 |
| ATOM | 1757 | CB | THR | D | 69 | 1.660 | -97.407 | -7.660 | 1.00185.26 |
| ATOM | 1758 | OG1 | THR | D | 69 | 0.812 | -98.456 | -7.136 | 1.00179.57 |
| ATOM | 1759 | CG2 | THR | D | 69 | 0.766 | -96.457 | -8.467 | 1.00182.32 |
| ATOM | 1760 | N | GLY | D | 70 | 5.179 | -97.455 | -8.173 | 1.00179.49 |
| ATOM | 1761 | CA | GLY | D | 70 | 6.506 | -97.521 | -7.581 | 1.00179.16 |
| ATOM | 1762 | C | GLY | D | 70 | 6.711 | -96.437 | -6.560 | 1.00184.70 |
| ATOM | 1763 | O | GLY | D | 70 | 6.380 | -95.271 | -6.819 | 1.00185.83 |
| ATOM | 1764 | N | SER | D | 71 | 7.294 | -96.813 | -5.412 | 1.00180.30 |
| ATOM | 1765 | CA | SER | D | 71 | 7.521 | -95.930 | -4.264 | 1.00178.95 |
| ATOM | 1766 | C | SER | D | 71 | 8.924 | -96.155 | -3.657 | 1.00176.12 |
| ATOM | 1767 | O | SER | D | 71 | 9.622 | -97.110 | -4.027 | 1.00174.41 |
| ATOM | 1768 | CB | SER | D | 71 | 6.417 | -96.171 | -3.220 | 1.00185.24 |
| ATOM | 1769 | OG | SER | D | 71 | 5.606 | -97.332 | -3.449 | 1.00191.46 |
| ATOM | 1770 | N | GLY | D | 72 | 9.305 | -95.289 | -2.729 | 1.00170.35 |
| ATOM | 1771 | CA | GLY | D | 72 | 10.571 | -95.412 | -2.020 | 1.00170.68 |
| ATOM | 1772 | C | GLY | D | 72 | 11.660 | -94.487 | -2.501 | 1.00170.89 |
| ATOM | 1773 | O | GLY | D | 72 | 11.625 | -94.034 | -3.651 | 1.00167.84 |
| ATOM | 1774 | N | SER | D | 73 | 12.660 | -94.241 | -1.617 | 1.00167.43 |
| ATOM | 1775 | CA | SER | D | 73 | 13.757 | -93.328 | -1.908 | 1.00166.67 |
| ATOM | 1776 | C | SER | D | 73 | 14.978 | -93.690 | -1.111 | 1.00168.74 |
| ATOM | 1777 | O | SER | D | 73 | 14.857 | -94.291 | -0.035 | 1.00165.24 |
| ATOM | 1778 | CB | SER | D | 73 | 13.337 | -91.886 | -1.624 | 1.00171.60 |
| ATOM | 1779 | OG | SER | D | 73 | 14.204 | -90.938 | -2.224 | 1.00181.40 |
| ATOM | 1780 | N | GLY | D | 74 | 16.135 | -93.358 | -1.690 | 1.00169.50 |
| ATOM | 1781 | CA | GLY | D | 74 | 17.440 | -93.627 | -1.114 | 1.00174.48 |
| ATOM | 1782 | C | GLY | D | 74 | 17.842 | -95.078 | -1.246 | 1.00184.14 |
| ATOM | 1783 | O | GLY | D | 74 | 18.356 | -95.469 | -2.293 | 1.00183.08 |
| ATOM | 1784 | N | THR | D | 75 | 17.621 | -95.888 | -0.185 | 1.00185.46 |
| ATOM | 1785 | CA | THR | D | 75 | 18.002 | -97.305 | -0.161 | 1.00186.96 |
| ATOM | 1786 | C | THR | D | 75 | 16.836 | -98.278 | -0.080 | 1.00192.46 |
| ATOM | 1787 | O | THR | D | 75 | 17.062 | -99.473 | -0.225 | 1.00191.52 |
| ATOM | 1788 | CB | THR | D | 75 | 18.981 | -97.572 | 0.993 | 1.00198.02 |
| ATOM | 1789 | OG1 | THR | D | 75 | 18.339 | -97.329 | 2.247 | 1.00199.48 |
| ATOM | 1790 | CG2 | THR | D | 75 | 20.239 | -96.746 | 0.888 | 1.00198.65 |
| ATOM | 1791 | N | ASP | D | 76 | 15.612 | -97.799 | 0.195 | 1.00191.12 |
| ATOM | 1792 | CA | ASP | D | 76 | 14.453 | -98.673 | 0.391 | 1.00190.88 |
| ATOM | 1793 | C | ASP | D | 76 | 13.360 | -98.376 | -0.585 | 1.00189.06 |
| ATOM | 1794 | O | ASP | D | 76 | 12.939 | -97.221 | -0.698 | 1.00187.77 |
| ATOM | 1795 | CB | ASP | D | 76 | 13.940 | -98.587 | 1.843 | 1.00196.37 |
| ATOM | 1796 | CG | ASP | D | 76 | 15.013 | -98.565 | 2.931 | 1.00217.24 |
| ATOM | 1797 | OD1 | ASP | D | 76 | 15.076 | -97.558 | 3.684 | 1.00219.21 |
| ATOM | 1798 | OD2 | ASP | D | 76 | 15.788 | -99.558 | 3.034 | 1.00228.14 |
| ATOM | 1799 | N | PHE | D | 77 | 12.913 | -99.416 | -1.305 | 1.00183.27 |
| ATOM | 1800 | CA | PHE | D | 77 | 11.924 | -99.300 | -2.384 | 1.00181.25 |
| ATOM | 1801 | C | PHE | D | 77 | 10.842 | -100.332 | -2.342 | 1.00182.19 |
| ATOM | 1802 | O | PHE | D | 77 | 11.042 | -101.397 | -1.786 | 1.00181.92 |
| ATOM | 1803 | CB | PHE | D | 77 | 12.617 | -99.324 | -3.766 | 1.00182.29 |
| ATOM | 1804 | CG | PHE | D | 77 | 13.637 | -98.235 | -3.932 | 1.00184.58 |
| ATOM | 1805 | CD1 | PHE | D | 77 | 14.969 | -98.459 | -3.612 | 1.00190.09 |
| ATOM | 1806 | CD2 | PHE | D | 77 | 13.267 | -96.983 | -4.402 | 1.00186.38 |
| ATOM | 1807 | CE1 | PHE | D | 77 | 15.913 | -97.443 | -3.720 | 1.00192.50 |
| ATOM | 1808 | CE2 | PHE | D | 77 | 14.206 | -95.954 | -4.484 | 1.00190.61 |
| ATOM | 1809 | CZ | PHE | D | 77 | 15.524 | -96.182 | -4.108 | 1.00191.09 |
| ATOM | 1810 | N | THR | D | 78 | 9.692 | -100.012 | -2.932 | 1.00177.56 |
| ATOM | 1811 | CA | THR | D | 78 | 8.548 | -100.906 | -2.986 | 1.00178.90 |
| ATOM | 1812 | C | THR | D | 78 | 7.844 | -100.821 | -4.331 | 1.00184.34 |
| ATOM | 1813 | O | THR | D | 78 | 7.777 | -99.759 | -4.958 | 1.00183.53 |
| ATOM | 1814 | CB | THR | D | 78 | 7.504 | -100.627 | -1.877 | 1.00188.40 |
| ATOM | 1815 | OG1 | THR | D | 78 | 6.836 | -99.394 | -2.135 | 1.00192.49 |
| ATOM | 1816 | CG2 | THR | D | 78 | 8.079 | -100.641 | -0.480 | 1.00187.10 |
| ATOM | 1817 | N | LEU | D | 79 | 7.292 | -101.942 | -4.759 | 1.00182.10 |
| ATOM | 1818 | CA | LEU | D | 79 | 6.500 | -102.000 | -5.972 | 1.00180.69 |
| ATOM | 1819 | C | LEU | D | 79 | 5.115 | -102.409 | -5.546 | 1.00190.01 |
| ATOM | 1820 | O | LEU | D | 79 | 4.967 | -103.361 | -4.777 | 1.00192.54 |
| ATOM | 1821 | CB | LEU | D | 79 | 7.046 | -102.978 | -7.010 | 1.00179.15 |
| ATOM | 1822 | CG | LEU | D | 79 | 6.153 | -103.104 | -8.238 | 1.00181.84 |
| ATOM | 1823 | CD1 | LEU | D | 79 | 6.053 | -101.824 | -8.970 | 1.00179.61 |
| ATOM | 1824 | CD2 | LEU | D | 79 | 6.651 | -104.123 | -9.156 | 1.00186.16 |
| ATOM | 1825 | N | THR | D | 80 | 4.106 | -101.697 | -6.034 | 1.00186.79 |
| ATOM | 1826 | CA | THR | D | 80 | 2.743 | -101.989 | -5.679 | 1.00188.77 |
| ATOM | 1827 | C | THR | D | 80 | 1.892 | -102.273 | -6.908 | 1.00193.47 |
| ATOM | 1828 | O | THR | D | 80 | 1.973 | -101.553 | -7.905 | 1.00191.42 |
| ATOM | 1829 | CB | THR | D | 80 | 2.184 | -100.826 | -4.846 | 1.00195.92 |
| ATOM | 1830 | OG1 | THR | D | 80 | 3.007 | -100.615 | -3.703 | 1.00195.43 |
| ATOM | 1831 | CG2 | THR | D | 80 | 0.747 | -101.044 | -4.425 | 1.00196.44 |
| ATOM | 1832 | N | ILE | D | 81 | 1.052 | -103.311 | -6.809 | 1.00192.77 |
| ATOM | 1833 | CA | ILE | D | 81 | 0.032 | -103.634 | -7.797 | 1.00193.20 |
| ATOM | 1834 | C | ILE | D | 81 | -1.331 | -103.428 | -7.173 | 1.00200.67 |
| ATOM | 1835 | O | ILE | D | 81 | -1.751 | -104.204 | -6.313 | 1.00201.01 |
| ATOM | 1836 | CB | ILE | D | 81 | 0.152 | -105.017 | -8.401 | 1.00196.53 |
| ATOM | 1837 | CG1 | ILE | D | 81 | 1.545 | -105.177 | -9.029 | 1.00193.45 |
| ATOM | 1838 | CG2 | ILE | D | 81 | -1.002 | -105.216 | -9.420 | 1.00198.93 |
| ATOM | 1839 | CD1 | ILE | D | 81 | 1.940 | -106.547 | -9.327 | 1.00196.84 |
| ATOM | 1840 | N | SER | D | 82 | -1.986 | -102.335 | -7.590 | 1.00199.40 |
| ATOM | 1841 | CA | SER | D | 82 | -3.324 | -101.937 | -7.222 | 1.00203.31 |
| ATOM | 1842 | C | SER | D | 82 | -4.127 | -102.884 | -8.098 | 1.00210.21 |
| ATOM | 1843 | O | SER | D | 82 | -3.753 | -103.163 | -9.247 | 1.00208.34 |
| ATOM | 1844 | CB | SER | D | 82 | -3.576 | -100.484 | -7.626 | 1.00206.99 |
| ATOM | 1845 | OG | SER | D | 82 | -2.605 | -99.594 | -7.081 | 1.00215.69 |
| ATOM | 1846 | N | SER | D | 83 | -5.100 | -103.517 | -7.476 | 1.00210.94 |
| ATOM | 1847 | CA | SER | D | 83 | -5.990 | -104.558 | -7.996 | 1.00214.12 |
| ATOM | 1848 | C | SER | D | 83 | -5.351 | -105.552 | -8.948 | 1.00214.79 |
| ATOM | 1849 | O | SER | D | 83 | -5.291 | -105.299 | -10.147 | 1.00212.61 |
| ATOM | 1850 | CB | SER | D | 83 | -7.289 | -103.979 | -8.548 | 1.00220.53 |
| ATOM | 1851 | OG | SER | D | 83 | -7.943 | -104.843 | -9.467 | 1.00231.50 |
| ATOM | 1852 | N | VAL | D | 84 | -4.892 | -106.684 | -8.417 | 1.00211.27 |
| ATOM | 1853 | CA | VAL | D | 84 | -4.267 | -107.749 | -9.197 | 1.00209.85 |
| ATOM | 1854 | C | VAL | D | 84 | -5.289 | -108.341 | -10.195 | 1.00214.07 |
| ATOM | 1855 | O | VAL | D | 84 | -6.425 | -108.646 | -9.823 | 1.00218.37 |
| ATOM | 1856 | CB | VAL | D | 84 | -3.642 | -108.833 | -8.273 | 1.00214.57 |
| ATOM | 1857 | CG1 | VAL | D | 84 | -3.155 | -110.027 | -9.074 | 1.00215.31 |
| ATOM | 1858 | CG2 | VAL | D | 84 | -2.494 | -108.270 | -7.443 | 1.00210.40 |
| ATOM | 1859 | N | LYS | D | 85 | -4.882 | -108.448 | -11.466 | 1.00205.27 |
| ATOM | 1860 | CA | LYS | D | 85 | -5.696 | -109.023 | -12.514 | 1.00206.16 |
| ATOM | 1861 | C | LYS | D | 85 | -5.084 | -110.331 | -12.885 | 1.00209.74 |
| ATOM | 1862 | O | LYS | D | 85 | -3.893 | -110.549 | -12.658 | 1.00205.61 |
| ATOM | 1863 | CB | LYS | D | 85 | -5.791 | -108.092 | -13.720 | 1.00204.13 |
| ATOM | 1864 | CG | LYS | D | 85 | -6.994 | -107.169 | -13.609 | 1.00188.93 |
| ATOM | 1865 | CD | LYS | D | 85 | -6.871 | -105.991 | -14.516 | 1.00193.08 |
| ATOM | 1866 | CE | LYS | D | 85 | -7.774 | -104.843 | -14.103 | 1.00206.34 |
| ATOM | 1867 | NZ | LYS | D | 85 | -7.555 | -103.596 | -14.911 | 1.00209.61 |
| ATOM | 1868 | N | ALA | D | 86 | -5.921 | -111.204 | -13.450 | 1.00210.66 |
| ATOM | 1869 | CA | ALA | D | 86 | -5.631 | -112.558 | -13.894 | 1.00212.08 |
| ATOM | 1870 | C | ALA | D | 86 | -4.171 | -112.752 | -14.360 | 1.00211.52 |
| ATOM | 1871 | O | ALA | D | 86 | -3.395 | -113.573 | -13.813 | 1.00209.68 |
| ATOM | 1872 | CB | ALA | D | 86 | -6.632 | -112.956 | -14.995 | 1.00216.81 |
| ATOM | 1873 | N | GLU | D | 87 | -3.789 | -111.936 | -15.324 | 1.00205.45 |
| ATOM | 1874 | CA | GLU | D | 87 | -2.488 | -112.114 | -15.894 | 1.00202.29 |
| ATOM | 1875 | C | GLU | D | 87 | -1.367 | -111.429 | -15.250 | 1.00202.93 |
| ATOM | 1876 | O | GLU | D | 87 | -0.270 | -111.456 | -15.806 | 1.00200.02 |
| ATOM | 1877 | CB | GLU | D | 87 | -2.472 | -111.997 | -17.417 | 1.00203.98 |
| ATOM | 1878 | CG | GLU | D | 87 | -3.198 | -110.822 | -18.010 | 1.00206.37 |
| ATOM | 1879 | CD | GLU | D | 87 | -2.779 | -110.681 | -19.452 | 1.00210.19 |
| ATOM | 1880 | OE1 | GLU | D | 87 | -1.858 | -109.874 | -19.718 | 1.00211.63 |
| ATOM | 1881 | OE2 | GLU | D | 87 | -3.262 | -111.483 | -20.284 | 1.00184.05 |
| ATOM | 1882 | N | ASP | D | 88 | -1.573 | -110.892 | -14.046 | 1.00200.66 |
| ATOM | 1883 | CA | ASP | D | 88 | -0.477 | -110.217 | -13.353 | 1.00197.72 |
| ATOM | 1884 | C | ASP | D | 88 | 0.558 | -111.167 | -12.749 | 1.00204.14 |
| ATOM | 1885 | O | ASP | D | 88 | 1.615 | -110.729 | -12.269 | 1.00200.68 |
| ATOM | 1886 | CB | ASP | D | 88 | -0.970 | -109.158 | -12.376 | 1.00198.54 |
| ATOM | 1887 | CG | ASP | D | 88 | -1.752 | -107.998 | -13.001 | 1.00206.72 |
| ATOM | 1888 | OD1 | ASP | D | 88 | -1.605 | -107.764 | -14.220 | 1.00205.23 |
| ATOM | 1889 | OD2 | ASP | D | 88 | -2.470 | -107.292 | -12.252 | 1.00212.88 |
| ATOM | 1890 | N | LEU | D | 89 | 0.283 | -112.484 | -12.856 | 1.00205.96 |
| ATOM | 1891 | CA | LEU | D | 89 | 1.203 | -113.548 | -12.434 | 1.00206.34 |
| ATOM | 1892 | C | LEU | D | 89 | 2.552 | -113.308 | -13.099 | 1.00208.82 |
| ATOM | 1893 | O | LEU | D | 89 | 2.613 | -113.160 | -14.325 | 1.00207.67 |
| ATOM | 1894 | CB | LEU | D | 89 | 0.686 | -114.925 | -12.906 | 1.00209.73 |
| ATOM | 1895 | CG | LEU | D | 89 | 1.339 | -116.220 | -12.348 | 1.00213.51 |
| ATOM | 1896 | CD1 | LEU | D | 89 | 0.812 | -116.556 | -11.000 | 1.00215.07 |
| ATOM | 1897 | CD2 | LEU | D | 89 | 2.903 | -116.335 | -12.466 | 1.00209.74 |
| ATOM | 1898 | N | ALA | D | 90 | 3.628 | -113.297 | -12.299 | 1.00204.73 |
| ATOM | 1899 | CA | ALA | D | 90 | 4.975 | -113.111 | -12.804 | 1.00202.90 |
| ATOM | 1900 | C | ALA | D | 90 | 5.995 | -112.947 | -11.676 | 1.00206.56 |
| ATOM | 1901 | O | ALA | D | 90 | 5.634 | -112.964 | -10.498 | 1.00206.82 |
| ATOM | 1902 | CB | ALA | D | 90 | 5.012 | -111.897 | -13.711 | 1.00201.60 |
| ATOM | 1903 | N | VAL | D | 91 | 7.283 | -112.814 | -12.055 | 1.00201.49 |
| ATOM | 1904 | CA | VAL | D | 91 | 8.410 | -112.579 | -11.143 | 1.00198.71 |
| ATOM | 1905 | C | VAL | D | 91 | 8.779 | -111.120 | -11.324 | 1.00198.81 |
| ATOM | 1906 | O | VAL | D | 91 | 8.942 | -110.662 | -12.459 | 1.00198.17 |
| ATOM | 1907 | CB | VAL | D | 91 | 9.631 | -113.478 | -11.422 | 1.00202.32 |
| ATOM | 1908 | CG1 | VAL | D | 91 | 10.739 | -113.205 | -10.419 | 1.00200.40 |
| ATOM | 1909 | CG2 | VAL | D | 91 | 9.239 | -114.946 | -11.384 | 1.00205.18 |
| ATOM | 1910 | N | TYR | D | 92 | 8.911 | -110.392 | -10.215 | 1.00191.93 |
| ATOM | 1911 | CA | TYR | D | 92 | 9.237 | -108.975 | -10.248 | 1.00187.56 |
| ATOM | 1912 | C | TYR | D | 92 | 10.666 | -108.729 | -9.774 | 1.00192.96 |
| ATOM | 1913 | O | TYR | D | 92 | 11.112 | -109.317 | -8.787 | 1.00194.80 |
| ATOM | 1914 | CB | TYR | D | 92 | 8.190 | -108.196 | -9.456 | 1.00185.58 |
| ATOM | 1915 | CG | TYR | D | 92 | 6.789 | -108.290 | -10.057 | 1.00185.71 |
| ATOM | 1916 | CD2 | TYR | D | 92 | 5.982 | -109.418 | -9.854 | 1.00187.79 |
| ATOM | 1917 | CD1 | TYR | D | 92 | 6.322 | -107.318 | -10.927 | 1.00186.38 |
| ATOM | 1918 | CE2 | TYR | D | 92 | 4.701 | -109.510 | -10.419 | 1.00189.42 |
| ATOM | 1919 | CE1 | TYR | D | 92 | 5.034 | -107.380 | -11.465 | 1.00187.75 |
| ATOM | 1920 | CZ | TYR | D | 92 | 4.222 | -108.471 | -11.207 | 1.00194.58 |
| ATOM | 1921 | OH | TYR | D | 92 | 2.968 | -108.515 | -11.786 | 1.00196.32 |
| ATOM | 1922 | N | TYR | D | 93 | 11.402 | -107.908 | -10.514 | 1.00188.88 |
| ATOM | 1923 | CA | TYR | D | 93 | 12.790 | -107.599 | -10.174 | 1.00189.71 |
| ATOM | 1924 | C | TYR | D | 93 | 13.010 | -106.112 | -10.046 | 1.00192.90 |
| ATOM | 1925 | O | TYR | D | 93 | 12.288 | -105.333 | -10.654 | 1.00193.36 |
| ATOM | 1926 | CB | TYR | D | 93 | 13.750 | -108.118 | -11.248 | 1.00192.77 |
| ATOM | 1927 | CG | TYR | D | 93 | 13.688 | -109.606 | -11.492 | 1.00198.17 |
| ATOM | 1928 | CD2 | TYR | D | 93 | 14.533 | -110.481 | -10.813 | 1.00200.41 |
| ATOM | 1929 | CD1 | TYR | D | 93 | 12.824 | -110.140 | -12.442 | 1.00201.67 |
| ATOM | 1930 | CE2 | TYR | D | 93 | 14.493 | -111.856 | -11.049 | 1.00203.05 |
| ATOM | 1931 | CE1 | TYR | D | 93 | 12.791 | -111.511 | -12.704 | 1.00206.17 |
| ATOM | 1932 | CZ | TYR | D | 93 | 13.628 | -112.367 | -12.008 | 1.00213.37 |
| ATOM | 1933 | OH | TYR | D | 93 | 13.558 | -113.719 | -12.264 | 1.00217.03 |
| ATOM | 1934 | N | CYS | D | 94 | 14.005 | -105.715 | -9.263 | 1.00187.97 |
| ATOM | 1935 | CA | CYS | D | 94 | 14.379 | -104.324 | -9.147 | 1.00186.24 |
| ATOM | 1936 | C | CYS | D | 94 | 15.782 | -104.274 | -9.620 | 1.00183.58 |
| ATOM | 1937 | O | CYS | D | 94 | 16.537 | -105.215 | -9.392 | 1.00181.58 |
| ATOM | 1938 | CB | CYS | D | 94 | 14.232 | -103.775 | -7.721 | 1.00188.19 |
| ATOM | 1939 | SG | CYS | D | 94 | 15.336 | -104.522 | -6.456 | 1.00194.79 |
| ATOM | 1940 | N | GLN | D | 95 | 16.124 | -103.202 | -10.303 | 1.00178.44 |
| ATOM | 1941 | CA | GLN | D | 95 | 17.463 | -102.998 | -10.810 | 1.00179.23 |
| ATOM | 1942 | C | GLN | D | 95 | 17.951 | -101.614 | -10.455 | 1.00183.94 |
| ATOM | 1943 | O | GLN | D | 95 | 17.194 | -100.657 | -10.595 | 1.00181.63 |
| ATOM | 1944 | CB | GLN | D | 95 | 17.491 | -103.163 | -12.327 | 1.00179.69 |
| ATOM | 1945 | CG | GLN | D | 95 | 18.879 | -102.956 | -12.896 | 1.00180.44 |
| ATOM | 1946 | CD | GLN | D | 95 | 18.854 | -102.687 | -14.356 | 1.00200.87 |
| ATOM | 1947 | OE1 | GLN | D | 95 | 18.392 | -101.647 | -14.821 | 1.00183.33 |
| ATOM | 1948 | NE2 | GLN | D | 95 | 19.400 | -103.603 | -15.105 | 1.00215.32 |
| ATOM | 1949 | N | GLN | D | 96 | 19.210 | -101.503 | -10.022 | 1.00183.00 |
| ATOM | 1950 | CA | GLN | D | 96 | 19.809 | -100.209 | -9.769 | 1.00183.65 |
| ATOM | 1951 | C | GLN | D | 96 | 20.645 | -99.867 | -10.991 | 1.00191.02 |
| ATOM | 1952 | O | GLN | D | 96 | 21.311 | -100.742 | -11.526 | 1.00193.49 |
| ATOM | 1953 | CB | GLN | D | 96 | 20.644 | -100.202 | -8.472 | 1.00186.66 |
| ATOM | 1954 | CG | GLN | D | 96 | 21.955 | -100.972 | -8.473 | 1.00202.10 |
| ATOM | 1955 | CD | GLN | D | 96 | 23.108 | -100.243 | -9.144 | 1.00224.37 |
| ATOM | 1956 | OE1 | GLN | D | 96 | 23.194 | -99.010 | -9.169 | 1.00213.64 |
| ATOM | 1957 | NE2 | GLN | D | 96 | 24.009 | -100.998 | -9.741 | 1.00227.63 |
| ATOM | 1958 | N | TYR | D | 97 | 20.596 | -98.630 | -11.456 | 1.00187.39 |
| ATOM | 1959 | CA | TYR | D | 97 | 21.413 | -98.236 | -12.594 | 1.00189.45 |
| ATOM | 1960 | C | TYR | D | 97 | 22.182 | -96.969 | -12.256 | 1.00195.65 |
| ATOM | 1961 | O | TYR | D | 97 | 22.461 | -96.129 | -13.112 | 1.00196.37 |
| ATOM | 1962 | CB | TYR | D | 97 | 20.574 | -98.110 | -13.868 | 1.00189.29 |
| ATOM | 1963 | CG | TYR | D | 97 | 19.325 | -97.259 | -13.751 | 1.00188.50 |
| ATOM | 1964 | CD2 | TYR | D | 97 | 19.285 | -95.969 | -14.271 | 1.00189.72 |
| ATOM | 1965 | CD1 | TYR | D | 97 | 18.152 | -97.779 | -13.214 | 1.00187.16 |
| ATOM | 1966 | CE2 | TYR | D | 97 | 18.128 | -95.202 | -14.213 | 1.00188.12 |
| ATOM | 1967 | CE1 | TYR | D | 97 | 16.990 | -97.024 | -13.158 | 1.00183.43 |
| ATOM | 1968 | CZ | TYR | D | 97 | 16.985 | -95.736 | -13.656 | 1.00191.55 |
| ATOM | 1969 | OH | TYR | D | 97 | 15.853 | -94.974 | -13.622 | 1.00191.58 |
| ATOM | 1970 | N | PHE | D | 98 | 22.553 | -96.859 | -10.985 | 1.00193.06 |
| ATOM | 1971 | CA | PHE | D | 98 | 23.288 | -95.729 | -10.449 | 1.00194.54 |
| ATOM | 1972 | C | PHE | D | 98 | 24.736 | -95.873 | -10.852 | 1.00199.60 |
| ATOM | 1973 | O | PHE | D | 98 | 25.283 | -95.021 | -11.563 | 1.00199.35 |
| ATOM | 1974 | CB | PHE | D | 98 | 23.138 | -95.725 | -8.924 | 1.00196.79 |
| ATOM | 1975 | CG | PHE | D | 98 | 23.602 | -94.450 | -8.292 | 1.00200.38 |
| ATOM | 1976 | CD2 | PHE | D | 98 | 22.779 | -93.326 | -8.272 | 1.00200.51 |
| ATOM | 1977 | CD1 | PHE | D | 98 | 24.851 | -94.373 | -7.690 | 1.00207.68 |
| ATOM | 1978 | CE2 | PHE | D | 98 | 23.217 | -92.137 | -7.697 | 1.00205.47 |
| ATOM | 1979 | CE1 | PHE | D | 98 | 25.293 | -93.182 | -7.115 | 1.00210.90 |
| ATOM | 1980 | CZ | PHE | D | 98 | 24.484 | -92.065 | -7.145 | 1.00208.04 |
| ATOM | 1981 | N | ARG | D | 99 | 25.335 | -96.985 | -10.419 | 1.00197.90 |
| ATOM | 1982 | CA | ARG | D | 99 | 26.691 | -97.337 | -10.765 | 1.00202.02 |
| ATOM | 1983 | C | ARG | D | 99 | 26.708 | -98.772 | -11.257 | 1.00203.73 |
| ATOM | 1984 | O | ARG | D | 99 | 26.411 | -99.731 | -10.518 | 1.00201.16 |
| ATOM | 1985 | CB | ARG | D | 99 | 27.714 | -96.997 | -9.671 | 1.00207.32 |
| ATOM | 1986 | CG | ARG | D | 99 | 29.083 | -96.572 | -10.260 | 1.00219.16 |
| ATOM | 1987 | CD | ARG | D | 99 | 28.950 | -95.521 | -11.369 | 1.00213.79 |
| ATOM | 1988 | NE | ARG | D | 99 | 29.938 | -95.671 | -12.444 | 1.00207.97 |
| ATOM | 1989 | CZ | ARG | D | 99 | 29.693 | -96.166 | -13.658 | 1.00202.38 |
| ATOM | 1990 | NH1 | ARG | D | 99 | 28.474 | -96.614 | -13.972 | 1.00161.04 |
| ATOM | 1991 | NH2 | ARG | D | 99 | 30.663 | -96.227 | -14.564 | 1.00194.17 |
| ATOM | 1992 | N | TYR | D | 100 | 26.952 | -98.901 | -12.573 | 1.00200.41 |
| ATOM | 1993 | CA | TYR | D | 100 | 26.862 | -100.171 | -13.269 | 1.00199.62 |
| ATOM | 1994 | C | TYR | D | 100 | 25.351 | -100.478 | -13.190 | 1.00196.13 |
| ATOM | 1995 | O | TYR | D | 100 | 24.531 | -99.578 | -12.992 | 1.00194.78 |
| ATOM | 1996 | CB | TYR | D | 100 | 27.658 | -101.279 | -12.538 | 1.00203.81 |
| ATOM | 1997 | CG | TYR | D | 100 | 29.140 | -101.030 | -12.416 | 1.00211.93 |
| ATOM | 1998 | CD1 | TYR | D | 100 | 30.009 | -101.426 | -13.415 | 1.00218.30 |
| ATOM | 1999 | CD2 | TYR | D | 100 | 29.681 | -100.441 | -11.273 | 1.00214.67 |
| ATOM | 2000 | CE1 | TYR | D | 100 | 31.385 | -101.215 | -13.306 | 1.00226.00 |
| ATOM | 2001 | CE2 | TYR | D | 100 | 31.054 | -100.196 | -11.163 | 1.00221.29 |
| ATOM | 2002 | CZ | TYR | D | 100 | 31.906 | -100.594 | -12.183 | 1.00233.84 |
| ATOM | 2003 | OH | TYR | D | 100 | 33.267 | -100.399 | -12.096 | 1.00241.18 |
| ATOM | 2004 | N | ARG | D | 101 | 24.980 | -101.733 | -13.312 | 1.00187.76 |
| ATOM | 2005 | CA | ARG | D | 101 | 23.582 | -102.115 | -13.190 | 1.00182.13 |
| ATOM | 2006 | C | ARG | D | 101 | 23.530 | -103.432 | -12.500 | 1.00186.38 |
| ATOM | 2007 | O | ARG | D | 101 | 24.337 | -104.270 | -12.797 | 1.00190.29 |
| ATOM | 2008 | CB | ARG | D | 101 | 22.876 | -102.178 | -14.546 | 1.00179.52 |
| ATOM | 2009 | CG | ARG | D | 101 | 22.825 | -100.851 | -15.291 | 1.00186.38 |
| ATOM | 2010 | CD | ARG | D | 101 | 21.875 | -100.875 | -16.449 | 1.00195.88 |
| ATOM | 2011 | NE | ARG | D | 101 | 21.789 | -99.563 | -17.074 | 1.00214.61 |
| ATOM | 2012 | CZ | ARG | D | 101 | 20.641 | -98.972 | -17.390 | 1.00239.43 |
| ATOM | 2013 | NH1 | ARG | D | 101 | 19.489 | -99.595 | -17.182 | 1.00233.84 |
| ATOM | 2014 | NH2 | ARG | D | 101 | 20.638 | -97.766 | -17.953 | 1.00224.93 |
| ATOM | 2015 | N | THR | D | 102 | 22.697 | -103.605 | -11.499 | 1.00180.66 |
| ATOM | 2016 | CA | THR | D | 102 | 22.627 | -104.893 | -10.789 | 1.00181.37 |
| ATOM | 2017 | C | THR | D | 102 | 21.181 | -105.213 | -10.521 | 1.00184.97 |
| ATOM | 2018 | O | THR | D | 102 | 20.426 | -104.301 | -10.177 | 1.00184.42 |
| ATOM | 2019 | CB | THR | D | 102 | 23.426 | -104.876 | -9.460 | 1.00188.22 |
| ATOM | 2020 | OG1 | THR | D | 102 | 22.903 | -103.861 | -8.622 | 1.00186.90 |
| ATOM | 2021 | CG2 | THR | D | 102 | 24.932 | -104.662 | -9.642 | 1.00188.81 |
| ATOM | 2022 | N | PHE | D | 103 | 20.785 | -106.490 | -10.657 | 1.00181.86 |
| ATOM | 2023 | CA | PHE | D | 103 | 19.395 | -106.882 | -10.388 | 1.00180.59 |
| ATOM | 2024 | C | PHE | D | 103 | 19.243 | -107.488 | -9.024 | 1.00191.12 |
| ATOM | 2025 | O | PHE | D | 103 | 20.172 | -108.145 | -8.559 | 1.00195.00 |
| ATOM | 2026 | CB | PHE | D | 103 | 18.908 | -107.918 | -11.400 | 1.00181.68 |
| ATOM | 2027 | CG | PHE | D | 103 | 18.625 | -107.369 | -12.763 | 1.00181.62 |
| ATOM | 2028 | CD2 | PHE | D | 103 | 19.619 | -107.342 | -13.737 | 1.00184.31 |
| ATOM | 2029 | CD1 | PHE | D | 103 | 17.359 | -106.889 | -13.085 | 1.00182.06 |
| ATOM | 2030 | CE2 | PHE | D | 103 | 19.356 | -106.844 | -15.010 | 1.00186.35 |
| ATOM | 2031 | CE1 | PHE | D | 103 | 17.095 | -106.389 | -14.357 | 1.00182.37 |
| ATOM | 2032 | CZ | PHE | D | 103 | 18.094 | -106.388 | -15.320 | 1.00182.53 |
| ATOM | 2033 | N | GLY | D | 104 | 18.063 | -107.333 | -8.417 | 1.00188.14 |
| ATOM | 2034 | CA | GLY | D | 104 | 17.759 | -107.993 | -7.154 | 1.00189.56 |
| ATOM | 2035 | C | GLY | D | 104 | 17.440 | -109.459 | -7.444 | 1.00195.67 |
| ATOM | 2036 | O | GLY | D | 104 | 17.351 | -109.882 | -8.614 | 1.00194.85 |
| ATOM | 2037 | N | GLY | D | 105 | 17.244 | -110.236 | -6.387 | 1.00193.90 |
| ATOM | 2038 | CA | GLY | D | 105 | 16.913 | -111.651 | -6.533 | 1.00194.96 |
| ATOM | 2039 | C | GLY | D | 105 | 15.532 | -111.950 | -7.099 | 1.00197.55 |
| ATOM | 2040 | O | GLY | D | 105 | 15.254 | -113.084 | -7.493 | 1.00198.82 |
| ATOM | 2041 | N | GLY | D | 106 | 14.667 | -110.943 | -7.123 | 1.00191.42 |
| ATOM | 2042 | CA | GLY | D | 106 | 13.300 | -111.072 | -7.602 | 1.00190.05 |
| ATOM | 2043 | C | GLY | D | 106 | 12.310 | -111.603 | -6.588 | 1.00194.19 |
| ATOM | 2044 | O | GLY | D | 106 | 12.677 | -112.321 | -5.645 | 1.00193.29 |
| ATOM | 2045 | N | THR | D | 107 | 11.031 | -111.244 | -6.798 | 1.00192.34 |
| ATOM | 2046 | CA | THR | D | 107 | 9.900 | -111.717 | -5.997 | 1.00194.83 |
| ATOM | 2047 | C | THR | D | 107 | 8.846 | -112.415 | -6.869 | 1.00202.67 |
| ATOM | 2048 | O | THR | D | 107 | 8.360 | -111.821 | -7.830 | 1.00202.14 |
| ATOM | 2049 | CB | THR | D | 107 | 9.290 | -110.667 | -5.065 | 1.00203.22 |
| ATOM | 2050 | OG1 | THR | D | 107 | 7.903 | -110.441 | -5.321 | 1.00202.67 |
| ATOM | 2051 | CG2 | THR | D | 107 | 10.156 | -109.442 | -4.777 | 1.00200.85 |
| ATOM | 2052 | N | LYS | D | 108 | 8.504 | -113.671 | -6.542 | 1.00202.28 |
| ATOM | 2053 | CA | LYS | D | 108 | 7.532 | -114.446 | -7.315 | 1.00203.84 |
| ATOM | 2054 | C | LYS | D | 108 | 6.094 | -114.219 | -6.822 | 1.00210.93 |
| ATOM | 2055 | O | LYS | D | 108 | 5.816 | -114.367 | -5.637 | 1.00211.53 |
| ATOM | 2056 | CB | LYS | D | 108 | 7.906 | -115.940 | -7.291 | 1.00207.66 |
| ATOM | 2057 | CG | LYS | D | 108 | 6.853 | -116.891 | -7.874 | 1.00216.06 |
| ATOM | 2058 | CD | LYS | D | 108 | 7.318 | -118.353 | -7.932 | 1.00219.36 |
| ATOM | 2059 | CE | LYS | D | 108 | 7.021 | -119.112 | -6.653 | 1.00223.84 |
| ATOM | 2060 | NZ | LYS | D | 108 | 7.844 | -120.347 | -6.528 | 1.00231.67 |
| ATOM | 2061 | N | LEU | D | 109 | 5.184 | -113.874 | -7.744 | 1.00208.77 |
| ATOM | 2062 | CA | LEU | D | 109 | 3.769 | -113.687 | -7.437 | 1.00210.43 |
| ATOM | 2063 | C | LEU | D | 109 | 2.963 | -114.893 | -7.948 | 1.00216.86 |
| ATOM | 2064 | O | LEU | D | 109 | 2.945 | -115.165 | -9.162 | 1.00217.24 |
| ATOM | 2065 | CB | LEU | D | 109 | 3.192 | -112.372 | -8.037 | 1.00208.67 |
| ATOM | 2066 | CG | LEU | D | 109 | 1.685 | -112.136 | -7.770 | 1.00215.64 |
| ATOM | 2067 | CD1 | LEU | D | 109 | 1.483 | -111.618 | -6.405 | 1.00216.20 |
| ATOM | 2068 | CD2 | LEU | D | 109 | 1.081 | -111.163 | -8.753 | 1.00216.91 |
| ATOM | 2069 | N | GLU | D | 110 | 2.292 | -115.591 | -7.002 | 1.00213.68 |
| ATOM | 2070 | CA | GLU | D | 110 | 1.368 | -116.697 | -7.252 | 1.00215.12 |
| ATOM | 2071 | C | GLU | D | 110 | -0.069 | -116.175 | -7.064 | 1.00216.63 |
| ATOM | 2072 | O | GLU | D | 110 | -0.266 | -115.181 | -6.345 | 1.00217.00 |
| ATOM | 2073 | CB | GLU | D | 110 | 1.645 | -117.809 | -6.268 | 1.00218.29 |
| ATOM | 2074 | CG | GLU | D | 110 | 2.682 | -118.778 | -6.763 | 1.00223.34 |
| ATOM | 2075 | CD | GLU | D | 110 | 2.011 | -120.116 | -6.983 | 1.00241.29 |
| ATOM | 2076 | OE1 | GLU | D | 110 | 2.049 | -120.939 | -6.042 | 1.00231.39 |
| ATOM | 2077 | OE2 | GLU | D | 110 | 1.304 | -120.275 | -8.006 | 1.00235.24 |
| ATOM | 2078 | N | ILE | D | 111 | -1.058 | -116.786 | -7.734 | 1.00209.72 |
| ATOM | 2079 | CA | ILE | D | 111 | -2.427 | -116.298 | -7.592 | 1.00209.45 |
| ATOM | 2080 | C | ILE | D | 111 | -3.282 | -117.272 | -6.841 | 1.00213.92 |
| ATOM | 2081 | O | ILE | D | 111 | -3.237 | -118.457 | -7.146 | 1.00216.23 |
| ATOM | 2082 | CB | ILE | D | 111 | -3.038 | -115.742 | -8.918 | 1.00211.76 |
| ATOM | 2083 | CG1 | ILE | D | 111 | -2.371 | -114.401 | -9.272 | 1.00206.29 |
| ATOM | 2084 | CG2 | ILE | D | 111 | -4.569 | -115.572 | -8.873 | 1.00216.45 |
| ATOM | 2085 | CD1 | ILE | D | 111 | -2.039 | -114.276 | -10.603 | 1.00204.47 |
| ATOM | 2086 | N | LYS | D | 112 | -4.034 | -116.783 | -5.837 | 1.00209.55 |
| ATOM | 2087 | CA | LYS | D | 112 | -4.971 | -117.588 | -5.055 | 1.00214.52 |
| ATOM | 2088 | C | LYS | D | 112 | -6.340 | -117.653 | -5.834 | 1.00225.19 |
| ATOM | 2089 | O | LYS | D | 112 | -6.885 | -116.609 | -6.217 | 1.00224.58 |
| ATOM | 2090 | CB | LYS | D | 112 | -5.132 | -117.015 | -3.634 | 1.00215.44 |
| ATOM | 2091 | CG | LYS | D | 112 | -5.19,0 | -118.071 | -2.526 | 1.00210.41 |
| ATOM | 2092 | CD | LYS | D | 112 | -6.095 | -117.694 | -1.337 | 1.00208.37 |
| ATOM | 2093 | CE | LYS | D | 112 | -6.059 | -118.727 | -0.235 | 1.00210.21 |
| ATOM | 2094 | NZ | LYS | D | 112 | -4.692 | -118.905 | 0.343 | 1.00208.55 |
| ATOM | 2095 | N | ARG | D | 113 | -6.833 | -118.898 | -6.118 | 1.00226.17 |
| ATOM | 2096 | CA | ARG | D | 113 | -8.029 | -119.303 | -6.898 | 1.00230.36 |
| ATOM | 2097 | C | ARG | D | 113 | -7.993 | -120.819 | -7.000 | 1.00234.06 |
| ATOM | 2098 | O | ARG | D | 113 | -6.990 | -121.445 | -6.636 | 1.00230.63 |
| ATOM | 2099 | CB | ARG | D | 113 | -7.984 | -118.769 | -8.364 | 1.00229.82 |
| ATOM | 2100 | CG | ARG | D | 113 | -6.619 | -119.003 | -9.064 | 1.00234.74 |
| ATOM | 2101 | CD | ARG | D | 113 | -6.637 | -118.853 | -10.556 | 1.00235.50 |
| ATOM | 2102 | NE | ARG | D | 113 | -6.924 | -120.127 | -11.198 | 1.00236.25 |
| ATOM | 2103 | CZ | ARG | D | 113 | -6.687 | -120.385 | -12.479 | 1.00235.48 |
| ATOM | 2104 | NH1 | ARG | D | 113 | -6.136 | -119.452 | -13.263 | 1.00208.20 |
| ATOM | 2105 | NH2 | ARG | D | 113 | -6.991 | -121.577 | -12.989 | 1.00220.17 |
| ATOM | 2106 | N | ALA | D | 114 | -9.049 | -121.409 | -7.562 | 1.00231.62 |
| ATOM | 2107 | CA | ALA | D | 114 | -9.061 | -122.849 | -7.801 | 1.00234.83 |
| ATOM | 2108 | C | ALA | D | 114 | -8.547 | -123.086 | -9.225 | 1.00244.40 |
| ATOM | 2109 | O | ALA | D | 114 | -8.026 | -122.178 | -9.886 | 1.00190.32 |
| ATOM | 2110 | CB | ALA | D | 114 | -10.464 | -123.424 | -7.630 | 1.00237.35 |
| ATOM | 2112 | N | GLY | E | 5 | 27.912 | -80.762 | -36.382 | 1.00190.30 |
| ATOM | 2113 | CA | GLY | E | 5 | 29.114 | -79.940 | -36.433 | 1.00191.93 |
| ATOM | 2114 | C | GLY | E | 5 | 29.761 | -79.669 | -35.086 | 1.00196.83 |
| ATOM | 2115 | O | GLY | E | 5 | 29.401 | -80.286 | -34.080 | 1.00195.48 |
| ATOM | 2116 | N | GLU | E | 6 | 30.705 | -78.711 | -35.051 | 1.00195.47 |
| ATOM | 2117 | CA | GLU | E | 6 | 31.470 | -78.372 | -33.845 | 1.00194.57 |
| ATOM | 2118 | C | GLU | E | 6 | 30.702 | -77.628 | -32.773 | 1.00195.54 |
| ATOM | 2119 | O | GLU | E | 6 | 29.643 | -77.039 | -32.995 | 1.00193.14 |
| ATOM | 2120 | CB | GLU | E | 6 | 32.749 | -77.570 | -34.207 | 1.00198.44 |
| ATOM | 2121 | CG | GLU | E | 6 | 33.928 | -77.704 | -33.241 | 1.00211.27 |
| ATOM | 2122 | CD | GLU | E | 6 | 34.262 | -79.084 | -32.693 | 1.00241.32 |
| ATOM | 2123 | OE1 | GLU | E | 6 | 34.560 | -79.985 | -33.510 | 1.00249.04 |
| ATOM | 2124 | OE2 | GLU | E | 6 | 34.232 | -79.262 | -31.452 | 1.00230.42 |
| ATOM | 2125 | N | VAL | E | 7 | 31.266 | -77.689 | -31.589 | 1.00192.06 |
| ATOM | 2126 | CA | VAL | E | 7 | 30.891 | -76.891 | -30.460 | 1.00189.19 |
| ATOM | 2127 | C | VAL | E | 7 | 31.946 | -75.786 | -30.491 | 1.00199.28 |
| ATOM | 2128 | O | VAL | E | 7 | 33.138 | -76.061 | -30.428 | 1.00201.59 |
| ATOM | 2129 | CB | VAL | E | 7 | 30.872 | -77.656 | -29.121 | 1.00190.79 |
| ATOM | 2130 | CG1 | VAL | E | 7 | 32.072 | -78.598 | -28.938 | 1.00193.75 |
| ATOM | 2131 | CG2 | VAL | E | 7 | 30.748 | -76.695 | -27.947 | 1.00187.49 |
| ATOM | 2132 | N | CYS | E | 8 | 31.529 | -74.554 | -30.682 | 1.00198.67 |
| ATOM | 2133 | CA | CYS | E | 8 | 32.488 | -73.462 | -30.686 | 1.00201.29 |
| ATOM | 2134 | C | CYS | E | 8 | 32.291 | -72.683 | -29.398 | 1.00201.30 |
| ATOM | 2135 | O | CYS | E | 8 | 31.240 | -72.781 | -28.771 | 1.00199.02 |
| ATOM | 2136 | CB | CYS | E | 8 | 32.344 | -72.569 | -31.916 | 1.00204.24 |
| ATOM | 2137 | SG | CYS | E | 8 | 32.038 | -73.461 | -33.461 | 1.00211.47 |
| ATOM | 2138 | N | PRO | E | 9 | 33.299 | -71.936 | -28.961 | 1.00197.03 |
| ATOM | 2139 | CA | PRO | E | 9 | 33.147 | -71.179 | -27.714 | 1.00194.11 |
| ATOM | 2140 | C | PRO | E | 9 | 32.463 | -69.851 | -27.968 | 1.00195.35 |
| ATOM | 2141 | O | PRO | E | 9 | 32.379 | -69.426 | -29.121 | 1.00195.82 |
| ATOM | 2142 | CB | PRO | E | 9 | 34.588 | -70.952 | -27.293 | 1.00198.59 |
| ATOM | 2143 | CG | PRO | E | 9 | 35.338 | -70.828 | -28.622 | 1.00206.81 |
| ATOM | 2144 | CD | PRO | E | 9 | 34.629 | -71.736 | -29.582 | 1.00202.38 |
| ATOM | 2145 | N | GLY | E | 10 | 32.035 | -69.184 | -26.897 | 1.00189.98 |
| ATOM | 2146 | CA | GLY | E | 10 | 31.445 | -67.856 | -26.990 | 1.00189.31 |
| ATOM | 2147 | C | GLY | E | 10 | 32.311 | -66.912 | -27.810 | 1.00196.49 |
| ATOM | 2148 | O | GLY | E | 10 | 33.537 | -66.938 | -27.684 | 1.00199.14 |
| ATOM | 2149 | N | MET | E | 11 | 31.683 | -66.108 | -28.692 | 1.00192.03 |
| ATOM | 2150 | CA | MET | E | 11 | 32.379 | -65.166 | -29.562 | 1.00193.51 |
| ATOM | 2151 | C | MET | E | 11 | 31.907 | -63.765 | -29.453 | 1.00195.34 |
| ATOM | 2152 | O | MET | E | 11 | 30.729 | -63.504 | -29.210 | 1.00192.66 |
| ATOM | 2153 | CB | MET | E | 11 | 32.337 | -65.603 | -31.009 | 1.00197.71 |
| ATOM | 2154 | CG | MET | E | 11 | 32.927 | -66.939 | -31.196 | 1.00202.78 |
| ATOM | 2155 | SD | MET | E | 11 | 33.316 | -67.250 | -32.893 | 1.00211.19 |
| ATOM | 2156 | CE | MET | E | 11 | 34.526 | -68.607 | -32.682 | 1.00210.07 |
| ATOM | 2157 | N | ASP | E | 12 | 32.846 | -62.859 | -29.715 | 1.00193.90 |
| ATOM | 2158 | CA | ASP | E | 12 | 32.706 | -61.411 | -29.626 | 1.00194.03 |
| ATOM | 2159 | C | ASP | E | 12 | 33.312 | -60.813 | -30.887 | 1.00196.66 |
| ATOM | 2160 | O | ASP | E | 12 | 34.519 | -60.595 | -30.959 | 1.00198.31 |
| ATOM | 2161 | CB | ASP | E | 12 | 33.418 | -60.915 | -28.337 | 1.00196.74 |
| ATOM | 2162 | CG | ASP | E | 12 | 33.175 | -59.468 | -27.883 | 1.00206.69 |
| ATOM | 2163 | OD2 | ASP | E | 12 | 33.758 | -59.057 | -26.838 | 1.00209.66 |
| ATOM | 2164 | OD1 | ASP | E | 12 | 32.513 | -58.720 | -28.617 | 1.00207.35 |
| ATOM | 2165 | N | ILE | E | 13 | 32.464 | -60.570 | -31.886 | 1.00191.03 |
| ATOM | 2166 | CA | ILE | E | 13 | 32.863 | -60.091 | -33.208 | 1.00192.87 |
| ATOM | 2167 | C | ILE | E | 13 | 32.586 | -58.596 | -33.365 | 1.00194.77 |
| ATOM | 2168 | O | ILE | E | 13 | 31.440 | -58.168 | -33.261 | 1.00191.51 |
| ATOM | 2169 | CB | ILE | E | 13 | 32.249 | -60.995 | -34.301 | 1.00196.03 |
| ATOM | 2170 | CG1 | ILE | E | 13 | 32.430 | -62.477 | -33.916 | 1.00195.15 |
| ATOM | 2171 | CG2 | ILE | E | 13 | 32.904 | -60.730 | -35.650 | 1.00200.28 |
| ATOM | 2172 | CD1 | ILE | E | 13 | 31.462 | -63.378 | -34.481 | 1.00203.72 |
| ATOM | 2173 | N | ARG | E | 14 | 33.670 | -57.816 | -33.587 | 1.00193.23 |
| ATOM | 2174 | CA | ARG | E | 14 | 33.711 | -56.361 | -33.646 | 1.00193.96 |
| ATOM | 2175 | C | ARG | E | 14 | 34.471 | -55.816 | -34.836 | 1.00200.99 |
| ATOM | 2176 | O | ARG | E | 14 | 35.374 | -56.474 | -35.347 | 1.00202.06 |
| ATOM | 2177 | CB | ARG | E | 14 | 34.453 | -55.833 | -32.399 | 1.00191.71 |
| ATOM | 2178 | CG | ARG | E | 14 | 33.705 | -55.993 | -31.094 | 1.00189.87 |
| ATOM | 2179 | CD | ARG | E | 14 | 34.596 | -55.826 | -29.914 | 1.00190.07 |
| ATOM | 2180 | NE | ARG | E | 14 | 33.873 | -56.184 | -28.707 | 1.00199.20 |
| ATOM | 2181 | CZ | ARG | E | 14 | 33.594 | -55.329 | -27.729 | 1.00216.94 |
| ATOM | 2182 | NH1 | ARG | E | 14 | 33.993 | -54.060 | -27.809 | 1.00207.91 |
| ATOM | 2183 | NH2 | ARG | E | 14 | 32.912 | -55.733 | -26.663 | 1.00198.07 |
| ATOM | 2184 | N | ASN | E | 15 | 34.161 | -54.565 | -35.209 | 1.00199.96 |
| ATOM | 2185 | CA | ASN | E | 15 | 34.894 | -53.748 | -36.179 | 1.00204.97 |
| ATOM | 2186 | C | ASN | E | 15 | 34.910 | -54.183 | -37.651 | 1.00213.03 |
| ATOM | 2187 | O | ASN | E | 15 | 34.493 | -53.403 | -38.515 | 1.00215.01 |
| ATOM | 2188 | CB | ASN | E | 15 | 36.331 | -53.485 | -35.676 | 1.00207.54 |
| ATOM | 2189 | CG | ASN | E | 15 | 36.406 | -53.008 | -34.242 | 1.00216.10 |
| ATOM | 2190 | OD1 | ASN | E | 15 | 35.688 | -52.097 | -33.819 | 1.00206.15 |
| ATOM | 2191 | ND2 | ASN | E | 15 | 37.268 | -53.622 | -33.458 | 1.00203.36 |
| ATOM | 2192 | N | ASN | E | 16 | 35.452 | -55.394 | -37.924 | 1.00210.46 |
| ATOM | 2193 | CA | ASN | E | 16 | 35.647 | -56.051 | -39.233 | 1.00213.61 |
| ATOM | 2194 | C | ASN | E | 16 | 34.786 | -57.316 | -39.320 | 1.00218.81 |
| ATOM | 2195 | O | ASN | E | 16 | 34.679 | -58.046 | -38.328 | 1.00216.04 |
| ATOM | 2196 | CB | ASN | E | 16 | 37.091 | -56.632 | -39.293 | 1.00214.01 |
| ATOM | 2197 | CG | ASN | E | 16 | 38.239 | -55.953 | -40.033 | 1.00242.03 |
| ATOM | 2198 | OD1 | ASN | E | 16 | 38.056 | -54.969 | -40.754 | 1.00239.62 |
| ATOM | 2199 | ND2 | ASN | E | 16 | 39.466 | -56.579 | -39.858 | 1.00240.96 |
| ATOM | 2200 | N | LEU | E | 17 | 34.371 | -57.709 | -40.527 | 1.00218.96 |
| ATOM | 2201 | CA | LEU | E | 17 | 33.734 | -59.013 | -40.663 | 1.00217.56 |
| ATOM | 2202 | C | LEU | E | 17 | 34.717 | -60.180 | -40.676 | 1.00224.83 |
| ATOM | 2203 | O | LEU | E | 17 | 34.265 | -61.314 | -40.585 | 1.00221.43 |
| ATOM | 2204 | CB | LEU | E | 17 | 32.913 | -59.063 | -41.924 | 1.00219.82 |
| ATOM | 2205 | CG | LEU | E | 17 | 31.597 | -58.368 | -41.832 | 1.00223.48 |
| ATOM | 2206 | CD1 | LEU | E | 17 | 30.910 | -58.391 | -43.169 | 1.00226.56 |
| ATOM | 2207 | CD2 | LEU | E | 17 | 30.724 | -59.000 | -40.756 | 1.00221.66 |
| ATOM | 2208 | N | THR | E | 18 | 36.040 | -59.936 | -40.820 | 1.00227.88 |
| ATOM | 2209 | CA | THR | E | 18 | 37.034 | -61.027 | -40.886 | 1.00230.30 |
| ATOM | 2210 | C | THR | E | 18 | 36.804 | -62.178 | -39.878 | 1.00231.66 |
| ATOM | 2211 | O | THR | E | 18 | 36.685 | -63.335 | -40.283 | 1.00231.95 |
| ATOM | 2212 | CB | THR | E | 18 | 38.483 | -60.514 | -40.826 | 1.00241.80 |
| ATOM | 2213 | OG1 | THR | E | 18 | 38.776 | -60.077 | -39.491 | 1.00240.89 |
| ATOM | 2214 | CG2 | THR | E | 18 | 38.775 | -59.436 | -41.869 | 1.00243.03 |
| ATOM | 2215 | N | ARG | E | 19 | 36.704 | -61.853 | -38.587 | 1.00224.73 |
| ATOM | 2216 | CA | ARG | E | 19 | 36.583 | -62.832 | -37.505 | 1.00221.13 |
| ATOM | 2217 | C | ARG | E | 19 | 35.330 | -63.703 | -37.580 | 1.00223.17 |
| ATOM | 2218 | O | ARG | E | 19 | 35.301 | -64.783 | -36.994 | 1.00220.80 |
| ATOM | 2219 | CB | ARG | E | 19 | 36.636 | -62.125 | -36.133 | 1.00217.49 |
| ATOM | 2220 | CG | ARG | E | 19 | 37.883 | -61.298 | -35.834 | 1.00226.76 |
| ATOM | 2221 | CD | ARG | E | 19 | 38.075 | -61.094 | -34.336 | 1.00235.47 |
| ATOM | 2222 | NE | ARG | E | 19 | 38.089 | -62.379 | -33.615 | 1.00249.17 |
| ATOM | 2223 | CZ | ARG | E | 19 | 37.363 | -62.645 | -32.533 | 1.00260.55 |
| ATOM | 2224 | NH1 | ARG | E | 19 | 36.611 | -61.706 | -31.986 | 1.00246.40 |
| ATOM | 2225 | NH2 | ARG | E | 19 | 37.403 | -63.850 | -31.975 | 1.00242.85 |
| ATOM | 2226 | N | LEU | E | 20 | 34.293 | -63.213 | -38.275 | 1.00220.49 |
| ATOM | 2227 | CA | LEU | E | 20 | 32.989 | -63.863 | -38.393 | 1.00218.02 |
| ATOM | 2228 | C | LEU | E | 20 | 33.027 | -65.238 | -39.031 | 1.00224.59 |
| ATOM | 2229 | O | LEU | E | 20 | 32.211 | -66.098 | -38.698 | 1.00222.15 |
| ATOM | 2230 | CB | LEU | E | 20 | 32.003 | -62.944 | -39.117 | 1.00218.05 |
| ATOM | 2231 | CG | LEU | E | 20 | 30.533 | -63.341 | -39.105 | 1.00218.49 |
| ATOM | 2232 | CD1 | LEU | E | 20 | 30.010 | -63.581 | -37.695 | 1.00214.22 |
| ATOM | 2233 | CD2 | LEU | E | 20 | 29.723 | -62.298 | -39.790 | 1.00219.46 |
| ATOM | 2234 | N | HIS | E | 21 | 33.994 | -65.456 | -39.911 | 1.00225.92 |
| ATOM | 2235 | CA | HIS | E | 21 | 34.172 | -66.727 | -40.588 | 1.00228.56 |
| ATOM | 2236 | C | HIS | E | 21 | 34.668 | -67.819 | -39.653 | 1.00230.28 |
| ATOM | 2237 | O | HIS | E | 21 | 34.638 | -68.989 | -40.023 | 1.00231.01 |
| ATOM | 2238 | CB | HIS | E | 21 | 35.061 | -66.543 | -41.812 | 1.00235.48 |
| ATOM | 2239 | CG | HIS | E | 21 | 34.549 | -65.479 | -42.743 | 1.00241.44 |
| ATOM | 2240 | ND1 | HIS | E | 21 | 33.581 | -65.754 | -43.699 | 1.00244.47 |
| ATOM | 2241 | CD2 | HIS | E | 21 | 34.867 | -64.165 | -42.814 | 1.00244.74 |
| ATOM | 2242 | CE1 | HIS | E | 21 | 33.367 | -64.612 | -44.338 | 1.00245.81 |
| ATOM | 2243 | NE2 | HIS | E | 21 | 34.120 | -63.629 | -43.846 | 1.00246.37 |
| ATOM | 2244 | N | GLU | E | 22 | 35.040 | -67.448 | -38.406 | 1.00223.60 |
| ATOM | 2245 | CA | GLU | E | 22 | 35.408 | -68.410 | -37.365 | 1.00221.30 |
| ATOM | 2246 | C | GLU | E | 22 | 34.196 | -69.263 | -36.978 | 1.00222.53 |
| ATOM | 2247 | O | GLU | E | 22 | 34.357 | -70.309 | -36.342 | 1.00221.67 |
| ATOM | 2248 | CB | GLU | E | 22 | 35.939 | -67.707 | -36.120 | 1.00220.49 |
| ATOM | 2249 | CG | GLU | E | 22 | 37.420 | -67.914 | -35.903 | 1.00231.75 |
| ATOM | 2250 | CD | GLU | E | 22 | 38.116 | -66.656 | -35.436 | 1.00251.97 |
| ATOM | 2251 | OE1 | GLU | E | 22 | 37.775 | -66.173 | -34.332 | 1.00243.41 |
| ATOM | 2252 | OE2 | GLU | E | 22 | 39.063 | -66.206 | -36.123 | 1.00247.26 |
| ATOM | 2253 | N | LEU | E | 23 | 32.986 | -68.825 | -37.364 | 1.00217.35 |
| ATOM | 2254 | CA | LEU | E | 23 | 31.748 | -69.551 | -37.082 | 1.00214.21 |
| ATOM | 2255 | C | LEU | E | 23 | 31.372 | -70.555 | -38.151 | 1.00221.41 |
| ATOM | 2256 | O | LEU | E | 23 | 30.379 | -71.270 | -37.981 | 1.00219.32 |
| ATOM | 2257 | CB | LEU | E | 23 | 30.588 | -68.559 | -36.941 | 1.00211.66 |
| ATOM | 2258 | CG | LEU | E | 23 | 30.644 | -67.668 | -35.738 | 1.00213.96 |
| ATOM | 2259 | CD1 | LEU | E | 23 | 29.462 | -66.731 | -35.719 | 1.00211.96 |
| ATOM | 2260 | CD2 | LEU | E | 23 | 30.711 | -68.506 | -34.477 | 1.00215.69 |
| ATOM | 2261 | N | GLU | E | 24 | 32.118 | -70.586 | -39.271 | 1.00222.24 |
| ATOM | 2262 | CA | GLU | E | 24 | 31.756 | -71.401 | -40.422 | 1.00224.40 |
| ATOM | 2263 | C | GLU | E | 24 | 31.467 | -72.878 | -40.175 | 1.00226.88 |
| ATOM | 2264 | O | GLU | E | 24 | 30.574 | -73.414 | -40.819 | 1.00227.07 |
| ATOM | 2265 | CB | GLU | E | 24 | 32.683 | -71.169 | -41.604 | 1.00230.80 |
| ATOM | 2266 | CG | GLU | E | 24 | 34.049 | -71.821 | -41.473 | 1.00243.35 |
| ATOM | 2267 | CD | GLU | E | 24 | 34.958 | -71.526 | -42.649 | 1.00266.86 |
| ATOM | 2268 | OE1 | GLU | E | 24 | 34.680 | -70.559 | -43.391 | 1.00259.23 |
| ATOM | 2269 | OE2 | GLU | E | 24 | 35.948 | -72.267 | -42.837 | 1.00265.59 |
| ATOM | 2270 | N | ASN | E | 25 | 32.188 | -73.522 | -39.245 | 1.00221.65 |
| ATOM | 2271 | CA | ASN | E | 25 | 31.979 | -74.919 | -38.891 | 1.00220.65 |
| ATOM | 2272 | C | ASN | E | 25 | 31.492 | -74.881 | -37.462 | 0.70218.45 |
| ATOM | 2273 | O | ASN | E | 25 | 32.292 | -74.953 | -36.524 | 0.70216.67 |
| ATOM | 2274 | CB | ASN | E | 25 | 33.284 | -75.728 | -39.030 | 1.00226.31 |
| ATOM | 2275 | CG | ASN | E | 25 | 34.021 | -75.610 | -40.369 | 1.00251.67 |
| ATOM | 2276 | OD1 | ASN | E | 25 | 33.444 | -75.728 | -41.464 | 1.00245.70 |
| ATOM | 2277 | ND2 | ASN | E | 25 | 35.338 | -75.400 | -40.293 | 1.00246.35 |
| ATOM | 2278 | N | CYS | E | 26 | 30.177 | -74.669 | -37.296 | 1.00212.32 |
| ATOM | 2279 | CA | CYS | E | 26 | 29.587 | -74.494 | -35.965 | 1.00208.26 |
| ATOM | 2280 | C | CYS | E | 26 | 28.104 | -74.852 | -35.833 | 1.00207.15 |
| ATOM | 2281 | O | CYS | E | 26 | 27.267 | -74.237 | -36.493 | 1.00206.78 |
| ATOM | 2282 | CB | CYS | E | 26 | 29.843 | -73.074 | -35.470 | 1.00207.62 |
| ATOM | 2283 | SG | CYS | E | 26 | 30.083 | -72.950 | -33.687 | 1.00208.34 |
| ATOM | 2284 | N | SER | E | 27 | 27.780 | -75.761 | -34.894 | 1.00198.98 |
| ATOM | 2285 | CA | SER | E | 27 | 26.405 | -76.160 | -34.617 | 1.00195.08 |
| ATOM | 2286 | C | SER | E | 27 | 25.891 | -75.518 | -33.378 | 1.00192.07 |
| ATOM | 2287 | O | SER | E | 27 | 24.737 | -75.093 | -33.327 | 1.00188.85 |
| ATOM | 2288 | CB | SER | E | 27 | 26.311 | -77.667 | -34.458 | 1.00199.87 |
| ATOM | 2289 | OG | SER | E | 27 | 26.007 | -78.232 | -35.720 | 1.00213.74 |
| ATOM | 2290 | N | VAL | E | 28 | 26.725 | -75.516 | -32.347 | 1.00187.21 |
| ATOM | 2291 | CA | VAL | E | 28 | 26.392 | -74.968 | -31.051 | 1.00184.64 |
| ATOM | 2292 | C | VAL | E | 28 | 27.454 | -73.975 | -30.689 | 1.00188.85 |
| ATOM | 2293 | O | VAL | E | 28 | 28.629 | -74.295 | -30.774 | 1.00190.85 |
| ATOM | 2294 | CB | VAL | E | 28 | 26.351 | -76.082 | -29.953 | 1.00188.60 |
| ATOM | 2295 | CG1 | VAL | E | 28 | 26.108 | -75.503 | -28.563 | 1.00185.75 |
| ATOM | 2296 | CG2 | VAL | E | 28 | 25.306 | -77.144 | -30.252 | 1.00188.82 |
| ATOM | 2297 | N | ILE | E | 29 | 27.066 | -72.806 | -30.223 | 1.00184.12 |
| ATOM | 2298 | CA | ILE | E | 29 | 28.014 | -71.870 | -29.641 | 1.00184.41 |
| ATOM | 2299 | C | ILE | E | 29 | 27.829 | -72.042 | -28.125 | 1.00189.86 |
| ATOM | 23.00 | O | ILE | E | 29 | 26.751 | -71.766 | -27.583 | 1.00190.05 |
| ATOM | 2301 | CB | ILE | E | 29 | 27.822 | -70.413 | -30.104 | 1.00186.88 |
| ATOM | 2302 | CG1 | ILE | E | 29 | 28.375 | -70.236 | -31.528 | 1.00189.31 |
| ATOM | 2303 | CG2 | ILE | E | 29 | 28.493 | -69.436 | -29.112 | 1.00186.14 |
| ATOM | 2304 | CD1 | ILE | E | 29 | 27.912 | -68.987 | -32.200 | 1.00192.49 |
| ATOM | 2305 | N | GLU | E | 30 | 28.860 | -72.538 | -27.453 | 1.00186.81 |
| ATOM | 2306 | CA | GLU | E | 30 | 28.823 | -72.802 | -26.018 | 1.00185.57 |
| ATOM | 2307 | C | GLU | E | 30 | 29.345 | -71.539 | -25.372 | 1.00191.48 |
| ATOM | 2308 | O | GLU | E | 30 | 30.558 | -71.336 | -25.288 | 1.00194.45 |
| ATOM | 2309 | CB | GLU | E | 30 | 29.688 | -74.044 | -25.734 | 1.00188.36 |
| ATOM | 2310 | CG | GLU | E | 30 | 29.660 | -74.566 | -24.311 | 1.00194.02 |
| ATOM | 2311 | CD | GLU | E | 30 | 30.392 | -75.875 | -24.111 | 1.00199.03 |
| ATOM | 2312 | OE1 | GLU | E | 30 | 31.614 | -75.912 | -24.375 | 1.00215.71 |
| ATOM | 2313 | OE2 | GLU | E | 30 | 29.739 | -76.875 | -23.741 | 1.00167.96 |
| ATOM | 2314 | N | GLY | E | 31 | 28.414 | -70.665 | -25.011 | 1.00186.77 |
| ATOM | 2315 | CA | GLY | E | 31 | 28.691 | -69.299 | -24.574 | 1.00187.62 |
| ATOM | 2316 | C | GLY | E | 31 | 27.776 | -68.312 | -25.305 | 1.00191.54 |
| ATOM | 2317 | O | GLY | E | 31 | 26.683 | -68.692 | -25.755 | 1.00193.21 |
| ATOM | 2318 | N | HIS | E | 32 | 28.175 | -67.036 | -25.423 | 1.00183.86 |
| ATOM | 2319 | CA | HIS | E | 32 | 27.309 | -66.073 | -26.097 | 1.00181.26 |
| ATOM | 2320 | C | HIS | E | 32 | 27.808 | -65.743 | -27.484 | 1.00182.85 |
| ATOM | 2321 | O | HIS | E | 32 | 28.927 | -66.115 | -27.835 | 1.00185.20 |
| ATOM | 2322 | CB | HIS | E | 32 | 27.177 | -64.796 | -25.255 | 1.00181.54 |
| ATOM | 2323 | CG | HIS | E | 32 | 28.473 | -64.104 | -25.001 | 1.00186.07 |
| ATOM | 2324 | ND1 | HIS | E | 32 | 28.933 | -63.901 | -23.721 | 1.00187.47 |
| ATOM | 2325 | CD2 | HIS | E | 32 | 29.363 | -63.581 | -25.879 | 1.00189.88 |
| ATOM | 2326 | CE1 | HIS | E | 32 | 30.092 | -63.281 | -23.853 | 1.00188.92 |
| ATOM | 2327 | NE2 | HIS | E | 32 | 30.388 | -63.061 | -25.137 | 1.00190.66 |
| ATOM | 2328 | N | LEU | E | 33 | 26.988 | -65.024 | -28.264 | 1.00174.23 |
| ATOM | 2329 | CA | LEU | E | 33 | 27.397 | -64.510 | -29.549 | 1.00173.06 |
| ATOM | 2330 | C | LEU | E | 33 | 27.045 | -63.057 | -29.552 | 1.00175.61 |
| ATOM | 2331 | O | LEU | E | 33 | 25.870 | -62.713 | -29.418 | 1.00173.66 |
| ATOM | 2332 | CB | LEU | E | 33 | 26.742 | -65.241 | -30.728 | 1.00172.19 |
| ATOM | 2333 | CG | LEU | E | 33 | 27.166 | -64.773 | -32.121 | 1.00177.04 |
| ATOM | 2334 | CD1 | LEU | E | 33 | 28.656 | -64.738 | -32.279 | 1.00177.86 |
| ATOM | 2335 | CD2 | LEU | E | 33 | 26.562 | -65.599 | -33.180 | 1.00179.00 |
| ATOM | 2336 | N | GLN | E | 34 | 28.073 | -62.207 | -29.632 | 1.00173.47 |
| ATOM | 2337 | CA | GLN | E | 34 | 27.942 | -60.759 | -29.755 | 1.00174.45 |
| ATOM | 2338 | C | GLN | E | 34 | 28.561 | -60.343 | -31.103 | 1.00181.80 |
| ATOM | 2339 | O | GLN | E | 34 | 29.724 | -60.665 | -31.380 | 1.00185.61 |
| ATOM | 2340 | CB | GLN | E | 34 | 28.663 | -60.015 | -28.622 | 1.00175.69 |
| ATOM | 2341 | CG | GLN | E | 34 | 28.107 | -60.200 | -27.221 | 1.00189..85 |
| ATOM | 2342 | CD | GLN | E | 34 | 28.846 | -59.389 | -26.162 | 1.00207.69 |
| ATOM | 2343 | OE1 | GLN | E | 34 | 28.478 | -59.395 | -24.983 | 1.00205.33 |
| ATOM | 2344 | NE2 | GLN | E | 34 | 29.914 | -58.690 | -26.544 | 1.00197.23 |
| ATOM | 2345 | N | ILE | E | 35 | 27.773 | -59.663 | -31.951 | 1.00176.21 |
| ATOM | 2346 | CA | ILE | E | 35 | 28.236 | -59.111 | -33.217 | 1.00178.19 |
| ATOM | 2347 | C | ILE | E | 35 | 27.950 | -57.637 | -33.107 | 1.00185.67 |
| ATOM | 2348 | O | ILE | E | 35 | 26.789 | -57.238 | -32.939 | 1.00184.68 |
| ATOM | 2349 | CB | ILE | E | 35 | 27.600 | -59.743 | -34.452 | 1.00180.97 |
| ATOM | 2350 | CG1 | ILE | E | 35 | 27.684 | -61.263 | -34.394 | 1.00178.55 |
| ATOM | 2351 | CG2 | ILE | E | 35 | 28.268 | -59.180 | -35.722 | 1.00186.08 |
| ATOM | 2352 | CD1 | ILE | E | 35 | 26.981 | -61.971 | -35.497 | 1.00183.95 |
| ATOM | 2353 | N | LEU | E | 36 | 29.007 | -56.824 | -33.154 | 1.00186.01 |
| ATOM | 2354 | CA | LEU | E | 36 | 28.841 | -55.418 | -32.872 | 1.00188.00 |
| ATOM | 2355 | C | LEU | E | 36 | 29.854 | -54.463 | -33.440 | 1.00194.09 |
| ATOM | 2356 | O | LEU | E | 36 | 30.974 | -54.824 | -33.793 | 1.00195.15 |
| ATOM | 2357 | CB | LEU | E | 36 | 28.735 | -55.209 | -31.329 | 1.00186.42 |
| ATOM | 2358 | CG | LEU | E | 36 | 29.940 | -55.531 | -30.446 | 1.00191.59 |
| ATOM | 2359 | CD1 | LEU | E | 36 | 29.782 | -54.895 | -29.103 | 1.00191.87 |
| ATOM | 2360 | CD2 | LEU | E | 36 | 30.075 | -56.989 | -30.204 | 1.00190.62 |
| ATOM | 2361 | N | LEU | E | 37 | 29.447 | -53.206 | -33.434 | 1.00191.02 |
| ATOM | 2362 | CA | LEU | E | 37 | 30.281 | -52.099 | -33.777 | 1.00194.35 |
| ATOM | 2363 | C | LEU | E | 37 | 30.990 | -52.258 | -35.128 | 1.00205.69 |
| ATOM | 2364 | O | LEU | E | 37 | 32.214 | -52.182 | -35.222 | 1.00207.67 |
| ATOM | 2365 | CB | LEU | E | 37 | 31.244 | -51.804 | -32.607 | 1.00193.19 |
| ATOM | 2366 | CG | LEU | E | 37 | 30.612 | -51.458 | -31.264 | 1.00194.32 |
| ATOM | 2367 | CD1 | LEU | E | 37 | 31.625 | -51.555 | -30.167 | 1.00192.32 |
| ATOM | 2368 | CD2 | LEU | E | 37 | 30.010 | -50.072 | -31.283 | 1.00201.76 |
| ATOM | 2369 | N | MET | E | 38 | 30.194 | -52.465 | -36.178 | 1.00205.24 |
| ATOM | 2370 | CA | MET | E | 38 | 30.674 | -52.532 | -37.556 | 1.00208.91 |
| ATOM | 2371 | C | MET | E | 38 | 30.133 | -51.379 | -38.316 | 1.00213.96 |
| ATOM | 2372 | O | MET | E | 38 | 29.020 | -51.415 | -38.844 | 1.00213.31 |
| ATOM | 2373 | CB | MET | E | 38 | 30.330 | -53.850 | -38.166 | 1.00210.41 |
| ATOM | 2374 | CG | MET | E | 38 | 31.136 | -54.859 | -37.507 | 1.00212.57 |
| ATOM | 2375 | SD | MET | E | 38 | 30.965 | -56.474 | -38.132 | 1.00216.21 |
| ATOM | 2376 | CE | MET | E | 38 | 31.890 | -57.271 | -36.932 | 1.00210.50 |
| ATOM | 2377 | N | PHE | E | 39 | 30.926 | -50.324 | -38.318 | 1.00211.67 |
| ATOM | 2378 | CA | PHE | E | 39 | 30.538 | -49.050 | -38.856 | 1.00214.01 |
| ATOM | 2379 | C | PHE | E | 39 | 30.725 | -48.879 | -40.337 | 1.00220.85 |
| ATOM | 2380 | O | PHE | E | 39 | 30.068 | -48.011 | -40.918 | 1.00223.10 |
| ATOM | 2381 | CB | PHE | E | 39 | 31.258 | -47.940 | -38.109 | 1.00216.79 |
| ATOM | 2382 | CG | PHE | E | 39 | 30.981 | -47.854 | -36.631 | 1.00214.43 |
| ATOM | 2383 | CD1 | PHE | E | 39 | 32.009 | -47.685 | -35.728 | 1.00218.04 |
| ATOM | 2384 | CD2 | PHE | E | 39 | 29.695 | -47.958 | -36.141 | 1.00212.97 |
| ATOM | 2385 | CE1 | PHE | E | 39 | 31.753 | -47.550 | -34.363 | 1.00216.47 |
| ATOM | 2386 | CE2 | PHE | E | 39 | 29.446 | -47.862 | -34.773 | 1.00213.33 |
| ATOM | 2387 | CZ | PHE | E | 39 | 30.476 | -47.655 | -33.891 | 1.00211.86 |
| ATOM | 2388 | N | LYS | E | 40 | 31.620 | -49.665 | -40.958 | 1.00216.91 |
| ATOM | 2389 | CA | LYS | E | 40 | 31.898 | -49.475 | -42.383 | 1.00220.65 |
| ATOM | 2390 | C | LYS | E | 40 | 31.346 | -50.530 | -43.329 | 1.00222.92 |
| ATOM | 2391 | O | LYS | E | 40 | 31.478 | -50.405 | -44.553 | 1.00224.72 |
| ATOM | 2392 | CB | LYS | E | 40 | 33.377 | -49.150 | -42.637 | 1.00226.35 |
| ATOM | 2393 | CG | LYS | E | 40 | 33.757 | -47.711 | -42.249 | 1.00231.72 |
| ATOM | 2394 | CD | LYS | E | 40 | 35.116 | -47.256 | -42.817 | 1.00236.87 |
| ATOM | 2395 | CE | LYS | E | 40 | 36.353 | -47.900 | -42.205 | 1.00234.87 |
| ATOM | 2396 | NZ | LYS | E | 40 | 36.489 | -47.639 | -40.743 | 1.00231.80 |
| ATOM | 2397 | N | THR | E | 41 | 30.683 | -51.544 | -42.760 | 1.00216.48 |
| ATOM | 2398 | CA | THR | E | 41 | 30.031 | -52.612 | -43.518 | 1.00216.23 |
| ATOM | 2399 | C | THR | E | 41 | 28.849 | -52.045 | -44.331 | 1.00222.87 |
| ATOM | 2400 | O | THR | E | 41 | 28.281 | -50.999 | -43.977 | 1.00223.52 |
| ATOM | 2401 | CB | THR | E | 41 | 29.660 | -53.820 | -42.608 | 1.00217.13 |
| ATOM | 2402 | OG1 | THR | E | 41 | 29.104 | -53.363 | -41.371 | 1.00212.47 |
| ATOM | 2403 | CG2 | THR | E | 41 | 30.854 | -54.681 | -42.278 | 1.00214.22 |
| ATOM | 2404 | N | ARG | E | 42 | 28.520 | -52.721 | -45.445 | 1.00220.22 |
| ATOM | 2405 | CA | ARG | E | 42 | 27.473 | -52.327 | -46.376 | 1.00221.62 |
| ATOM | 2406 | C | ARG | E | 42 | 26.653 | -53.567 | -46.756 | 1.00224.84 |
| ATOM | 2407 | O | ARG | E | 42 | 27.093 | -54.685 | -46.488 | 1.00223.17 |
| ATOM | 2408 | CB | ARG | E | 42 | 28.113 | -51.682 | -47.612 | 1.00224.41 |
| ATOM | 2409 | CG | ARG | E | 42 | 29.142 | -50.605 | -47.288 | 1.00230.96 |
| ATOM | 2410 | CD | ARG | E | 42 | 30.525 | -50.980 | -47.788 | 1.00236.77 |
| ATOM | 2411 | NE | ARG | E | 42 | 30.995 | -50.017 | -48.789 | 1.00244.00 |
| ATOM | 2412 | CZ | ARG | E | 42 | 32.134 | -50.119 | -49.469 | 1.00249.77 |
| ATOM | 2413 | NH1 | ARG | E | 42 | 32.941 | -51.155 | -49.274 | 1.00233.66 |
| ATOM | 2414 | NH2 | ARG | E | 42 | 32.475 | -49.183 | -50.349 | 1.00241.43 |
| ATOM | 2415 | N | PRO | E | 43 | 25.442 | -53.409 | -47.323 | 1.00222.40 |
| ATOM | 2416 | CA | PRO | E | 43 | 24.621 | -54.594 | -47.656 | 1.00220.77 |
| ATOM | 2417 | C | PRO | E | 43 | 25.313 | -55.669 | -48.502 | 1.00227.06 |
| ATOM | 2418 | O | PRO | E | 43 | 25.099 | -56.866 | -48.281 | 1.00224.64 |
| ATOM | 2419 | CB | PRO | E | 43 | 23.414 | -53.988 | -48.366 | 1.00225.14 |
| ATOM | 2420 | CG | PRO | E | 43 | 23.326 | -52.591 | -47.841 | 1.00230.23 |
| ATOM | 2421 | CD | PRO | E | 43 | 24.735 | -52.154 | -47.650 | 1.00226.84 |
| ATOM | 2422 | N | GLU | E | 44 | 26.191 | -55.235 | -49.420 | 1.00228.77 |
| ATOM | 2423 | CA | GLU | E | 44 | 26.958 | -56.117 | -50.299 | 1.00231.78 |
| ATOM | 2424 | C | GLU | E | 44 | 27.824 | -57.103 | -49.533 | 1.00233.10 |
| ATOM | 2425 | O | GLU | E | 44 | 28.011 | -58.217 | -50.009 | 1.00233.75 |
| ATOM | 2426 | CB | GLU | E | 44 | 27.803 | -55.316 | -51.296 | 1.00239.38 |
| ATOM | 2427 | CG | GLU | E | 44 | 28.774 | -54.336 | -50.645 | 1.00253.39 |
| ATOM | 2428 | CD | GLU | E | 44 | 29.113 | -53.072 | -51.414 | 1.00277.00 |
| ATOM | 2429 | OE1 | GLU | E | 44 | 28.651 | -52.908 | -52.568 | 1.00277.81 |
| ATOM | 2430 | OE2 | GLU | E | 44 | 29.855 | -52.238 | -50.848 | 1.00275.47 |
| ATOM | 2431 | N | ASP | E | 45 | 28.320 | -56.709 | -48.343 | 1.00226.13 |
| ATOM | 2432 | CA | ASP | E | 45 | 29.135 | -57.560 | -47.480 | 1.00222.52 |
| ATOM | 2433 | C | ASP | E | 45 | 28.328 | -58.734 | -46.895 | 1.00219.85 |
| ATOM | 2434 | O | ASP | E | 45 | 28.909 | -59.735 | -46.484 | 1.00216.59 |
| ATOM | 2435 | CB | ASP | E | 45 | 29.787 | -56.708 | -46.373 | 1.00223.04 |
| ATOM | 2436 | CG | ASP | E | 45 | 30.761 | -55.657 | -46.909 | 1.00241.04 |
| ATOM | 2437 | OD1 | ASP | E | 45 | 30.306 | -54.732 | -47.630 | 1.00244.07 |
| ATOM | 2438 | OD2 | ASP | E | 45 | 31.988 | -55.831 | -46.728 | 1.00250.01 |
| ATOM | 2439 | N | PHE | E | 46 | 26.998 | -58.626 | -46.891 | 1.00215.66 |
| ATOM | 2440 | CA | PHE | E | 46 | 26.102 | -59.638 | -46.343 | 1.00212.72 |
| ATOM | 2441 | C | PHE | E | 46 | 25.268 | -60.390 | -47.371 | 1.00222.83 |
| ATOM | 2442 | O | PHE | E | 46 | 24.539 | -61.313 | -47.004 | 1.00218.72 |
| ATOM | 2443 | CB | PHE | E | 46 | 25.181 | -59.002 | -45.297 | 1.00210.92 |
| ATOM | 2444 | CG | PHE | E | 46 | 25.912 | -58.340 | -44.158 | 1.00210.43 |
| ATOM | 2445 | CD2 | PHE | E | 46 | 25.721 | -56.995 | -43.880 | 1.00213.14 |
| ATOM | 2446 | CD1 | PHE | E | 46 | 26.818 | -59.056 | -43.380 | 1.00211.07 |
| ATOM | 2447 | CE2 | PHE | E | 46 | 26.412 | -56.382 | -42.835 | 1.00214.23 |
| ATOM | 2448 | CE1 | PHE | E | 46 | 27.489 | -58.448 | -42.323 | 1.00210.11 |
| ATOM | 2449 | CZ | PHE | E | 46 | 27.280 | -57.121 | -42.055 | 1.00209.86 |
| ATOM | 2450 | N | ARG | E | 47 | 25.358 | -60.007 | -48.651 | 1.00229.04 |
| ATOM | 2451 | CA | ARG | E | 47 | 24.618 | -60.683 | -49.736 | 1.00233.12 |
| ATOM | 2452 | C | ARG | E | 47 | 25.021 | -62.169 | -49.896 | 1.00241.29 |
| ATOM | 2453 | O | ARG | E | 47 | 24.177 | -63.009 | -50.238 | 1.00240.96 |
| ATOM | 2454 | CB | ARG | E | 47 | 24.843 | -59.966 | -51.073 | 1.00237.88 |
| ATOM | 2455 | CG | ARG | E | 47 | 24.190 | -58.604 | -51.172 | 1.00247.35 |
| ATOM | 2456 | CD | ARG | E | 47 | 24.204 | -58.178 | -52.615 | 1.00266.76 |
| ATOM | 2457 | NE | ARG | E | 47 | 25.502 | -57.624 | -53.013 | 1.00274.69 |
| ATOM | 2458 | CZ | ARG | E | 47 | 26.342 | -58.186 | -53.879 | 1.00283.07 |
| ATOM | 2459 | NH1 | ARG | E | 47 | 26.033 | -59.339 | -54.463 | 1.00273.99 |
| ATOM | 2460 | NH2 | ARG | E | 47 | 27.494 | -57.597 | -54.172 | 1.00270.92 |
| ATOM | 2461 | N | ASP | E | 48 | 26.318 | -62.466 | -49.630 | 1.00240.30 |
| ATOM | 2462 | CA | ASP | E | 48 | 27.009 | -63.764 | -49.769 | 1.00240.95 |
| ATOM | 2463 | C | ASP | E | 48 | 27.293 | -64.480 | -48.452 | 1.00238.74 |
| ATOM | 2464 | O | ASP | E | 48 | 28.084 | -65.429 | -48.442 | 1.00238.53 |
| ATOM | 2465 | CB | ASP | E | 48 | 28.372 | -63.537 | -50.510 | 1.00247.94 |
| ATOM | 2466 | CG | ASP | E | 48 | 29.584 | -63.095 | -49.647 | 1.00257.66 |
| ATOM | 2467 | OD1 | ASP | E | 48 | 30.715 | -63.578 | -49.909 | 1.00260.92 |
| ATOM | 2468 | OD2 | ASP | E | 48 | 29.387 | -62.299 | -48.688 | 1.00259.51 |
| ATOM | 2469 | N | LEU | E | 49 | 26.711 | -64.019 | -47.354 | 1.00230.65 |
| ATOM | 2470 | CA | LEU | E | 49 | 27.059 | -64.490 | -46.027 | 1.00226.23 |
| ATOM | 2471 | C | LEU | E | 49 | 25.960 | -65.271 | -45.316 | 1.00227.93 |
| ATOM | 2472 | O | LEU | E | 49 | 24.854 | -64.768 | -45.110 | 1.00225.98 |
| ATOM | 2473 | CB | LEU | E | 49 | 27.507 | -63.260 | -45.238 | 1.00224.43 |
| ATOM | 2474 | CG | LEU | E | 49 | 28.515 | -63.451 | -44.151 | 1.00225.71 |
| ATOM | 2475 | CD1 | LEU | E | 49 | 29.763 | -64.144 | -44.667 | 1.00228.97 |
| ATOM | 2476 | CD2 | LEU | E | 49 | 28.868 | -62.120 | -43.570 | 1.00225.36 |
| ATOM | 2477 | N | SER | E | 50 | 26.282 | -66.511 | -44.942 | 1.00223.93 |
| ATOM | 2478 | CA | SER | E | 50 | 25.344 | -67.430 | -44.321 | 1.00220.52 |
| ATOM | 2479 | C | SER | E | 50 | 26.045 | -68.407 | -43.399 | 1.00224.01 |
| ATOM | 2480 | O | SER | E | 50 | 27.121 | -68.908 | -43.746 | 1.00226.92 |
| ATOM | 2481 | CB | SER | E | 50 | 24.625 | -68.228 | -45.405 | 1.00225.59 |
| ATOM | 2482 | OG | SER | E | 50 | 23.463 | -68.843 | -44.880 | 1.00230.25 |
| ATOM | 2483 | N | PHE | E | 51 | 25.417 | -68.707 | -42.240 | 1.00216.19 |
| ATOM | 2484 | CA | PHE | E | 51 | 25.893 | -69.705 | -41.273 | 1.00213.82 |
| ATOM | 2485 | C | PHE | E | 51 | 24.761 | -70.696 | -40.990 | 1.00215.45 |
| ATOM | 2486 | O | PHE | E | 51 | 24.155 | -70.687 | -39.912 | 1.00211.83 |
| ATOM | 2487 | CB | PHE | E | 51 | 26.445 | -69.047 | -40.011 | 1.00213.12 |
| ATOM | 2488 | CG | PHE | E | 51 | 27.612 | -68.169 | -40.350 | 1.00217.61 |
| ATOM | 2489 | CD1 | PHE | E | 51 | 28.866 | -68.719 | -40.612 | 1.00223.97 |
| ATOM | 2490 | CD2 | PHE | E | 51 | 27.443 | -66.802 | -40.508 | 1.00220.59 |
| ATOM | 2491 | CE1 | PHE | E | 51 | 29.951 | -67.902 | -40.972 | 1.00227.81 |
| ATOM | 2492 | CE2 | PHE | E | 51 | 28.521 | -65.986 | -40.866 | 1.00226.40 |
| ATOM | 2493 | CZ | PHE | E | 51 | 29.771 | -66.539 | -41.094 | 1.00226.94 |
| ATOM | 2494 | N | PRO | E | 52 | 24.468 | -71.563 | -41.988 | 1.00214.14 |
| ATOM | 2495 | CA | PRO | E | 52 | 23.326 | -72.471 | -41.874 | 1.00212.52 |
| ATOM | 2496 | C | PRO | E | 52 | 23.517 | -73.593 | -40.886 | 1.00214.19 |
| ATOM | 2497 | O | PRO | E | 52 | 22.547 | -74.268 | -40.559 | 1.00212.68 |
| ATOM | 2498 | CB | PRO | E | 52 | 23.183 | -73.015 | -43.292 | 1.00218.25 |
| ATOM | 2499 | CG | PRO | E | 52 | 24.571 | -73.016 | -43.819 | 1.00225.74 |
| ATOM | 2500 | CD | PRO | E | 52 | 25.142 | -71.737 | -43.289 | 1.00220.21 |
| ATOM | 2501 | N | LYS | E | 53 | 24.761 | -73.802 | -40.433 | 1.00209.82 |
| ATOM | 2502 | CA | LYS | E | 53 | 25.087 | -74.833 | -39.459 | 1.00206.87 |
| ATOM | 2503 | C | LYS | E | 53 | 24.665 | -74.466 | -38.042 | 1.00204.87 |
| ATOM | 2504 | O | LYS | E | 53 | 24.495 | -75.377 | -37.234 | 1.00203.12 |
| ATOM | 2505 | CB | LYS | E | 53 | 26.589 | -75.157 | -39.480 | 1.00210.62 |
| ATOM | 2506 | CG | LYS | E | 53 | 27.036 | -75.989 | -40.658 | 1.00221.74 |
| ATOM | 2507 | CD | LYS | E | 53 | 28.508 | -76.418 | -40.454 | 1.00231.38 |
| ATOM | 2508 | CE | LYS | E | 53 | 28.989 | -77.503 | -41.403 | 1.00248.74 |
| ATOM | 2509 | NZ | LYS | E | 53 | 30.481 | -77.546 | -41.497 | 1.00260.38 |
| ATOM | 2510 | N | LEU | E | 54 | 24.557 | -73.166 | -37.709 | 1.00197.84 |
| ATOM | 2511 | CA | LEU | E | 54 | 24.226 | -72.750 | -36.348 | 1.00193.01 |
| ATOM | 2512 | C | LEU | E | 54 | 22.796 | -73.053 | -35.943 | 1.00191.32 |
| ATOM | 2513 | O | LEU | E | 54 | 21.854 | -72.573 | -36.573 | 1.00190.05 |
| ATOM | 2514 | CB | LEU | E | 54 | 24.584 | -71.293 | -36.116 | 1.00192.64 |
| ATOM | 2515 | CG | LEU | E | 54 | 24.456 | -70.815 | -34.687 | 1.00194.02 |
| ATOM | 2516 | CD1 | LEU | E | 54 | 25.467 | -71.507 | -33.776 | 1.00194.20 |
| ATOM | 2517 | CD2 | LEU | E | 54 | 24.615 | -69.334 | -34.626 | 1.00194.58 |
| ATOM | 2518 | N | ILE | E | 55 | 22.654 | -73.878 | -34.899 | 1.00184.72 |
| ATOM | 2519 | CA | ILE | E | 55 | 21.369 | -74.357 | -34.414 | 1.00182.14 |
| ATOM | 2520 | C | ILE | E | 55 | 21.089 | -73.777 | -33.071 | 1.00181.29 |
| ATOM | 2521 | O | ILE | E | 55 | 19.931 | -73.504 | -32.736 | 1.00179.11 |
| ATOM | 2522 | CB | ILE | E | 55 | 21.356 | -75.912 | -34.335 | 1.00185.99 |
| ATOM | 2523 | CG1 | ILE | E | 55 | 21.665 | -76.548 | -35.693 | 1.00190.47 |
| ATOM | 2524 | CG2 | ILE | E | 55 | 20.034 | -76.450 | -33.795 | 1.00184.36 |
| ATOM | 2525 | CD1 | ILE | E | 55 | 22.781 | -77.507 | -35.665 | 1.00204.18 |
| ATOM | 2526 | N | MET | E | 56 | 22.134 | -73.590 | -32.287 | 1.00176.70 |
| ATOM | 2527 | CA | MET | E | 56 | 21.905 | -73.194 | -30.930 | 1.00175.02 |
| ATOM | 2528 | C | MET | E | 56 | 23.007 | -72.361 | -30.309 | 1.00178.16 |
| ATOM | 2529 | O | MET | E | 56 | 24.178 | -72.611 | -30.569 | 1.00179.68 |
| ATOM | 2530 | CB | MET | E | 56 | 21.792 | -74.492 | -30.151 | 1.00177.15 |
| ATOM | 2531 | CG | MET | E | 56 | 20.912 | -74.424 | -28.978 | 1.00179.44 |
| ATOM | 2532 | SD | MET | E | 56 | 21.562 | -75.340 | -27.563 | 1.00183.29 |
| ATOM | 2533 | CE | MET | E | 56 | 22.142 | -76.951 | -28.344 | 1.00181.96 |
| ATOM | 2534 | N | ILE | E | 57 | 22.629 | -71.424 | -29.430 | 1.00171.34 |
| ATOM | 2535 | CA | ILE | E | 57 | 23.538 | -70.593 | -28.640 | 1.00169.98 |
| ATOM | 2536 | C | ILE | E | 57 | 23.170 | -70.873 | -27.176 | 1.00174.04 |
| ATOM | 2537 | O | ILE | E | 57 | 21.995 | -70.764 | -26.836 | 1.00175.13 |
| ATOM | 2538 | CB | ILE | E | 57 | 23.366 | -69.112 | -29.013 | 1.00172.38 |
| ATOM | 2539 | CG1 | ILE | E | 57 | 23.758 | -68.891 | -30.477 | 1.00174.79 |
| ATOM | 2540 | CG2 | ILE | E | 57 | 24.157 | -68.217 | -28.058 | 1.00170.82 |
| ATOM | 2541 | CD1 | ILE | E | 57 | 23.390 | -67.534 | -31.079 | 1.00182.82 |
| ATOM | 2542 | N | THR | E | 58 | 24.119 | -71.256 | -26.317 | 1.00168.58 |
| ATOM | 2543 | CA | THR | E | 58 | 23.776 | -71.595 | -24.928 | 1.00166.65 |
| ATOM | 2544 | C | THR | E | 58 | 23.419 | -70.383 | -24.055 | 1.00170.17 |
| ATOM | 2545 | O | THR | E | 58 | 22.624 | -70.497 | -23.118 | 1.00167.37 |
| ATOM | 2546 | CB | THR | E | 58 | 24.911 | -72.407 | -24.329 | 1.00171.53 |
| ATOM | 2547 | OG1 | THR | E | 58 | 25.212 | -73.507 | -25.198 | 1.00168.23 |
| ATOM | 2548 | CG2 | THR | E | 58 | 24.693 | -72.788 | -22.862 | 1.00169.84 |
| ATOM | 2549 | N | ASP | E | 59 | 24.041 | -69.241 | -24.333 | 1.00169.85 |
| ATOM | 2550 | CA | ASP | E | 59 | 23.826 | -68.046 | -23.523 | 1.00170.97 |
| ATOM | 2551 | C | ASP | E | 59 | 22.881 | -67.066 | -24.198 | 1.00176.29 |
| ATOM | 2552 | O | ASP | E | 59 | 21.666 | -67.253 | -24.152 | 1.00177.93 |
| ATOM | 2553 | CB | ASP | E | 59 | 25.169 | -67.380 | -23.124 | 1.00174.96 |
| ATOM | 2554 | CG | ASP | E | 59 | 26.064 | -68.215 | -22.203 | 1.00197.37 |
| ATOM | 2555 | OD1 | ASP | E | 59 | 25.616 | -69.303 | -21.752 | 1.00200.42 |
| ATOM | 2556 | OD2 | ASP | E | 59 | 27.214 | -67.783 | -21.930 | 1.00205.45 |
| ATOM | 2557 | N | TYR | E | 60 | 23.420 | -66.030 | -24.832 | 1.00170.89 |
| ATOM | 2558 | CA | TYR | E | 60 | 22.593 | -65.026 | -25.475 | 1.00169.59 |
| ATOM | 2559 | C | TYR | E | 60 | 23.161 | -64.595 | -26.839 | 1.00173.76 |
| ATOM | 2560 | O | TYR | E | 60 | 24.348 | -64.814 | -27.137 | 1.00175.28 |
| ATOM | 2561 | CB | TYR | E | 60 | 22.408 | -63.824 | -24.527 | 1.00170.01 |
| ATOM | 2562 | CG | TYR | E | 60 | 23.692 | -63.093 | -24.218 | 1.00171.92 |
| ATOM | 2563 | CD1 | TYR | E | 60 | 24.237 | -62.178 | -25.123 | 1.00175.00 |
| ATOM | 2564 | CD2 | TYR | E | 60 | 24.352 | -63.289 | -23.013 | 1.00172.85 |
| ATOM | 2565 | CE1 | TYR | E | 60 | 25.427 | -61.513 | -24.851 | 1.00176.57 |
| ATOM | 2566 | CE2 | TYR | E | 60 | 25.543 | -62.626 | -22.727 | 1.00175.77 |
| ATOM | 2567 | CZ | TYR | E | 60 | 26.073 | -61.738 | -23.647 | 1.00186.72 |
| ATOM | 2568 | OH | TYR | E | 60 | 27.235 | -61.087 | -23.333 | 1.00193.30 |
| ATOM | 2569 | N | LEU | E | 61 | 22.297 | -63.951 | -27.651 | 1.00167.80 |
| ATOM | 2570 | CA | LEU | E | 61 | 22.629 | -63.354 | -28.948 | 1.00166.94 |
| ATOM | 2571 | C | LEU | E | 61 | 22.429 | -61.856 | -28.835 | 1.00165.03 |
| ATOM | 2572 | O | LEU | E | 61 | 21.343 | -61.409 | -28.472 | 1.00162.44 |
| ATOM | 2573 | CB | LEU | E | 61 | 21.721 | -63.895 | -30.057 | 1.00167.08 |
| ATOM | 2574 | CG | LEU | E | 61 | 21.944 | -63.361 | -31.451 | 1.00172.98 |
| ATOM | 2575 | CD1 | LEU | E | 61 | 23.396 | -63.592 | -31.907 | 1.00174.51 |
| ATOM | 2576 | CD2 | LEU | E | 61 | 20.942 | -63.965 | -32.413 | 1.00173.73 |
| ATOM | 2577 | N | LEU | E | 62 | 23.480 | -61.099 | -29.128 | 1.00160.27 |
| ATOM | 2578 | CA | LEU | E | 62 | 23.474 | -59.647 | -29.093 | 1.00160.75 |
| ATOM | 2579 | C | LEU | E | 62 | 24.011 | -59.057 | -30.408 | 1.00169.79 |
| ATOM | 2580 | O | LEU | E | 62 | 25.129 | -59.366 | -30.836 | 1.00170.96 |
| ATOM | 2581 | CB | LEU | E | 62 | 24.265 | -59.150 | -27.878 | 1.00159.45 |
| ATOM | 2582 | CG | LEU | E | 62 | 24.775 | -57.705 | -27.808 | 1.00164.24 |
| ATOM | 2583 | CD1 | LEU | E | 62 | 23.703 | -56.628 | -27.963 | 1.00164.66 |
| ATOM | 2584 | CD2 | LEU | E | 62 | 25.523 | -57.507 | -26.557 | 1.00165.61 |
| ATOM | 2585 | N | LEU | E | 63 | 23.185 | -58.208 | -31.049 | 1.00167.84 |
| ATOM | 2586 | CA | LEU | E | 63 | 23.515 | -57.499 | -32.286 | 1.00169..68 |
| ATOM | 2587 | C | LEU | E | 63 | 23.325 | -56.012 | -31.991 | 1.00175.72 |
| ATOM | 2588 | O | LEU | E | 63 | 22.253 | -55.562 | -31.553 | 1.00174.17 |
| ATOM | 2589 | CB | LEU | E | 63 | 22.631 | -57.919 | -33.472 | 1.00169.93 |
| ATOM | 2590 | CG | LEU | E | 63 | 22.750 | -59.299 | -34.142 | 1.00172.66 |
| ATOM | 2591 | CD1 | LEU | E | 63 | 24.143 | -59.765 | -34.310 | 1.00172.03 |
| ATOM | 2592 | CD2 | LEU | E | 63 | 21.988 | -60.315 | -33.419 | 1.00173.40 |
| ATOM | 2593 | N | PHE | E | 64 | 24.400 | -55.261 | -32.195 | 1.00175.49 |
| ATOM | 2594 | CA | PHE | E | 64 | 24.453 | -53.844 | -31.870 | 1.00177.54 |
| ATOM | 2595 | C | PHE | E | 64 | 25.294 | -53.082 | -32.877 | 1.00179.72 |
| ATOM | 2596 | O | PHE | E | 64 | 26.457 | -53.412 | -33.096 | 1.00180.08 |
| ATOM | 2597 | CB | PHE | E | 64 | 25.024 | -53.674 | -30.434 | 1.00179.15 |
| ATOM | 2598 | CG | PHE | E | 64 | 25.415 | -52.279 | -30.000 | 1.00184.16 |
| ATOM | 2599 | CD1 | PHE | E | 64 | 24.447 | -51.305 | -29.764 | 1.00189.52 |
| ATOM | 2600 | CD2 | PHE | E | 64 | 26.745 | -51.949 | -29.793 | 1.00188.36 |
| ATOM | 2601 | CE1 | PHE | E | 64 | 24.810 | -50.012 | -29.360 | 1.00193.78 |
| ATOM | 2602 | CE2 | PHE | E | 64 | 27.104 | -50.658 | -29.389 | 1.00194.53 |
| ATOM | 2603 | CZ | PHE | E | 64 | 26.134 | -49.700 | -29.176 | 1.00194.39 |
| ATOM | 2604 | N | ARG | E | 65 | 24.726 | -52.062 | -33.489 | 1.00174.49 |
| ATOM | 2605 | CA | ARG | E | 65 | 25.504 | -51.262 | -34.420 | 1.00176.37 |
| ATOM | 2606 | C | ARG | E | 65 | 26.229 | -52.050 | -35.538 | 1.00182.13 |
| ATOM | 2607 | O | ARG | E | 65 | 27.423 | -51.865 | -35.729 | 1.00183.65 |
| ATOM | 2608 | CB | ARG | E | 65 | 26.482 | -50.346 | -33.651 | 1.00172.01 |
| ATOM | 2609 | CG | ARG | E | 65 | 25.829 | -49.285 | -32.784 | 1.00169.53 |
| ATOM | 2610 | CD | ARG | E | 65 | 25.318 | -48.107 | -33.575 | 1.00173.21 |
| ATOM | 2611 | NE | ARG | E | 65 | 24.904 | -47.037 | -32.674 | 1.00177.78 |
| ATOM | 2612 | CZ | ARG | E | 65 | 24.674 | -45.784 | -33.052 | 1.00204.44 |
| ATOM | 2613 | NH1 | ARG | E | 65 | 24.793 | -45.434 | -34.334 | 1.00204.01 |
| ATOM | 2614 | NH2 | ARG | E | 65 | 24.333 | -44.867 | -32.155 | 1.00189.35 |
| ATOM | 2615 | N | VAL | E | 66 | 25.514 | -52.910 | -36.269 | 1.00178.46 |
| ATOM | 2616 | CA | VAL | E | 66 | 26.086 | -53.646 | -37.402 | 1.00179.53 |
| ATOM | 2617 | C | VAL | E | 66 | 25.443 | -53.027 | -38.645 | 1.00190.01 |
| ATOM | 2618 | O | VAL | E | 66 | 24.255 | -53.251 | -38.947 | 1.00186.65 |
| ATOM | 2619 | CB | VAL | E | 66 | 25.922 | -55.189 | -37.324 | 1.00178.89 |
| ATOM | 2620 | CG1 | VAL | E | 66 | 26.557 | -55.875 | -38.528 | 1.00180.23 |
| ATOM | 2621 | CG2 | VAL | E | 66 | 26.499 | -55.744 | -36.024 | 1.00175.16 |
| ATOM | 2622 | N | TYR | E | 67 | 26.227 | -52.176 | -39.318 | 1.00195.82 |
| ATOM | 2623 | CA | TYR | E | 67 | 25.773 | -51.423 | -40.487 | 1.00201.79 |
| ATOM | 2624 | C | TYR | E | 67 | 25.695 | -52.298 | -41.732 | 1.00211.76 |
| ATOM | 2625 | O | TYR | E | 67 | 26.472 | -53.249 | -41.892 | 1.00210.90 |
| ATOM | 2626 | CB | TYR | E | 67 | 26.632 | -50.170 | -40.721 | 1.00206.21 |
| ATOM | 2627 | CG | TYR | E | 67 | 26.453 | -49.088 | -39.675 | 1.00207.15 |
| ATOM | 2628 | CD2 | TYR | E | 67 | 26.306 | -47.761 | -40.042 | 1.00212.69 |
| ATOM | 2629 | CD1 | TYR | E | 67 | 26.504 | -49.385 | -38.312 | 1.00204.56 |
| ATOM | 2630 | CE2 | TYR | E | 67 | 26.197 | -46.752 | -39.085 | 1.00214.55 |
| ATOM | 2631 | CE1 | TYR | E | 67 | 26.364 | -48.390 | -37.346 | 1.00204.99 |
| ATOM | 2632 | CZ | TYR | E | 67 | 26.217 | -47.072 | -37.737 | 1.00219.62 |
| ATOM | 2633 | OH | TYR | E | 67 | 26.121 | -46.075 | -36.795 | 1.00224.11 |
| ATOM | 2634 | N | GLY | E | 68 | 24.711 | -52.005 | -42.573 | 1.00212.80 |
| ATOM | 2635 | CA | GLY | E | 68 | 24.493 | -52.743 | -43.812 | 1.00215.07 |
| ATOM | 2636 | C | GLY | E | 68 | 23.714 | -54.039 | -43.681 | 1.00215.47 |
| ATOM | 2637 | O | GLY | E | 68 | 23.319 | -54.609 | -44.698 | 1.00217.98 |
| ATOM | 2638 | N | LEU | E | 69 | 23.480 | -54.528 | -42.446 | 1.00205.53 |
| ATOM | 2639 | CA | LEU | E | 69 | 22.736 | -55.771 | -42.236 | 1.00201.74 |
| ATOM | 2640 | C | LEU | E | 69 | 21.254 | -55.460 | -42.192 | 1.00205.34 |
| ATOM | 2641 | O | LEU | E | 69 | 20.812 | -54.702 | -41.326 | 1.00203.87 |
| ATOM | 2642 | CB | LEU | E | 69 | 23.221 | -56.482 | -40.964 | 1.00197.47 |
| ATOM | 2643 | CG | LEU | E | 69 | 22.717 | -57.892 | -40.659 | 1.00198.25 |
| ATOM | 2644 | CD1 | LEU | E | 69 | 22.876 | -58.819 | -41.846 | 1.00200.16 |
| ATOM | 2645 | CD2 | LEU | E | 69 | 23.427 | -58.448 | -39.427 | 1.00195.84 |
| ATOM | 2646 | N | GLU | E | 70 | 20.497 | -56.034 | -43.148 | 1.00203.27 |
| ATOM | 2647 | CA | GLU | E | 70 | 19.066 | -55.781 | -43.391 | 1.00203.50 |
| ATOM | 2648 | C | GLU | E | 70 | 18.075 | -56.850 | -42.877 | 1.00202.93 |
| ATOM | 2649 | O | GLU | E | 70 | 16.884 | -56.576 | -42.688 | 1.00201.71 |
| ATOM | 2650 | CB | GLU | E | 70 | 18.859 | -55.528 | -44.894 | 1.00209.35 |
| ATOM | 2651 | CG | GLU | E | 70 | 19.653 | -54.328 | -45.388 | 1.00222.87 |
| ATOM | 2652 | CD | GLU | E | 70 | 19.688 | -54.070 | -46.884 | 1.00248.25 |
| ATOM | 2653 | OE1 | GLU | E | 70 | 19.376 | -52.929 | -47.300 | 1.00235.06 |
| ATOM | 2654 | OE2 | GLU | E | 70 | 20.192 | -54.950 | -47.619 | 1.00247.85 |
| ATOM | 2655 | N | SER | E | 71 | 18.567 | -58.061 | -42.694 | 1.00196.85 |
| ATOM | 2656 | CA | SER | E | 71 | 17.792 | -59.198 | -42.245 | 1.00193.81 |
| ATOM | 2657 | C | SER | E | 71 | 18.796 | -60.204 | -41.732 | 1.00196.83 |
| ATOM | 2658 | O | SER | E | 71 | 19.961 | -60.186 | -42.140 | 1.00198.66 |
| ATOM | 2659 | CB | SER | E | 71 | 17.014 | -59.808 | -43.412 | 1.00198.99 |
| ATOM | 2660 | OG | SER | E | 71 | 16.265 | -60.945 | -43.011 | 1.00205.64 |
| ATOM | 2661 | N | LEU | E | 72 | 18.342 | -61.086 | -40.846 | 1.00189.69 |
| ATOM | 2662 | CA | LEU | E | 72 | 19.152 | -62.176 | -40.325 | 1.00186.70 |
| ATOM | 2663 | | LEU | E | 72 | 18.751 | -63.499 | -40.952 | 1.00191.05 |
| ATOM | 2664 | O | LEU | E | 72 | 19.353 | -64.507 | -40.611 | 1.00189.12 |
| ATOM | 2665 | CB | LEU | E | 72 | 19.008 | -62.286 | -38.803 | 1.00182.94 |
| ATOM | 2666 | CG | LEU | E | 72 | 19.239 | -61.017 | -38.003 | 1.00186.91 |
| ATOM | 2667 | CD1 | LEU | E | 72 | 18.939 | -61.238 | -36.530 | 1.00183.34 |
| ATOM | 2668 | CD2 | LEU | E | 72 | 20.637 | -60.505 | -38.192 | 1.00189.87 |
| ATOM | 2669 | N | LYS | E | 73 | 17.752 | -63.516 | -41.856 | 1.00190.50 |
| ATOM | 2670 | CA | LYS | E | 73 | 17.232 | -64.736 | -42.475 | 1.00191.52 |
| ATOM | 2671 | C | LYS | E | 73 | 18.271 | -65.564 | -43.237 | 1.00198.31 |
| ATOM | 2672 | O | LYS | E | 73 | 18.098 | -66.777 | -43.357 | 1.00197.77 |
| ATOM | 2673 | CB | LYS | E | 73 | 16.017 | -64.426 | -43.366 | 1.00197.26 |
| ATOM | 2674 | CG | LYS | E | 73 | 16.392 | -63.931 | -44.769 | 1.00221.92 |
| ATOM | 2675 | CD | LYS | E | 73 | 15.232 | -63.426 | -45.623 | 1.00234.55 |
| ATOM | 2676 | CE | LYS | E | 73 | 14.275 | -64.451 | -46.195 | 1.00246.97 |
| ATOM | 2677 | NZ | LYS | E | 73 | 12.950 | -63.850 | -46.530 | 1.00257.96 |
| ATOM | 2678 | N | ASP | E | 74 | 19.303 | -64.898 | -43.800 | 1.00197.32 |
| ATOM | 2679 | CA | ASP | E | 74 | 20.367 | -65.557 | -44.546 | 1.00199.31 |
| ATOM | 2680 | C | ASP | E | 74 | 21.532 | -65.804 | -43.624 | 1.00201.40 |
| ATOM | 2681 | O | ASP | E | 74 | 22.249 | -66.782 | -43.802 | 1.00201.86 |
| ATOM | 2682 | CB | ASP | E | 74 | 20.838 | -64.722 | -45.750 | 1.00205.61 |
| ATOM | 2683 | CG | ASP | E | 74 | 19.744 | -64.026 | -46.553 | 1.00219.18 |
| ATOM | 2684 | OD2 | ASP | E | 74 | 19.520 | -64.413 | -47.726 | 1.00229.32 |
| ATOM | 2685 | OD1 | ASP | E | 74 | 19.142 | -63.061 | -46.024 | 1.00218.24 |
| ATOM | 2686 | N | LEU | E | 75 | 21.749 | -64.919 | -42.654 | 1.00195.55 |
| ATOM | 2687 | CA | LEU | E | 75 | 22.846 | -65.113 | -41.729 | 1.00193.35 |
| ATOM | 2688 | C | LEU | E | 75 | 22.624 | -66.337 | -40.804 | 1.00197.60 |
| ATOM | 2689 | O | LEU | E | 75 | 23.466 | -67.243 | -40.790 | 1.00197.65 |
| ATOM | 2690 | CB | LEU | E | 75 | 23.060 | -63.838 | -40.936 | 1.00191.34 |
| ATOM | 2691 | CG | LEU | E | 75 | 24.434 | -63.582 | -40.354 | 1.00194.18 |
| ATOM | 2692 | CD1 | LEU | E | 75 | 25.527 | -63.642 | -41.409 | 1.00196.90 |
| ATOM | 2693 | CD2 | LEU | E | 75 | 24.434 | -62.223 | -39.680 | 1.00196.18 |
| ATOM | 2694 | N | PHE | E | 76 | 21.478 | -66.394 | -40.071 | 1.00192.83 |
| ATOM | 2695 | CA | PHE | E | 76 | 21.190 | -67.490 | -39.108 | 1.00189.03 |
| ATOM | 2696 | C | PHE | E | 76 | 19.865 | -68.177 | -39.398 | 1.00195.86 |
| ATOM | 2697 | O | PHE | E | 76 | 18.947 | -68.154 | -38.561 | 1.00192.12 |
| ATOM | 2698 | CB | PHE | E | 76 | 21.197 | -66.950 | -37.683 | 1.00186.33 |
| ATOM | 2699 | CG | PHE | E | 76 | 22.432 | -66.170 | -37.334 | 1.00186.59 |
| ATOM | 2700 | CD2 | PHE | E | 76 | 22.348 | -64.826 | -37.010 | 1.00187.73 |
| ATOM | 2701 | CD1 | PHE | E | 76 | 23.687 | -66.779 | -37.339 | 1.00188.47 |
| ATOM | 2702 | CE2 | PHE | E | 76 | 23.489 | -64.109 | -36.666 | 1.00190.53 |
| ATOM | 2703 | CE1 | PHE | E | 76 | 24.829 | -66.058 | -37.012 | 1.00189.50 |
| ATOM | 2704 | CZ | PHE | E | 76 | 24.718 | -64.735 | -36.653 | 1.00188.83 |
| ATOM | 2705 | N | PRO | E | 77 | 19.775 | -68.827 | -40.586 | 1.00198.47 |
| ATOM | 2706 | CA | PRO | E | 77 | 18.492 | -69.424 | -41.004 | 1.00199.27 |
| ATOM | 2707 | C | PRO | E | 77 | 17.974 | -70.569 | -40.140 | 1.00201.11 |
| ATOM | 2708 | O | PRO | E | 77 | 16.758 | -70.802 | -40.092 | 1.00200.33 |
| ATOM | 2709 | CB | PRO | E | 77 | 18.775 | -69.889 | -42.440 | 1.00204.88 |
| ATOM | 2710 | CG | PRO | E | 77 | 20.282 | -70.074 | -42.503 | 1.00210.19 |
| ATOM | 2711 | CD | PRO | E | 77 | 20.831 | -69.030 | -41.610 | 1.00203.64 |
| ATOM | 2712 | N | ASN | E | 78 | 18.910 | -71.290 | -39.481 | 1.00195.34 |
| ATOM | 2713 | CA | ASN | E | 78 | 18.638 | -72.493 | -38.706 | 1.00191.99 |
| ATOM | 2714 | C | ASN | E | 78 | 18.737 | -72.361 | -37.204 | 1.00190.82 |
| ATOM | 2715 | O | ASN | E | 78 | 18.559 | -73.362 | -36.505 | 1.00189.91 |
| ATOM | 2716 | CB | ASN | E | 78 | 19.498 | -73.637 | -39.228 | 1.00191.18 |
| ATOM | 2717 | CG | ASN | E | 78 | 19.082 | -74.066 | -40.605 | 1.00207.69 |
| ATOM | 2718 | OD1 | ASN | E | 78 | 17.889 | -74.265 | -40.872 | 1.00196.81 |
| ATOM | 2719 | ND2 | ASN | E | 78 | 20.044 | -74.156 | -41.522 | 1.00201.87 |
| ATOM | 2720 | N | LEU | E | 79 | 19.017 | -71.151 | -36.698 | 1.00183.87 |
| ATOM | 2721 | CA | LEU | E | 79 | 19.107 | -70.919 | -35.259 | 1.00179.34 |
| ATOM | 2722 | C | LEU | E | 79 | 17.728 | -71.173 | -34.668 | 1.00184.27 |
| ATOM | 2723 | O | LEU | E | 79 | 16.773 | -70.459 | -34.992 | 1.00185.10 |
| ATOM | 2724 | CB | LEU | E | 79 | 19.606 | -69.512 | -34.942 | 1.00177.52 |
| ATOM | 2725 | CG | LEU | E | 79 | 19.636 | -69.171 | -33.473 | 1.00175.99 |
| ATOM | 2726 | CD1 | LEU | E | 79 | 20.726 | -69.923 | -32.752 | 1.00175.09 |
| ATOM | 2727 | CD2 | LEU | E | 79 | 19.839 | -67.746 | -33.295 | 1.00173.84 |
| ATOM | 2728 | N | THR | E | 80 | 17.638 | -72.237 | -33.842 | 1.00179.71 |
| ATOM | 2729 | CA | THR | E | 80 | 16.404 | -72.811 | -33.289 | 1.00178.14 |
| ATOM | 2730 | C | THR | E | 80 | 16.193 | -72.529 | -31.816 | 1.00178.57 |
| ATOM | 2731 | O | THR | E | 80 | 15.050 | -72.369 | -31.374 | 1.00177.86 |
| ATOM | 2732 | CB | THR | E | 80 | 16.367 | -74.343 | -33.593 | 1.00187.50 |
| ATOM | 2733 | OG1 | THR | E | 80 | 16.520 | -74.564 | -34.994 | 1.00196.24 |
| ATOM | 2734 | CG2 | THR | E | 80 | 15.058 | -75.005 | -33.188 | 1.00180.91 |
| ATOM | 2735 | N | VAL | E | 81 | 17.281 | -72.533 | -31.046 | 1.00172.91 |
| ATOM | 2736 | CA | VAL | E | 81 | 17.224 | -72.354 | -29.600 | 1.00170.72 |
| ATOM | 2737 | C | VAL | E | 81 | 18.328 | -71.421 | -29.126 | 1.00178.24 |
| ATOM | 2738 | O | VAL | E | 81 | 19.478 | -71.492 | -29.575 | 1.00179.01 |
| ATOM | 2739 | CB | VAL | E | 81 | 17.328 | -73.717 | -28.843 | 1.00171.97 |
| ATOM | 2740 | CG1 | VAL | E | 81 | 17.314 | -73.535 | -27.324 | 1.00169.77 |
| ATOM | 2741 | CG2 | VAL | E | 81 | 16.240 | -74.676 | -29.261 | 1.00171.64 |
| ATOM | 2742 | N | ILE | E | 82 | 17.966 | -70.553 | -28.201 | 1.00175.53 |
| ATOM | 2743 | CA | ILE | E | 82 | 18.896 | -69.721 | -27.458 | 1.00175.30 |
| ATOM | 2744 | C | ILE | E | 82 | 18.564 | -70.134 | -26.017 | 1.00179.61 |
| ATOM | 2745 | O | ILE | E | 82 | 17.454 | -69.879 | -25.560 | 1.00181.02 |
| ATOM | 2746 | CB | ILE | E | 82 | 18.712 | -68.205 | -27.703 | 1.00178.35 |
| ATOM | 2747 | CG1 | ILE | E | 82 | 18.911 | -67.828 | -29.167 | 1.00178.59 |
| ATOM | 2748 | CG2 | ILE | E | 82 | 19.665 | -67.438 | -26.803 | 1.00179.79 |
| ATOM | 2749 | CD1 | ILE | E | 82 | 18.393 | -66.488 | -29.491 | 1.00175.19 |
| ATOM | 2750 | N | ARG | E | 83 | 19.471 | -70.817 | -25.334 | 1.00174.24 |
| ATOM | 2751 | CA | ARG | E | 83 | 19.186 | -71.349 | -24.007 | 1.00173.20 |
| ATOM | 2752 | C | ARG | E | 83 | 19.122 | -70.332 | -22.867 | 1.00176.94 |
| ATOM | 2753 | O | ARG | E | 83 | 18.357 | -70.514 | -21.909 | 1.00175.18 |
| ATOM | 2754 | CB | ARG | E | 83 | 20.118 | -72.517 | -23.688 | 1.00173.12 |
| ATOM | 2755 | CG | ARG | E | 83 | 19.967 | -73.668 | -24.667 | 1.00176.74 |
| ATOM | 2756 | CD | ARG | E | 83 | 20.701 | -74.889 | -24.193 | 1.00184.58 |
| ATOM | 2757 | NE | ARG | E | 83 | 19.973 | -75.623 | -23.159 | 1.00192.85 |
| ATOM | 2758 | CZ | ARG | E | 83 | 19.038 | -76.534 | -23.409 | 1.00202.35 |
| ATOM | 2759 | NH1 | ARG | E | 83 | 18.688 | -76.811 | -24.659 | 1.00180.49 |
| ATOM | 2760 | NH2 | ARG | E | 83 | 18.437 | -77.167 | -22.410 | 1.00190.84 |
| ATOM | 2761 | N | GLY | E | 84 | 19.909 | -69.279 | -22.967 | 1.00174.94 |
| ATOM | 2762 | CA | GLY | E | 84 | 19.871 | -68.250 | -21.946 | 1.00175.76 |
| ATOM | 2763 | C | GLY | E | 84 | 20.458 | -68.675 | -20.620 | 1.00181.73 |
| ATOM | 2764 | O | GLY | E | 84 | 19.985 | -68.214 | -19.582 | 1.00181.69 |
| ATOM | 2765 | N | SER | E | 85 | 21.501 | -69.542 | -20.637 | 1.00180.07 |
| ATOM | 2766 | CA | SER | E | 85 | 22.184 | -70.025 | -19.417 | 1.00180.98 |
| ATOM | 2767 | C | SER | E | 85 | 22.790 | -68.870 | -18.646 | 1.00186.48 |
| ATOM | 2768 | O | SER | E | 85 | 22.747 | -68.870 | -17.414 | 1.00187.61 |
| ATOM | 2769 | CB | SER | E | 85 | 23.254 | -71.054 | -19.754 | 1.00186.06 |
| ATOM | 2770 | OG | SER | E | 85 | 22.641 | -72.211 | -20.291 | 1.00197.31 |
| ATOM | 2771 | N | ARG | E | 86 | 23.396 | -67.918 | -19.382 | 1.00181.98 |
| ATOM | 2772 | CA | ARG | E | 86 | 23.864 | -66.622 | -18.915 | 1.00181.47 |
| ATOM | 2773 | C | ARG | E | 86 | 23.139 | -65.624 | -19.804 | 1.00181.77 |
| ATOM | 2774 | O | ARG | E | 86 | 22.841 | -65.916 | -20.970 | 1.00181.26 |
| ATOM | 2775 | CB | ARG | E | 86 | 25.378 | -66.462 | -19.018 | 1.00183.44 |
| ATOM | 2776 | CG | ARG | E | 86 | 26.120 | -67.009 | -17.823 | 1.00195.04 |
| ATOM | 2777 | CD | ARG | E | 86 | 27.040 | -68.131 | -18.228 | 1.00208.09 |
| ATOM | 2778 | NE | ARG | E | 86 | 26.574 | -69.397 | -17.659 | 1.00217.74 |
| ATOM | 2779 | CZ | ARG | E | 86 | 27.244 | -70.542 | -17.700 | 1.00222.22 |
| ATOM | 2780 | NH1 | ARG | E | 86 | 28.433 | -70.603 | -18.290 | 1.00209.39 |
| ATOM | 2781 | NH2 | ARG | E | 86 | 26.736 | -71.633 | -17.141 | 1.00196.49 |
| ATOM | 2782 | N | LEU | E | 87 | 22.799 | -64.474 | -19.240 | 1.00175.98 |
| ATOM | 2783 | CA | LEU | E | 87 | 22.026 | -63.474 | -19.958 | 1.00175.19 |
| ATOM | 2784 | C | LEU | E | 87 | 22.713 | -62.127 | -19.992 | 1.00178.90 |
| ATOM | 2785 | O | LEU | E | 87 | 23.607 | -61.852 | -19.188 | 1.00179.89 |
| ATOM | 2786 | CB | LEU | E | 87 | 20.656 | -63.311 | -19.288 | 1.00174.99 |
| ATOM | 2787 | CG | LEU | E | 87 | 19.785 | -64.536 | -19.139 | 1.00178.18 |
| ATOM | 2788 | CD1 | LEU | E | 87 | 18.644 | -64.247 | -18.198 | 1.00179.32 |
| ATOM | 2789 | CD2 | LEU | E | 87 | 19.245 | -64.983 | -20.461 | 1.00179.66 |
| ATOM | 2790 | N | PHE | E | 88 | 22.255 | -61.266 | -20.900 | 1.00173.38 |
| ATOM | 2791 | CA | PHE | E | 88 | 22.765 | -59.921 | -21.019 | 1.00173.37 |
| ATOM | 2792 | C | PHE | E | 88 | 21.701 | -59.093 | -20.345 | 1.00178.78 |
| ATOM | 2793 | O | PHE | E | 88 | 20.647 | -58.878 | -20.939 | 1.00178.53 |
| ATOM | 2794 | CB | PHE | E | 88 | 22.936 | -59.554 | -22.499 | 1.00174.47 |
| ATOM | 2795 | CG | PHE | E | 88 | 23.702 | -58.286 | -22.706 | 1.00176.61 |
| ATOM | 2796 | CD1 | PHE | E | 88 | 25.086 | -58.292 | -22.733 | 1.00179.18 |
| ATOM | 2797 | CD2 | PHE | E | 88 | 23.043 | -57.085 | -22.881 | 1.00180.57 |
| ATOM | 2798 | CE1 | PHE | E | 88 | 25.795 | -57.120 | -22.907 | 1.00183.05 |
| ATOM | 2799 | CE2 | PHE | E | 88 | 23.754 | -55.906 | -23.047 | 1.00186.38 |
| ATOM | 2800 | CZ | PHE | E | 88 | 25.128 | -55.931 | -23.071 | 1.00185.07 |
| ATOM | 2801 | N | PHE | E | 89 | 21.931 | -58.707 | -19.078 | 1.00176.57 |
| ATOM | 2802 | CA | PHE | E | 89 | 20.924 | -57.998 | -18.279 | 1.00178.27 |
| ATOM | 2803 | C | PHE | E | 89 | 19.526 | -58.612 | -18.475 | 1.00180.58 |
| ATOM | 2804 | O | PHE | E | 89 | 18.718 | -57.987 | -19.144 | 1.00180.58 |
| ATOM | 2805 | CB | PHE | E | 89 | 20.806 | -56.520 | -18.691 | 1.00183.00 |
| ATOM | 2806 | CG | PHE | E | 89 | 20.092 | -55.627 | -17.699 | 1.00187.96 |
| ATOM | 2807 | CD1 | PHE | E | 89 | 20.635 | -54.403 | -17.331 | 1.00194.81 |
| ATOM | 2808 | CD2 | PHE | E | 89 | 18.878 | -56.017 | -17.124 | 1.00190.94 |
| ATOM | 2809 | CE1 | PHE | E | 89 | 19.992 | -53.586 | -16.401 | 1.00198.82 |
| ATOM | 2810 | CE2 | PHE | E | 89 | 18.229 | -55.202 | -16.199 | 1.00197.17 |
| ATOM | 2811 | CZ | PHE | E | 89 | 18.788 | -53.986 | -15.846 | 1.00198.39 |
| ATOM | 2812 | N | ASN | E | 90 | 19.216 | -59.801 | -17.925 | 1.00175.34 |
| ATOM | 2813 | CA | ASN | E | 90 | 17.913 | -60.481 | -18.103 | 1.00173.68 |
| ATOM | 2814 | C | ASN | E | 90 | 17.481 | -60.831 | -19.540 | 1.00172.91 |
| ATOM | 2815 | O | ASN | E | 90 | 16.408 | -61.416 | -19.688 | 1.00172.46 |
| ATOM | 2816 | CB | ASN | E | 90 | 16.763 | -59.806 | -17.337 | 1.00180.08 |
| ATOM | 2817 | CG | ASN | E | 90 | 17.065 | -59.588 | -15.884 | 1.00232.02 |
| ATOM | 2818 | OD1 | ASN | E | 90 | 18.013 | -58.871 | -15.525 | 1.00231.93 |
| ATOM | 2819 | ND2 | ASN | E | 90 | 16.267 | -60.205 | -15.017 | 1.00231.15 |
| ATOM | 2820 | N | TYR | E | 91 | 18.282 | -60.508 | -20.580 | 1.00165.62 |
| ATOM | 2821 | CA | TYR | E | 91 | 17.895 | -60.795 | -21.958 | 1.00163.02 |
| ATOM | 2822 | C | TYR | E | 91 | 18.701 | -61.872 | -22.614 | 1.00163.85 |
| ATOM | 2823 | O | TYR | E | 91 | 19.923 | -61.922 | -22.471 | 1.00162.85 |
| ATOM | 2824 | CB | TYR | E | 91 | 17.922 | -59.542 | -22.829 | 1.00165.33 |
| ATOM | 2825 | CG | TYR | E | 91 | 17.140 | -58.402 | -22.241 | 1.00168.43 |
| ATOM | 2826 | CD2 | TYR | E | 91 | 17.783 | -57.296 | -21.712 | 1.00171.71 |
| ATOM | 2827 | CD1 | TYR | E | 91 | 15.750 | -58.407 | -22.251 | 1.00170.39 |
| ATOM | 2828 | CE2 | TYR | E | 91 | 17.069 | -56.257 | -21.114 | 1.00175.19 |
| ATOM | 2829 | CE1 | TYR | E | 91 | 15.019 | -57.367 | -21.666 | 1.00173.69 |
| ATOM | 2830 | CZ | TYR | E | 91 | 15.686 | -56.302 | -21.074 | 1.00180.28 |
| ATOM | 2831 | OH | TYR | E | 91 | 14.974 | -55.268 | -20.511 | 1.00178.14 |
| ATOM | 2832 | N | ALA | E | 92 | 18.012 | -62.735 | -23.346 | 1.00160.00 |
| ATOM | 2833 | CA | ALA | E | 92 | 18.656 | -63.791 | -24.115 | 1.00159.46 |
| ATOM | 2834 | C | ALA | E | 92 | 18.820 | -63.345 | -25.582 | 1.00165.58 |
| ATOM | 2835 | O | ALA | E | 92 | 19.590 | -63.960 | -26.326 | 1.00166.58 |
| ATOM | 2836 | CB | ALA | E | 92 | 17.853 | -65.072 | -24.030 | 1.00158.87 |
| ATOM | 2837 | N | LEU | E | 93 | 18.082 | -62.295 | -26.001 | 1.00161.68 |
| ATOM | 2838 | CA | LEU | E | 93 | 18.144 | -61.760 | -27.357 | 1.00162.51 |
| ATOM | 2839 | C | LEU | E | 93 | 18.133 | -60.237 | -27.315 | 1.00170.84 |
| ATOM | 2840 | O | LEU | E | 93 | 17.174 | -59.638 | -26.807 | 1.00174.02 |
| ATOM | 2841 | CB | LEU | E | 93 | 16.978 | -62.282 | -28.218 | 1.00161.93 |
| ATOM | 2842 | CG | LEU | E | 93 | 16.945 | -61.780 | -29.667 | 1.00167.00 |
| ATOM | 2843 | CD1 | LEU | E | 93 | 18.178 | -62.252 | -30.433 | 1.00166.62 |
| ATOM | 2844 | CD2 | LEU | E | 93 | 15.682 | -62.190 | -30.377 | 1.00168.02 |
| ATOM | 2845 | N | VAL | E | 94 | 19.194 | -59.609 | -27.852 | 1.00165.99 |
| ATOM | 2846 | CA | VAL | E | 94 | 19.338 | -58.150 | -27.862 | 1.00166.45 |
| ATOM | 2847 | C | VAL | E | 94 | 19.626 | -57.642 | -29.283 | 1.00171.68 |
| ATOM | 2848 | O | VAL | E | 94 | 20.628 | -58.017 | -29.896 | 1.00171.86 |
| ATOM | 2849 | CB | VAL | E | 94 | 20.401 | -57.679 | -26.832 | 1.00169.12 |
| ATOM | 2850 | CG1 | VAL | E | 94 | 20.533 | -56.164 | -26.788 | 1.00170.73 |
| ATOM | 2851 | CG2 | VAL | E | 94 | 20.088 | -58.204 | -25.447 | 1.00167.28 |
| ATOM | 2852 | N | ILE | E | 95 | 18.727 | -56.791 | -29.796 | 1.00168.62 |
| ATOM | 2853 | CA | ILE | E | 95 | 18.814 | -56.130 | -31.102 | 1.00169.60 |
| ATOM | 2854 | C | ILE | E | 95 | 18.740 | -54.634 | -30.781 | 1.00172.68 |
| ATOM | 2855 | O | ILE | E | 95 | 17.658 | -54.102 | -30.487 | 1.00170.08 |
| ATOM | 2856 | CB | ILE | E | 95 | 17.656 | -56.578 | -32.026 | 1.00172.90 |
| ATOM | 2857 | CG1 | ILE | E | 95 | 17.586 | -58.109 | -32.182 | 1.00170.00 |
| ATOM | 2858 | CG2 | ILE | E | 95 | 17.740 | -55.886 | -33.367 | 1.00177.83 |
| ATOM | 2859 | CD1 | ILE | E | 95 | 18.672 | -58.757 | -33.017 | 1.00171.24 |
| ATOM | 2860 | N | PHE | E | 96 | 19.900 | -53.974 | -30.795 | 1.00171.67 |
| ATOM | 2861 | CA | PHE | E | 96 | 20.011 | -52.592 | -30.371 | 1.00174.46 |
| ATOM | 2862 | C | PHE | E | 96 | 20.809 | -51.725 | -31.324 | 1.00180.83 |
| ATOM | 2863 | O | PHE | E | 96 | 21.924 | -52.066 | -31.693 | 1.00177.59 |
| ATOM | 2864 | CB | PHE | E | 96 | 20.632 | -52.563 | -28.970 | 1.00175.25 |
| ATOM | 2865 | CG | PHE | E | 96 | 20.729 | -51.211 | -28.313 | 1.00179.66 |
| ATOM | 2866 | CD1 | PHE | E | 96 | 19.592 | -50.480 | -28.021 | 1.00183.82 |
| ATOM | 2867 | CD2 | PHE | E | 96 | 21.952 | -50.711 | -27.899 | 1.00184.28 |
| ATOM | 2868 | CE1 | PHE | E | 96 | 19.682 | -49.246 | -27.380 | 1.00187.85 |
| ATOM | 2869 | CE2 | PHE | E | 96 | 22.045 | -49.471 | -27.262 | 1.00190.09 |
| ATOM | 2870 | CZ | PHE | E | 96 | 20.908 | -48.749 | -27.001 | 1.00189.46 |
| ATOM | 2871 | N | GLU | E | 97 | 20.226 | -50.592 | -31.724 | 1.00182.89 |
| ATOM | 2872 | CA | GLU | E | 97 | 20.852 | -49.641 | -32.630 | 1.00186.67 |
| ATOM | 2873 | C | GLU | E | 97 | 21.392 | -50.336 | -33.865 | 1.00194.47 |
| ATOM | 2874 | O | GLU | E | 97 | 22.528 | -50.122 | -34.247 | 1.00194.76 |
| ATOM | 2875 | CB | GLU | E | 97 | 21.888 | -48.772 | -31.901 | 1.00188.95 |
| ATOM | 2876 | CG | GLU | E | 97 | 21.240 | -47.843 | -30.888 | 1.00199.31 |
| ATOM | 2877 | CD | GLU | E | 97 | 22.080 | -46.764 | -30.229 | 1.00219.81 |
| ATOM | 2878 | OE1 | GLU | E | 97 | 21.449 | -45.829 | -29.683 | 1.00197.61 |
| ATOM | 2879 | OE2 | GLU | E | 97 | 23.329 | -46.887 | -30.164 | 1.00218.54 |
| ATOM | 2880 | N | MET | E | 98 | 20.569 | -51.229 | -34.439 | 1.00194.51 |
| ATOM | 2881 | CA | MET | E | 98 | 20.806 | -51.944 | -35.696 | 1.00196.90 |
| ATOM | 2882 | C | MET | E | 98 | 20.191 | -51.103 | -36.823 | 1.00206.23 |
| ATOM | 2883 | O | MET | E | 98 | 19.078 | -51.352 | -37.325 | 1.00206.42 |
| ATOM | 2884 | CB | MET | E | 98 | 20.240 | -53.366 | -35.657 | 1.00196.87 |
| ATOM | 2885 | CG | MET | E | 98 | 21.007 | -54.269 | -34.743 | 1.00198.65 |
| ATOM | 2886 | SD | MET | E | 98 | 22.630 | -54.733 | -35.365 | 1.00205.73 |
| ATOM | 2887 | CE | MET | E | 98 | 22.181 | -55.901 | -36.671 | 1.00202.87 |
| ATOM | 2888 | N | VAL | E | 99 | 20.910 | -50.021 | -37.132 | 1.00206.79 |
| ATOM | 2889 | CA | VAL | E | 99 | 20.515 | -49.079 | -38.142 | 1.00211.29 |
| ATOM | 2890 | C | VAL | E | 99 | 20.782 | -49.876 | -39.370 | 1.00217.99 |
| ATOM | 2891 | O | VAL | E | 99 | 21.882 | -50.409 | -39.537 | 1.00218.07 |
| ATOM | 2892 | CB | VAL | E | 99 | 21.290 | -47.745 | -38.181 | 1.00218.69 |
| ATOM | 2893 | CG1 | VAL | E | 99 | 20.461 | -46.717 | -38.946 | 1.00223.17 |
| ATOM | 2894 | CG2 | VAL | E | 99 | 21.592 | -47.221 | -36.775 | 1.00216.83 |
| ATOM | 2895 | N | HIS | E | 100 | 19.718 | -50.108 | -40.106 | 1.00216.01 |
| ATOM | 2896 | CA | HIS | E | 100 | 19.564 | -50.833 | -41.368 | 1.00217.10 |
| ATOM | 2897 | C | HIS | E | 100 | 18.745 | -52.090 | -41.285 | 1.00216.04 |
| ATOM | 2898 | O | HIS | E | 100 | 18.309 | -52.549 | -42.334 | 1.00217.47 |
| ATOM | 2899 | CB | HIS | E | 100 | 20.852 | -51.066 | -42.197 | 1.00219.75 |
| ATOM | 2900 | CG | HIS | E | 100 | 21.659 | -49.832 | -42.452 | 1.00226.79 |
| ATOM | 2901 | ND1 | HIS | E | 100 | 23.034 | -49.842 | -42.326 | 1.00228.43 |
| ATOM | 2902 | CD2 | HIS | E | 100 | 21.258 | -48.577 | -42.751 | 1.00232.39 |
| ATOM | 2903 | CE1 | HIS | E | 100 | 23.430 | -48.612 | -42.586 | 1.00231.74 |
| ATOM | 2904 | NE2 | HIS | E | 100 | 22.398 | -47.820 | -42.870 | 1.00234.63 |
| ATOM | 2905 | N | LEU | E | 101 | 18.512 | -52.653 | -40.089 | 1.00206.81 |
| ATOM | 2906 | CA | LEU | E | 101 | 17.755 | -53.901 | -40.001 | 1.00203.12 |
| ATOM | 2907 | C | LEU | E | 101 | 16.294 | -53.662 | -40.298 | 1.00208.45 |
| ATOM | 2908 | O | LEU | E | 101 | 15.678 | -52.787 | -39.693 | 1.00207.91 |
| ATOM | 2909 | CB | LEU | E | 101 | 17.974 | -54.601 | -38.656 | 1.00198.42 |
| ATOM | 2910 | CG | LEU | E | 101 | 17.659 | -56.100 | -38.590 | 1.00199.13 |
| ATOM | 2911 | CD1 | LEU | E | 101 | 18.736 | -56.937 | -39.264 | 1.00198.64 |
| ATOM | 2912 | CD2 | LEU | E | 101 | 17.517 | -56.548 | -37.162 | 1.00197.61 |
| ATOM | 2913 | N | LYS | E | 102 | 15.780 | -54.378 | -41.303 | 1.00207.57 |
| ATOM | 2914 | CA | LYS | E | 102 | 14.408 | -54.235 | -41.788 | 1.00210.19 |
| ATOM | 2915 | C | LYS | E | 102 | 13.472 | -55.304 | -41.218 | 1.00214.61 |
| ATOM | 2916 | O | LYS | E | 102 | 12.260 | -55.080 | -41.117 | 1.00214.77 |
| ATOM | 2917 | CB | LYS | E | 102 | 14.394 | -54.215 | -43.328 | 1.00215.40 |
| ATOM | 2918 | CG | LYS | E | 102 | 15.008 | -52.933 | -43.887 | 1.00224.14 |
| ATOM | 2919 | CD | LYS | E | 102 | 15.428 | -52.992 | -45.340 | 1.00233.32 |
| ATOM | 2920 | CE | LYS | E | 102 | 15.833 | -51.609 | -45.824 | 1.00239.02 |
| ATOM | 2921 | NZ | LYS | E | 102 | 16.816 | -51.659 | -46.942 | 1.00243.77 |
| ATOM | 2922 | N | GLU | E | 103 | 14.037 | -56.461 | -40.840 | 1.00210.55 |
| ATOM | 2923 | CA | GLU | E | 103 | 13.296 | -57.581 | -40.254 | 1.00208.24 |
| ATOM | 2924 | C | GLU | E | 103 | 14.282 | -58.425 | -39.483 | 1.00208.90 |
| ATOM | 2925 | O | GLU | E | 103 | 15.473 | -58.372 | -39.778 | 1.00209.79 |
| ATOM | 2926 | CB | GLU | E | 103 | 12.632 | -58.438 | -41.351 | 1.00211.49 |
| ATOM | 2927 | CG | GLU | E | 103 | 13.632 | -58.996 | -42.352 | 1.00228.95 |
| ATOM | 2928 | CD | GLU | E | 103 | 13.073 | -59.618 | -43.616 | 1.00269.43 |
| ATOM | 2929 | OE1 | GLU | E | 103 | 11.993 | -59.192 | -44.092 | 1.00274.44 |
| ATOM | 2930 | OE2 | GLU | E | 103 | 13.766 | -60.502 | -44.168 | 1.00269.87 |
| ATOM | 2931 | N | LEU | E | 104 | 13.803 | -59.221 | -38.517 | 1.00201.53 |
| ATOM | 2932 | CA | LEU | E | 104 | 14.665 | -60.178 | -37.814 | 1.00197.58 |
| ATOM | 2933 | C | LEU | E | 104 | 14.918 | -61.343 | -38.743 | 1.00199.70 |
| ATOM | 2934 | O | LEU | E | 104 | 16.055 | -61.662 | -39.034 | 1.00199.52 |
| ATOM | 2935 | CB | LEU | E | 104 | 14.049 | -60.711 | -36.507 | 1.00194.78 |
| ATOM | 2936 | CG | LEU | E | 104 | 13.860 | -59.717 | -35.371 | 1.00200.02 |
| ATOM | 2937 | CD1 | LEU | E | 104 | 13.429 | -60.413 | -34.097 | 1.00197.75 |
| ATOM | 2938 | CD2 | LEU | E | 104 | 15.113 | -58.948 | -35.103 | 1.00203.95 |
| ATOM | 2939 | N | GLY | E | 105 | 13.853 | -61.991 | -39.172 | 1.00195.60 |
| ATOM | 2940 | CA | GLY | E | 105 | 13.947 | -63.116 | -40.085 | 1.00196.11 |
| ATOM | 2941 | C | GLY | E | 105 | 14.530 | -64.395 | -39.530 | 1.00197.63 |
| ATOM | 2942 | O | GLY | E | 105 | 14.964 | -65.239 | -40.310 | 1.00197.55 |
| ATOM | 2943 | N | LEU | E | 106 | 14.530 | -64.571 | -38.194 | 1.00192.36 |
| ATOM | 2944 | CA | LEU | E | 106 | 15.029 | -65.789 | -37.529 | 1.00189.48 |
| ATOM | 2945 | C | LEU | E | 106 | 13.901 | -66.807 | -37.558 | 1.00193.82 |
| ATOM | 2946 | O | LEU | E | 106 | 13.374 | -67.171 | -36.517 | 1.00190.71 |
| ATOM | 2947 | CB | LEU | E | 106 | 15.482 | -65.468 | -36.080 | 1.00186.35 |
| ATOM | 2948 | CG | LEU | E | 106 | 16.663 | -64.487 | -35.962 | 1.00190.30 |
| ATOM | 2949 | CD1 | LEU | E | 106 | 16.848 | -63.990 | -34.546 | 1.00187.88 |
| ATOM | 2950 | CD2 | LEU | E | 106 | 17.949 | -65.087 | -36.505 | 1.00191.97 |
| ATOM | 2951 | N | TYR | E | 107 | 13.519 | -67.251 | -38.767 | 1.00194.71 |
| ATOM | 2952 | CA | TYR | E | 107 | 12.353 | -68.115 | -39.008 | 1.00195.93 |
| ATOM | 2953 | C | TYR | E | 107 | 12.354 | -69.510 | -38.410 | 1.00196.21 |
| ATOM | 2954 | O | TYR | E | 107 | 11.284 | -70.158 | -38.364 | 1.00195.35 |
| ATOM | 2955 | CB | TYR | E | 107 | 11.925 | -68.124 | -40.478 | 1.00201.82 |
| ATOM | 2956 | CG | TYR | E | 107 | 12.935 | -68.753 | -41.412 | 1.00206.44 |
| ATOM | 2957 | CD1 | TYR | E | 107 | 12.830 | -70.088 | -41.788 | 1.00208.80 |
| ATOM | 2958 | CD2 | TYR | E | 107 | 13.981 | -68.002 | -41.948 | 1.00209.16 |
| ATOM | 2959 | CE1 | TYR | E | 107 | 13.755 | -70.665 | -42.654 | 1.00211.84 |
| ATOM | 2960 | CE2 | TYR | E | 107 | 14.910 | -68.569 | -42.810 | 1.00212.05 |
| ATOM | 2961 | CZ | TYR | E | 107 | 14.785 | -69.895 | -43.174 | 1.00219.83 |
| ATOM | 2962 | OH | TYR | E | 107 | 15.702 | -70.428 | -44.045 | 1.00222.64 |
| ATOM | 2963 | N | ASN | E | 108 | 13.545 | -69.956 | -37.929 | 1.00189.14 |
| ATOM | 2964 | CA | ASN | E | 108 | 13.691 | -71.233 | -37.241 | 1.00185.35 |
| ATOM | 2965 | C | ASN | E | 108 | 13.776 | -71.097 | -35.736 | 1.00182.34 |
| ATOM | 2966 | O | ASN | E | 108 | 13.872 | -72.123 | -35.058 | 1.00179.59 |
| ATOM | 2967 | CB | ASN | E | 108 | 14.844 | -72.058 | -37.790 | 1.00185.83 |
| ATOM | 2968 | CG | ASN | E | 108 | 14.423 | -72.962 | -38.914 | 1.00214.49 |
| ATOM | 2969 | OD1 | ASN | E | 108 | 13.673 | -72.570 | -39.812 | 1.00215.20 |
| ATOM | 2970 | ND2 | ASN | E | 108 | 14.962 | -74.174 | -38.920 | 1.00206.12 |
| ATOM | 2971 | N | LEU | E | 109 | 13.707 | -69.858 | -35.194 | 1.00176.36 |
| ATOM | 2972 | CA | LEU | E | 109 | 13.800 | -69.663 | -33.744 | 1.00172.95 |
| ATOM | 2973 | C | LEU | E | 109 | 12.518 | -70.066 | -33.026 | 1.00175.91 |
| ATOM | 2974 | O | LEU | E | 109 | 11.498 | -69.393 | -33.127 | 1.00174.85 |
| ATOM | 2975 | CB | LEU | E | 109 | 14.248 | -68.250 | -33.390 | 1.00172.61 |
| ATOM | 2976 | CG | LEU | E | 109 | 14.420 | -67.956 | -31.908 | 1.00174.80 |
| ATOM | 2977 | CD1 | LEU | E | 109 | 15.609 | -68.722 | -31.319 | 1.00173.54 |
| ATOM | 2978 | CD2 | LEU | E | 109 | 14.573 | -66.472 | -31.671 | 1.00177.15 |
| ATOM | 2979 | N | MET | E | 110 | 12.585 | -71.181 | -32.307 | 1.00173.32 |
| ATOM | 2980 | CA | MET | E | 110 | 11.441 | -71.800 | -31.662 | 1.00173.48 |
| ATOM | 2981 | C | MET | E | 110 | 11.323 | -71.568 | -30.189 | 1.00177.79 |
| ATOM | 2982 | O | MET | E | 110 | 10.209 | -71.554 | -29.638 | 1.00179.61 |
| ATOM | 2983 | CB | MET | E | 110 | 11.490 | -73.304 | -31.926 | 1.00175.77 |
| ATOM | 2984 | CG | MET | E | 110 | 11.299 | -73.659 | -33.372 | 1.00182.02 |
| ATOM | 2985 | SD | MET | E | 110 | 9.517 | -73.431 | -33.776 | 1.00188.77 |
| ATOM | 2986 | CE | MET | E | 110 | 9.444 | -71.687 | -34.553 | 1.00187.26 |
| ATOM | 2987 | N | ASN | E | 111 | 12.468 | -71.458 | -29.539 | 1.00171.64 |
| ATOM | 2988 | CA | ASN | E | 111 | 12.497 | -71.354 | -28.106 | 1.00169.59 |
| ATOM | 2989 | C | ASN | E | 111 | 13.682 | -70.542 | -27.590 | 1.00170.39 |
| ATOM | 2990 | O | ASN | E | 111 | 14.800 | -70.648 | -28.087 | 1.00168.69 |
| ATOM | 2991 | CB | ASN | E | 111 | 12.502 | -72.767 | -27.514 | 1.00170.07 |
| ATOM | 2992 | CG | ASN | E | 111 | 12.403 | -72.854 | -26.019 | 1.00196.20 |
| ATOM | 2993 | OD1 | ASN | E | 111 | 13.405 | -72.725 | -25.292 | 1.00195.05 |
| ATOM | 2994 | ND2 | ASN | E | 111 | 11.210 | -73.094 | -25.520 | 1.00185.83 |
| ATOM | 2995 | N | ILE | E | 112 | 13.411 | -69.732 | -26.575 | 1.00165.18 |
| ATOM | 2996 | CA | ILE | E | 112 | 14.374 | -68.999 | -25.779 | 1.00162.75 |
| ATOM | 2997 | C | ILE | E | 112 | 14.111 | -69.560 | -24.381 | 1.00169.44 |
| ATOM | 2998 | O | ILE | E | 112 | 13.095 | -69.258 | -23.756 | 1.00170.64 |
| ATOM | 2999 | CB | ILE | E | 112 | 14.225 | -67.489 | -25.912 | 1.00164.33 |
| ATOM | 3000 | CG1 | ILE | E | 112 | 14.631 | -67.061 | -27.330 | 1.00164.68 |
| ATOM | 3001 | CG2 | ILE | E | 112 | 15.059 | -66.813 | -24.850 | 1.00162.10 |
| ATOM | 3002 | CD1 | ILE | E | 112 | 14.253 | -65.647 | -27.743 | 1.00169.35 |
| ATOM | 3003 | N | THR | E | 113 | 14.987 | -70.463 | -23.949 | 1.00166.28 |
| ATOM | 3004 | CA | THR | E | 113 | 14.808 | -71.267 | -22.767 | 1.00166.87 |
| ATOM | 3005 | C | THR | E | 113 | 14.762 | -70.510 | -21.483 | 1.00175.94 |
| ATOM | 3006 | O | THR | E | 113 | 14.000 | -70.862 | -20.582 | 1.00177.47 |
| ATOM | 3007 | CB | THR | E | 113 | 15.784 | -72.410 | -22.767 | 1.00173.29 |
| ATOM | 3008 | OG1 | THR | E | 113 | 15.910 | -72.888 | -24.105 | 1.00167.88 |
| ATOM | 3009 | CG2 | THR | E | 113 | 15.329 | -73.553 | -21.862 | 1.00174.83 |
| ATOM | 3010 | N | ARG | E | 114 | 15.571 | -69.476 | -21.391 | 1.00173.86 |
| ATOM | 3011 | CA | ARG | E | 114 | 15.610 | -68.633 | -20.215 | 1.00174.08 |
| ATOM | 3012 | C | ARG | E | 114 | 15.874 | -67.232 | -20.685 | 1.00178.38 |
| ATOM | 3013 | O | ARG | E | 114 | 16.632 | -67.043 | -21.634 | 1.00178.99 |
| ATOM | 3014 | CB | ARG | E | 114 | 16.714 | -69.129 | -19.255 | 1.00172.10 |
| ATOM | 3015 | CG | ARG | E | 114 | 16.745 | -68.406 | -17.909 | 1.00171.43 |
| ATOM | 3016 | CD | ARG | E | 114 | 17.862 | -68.895 | -17.026 | 1.00163.95 |
| ATOM | 3017 | NE | ARG | E | 114 | 18.217 | -67.895 | -16.024 | 1.00163.02 |
| ATOM | 3018 | CZ | ARG | E | 114 | 19.396 | -67.285 | -15.950 | 1.00181.31 |
| ATOM | 3019 | NH1 | ARG | E | 114 | 20.352 | -67.570 | -16.818 | 1.00168.19 |
| ATOM | 3020 | NH2 | ARG | E | 114 | 19.625 | -66.377 | -15.013 | 1.00178.46 |
| ATOM | 3021 | N | GLY | E | 115 | 15.253 | -66.270 | -20.029 | 1.00174.82 |
| ATOM | 3022 | CA | GLY | E | 115 | 15.478 | -64.871 | -20.348 | 1.00175.81 |
| ATOM | 3023 | C | GLY | E | 115 | 14.423 | -64.268 | -21.239 | 1.00179.59 |
| ATOM | 3024 | O | GLY | E | 115 | 13.497 | -64.971 | -21.650 | 1.00178.34 |
| ATOM | 3025 | N | SER | E | 116 | 14.561 | -62.946 | -21.510 | 1.00176.43 |
| ATOM | 3026 | CA | SER | E | 116 | 13.653 | -62.125 | -22.309 | 1.00176.41 |
| ATOM | 3027 | C | SER | E | 116 | 14.301 | -61.511 | -23.570 | 1.00176.19 |
| ATOM | 3028 | O | SER | E | 116 | 15.483 | -61.712 | -23.837 | 1.00175.59 |
| ATOM | 3029 | CB | SER | E | 116 | 13.027 | -61.039 | -21.441 | 1.00182.46 |
| ATOM | 3030 | OG | SER | E | 116 | 12.419 | -61.584 | -20.284 | 1.00190.11 |
| ATOM | 3031 | N | VAL | E | 117 | 13.509 | -60.769 | -24.338 | 1.00170.20 |
| ATOM | 3032 | CA | VAL | E | 117 | 13.924 | -60.156 | -25.585 | 1.00169.52 |
| ATOM | 3033 | C | VAL | E | 117 | 13.904 | -58.654 | -25.470 | 1.00175.72 |
| ATOM | 3034 | O | VAL | E | 117 | 12.898 | -58.085 | -25.034 | 1.00178.51 |
| ATOM | 3035 | CB | VAL | E | 117 | 12.985 | -60.648 | -26.700 | 1.00173.28 |
| ATOM | 3036 | CG1 | VAL | E | 117 | 13.190 | -59.895 | -27.989 | 1.00174.82 |
| ATOM | 3037 | CG2 | VAL | E | 117 | 13.188 | -62.119 | -26.945 | 1.00171.02 |
| ATOM | 3038 | N | ARG | E | 118 | 15.007 | -58.004 | -25.879 | 1.00171.58 |
| ATOM | 3039 | CA | ARG | E | 118 | 15.095 | -56.541 | -25.947 | 1.00173.70 |
| ATOM | 3040 | C | ARG | E | 118 | 15.444 | -56.131 | -27.347 | 1.00179.38 |
| ATOM | 3041 | O | ARG | E | 118 | 16.557 | -56.386 | -27.784 | 1.00177.40 |
| ATOM | 3042 | CB | ARG | E | 118 | 16.105 | -55.940 | -24.966 | 1.00171.08 |
| ATOM | 3043 | CG | ARG | E | 118 | 16.047 | -54.416 | -24.969 | 1.00169.45 |
| ATOM | 3044 | CD | ARG | E | 118 | 16.029 | -53.908 | -23.576 | 1.00170.29 |
| ATOM | 3045 | NE | ARG | E | 118 | 16.190 | -52.467 | -23.477 | 1.00183.64 |
| ATOM | 3046 | CZ | ARG | E | 118 | 15.942 | -51.778 | -22.366 | 1.00211.82 |
| ATOM | 3047 | NH1 | ARG | E | 118 | 15.452 | -52.388 | -21.291 | 1.00201.35 |
| ATOM | 3048 | NH2 | ARG | E | 118 | 16.116 | -50.460 | -22.341 | 1.00208.19 |
| ATOM | 3049 | N | ILE | E | 119 | 14.486 | -55.534 | -28.055 | 1.00179.89 |
| ATOM | 3050 | CA | ILE | E | 119 | 14.624 | -55.049 | -29.429 | 1.00182.36 |
| ATOM | 3051 | C | ILE | E | 119 | 14.273 | -53.561 | -29.374 | 1.00191.89 |
| ATOM | 3052 | O | ILE | E | 119 | 13.097 | -53.182 | -29.335 | 1.00192.31 |
| ATOM | 3053 | CB | ILE | E | 119 | 13.715 | -55.831 | -30.393 | 1.00184.83 |
| ATOM | 3054 | CG1 | ILE | E | 119 | 14.037 | -57.317 | -30.394 | 1.00180.96 |
| ATOM | 3055 | CG2 | ILE | E | 119 | 13.811 | -55.247 | -31.780 | 1.00189.22 |
| ATOM | 3056 | CD1 | ILE | E | 119 | 13.064 | -58.107 | -31.131 | 1.00183.23 |
| ATOM | 3057 | N | GLU | E | 120 | 15.307 | -52.731 | -29.366 | 1.00192.13 |
| ATOM | 3058 | CA | GLU | E | 120 | 15.200 | -51.309 | -29.108 | 1.00196.32 |
| ATOM | 3059 | C | GLU | E | 120 | 16.005 | -50.410 | -30.053 | 1.00202.59 |
| ATOM | 3060 | O | GLU | E | 120 | 17.167 | -50.704 | -30.326 | 1.00201.57 |
| ATOM | 3061 | CB | GLU | E | 120 | 15.674 | -51.070 | -27.644 | 1.00197.26 |
| ATOM | 3062 | CG | GLU | E | 120 | 15.817 | -49.606 | -27.236 | 1.00209.96 |
| ATOM | 3063 | CD | GLU | E | 120 | 15.394 | -49.215 | -25.838 | 1.00217.56 |
| ATOM | 3064 | OE1 | GLU | E | 120 | 15.834 | -49.895 | -24.887 | 1.00198.71 |
| ATOM | 3065 | OE2 | GLU | E | 120 | 14.694 | -48.184 | -25.694 | 1.00204.24 |
| ATOM | 3066 | N | LYS | E | 121 | 15.413 | -49.274 | -30.478 | 1.00201.45 |
| ATOM | 3067 | CA | LYS | E | 121 | 16.082 | -48.224 | -31.245 | 1.00203.74 |
| ATOM | 3068 | C | LYS | E | 121 | 16.622 | -48.689 | -32.584 | 1.00208.91 |
| ATOM | 3069 | O | LYS | E | 121 | 17.774 | -48.422 | -32.931 | 1.00209.49 |
| ATOM | 3070 | CB | LYS | E | 121 | 17.170 | -47.526 | -30.392 | 1.00205.56 |
| ATOM | 3071 | CG | LYS | E | 121 | 16.656 | -46.597 | -29.279 | 1.00214.58 |
| ATOM | 3072 | CD | LYS | E | 121 | 17.817 | -46.067 | -28.401 | 1.00213.98 |
| ATOM | 3073 | CE | LYS | E | 121 | 17.395 | -45.545 | -27.044 | 1.00210.79 |
| ATOM | 3074 | NZ | LYS | E | 121 | 16.999 | -44.129 | -27.109 | 1.00225.94 |
| ATOM | 3075 | N | ASN | E | 122 | 15.766 | -49.385 | -33.331 | 1.00206.28 |
| ATOM | 3076 | CA | ASN | E | 122 | 16.050 | -49.913 | -34.656 | 1.00206.85 |
| ATOM | 3077 | C | ASN | E | 122 | 15.175 | -49.195 | -35.680 | 1.00216.44 |
| ATOM | 3078 | O | ASN | E | 122 | 14.016 | -49.551 | -35.915 | 1.00215.56 |
| ATOM | 3079 | CB | ASN | E | 122 | 15.876 | -51.416 | -34.689 | 1.00200.30 |
| ATOM | 3080 | CG | ASN | E | 122 | 16.653 | -52.083 | -33.599 | 1.00216.25 |
| ATOM | 3081 | OD1 | ASN | E | 122 | 17.887 | -52.114 | -33.628 | 1.00208.77 |
| ATOM | 3082 | ND2 | ASN | E | 122 | 15.953 | -52.576 | -32.582 | 1.00207.65 |
| ATOM | 3083 | N | ASN | E | 123 | 15.723 | -48.104 | -36.213 | 1.00218.26 |
| ATOM | 3084 | CA | ASN | E | 123 | 15.045 | -47.347 | -37.230 | 1.00223.30 |
| ATOM | 3085 | C | ASN | E | 123 | 15.268 | -48.194 | -38.452 | 1.00230.44 |
| ATOM | 3086 | O | ASN | E | 123 | 16.395 | -48.609 | -38.735 | 1.00229.46 |
| ATOM | 3087 | CB | ASN | E | 123 | 15.600 | -45.923 | -37.437 | 1.00226.78 |
| ATOM | 3088 | CG | ASN | E | 123 | 17.049 | -45.649 | -37.086 | 1.00243.17 |
| ATOM | 3089 | OD1 | ASN | E | 123 | 17.572 | -46.092 | -36.048 | 1.00230.14 |
| ATOM | 3090 | ND2 | ASN | E | 123 | 17.689 | -44.804 | -37.909 | 1.00237.26 |
| ATOM | 3091 | N | GLU | E | 124 | 14.162 | -48.578 | -39.046 | 1.00229.88 |
| ATOM | 3092 | CA | GLU | E | 124 | 13.929 | -49.423 | -40.210 | 1.00230.93 |
| ATQM | 3093 | C | GLU | E | 124 | 13.267 | -50.719 | -39.802 | 1.00232.06 |
| ATOM | 3094 | O | GLU | E | 124 | 12.734 | -51.382 | -40.680 | 1.00232.44 |
| ATOM | 3095 | CB | GLU | E | 124 | 15.172 | -49.726 | -41.103 | 1.00232.89 |
| ATOM | 3096 | CG | GLU | E | 124 | 15.894 | -48.536 | -41.730 | 1.00248.34 |
| ATOM | 3097 | CD | GLU | E | 124 | 15.046 | -47.443 | -42.347 | 1.00271.37 |
| ATOM | 3098 | OE1 | GLU | E | 124 | 14.103 | -47.772 | -43.102 | 1.00269.99 |
| ATOM | 3099 | OE2 | GLU | E | 124 | 15.356 | -46.252 | -42.111 | 1.00266.82 |
| ATOM | 3100 | N | LEU | E | 125 | 13.278 | -51.100 | -38.508 | 1.00225.73 |
| ATOM | 3101 | CA | LEU | E | 125 | 12.798 | -52.429 | -38.116 | 1.00222.62 |
| ATOM | 3102 | C | LEU | E | 125 | 11.310 | -52.701 | -38.116 | 1.00230.85 |
| ATOM | 3103 | O | LEU | E | 125 | 10.568 | -52.117 | -37.321 | 1.00231.16 |
| ATOM | 3104 | CB | LEU | E | 125 | 13.490 | -52.944 | -36.834 | 1.00218.15 |
| ATOM | 3105 | CG | LEU | E | 125 | 13.308 | -54.431 | -36.414 | 1.00217.44 |
| ATOM | 3106 | CD1 | LEU | E | 125 | 13.748 | -55.394 | -37.493 | 1.00216.47 |
| ATOM | 3107 | CD2 | LEU | E | 125 | 14.100 | -54.737 | -35.180 | 1.00215.38 |
| ATOM | 3108 | N | CYS | E | 126 | 10.894 | -53.635 | -38.991 | 1.00230.78 |
| ATOM | 3109 | CA | CYS | E | 126 | 9.515 | -54.098 | -39.128 | 1.00232.99 |
| ATOM | 3110 | C | CYS | E | 126 | 9.443 | -55.597 | -38.907 | 1.00233.48 |
| ATOM | 3111 | O | CYS | E | 126 | 10.410 | -56.174 | -38.407 | 1.00230.94 |
| ATOM | 3112 | CB | CYS | E | 126 | 8.924 | -53.688 | -40.470 | 1.00238.89 |
| ATOM | 3113 | SG | CYS | E | 126 | 8.660 | -51.906 | -40.635 | 1.00248.77 |
| ATOM | 3114 | N | TYR | E | 127 | 8.295 | -56.226 | -39.223 | 1.00229.22 |
| ATOM | 3115 | CA | TYR | E | 127 | 8.067 | -57.653 | -39.001 | 1.00225.30 |
| ATOM | 3116 | C | TYR | E | 127 | 8.226 | -58.030 | -37.523 | 1.00220.39 |
| ATOM | 3117 | O | TYR | E | 127 | 8.716 | -59.101 | -37.192 | 1.00215.83 |
| ATOM | 3118 | CB | TYR | E | 127 | 8.887 | -58.524 | -39.983 | 1.00227.50 |
| ATOM | 3119 | CG | TYR | E | 127 | 8.292 | -58.554 | -41.379 | 1.00235.61 |
| ATOM | 3120 | CD1 | TYR | E | 127 | 8.812 | -57.761 | -42.404 | 1.00242.00 |
| ATOM | 3121 | CD2 | TYR | E | 127 | 7.194 | -59.360 | -41.673 | 1.00237.27 |
| ATOM | 3122 | CE1 | TYR | E | 127 | 8.248 | -57.769 | -43.688 | 1.00247.85 |
| ATOM | 3123 | CE2 | TYR | E | 127 | 6.632 | -59.388 | -42.953 | 1.00242.32 |
| ATOM | 3124 | CZ | TYR | E | 127 | 7.156 | -58.586 | -43.958 | 1.00254.90 |
| ATOM | 3125 | OH | TYR | E | 127 | 6.590 | -58.621 | -45.218 | 1.00259.42 |
| ATOM | 3126 | N | LEU | E | 128 | 7.796 | -57.125 | -36.642 | 1.00215.53 |
| ATOM | 3127 | CA | LEU | E | 128 | 7.813 | -57.324 | -35.201 | 1.00211.50 |
| ATOM | 3128 | C | LEU | E | 128 | 6.422 | -57.612 | -34.680 | 1.00213.42 |
| ATOM | 3129 | O | LEU | E | 128 | 6.263 | -58.510 | -33.852 | 1.00209.21 |
| ATOM | 3130 | CB | LEU | E | 128 | 8.376 | -56.089 | -34.505 | 1.00212.12 |
| ATOM | 3131 | CG | LEU | E | 128 | 9.840 | -55.811 | -34.755 | 1.00214.78 |
| ATOM | 3132 | CD1 | LEU | E | 128 | 10.268 | -54.581 | -34.011 | 1.00215.14 |
| ATOM | 3133 | CD2 | LEU | E | 128 | 10.701 | -57.011 | -34.385 | 1.00213.22 |
| ATOM | 3134 | N | ALA | E | 129 | 5.411 | -56.858 | -35.159 | 1.00212.72 |
| ATOM | 3135 | CA | ALA | E | 129 | 4.017 | -57.062 | -34.750 | 1.00213.13 |
| ATOM | 3136 | C | ALA | E | 129 | 3.487 | -58.426 | -35.225 | 1.00214.06 |
| ATOM | 3137 | O | ALA | E | 129 | 2.502 | -58.934 | -34.678 | 1.00213.33 |
| ATOM | 3138 | CB | ALA | E | 129 | 3.140 | -55.951 | -35.293 | 1.00218.57 |
| ATOM | 3139 | N | THR | E | 130 | 4.159 | -59.014 | -36.232 | 1.00208.37 |
| ATOM | 3140 | CA | THR | E | 130 | 3.803 | -60.280 | -36.853 | 1.00206.56 |
| ATOM | 3141 | C | THR | E | 130 | 4.315 | -61.485 | -36.115 | 1.00210.00 |
| ATOM | 3142 | O | THR | E | 130 | 3.968 | -62.601 | -36.493 | 1.00210.58 |
| ATOM | 3143 | CB | THR | E | 130 | 4.290 | -60.330 | -38.303 | 1.00200.79 |
| ATOM | 3144 | OG1 | THR | E | 130 | 5.685 | -60.054 | -38.370 | 1.00187.94 |
| ATOM | 3145 | CG2 | THR | E | 130 | 3.561 | -59.372 | -39.173 | 1.00204.35 |
| ATOM | 3146 | N | ILE | E | 131 | 5.179 | -61.282 | -35.113 | 1.00204.44 |
| ATOM | 3147 | CA | ILE | E | 131 | 5.778 | -62.364 | -34.340 | 1.00200.07 |
| ATOM | 3148 | C | ILE | E | 131 | 4.979 | -62.594 | -33.065 | 1.00201.36 |
| ATOM | 3149 | O | ILE | E | 131 | 4.775 | -61.659 | -32.283 | 1.00201.51 |
| ATOM | 3150 | CB | ILE | E | 131 | 7.253 | -62.038 | -33.974 | 1.00201.27 |
| ATOM | 3151 | CG1 | ILE | E | 131 | 8.144 | -61.852 | -35.207 | 1.00203.09 |
| ATOM | 3152 | CG2 | ILE | E | 131 | 7.821 | -63.107 | -33.052 | 1.00198.80 |
| ATOM | 3153 | CD1 | ILE | E | 131 | 9.481 | -61.142 | -34.904 | 1.00214.16 |
| ATOM | 3154 | N | ASP | E | 132 | 4.596 | -63.844 | -32.819 | 1.00195.30 |
| ATOM | 3155 | CA | ASP | E | 132 | 3.991 | -64.210 | -31.548 | 1.00193.98 |
| ATOM | 3156 | C | ASP | E | 132 | 5.123 | -64.741 | -30.655 | 1.00192.77 |
| ATOM | 3157 | O | ASP | E | 132 | 5.545 | -65.914 | -30.743 | 1.00190.71 |
| ATOM | 3158 | CB | ASP | E | 132 | 2.888 | -65.262 | -31.699 | 1.00196.95 |
| ATOM | 3159 | CG | ASP | E | 132 | 2.243 | -65.693 | -30.384 | 1.00209.98 |
| ATOM | 3160 | OD2 | ASP | E | 132 | 1.429 | -66.640 | -30.405 | 1.00215.84 |
| ATOM | 3161 | OD1 | ASP | E | 132 | 2.554 | -65.078 | -29.326 | 1.00210.44 |
| ATOM | 3162 | N | TRP | E | 133 | 5.608 | -63.865 | -29.789 | 1.00186.19 |
| ATOM | 3163 | CA | TRP | E | 133 | 6.683 | -64.215 | -28.892 | 1.00182.22 |
| ATOM | 3164 | C | TRP | E | 133 | 6.263 | -65.219 | -27.804 | 1.00184.18 |
| ATOM | 3165 | O | TRP | E | 133 | 7.115 | -65.921 | -27.272 | 1.00181.52 |
| ATOM | 3166 | CB | TRP | E | 133 | 7.278 | -62.940 | -28.300 | 1.00181.16 |
| ATOM | 3167 | CG | TRP | E | 133 | 8.058 | -62.099 | -29.277 | 1.00183.02 |
| ATOM | 3168 | CD1 | TRP | E | 133 | 7.684 | -60.898 | -29.804 | 1.00188.73 |
| ATOM | 3169 | CD2 | TRP | E | 133 | 9.372 | -62.372 | -29.794 | 1.00181.53 |
| ATOM | 3170 | NE1 | TRP | E | 133 | 8.678 | -60.408 | -30.619 | 1.00188.68 |
| ATOM | 3171 | CE2 | TRP | E | 133 | 9.730 | -61.289 | -30.623 | 1.00187.41 |
| ATOM | 3172 | CE3 | TRP | E | 133 | 10.273 | -63.442 | -29.658 | 1.00180.46 |
| ATOM | 3173 | CZ2 | TRP | E | 133 | 10.943 | -61.248 | -31.319 | 1.00186.25 |
| ATOM | 3174 | CZ3 | TRP | E | 133 | 11.485 | -63.388 | -30.330 | 1.00181.59 |
| ATOM | 3175 | CH2 | TRP | E | 133 | 11.810 | -62.300 | -31.147 | 1.00184.10 |
| ATOM | 3176 | N | SER | E | 134 | 4.962 | -65.324 | -27.500 | 1.00183.16 |
| ATOM | 3177 | CA | SER | E | 134 | 4.473 | -66.273 | -26.490 | 1.00182.84 |
| ATOM | 3178 | C | SER | E | 134 | 4.729 | -67.708 | -26.862 | 1.00184.35 |
| ATOM | 3179 | O | SER | E | 134 | 4.722 | -68.554 | -25.981 | 1.00182.42 |
| ATOM | 3180 | CB | SER | E | 134 | 3.000 | -66.049 | -26.146 | 1.00192.31 |
| ATOM | 3181 | OG | SER | E | 134 | 2.111 | -66.313 | -27.220 | 1.00209.64 |
| ATOM | 3182 | N | ARG | E | 135 | 5.000 | -67.986 | -28.144 | 1.00182.10 |
| ATOM | 3183 | CA | ARG | E | 135 | 5.369 | -69.331 | -28.604 | 1.00181.25 |
| ATOM | 3184 | C | ARG | E | 135 | 6.871 | -69.627 | -28.339 | 1.00181.24 |
| ATOM | 3185 | O | ARG | E | 135 | 7.318 | -70.775 | -28.453 | 1.00178.24 |
| ATOM | 3186 | CB | ARG | E | 135 | 5.131 | -69.456 | -30.121 | 1.00184.72 |
| ATOM | 3187 | CG | ARG | E | 135 | 3.672 | -69.329 | -30.572 | 1.00198.02 |
| ATOM | 3188 | CD | ARG | E | 135 | 2.824 | -70.590 | -30.311 | 1.00204.52 |
| ATOM | 3189 | NE | ARG | E | 135 | 1.629 | -70.298 | -29.509 | 1.00209.86 |
| ATOM | 3190 | CZ | ARG | E | 135 | 1.529 | -70.509 | -28.198 | 1.00215.28 |
| ATOM | 3191 | NH1 | ARG | E | 135 | 2.537 | -71.047 | -27.525 | 1.00195.63 |
| ATOM | 3192 | NH2 | ARG | E | 135 | 0.413 | -70.193 | -27.552 | 1.00198.56 |
| ATOM | 3193 | N | ILE | E | 136 | 7.646 | -68.571 | -28.080 | 1.00177.14 |
| ATOM | 3194 | CA | ILE | E | 136 | 9.090 | -68.640 | -27.956 | 1.00174.94 |
| ATOM | 3195 | C | ILE | E | 136 | 9.593 | -68.449 | -26.524 | 1.00178.02 |
| ATOM | 3196 | O | ILE | E | 136 | 10.495 | -69.149 | -26.103 | 1.00177.22 |
| ATOM | 3197 | CB | ILE | E | 136 | 9.682 | -67.638 | -28.968 | 1.00179.27 |
| ATOM | 3198 | CG1 | ILE | E | 136 | 9.227 | -67.984 | -30.413 | 1.00181.96 |
| ATOM | 3199 | CG2 | ILE | E | 136 | 11.198 | -67.562 | -28.858 | 1.00178.03 |
| ATOM | 3200 | CD1 | ILE | E | 136 | 9.481 | -66.904 | -31.460 | 1.00194.32 |
| ATOM | 3201 | N | LEU | E | 137 | 8.993 | -67.544 | -25.767 | 1.00176.20 |
| ATOM | 3202 | CA | LEU | E | 137 | 9.372 | -67.269 | -24.381 | 1.00176.29 |
| ATOM | 3203 | C | LEU | E | 137 | 8.233 | -67.515 | -23.453 | 1.00184.73 |
| ATOM | 3204 | O | LEU | E | 137 | 7.100 | -67.079 | -23.727 | 1.00186.97 |
| ATOM | 3205 | CB | LEU | E | 137 | 9.697 | -65.799 | -24.201 | 1.00177.48 |
| ATOM | 3206 | CG | LEU | E | 137 | 10.738 | -65.204 | -25.046 | 1.00182.54 |
| ATOM | 3207 | CD1 | LEU | E | 137 | 10.142 | -64.149 | -25.904 | 1.00185.04 |
| ATOM | 3208 | CD2 | LEU | E | 137 | 11.747 | -64.563 | -24.203 | 1.00184.43 |
| ATOM | 3209 | N | ASP | E | 138 | 8.531 | -68.126 | -22.311 | 1.00182.69 |
| ATOM | 3210 | CA | ASP | E | 138 | 7.517 | -68.299 | -21.285 | 1.00185.67 |
| ATOM | 3211 | C | ASP | E | 138 | 7.018 | -66.927 | -20.795 | 1.00191.98 |
| ATOM | 3212 | O | ASP | E | 138 | 5.802 | -66.701 | -20.660 | 1.00192.05 |
| ATOM | 3213 | CB | ASP | E | 138 | 8.106 | -69.075 | -20.101 | 1.00188.08 |
| ATOM | 3214 | CG | ASP | E | 138 | 8.138 | -70.577 | -20.276 | 1.00205.56 |
| ATOM | 3215 | OD1 | ASP | E | 138 | 7.342 | -71.106 | -21.105 | 1.00208.43 |
| ATOM | 3216 | OD2 | ASP | E | 138 | 8.934 | -71.239 | -19.564 | 1.00209.81 |
| ATOM | 3217 | N | SER | E | 139 | 7.992 | -66.011 | -20.551 | 1.00190.10 |
| ATOM | 3218 | CA | SER | E | 139 | 7.785 | -64.640 | -20.046 | 1.00192.18 |
| ATOM | 3219 | C | SER | E | 139 | 7.878 | -63.590 | -21.128 | 1.00196.85 |
| ATOM | 3220 | O | SER | E | 139 | 8.983 | -63.245 | -21.527 | 1.00196.63 |
| ATOM | 3221 | CB | SER | E | 139 | 8.809 | -64.306 | -18.962 | 1.00194.45 |
| ATOM | 3222 | OG | SER | E | 139 | 10.122 | -64.604 | -19.421 | 1.00198.39 |
| ATOM | 3223 | N | VAL | E | 140 | 6.738 | -63.078 | -21.599 | 1.00194.15 |
| ATOM | 3224 | CA | VAL | E | 140 | 6.760 | -62.050 | -22.633 | 1.00195.56 |
| ATOM | 3225 | C | VAL | E | 140 | 6.506 | -60.712 | -21.983 | 1.00204.99 |
| ATOM | 3226 | O | VAL | E | 140 | 6.658 | -59.652 | -22.601 | 1.00207.30 |
| ATOM | 3227 | CB | VAL | E | 140 | 5.801 | -62.314 | -23.795 | 1.00199.97 |
| ATOM | 3228 | CG1 | VAL | E | 140 | 6.143 | -63.620 | -24.482 | 1.00197.15 |
| ATOM | 3229 | CG2 | VAL | E | 140 | 4.347 | -62.310 | -23.333 | 1.00202.09 |
| ATOM | 3230 | N | GLU | E | 141 | 6.145 | -60.774 | -20.703 | 1.00202.82 |
| ATOM | 3231 | CA | GLU | E | 141 | 5.842 | -59.645 | -19.826 | 1.00205.72 |
| ATOM | 3232 | C | GLU | E | 141 | 7.107 | -58.777 | -19.641 | 1.00209.35 |
| ATOM | 3233 | O | GLU | E | 141 | 7.021 | -57.554 | -19.529 | 1.00210.50 |
| ATOM | 3234 | CB | GLU | E | 141 | 5.370 | -60.181 | -18.456 | 1.00207.63 |
| ATOM | 3235 | CG | GLU | E | 141 | 4.332 | -61.299 | -18.508 | 1.00219.45 |
| ATOM | 3236 | CD | GLU | E | 141 | 4.877 | -62.718 | -18.508 | 1.00249.96 |
| ATOM | 3237 | OE1 | GLU | E | 141 | 5.854 | -62.986 | -17.770 | 1.00256.95 |
| ATOM | 3238 | OE2 | GLU | E | 141 | 4.298 | -63.576 | -19.214 | 1.00246.57 |
| ATOM | 3239 | N | ASP | E | 142 | 8.285 | -59.440 | -19.650 | 1.00203.77 |
| ATOM | 3240 | CA | ASP | E | 142 | 9.581 | -58.802 | -19.472 | 1.00203.22 |
| ATOM | 3241 | C | ASP | E | 142 | 10.282 | -58.370 | -20.773 | 1.00208.44 |
| ATOM | 3242 | O | ASP | E | 142 | 11.367 | -57.778 | -20.724 | 1.00209.59 |
| ATOM | 3243 | CB | ASP | E | 142 | 10.465 | -59.649 | -18.556 | 1.00202.08 |
| ATOM | 3244 | CG | ASP | E | 142 | 9.856 | -59.921 | -17.188 | 1.00207.25 |
| ATOM | 3245 | OD1 | ASP | E | 142 | 9.952 | -61.080 | -16.715 | 1.00205.58 |
| ATOM | 3246 | OD2 | ASP | E | 142 | 9.267 | -58.982 | -16.599 | 1.00213.47 |
| ATOM | 3247 | N | ASN | E | 143 | 9.646 | -58.625 | -21.929 | 1.00203.51 |
| ATOM | 3248 | CA | ASN | E | 143 | 10.155 | -58.163 | -23.219 | 1.00202.44 |
| ATOM | 3249 | C | ASN | E | 143 | 10.146 | -56.627 | -23.250 | 1.00207.21 |
| ATOM | 3250 | O | ASN | E | 143 | 9.318 | -55.986 | -22.581 | 1.00209.60 |
| ATOM | 3251 | CB | ASN | E | 143 | 9.304 | -58.704 | -24.360 | 1.00199.49 |
| ATOM | 3252 | CG | ASN | E | 143 | 9.610 | -60.109 | -24.679 | 1.00207.69 |
| ATOM | 3253 | OD1 | ASN | E | 143 | 10.479 | -60.731 | -24.069 | 1.00203.81 |
| ATOM | 3254 | ND2 | ASN | E | 143 | 8.893 | -60.639 | -25.636 | 1.00197.37 |
| ATOM | 3255 | N | HIS | E | 144 | 11.073 | -56.051 | -24.017 | 1.00200.69 |
| ATOM | 3256 | CA | HIS | E | 144 | 11.193 | -54.623 | -24.139 | 1.00202.34 |
| ATOM | 3257 | C | | E | 144 | 11.454 | -54.311 | -25.590 | 1.00204.85 |
| ATOM | 3258 | O | HIS | E | 144 | 12.596 | -54.293 | -26.053 | 1.00202.38 |
| ATOM | 3259 | CB | HIS | E | 144 | 12.286 | -54.138 | -23.204 | 1.00203.11 |
| ATOM | 3260 | CG | HIS | E | 144 | 12.306 | -52.668 | -23.028 | 1.00210.39 |
| ATOM | 3261 | ND1 | HIS | E | 144 | 13.182 | -51.887 | -23.730 | 1.00213.62 |
| ATOM | 3262 | CD2 | HIS | E | 144 | 11.591 | -51.889 | -22.189 | 1.00215.28 |
| ATOM | 3263 | CE1 | HIS | E | 144 | 12.967 | -50.648 | -23.316 | 1.00216.66 |
| ATOM | 3264 | NE2 | HIS | E | 144 | 11.996 | -50.600 | -22.409 | 1.00218.15 |
| ATOM | 3265 | N | | E | 145 | 10.360 | -54.149 | -26.330 | 1.00203.74 |
| ATOM | 3266 | CA | ILE | E | 145 | 10.375 | -53.946 | -27.772 | 1.00204.97 |
| ATOM | 3267 | C | ILE | E | 145 | 9.800 | -52.593 | -28.075 | 1.00215.22 |
| ATOM | 3268 | O | ILE | E | 145 | 8.581 | -52.436 | -28.183 | 1.00216.85 |
| ATOM | 3269 | CB | ILE | E | 145 | 9.621 | -55.093 | -28.474 | 1.00206.07 |
| ATOM | 3270 | CG1 | ILE | E | 145 | 10.041 | -56.506 | -27.967 | 1.00202.34 |
| ATOM | 3271 | CG2 | ILE | E | 145 | 9.639 | -54.943 | -29.993 | 1.00207.33 |
| ATOM | 3272 | CD1 | ILE | E | 145 | 11.348 | -56.912 | -27.888 | 1.00208.65 |
| ATOM | 3273 | N | VAL | E | 146 | 10.692 | -51.599 | -28.170 | 1.00214.80 |
| ATOM | 3274 | CA | VAL | E | 146 | 10.333 | -50.191 | -28.330 | 1.00219.35 |
| ATOM | 3275 | C | VAL | E | 146 | 11.261 | -49.414 | -29.266 | 1.00225.24 |
| ATOM | 3276 | O | VAL | E | 146 | 12.398 | -49.818 | -29.521 | 1.00223.68 |
| ATOM | 3277 | CB | VAL | E | 146 | 10.286 | -49.495 | -26.933 | 1.00224.48 |
| ATOM | 3278 | CG1 | VAL | E | 146 | 9.263 | -50.149 | -26.002 | 1.00223.63 |
| ATOM | 3279 | CG2 | VAL | E | 146 | 11.666 | -49.463 | -26.281 | 1.00222.19 |
| ATOM | 3280 | N | LEU | E | 147 | 10.787 | -48.244 | -29.688 | 1.00224.87 |
| ATOM | 3281 | CA | LEU | E | 147 | 11.510 | -47.300 | -30.522 | 1.00226.99 |
| ATOM | 3282 | C | LEU | E | 147 | 12.042 | -47.919 | -31.838 | 1.00231.64 |
| ATOM | 3283 | O | LEU | E | 147 | 13.180 | -47.708 | -32.235 | 1.00230.97 |
| ATOM | 3284 | CB | LEU | E | 147 | 12.572 | -46.522 | -29.697 | 1.00226.88 |
| ATOM | 3285 | CG | LEU | E | 147 | 12.080 | -45.830 | -28.397 | 1.00232.35 |
| ATOM | 3286 | CD1 | LEU | E | 147 | 13.241 | -45.442 | -27.475 | 1.00231.82 |
| ATOM | 3287 | CD2 | LEU | E | 147 | 11.183 | -44.630 | -28.692 | 1.00238.45 |
| ATOM | 3288 | N | ASN | E | 148 | 11.183 | -48.671 | -32.522 | 1.00229.80 |
| ATOM | 3289 | CA | ASN | E | 148 | 11.489 | -49.258 | -33.827 | 1.00230.43 |
| ATOM | 3290 | C | ASN | E | 148 | 10.563 | -48.648 | -34.895 | 1.00242.79 |
| ATOM | 3291 | O | ASN | E | 148 | 9.583 | -47.982 | -34.535 | 1.00246.23 |
| ATOM | 3292 | CB | ASN | E | 148 | 11.333 | -50.777 | -33.764 | 1.00225.00 |
| ATOM | 3293 | CG | ASN | E | 148 | 12.157 | -51.412 | -32.663 | 1.00235.07 |
| ATOM | 3294 | OD1 | ASN | E | 148 | 13.387 | -51.329 | -32.649 | 1.00224.86 |
| ATOM | 3295 | ND2 | ASN | E | 148 | 11.498 | -52.065 | -31.713 | 1.00224.04 |
| ATOM | 3296 | N | LYS | E | 149 | 10.849 | -48.871 | -36.208 | 1.00241.61 |
| ATOM | 3297 | CA | LYS | E | 149 | 9.963 | -48.366 | -37.276 | 1.00245.96 |
| ATOM | 3298 | C | LYS | E | 149 | 8.519 | -48.815 | -37.010 | 1.00253.72 |
| ATOM | 3299 | O | LYS | E | 149 | 7.590 | -48.055 | -37.255 | 1.00258.37 |
| ATOM | 3300 | CB | LYS | E | 149 | 10.409 | -48.819 | -38.674 | 1.00247.39 |
| ATOM | 3301 | CG | LYS | E | 149 | 9.583 | -48.171 | -39.788 | 1.00250.74 |
| ATOM | 3302 | CD | LYS | E | 149 | 9.971 | -48.599 | -41.186 | 1.00256.23 |
| ATOM | 3303 | CE | LYS | E | 149 | 8.844 | -48.268 | -42.142 | 1.00264.82 |
| ATOM | 3304 | NZ | LYS | E | 149 | 8.847 | -49.150 | -43.342 | 1.00271.66 |
| ATOM | 3305 | N | ASP | E | 150 | 8.361 | -50.015 | -36.444 | 1.00247.57 |
| ATOM | 3306 | CA | ASP | E | 150 | 7.113 | -50.668 | -36.079 | 1.00247.35 |
| ATOM | 3307 | C | ASP | E | 150 | 6.169 | -49.838 | -35.198 | 1.00255.74 |
| ATOM | 3308 | O | ASP | E | 150 | 4.948 | -49.999 | -35.294 | 1.00256.65 |
| ATOM | 3309 | CB | ASP | E | 150 | 7.471 | -52.002 | -35.426 | 1.00243.96 |
| ATOM | 3310 | CG | ASP | E | 150 | 6.318 | -52.712 | -34.778 | 1.00251.63 |
| ATOM | 3311 | OD2 | ASP | E | 150 | 6.347 | -52.879 | -33.545 | 1.00254.53 |
| ATOM | 3312 | OD1 | ASP | E | 150 | 5.393 | -53.118 | -35.504 | 1.00253.62 |
| ATOM | 3313 | N | ASP | E | 151 | 6.733 | -48.971 | -34.345 | 1.00254.50 |
| ATOM | 3314 | CA | ASP | E | 151 | 5.981 | -48.099 | -33.442 | 1.00257.73 |
| ATOM | 3315 | C | ASP | E | 151 | 5.222 | -47.015 | -34.182 | 1.00263.97 |
| ATOM | 3316 | O | ASP | E | 151 | 4.195 | -46.552 | -33.685 | 1.00264.91 |
| ATOM | 3317 | CB | ASP | E | 151 | 6.942 | -47.343 | -32.515 | 1.00259.50 |
| ATOM | 3318 | CG | ASP | E | 151 | 7.599 | -48.100 | -31.392 | 1.00269.11 |
| ATOM | 3319 | OD1 | ASP | E | 151 | 7.741 | -49.338 | -31.506 | 1.00265.36 |
| ATOM | 3320 | OD2 | ASP | E | 151 | 8.056 | -47.445 | -30.433 | 1.00276.45 |
| ATOM | 3321 | N | ASN | E | 152 | 5.795 | -46.535 | -35.303 | 1.00261.70 |
| ATOM | 3322 | CA | ASN | E | 152 | 5.346 | -45.396 | -36.092 | 1.00262.46 |
| ATOM | 3323 | C | ASN | E | 152 | 4.504 | -45.787 | -37.319 | 1.00266.52 |
| ATOM | 3324 | O | ASN | E | 152 | 4.885 | -45.520 | -38.465 | 1.00266.28 |
| ATOM | 3325 | CB | ASN | E | 152 | 6.554 | -44.496 | -36.412 | 1.00259.59 |
| ATOM | 3326 | CG | ASN | E | 152 | 7.801 | -44.838 | -35.586 | 1.00260.81 |
| ATOM | 3327 | OD1 | ASN | E | 152 | 7.820 | -44.784 | -34.343 | 1.00247.20 |
| ATOM | 3328 | ND2 | ASN | E | 152 | 8.832 | -45.319 | -36.247 | 1.00254.80 |
| ATOM | 3329 | N | GLU | E | 153 | 3.346 | -46.450 | -37.021 | 1.00265.11 |
| ATOM | 3330 | CA | GLU | E | 153 | 2.176 | -46.966 | -37.775 | 1.00265.17 |
| ATOM | 3331 | C | GLU | E | 153 | 2.306 | -47.591 | -39.172 | 1.00267.34 |
| ATOM | 3332 | O | GLU | E | 153 | 1.491 | -48.460 | -39.515 | 1.00267.13 |
| ATOM | 3333 | CB | GLU | E | 153 | 0.982 | -45.975 | -37.720 | 1.00267.16 |
| ATOM | 3334 | CG | GLU | E | 153 | -0.379 | -46.652 | -37.632 | 1.00272.02 |
| ATOM | 3335 | CD | GLU | E | 153 | -1.581 | -45.758 | -37.856 | 1.00279.42 |
| ATOM | 3336 | OE1 | GLU | E | 153 | -1.604 | -45.027 | -38.873 | 1.00276.01 |
| ATOM | 3337 | OE2 | GLU | E | 153 | -2.526 | -45.831 | -37.039 | 1.00268.00 |
| ATOM | 3338 | N | GLU | E | 154 | 3.296 | -47.152 | -39.974 | 1.00263.84 |
| ATOM | 3339 | CA | GLU | E | 154 | 3.413 | -47.595 | -41.351 | 1.00262.91 |
| ATOM | 3340 | C | GLU | E | 154 | 4.496 | -48.613 | -41.739 | 1.00261.68 |
| ATOM | 3341 | O | GLU | E | 154 | 5.308 | -48.390 | -42.647 | 1.00261.48 |
| ATOM | 3342 | CB | GLU | E | 154 | 3.216 | -46.432 | -42.333 | 1.00265.14 |
| ATOM | 3343 | CG | GLU | E | 154 | 1.799 | -45.881 | -42.248 | 1.00271.87 |
| ATOM | 3344 | CD | GLU | E | 154 | 1.464 | -44.804 | -43.256 | 1.00288.24 |
| ATOM | 3345 | OE1 | GLU | E | 154 | 1.140 | -45.150 | -44.416 | 1.00288.58 |
| ATOM | 3346 | OE2 | GLU | E | 154 | 1.480 | -43.611 | -42.876 | 1.00286.20 |
| ATOM | 3347 | N | CYS | E | 155 | 4.422 | -49.776 | -41.067 | 1.00255.80 |
| ATOM | 3348 | CA | CYS | E | 155 | 5.201 | -50.946 | -41.415 | 1.00254.35 |
| ATOM | 3349 | C | CYS | E | 155 | 4.222 | -51.677 | -42.303 | 1.00255.43 |
| ATOM | 3350 | O | CYS | E | 155 | 3.222 | -52.225 | -41.812 | 1.00253.60 |
| ATOM | 3351 | CB | CYS | E | 155 | 5.531 | -51.812 | -40.205 | 1.00249.22 |
| ATOM | 3352 | SG | CYS | E | 155 | 7.107 | -51.440 | -39.393 | 1.00249.63 |
| ATOM | 3353 | N | GLY | E | 156 | 4.502 | -51.705 | -43.589 | 1.00252.77 |
| ATOM | 3354 | CA | GLY | E | 156 | 3.662 | -52.429 | -44.527 | 1.00252.49 |
| ATOM | 3355 | C | GLY | E | 156 | 3.930 | -53.921 | -44.471 | 1.00253.45 |
| ATOM | 3356 | O | GLY | E | 156 | 4.269 | -54.524 | -45.494 | 1.00254.21 |
| ATOM | 3357 | N | ASP | E | 157 | 3.801 | -54.532 | -43.274 | 1.00246.37 |
| ATOM | 3358 | CA | ASP | E | 157 | 4.050 | -55.960 | -43.078 | 1.00241.60 |
| ATOM | 3359 | C | ASP | E | 157 | 3.095 | -56.797 | -43.886 | 1.00243.77 |
| ATOM | 3360 | O | ASP | E | 157 | 1.891 | -56.557 | -43.852 | 1.00244.57 |
| ATOM | 3361 | CB | ASP | E | 157 | 3.929 | -56.332 | -41.605 | 1.00240.19 |
| ATOM | 3362 | CG | ASP | E | 157 | 5.045 | -55.831 | -40.694 | 1.00255.63 |
| ATOM | 3363 | OD2 | ASP | E | 157 | 4.820 | -55.758 | -39.466 | 1.00260.85 |
| ATOM | 3364 | OD1 | ASP | E | 157 | 6.161 | -55.551 | -41.202 | 1.00258.38 |
| ATOM | 3365 | N | ILE | E | 158 | 3.640 | -57.763 | -44.638 | 1.00238.32 |
| ATOM | 3366 | CA | ILE | E | 158 | 2.875 | -58.643 | -45.528 | 1.00239.35 |
| ATOM | 3367 | C | ILE | E | 158 | 3.331 | -60.080 | -45.381 | 1.00239.04 |
| ATOM | 3368 | O | ILE | E | 158 | 4.521 | -60.348 | -45.542 | 1.00237.44 |
| ATOM | 3369 | CB | ILE | E | 158 | 2.915 | -58.181 | -47.021 | 1.00247.07 |
| ATOM | 3370 | CG1 | ILE | E | 158 | 4.348 | -58.033 | -47.609 | 1.00246.79 |
| ATOM | 3371 | CG2 | ILE | E | 158 | 2.016 | -56.981 | -47.311 | 1.00249.81 |
| ATOM | 3372 | CD1 | ILE | E | 158 | 4.634 | -58.967 | -48.780 | 1.00249.78 |
| ATOM | 3373 | N | CYS | E | 159 | 2.396 | -61.010 | -45.093 | 1.00233.45 |
| ATOM | 3374 | CA | CYS | E | 159 | 2.730 | -62.421 | -44.837 | 1.00247.83 |
| ATOM | 3375 | C | CYS | E | 159 | 2.532 | -63.412 | -46.013 | 1.00212.30 |
| ATOM | 3376 | O | CYS | E | 159 | 3.112 | -64.515 | -46.030 | 1.00153.50 |
| ATOM | 3377 | CB | CYS | E | 159 | 2.054 | -62.906 | -43.555 | 1.00245.20 |
| ATOM | 3378 | SG | CYS | E | 159 | 2.641 | -62.090 | -42.029 | 1.00245.62 |
| ATOM | 3379 | N | ASN | E | 168 | -2.106 | -67.815 | -45.399 | 1.00237.72 |
| ATOM | 3380 | CA | ASN | E | 168 | -1.440 | -69.088 | -45.099 | 1.00234.29 |
| ATOM | 3381 | C | ASN | E | 168 | -0.777 | -69.160 | -43.717 | 1.00232.00 |
| ATOM | 3382 | O | ASN | E | 168 | -0.555 | -70.265 | -43.213 | 1.00228.54 |
| ATOM | 3383 | CB | ASN | E | 168 | -0.419 | -69.450 | -46.186 | 1.00236.07 |
| ATOM | 3384 | CG | ASN | E | 168 | -0.946 | -70.368 | -47.277 | 1.00253.02 |
| ATOM | 3385 | OD1 | ASN | E | 168 | -1.740 | -71.292 | -47.042 | 1.00239.72 |
| ATOM | 3386 | ND2 | ASN | E | 168 | -0.460 | -70.172 | -48.493 | 1.00249.53 |
| ATOM | 3387 | N | CYS | E | 169 | -0.438 | -68.006 | -43.121 | 1.00226.85 |
| ATOM | 3388 | CA | CYS | E | 169 | 0.208 | -67.995 | -41.815 | 1.00222.25 |
| ATOM | 3389 | C | CYS | E | 169 | -0.788 | -68.180 | -40.666 | 1.00220.36 |
| ATOM | 3390 | O | CYS | E | 169 | -1.925 | -67.718 | -40.754 | 1.00221.65 |
| ATOM | 3391 | CB | CYS | E | 169 | 1.076 | -66.755 | -41.623 | 1.00223.06 |
| ATOM | 3392 | SG | CYS | E | 169 | 2.448 | -66.587 | -42.805 | 1.00228.60 |
| ATOM | 3393 | N | PRO | E | 170 | -0.367 | -68.864 | -39.583 | 1.00211.04 |
| ATOM | 3394 | CA | PRO | E | 170 | -1.286 | -69.097 | -38.465 | 1.00209.17 |
| ATOM | 3395 | C | PRO | E | 170 | -1.502 | -67.857 | -37.609 | 1.00209.64 |
| ATOM | 3396 | O | PRO | E | 170 | -0.542 | -67.237 | -37.156 | 1.00207.87 |
| ATOM | 3397 | CB | PRO | E | 170 | -0.618 | -70.248 | -37.704 | 1.00207.71 |
| ATOM | 3398 | CG | PRO | E | 170 | 0.831 | -70.124 | -38.013 | 1.00210.62 |
| ATOM | 3399 | CD | PRO | E | 170 | 0.952 | -69.506 | -39.359 | 1.00209.05 |
| ATOM | 3400 | N | ALA | E | 171 | -2.745 | -67.455 | -37.440 | 1.00205.68 |
| ATOM | 3401 | CA | ALA | E | 171 | -2.960 | -66.311 | -36.584 | 1.00204.94 |
| ATOM | 3402 | C | ALA | E | 171 | -3.107 | -66.796 | -35.130 | 1.00205.85 |
| ATOM | 3403 | O | ALA | E | 171 | -3.464 | -67.952 | -34.885 | 1.00204.01 |
| ATOM | 3404 | CB | ALA | E | 171 | -4.178 | -65.523 | -37.027 | 1.00209.84 |
| ATOM | 3405 | N | THR | E | 172 | -2.745 | -65.920 | -34.172 | 1.00201.52 |
| ATOM | 3406 | CA | THR | E | 172 | -2.858 | -66.098 | -32.712 | 1.00199.77 |
| ATOM | 3407 | C | THR | E | 172 | -3.434 | -64.821 | -32.139 | 1.00210.73 |
| ATOM | 3408 | O | THR | E | 172 | -3.277 | -63.777 | -32.766 | 1.00212.86 |
| ATOM | 3409 | CB | THR | E | 172 | -1.514 | -66.265 | -32.021 | 1.00194.04 |
| ATOM | 3410 | OG1 | THR | E | 172 | -0.722 | -65.075 | -32.155 | 1.00187.31 |
| ATOM | 3411 | CG2 | THR | E | 172 | -0.782 | -67.554 | -32.396 | 1.00187.40 |
| ATOM | 3412 | N | VAL | E | 173 | -4.051 | -64.872 | -30.944 | 1.00211.17 |
| ATOM | 3413 | CA | VAL | E | 173 | -4.671 | -63.688 | -30.318 | 1.00215.85 |
| ATOM | 3414 | C | VAL | E | 173 | -3.775 | -63.084 | -29.253 | 1.00222.92 |
| ATOM | 3415 | O | VAL | E | 173 | -3.596 | -63.707 | -28.207 | 1.00221.69 |
| ATOM | 3416 | CB | VAL | E | 173 | -6.110 | -63.989 | -29.781 | 1.00222.43 |
| ATOM | 3417 | CG1 | VAL | E | 173 | -6.152 | -65.242 | -28.908 | 1.00219.96 |
| ATOM | 3418 | CG2 | VAL | E | 173 | -6.729 | -62.787 | -29.057 | 1.00225.44 |
| ATOM | 3419 | N | ILE | E | 174 | -3.209 | -61.883 | -29.475 | 1.00222.60 |
| ATOM | 3420 | CA | ILE | E | 174 | -2.369 | -61.329 | -28.408 | 1.00221.73 |
| ATOM | 3421 | C | ILE | E | 174 | -3.063 | -60.263 | -27.577 | 1.00229.49 |
| ATOM | 3422 | O | ILE | E | 174 | -3.118 | -60.406 | -26.354 | 1.00228.87 |
| ATOM | 3423 | CB | ILE | E | 174 | -0.869 | -61.130 | -28.759 | 1.00222.48 |
| ATOM | 3424 | CG1 | ILE | E | 174 | -0.182 | -62.518 | -28.945 | 1.00218.87 |
| ATOM | 3425 | CG2 | ILE | E | 174 | -0.105 | -60.279 | -27.732 | 1.00223.62 |
| ATOM | 3426 | CD1 | ILE | E | 174 | -0.163 | -63.538 | -27.726 | 1.00216.93 |
| ATOM | 3427 | N | ASN | E | 175 | -3.666 | -59.269 | -28.226 | 1.00229.53 |
| ATOM | 3428 | CA | ASN | E | 175 | -4.479 | -58.269 | -27.544 | 1.00233.02 |
| ATOM | 3429 | C | ASN | E | 175 | -5.864 | -58.935 | -27.384 | 1.00237.88 |
| ATOM | 3430 | O | ASN | E | 175 | -6.012 | -59.861 | -26.593 | 1.00234.63 |
| ATOM | 3431 | CB | ASN | E | 175 | -4.503 | -56.968 | -28.370 | 1.00237.61 |
| ATOM | 3432 | CG | ASN | E | 175 | -3.158 | -56.266 | -28.492 | 1.00255.94 |
| ATOM | 3433 | OD1 | ASN | E | 175 | -2.084 | -56.851 | -28.290 | 1.00245.41 |
| ATOM | 3434 | ND2 | ASN | E | 175 | -3.189 | -54.971 | -28.818 | 1.00250.90 |
| ATOM | 3435 | N | GLY | E | 176 | -6.838 | -58.508 | -28.163 | 1.00239.62 |
| ATOM | 3436 | CA | GLY | E | 176 | -8.157 | -59.127 | -28.210 | 1.00242.75 |
| ATOM | 3437 | C | GLY | E | 176 | -8.441 | -59.672 | -29.599 | 1.00248.34 |
| ATOM | 3438 | O | GLY | E | 176 | -9.464 | -60.327 | -29.833 | 1.00249.50 |
| ATOM | 3439 | N | GLN | E | 177 | -7.487 | -59.446 | -30.519 | 1.00243.43 |
| ATOM | 3440 | CA | GLN | E | 177 | -7.594 | -59.797 | -31.929 | 1.00243.13 |
| ATOM | 3441 | C | GLN | E | 177 | -6.550 | -60.783 | -32.427 | 1.00240.81 |
| ATOM | 3442 | O | GLN | E | 177 | -5.415 | -60.844 | -31.926 | 1.00235.92 |
| ATOM | 3443 | CB | GLN | E | 177 | -7.626 | -58.520 | -32.797 | 1.00247.86 |
| ATOM | 3444 | CG | GLN | E | 177 | -6.340 | -57.704 | -32.740 | 1.00248.66 |
| ATOM | 3445 | CD | GLN | E | 177 | -6.444 | -56.405 | -33.468 | 1.00251.12 |
| ATOM | 3446 | OE1 | GLN | E | 177 | -7.399 | -55.632 | -33.299 | 1.00249.02 |
| ATOM | 3447 | NE2 | GLN | E | 177 | -5.410 | -56.099 | -34.225 | 1.00238.39 |
| ATOM | 3448 | N | PHE | E | 178 | -6.953 | -61.542 | -33.447 | 1.00236.91 |
| ATOM | 3449 | CA | PHE | E | 178 | -6.072 | -62.497 | -34.086 | 1.00232.37 |
| ATOM | 3450 | C | PHE | E | 178 | -5.169 | -61.774 | -35.058 | 1.00235.27 |
| ATOM | 3451 | O | PHE | E | 178 | -5.616 | -60.901 | -35.803 | 1.00239.30 |
| ATOM | 3452 | CB | PHE | E | 178 | -6.845 | -63.667 | -34.711 | 1.00234.60 |
| ATOM | 3453 | CG | PHE | E | 178 | -7.296 | -64.675 | -33.671 | 1.00234.24 |
| ATOM | 3454 | CD1 | PHE | E | 178 | -8.545 | -64.572 | -33.074 | 1.00239.70 |
| ATOM | 3455 | CD2 | PHE | E | 178 | -6.461 | -65.715 | -33.273 | 1.00232.54 |
| ATOM | 3456 | CE1 | PHE | E | 178 | -8.956 | -65.499 | -32.108 | 1.00239.20 |
| ATOM | 3457 | CE2 | PHE | E | 178 | -6.872 | -66.637 | -32.300 | 1.00233.78 |
| ATOM | 3458 | CZ | PHE | E | 178 | -8.118 | -66.524 | -31.727 | 1.00234.26 |
| ATOM | 3459 | N | VAL | E | 179 | -3.879 | -62.088 | -34.977 | 1.00226.07 |
| ATOM | 3460 | CA | VAL | E | 179 | -2.829 | -61.487 | -35.780 | 1.00224.74 |
| ATOM | 3461 | C | VAL | E | 179 | -2.061 | -62.643 | -36.440 | 1.00228.36 |
| ATOM | 3462 | O | VAL | E | 179 | -1.568 | -63.525 | -35.733 | 1.00225.86 |
| ATOM | 3463 | CB | VAL | E | 179 | -1.883 | -60.604 | -34.911 | 1.00225.05 |
| ATOM | 3464 | CG1 | VAL | E | 179 | -0.966 | -59.759 | -35.779 | 1.00225.44 |
| ATOM | 3465 | CG2 | VAL | E | 179 | -2.643 | -59.728 | -33.918 | 1.00226.47 |
| ATOM | 3466 | N | GLU | E | 180 | -1.973 | -62.632 | -37.787 | 1.00227.03 |
| ATOM | 3467 | CA | GLU | E | 180 | -1.261 | -63.594 | -38.656 | 1.00225.74 |
| ATOM | 3468 | C | GLU | E | 180 | 0.230 | -63.655 | -38.264 | 1.00226.04 |
| ATOM | 3469 | O | GLU | E | 180 | 0.810 | -62.586 | -38.045 | 1.00226.81 |
| ATOM | 3470 | CB | GLU | E | 180 | -1.363 | -63.083 | -40.091 | 1.00231.18 |
| ATOM | 3471 | CG | GLU | E | 180 | -1.520 | -64.166 | -41.136 | 1.00247.47 |
| ATOM | 3472 | CD | GLU | E | 180 | -1.340 | -63.724 | -42.580 | 1.00282.69 |
| ATOM | 3473 | OE1 | GLU | E | 180 | -1.705 | -62.572 | -42.913 | 1.00283.36 |
| ATOM | 3474 | OE2 | GLU | E | 180 | -0.882 | -64.558 | -43.394 | 1.00283.91 |
| ATOM | 3475 | N | ARG | E | 181 | 0.835 | -64.834 | -38.102 | 1.00217.91 |
| ATOM | 3476 | CA | ARG | E | 181 | 2.208 | -64.829 | -37.620 | 1.00214.17 |
| ATOM | 3477 | C | ARG | E | 181 | 3.252 | -65.238 | -38.609 | 1.00221.62 |
| ATOM | 3478 | O | ARG | E | 181 | 3.214 | -66.330 | -39.154 | 1.00220.61 |
| ATOM | 3479 | CB | ARG | E | 181 | 2.329 | -65.513 | -36.273 | 1.00207.37 |
| ATOM | 3480 | CG | ARG | E | 181 | 1.367 | -64.998 | -35.192 | 1.00206.93 |
| ATOM | 3481 | CD | ARG | E | 181 | 1.690 | -63.636 | -34.617 | 1.00201.28 |
| ATOM | 3482 | NE | ARG | E | 181 | 0.677 | -63.182 | -33.662 | 1.00202.00 |
| ATOM | 3483 | CZ | ARG | E | 181 | 0.775 | -62.071 | -32.938 | 1.00218.95 |
| ATOM | 3484 | NH1 | ARG | E | 181 | 1.852 | -61.300 | -33.035 | 1.00206.33 |
| ATOM | 3485 | NH2 | ARG | E | 181 | -0.211 | -61.710 | -32.127 | 1.00207.74 |
| ATOM | 3486 | N | CYS | E | 182 | 4.168 | -64.320 | -38.880 | 1.00223.20 |
| ATOM | 3487 | CA | CYS | E | 182 | 5.240 | -64.510 | -39.838 | 1.00225.90 |
| ATOM | 3488 | C | CYS | E | 182 | 6.495 | -63.714 | -39.490 | 1.00227.43 |
| ATOM | 3489 | O | CYS | E | 182 | 6.445 | -62.789 | -38.681 | 1.00225.99 |
| ATOM | 3490 | CB | CYS | E | 182 | 4.747 | -64.262 | -41.266 | 1.00232.28 |
| ATOM | 3491 | SG | CYS | E | 182 | 4.631 | -62.525 | -41.761 | 1.00241.16 |
| ATOM | 3492 | N | TRP | E | 183 | 7.622 | -64.127 | -40.079 | 1.00224.36 |
| ATOM | 3493 | CA | TRP | E | 183 | 8.941 | -63.543 | -39.872 | 1.00224.03 |
| ATOM | 3494 | C | TRP | E | 183 | 9.344 | -62.622 | -41.003 | 1.00233.48 |
| ATOM | 3495 | O | TRP | E | 183 | 10.030 | -61.635 | -40.753 | 1.00233.18 |
| ATOM | 3496 | CB | TRP | E | 183 | 9.991 | -64.640 | -39.724 | 1.00220.46 |
| ATOM | 3497 | CG | TRP | E | 183 | 9.909 | -65.413 | -38.441 | 1.00218.03 |
| ATOM | 3498 | CD1 | TRP | E | 183 | 9.310 | -66.630 | -38.246 | 1.00219.93 |
| ATOM | 3499 | CD2 | TRP | E | 183 | 10.486 | -65.041 | -37.181 | 1.00215.13 |
| ATOM | 3500 | NE1 | TRP | E | 183 | 9.476 | -67.037 | -36.938 | 1.00216.20 |
| ATOM | 3501 | CE2 | TRP | E | 183 | 10.208 | -66.086 | -36.266 | 1.00216.57 |
| ATOM | 3502 | CE3 | TRP | E | 183 | 11.203 | -63.920 | -36.729 | 1.00215.81 |
| ATOM | 3503 | CZ2 | TRP | E | 183 | 10.640 | -66.045 | -34.935 | 1.00213.10 |
| ATOM | 3504 | CZ3 | TRP | E | 183 | 11.640 | -63.891 | -35.412 | 1.00214.32 |
| ATOM | 3505 | CH2 | TRP | E | 183 | 11.352 | -64.937 | -34.530 | 1.00212.52 |
| ATOM | 3506 | N | THR | E | 184 | 8.990 | -62.989 | -42.253 | 1.00235.29 |
| ATOM | 3507 | CA | THR | E | 184 | 9.264 | -62.235 | -43.489 | 1.00239.99 |
| ATOM | 3508 | C | THR | E | 184 | 8.097 | -62.462 | -44.478 | 1.00247.12 |
| ATOM | 3509 | O | THR | E | 184 | 7.222 | -63.294 | -44.216 | 1.00245.98 |
| ATOM | 3510 | CB | THR | E | 184 | 10.559 | -62.725 | -44.170 | 1.00252.25 |
| ATOM | 3511 | OG1 | THR | E | 184 | 10.287 | -63.955 | -44.839 | 1.00252.93 |
| ATOM | 3512 | CG2 | THR | E | 184 | 11.714 | -62.941 | -43.208 | 1.00248.83 |
| ATOM | 3513 | N | HIS | E | 185 | 8.120 | -61.786 | -45.641 | 1.00247.01 |
| ATOM | 3514 | CA | HIS | E | 185 | 7.086 | -61.987 | -46.653 | 1.00249.51 |
| ATOM | 3515 | C | HIS | E | 185 | 7.050 | -63.422 | -47.155 | 1.00248.27 |
| ATOM | 3516 | O | HIS | E | 185 | 5.998 | -63.885 | -47.583 | 1.00249.08 |
| ATOM | 3517 | CB | HIS | E | 185 | 7.238 | -60.993 | -47.809 | 1.00255.71 |
| ATOM | 3518 | CG | HIS | E | 185 | 8.579 | -61.006 | -48.478 | 1.00260.62 |
| ATOM | 3519 | ND1 | HIS | E | 185 | 9.668 | -61.682 | -47.929 | 1.00259.01 |
| ATOM | 3520 | CD2 | HIS | E | 185 | 8.970 | -60.402 | -49.624 | 1.00267.23 |
| ATOM | 3521 | CE1 | HIS | E | 185 | 10.671 | -61.475 | -48.765 | 1.00261.09 |
| ATOM | 3522 | NE2 | HIS | E | 185 | 10.299 | -60.710 | -49.801 | 1.00266.32 |
| ATOM | 3523 | N | SER | E | 186 | 8.169 | -64.145 | -47.045 | 1.00240.29 |
| ATOM | 3524 | CA | SER | E | 186 | 8.205 | -65.514 | -47.522 | 1.00239.10 |
| ATOM | 3525 | C | SER | E | 186 | 8.359 | -66.600 | -46.441 | 1.00236.82 |
| ATOM | 3526 | O | SER | E | 186 | 8.456 | -67.774 | -46.789 | 1.00236.41 |
| ATOM | 3527 | CB | SER | E | 186 | 9.234 | -65.657 | -48.638 | 1.00244.29 |
| ATOM | 3528 | OG | SER | E | 186 | 10.538 | -65.397 | -48.150 | 1.00248.19 |
| ATOM | 3529 | N | HIS | E | 187 | 8.344 | -66.241 | -45.144 | 1.00228.38 |
| ATOM | 3530 | CA | HIS | E | 187 | 8.467 | -67.249 | -44.087 | 1.00223.73 |
| ATOM | 3531 | C | HIS | E | 187 | 7.427 | -67.057 | -42.980 | 1.00223.51 |
| ATOM | 3532 | O | HIS | E | 187 | 7.453 | -66.046 | -42.262 | 1.00221.36 |
| ATOM | 3533 | CB | HIS | E | 187 | 9.881 | -67.259 | -43.469 | 1.00222.12 |
| ATOM | 3534 | CG | HIS | E | 187 | 11.005 | -67.607 | -44.402 | 1.00227.30 |
| ATOM | 3535 | ND1 | HIS | E | 187 | 11.314 | -68.922 | -44.710 | 1.00229.11 |
| ATOM | 3536 | CD2 | HIS | E | 187 | 11.921 | -66.800 | -44.989 | 1.00230.75 |
| ATOM | 3537 | CE1 | HIS | E | 187 | 12.373 | -68.868 | -45.507 | 1.00230.50 |
| ATOM | 3538 | NE2 | HIS | E | 187 | 12.767 | -67.611 | -45.711 | 1.00231.93 |
| ATOM | 3539 | N | CYS | E | 188 | 6.538 | -68.053 | -42.806 | 1.00218.88 |
| ATOM | 3540 | CA | CYS | E | 188 | 5.556 | -68.016 | -41.716 | 1.00216.76 |
| ATOM | 3541 | C | CYS | E | 188 | 6.292 | -68.373 | -40.416 | 1.00216.25 |
| ATOM | 3542 | O | CYS | E | 188 | 7.421 | -68.923 | -40.443 | 1.00216.32 |
| ATOM | 3543 | CB | CYS | E | 188 | 4.388 | -68.982 | -41.956 | 1.00218.21 |
| ATOM | 3544 | SG | CYS | E | 188 | 3.184 | -68.453 | -43.211 | 1.00227.18 |
| ATOM | 3545 | N | GLN | E | 189 | 5.646 | -68.049 | -39.275 | 1.00208.27 |
| ATOM | 3546 | CA | GLN | E | 189 | 6.112 | -68.485 | -37.972 | 1.00203.19 |
| ATOM | 3547 | C | GLN | E | 189 | 5.493 | -69.844 | -37.818 | 1.00205.06 |
| ATOM | 3548 | O | GLN | E | 189 | 4.287 | -70.028 | -38.054 | 1.00205.15 |
| ATOM | 3549 | CB | GLN | E | 189 | 5.643 | -67.582 | -36.838 | 1.00203.17 |
| ATOM | 3550 | CG | GLN | E | 189 | 6.157 | -68.045 | -35.470 | 1.00201.08 |
| ATOM | 3551 | CD | GLN | E | 189 | 5.718 | -67.165 | -34.329 | 1.00198.08 |
| ATOM | 3552 | OE1 | GLN | E | 189 | 5.023 | -66.157 | -34.534 | 1.00186.53 |
| ATOM | 3553 | NE2 | GLN | E | 189 | 6.132 | -67.519 | -33.098 | 1.00178.27 |
| ATOM | 3554 | N | LYS | E | 190 | 6.336 | -70.806 | -37.450 | 1.00200.02 |
| ATOM | 3555 | CA | LYS | E | 190 | 5.905 | -72.167 | -37.252 | 1.00198.97 |
| ATOM | 3556 | C | LYS | E | 190 | 5.136 | -72.272 | -35.958 | 1.00200.06 |
| ATOM | 3557 | O | LYS | E | 190 | 5.558 | -71.766 | -34.914 | 1.00197.60 |
| ATOM | 3558 | CB | LYS | E | 190 | 7.103 | -73.121 | -37.253 | 1.00199.85 |
| ATOM | 3559 | CG | LYS | E | 190 | 6.695 | -74.565 | -37.055 | 1.00201.00 |
| ATOM | 3560 | CD | LYS | E | 190 | 7.317 | -75.479 | -38.067 | 1.00201.21 |
| ATOM | 3561 | CE | LYS | E | 190 | 6.892 | -76.890 | -37.824 | 1.00196.41 |
| ATOM | 3562 | NZ | LYS | E | 190 | 5.436 | -77.048 | -38.088 | 1.00200.37 |
| ATOM | 3563 | N | VAL | E | 191 | 3.992 | -72.902 | -36.042 | 1.00196.89 |
| ATOM | 3564 | CA | VAL | E | 191 | 3.192 | -73.131 | -34.870 | 1.00195.54 |
| ATOM | 3565 | C | VAL | E | 191 | 2.886 | -74.600 | -34.861 | 1.00203.61 |
| ATOM | 3566 | O | VAL | E | 191 | 2.581 | -75.213 | -35.904 | 1.00206.78 |
| ATOM | 3567 | CB | VAL | E | 191 | 1.956 | -72.228 | -34.778 | 1.00199.71 |
| ATOM | 3568 | CG1 | VAL | E | 191 | 1.022 | -72.680 | -33.664 | 1.00198.45 |
| ATOM | 3569 | CG2 | VAL | E | 191 | 2.372 | -70.796 | -34.539 | 1.00199.28 |
| ATOM | 3570 | N | CYS | E | 192 | 3.079 | -75.177 | -33.679 | 1.00197.95 |
| ATOM | 3571 | CA | CYS | E | 192 | 2.854 | -76.575 | -33.446 | 1.00196.95 |
| ATOM | 3572 | C | CYS | E | 192 | 1.668 | -76.657 | -32.556 | 1.00196.24 |
| ATOM | 3573 | O | CYS | E | 192 | 1.461 | -75.725 | -31.775 | 1.00194.33 |
| ATOM | 3574 | CB | CYS | E | 192 | 4.079 | -77.203 | -32.797 | 1.00195.85 |
| ATOM | 3575 | SG | CYS | E | 192 | 5.474 | -77.452 | -33.933 | 1.00200.43 |
| ATOM | 3576 | N | PRO | E | 193 | 0.870 | -77.738 | -32.638 | 1.00191.90 |
| ATOM | 3577 | CA | PRO | E | 193 | -0.277 | -77.834 | -31.739 | 1.00191.75 |
| ATOM | 3578 | C | PRO | E | 193 | 0.153 | -77.624 | -30.296 | 1.00191.85 |
| ATOM | 3579 | O | PRO | E | 193 | 1.299 | -77.876 | -29.931 | 1.00189.70 |
| ATOM | 3580 | CB | PRO | E | 193 | -0.838 | -79.242 | -31.987 | 1.00195.05 |
| ATOM | 3581 | CG | PRO | E | 193 | 0.146 | -79.956 | -32.838 | 1.00199.45 |
| ATOM | 3582 | CD | PRO | E | 193 | 0.974 | -78.917 | -33.525 | 1.00194.71 |
| ATOM | 3583 | N | THR | E | 194 | -0.765 | -77.097 | -29.509 | 1.00188.45 |
| ATOM | 3584 | CA | THR | E | 194 | -0.674 | -76.804 | -28.083 | 1.00186.94 |
| ATOM | 3585 | C | THR | E | 194 | -0.080 | -78.031 | -27.305 | 1.00187.89 |
| ATOM | 3586 | O | THR | E | 194 | 0.796 | -77.867 | -26.447 | 1.00184.52 |
| ATOM | 3587 | CB | THR | E | 194 | -2.116 | -76.387 | -27.652 | 1.00200.85 |
| ATOM | 3588 | OG1 | THR | E | 194 | -3.045 | -77.429 | -28.026 | 1.00206.95 |
| ATOM | 3589 | CG2 | THR | E | 194 | -2.599 | -75.064 | -28.329 | 1.00196.58 |
| ATOM | 3590 | N | ILE | E | 195 | -0.552 | -79.256 | -27.676 | 1.00184.86 |
| ATOM | 3591 | CA | ILE | E | 195 | -0.138 | -80.561 | -27.143 | 1.00182.66 |
| ATOM | 3592 | C | ILE | E | 195 | 1.321 | -80.910 | -27.361 | 1.00185.08 |
| ATOM | 3593 | O | ILE | E | 195 | 1.830 | -81.696 | -26.567 | 1.00183.77 |
| ATOM | 3594 | CB | ILE | E | 195 | -1.066 | -81.706 | -27.545 | 1.00186.90 |
| ATOM | 3595 | CG1 | ILE | E | 195 | -1.539 | -81.581 | -28.998 | 1.00188.35 |
| ATOM | 3596 | CG2 | ILE | E | 195 | -2.227 | -81.740 | -26.612 | 1.00189.53 |
| ATOM | 3597 | CD1 | ILE | E | 195 | -0.701 | -82.293 | -29.975 | 1.00193.63 |
| ATOM | 3598 | N | CYS | E | 196 | 2.004 | -80.330 | -28.403 | 1.00182.07 |
| ATOM | 3599 | CA | CYS | E | 196 | 3.454 | -80.532 | -28.689 | 1.00180.96 |
| ATOM | 3600 | C | CYS | E | 196 | 4.298 | -79.810 | -27.700 | 1.00183.24 |
| ATOM | 3601 | O | CYS | E | 196 | 5.511 | -80.068 | -27.620 | 1.00181.86 |
| ATOM | 3602 | CB | CYS | E | 196 | 3.857 | -80.105 | -30.098 | 1.00182.26 |
| ATOM | 3603 | SG | CYS | E | 196 | 3.214 | -81.133 | -31.427 | 1.00189.29 |
| ATOM | 3604 | N | LYS | E | 197 | 3.689 | -78.817 | -27.036 | 1.00180.01 |
| ATOM | 3605 | CA | LYS | E | 197 | 4.386 | -77.988 | -26.088 | 1.00179.37 |
| ATOM | 3606 | C | LYS | E | 197 | 5.592 | -77.370 | -26.801 | 1.00183.81 |
| ATOM | 3607 | O | LYS | E | 197 | 5.460 | -76.846 | -27.925 | 1.00183.81 |
| ATOM | 3608 | CB | LYS | E | 197 | 4.782 | -78.820 | -24.848 | 1.00181.51 |
| ATOM | 3609 | CG | LYS | E | 197 | 3.567 | -79.278 | -24.101 | 1.00201.75 |
| ATOM | 3610 | CD | LYS | E | 197 | 3.869 | -80.374 | -23.128 | 1.00216.14 |
| ATOM | 3611 | CE | LYS | E | 197 | 3.010 | -80.251 | -21.886 | 1.00233.01 |
| ATOM | 3612 | NZ | LYS | E | 197 | 1.773 | -79.440 | -22.108 | 1.00246.37 |
| ATOM | 3613 | N | SER | E | 198 | 6.764 | -77.499 | -26.178 | 1.00179.29 |
| ATOM | 3614 | CA | SER | E | 198 | 7.971 | -76.926 | -26.706 | 1.00177.90 |
| ATOM | 3615 | C | SER | E | 198 | 8.762 | -77.908 | -27.513 | 1.00182.02 |
| ATOM | 3616 | O | SER | E | 198 | 9.793 | -77.516 | -28.020 | 1.00181.95 |
| ATOM | 3617 | CB | SER | E | 198 | 8.814 | -76.376 | -25.563 | 1.00179.03 |
| ATOM | 3618 | OG | SER | E | 198 | 9.163 | -77.392 | -24.636 | 1.00184.16 |
| ATOM | 3619 | N | HIS | E | 199 | 8.292 | -79.152 | -27.684 | 1.00178.64 |
| ATOM | 3620 | CA | HIS | E | 199 | 9.076 | -80.167 | -28.388 | 1.00178.99 |
| ATOM | 3621 | C | HIS | E | 199 | 9.260 | -79.986 | -29.852 | 1.00182.92 |
| ATOM | 3622 | O | HIS | E | 199 | 10.212 | -80.548 | -30.400 | 1.00183.12 |
| ATOM | 3623 | CB | HIS | E | 199 | 8.567 | -81.560 | -28.072 | 1.00181.30 |
| ATOM | 3624 | CG | HIS | E | 199 | 8.419 | -81.773 | -26.603 | 1.00184.43 |
| ATOM | 3625 | ND1 | HIS | E | 199 | 7.245 | -82.222 | -26.060 | 1.00187.26 |
| ATOM | 3626 | CD2 | HIS | E | 199 | 9.309 | -81.545 | -25.602 | 1.00185.15 |
| ATOM | 3627 | CE1 | HIS | E | 199 | 7.454 | -82.276 | -24.755 | 1.00186.09 |
| ATOM | 3628 | NE2 | HIS | E | 199 | 8.684 | -81.878 | -24.433 | 1.00185.13 |
| ATOM | 3629 | N | GLY | E | 200 | 8.388 | -79.198 | -30.472 | 1.00180.45 |
| ATOM | 3630 | CA | GLY | E | 200 | 8.418 | -78.996 | -31.913 | 1.00183.08 |
| ATOM | 3631 | C | GLY | E | 200 | 7.634 | -80.085 | -32.616 | 1.00193.28 |
| ATOM | 3632 | O | GLY | E | 200 | 7.159 | -81.035 | -31.977 | 1.00193.78 |
| ATOM | 3633 | N | CYS | E | 201 | 7.501 | -79.968 | -33.940 | 1.00193.98 |
| ATOM | 3634 | CA | CYS | E | 201 | 6.724 | -80.919 | -34.716 | 1.00197.23 |
| ATOM | 3635 | C | CYS | E | 201 | 7.224 | -80.927 | -36.143 | 1.00205.27 |
| ATOM | 3636 | O | CYS | E | 201 | 7.975 | -80.026 | -36.543 | 1.00204.56 |
| ATOM | 3637 | CB | CYS | E | 201 | 5.245 | -80.535 | -34.657 | 1.00198.65 |
| ATOM | 3638 | SG | CYS | E | 201 | 4.876 | -78.864 | -35.290 | 1.00203.52 |
| ATOM | 3639 | N | THR | E | 202 | 6.760 | -81.916 | -36.927 | 1.00205.46 |
| ATOM | 3640 | CA | THR | E | 202 | 7.034 | -82.008 | -38.366 | 1.00208.27 |
| ATOM | 3641 | C | THR | E | 202 | 6.047 | -81.082 | -39.109 | 1.00213.53 |
| ATOM | 3642 | O | THR | E | 202 | 5.137 | -80.510 | -38.498 | 1.00210.98 |
| ATOM | 3643 | CB | THR | E | 202 | 6.894 | -83.459 | -38.871 | 1.00215.67 |
| ATOM | 3644 | OG1 | THR | E | 202 | 5.535 | -83.881 | -38.722 | 1.00214.92 |
| ATOM | 3645 | CG2 | THR | E | 202 | 7.824 | -84.423 | -38.143 | 1.00211.75 |
| ATOM | 3646 | N | ALA | E | 203 | 6.198 | -80.976 | -40.438 | 1.00213.89 |
| ATOM | 3647 | CA | ALA | E | 203 | 5.324 | -80.153 | -41.274 | 1.00215.97 |
| ATOM | 3648 | C | ALA | E | 203 | 3.875 | -80.586 | -41.142 | 1.00221.64 |
| ATOM | 3649 | O | ALA | E | 203 | 2.973 | -79.751 | -41.183 | 1.00220.99 |
| ATOM | 3650 | CB | ALA | E | 203 | 5.761 | -80.239 | -42.729 | 1.00220.08 |
| ATOM | 3651 | N | GLU | E | 204 | 3.662 | -81.885 | -40.921 | 1.00220.30 |
| ATOM | 3652 | CA | GLU | E | 204 | 2.331 | -82.468 | -40.796 | 1.00221.75 |
| ATOM | 3653 | C | GLU | E | 204 | 1.722 | -82.304 | -39.405 | 1.00222.36 |
| ATOM | 3654 | O | GLU | E | 204 | 0.619 | -82.798 | -39.179 | 1.00223.65 |
| ATOM | 3655 | CB | GLU | E | 204 | 2.328 | -83.946 | -41.222 | 1.00225.45 |
| ATOM | 3656 | CG | GLU | E | 204 | 2.751 | -84.177 | -42.660 | 1.00240.95 |
| ATOM | 3657 | CD | GLU | E | 204 | 4.246 | -84.260 | -42.918 | 1.00264.56 |
| ATOM | 3658 | OE1 | GLU | E | 204 | 5.037 | -84.278 | -41.946 | 1.00251.75 |
| ATOM | 3659 | OE2 | GLU | E | 204 | 4.625 | -84.334 | -44.109 | 1.00264.86 |
| ATOM | 3660 | N | GLY | E | 205 | 2.429 | -81.620 | -38.499 | 1.00213.75 |
| ATOM | 3661 | CA | GLY | E | 205 | 1.973 | -81.362 | -37.133 | 1.00209.67 |
| ATOM | 3662 | C | GLY | E | 205 | 2.233 | -82.449 | -36.104 | 1.00207.40 |
| ATOM | 3663 | O | GLY | E | 205 | 1.697 | -82.372 | -34.999 | 1.00204.50 |
| ATOM | 3664 | N | LEU | E | 206 | 3.068 | -83.454 | -36.442 | 1.00202.15 |
| ATOM | 3665 | CA | LEU | E | 206 | 3.381 | -84.583 | -35.560 | 1.00199.62 |
| ATOM | 3666 | C | LEU | E | 206 | 4.479 | -84.229 | -34.593 | 1.00199.11 |
| ATOM | 3667 | O | LEU | E | 206 | 5.573 | -83.817 | -34.996 | 1.00198.85 |
| ATOM | 3668 | CB | LEU | E | 206 | 3.711 | -85.850 | -36.353 | 1.00201.68 |
| ATOM | 3669 | CG | LEU | E | 206 | 2.693 | -86.250 | -37.414 | 1.00209.58 |
| ATOM | 3670 | CD1 | LEU | E | 206 | 3.210 | -87.386 | -38.249 | 1.00212.56 |
| ATOM | 3671 | CD2 | LEU | E | 206 | 1.356 | -86.603 | -36.796 | 1.00212.60 |
| ATOM | 3672 | N | CYS | E | 207 | 4.167 | -84.346 | -33.305 | 1.00192.00 |
| ATOM | 3673 | CA | CYS | E | 207 | 5.103 | -83.949 | -32.268 | 1.00188.28 |
| ATOM | 3674 | C | CYS | E | 207 | 6.402 | -84.684 | -32.320 | 1.00191.81 |
| ATOM | 3675 | O | CYS | E | 207 | 6.453 | -85.891 | -32.576 | 1.00192.11 |
| ATOM | 3676 | CB | CYS | E | 207 | 4.489 | -83.992 | -30.869 | 1.00186.78 |
| ATOM | 3677 | SG | CYS | E | 207 | 2.987 | -82.984 | -30.648 | 1.00190.37 |
| ATOM | 3678 | N | CYS | E | 208 | 7.460 | -83.925 | -32.101 | 1.00188.67 |
| ATOM | 3679 | CA | CYS | E | 208 | 8.808 | -84.445 | -31.942 | 1.00190.08 |
| ATOM | 3680 | C | CYS | E | 208 | 8.847 | -85.221 | -30.624 | 1.00192.39 |
| ATOM | 3681 | O | CYS | E | 208 | 7.927 | -85.055 | -29.814 | 1.00192.43 |
| ATOM | 3682 | CB | CYS | E | 208 | 9.793 | -83.287 | -31.907 | 1.00190.05 |
| ATOM | 3683 | SG | CYS | E | 208 | 9.992 | -82.455 | -33.498 | 1.00196.29 |
| ATOM | 3684 | N | HIS | E | 209 | 9.913 | -86.034 | -30.385 | 1.00185.95 |
| ATOM | 3685 | CA | HIS | E | 209 | 10.066 | -86.717 | -29.114 | 1.00183.68 |
| ATOM | 3686 | C | HIS | E | 209 | 10.130 | -85.669 | -27.956 | 1.00183.96 |
| ATOM | 3687 | O | HIS | E | 209 | 10.622 | -84.548 | -28.150 | 1.00182.44 |
| ATOM | 3688 | CB | HIS | E | 209 | 11.332 | -87.557 | -29.129 | 1.00185.10 |
| ATOM | 3689 | CG | HIS | E | 209 | 11.464 | -88.460 | -27.945 | 1.00188.59 |
| ATOM | 3690 | ND1 | HIS | E | 209 | 12.135 | -88.064 | -26.806 | 1.00188.81 |
| ATOM | 3691 | CD2 | HIS | E | 209 | 11.003 | -89.718 | -27.766 | 1.00192.23 |
| ATOM | 3692 | CE1 | HIS | E | 209 | 12.056 | -89.087 | -25.973 | 1.00189.35 |
| ATOM | 3693 | NE2 | HIS | E | 209 | 11.377 | -90.102 | -26.505 | 1.00191.71 |
| ATOM | 3694 | N | SER | E | 210 | 9.634 | -86.063 | -26.761 | 1.00178.93 |
| ATOM | 3695 | CA | SER | E | 210 | 9.591 | -85.232 | -25.560 | 1.00176.10 |
| ATOM | 3696 | C | SER | E | 210 | 10.960 | -84.770 | -25.079 | 1.00181.15 |
| ATOM | 3697 | O | SER | E | 210 | 11.024 | -83.809 | -24.312 | 1.00180.28 |
| ATOM | 3698 | CB | SER | E | 210 | 8.820 | -85.921 | -24.448 | 1.00176.55 |
| ATOM | 3699 | OG | SER | E | 210 | 9.326 | -87.221 | -24.243 | 1.00178.71 |
| ATOM | 3700 | N | GLU | E | 211 | 12.052 | -85.411 | -25.536 | 1.00179.29 |
| ATOM | 3701 | CA | GLU | E | 211 | 13.379 | -84.931 | -25.193 | 1.00179.19 |
| ATOM | 3702 | C | GLU | E | 211 | 13.925 | -83.916 | -26.196 | 1.00187.23 |
| ATOM | 3703 | O | GLU | E | 211 | 14.968 | -83.319 | -25.927 | 1.00186.54 |
| ATOM | 3704 | CB | GLU | E | 211 | 14.357 | -86.045 | -24.895 | 1.00181.65 |
| ATOM | 3705 | CG | GLU | E | 211 | 15.003 | -85.753 | -23.552 | 1.00190.28 |
| ATOM | 3706 | CD | GLU | E | 211 | 14.145 | -86.008 | -22.322 | 1.00208.96 |
| ATOM | 3707 | OE1 | GLU | E | 211 | 13.749 | -87.175 | -22.088 | 1.00212.02 |
| ATOM | 3708 | OE2 | GLU | E | 211 | 13.804 | -85.032 | -21.619 | 1.00194.96 |
| ATOM | 3709 | N | CYS | E | 212 | 13.203 | -83.699 | -27.340 | 1.00187.44 |
| ATOM | 3710 | CA | CYS | E | 212 | 13.573 | -82.700 | -28.363 | 1.00188.30 |
| ATOM | 3711 | C | CYS | E | 212 | 13.136 | -81.349 | -27.962 | 1.00185.37 |
| ATOM | 3712 | O | CYS | E | 212 | 12.213 | -81.223 | -27.153 | 1.00184.16 |
| ATOM | 3713 | CB | CYS | E | 212 | 13.043 | -83.038 | -29.750 | 1.00192.77 |
| ATOM | 3714 | SG | CYS | E | 212 | 13.437 | -84.712 | -30.321 | 1.00201.00 |
| ATOM | 3715 | N | LEU | E | 213 | 13.778 | -80.321 | -28.560 | 1.00178.14 |
| ATOM | 3716 | CA | LEU | E | 213 | 13.427 | -78.939 | -28.256 | 1.00174.85 |
| ATOM | 3717 | C | LEU | E | 213 | 12.730 | -78.115 | -29.322 | 1.00181.05 |
| ATOM | 3718 | O | LEU | E | 213 | 11.573 | -77.842 | -29.089 | 1.00182.31 |
| ATOM | 3719 | CB | LEU | E | 213 | 14.430 | -78.161 | -27.391 | 1.00172.00 |
| ATOM | 3720 | CG | LEU | E | 213 | 14.077 | -76.733 | -26.969 | 1.00172.79 |
| ATOM | 3721 | CD1 | LEU | E | 213 | 12.789 | -76.657 | -26.176 | 1.00171.41 |
| ATOM | 3722 | CD2 | LEU | E | 213 | 15.108 | -76.186 | -26.101 | 1.00173.41 |
| ATOM | 3723 | N | GLY | E | 214 | 13.297 | -77.710 | -30.430 | 1.00177.83 |
| ATOM | 3724 | CA | GLY | E | 214 | 12.408 | -76.899 | -31.267 | 1.00178.80 |
| ATOM | 3725 | C | GLY | E | 214 | 11.798 | -77.541 | -32.491 | 1.00185.25 |
| ATOM | 3726 | O | GLY | E | 214 | 10.802 | -77.066 | -33.055 | 1.00185.58 |
| ATOM | 3727 | N | ASN | E | 215 | 12.465 | -78.577 | -32.942 | 1.00183.59 |
| ATOM | 3728 | CA | ASN | E | 215 | 12.222 | -79.239 | -34.205 | 1.00186.15 |
| ATOM | 3729 | C | ASN | E | 215 | 12.980 | -80.578 | -34.219 | 1.00189.51 |
| ATOM | 3730 | O | ASN | E | 215 | 13.840 | -80.836 | -33.372 | 1.00185.67 |
| ATOM | 3731 | CB | ASN | E | 215 | 12.724 | -78.282 | -35.357 | 1.00190.80 |
| ATOM | 3732 | CG | ASN | E | 215 | 12.320 | -78.672 | -36.751 | 1.00212.57 |
| ATOM | 3733 | OD1 | ASN | E | 215 | 11.379 | -79.463 | -36.975 | 1.00202.97 |
| ATOM | 3734 | ND2 | ASN | E | 215 | 13.006 | -78.138 | -37.743 | 1.00205.29 |
| ATOM | 3735 | N | CYS | E | 216 | 12.635 | -81.429 | -35.171 | 1.00190.90 |
| ATOM | 3736 | CA | CYS | E | 216 | 13.277 | -82.723 | -35.367 | 1.00194.18 |
| ATOM | 3737 | C | CYS | E | 216 | 13.199 | -83.083 | -36.851 | 1.00201.56 |
| ATOM | 3738 | O | CYS | E | 216 | 12.308 | -82.578 | -37.560 | 1.00201.85 |
| ATOM | 3739 | CB | CYS | E | 216 | 12.591 | -83.788 | -34.511 | 1.00194.92 |
| ATOM | 3740 | SG | CYS | E | 216 | 10.785 | -83.870 | -34.741 | 1.00199.66 |
| ATOM | 3741 | N | SER | E | 217 | 14.113 | -83.963 | -37.311 | 1.00200.11 |
| ATOM | 3742 | CA | SER | E | 217 | 14.098 | -84.484 | -38.673 | 1.00203.78 |
| ATOM | 3743 | C | SER | E | 217 | 13.097 | -85.653 | -38.815 | 1.00209.57 |
| ATOM | 3744 | O | SER | E | 217 | 12.623 | -85.934 | -39.919 | 1.00212.30 |
| ATOM | 3745 | CB | SER | E | 217 | 15.498 | -84.893 | -39.109 | 1.00210.14 |
| ATOM | 3746 | OG | SER | E | 217 | 16.017 | -85.929 | -38.296 | 1.00220.07 |
| ATOM | 3747 | N | GLN | E | 218 | 12.783 | -86.324 | -37.697 | 1.00204.74 |
| ATOM | 3748 | CA | GLN | E | 218 | 11.811 | -87.407 | -37.629 | 1.00206.27 |
| ATOM | 3749 | C | GLN | E | 218 | 11.078 | -87.331 | -36.295 | 1.00207.67 |
| ATOM | 3750 | O | GLN | E | 218 | 11.672 | -86.948 | -35.272 | 1.00204.11 |
| ATOM | 3751 | CB | GLN | E | 218 | 12.484 | -88.776 | -37.736 | 1.00210.43 |
| ATOM | 3752 | CG | GLN | E | 218 | 13.193 | -89.050 | -39.044 | 1.00231.64 |
| ATOM | 3753 | CD | GLN | E | 218 | 13.910 | -90.374 | -39.024 | 1.00263.13 |
| ATOM | 3754 | OE1 | GLN | E | 218 | 13.533 | -91.321 | -38.324 | 1.00258.55 |
| ATOM | 3755 | NE2 | GLN | E | 218 | 14.910 | -90.497 | -39.868 | 1.00265.01 |
| ATOM | 3756 | N | PRO | E | 219 | 9.784 | -87.686 | -36.279 | 1.00205.72 |
| ATOM | 3757 | CA | PRO | E | 219 | 9.056 | -87.672 | -35.012 | 1.00203.13 |
| ATOM | 3758 | C | PRO | E | 219 | 9.403 | -88.866 | -34.151 | 1.00205.03 |
| ATOM | 3759 | O | PRO | E | 219 | 9.939 | -89.878 | -34.629 | 1.00205.21 |
| ATOM | 3760 | CB | PRO | E | 219 | 7.594 | -87.720 | -35.443 | 1.00206.63 |
| ATOM | 3761 | CG | PRO | E | 219 | 7.630 | -88.510 | -36.699 | 1.00215.16 |
| ATOM | 3762 | CD | PRO | E | 219 | 8.922 | -88.137 | -37.391 | 1.00211.09 |
| ATOM | 3763 | N | ASP | E | 220 | 9.101 | -88.713 | -32.870 | 1.00200.59 |
| ATOM | 3764 | CA | ASP | E | 220 | 9.259 | -89.747 | -31.865 | 1.00201.51 |
| ATOM | 3765 | C | ASP | E | 220 | 10.610 | -90.486 | -31.922 | 1.00204.79 |
| ATOM | 3766 | O | ASP | E | 220 | 10.659 | -91.718 | -31.944 | 1.00205.26 |
| ATOM | 3767 | CB | ASP | E | 220 | 8.052 | -90.722 | -31.922 | 1.00206.33 |
| ATOM | 3768 | CG | ASP | E | 220 | 8.007 | -91.812 | -30.842 | 1.00222.73 |
| ATOM | 3769 | OD2 | ASP | E | 220 | 7.492 | -92.919 | -31.132 | 1.00229.67 |
| ATOM | 3770 | OD1 | ASP | E | 220 | 8.482 | -91.554 | -29.708 | 1.00223.65 |
| ATOM | 3771 | N | ASP | E | 221 | 11.703 | -89.725 | -31.970 | 1.00200.14 |
| ATOM | 3772 | CA | ASP | E | 221 | 13.038 | -90.301 | -31.941 | 1.00201.22 |
| ATOM | 3773 | C | ASP | E | 221 | 13.999 | -89.308 | -31.313 | 1.00203.38 |
| ATOM | 3774 | O | ASP | E | 221 | 14.260 | -88.263 | -31.914 | 1.00202.75 |
| ATOM | 3775 | CB | ASP | E | 221 | 13.516 | -90.721 | -33.331 | 1.00206.38 |
| ATOM | 3776 | CG | ASP | E | 221 | 14.791 | -91.563 | -33.342 | 1.00221.59 |
| ATOM | 3777 | OD2 | ASP | E | 221 | 14.968 | -92.352 | -34.285 | 1.00232.91 |
| ATOM | 3778 | OD1 | ASP | E | 221 | 15.638 | -91.399 | -32.426 | 1.00220.89 |
| ATOM | 3779 | N | PRO | E | 222 | 14.565 | -89.638 | -30.126 | 1.00199.08 |
| ATOM | 3780 | CA | PRO | E | 222 | 15.479 | -88.701 | -29.436 | 1.00197.26 |
| ATOM | 3781 | C | PRO | E | 222 | 16.913 | -88.570 | -29.989 | 1.00204.12 |
| ATOM | 3782 | O | PRO | E | 222 | 17.751 | -87.858 | -29.422 | 1.00202.08 |
| ATOM | 3783 | CB | PRO | E | 222 | 15.445 | -89.194 | -27.991 | 1.00197.96 |
| ATOM | 3784 | CG | PRO | E | 222 | 15.219 | -90.643 | -28.125 | 1.00204.83 |
| ATOM | 3785 | CD | PRO | E | 222 | 14.336 | -90.847 | -29.316 | 1.00201.58 |
| ATOM | 3786 | N | THR | E | 223 | 17.193 | -89.252 | -31.091 | 1.00204.83 |
| ATOM | 3787 | CA | THR | E | 223 | 18.475 | -89.166 | -31.774 | 1.00206.77 |
| ATOM | 3788 | C | THR | E | 223 | 18.265 | -88.332 | -33.048 | 1.00210.61 |
| ATOM | 3789 | O | THR | E | 223 | 19.205 | -88.161 | -33.815 | 1.00211.99 |
| ATOM | 3790 | CB | THR | E | 223 | 18.994 | -90.583 | -32.137 | 1.00222.91 |
| ATOM | 3791 | OG1 | THR | E | 223 | 18.158 | -91.150 | -33.144 | 1.00227.48 |
| ATOM | 3792 | CG2 | THR | E | 223 | 19.050 | -91.532 | -30.941 | 1.00221.75 |
| ATOM | 3793 | N | LYS | E | 224 | 17.029 | -87.851 | -33.293 | 1.00205.97 |
| ATOM | 3794 | CA | LYS | E | 224 | 16.676 | -87.085 | -34.494 | 1.00206.43 |
| ATOM | 3795 | C | LYS | E | 224 | 16.219 | -85.668 | -34.189 | 1.00208.74 |
| ATOM | 3796 | O | LYS | E | 224 | 15.622 | -85.000 | -35.045 | 1.00209.72 |
| ATOM | 3797 | CB | LYS | E | 224 | 15.666 | -87.849 | -35.372 | 1.00210.22 |
| ATOM | 3798 | CG | LYS | E | 224 | 16.362 | -88.550 | -36.545 | 1.00214.13 |
| ATOM | 3799 | CD | LYS | E | 224 | 16.587 | -90.009 | -36.317 | 1.00216.78 |
| ATOM | 3800 | CE | LYS | E | 224 | 17.714 | -90.548 | -37.152 | 1.00213.82 |
| ATOM | 3801 | NZ | LYS | E | 224 | 18.903 | -90.885 | -36.325 | 1.00213.01 |
| ATOM | 3802 | N | CYS | E | 225 | 16.543 | -85.199 | -32.978 | 1.00202.54 |
| ATOM | 3803 | CA | CYS | E | 225 | 16.201 | -83.844 | -32.539 | 1.00200.01 |
| ATOM | 3804 | C | CYS | E | 225 | 17.100 | -82.815 | -33.166 | 1.00203.64 |
| ATOM | 3805 | O | CYS | E | 225 | 18.322 | -83.020 | -33.218 | 1.00205.98 |
| ATOM | 3806 | CB | CYS | E | 225 | 16.296 | -83.710 | -31.023 | 1.00198.04 |
| ATOM | 3807 | SG | CYS | E | 225 | 15.449 | -84.987 | -30.074 | 1.00201.76 |
| ATOM | 3808 | N | VAL | E | 226 | 16.544 | -81.632 | -33.446 | 1.00195.63 |
| ATOM | 3809 | CA | VAL | E | 226 | 17.382 | -80.527 | -33.863 | 1.00193.79 |
| ATOM | 3810 | C | VAL | E | 226 | 18.045 | -79.938 | -32.580 | 1.00190.14 |
| ATOM | 3811 | O | VAL | E | 226 | 19.196 | -79.528 | -32.634 | 1.00189.89 |
| ATOM | 3812 | CB | VAL | E | 226 | 16.545 | -79.490 | -34.665 | 1.00198.51 |
| ATOM | 3813 | CG1 | VAL | E | 226 | 17.259 | -78.146 | -34.790 | 1.00198.10 |
| ATOM | 3814 | CG2 | VAL | E | 226 | 16.154 | -80.036 | -36.045 | 1.00201.38 |
| ATOM | 3815 | N | ALA | E | 227 | 17.377 | -80.038 | -31.421 | 1.00181.68 |
| ATOM | 3816 | CA | ALA | E | 227 | 17.812 | -79.360 | -30.216 | 1.00178.85 |
| ATOM | 3817 | C | ALA | E | 227 | 18.266 | -79.977 | -28.861 | 1.00181.77 |
| ATOM | 3818 | O | ALA | E | 227 | 19.373 | -79.662 | -28.428 | 1.00182.09 |
| ATOM | 3819 | CB | ALA | E | 227 | 16.840 | -78.241 | -29.943 | 1.00178.09 |
| ATOM | 3820 | N | CYS | E | 228 | 17.397 | -80.666 | -28.122 | 1.00177.28 |
| ATOM | 3821 | CA | CYS | E | 228 | 17.491 | -81.179 | -26.728 | 1.00176.49 |
| ATOM | 3822 | C | CYS | E | 228 | 16.879 | -80.252 | -25.740 | 1.00177.34 |
| ATOM | 3823 | O | CYS | E | 228 | 17.385 | -79.160 | -25.485 | 1.00175.88 |
| ATOM | 3824 | CB | CYS | E | 228 | 18.846 | -81.671 | -26.228 | 1.00178.06 |
| ATOM | 3825 | SG | CYS | E | 228 | 19.586 | -82.982 | -27.222 | 1.00185.37 |
| ATOM | 3826 | N | ARG | E | 229 | 15.803 | -80.725 | -25.130 | 1.00172.07 |
| ATOM | 3827 | CA | ARG | E | 229 | 15.107 | -80.045 | -24.060 | 1.00169.32 |
| ATOM | 3828 | C | ARG | E | 229 | 16.037 | -80.036 | -22.845 | 1.00171.94 |
| ATOM | 3829 | O | ARG | E | 229 | 16.181 | -78.991 | -22.222 | 1.00170.44 |
| ATOM | 3830 | CB | ARG | E | 229 | 13.811 | -80.811 | -23.752 | 1.00169.44 |
| ATOM | 3831 | CG | ARG | E | 229 | 13.005 | -80.291 | -22.557 | 1.00176.78 |
| ATOM | 3832 | CD | ARG | E | 229 | 11.739 | -81.110 | -22.320 | 1.00183.66 |
| ATOM | 3833 | NE | ARG | E | 229 | 11.602 | -81.565 | -20.933 | 1.00196.67 |
| ATOM | 3834 | CZ | ARG | E | 229 | 11.248 | -82.799 | -20.566 | 1.00216.76 |
| ATOM | 3835 | NH1 | ARG | E | 229 | 10.938 | -83.710 | -21.482 | 1.00205.45 |
| ATOM | 3836 | NH2 | ARG | E | 229 | 11.187 | -83.125 | -19.278 | 1.00205.62 |
| ATOM | 3837 | N | ASN | E | 230 | 16.702 | -81.187 | -22.542 | 1.00168.77 |
| ATOM | 3838 | CA | ASN | E | 230 | 17.552 | -81.313 | -21.377 | 1.00167.70 |
| ATOM | 3839 | C | ASN | E | 230 | 19.041 | -81.350 | -21.694 | 1.00171.91 |
| ATOM | 3840 | O | ASN | E | 230 | 19.685 | -80.295 | -21.806 | 1.00171.26 |
| ATOM | 3841 | CB | ASN | E | 230 | 17.068 | -82.468 | -20.498 | 1.00166.39 |
| ATOM | 3842 | CG | ASN | E | 230 | 15.679 | -82.257 | -19.929 | 1.00189.24 |
| ATOM | 3843 | OD1 | ASN | E | 230 | 15.447 | -81.349 | -19.125 | 1.00176.16 |
| ATOM | 3844 | ND2 | ASN | E | 230 | 14.735 | -83.113 | -20.300 | 1.00187.51 |
| ATOM | 3845 | N | PHE | E | 231 | 19.593 | -82.542 | -21.823 | 1.00169.51 |
| ATOM | 3846 | CA | PHE | E | 231 | 21.027 | -82.694 | -22.051 | 1.00170.03 |
| ATOM | 3847 | C | PHE | E | 231 | 21.338 | -83.536 | -23.258 | 1.00180.57 |
| ATOM | 3848 | O | PHE | E | 231 | 20.502 | -84.315 | -23.732 | 1.00183.15 |
| ATOM | 3849 | CB | PHE | E | 231 | 21.739 | -83.248 | -20.801 | 1.00170.77 |
| ATOM | 3850 | CG | PHE | E | 231 | 21.669 | -82.314 | -19.631 | 1.00168.59 |
| ATOM | 3851 | CD2 | PHE | E | 231 | 20.826 | -82.572 | -18.573 | 1.00168.66 |
| ATOM | 3852 | CD1 | PHE | E | 231 | 22.460 | -81.176 | -19.585 | 1.00169.39 |
| ATOM | 3853 | CE2 | PHE | E | 231 | 20.730 | -81.681 | -17.518 | 1.00170.35 |
| ATOM | 3854 | CE1 | PHE | E | 231 | 22.371 | -80.290 | -18.526 | 1.00168.72 |
| ATOM | 3855 | CZ | PHE | E | 231 | 21.483 | -80.531 | -17.517 | 1.00167.63 |
| ATOM | 3856 | N | TYR | E | 232 | 22.553 | -83379 | -23.758 | 1.00178.78 |
| ATOM | 3857 | CA | TYR | E | 232 | 23.002 | -84.076 | -24.943 | 1.00181.33 |
| ATOM | 3858 | C | TYR | E | 232 | 24.126 | -85.031 | -24.598 | 1.00188.12 |
| ATOM | 3859 | O | TYR | E | 232 | 25.118 | -84.640 | -23.981 | 1.00187.86 |
| ATOM | 3860 | CB | TYR | E | 232 | 23.434 | -83.073 | -26.048 | 1.00182.96 |
| ATOM | 3861 | CG | TYR | E | 232 | 24.169 | -83.761 | -27.168 | 1.00188.24 |
| ATOM | 3862 | CD1 | TYR | E | 232 | 23.478 | -84.463 | -28.152 | 1.00190.91 |
| ATOM | 3863 | CD2 | TYR | E | 232 | 25.558 | -83.851 | -27.159 | 1.00192.07 |
| ATOM | 3864 | CE1 | TYR | E | 232 | 24.157 | -85.193 | -29.132 | 1.00194.36 |
| ATOM | 3865 | CE2 | TYR | E | 232 | 26.247 | -84.589 | -28.122 | 1.00196.58 |
| ATOM | 3866 | CZ | TYR | E | 232 | 25.544 | -85.254 | -29.113 | 1.00202.92 |
| ATOM | 3867 | OH | TYR | E | 232 | 26.242 | -85.959 | -30.066 | 1.00204.39 |
| ATOM | 3868 | N | LEU | E | 233 | 24.009 | -86.270 | -25.055 | 1.00186.93 |
| ATOM | 3869 | CA | LEU | E | 233 | 25.053 | -87.255 | -24.837 | 1.00189.08 |
| ATOM | 3870 | C | LEU | E | 233 | 25.076 | -88.271 | -25.937 | 1.00196.05 |
| ATOM | 3871 | O | LEU | E | 233 | 24.048 | -88.894 | -26.217 | 1.00196.57 |
| ATOM | 3872 | CB | LEU | E | 233 | 24.803 | -87.976 | -23.499 | 1.00188.91 |
| ATOM | 3873 | CG | LEU | E | 233 | 25.840 | -88.976 | -23.044 | 1.00197.11 |
| ATOM | 3874 | CD1 | LEU | E | 233 | 25.913 | -88.996 | -21.554 | 1.00196.53 |
| ATOM | 3875 | CD2 | LEU | E | 233 | 25.472 | -90.343 | -23.432 | 1.00203.06 |
| ATOM | 3876 | N | ASP | E | 234 | 26.267 | -88.508 | -26.497 | 1.00194.45 |
| ATOM | 3877 | CA | ASP | E | 234 | 26.513 | -89.566 | -27.469 | 1.00197.78 |
| ATOM | 3878 | C | ASP | E | 234 | 25.394 | -89.705 | -28.523 | 1.00200.31 |
| ATOM | 3879 | O | ASP | E | 234 | 24.749 | -90.755 | -28.626 | 1.00200.30 |
| ATOM | 3880 | CB | ASP | E | 234 | 26.760 | -90.901 | -26.718 | 1.00202.51 |
| ATOM | 3881 | CG | ASP | E | 234 | 27.990 | -90.947 | -25.793 | 1.00218.00 |
| ATOM | 3882 | OD1 | ASP | E | 234 | 28.975 | -90.223 | -26.068 | 1.00220.21 |
| ATOM | 3883 | OD2 | ASP | E | 234 | 27.991 | -91.763 | -24.832 | 1.00224.79 |
| ATOM | 3884 | N | GLY | E | 235 | 25.157 | -88.608 | -29.246 | 1.00194.76 |
| ATOM | 3885 | CA | GLY | E | 235 | 24.162 | -88.491 | -30.311 | 1.00193.46 |
| ATOM | 3886 | C | GLY | E | 235 | 22.719 | -88.267 | -29.895 | 1.00191.83 |
| ATOM | 3887 | O | GLY | E | 235 | 21.894 | -87.848 | -30.714 | 1.00191.15 |
| ATOM | 3888 | N | ARG | E | 236 | 22.429 | -88.425 | -28.614 | 1.00184.64 |
| ATOM | 3889 | CA | ARG | E | 236 | 21.075 | -88.474 | -28.098 | 1.00181.72 |
| ATOM | 3890 | C | ARG | E | 236 | 20.665 | -87.397 | -27.115 | 1.00184.51 |
| ATOM | 3891 | O | ARG | E | 236 | 21.499 | -86.889 | -26.383 | 1.00183.01 |
| ATOM | 3892 | CB | ARG | E | 236 | 20.975 | -89.845 | -27.432 | 1.00178.95 |
| ATOM | 3893 | CG | ARG | E | 236 | 19.605 | -90.315 | -27.004 | 1.00168.89 |
| ATOM | 3894 | CD | ARG | E | 236 | 19.717 | -91.687 | -26.407 | 1.00159.23 |
| ATOM | 3895 | NE | ARG | E | 236 | 20.237 | -91.578 | -25.056 | 1.00145.55 |
| ATOM | 3896 | CZ | ARG | E | 236 | 20.763 | -92.577 | -24.373 | 1.00164.02 |
| ATOM | 3897 | NH1 | ARG | E | 236 | 20.892 | -93.774 | -24.929 | 1.00174.66 |
| ATOM | 3898 | NH2 | ARG | E | 236 | 21.169 | -92.392 | -23.125 | 1.00136.33 |
| ATOM | 3899 | N | CYS | E | 237 | 19.367 | -87.073 | -27.074 | 1.00181.84 |
| ATOM | 3900 | CA | CYS | E | 237 | 18.811 | -86.151 | -26.090 | 1.00180.10 |
| ATOM | 3901 | C | CYS | E | 237 | 18.350 | -86.975 | -24.907 | 1.00178.68 |
| ATOM | 3902 | O | CYS | E | 237 | 17.511 | -87.879 | -25.061 | 1.00178.98 |
| ATOM | 3903 | CB | CYS | E | 237 | 17.657 | -85.332 | -26.663 | 1.00180.99 |
| ATOM | 3904 | SG | CYS | E | 237 | 18.127 | -84.191 | -27.982 | 1.00186.15 |
| ATOM | 3905 | N | VAL | E | 238 | 18.922 | -86.671 | -23.733 | 1.00170.90 |
| ATOM | 3906 | CA | VAL | E | 238 | 18.649 | -87.339 | -22.460 | 1.00170.29 |
| ATOM | 3907 | C | VAL | E | 238 | 18.136 | -86.410 | -21.367 | 1.00171.95 |
| ATOM | 3908 | O | VAL | E | 238 | 18.523 | -85.242 | -21.307 | 1.00171.49 |
| ATOM | 3909 | CB | VAL | E | 238 | 19.844 | -88.155 | -21.938 | 1.00176.29 |
| ATOM | 3910 | CG1 | VAL | E | 238 | 20.236 | -89.229 | -22.923 | 1.00179.25 |
| ATOM | 3911 | CG2 | VAL | E | 238 | 21.035 | -87.273 | -21.600 | 1.00175.35 |
| ATOM | 3912 | N | GLU | E | 239 | 17.298 | -86.952 | -20.469 | 1.00166.95 |
| ATOM | 3913 | CA | GLU | E | 239 | 16.746 | -86.196 | -19.350 | 1.00164.49 |
| ATOM | 3914 | C | GLU | E | 239 | 17.816 | -85.742 | -18.361 | 1.00165.04 |
| ATOM | 3915 | O | GLU | E | 239 | 17.792 | -84.607 | -17.898 | 1.00162.71 |
| ATOM | 3916 | CB | GLU | E | 239 | 15.612 | -86.966 | -18.658 | 1.00166.86 |
| ATOM | 3917 | CG | GLU | E | 239 | 15.056 | -86.211 | -17.458 | 1.00178.27 |
| ATOM | 3918 | CD | GLU | E | 239 | 13.861 | -86.812 | -16.750 | 1.00195.68 |
| ATOM | 3919 | OE1 | GLU | E | 239 | 12.903 | -87.223 | -17.447 | 1.00200.78 |
| ATOM | 3920 | OE2 | GLU | E | 239 | 13.912 | -86.933 | -15.502 | 1.00176.05 |
| ATOM | 3921 | N | THR | E | 240 | 18.731 | -86.615 | -18.036 | 1.00163.00 |
| ATOM | 3922 | CA | THR | E | 240 | 19.809 | -86.268 | -17.128 | 1.00163.69 |
| ATOM | 3923 | C | THR | E | 240 | 21.061 | -86.950 | -17.584 | 1.00171.24 |
| ATOM | 3924 | O | THR | E | 240 | 20.979 | -87.966 | -18.279 | 1.00172.30 |
| ATOM | 3925 | CB | THR | E | 240 | 19.466 | -86.661 | -15.676 | 1.00171.77 |
| ATOM | 3926 | OG1 | THR | E | 240 | 20.562 | -86.291 | -14.842 | 1.00169.89 |
| ATOM | 3927 | CG2 | THR | E | 240 | 19.126 | -88.161 | -15.518 | 1.00171.47 |
| ATOM | 3928 | N | CYS | E | 241 | 22.225 | -86.427 | -17.172 | 1.00170.09 |
| ATOM | 3929 | CA | CYS | E | 241 | 23.498 | -87.092 | -17.445 | 1.00173.33 |
| ATOM | 3930 | C | CYS | E | 241 | 23.584 | -88.247 | -16.483 | 1.00178.41 |
| ATOM | 3931 | O | CYS | E | 241 | 23.487 | -88.022 | -15.274 | 1.00179.17 |
| ATOM | 3932 | CB | CYS | E | 241 | 24.685 | -86.160 | -17.243 | 1.00174.36 |
| ATOM | 3933 | SG | CYS | E | 241 | 24.768 | -84.789 | -18.415 | 1.00176.76 |
| ATOM | 3934 | N | PRO | E | 242 | 23.758 | -89.493 | -16.930 | 1.00173.89 |
| ATOM | 3935 | CA | PRO | E | 242 | 23.875 | -90.543 | -15.937 | 1.00174.49 |
| ATOM | 3936 | C | PRO | E | 242 | 25.338 | -90.609 | -15.497 | 1.00177.38 |
| ATOM | 3937 | O | PRO | E | 242 | 26.222 | -90.161 | -16.253 | 1.00178.05 |
| ATOM | 3938 | CB | PRO | E | 242 | 23.473 | -91.782 | -16.727 | 1.00178.23 |
| ATOM | 3939 | CG | PRO | E | 242 | 23.988 | -91.519 | -18.120 | 1.00182.91 |
| ATOM | 3940 | CD | PRO | E | 242 | 23.916 | -90.012 | -18.311 | 1.00175.89 |
| ATOM | 3941 | N | PRO | E | 243 | 25.619 | -91.159 | -14.300 | 1.00171.55 |
| ATOM | 3942 | CA | PRO | E | 243 | 27.010 | -91.418 | -13.921 | 1.00172.24 |
| ATOM | 3943 | C | PRO | E | 243 | 27.573 | -92.501 | -14.863 | 1.00175.78 |
| ATOM | 3944 | O | PRO | E | 243 | 26.833 | -93.370 | -15.337 | 1.00173.94 |
| ATOM | 3945 | CB | PRO | E | 243 | 26.880 | -91.929 | -12.504 | 1.00175.93 |
| ATOM | 3946 | CG | PRO | E | 243 | 25.482 | -91.534 | -12.068 | 1.00178.13 |
| ATOM | 3947 | CD | PRO | E | 243 | 24.693 | -91.678 | -13.282 | 1.00172.74 |
| ATOM | 3948 | N | PRO | E | 244 | 28.840 | -92.402 | -15.262 | 1.00175.61 |
| ATOM | 3949 | CA | PRO | E | 244 | 29.867 | -91.504 | -14.730 | 1.00176.16 |
| ATOM | 3950 | C | PRO | E | 244 | 30.045 | -90.152 | -15.388 | 1.00176.33 |
| ATOM | 3951 | O | PRO | E | 244 | 31.144 | -89.613 | -15.282 | 1.00175.83 |
| ATOM | 3952 | CB | PRO | E | 244 | 31.134 | -92.347 | -14.889 | 1.00183.05 |
| ATOM | 3953 | CG | PRO | E | 244 | 30.899 | -93.085 | -16.183 | 1.00188.34 |
| ATOM | 3954 | CD | PRO | E | 244 | 29,422 | -93.399 | -16.186 | 1.00181.08 |
| ATOM | 3955 | N | TYR | E | 245 | 29.019 | -89.620 | -16.087 | 1.00170.74 |
| ATOM | 3956 | CA | TYR | E | 245 | 29.122 | -88.316 | -16.765 | 1.00168.23 |
| ATOM | 3957 | C | TYR | E | 245 | 28.647 | -87.138 | -15.899 | 1.00171.37 |
| ATOM | 3958 | O | TYR | E | 245 | 27.802 | -87.325 | -15.029 | 1.00171.59 |
| ATOM | 3959 | CB | TYR | E | 245 | 28.392 | -88.297 | -18.115 | 1.00167.68 |
| ATOM | 3960 | CG | TYR | E | 245 | 28.817 | -89.405 | -19.037 | 1.00172.59 |
| ATOM | 3961 | CD2 | TYR | E | 245 | 28.012 | -90.520 | -19.228 | 1.00174.89 |
| ATOM | 3962 | CD1 | TYR | E | 245 | 30.026 | -89.349 | -19.720 | 1.00177.10 |
| ATOM | 3963 | CE2 | TYR | E | 245 | 28.418 | -91.586 | -20.036 | 1.00179.69 |
| ATOM | 3964 | CE1 | TYR | E | 245 | 30.440 | -90.402 | -20.550 | 1.00183.64 |
| ATOM | 3965 | CZ | TYR | E | 245 | 29.642 | -91.534 | -20.690 | 1.00190.76 |
| ATOM | 3966 | OH | TYR | E | 245 | 30.051 | -92.601 | -21.479 | 1.00191.98 |
| ATOM | 3967 | N | TYR | E | 246 | 29.170 | -85.927 | -16.153 | 1.00165.73 |
| ATOM | 3968 | CA | TYR | E | 246 | 28.772 | -84.709 | -15.443 | 1.00162.26 |
| ATOM | 3969 | C | TYR | E | 246 | 28.029 | -83.726 | -16.339 | 1.00166.24 |
| ATOM | 3970 | O | TYR | E | 246 | 28.310 | -83.613 | -17.542 | 1.00165.15 |
| ATOM | 3971 | CB | TYR | E | 246 | 29.980 | -84.019 | -14.833 | 1.00162.58 |
| ATOM | 3972 | CG | TYR | E | 246 | 30.714 | -84.906 | -13.866 | 1.00164.56 |
| ATOM | 3973 | CD2 | TYR | E | 246 | 31.876 | -85.573 | -14.245 | 1.00167.65 |
| ATOM | 3974 | CD1 | TYR | E | 246 | 30.226 | -85.116 | -12.577 | 1.00166.35 |
| ATOM | 3975 | CE2 | TYR | E | 246 | 32.541 | -86.421 | -13.363 | 1.00171.41 |
| ATOM | 3976 | CE1 | TYR | E | 246 | 30.870 | -85.976 | -11.693 | 1.00170.13 |
| ATOM | 3977 | CZ | TYR | E | 246 | 32.027 | -86.628 | -12.092 | 1.00177.60 |
| ATOM | 3978 | OH | TYR | E | 246 | 32.684 | -87.446 | -11.217 | 1.00179.34 |
| ATOM | 3979 | N | HIS | E | 247 | 27.085 | -83.002 | -15.728 | 1.00163.05 |
| ATOM | 3980 | CA | HIS | E | 247 | 26.303 | -81.987 | -16.423 | 1.00161.74 |
| ATOM | 3981 | C | HIS | E | 247 | 27.214 | -80.776 | -16.692 | 1.00170.39 |
| ATOM | 3982 | O | HIS | E | 247 | 27.809 | -80.230 | -15.763 | 1.00171.17 |
| ATOM | 3983 | CB | HIS | E | 247 | 25.101 | -81.561 | -15.579 | 1.00160.54 |
| ATOM | 3984 | CG | HIS | E | 247 | 24.178 | -82.667 | -15.188 | 1.00163.97 |
| ATOM | 3985 | ND1 | HIS | E | 247 | 23.055 | -82.948 | -15.924 | 1.00164.60 |
| ATOM | 3986 | CD2 | HIS | E | 247 | 24.184 | -83.454 | -14.087 | 1.00166.55 |
| ATOM | 3987 | CE1 | HIS | E | 247 | 22.413 | -83.895 | -15.261 | 1.00164.36 |
| ATOM | 3988 | NE2 | HIS | E | 247 | 23.069 | -84.251 | -14.166 | 1.00166.21 |
| ATOM | 3989 | N | PHE | E | 248 | 27.341 | -80.374 | -17.963 | 1.00168.62 |
| ATOM | 3990 | CA | PHE | E | 248 | 28.215 | -79.291 | -18.361 | 1.00169.08 |
| ATOM | 3991 | C | PHE | E | 248 | 27.499 | -78.194 | -19.133 | 1.00175.91 |
| ATOM | 3992 | O | PHE | E | 248 | 26.724 | -78.488 | -20.063 | 1.00176.30 |
| ATOM | 3993 | CB | PHE | E | 248 | 29.387 | -79.862 | -19.160 | 1.00172.45 |
| ATOM | 3994 | CG | PHE | E | 248 | 30.457 | -78.856 | -19.464 | 1.00174.50 |
| ATOM | 3995 | CD1 | PHE | E | 248 | 31.212 | -78.298 | -18.451 | 1.00177.91 |
| ATOM | 3996 | CD2 | PHE | E | 248 | 30.705 | -78.456 | -20.765 | 1.00176.89 |
| ATOM | 3997 | CE1 | PHE | E | 248 | 32.200 | -77.371 | -18.735 | 1.00179.13 |
| ATOM | 3998 | CE2 | PHE | E | 248 | 31.689 | -77.513 | -21.045 | 1.00180.41 |
| ATOM | 3999 | CZ | PHE | E | 248 | 32.427 | -76.981 | -20.029 | 1.00178.75 |
| ATOM | 4000 | N | GLN | E | 249 | 27.751 | -76.920 | -18.724 | 1.00173.25 |
| ATOM | 4001 | CA | GLN | E | 249 | 27.217 | -75.708 | -19.365 | 1.00172.30 |
| ATOM | 4002 | C | GLN | E | 249 | 25.660 | -75.772 | -19.522 | 1.00174.09 |
| ATOM | 4003 | O | GLN | E | 249 | 25.060 | -75.164 | -20.407 | 1.00171.77 |
| ATOM | 4004 | CB | GLN | E | 249 | 27.971 | -75.540 | -20.705 | 1.00175.01 |
| ATOM | 4005 | CG | GLN | E | 249 | 27.918 | -74.230 | -21.454 | 1.00212.46 |
| ATOM | 4006 | CD | GLN | E | 249 | 28.384 | -72.999 | -20.728 | 1.00242.40 |
| ATOM | 4007 | OE1 | GLN | E | 249 | 29.587 | -72.741 | -20.584 | 1.00240.03 |
| ATOM | 4008 | NE2 | GLN | E | 249 | 27.418 | -72.132 | -20.422 | 1.00232.89 |
| ATOM | 4009 | N | ASP | E | 250 | 25.030 | -76.530 | -18.635 | 1.00171.84 |
| ATOM | 4010 | CA | ASP | E | 250 | 23.605 | -76.765 | -18.636 | 1.00171.66 |
| ATOM | 4011 | C | ASP | E | 250 | 22.980 | -77.295 | -19.965 | 1.00174.25 |
| ATOM | 4012 | O | ASP | E | 250 | 21.773 | -77.114 | -20.211 | 1.00174.56 |
| ATOM | 4013 | CB | ASP | E | 250 | 22.834 | -75.618 | -17.976 | 1.00173.92 |
| ATOM | 4014 | CG | ASP | E | 250 | 21.608 | -76.123 | -17.221 | 1.00200.91 |
| ATOM | 4015 | OD1 | ASP | E | 250 | 21.786 | -76.860 | -16.201 | 1.00202.99 |
| ATOM | 4016 | OD2 | ASP | E | 250 | 20.469 | -75.833 | -17.672 | 1.00215.48 |
| ATOM | 4017 | N | TRP | E | 251 | 23.781 | -78.033 | -20.776 | 1.00168.06 |
| ATOM | 4018 | CA | TRP | E | 251 | 23.271 | -78.643 | -22.009 | 1.00166.25 |
| ATOM | 4019 | C | TRP | E | 251 | 23.944 | -79.943 | -22.441 | 1.00173.07 |
| ATOM | 4020 | O | TRP | E | 251 | 23.356 | -80.646 | -23.263 | 1.00175.39 |
| ATOM | 4021 | CB | TRP | E | 251 | 23.284 | -77.644 | -23.156 | 1.00163.41 |
| ATOM | 4022 | CG | TRP | E | 251 | 24.616 | -77.534 | -23.776 | 1.00165.28 |
| ATOM | 4023 | CD1 | TRP | E | 251 | 25.696 | -76.885 | -23.272 | 1.00168.50 |
| ATOM | 4024 | CD2 | TRP | E | 251 | 25.063 | -78.217 | -24.948 | 1.00166.95 |
| ATOM | 4025 | NE1 | TRP | E | 251 | 26.782 | -77.077 | -24.087 | 1.00170.39 |
| ATOM | 4026 | CE2 | TRP | E | 251 | 26.418 | -77.884 | -25.134 | 1.00172.74 |
| ATOM | 4027 | CE3 | TRP | E | 251 | 24.443 | -79.062 | -25.881 | 1.00169.04 |
| ATOM | 4028 | CZ2 | TRP | E | 251 | 27.165 | -78.362 | -26.219 | 1.00174.17 |
| ATOM | 4029 | CZ3 | TRP | E | 251 | 25.184 | -79.533 | -26.957 | 1.00172.54 |
| ATOM | 4030 | CH2 | TRP | E | 251 | 26.529 | -79.189 | -27.113 | 1.00174.62 |
| ATOM | 4031 | N | ARG | E | 252 | 25.186 | -80.237 | -21.980 | 1.00168.79 |
| ATOM | 4032 | CA | ARG | E | 252 | 25.840 | -81.492 | -22.374 | 1.00170.03 |
| ATOM | 4033 | C | ARG | E | 252 | 26.469 | -82.315 | -21.262 | 1.0017.8.09 |
| ATOM | 4034 | O | ARG | E | 252 | 26.754 | -81.791 | -20.188 | 1.00178.84 |
| ATOM | 4035 | CB | ARG | E | 252 | 26.796 | -81.308 | -23.544 | 1.00167.70 |
| ATOM | 4036 | CG | ARG | E | 252 | 27.994 | -80.487 | -23.234 | 1.00171.56 |
| ATOM | 4037 | CD | ARG | E | 252 | 29.021 | -80.721 | -24.302 | 1.00183.53 |
| ATOM | 4038 | NE | ARG | E | 252 | 30.069 | -79.699 | -24.295 | 1.00187.68 |
| ATOM | 4039 | CZ | ARG | E | 252 | 31.184 | -79.756 | -25.024 | 1.00199.10 |
| ATOM | 4040 | NH1 | ARG | E | 252 | 31.386 | -80.764 | -25.865 | 1.00186.77 |
| ATOM | 4041 | NH2 | ARG | E | 252 | 32.100 | -78.799 | -24.925 | 1.00181.29 |
| ATOM | 4042 | N | CYS | E | 253 | 26.669 | -83.626 | -21.526 | 1.00176.45 |
| ATOM | 4043 | CA | CYS | E | 253 | 27.288 | -84.589 | -20.595 | 1.00177.27 |
| ATOM | 4044 | C | CYS | E | 253 | 28.687 | -84.805 | -20.954 | 1.00183.35 |
| ATOM | 4045 | O | CYS | E | 253 | 28.994 | -85.060 | -22.117 | 1.00184.02 |
| ATOM | 4046 | CB | CYS | E | 253 | 26.548 | -85.910 | -20.583 | 1.00178.03 |
| ATOM | 4047 | SG | CYS | E | 253 | 24.807 | -85.752 | -20.194 | 1.00178.88 |
| ATOM | 4048 | N | VAL | E | 254 | 29.554 | -84.701 | -19.964 | 1.00181.36 |
| ATOM | 4049 | CA | VAL | E | 254 | 30.980 | -84.870 | -20.183 | 1.00184.69 |
| ATOM | 4050 | C | VAL | E | 254 | 31.527 | -85.816 | -19.096 | 1.00190.53 |
| ATOM | 4051 | O | VAL | E | 254 | 30.858 | -86.040 | -18.088 | 1.00189.11 |
| ATOM | 4052 | CB | VAL | E | 254 | 31.720 | -83.495 | -20.233 | 1.00188.69 |
| ATOM | 4053 | CG1 | VAL | E | 254 | 31.212 | -82.589 | -21.372 | 1.00186.90 |
| ATOM | 4054 | CG2 | VAL | E | 254 | 31.643 | -82.770 | -18.887 | 1.00187.36 |
| ATOM | 4055 | N | ASN | E | 255 | 32.723 | -86.388 | -19.311 | 1.00189.58 |
| ATOM | 4056 | CA | ASN | E | 255 | 33.352 | -87.278 | -18.324 | 1.00191.47 |
| ATOM | 4057 | C | ASN | E | 255 | 34.287 | -86.480 | -17.415 | 1.00195.05 |
| ATOM | 4058 | O | ASN | E | 255 | 34.640 | -85.325 | -17.713 | 1.00194.41 |
| ATOM | 4059 | CB | ASN | E | 255 | 34.103 | -88.461 | -18.995 | 1.00195.05 |
| ATOM | 4060 | CG | ASN | E | 255 | 35.103 | -87.988 | -20.011 | 1.00225.86 |
| ATOM | 4061 | OD1 | ASN | E | 255 | 35.831 | -87.015 | -19.779 | 1.00226.15 |
| ATOM | 4062 | ND2 | ASN | E | 255 | 35.139 | -88.582 | -21.197 | 1.00221.41 |
| ATOM | 4063 | N | PHE | E | 256 | 34.721 | -87.107 | -16.331 | 1.00192.04 |
| ATOM | 4064 | CA | PHE | E | 256 | 35.630 | -86.449 | -15.410 | 1.00192.52 |
| ATOM | 4065 | C | PHE | E | 256 | 36.881 | -85.874 | -16.101, | 1.00197.04 |
| ATOM | 4066 | O | PHE | E | 256 | 37.316 | -84.764 | -15.799 | 1.00194.58 |
| ATOM | 4067 | CB | PHE | E | 256 | 36.040 | -87.424 | -14.304 | 1.00197.52 |
| ATOM | 4068 | CG | PHE | E | 256 | 37.098 | -86.838 | -13.411 | 1.00200.33 |
| ATOM | 4069 | CD1 | PHE | E | 256 | 36.777 | -85.858 | -12.480 | 1.00200.43 |
| ATOM | 4070 | CD2 | PHE | E | 256 | 38.428 | -87.189 | -13.568 | 1.00206.43 |
| ATOM | 4071 | CE1 | PHE | E | 256 | 37.759 | -85.272 | -11.700 | 1.00203.02 |
| ATOM | 4072 | CE2 | PHE | E | 256 | 39.405 | -86.614 | -12.776 | 1.00211.12 |
| ATOM | 4073 | CZ | PHE | E | 256 | 39.068 | -85.650 | -11.859 | 1.00206.77 |
| ATOM | 4074 | N | SER | E | 257 | 37.456 | -86.656 | -17.005 | 1.00196.92 |
| ATOM | 4075 | CA | SER | E | 257 | 38.682 | -86.334 | -17.705 | 1.00199.31 |
| ATOM | 4076 | C | SER | E | 257 | 38.568 | -85.023 | -18.490 | 1.00200.87 |
| ATOM | 4077 | O | SER | E | 257 | 39.512 | -84.241 | -18.510 | 1.00202.11 |
| ATOM | 4078 | CB | SER | E | 257 | 39.086 | -87.513 | -18.586 | 1.00205.82 |
| ATOM | 4079 | OG | SER | E | 257 | 39.990 | -87.157 | -19.616 | 1.00215.57 |
| ATOM | 4080 | N | PHE | E | 258 | 37.417 | -84.781 | -19.107 | 1.00194.67 |
| ATOM | 4081 | CA | PHE | E | 258 | 37.145 | -83.573 | -19.863 | 1.00193.56 |
| ATOM | 4082 | C | PHE | E | 258 | 37.149 | -82.383 | -18.925 | 1.00196.85 |
| ATOM | 4083 | O | PHE | E | 258 | 37.763 | -81.360 | -19.242 | 1.00197.66 |
| ATOM | 4084 | CB | PHE | E | 258 | 35.769 | -83.687 | -20.533 | 1.00193.47 |
| ATOM | 4085 | CG | PHE | E | 258 | 35.248 | -82.433 | -21.221 | 1.00193.05 |
| ATOM | 4086 | CD1 | PHE | E | 258 | 35.357 | -82.279 | -22.598 | 1.00198.11 |
| ATOM | 4087 | CD2 | PHE | E | 258 | 34.607 | -81.430 | -20.493 | 1.00192.08 |
| ATOM | 4088 | CE1 | PHE | E | 258 | 34.872 | -81.125 | -23.230 | 1.00197.04 |
| ATOM | 4089 | CE2 | PHE | E | 258 | 34.107 | -80.286 | -21.126 | 1.00192.93 |
| ATOM | 4090 | CZ | PHE | E | 258 | 34.245 | -80.137 | -22.487 | 1.00192.43 |
| ATOM | 4091 | N | CYS | E | 259 | 36.441 | -82.507 | -17.780 | 1.00191.14 |
| ATOM | 4092 | CA | CYS | E | 259 | 36.347 | -81.442 | -16.785 | 1.00189.04 |
| ATOM | 4093 | C | CYS | E | 259 | 37.716 | -81.114 | -16.228 | 1.00195.62 |
| ATOM | 4094 | O | CYS | E | 259 | 38.018 | -79.953 | -15.933 | 1.00193.72 |
| ATOM | 4095 | CB | CYS | E | 259 | 35.387 | -81.839 | -15.674 | 1.00188.02 |
| ATOM | 4096 | SG | CYS | E | 259 | 34.884 | -80.461 | -14.613 | 1.00189.48 |
| ATOM | 4097 | N | GLN | E | 260 | 38.538 | -82.159 | -16.079 | 1.00196.22 |
| ATOM | 4098 | CA | GLN | E | 260 | 39.896 | -82.059 | -15.569 | 1.00198.96 |
| ATOM | 4099 | C | GLN | E | 260 | 40.800 | -81.343 | -16.545 | 1.00203.73 |
| ATOM | 4100 | O | GLN | E | 260 | 41.579 | -80.476 | -16.141 | 1.00203.71 |
| ATOM | 4101 | CB | GLN | E | 260 | 40.425 | -83.448 | -15.247 | 1.00203.52 |
| ATOM | 4102 | CG | GLN | E | 260 | 41.820 | -83.432 | -14.727 | 1.00210.19 |
| ATOM | 4103 | CD | GLN | E | 260 | 42.046 | -84.671 | -13.967 | 1.00235.24 |
| ATOM | 4104 | OE1 | GLN | E | 260 | 42.017 | -84.640 | -12.741 | 1.00232.48 |
| ATOM | 4105 | NE2 | GLN | E | 260 | 42.127 | -85.808 | -14.653 | 1.00230.43 |
| ATOM | 4106 | N | ASP | E | 261 | 40.693 | -81.700 | -17.824 | 1.00201.21 |
| ATOM | 4107 | CA | ASP | E | 261 | 41.476 | -81.068 | -18.874 | 1.00202.91 |
| ATOM | 4108 | C | ASP | E | 261 | 41.196 | -79.563 | -18.954 | 1.00202.01 |
| ATOM | 4109 | O | ASP | E | 261 | 42.128 | -78.776 | -19.123 | 1.00203.66 |
| ATOM | 4110 | CB | ASP | E | 261 | 41.274 | -81.795 | -20.219 | 1.00206.74 |
| ATOM | 4111 | CG | ASP | E | 261 | 41.890 | -83.205 | -20.304 | 1.00228.81 |
| ATOM | 4112 | OD1 | ASP | E | 261 | 42.574 | -83.631 | -19.331 | 1.00232.23 |
| ATOM | 4113 | OD2 | ASP | E | 261 | 41.684 | -83.885 | -21.338 | 1.00239.48 |
| ATOM | 4114 | N | LEU | E | 262 | 39.935 | -79.166 | -18.769 | 1.00193.04 |
| ATOM | 4115 | CA | LEU | E | 262 | 39.545 | -77.759 | -18.762 | 1.00190.09 |
| ATOM | 4116 | C | LEU | E | 262 | 40.206 | -77.017 | -17.621 | 1.00196.98 |
| ATOM | 4117 | O | LEU | E | 262 | 40.779 | -75.938 | -17.810 | 1.00196.58 |
| ATOM | 4118 | CB | LEU | E | 262 | 38.039 | -77.651 | -18.585 | 1.00185.89 |
| ATOM | 4119 | CG | LEU | E | 262 | 37.254 | -77.483 | -19.842 | 1.00188.37 |
| ATOM | 4120 | CD1 | LEU | E | 262 | 37.318 | -78.696 | -20.776 | 1.00190.26 |
| ATOM | 4121 | CD2 | LEU | E | 262 | 35.888 | -77.000 | -19.522 | 1.00187.72 |
| ATOM | 4122 | N | HIS | E | 263 | 40.101 | -77.603 | -16.430 | 1.00196.60 |
| ATOM | 4123 | CA | HIS | E | 263 | 40.650 | -77.059 | -15.201 | 1.00198.83 |
| ATOM | 4124 | C | HIS | E | 263 | 42.129 | -76.750 | -15.316 | 1.00210.08 |
| ATOM | 4125 | O | HIS | E | 263 | 42.562 | -75.667 | -14.907 | 1.00211.45 |
| ATOM | 4126 | CB | HIS | E | 263 | 40.425 | -78.037 | -14.046 | 1.00200.25 |
| ATOM | 4127 | CG | HIS | E | 263 | 41.020 | -77.545 | -12.771 | 1.00205.01 |
| ATOM | 4128 | ND1 | HIS | E | 263 | 42.340 | -77.810 | -12.448 | 1.00210.62 |
| ATOM | 4129 | CD2 | HIS | E | 263 | 40.482 | -76.759 | -11.813 | 1.00204.98 |
| ATOM | 4130 | CE1 | HIS | E | 263 | 42.550 | -77.204 | -11.293 | 1.00210.66 |
| ATOM | 4131 | NE2 | HIS | E | 263 | 41.464 | -76.548 | -10.880 | 1.00207.70 |
| ATOM | 4132 | N | HIS | E | 264 | 42.899 | -77.700 | -15.874 | 1.00210.08 |
| ATOM | 4133 | CA | HIS | E | 264 | 44.334 | -77.564 | -16.006 | 1.00213.83 |
| ATOM | 4134 | C | HIS | E | 264 | 44.745 | -76.516 | -17.015 | 1.00215.60 |
| ATOM | 4135 | O | HIS | E | 264 | 44.679 | -76.693 | -18.232 | 1.00214.86 |
| ATOM | 4136 | CB | HIS | E | 264 | 45.026 | -78.908 | -15.998 | 1.00219.06 |
| ATOM | 4137 | CG | HIS | E | 264 | 44.974 | -79.534 | -14.624 | 1.00224.14 |
| ATOM | 4138 | ND1 | HIS | E | 264 | 44.109 | -80.583 | -14.325 | 1.00225.26 |
| ATOM | 4139 | CD2 | HIS | E | 264 | 45.647 | -79.199 | -13.494 | 1.00228.36 |
| ATOM | 4140 | CE1 | HIS | E | 264 | 44.321 | -80.877 | -13.047 | 1.00226.38 |
| ATOM | 4141 | NE2 | HIS | E | 264 | 45.233 | -80.070 | -12.503 | 1.00228.58 |
| ATOM | 4142 | N | LYS | E | 265 | 44.952 | -75.325 | -16.420 | 1.00210.25 |
| ATOM | 4143 | CA | LYS | E | 265 | 45.170 | -74.012 | -16.996 | 1.00212.48 |
| ATOM | 4144 | C | LYS | E | 265 | 44.014 | -73.568 | -17.860 | 1.00204.27 |
| ATOM | 4145 | O | LYS | E | 265 | 42.966 | -73.290 | -17.286 | 1.00158.06 |
| ATOM | 4146 | CB | LYS | E | 265 | 46.562 | -73.780 | -17.597 | 1.00218.46 |
| ATOM | 4147 | CG | LYS | E | 265 | 47.391 | -72.837 | -16.716 | 1.00216.10 |
| ATOM | 4148 | CD | LYS | E | 265 | 48.501 | -72.077 | -17.458 | 1.00214.34 |
| ATOM | 4149 | CE | LYS | E | 265 | 48.035 | -70.937 | -18.355 | 1.00208.08 |
| ATOM | 4150 | NZ | LYS | E | 265 | 47.450 | -69.785 | -17.608 | 1.00204.39 |
| ATOM | 4151 | N | GLN | E | 276 | 38.818 | -72.227 | -13.908 | 1.00191.28 |
| ATOM | 4152 | CA | GLN | E | 276 | 39.360 | -73.495 | -13.359 | 1.00193.77 |
| ATOM | 4153 | C | GLN | E | 276 | 38.189 | -74.323 | -12.838 | 1.00193.67 |
| ATOM | 4154 | O | GLN | E | 276 | 37.746 | -74.132 | -11.701 | 1.00193.21 |
| ATOM | 4155 | CB | GLN | E | 276 | 40.375 | -73.253 | -12.211 | 1.00198.53 |
| ATOM | 4156 | CG | GLN | E | 276 | 41.429 | -72.154 | -12.456 | 1.00218.34 |
| ATOM | 4157 | CD | GLN | E | 276 | 41.875 | -71.447 | -11.185 | 1.00233.70 |
| ATOM | 4158 | OE1 | GLN | E | 276 | 41.585 | -70.260 | -10.974 | 1.00225.94 |
| ATOM | 4159 | NE2 | GLN | E | 276 | 42.624 | -72.138 | -10.330 | 1.00225.95 |
| ATOM | 4160 | N | TYR | E | 277 | 37.671 | -75.217 | -13.698 | 1.00186.44 |
| ATOM | 4161 | CA | TYR | E | 277 | 36.472 | -76.022 | -13.470 | 1.00182.61 |
| ATOM | 4162 | C | TYR | E | 277 | 36.538 | -76.980 | -12.316 | 1.00184.61 |
| ATOM | 4163 | O | TYR | E | 277 | 37.590 | -77.543 | -12.025 | 1.00187.17 |
| ATOM | 4164 | CB | TYR | E | 277 | 36.032 | -76.708 | -14.756 | 1.00182.96 |
| ATOM | 4165 | CG | TYR | E | 277 | 35.346 | -75.747 | -15.691 | 1.00183.67 |
| ATOM | 4166 | CD2 | TYR | E | 277 | 33.962 | -75.703 | -15.780 | 1.00183.09 |
| ATOM | 4167 | CD1 | TYR | E | 277 | 36.078 | -74.856 | -16.470 | 1.00186.37 |
| ATOM | 4168 | CE2 | TYR | E | 277 | 33.316 | -74.784 | -16.612 | 1.00182.67 |
| ATOM | 4169 | CE1 | TYR | E | 277 | 35.446 | -73.936 | -17.312 | 1.00186.04 |
| ATOM | 4170 | CZ | TYR | E | 277 | 34.061 | -73.906 | -17.386 | 1.00190.12 |
| ATOM | 4171 | OH | TYR | E | 277 | 33.417 | -73.015 | -18.224 | 1.00186.10 |
| ATOM | 4172 | N | VAL | E | 278 | 35.413 | -77.137 | -11.629 | 1.00176.19 |
| ATOM | 4173 | CA | VAL | E | 278 | 35.323 | -77.984 | -10.450 | 1.00175.95 |
| ATOM | 4174 | C | VAL | E | 278 | 34.146 | -78.932 | -10.576 | 1.00178.94 |
| ATOM | 4175 | O | VAL | E | 278 | 33.281 | -78.736 | -11.417 | 1.00175.49 |
| ATOM | 4176 | CB | VAL | E | 278 | 35.218 | -77.125 | -9.168 | 1.00178.67 |
| ATOM | 4177 | CG1 | VAL | E | 278 | 36.378 | -76.158 | -9.068 | 1.00179.61 |
| ATOM | 4178 | CG2 | VAL | E | 278 | 33.918 | -76.351 | -9.126 | 1.00175.44 |
| ATOM | 4179 | N | ILE | E | 279 | 34.109 | -79.950 | -9.741 | 1.00178.71 |
| ATOM | 4180 | CA | ILE | E | 279 | 32.983 | -80.854 | -9.704 | 1.00177.96 |
| ATOM | 4181 | C | ILE | E | 279 | 32.185 | -80.521 | -8.481 | 1.00180.61 |
| ATOM | 4182 | O | ILE | E | 279 | 32.747 | -80.374 | -7.387 | 1.00182.45 |
| ATOM | 4183 | CB | ILE | E | 279 | 33.424 | -82.324 | -9.738 | 1.00184.55 |
| ATOM | 4184 | CG1 | ILE | E | 279 | 33.929 | -82.646 | -11.169 | 1.00185.33 |
| ATOM | 4185 | CG2 | ILE | E | 279 | 32.228 | -83.245 | -9.409 | 1.00185.19 |
| ATOM | 4186 | CD1 | ILE | E | 279 | 35.330 | -82.731 | -11.374 | 1.00195.08 |
| ATOM | 4187 | N | HIS | E | 280 | 30.875 | -80.395 | -8.667 | 1.00174.27 |
| ATOM | 4188 | CA | HIS | E | 280 | 29.936 | -80.169 | -7.585 | 1.00174.67 |
| ATOM | 4189 | C | HIS | E | 280 | 28.530 | -80.682 | -7.960 | 1.00178.26 |
| ATOM | 4190 | O | HIS | E | 280 | 28.011 | -80.308 | -9.018 | 1.00175.61 |
| ATOM | 4191 | CB | HIS | E | 280 | 29.897 | -78.697 | -7.165 | 1.00174.85 |
| ATOM | 4192 | CG | HIS | E | 280 | 28.883 | -78.417 | -6.086 | 1.00178.88 |
| ATOM | 4193 | ND1 | HIS | E | 280 | 29.041 | -78.914 | -4.799 | 1.00183.74 |
| ATOM | 4194 | CD2 | HIS | E | 280 | 27.723 | -77.706 | -6.139 | 1.00178.62 |
| ATOM | 4195 | CE1 | HIS | E | 280 | 27.997 | -78.475 | -4.111 | 1.00182.69 |
| ATOM | 4196 | NE2 | HIS | E | 280 | 27.175 | -77.743 | -4.873 | 1.00179.98 |
| ATOM | 4197 | N | ASN | E | 281 | 27.909 | -81.538 | -7.091 | 1.00176.15 |
| ATOM | 4198 | CA | ASN | E | 281 | 26.556 | -82.079 | -7.300 | 1.00174.31 |
| ATOM | 4199 | C | ASN | E | 281 | 26.382 | -82.688 | -8.722 | 1.00178.01 |
| ATOM | 4200 | O | ASN | E | 281 | 25.375 | -82.431 | -9.356 | 1.00176.37 |
| ATOM | 4201 | CB | ASN | E | 281 | 25.533 | -80.934 | -7.067 | 1.00170.92 |
| ATOM | 4202 | CG | ASN | E | 281 | 24.185 | -81.288 | -6.491 | 1.00201.33 |
| ATOM | 4203 | OD1 | ASN | E | 281 | 23.125 | -80.951 | -7.060 | 1.00189.94 |
| ATOM | 4204 | ND2 | ASN | E | 281 | 24.194 | -81.867 | -5.293 | 1.00198.64 |
| ATOM | 4205 | N | ASN | E | 282 | 27.368 | -83.445 | -9.236 | 1.00175.95 |
| ATOM | 4206 | CA | ASN | E | 282 | 27.313 | -84.044 | -10.583 | 1.00174.61 |
| ATOM | 4207 | C | ASN | E | 282 | 27.289 | -83.057 | -11.779 | 1.00172.46 |
| ATOM | 4208 | O | ASN | E | 282 | 26.823 | -83.359 | -12.888 | 1.00169.71 |
| ATOM | 4209 | CB | ASN | E | 282 | 26.276 | -85.140 | -10.652 | 1.00177.32 |
| ATOM | 4210 | CG | ASN | E | 282 | 26.653 | -86.293 | -9.757 | 1.00199.67 |
| ATOM | 4211 | OD1 | ASN | E | 282 | 26.041 | -86.507 | -8.709 | 1.00198.11 |
| ATOM | 4212 | ND2 | ASN | E | 282 | 27.675 | -87.066 | -10.152 | 1.00186.89 |
| ATOM | 4213 | N | LYS | E | 283 | 27.864 | -81.889 | -11.526 | 1.00166.53 |
| ATOM | 4214 | CA | LYS | E | 283 | 28.019 | -80.843 | -12.498 | 1.00163.92 |
| ATOM | 4215 | C | LYS | E | 283 | 29.478 | -80.479 | -12.556 | 1.00172.36 |
| ATOM | 4216 | O | LYS | E | 283 | 30.195 | -80.562 | -11.551 | 1.00173.83 |
| ATOM | 4217 | CB | LYS | E | 283 | 27.208 | -79.610 | -12.100 | 1.00163.61 |
| ATOM | 4218 | CG | LYS | E | 283 | 25.715 | -79.854 | -11.864 | 1.00178.24 |
| ATOM | 4219 | CD | LYS | E | 283 | 25.133 | -79.062 | -10.645 | 1.00180.11 |
| ATOM | 4220 | CE | LYS | E | 283 | 24.870 | -77.611 | -10.914 | 1.00170.16 |
| ATOM | 4221 | NZ | LYS | E | 283 | 24.505 | -76.911 | -9.658 | 1.00170.88 |
| ATOM | 4222 | N | CYS | E | 284 | 29.936 | -80.122 | -13.750 | 1.00171.63 |
| ATOM | 4223 | CA | CYS | E | 284 | 31.280 | -79.604 | -13.969 | 1.00174.37 |
| ATOM | 4224 | C | CYS | E | 284 | 31.036 | -78.083 | -14.085 | 1.00172.81 |
| ATOM | 4225 | O | CYS | E | 284 | 30.343 | -77.592 | -14.975 | 1.00170.16 |
| ATOM | 4226 | CB | CYS | E | 284 | 31.900 | -80.212 | -15.219 | 1.00177.52 |
| ATOM | 4227 | SG | CYS | E | 284 | 33.468 | -79.478 | -15.699 | 1.00184.66 |
| ATOM | 4228 | N | ILE | E | 285 | 31.514 | -77.360 | -13.110 | 1.00167.81 |
| ATOM | 4229 | CA | ILE | E | 285 | 31.151 | -75.983 | -12.891 | 1.00165.88 |
| ATOM | 4230 | C | ILE | E | 285 | 32.343 | -75.039 | -12.869 | 1.00173.87 |
| ATOM | 4231 | O | ILE | E | 285 | 33.439 | -75.431 | -12.442 | 1.00175.84 |
| ATOM | 4232 | CB | ILE | E | 285 | 30.439 | -76.100 | -11.509 | 1.00168.65 |
| ATOM | 4233 | CG1 | ILE | E | 285 | 29.599 | -74.938 | -11.039 | 1.00167.76 |
| ATOM | 4234 | CG2 | ILE | E | 285 | 31.313 | -76.658 | -10.386 | 1.00171.28 |
| ATOM | 4235 | CD1 | ILE | E | 285 | 29.100 | -75.205 | -9.547 | 1.00169.44 |
| ATOM | 4236 | N | PRO | E | 286 | 32.141 | -73.775 | -13.303 | 1.00169.87 |
| ATOM | 4237 | CA | PRO | E | 286 | 33.255 | -72.821 | -13.306 | 1.00171.04 |
| ATOM | 4238 | C | PRO | E | 286 | 33.909 | -72.619 | -11.948 | 1.00177.18 |
| ATOM | 4239 | O | PRO | E | 286 | 35.132 | -72.598 | -11.869 | 1.00178.90 |
| ATOM | 4240 | CB | PRO | E | 286 | 32.606 | -71.533 | -13.800 | 1.00171.25 |
| ATOM | 4241 | CG | PRO | E | 286 | 31.413 | -71.988 | -14.565 | 1.00173.71 |
| ATOM | 4242 | CD | PRO | E | 286 | 30.904 | -73.162 | -13.832 | 1.00168.94 |
| ATOM | 4243 | N | GLU | E | 287 | 33.086 | -72.536 | -10.886 | 1.00175.35 |
| ATOM | 4244 | CA | GLU | E | 287 | 33.483 | -72.227 | -9.524 | 1.00179.30 |
| ATOM | 4245 | C | GLU | E | 287 | 32.684 | -72.941 | -8.477 | 1.00185.14 |
| ATOM | 4246 | O | GLU | E | 287 | 31.479 | -73.114 | -8.642 | 1.00183.17 |
| ATOM | 4247 | CB | GLU | E | 287 | 33.149 | -70.743 | -9.329 | 1.00181.09 |
| ATOM | 4248 | CG | GLU | E | 287 | 33.802 | -70.009 | -8.169 | 1.00204.13 |
| ATOM | 4249 | CD | GLU | E | 287 | 33.543 | -68.513 | -8.237 | 1.00236.59 |
| ATOM | 4250 | OE1 | GLU | E | 287 | 32.369 | -68.113 | -8.051 | 1.00235.43 |
| ATOM | 4251 | OE2 | GLU | E | 287 | 34.501 | -67.743 | -8.497 | 1.00228.91 |
| ATOM | 4252 | N | CYS | E | 288 | 33.319 | -73.190 | -7.323 | 1.00185.23 |
| ATOM | 4253 | CA | CYS | E | 288 | 32.673 | -73.748 | -6.145 | 1.00186.51 |
| ATOM | 4254 | C | CYS | E | 288 | 31.626 | -72.785 | -5.637 | 1.00192.78 |
| ATOM | 4255 | O | CYS | E | 288 | 31.913 | -71.589 | -5.540 | 1.00194.38 |
| ATOM | 4256 | CB | CYS | E | 288 | 33.701 | -73.995 | -5.052 | 1.00189.71 |
| ATOM | 4257 | SG | CYS | E | 288 | 34.482 | -75.631 | -5.092 | 1.00195.13 |
| ATOM | 4258 | N | PRO | E | 289 | 30.459 | -73.289 | -5.208 | 1.00188.79 |
| ATOM | 4259 | CA | PRO | E | 289 | 29.454 | -72.410 | -4.617 | 1.00188.26 |
| ATOM | 4260 | C | PRO | E | 289 | 29.840 | -72.031 | -3.189 | 1.00195.65 |
| ATOM | 4261 | O | PRO | E | 289 | 30.821 | -72.543 | -2.630 | 1.00196.23 |
| ATOM | 4262 | CB | PRO | E | 289 | 28.189 | -73.258 | -4.645 | 1.00188.54 |
| ATOM | 4263 | CG | PRO | E | 289 | 28.670 | -74.618 | -4.528 | 1.00194.14 |
| ATOM | 4264 | CD | PRO | E | 289 | 29.982 | -74.678 | -5.255 | 1.00190.12 |
| ATOM | 4265 | N | SER | E | 290 | 29.055 | -71.117 | -2.612 | 1.00194.08 |
| ATOM | 4266 | CA | SER | E | 290 | 29.224 | -70.601 | -1.266 | 1.00197.22 |
| ATOM | 4267 | C | SER | E | 290 | 29.125 | -71.737 | -0.249 | 1.00202.02 |
| ATOM | 4268 | O | SER | E | 290 | 28.219 | -72.573 | -0.338 | 1.00199.64 |
| ATOM | 4269 | CB | SER | E | 290 | 28.168 | -69.533 | -0.992 | 1.00201.26 |
| ATOM | 4270 | OG | SER | E | 290 | 27.960 | -68.703 | -2.129 | 1.00206.63 |
| ATOM | 4271 | N | GLY | E | 291 | 30.084 | -71.753 | 0.673 | 1.00201.64 |
| ATOM | 4272 | CA | GLY | E | 291 | 30.202 | -72.738 | 1.744 | 1.00204.28 |
| ATOM | 4273 | C | GLY | E | 291 | 31.189 | -73.842 | 1.441 | 1.00208.32 |
| ATOM | 4274 | O | GLY | E | 291 | 31.483 | -74.683 | 2.304 | 1.00210.27 |
| ATOM | 4275 | N | TYR | E | 292 | 31.727 | -73.815 | 0.212 | 1.00202.91 |
| ATOM | 4276 | CA | TYR | E | 292 | 32.633 | -74.827 | -0.302 | 1.00203.52 |
| ATOM | 4277 | C | TYR | E | 292 | 33.926 | -74.254 | -0.829 | 1.00210.70 |
| ATOM | 4278 | O | TYR | E | 292 | 33.974 | -73.088 | -1.227 | 1.00210.31 |
| ATOM | 4279 | CB | TYR | E | 292 | 31.954 | -75.561 | -1.462 | 1.00200.66 |
| ATOM | 4280 | CG | TYR | E | 292 | 30.752 | -76.382 | -1.071 | 1.00200.53 |
| ATOM | 4281 | CD1 | TYR | E | 292 | 30.894 | -77.700 | -0.668 | 1.00204.04 |
| ATOM | 4282 | CD2 | TYR | E | 292 | 29.463 | -75.891 | -1.244 | 1.00198.37 |
| ATOM | 4283 | CE1 | TYR | E | 292 | 29.799 | -78.468 | -0.321 | 1.00204.00 |
| ATOM | 4284 | CE2 | TYR | E | 292 | 28.355 | -76.653 | -0.902 | 1.00198.68 |
| ATOM | 4285 | CZ | TYR | E | 292 | 28.533 | -77.941 | -0.434 | 1.00207.73 |
| ATOM | 4286 | OH | TYR | E | 292 | 27.471 | -78.733 | -0.102 | 1.00211.91 |
| ATOM | 4287 | N | THR | E | 293 | 34.979 | -75.090 | -0.826 | 1.00209.62 |
| ATOM | 4288 | CA | THR | E | 293 | 36.283 | -74.817 | -1.399 | 1.00210.52 |
| ATOM | 4289 | C | THR | E | 293 | 36.746 | -76.039 | -2.123 | 1.00215.50 |
| ATOM | 4290 | O | THR | E | 293 | 36.247 | -77.141 | -1.876 | 1.00214.03 |
| ATOM | 4291 | CB | THR | E | 293 | 37.215 | -74.011 | -0.490 | 1.00221.76 |
| ATOM | 4292 | OG1 | THR | E | 293 | 37.439 | -72.739 | -1.109 | 1.00217.80 |
| ATOM | 4293 | CG2 | THR | E | 293 | 38.534 | -74.711 | -0.156 | 1.00225.39 |
| ATOM | 4294 | N | MET | E | 294 | 37.655 | -75.837 | -3.055 | 1.00214.96 |
| ATOM | 4295 | CA | MET | E | 294 | 38.078 | -76.899 | -3.914 | 1.00216.78 |
| ATOM | 4296 | C | MET | E | 294 | 39.265 | -77.742 | -3.480 | 1.00228.86 |
| ATOM | 4297 | O | MET | E | 294 | 40.423 | -77.285 | -3.527 | 1.00230.42 |
| ATOM | 4298 | CB | MET | E | 294 | 38.253 | -76.356 | -5.322 | 1.00216.89 |
| ATOM | 4299 | CG | MET | E | 294 | 38.077 | -77.403 | -6.379 | 1.00220.00 |
| ATOM | 4300 | SD | MET | E | 294 | 39.633 | -78.117 | -6.943 | 1.00228.32 |
| ATOM | 4301 | CE | MET | E | 294 | 40.351 | -76.741 | -7.795 | 1.00224.44 |
| ATOM | 4302 | N | ASN | E | 295 | 38.951 | -79.013 | -3.095 | 1.00229.33 |
| ATOM | 4303 | CA | ASN | E | 295 | 39.923 | -80.081 | -2.812 | 1.00233.73 |
| ATOM | 4304 | C | ASN | E | 295 | 40.658 | -80.256 | -4.133 | 1.00238.00 |
| ATOM | 4305 | O | ASN | E | 295 | 40.037 | -80.467 | -5.184 | 1.00235.69 |
| ATOM | 4306 | CB | ASN | E | 295 | 39.237 | -81.384 | -2.402 | 1.00235.01 |
| ATOM | 4307 | CG | ASN | E | 295 | 40.201 | -82.395 | -1.797 | 1.00255.03 |
| ATOM | 4308 | OD1 | ASN | E | 295 | 40.748 | -82.218 | -0.695 | 1.00248.32 |
| ATOM | 4309 | ND2 | ASN | E | 295 | 40.430 | -83.483 | -2.508 | 1.00246.60 |
| ATOM | 4310 | N | SER | E | 296 | 41.975 | -80.078 | -4.077 | 1.00236.65 |
| ATOM | 4311 | CA | SER | E | 296 | 42.835 | -79.925 | -5.236 | 1.00236.02 |
| ATOM | 4312 | C | SER | E | 296 | 43.133 | -81.164 | -6.067 | 1.00238.24 |
| ATOM | 4313 | O | SER | E | 296 | 43.296 | -81.062 | -7.290 | 1.00236.25 |
| ATOM | 4314 | CB | SER | E | 296 | 44.110 | -79.185 | -4.837 | 1.00242.87 |
| ATOM | 4315 | OG | SER | E | 296 | 43.798 | -77.991 | -4.134 | 1.00249.02 |
| ATOM | 4316 | N | SER | E | 297 | 43.196 | -82.326 | -5.409 | 1.00235.00 |
| ATOM | 4317 | CA | SER | E | 297 | 43.590 | -83.584 | -6.036 | 1.00235.87 |
| ATOM | 4318 | C | SER | E | 297 | 42.527 | -84.274 | -6.879 | 1.00232.12 |
| ATOM | 4319 | O | SER | E | 297 | 42.835 | -85.250 | -7.572 | 1.00233.97 |
| ATOM | 4320 | CB | SER | E | 297 | 44.157 | -84.540 | -4.986 | 1.00244.75 |
| ATOM | 4321 | OG | SER | E | 297 | 45.430 | -84.124 | -4.511 | 1.00255.53 |
| ATOM | 4322 | N | ASN | E | 298 | 41.288 | -83.782 | -6.835 | 1.00219.93 |
| ATOM | 4323 | CA | ASN | E | 298 | 40.180 | -84.439 | -7.516 | 1.00215.01 |
| ATOM | 4324 | C | ASN | E | 298 | 39.086 | -83.479 | -7.942 | 1.00210.76 |
| ATOM | 4325 | O | ASN | E | 298 | 37.946 | -83.900 | -8.138 | 1.00207.74 |
| ATOM | 4326 | CB | ASN | E | 298 | 39.601 | -85.484 | -6.573 | 1.00214.94 |
| ATOM | 4327 | CG | ASN | E | 298 | 39.090 | -84.892 | -5.280 | 1.00232.71 |
| ATOM | 4328 | OD1 | ASN | E | 298 | 39.452 | -83.777 | -4.882 | 1.00219.13 |
| ATOM | 4329 | ND2 | ASN | E | 298 | 38.207 | -85.612 | -4.613 | 1.00230.17 |
| ATOM | 4330 | N | LEU | E | 299 | 39.411 | -82.183 | -8.030 | 1.00204.53 |
| ATOM | 4331 | CA | LEU | E | 299 | 38.508 | -81.110 | -8.470 | 1.00199.54 |
| ATOM | 4332 | C | LEU | E | 299 | 37.179 | -81.013 | -7.751 | 1.00198.32 |
| ATOM | 4333 | O | LEU | E | 299 | 36.277 | -80.327 | -8.215 | 1.00195.06 |
| ATOM | 4334 | CB | LEU | E | 299 | 38.237 | -81.265 | -9.966 | 1.00198.11 |
| ATOM | 4335 | CG | LEU | E | 299 | 39.439 | -81.418 | -10.837 | 1.00206.60 |
| ATOM | 4336 | CD1 | LEU | E | 299 | 39.035 | -81.605 | -12.240 | 1.00205.07 |
| ATOM | 4337 | CD2 | LEU | E | 299 | 40.342 | -80.220 | -10.716 | 1.00212.90 |
| ATOM | 4338 | N | LEU | E | 300 | 37.043 | -81.677 | -6.628 | 1.00194.39 |
| ATOM | 4339 | CA | LEU | E | 300 | 35.761 | -81.773 | -5.966 | 1.00191.51 |
| ATOM | 4340 | C | LEU | E | 300 | 35.534 | -80.702 | -4.933 | 1.00195.80 |
| ATOM | 4341 | O | LEU | E | 300 | 36.339 | -80.524 | -4.010 | 1.00198.92 |
| ATOM | 4342 | CB | LEU | E | 300 | 35.693 | -83.156 | -5.311 | 1.00194.03 |
| ATOM | 4343 | CG | LEU | E | 300 | 34.398 | -83.946 | -5.293 | 1.00195.93 |
| ATOM | 4344 | CD1 | LEU | E | 300 | 34.475 | -85.088 | -6.308 | 1.00196.73 |
| ATOM | 4345 | CD2 | LEU | E | 300 | 34.219 | -84.587 | -3.942 | 1.00199.61 |
| ATOM | 4346 | N | CYS | E | 301 | 34.403 | -80.018 | -5.049 | 1.00189.73 |
| ATOM | 4347 | CA | CYS | E | 301 | 34.022 | -79.049 | -4.034 | 1.00190.92 |
| ATOM | 4348 | C | CYS | E | 301 | 33.695 | -79.784 | -2.736 | 1.00196.75 |
| ATOM | 4349 | O | CYS | E | 301 | 32.854 | -80.684 | -2.747 | 1.00196.15 |
| ATOM | 4350 | CB | CYS | E | 301 | 32.831 | -78.228 | -4.499 | 1.00189.17 |
| ATOM | 4351 | SG | CYS | E | 301 | 33.210 | -77.054 | -5.812 | 1.00191.97 |
| ATOM | 4352 | N | THR | E | 302 | 34.374 | -79.412 | -1.638 | 1.00195.57 |
| ATOM | 4353 | CA | THR | E | 302 | 34.148 | -79.928 | -0.285 | 1.00198.15 |
| ATOM | 4354 | C | THR | E | 302 | 33.762 | -78.747 | 0.606 | 1.00203.83 |
| ATOM | 4355 | O | THR | E | 302 | 34.246 | -77.636 | 0.406 | 1.00202.44 |
| ATOM | 4356 | CB | THR | E | 302 | 35.387 | -80.610 | 0.269 | 1.00205.07 |
| ATOM | 4357 | OG1 | THR | E | 302 | 36.446 | -79.664 | 0.287 | 1.00201.25 |
| ATOM | 4358 | CG2 | THR | E | 302 | 35.801 | -81.816 | -0.543 | 1.00203.88 |
| ATOM | 4359 | N | PRO | E | 303 | 32.903 | -78.958 | 1.602 | 1.00204.10 |
| ATOM | 4360 | CA | PRO | E | 303 | 32.479 | -77.842 | 2.454 | 1.00206.12 |
| ATOM | 4361 | C | PRO | E | 303 | 33.569 | -77.344 | 3.369 | 1.00218.64 |
| ATOM | 4362 | O | PRO | E | 303 | 34.543 | -78.048 | 3.594 | 1.00219.97 |
| ATOM | 4363 | CB | PRO | E | 303 | 31.322 | -78.429 | 3.238 | 1.00208.87 |
| ATOM | 4364 | CG | PRO | E | 303 | 31.643 | -79.871 | 3.325 | 1.00214.74 |
| ATOM | 4365 | CD | PRO | E | 303 | 32.247 | -80.213 | 1.999 | 1.00207.45 |
| ATOM | 4366 | N | CYS | E | 304 | 33.387 | -76.148 | 3.912 | 1.00222.04 |
| ATOM | 4367 | CA | CYS | E | 304 | 34.388 | -75.520 | 4.747 | 1.00228.93 |
| ATOM | 4368 | C | CYS | E | 304 | 34.337 | -75.876 | 6.210 | 1.00237.79 |
| ATOM | 4369 | O | CYS | E | 304 | 33.281 | -75.776 | 6.827 | 1.00237.52 |
| ATOM | 4370 | CB | CYS | E | 304 | 34.375 | -74.010 | 4.546 | 1.00230.31 |
| ATOM | 4371 | SG | CYS | E | 304 | 34.621 | -73.503 | 2.833 | 1.00231.42 |
| ATOM | 4372 | N | LEU | E | 305 | 35.502 | -76.261 | 6.778 | 1.00239.18 |
| ATOM | 4373 | CA | LEU | E | 305 | 35.682 | -76.438 | 8.220 | 1.00244.47 |
| ATOM | 4374 | C | LEU | E | 305 | 35.892 | -74.976 | 8.675 | 1.00250.42 |
| ATOM | 4375 | O | LEU | E | 305 | 36.947 | -74.380 | 8.407 | 1.00250.65 |
| ATOM | 4376 | CB | LEU | E | 305 | 36.916 | -77.319 | 8.567 | 1.00244.41 |
| ATOM | 4377 | CG | LEU | E | 305 | 37.176 | -77.597 | 10.081 | 1.00248.03 |
| ATOM | 4378 | CD1 | LEU | E | 305 | 37.743 | -78.988 | 10.318 | 1.00247.74 |
| ATOM | 4379 | CD2 | LEU | E | 305 | 38.113 | -76.568 | 10.698 | 1.00249.45 |
| ATOM | 4380 | N | GLY | E | 306 | 34.857 | -74.403 | 9.292 | 1.00249.91 |
| ATOM | 4381 | CA | GLY | E | 306 | 34.845 | -73.004 | 9.706 | 1.00250.65 |
| ATOM | 4382 | C | GLY | E | 306 | 34.681 | -72.105 | 8.494 | 1.00253.43 |
| ATOM | 4383 | O | GLY | E | 306 | 34.275 | -72.601 | 7.430 | 1.00249.28 |
| ATOM | 4384 | N | PRO | E | 307 | 35.019 | -70.782 | 8.607 | 1.00250.35 |
| ATOM | 4385 | CA | PRO | E | 307 | 34.885 | -69.888 | 7.436 | 1.00245.14 |
| ATOM | 4386 | C | PRO | E | 307 | 35.699 | -70.349 | 6.234 | 1.00244.34 |
| ATOM | 4387 | O | PRO | E | 307 | 36.746 | -70.987 | 6.390 | 1.00245.81 |
| ATOM | 4388 | CB | PRO | E | 307 | 35.378 | -68.527 | 7.954 | 1.00249.48 |
| ATOM | 4389 | CG | PRO | E | 307 | 35.309 | -68.605 | 9.437 | 1.00255.48 |
| ATOM | 4390 | CD | PRO | E | 307 | 35.527 | -70.048 | 9.793 | 1.00252.58 |
| ATOM | 4391 | N | CYS | E | 308 | 35.198 | -70.042 | 5.035 | 1.00235.87 |
| ATOM | 4392 | CA | CYS | E | 308 | 35.881 | -70.399 | 3.794 | 1.00233.11 |
| ATOM | 4393 | C | CYS | E | 308 | 37.032 | -69.450 | 3.518 | 1.00239.94 |
| ATOM | 4394 | O | CYS | E | 308 | 36.899 | -68.242 | 3.743 | 1.00240.32 |
| ATOM | 4395 | CB | CYS | E | 308 | 34.921 | -70.406 | 2.609 | 1.00227.97 |
| ATOM | 4396 | SG | CYS | E | 308 | 33.659 | -71.705 | 2.660 | 1.00229.62 |
| ATOM | 4397 | N | PRO | E | 309 | 38.126 | -69.944 | 2.917 | 1.00238.09 |
| ATOM | 4398 | CA | PRO | E | 309 | 39.201 | -69.017 | 2.534 | 1.00239.39 |
| ATOM | 4399 | C | PRO | E | 309 | 38.764 | -68.208 | 1.310 | 1.00239.61 |
| ATOM | 4400 | O | PRO | E | 309 | 38.110 | -68.768 | 0.416 | 1.00236.35 |
| ATOM | 4401 | CB | PRO | E | 309 | 40.383 | -69.941 | 2.207 | 1.00242.88 |
| ATOM | 4402 | CG | PRO | E | 309 | 39.931 | -71.356 | 2.565 | 1.00247.46 |
| ATOM | 4403 | CD | PRO | E | 309 | 38.436 | -71.337 | 2.534 | 1.00239.63 |
| ATOM | 4404 | N | LYS | E | 310 | 39.054 | -66.892 | 1.300 | 1.00236.04 |
| ATOM | 4405 | CA | LYS | E | 310 | 38.762 | -66.027 | 0.145 | 1.00237.75 |
| ATOM | 4406 | C | LYS | E | 310 | 39.630 | -64.787 | 0.090 | 1.00255.34 |
| ATOM | 4407 | O | LYS | E | 310 | 39.883 | -64.282 | -1.007 | 1.00202.82 |
| ATOM | 4408 | CB | LYS | E | 310 | 37.258 | -65.708 | -0.086 | 1.00235.92 |
| ATOM | 4409 | CG | LYS | E | 310 | 36.655 | -66.350 | -1.361 | 1.00225.27 |
| ATOM | 4410 | CD | LYS | E | 310 | 36.786 | -65.512 | -2.634 | 1.00218.67 |
| ATOM | 4411 | CE | LYS | E | 310 | 36.614 | -66.371 | -3.857 | 1.00211.31 |
| ATOM | 4412 | NZ | LYS | E | 310 | 36.952 | -65.619 | -5.088 | 1.00211.82 |
| ATOM | 4545 | N | PHE | F | 705 | 19.900 | -49.424 | -19.209 | 1.00185.33 |
| ATOM | 4546 | CA | PHE | F | 705 | 20.275 | -50.514 | -20.103 | 1.00185.17 |
| ATOM | 4547 | C | PHE | F | 705 | 21.295 | -50.100 | -21.117 | 1.00193.52 |
| ATOM | 4548 | O | PHE | F | 705 | 22.257 | -50.831 | -21.309 | 1.00194.56 |
| ATOM | 4549 | CB | PHE | F | 705 | 19.075 | -51.099 | -20.827 | 1.00186.00 |
| ATOM | 4550 | CG | PHE | F | 705 | 19.446 | -52.181 | -21.814 | 1.00186.21 |
| ATOM | 4551 | CD2 | PHE | F | 705 | 19.444 | -51.930 | -23.178 | 1.00187.56 |
| ATOM | 4552 | CD1 | PHE | F | 705 | 19.825 | -53.443 | -21.375 | 1.00188.11 |
| ATOM | 4553 | CE2 | PHE | F | 705 | 19.776 | -52.932 | -24.088 | 1.00189.94 |
| ATOM | 4554 | CE1 | PHE | F | 705 | 20.160 | -54.442 | -22.285 | 1.00188.66 |
| ATOM | 4555 | CZ | PHE | F | 705 | 20.122 | -54.183 | -23.636 | 1.00187.83 |
| ATOM | 4556 | N | GLU | F | 706 | 21.063 | -48.993 | -21.844 | 1.00191.84 |
| ATOM | 4557 | CA | GLU | F | 706 | 22.067 | -48.476 | -22.784 | 1.00192.58 |
| ATOM | 4558 | C | GLU | F | 706 | 23.339 | -48.321 | -21.923 | 1.00196.89 |
| ATOM | 4559 | O | GLU | F | 706 | 24.394 | -48.869 | -22.259 | 1.00195.15 |
| ATOM | 4560 | CB | GLU | F | 706 | 21.595 | -47.117 | -23.360 | 1.00194.19 |
| ATOM | 4561 | CG | GLU | F | 706 | 22.679 | -46.223 | -23.953 | 1.00203.83 |
| ATOM | 4562 | CD | GLU | F | 706 | 22.396 | -45.707 | -25.350 | 1.00220.71 |
| ATOM | 4563 | OE1 | GLU | F | 706 | 23.044 | -46.201 | -26.303 | 1.00212.69 |
| ATOM | 4564 | OE2 | GLU | F | 706 | 21.523 | -44.819 | -25.496 | 1.00210.82 |
| ATOM | 4565 | N | ASP | F | 707 | 23.146 | -47.687 | -20.732 | 1.00195.23 |
| ATOM | 4566 | CA | ASP | F | 707 | 24.094 | -47.455 | -19.637 | 1.00195.93 |
| ATOM | 4567 | C | ASP | F | 707 | 24.726 | -48.781 | -19.142 | 1.00199.86 |
| ATOM | 4568 | O | ASP | F | 707 | 25.843 | -48.770 | -18.609 | 1.00200.04 |
| ATOM | 4569 | CB | ASP | F | 707 | 23.422 | -46.676 | -18.471 | 1.00198.28 |
| ATOM | 4570 | CG | ASP | F | 707 | 21.896 | -46.628 | -18.508 | 1.00212.14 |
| ATOM | 4571 | OD1 | ASP | F | 707 | 21.348 | -45.809 | -19.282 | 1.00213.03 |
| ATOM | 4572 | OD2 | ASP | F | 707 | 21.249 | -47.421 | -17.766 | 1.00217.74 |
| ATOM | 4573 | N | TYR | F | 708 | 24.007 | -49.916 | -19.313 | 1.00194.77 |
| ATOM | 4574 | CA | TYR | F | 708 | 24.530 | -51.234 | -18.976 | 1.00193.75 |
| ATOM | 4575 | C | TYR | F | 708 | 25.375 | -51.624 | -20.181 | 1.00192.06 |
| ATOM | 4576 | O | TYR | F | 708 | 26.595 | -51.672 | -20.059 | 1.00191.67 |
| ATOM | 4577 | CB | TYR | F | 708 | 23.384 | -52.238 | -18.714 | 1.00196.38 |
| ATOM | 4578 | CG | TYR | F | 708 | 23.785 | -53.697 | -18.550 | 1.00200.12 |
| ATOM | 4579 | CD2 | TYR | F | 708 | 23.982 | -54.251 | -17.287 | 1.00201.14 |
| ATOM | 4580 | CD1 | TYR | F | 708 | 23.825 | -54.558 | -19.646 | 1.00202.45 |
| ATOM | 4581 | CE2 | TYR | F | 708 | 24.249 | -55.613 | -17.121 | 1.00201.97 |
| ATOM | 4582 | CE1 | TYR | F | 708 | 24.139 | -55.911 | -19.495 | 1.00203.66 |
| ATOM | 4583 | CZ | TYR | F | 708 | 24.332 | -56.440 | -18.228 | 1.00209.05 |
| ATOM | 4584 | OH | TYR | F | 708 | 24.597 | -57.783 | -18.067 | 1.00209.27 |
| ATOM | 4585 | N | LEU | F | 709 | 24.729 | -51.780 | -21.361 | 1.00184.40 |
| ATOM | 4586 | CA | LEU | F | 709 | 25.309 | -52.165 | -22.642 | 1.00182.32 |
| ATOM | 4587 | C | LEU | F | 709 | 26.686 | -51.583 | -22.902 | 1.00184.12 |
| ATOM | 4588 | O | LEU | F | 709 | 27.588 | -52.316 | -23.284 | 1.00182.43 |
| ATOM | 4589 | CB | LEU | F | 709 | 24.363 | -51.826 | -23.784 | 1.00181.94 |
| ATOM | 4590 | CG | LEU | F | 709 | 24.516 | -52.751 | -24.968 | 1.00186.75 |
| ATOM | 4591 | CD1 | LEU | F | 709 | 23.177 | -53.192 | -25.479 | 1.00187.35 |
| ATOM | 4592 | CD2 | LEU | F | 709 | 25.339 | -52.120 | -26.071 | 1.00189.52 |
| ATOM | 4593 | N | HIS | F | 710 | 26.844 | -50.271 | -22.675 | 1.00179.97 |
| ATOM | 4594 | CA | HIS | F | 710 | 28.108 | -49.569 | -22.855 | 1.00178.88 |
| ATOM | 4595 | C | HIS | F | 710 | 29.151 | -50.089 | -21.879 | 1.00179.18 |
| ATOM | 4596 | O | HIS | F | 710 | 30.145 | -50.663 | -22.314 | 1.00178.29 |
| ATOM | 4597 | CB | HIS | F | 710 | 27.929 | -48.047 | -22.692 | 1.00179.80 |
| ATOM | 4598 | CG | HIS | F | 710 | 27.077 | -47.378 | -23.729 | 1.00183.06 |
| ATOM | 4599 | ND1 | HIS | F | 710 | 26.729 | -48.015 | -24.915 | 1.00184.75 |
| ATOM | 4600 | CD2 | HIS | F | 710 | 26.573 | -46.122 | -23.741 | 1.00184.44 |
| ATOM | 4601 | CE1 | HIS | F | 710 | 25.996 | -47.143 | -25.582 | 1.00184.10 |
| ATOM | 4602 | NE2 | HIS | F | 710 | 25.887 | -45.985 | -24.920 | 1.00184.35 |
| ATOM | 4603 | N | ASN | F | 711 | 28.897 | -49.943 | -20.567 | 1.00173.66 |
| ATOM | 4604 | CA | ASN | F | 711 | 29.771 | -50.419 | -19.498 | 1.00173.15 |
| ATOM | 4605 | C | ASN | F | 711 | 30.283 | -51.872 | -19.699 | 1.00176.40 |
| ATOM | 4606 | O | ASN | F | 711 | 31.379 | -52.185 | -19.246 | 1.00176.64 |
| ATOM | 4607 | CB | ASN | F | 711 | 29.073 | -50.271 | -18.143 | 1.00175.14 |
| ATOM | 4608 | CG | ASN | F | 711 | 29.275 | -48.940 | -17.447 | 1.00198.08 |
| ATOM | 4609 | OD1 | ASN | F | 711 | 30.290 | -48.259 | -17.635 | 1.00193.99 |
| ATOM | 4610 | ND2 | ASN | F | 711 | 28.307 | -48.537 | -16.614 | 1.00185.99 |
| ATOM | 4611 | O | VAL | F | 712 | 31.806 | -54.707 | -22.029 | 1.00169.94 |
| ATOM | 4612 | N | VAL | F | 712 | 29.501 | -52.747 | -20.384 | 1.00171.53 |
| ATOM | 4613 | CA | VAL | F | 712 | 29.884 | -54.143 | -20.695 | 1.00170.19 |
| ATOM | 4614 | C | VAL | F | 712 | 30.676 | -54.213 | -22.002 | 1.00172.12 |
| ATOM | 4615 | CB | VAL | F | 712 | 28.708 | -55.183 | -20.661 | 1.00173.30 |
| ATOM | 4616 | CG1 | VAL | F | 712 | 27.351 | -54.532 | -20.818 | 1.00172.82 |
| ATOM | 4617 | CG2 | VAL | F | 712 | 28.890 | -56.304 | -21.688 | 1.00172.86 |
| ATOM | 4618 | O | VAL | F | 713 | 32.557 | -53.252 | -25.667 | 1.00169.23 |
| ATOM | 4619 | N | VAL | F | 713 | 30.043 | -53.717 | -23.078 | 1.00168.79 |
| ATOM | 4620 | CA | VAL | F | 713 | 30.492 | -53.669 | -24.469 | 1.00167.59 |
| ATOM | 4621 | C | VAL | F | 713 | 31.855 | -52.928 | -24.704 | 1.00170.88 |
| ATOM | 4622 | CB | VAL | F | 713 | 29.285 | -53.114 | -25.275 | 1.00170.11 |
| ATOM | 4623 | CG1 | VAL | F | 713 | 29.682 | -52.312 | -26.495 | 1.00170.05 |
| ATOM | 4624 | CG2 | VAL | F | 713 | 28.310 | -54.221 | -25.627 | 1.00169.52 |
| ATOM | 4625 | O | PHE | F | 714 | 35.718 | -51.468 | -23.045 | 1.00156.77 |
| ATOM | 4626 | N | PHE | F | 714 | 32.239 | -51.984 | -23.790 | 1.00167.63 |
| ATOM | 4627 | CA | PHE | F | 714 | 33.424 | -51.114 | -23.898 | 1.00175.55 |
| ATOM | 4628 | C | PHE | F | 714 | 34.510 | -51.293 | -22.777 | 1.00194.14 |
| ATOM | 4629 | CB | PHE | F | 714 | 32.947 | -49.624 | -24.032 | 1.00176.93 |
| ATOM | 4630 | CG | PHE | F | 714 | 32.043 | -49.243 | -25.217 | 1.00177.36 |
| ATOM | 4631 | CD1 | PHE | F | 714 | 32.554 | -49.151 | -26.511 | 1.00178.99 |
| ATOM | 4632 | CD2 | PHE | F | 714 | 30.705 | -48.921 | -25.022 | 1.00178.41 |
| ATOM | 4633 | CE1 | PHE | F | 714 | 31.726 | -48.817 | -27.589 | 1.00178.89 |
| ATOM | 4634 | CE2 | PHE | F | 714 | 29.880 | -48.582 | -26.105 | 1.00180.35 |
| ATOM | 4635 | CZ | PHE | F | 714 | 30.399 | -48.524 | -27.377 | 1.00177.82 |
| END | | | | | | | | | |

| | | | | | | | | | |
|---|---|---|---|---|---|---|---|---|---|
| Note: The coordinates in this Appendix describe the asymmetric unit of the crystal unit cell. | | | | | | | | | |

## Claims

1. An insulin/IR complex in crystalline form wherein the complex comprises the components of the structure defined by the atomic coordinates shown in Appendix I or a subset thereof.

2. A method of identifying, designing or screening for a compound that can interact with IR, including performing structure-based identification, design, or screening of a compound based on the compound's interactions with an IR structure defined by the atomic coordinates shown in Appendix I or a subset thereof.

3. A method of identifying, designing or screening for a compound that can mimic insulin interacting with IR, including performing structure-based identification, design, or screening of a compound based on (i) the compound's interactions with an IR structure defined by the atomic coordinates shown in Appendix I or a subset thereof and/or (ii) the compound's similarity with an insulin structure in complex with an IR defined by the atomic coordinates shown in Appendix I or a subset thereof.

4. A method for identifying an agonist or antagonist compound comprising an entity selected from the group consisting of an antibody, a peptide, a non-peptide molecule and a chemical compound, wherein said compound is capable of enhancing, eliciting or blocking biological activity resulting from an interaction with insulin and/or the IR, wherein said process includes:
introducing into a suitable computer program parameters defining an interacting surface based on the conformation of insulin and/or IR corresponding to the atomic coordinates of Appendix I or a subset thereof, wherein said program displays a three-dimensional model of the interacting surface;
creating a three-dimensional structure of a test compound in said computer program;
displaying a superimposing model of said test compound on the three-dimensional model of the interacting surface; and
assessing whether said test compound model fits spatially into a binding site.

5. A method according to claim 4, wherein said method comprises incorporating said test compound in a biological activity assay and determining whether said test compound inhibits or enhances the biological activity of insulin or IR signalling or signalling by a derivative of insulin or IR.

6. Use of the atomic coordinates or a subset thereof as shown in Appendix I at least representing:
(i) insulin; and/or
(ii) one or more regions of insulin in complex with the N- and C-terminal regions of the IR α-chain,
in identifying, designing or screening for a compound that can mimic insulin interacting with IR, including performing structure-based identification, design, or screening of a compound based on (a) the compound's interactions with an IR structure and/or (b) the compound's similarity with an insulin structure in complex with an IR defined by the atomic coordinates or a subset thereof.

7. Use of the atomic coordinates or a subset thereof as shown in Appendix I at least representing:
(i) the N-terminal region of the IR α-chain;
(ii) the C-terminal region of the IR α-chain; and/or
(iii) one or more regions of the N- and C-terminal regions of the IR α-chain in complex with insulin,
in identifying, designing or screening for a compound that can interact with IR, including performing structure-based identification, design, or screening of a compound based on the compound's interactions with an IR structure defined by the atomic coordinates or a subset thereof.

8. A method of redesigning a compound which is known to bind to IR and/or IGF-1R comprising performing structure-based evaluation of (i) the compound based on the compound's interactions with an IR structure defined by the atomic coordinates of Appendix I or a subset thereof and redesigning or chemically modifying the compound as a result of the evaluation, or (ii) the compound's similarity with an insulin structure in complex with an IR defined by the atomic coordinates of Appendix I or a subset thereof and redesigning or chemically modifying the compound as a result of the evaluation.

9. A computer-assisted method of identifying a compound that interacts with IR and/or IGF-1R, which method comprises fitting the structure of:
(i) the Site 1 binding site of IR, the structure being defined by a subset of the atomic coordinates shown in Appendix I; and/or
(ii) portions of the N- and C-terminal regions of the IR α-chain, which are in complex with insulin, the structure being defined by a subset of the atomic coordinates shown in Appendix I,
to the structure of a candidate compound.

10. A computer-assisted method for identifying a molecule able to interact with IR and/or IGF-1R using a programmed computer comprising a processor, which method comprises the steps of:
(a) generating, using computer methods, a set of atomic coordinates of a structure that possesses energetically favourable interactions with the atomic coordinates of:
(i) the Site 1 binding site of IR, the structure being defined by a subset of the atomic coordinates shown in Appendix I; and/or
(ii) portions of the N- and C-terminal regions of the IR α-chain, which are in complex with insulin, the structure being defined by a subset of the atomic coordinates shown in Appendix I, which coordinates are entered into the computer thereby generating a criteria data set;
(b) comparing, using the processor, the criteria data set to a computer database of chemical structures; and
(c) selecting from the database, using computer methods, chemical structures which are complementary or similar to a region of the criteria data set.

11. A method according to claim 10, wherein said method further comprises
(d) outputting, to an output device, the selected chemical structures which are complementary to or similar to a region of the criteria data set.

12. A computer-assisted method for identifying mimetics of IR, insulin and/or IGF-1R using a programmed computer comprising a processor, the method comprising the steps of:
(a) generating a criteria data set from a set of atomic coordinates of:
(i) the Site 1 binding site of IR, the structure being defined by a subset of the atomic coordinates shown in Appendix I;
(ii) portions of the N- and C-terminal regions of the IR α-chain, which are in complex with insulin, the structure being defined by a subset of the atomic coordinates shown in Appendix I;
(iii) insulin, the structure being defined by a subset of the atomic coordinates shown in Appendix I; and/or
(iv) the Site 1 binding site of insulin or portions thereof, the structure being defined by a subset of the atomic coordinates shown in Appendix I, which coordinates are entered into the computer; and
(b):
(i) comparing, using the processor, the criteria data set to a computer database of chemical structures stored in a computer data storage system and selecting from the database, using computer methods, chemical structures having a region that is structurally similar to the criteria data set; or
(ii) constructing, using computer methods, a model of a chemical structure having a region that is structurally similar to the criteria data set.

13. A method according to claim 12, wherein said method further comprises (c) outputting to an output device:
(i) the selected chemical structures from step (b)(i) having a region similar to the criteria data set; or
(ii) the constructed model from step (b)(ii).

14. A method for evaluating the ability of a compound to interact with IR and/or IGF-1R, the method comprising the steps of:
(a) employing computational means to perform:
(i) a fitting operation between the compound and the binding surface of a computer model of the Site 1 binding site for insulin on IR; and/or
(ii) a superimposing operation between the compound and insulin, the Site 1 binding site of insulin, or a portion thereof, using atomic coordinates wherein the root mean square deviation between the atomic coordinates and a subset of atomic coordinates of Appendix I or a subset of atomic coordinates of one or more thereof at least representing the N-terminal region of the IR α-chain, the C-terminal region of IR α-chain, insulin, the Site 1 binding site of insulin, or a portion of the Site 1 binding site of insulin, is not more than 1.5 Å; and
(b) analysing the results of the fitting operation and/or superimposing operation to quantify the association between the compound and the binding surface model.

15. A method of using molecular replacement to obtain structural information about a molecule or a molecular complex of an unknown structure, comprising the steps of:
(i) generating an X-ray diffraction pattern of the crystallized molecule or molecular complex; and
(ii) applying the atomic coordinates of Appendix I, or a subset of atomic coordinates thereof at least representing the N-terminal region of the IR α-chain, the C-terminal region of IR α-chain, insulin, mimetics thereof, derivatives thereof, or portions thereof, to the X-ray diffraction pattern to generate a three-dimensional electron density map of at least a region of the molecule or molecular complex whose structure is unknown.

## Patentansprüche

1. Insulin/IR-Komplex in kristalliner Form, wobei der Komplex die Komponenten der Struktur, die durch die in Anhang I gezeigten Atomkoordinaten oder einer Teilmenge davon definiert ist, umfasst.

2. Verfahren zum Identifizieren, Konstruieren oder Screenen nach einer Verbindung, die mit IR wechselwirken kann, einschließlich das Durchführen strukturbasierter Identifizierung, Konstruktion oder Screening einer Verbindung, basierend auf den Wechselwirkungen der Verbindung mit einer IR-Struktur, die durch die in Anhang I gezeigten Atomkoordinaten oder einer Teilmenge davon definiert ist.

3. Verfahren zum Identifizieren, Konstruieren oder Screenen nach einer Verbindung, die das Wechselwirken von Insulin mit IR nachahmen kann, einschließlich das Durchführen strukturbasierter Identifizierung, Konstruktion oder Screening einer Verbindung, basierend auf (i) den Wechselwirkungen der Verbindung mit einer IR-Struktur, die durch die in Anhang I gezeigten Atomkoordinaten oder einer Teilmenge davon definiert ist und/oder (ii) der Ähnlichkeit der Verbindung mit einer Insulinstruktur im Komplex mit einem IR, die durch die in Anhang I gezeigten Atomkoordinaten oder einer Teilmenge davon definiert ist.

4. Verfahren zum Identifizieren einer Agonist- oder Antagonistverbindung, umfassend eine Einheit ausgewählt aus der Gruppe bestehend aus einem Antikörper, einem Peptid, einem Nicht-Peptidmolekül und einer chemischen Verbindung, wobei die Verbindung in der Lage ist, die aus einer Wechselwirkung mit Insulin und/oder dem IR resultierende biologische Aktivität zu erhöhen, auszulösen oder zu blockieren, wobei das Verfahren:
das Einführen von Parametern in ein geeignetes Computerprogramm, die, basierend auf der Konformation von Insulin und/oder IR entsprechend den Atomkoordinaten von Anhang I oder einer Teilmenge davon, eine wechselwirkende Oberfläche definieren, wobei das Programm ein dreidimensionales Modell der wechselwirkenden Oberfläche anzeigt;
das Erstellen einer dreidimensionalen Struktur einer Testverbindung in dem Computerprogramm;
das Anzeigen eines Überlagerungsmodells der Testverbindung auf dem dreidimensionalen Modell der wechselwirkenden Oberfläche; und
das Bewerten, ob das Testverbindungsmodell räumlich in eine Bindungsstelle passt, beinhaltet.

5. Verfahren nach Anspruch 4, wobei die Methode das Aufnehmen der Testverbindung in eine biologische Aktivitätsuntersuchung und das Bestimmen, ob die Testverbindung die biologische Aktivität eines Insulin- oder IR-Signals oder eines Insulin- oder IR-Derivatsignals hemmt oder erhöht, umfasst.

6. Verwendung der in Anhang I gezeigten Atomkoordinaten oder einer Teilmenge davon, die mindestens:
(i) Insulin; und/oder
(ii) eine oder mehr Bereiche von Insulin im Komplex mit den N- und C-terminalen Bereichen der IR-α-Kette
zum Identifizieren, Konstruieren oder Screenen nach einer Verbindung, die das Wechselwirken von Insulin mit IR nachahmen kann, einschließlich das Durchführen strukturbasierter Identifizierung, Konstruktion oder Screening einer Verbindung, basierend auf (a) den Wechselwirkungen der Verbindung mit einer IR-Struktur und/oder (b) der Ähnlichkeit der Verbindung mit einer Insulinstruktur im Komplex mit einem IR, die durch die in Anhang I gezeigten Atomkoordinaten oder einer Teilmenge davon definiert ist, darstellen.

7. Verwendung der in Anhang I gezeigten Atomkoordinaten oder einer Teilmenge davon, die mindestens:
(i) den N-terminalen Bereich der IR-α-Kette;
(ii) den C-terminalen Bereich der IR-α-Kette; und/oder
(iii) einen oder mehrere Bereiche der N- und C-terminalen Bereiche der IR-α-Kette im Komplex mit Insulin,
zum Identifizieren, Konstruieren oder Screenen nach einer Verbindung, die mit IR wechselwirken kann, einschließlich das Durchführen strukturbasierter Identifizierung, Konstruktion oder Screening einer Verbindung, basierend auf den Wechselwirkungen der Verbindung mit einer IR-Struktur, die durch die in Anhang I gezeigten Atomkoordinaten oder einer Teilmenge davon definiert ist, darstellen.

8. Verfahren zum Rekonstruieren einer Verbindung, von der bekannt ist, dass sie an IR und/oder IGF-1R bindet, umfassend das Durchführen einer strukturbasierten Bewertung der (i) Verbindung, basierend auf den Wechselwirkungen der Verbindung mit einer IR-Struktur, die durch die in Anhang I gezeigten Atomkoordinaten oder einer Teilmenge davon definiert ist und das Rekonstruieren oder chemische Modifizieren der Verbindung als ein Ergebnis der Bewertung oder (ii) der Ähnlichkeit der Verbindung mit einer Insulinstruktur im Komplex mit einem IR, die durch die in Anhang I gezeigten Atomkoordinaten oder einer Teilmenge davon definiert ist und das Rekonstruieren oder chemische Modifizieren der Verbindung als ein Ergebnis der Bewertung.

9. Computergestütztes Verfahren zum Identifizieren einer Verbindung, die mit IR und/oder IGF-1R wechselwirkt, wobei das Verfahren das Anpassen der Struktur von:
(i) der Bindungsstelle an Stelle 1 des IR, wobei die Struktur durch eine Teilmenge der in Anhang I gezeigten Atomkoordinaten definiert ist; und/oder
(ii) Teilen der N- und C-terminalen Bereiche der IR-α-Kette, die mit Insulin im Komplex sind, wobei die Struktur durch eine Teilmenge der in Anhang I gezeigten Atomkoordinaten definiert ist,
zur Struktur einer Kandidatenverbindung umfasst.

10. Computergestütztes Verfahren zum Identifizieren eines Moleküls, das unter Verwendung eines einen Prozessor umfassenden, programmierten Computers, in der Lage ist, mit IR und/oder IGF-1R zu wechselwirken, wobei das Verfahren die Schritte des:
(a) Erzeugens einer Menge von Atomkoordinaten, unter Verwendung von Computermethoden, mit einer Struktur, die energetisch günstige Wechselwirkungen mit den Atomkoordinaten von:
(i) der Bindungsstelle an Stelle 1 des IR, wobei die Struktur durch eine Teilmenge der in Anhang I gezeigten Atomkoordinaten definiert ist; und/oder
(ii) Teilen der N- und C-terminalen Bereiche der IR-α-Kette, die mit Insulin im Komplex sind, wobei die Struktur durch eine Teilmenge der in Anhang I gezeigten Atomkoordinaten definiert ist, aufweisen, wobei diese Koordinaten in den Computer eingegeben werden, wodurch ein Kriteriendatensatz erzeugt wird;
(b) Vergleichens des Kriteriendatensatzes, unter Verwendung des Prozessors, mit einer Computerdatenbank mit chemischen Strukturen; und
(c) Auswählens, unter Verwendung von Computermethoden, von chemischen Strukturen aus der Datenbank, die komplementär oder ähnlich zu einem Bereich des Kriteriendatensatzes sind, umfasst.

11. Verfahren nach Anspruch 10, wobei das Verfahren weiter
(d) das Ausgeben der ausgewählten chemischen Strukturen, die komplementär oder ähnlich zu einem Bereich des Kriteriendatensatzes sind, an eine Ausgabevorrichtung umfasst.

12. Computergestütztes Verfahren zum Identifizieren von Mimetika von IR, Insulin und/oder IGF-1R, unter Verwendung eines einen Prozessor umfassenden, programmierten Computers, wobei das Verfahren die Schritte des:
(a) Erzeugens eines Kriteriendatensatzes aus einer Menge von Atomkoordinaten von:
(i) der Bindungsstelle an Stelle 1 des IR, wobei die Struktur durch eine Teilmenge der in Anhang I gezeigten Atomkoordinaten definiert ist; und/oder
(ii) Teilen der N- und C-terminalen Bereiche der IR-α-Kette, die mit Insulin im Komplex sind, wobei die Struktur durch eine Teilmenge der in Anhang I gezeigten Atomkoordinaten definiert ist;
(iii) Insulin, wobei die Struktur durch eine Teilmenge der in Anhang I gezeigten Atomkoordinaten definiert ist; und/oder
(iv) der Bindungsstelle an Stelle 1 von Insulin oder Teilen davon, wobei die Struktur durch eine Teilmenge der in Anhang I gezeigten Atomkoordinaten definiert ist, wobei diese Koordinaten in den Computer eingegeben werden; und
(b):
(i) Vergleichens des Kriteriendatensatzes, unter Verwendung des Prozessors, mit einer Computerdatenbank mit chemischen Strukturen, die in einem Computerdatenspeichersystem gespeichert sind und Auswählens aus der Datenbank, unter Verwendung von Computermethoden, von chemischen Strukturen, die einen Bereich aufweisen, der dem Kriteriendatensatz strukturell ähnlich ist; oder
(ii) Erstellens, unter Verwendung von Computermethoden, eines Modells einer chemischen Struktur, die einen Bereich aufweist, der dem Kriteriendatensatz strukturell ähnlich ist, umfasst.

13. Verfahren nach Anspruch 12, wobei das Verfahren weiter (c) das Ausgaben:
(i) der ausgewählten chemischen Strukturen aus Schritt (b)(i), die einen Bereich aufweisen, der dem Kriteriendatensatz ähnlich ist; oder
(ii) des erstellten Modells aus Schritt (b)(ii) an eine Ausgabevorrichtung umfasst.

14. Verfahren zum Bewerten der Fähigkeit einer Verbindung, mit IR und/oder IGF-1R zu wechselwirken, wobei das Verfahren die Schritte des:
(a) Verwendens von Rechenmitteln zum Durchführen:
(i) eines Anpassungsvorgangs zwischen der Verbindung und der Bindungsoberfläche eines Computermodells der Bindungsstelle an Stelle 1 von Insulin an IR; und/oder
(ii) eines Überlagerungsvorgangs zwischen der Verbindung und Insulin, der Bindungsstelle an Stelle 1 von Insulin oder eines Teils davon, unter Verwendung von Atomkoordinaten, wobei die mittlere quadratische Abweichung zwischen den Atomkoordinaten und einer Teilmenge der Atomkoordinaten aus Anhang I oder einer Teilmenge von Atomkoordinaten von einem oder mehreren davon, die mindestens den N-terminalen Bereich der IR-α-Kette, den C-terminalen Bereich der IR-α-Kette, Insulin, der Bindungsstelle an Stelle 1 von Insulin oder einen Teil von der Bindungsstelle an Stelle 1 von Insulin darstellt, nicht mehr als 1,5 Ä beträgt; und
(b) Analysierens der Ergebnisse des Anpassungsvorgangs und/oder Überlagerungsvorgangs, um die Verbindung zwischen der Verbindung und dem Bindungsoberflächenmodell zu quantifizieren, umfasst.

15. Verfahren zur Verwendung von molekularem Austausch, um strukturelle Information über ein Molekül oder einen Molekülkomplex mit einer unbekannten Struktur zu erhalten, umfassend die Schritte des:
(i) Erzeugens eines Röntgenbeugungsmusters des kristallisierten Moleküls oder Molekülkomplexes; und
(ii) Anwendens der Atomkoordinaten aus Anhang I oder einer Teilmenge von dessen Atomkoordinaten, die mindestens den N-terminalen Bereich der IR-α-Kette, den C-terminalen Bereich der IR-α-Kette, Insulin, Mimetika davon, Derivate davon oder Teile davon repräsentieren, auf das Röntgenbeugungsmuster, um eine dreidimensionale Elektronendichtekarte von mindestens einem Bereich des Moleküls oder Molekülkomplexes zu erzeugen, deren Struktur unbekannt ist.

## Revendications

1. Complexe d'insuline/IR sous forme cristalline dans lequel le complexe comprend les composants de la structure définie par les coordonnées atomiques indiquées dans l'annexe I ou un sous-ensemble de celles-ci.

2. Procédé d'identification, de conception ou de criblage d'un composé qui peut interagir avec un IR, incluant la mise en oeuvre d'une identification, d'une conception, ou d'un criblage basé sur une structure d'un composé sur la base des interactions du composé avec une structure d'IR définie par les coordonnées atomiques indiquées dans l'annexe I ou un sous-ensemble de celles-ci.

3. Procédé d'identification, de conception ou de criblage d'un composé qui peut mimer une insuline interagissant avec un IR, incluant la mise en oeuvre d'une identification, d'une conception, ou d'un criblage basé sur une structure d'un composé sur la base (i) des interactions du composé avec une structure d'IR définie par les coordonnées atomiques indiquées dans l'annexe I ou un sous-ensemble de celles-ci et/ou (ii) de la similarité du composé avec une structure d'insuline dans un complexe avec un IR défini par les coordonnées atomiques indiquées dans l'annexe I ou un sous-ensemble de celles-ci.

4. Procédé d'identification d'un composé agoniste ou antagoniste comprenant une entité sélectionnée dans le groupe constitué d'un anticorps, d'un peptide, d'une molécule non peptidique et d'un composé chimique, dans lequel ledit composé est capable de renforcer, de déclencher ou de bloquer une activité biologique résultant d'une interaction avec une insuline et/ou l'IR, dans lequel ledit procédé comprend :
l'introduction dans un programme informatique approprié de paramètres définissant une surface d'interaction basée sur la conformation d'une insuline et/ou d'un IR correspondant aux coordonnées atomiques de l'annexe I ou à un sous-ensemble de celles-ci, dans lequel ledit programme affiche un modèle tridimensionnel de la surface d'interaction ;
la création d'une structure tridimensionnelle d'un composé de test dans ledit programme informatique ;
l'affichage d'un modèle de superposition dudit composé de test sur le modèle tridimensionnel de la surface d'interaction ; et
le fait d'évaluer si ledit modèle de composé de test s'ajuste ou non spatialement dans un site de liaison.

5. Procédé selon la revendication 4, dans lequel ledit procédé comprend l'incorporation dudit composé de test dans un essai d'activité biologique et le fait de déterminer si ledit composé de test inhibe ou renforce ou non l'activité biologique de la signalisation de l'insuline ou de l'IR ou la signalisation par un dérivé d'insuline ou d'IR.

6. Utilisation des coordonnées atomiques ou d'un sous-ensemble de celles-ci telles qu'indiquées dans l'annexe I représentant au moins :
(i) une insuline ; et/ou
(ii) une ou plusieurs régions d'insuline dans un complexe avec les régions N- et C-terminales de la chaîne a d'IR,
dans l'identification, la conception ou le criblage d'un composé qui peut mimer une insuline interagissant avec un IR, incluant la mise en oeuvre d'une identification, d'une conception, ou d'un criblage basé sur une structure d'un composé sur la base (a) des interactions du composé avec une structure d'IR et/ou (b) de la similarité du composé avec une structure d'insuline dans un complexe avec un IR défini par les coordonnées atomiques ou un sous-ensemble de celles-ci.

7. Utilisation des coordonnées atomiques ou d'un sous-ensemble de celles-ci telles qu'indiquées dans l'annexe I représentant au moins :
(i) la région N-terminale de la chaîne α d'IR ;
(ii) la région C-terminale de la chaîne α d'IR ; et/ou
(iii) une ou plusieurs régions des régions N- et C-terminales de la chaîne α d'IR dans un complexe avec l'insuline,
dans l'identification, la conception ou le criblage d'un composé qui peut interagir avec un IR, incluant la mise en oeuvre d'une identification, d'une conception, ou d'un criblage basé sur une structure d'un composé sur la base des interactions du composé avec une structure d'IR définie par les coordonnées atomiques ou un sous-ensemble de celles-ci.

8. Procédé de transformation d'un composé qui est connu pour se lier à un IR et/ou à un IGF-1R comprenant la mise en oeuvre d'une évaluation basée sur une structure (i) du composé sur la base des interactions du composé avec une structure d'IR définie par les coordonnées atomiques de l'annexe I ou un sous-ensemble de celles-ci et la transformation ou la modification chimique du composé à la suite de l'évaluation, ou (ii) de la similarité du composé avec une structure d'insuline dans un complexe avec un IR défini par les coordonnées atomiques de l'annexe I ou un sous-ensemble de celles-ci et la transformation ou la modification chimique du composé à la suite de l'évaluation.

9. Procédé assisté par ordinateur d'identification d'un composé qui interagit avec un IR et/ou un IGF-1R, lequel procédé comprend l'ajustement de la structure :
(i) du site de liaison Site 1 d'IR, la structure étant définie par un sous-ensemble des coordonnées atomiques indiquées dans l'annexe I ; et/ou
(ii) de portions des régions N- et C-terminales de la chaîne α d'IR, qui se trouvent dans un complexe avec une insuline, la structure étant définie par un sous-ensemble des coordonnées atomiques indiquées dans l'annexe I,
à la structure d'un composé candidat.

10. Procédé assisté par ordinateur pour identifier une molécule capable d'interagir avec un IR et/ou un IGF-1R en utilisant un ordinateur programmé comprenant un processeur, lequel procédé comprend les étapes suivantes :
(a) la génération, en utilisant des procédés informatiques, d'un ensemble de coordonnées atomiques d'une structure qui possède des interactions énergétiquement favorables avec les coordonnées atomiques :
(i) du site de liaison Site 1 d'IR, la structure étant définie par un sous-ensemble des coordonnées atomiques indiquées dans l'annexe I ; et/ou
(ii) de portions des régions N- et C-terminales de la chaîne α d'IR, qui se trouvent dans un complexe avec une insuline, la structure étant définie par un sous-ensemble des coordonnées atomiques indiquées dans l'annexe I, lesquelles coordonnées sont entrées dans l'ordinateur pour ainsi générer un ensemble de données de critère ;
(b) la comparaison, en utilisant le processeur, de l'ensemble de données de critère à une base de données informatiques de structures chimiques ; et
(c) la sélection à partir de la base de données, en utilisant des procédés informatiques, de structures chimiques qui sont complémentaires ou similaires à une région de l'ensemble de données de critère.

11. Procédé selon la revendication 10, dans lequel ledit procédé comprend en outre
(d) la sortie, vers un dispositif de sortie, des structures chimiques sélectionnées qui sont complémentaires ou similaires à une région de l'ensemble de données de critère.

12. Procédé assisté par ordinateur pour identifier des mimétiques d'IR, d'insuline et/ou d'IGF-1R en utilisant un ordinateur programmé comprenant un processeur, le procédé comprenant les étapes suivantes :
(a) la génération d'un ensemble de données de critère à partir d'un ensemble de coordonnées atomiques :
(i) du site de liaison Site 1 d'IR, la structure étant définie par un sous-ensemble des coordonnées atomiques indiquées dans l'annexe I ;
(ii) de portions des régions N- et C-terminales de la chaîne α d'IR, qui se trouvent dans un complexe avec une insuline, la structure étant définie par un sous-ensemble des coordonnées atomiques indiquées dans l'annexe I ;
(iii) de l'insuline, la structure étant définie par un sous-ensemble des coordonnées atomiques indiquées dans l'annexe I ; et/ou
(iv) du site de liaison Site 1 de l'insuline ou de portions de celui-ci, la structure étant définie par un sous-ensemble des coordonnées atomiques indiquées dans l'annexe I, lesquelles coordonnées sont entrées dans l'ordinateur ; et
(b) :
(i) la comparaison, en utilisant le processeur, de l'ensemble de données de critère à une base de données informatiques de structures chimiques stockées dans un système de stockage de données informatiques et la sélection à partir de la base de données, en utilisant des procédés informatiques, de structures chimiques ayant une région qui est structurellement similaire à l'ensemble de données de critère ; ou
(ii) la construction, en utilisant des procédés informatiques, d'un modèle d'une structure chimique ayant une région qui est structurellement similaire à l'ensemble de données de critère.

13. Procédé selon la revendication 12, dans lequel ledit procédé comprend en outre (c) la sortie vers un dispositif de sortie :
(i) des structures chimiques sélectionnées provenant de l'étape (b)(i) ayant une région similaire à l'ensemble de données de critère ; ou
(ii) du modèle construit provenant de l'étape (b)(ii).

14. Procédé d'évaluation de la capacité d'un composé à interagir avec un IR et/ou un IGF-1R, le procédé comprenant les étapes suivantes :
(a) l'utilisation de moyens de calcul pour mettre en oeuvre :
(i) une opération d'ajustement entre le composé et la surface de liaison d'un modèle informatique du site de liaison Site 1 pour une insuline sur un IR ; et/ou
(ii) une opération de superposition entre le composé et une insuline, le site de liaison Site 1 d'une insuline, ou une portion de celui-ci, en utilisant des coordonnées atomiques dans lequel l'écart quadratique moyen entre les coordonnés atomiques et un sous-ensemble de coordonnées atomiques de l'annexe I ou un sous-ensemble de coordonnées atomiques d'un ou de plusieurs de celles-ci représentant au moins la région N-terminale de la chaîne α d'IR, la région C-terminale de la chaîne α d'IR, une insuline, le site de liaison Site 1 d'une insuline, ou une portion du site de liaison Site 1 d'une insuline, n'est pas supérieur à 1,5 Å ; et
(b) l'analyse des résultats de l'opération d'ajustement et/ou de l'opération de superposition pour quantifier l'association entre le composé et le modèle de surface de liaison.

15. Procédé d'utilisation d'un remplacement moléculaire pour obtenir des informations structurelles concernant une molécule ou un complexe moléculaire de structure inconnue, comprenant les étapes suivantes :
(i) la génération d'un profil de diffraction des rayons X de la molécule cristallisée ou du complexe moléculaire cristallisé ; et
(ii) l'application des coordonnées atomiques de l'annexe I, ou d'un sous-ensemble de coordonnées atomiques de celles-ci représentant au moins la région N-terminale de la chaîne α d'IR, la région C-terminale de la chaîne α d'IR, une insuline, des mimétiques de ceux-ci, des dérivés de ceux-ci, ou des portions de ceux-ci, au profil de diffraction des rayons X pour générer une carte tridimensionnelle de densité électronique d'au moins une région de la molécule ou du complexe moléculaire dont la structure est inconnue.
